(19) Europäisches Patentamt
European Patent Office
Office européen des brevets

(11) EP 3 539 960 A1

(12) EUROPEAN PATENT APPLICATION
published in accordance with Art. 153(4) EPC

(43) Date of publication:
**18.09.2019 Bulletin 2019/38**

(21) Application number: **17870114.0**

(22) Date of filing: **10.11.2017**

(51) Int Cl.:
***C07D 471/04*** (2006.01) ***C07D 487/14*** (2006.01)
***C07D 487/04*** (2006.01) ***A61K 31/55*** (2006.01)
***A61P 35/00*** (2006.01) ***A61P 29/00*** (2006.01)

(86) International application number:
**PCT/CN2017/110565**

(87) International publication number:
**WO 2018/086605 (17.05.2018 Gazette 2018/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.11.2016 CN 201610990554**
**28.04.2017 CN 201710296368**

(71) Applicants:
- **Luoxin Pharmaceutical (Shanghai) Co., Ltd.**
  **China (Shanghai) Pilot Free Trade Zone**
  **Pudong New Area**
  **Shanghai 201210 (CN)**
- **Shandong Luoxin Pharmaceutical Group Stock Co., Ltd.**
  **Linyi, Shandong 276017 (CN)**

(72) Inventors:
- **HU, Yonghan**
  **Shanghai 201203 (CN)**
- **CAI, Dongmei**
  **Shanghai 201203 (CN)**
- **ZHU, Jiuxiang**
  **Shanghai 201203 (CN)**
- **DONG, Ping**
  **Shanghai 201203 (CN)**
- **DONG, Jiaqiang**
  **Shanghai 201210 (CN)**
- **WANG, Tie-Lin**
  **Shanghai 201210 (CN)**

(74) Representative: **Wohlfahrt, Jan Günther et al**
**Gleiss Große Schrell und Partner mbB**
**Patentanwälte Rechtsanwälte**
**Leitzstraße 45**
**70469 Stuttgart (DE)**

# (54) NITROGENOUS MACROCYCLIC COMPOUND, PREPARATION METHOD THEREFOR, PHARMACEUTICAL COMPOSITION AND APPLICATION THEREOF

(57) Disclosed in the present invention are a nitrogenous macrocyclic compound, a preparation method therefor, a pharmaceutical composition and an application thereof. The present invention provides a nitrogenous macrocyclic compound represented by formula **III-0**, a tautomer thereof, an optical isomer thereof, a hydrate thereof, a solvate thereof, a pharmacologically acceptable salt thereof, or a prodrug thereof. The compound can be effectively bond with bromodomains of a BET family: BRD4, BRD3, BRD2, and BRDT, so as to adjust transcription of a downstream gene c-myc and a related target gene thereof, and futher to adjust a downstream signal path and play a particular role, comprising treating diseases such as inflammatory diseases, cancers, and AIDS.

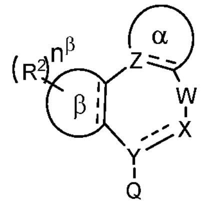

**III-0**

EP 3 539 960 A1

## Description

The present application claims the priority of Chinese Patent Application No. CN201610990554.X filed on November. 10, 2016. The present application claims the priority of Chinese patent application NO. CN201710296368.0 filed on April 28, 2017. The entire content of the aforementioned applications is hereby incorporated by reference.

## Field of invention

The present invention relates to a nitrogenous macrocyclic compound, a preparation method therefor, a pharmaceutical composition and a use thereof.

## Prior arts

Tumor is one of the leading causes of worldwide human death in recent years. The overall cure rate of tumor is low while the recurrence rate is high, thus the treatment of tumor shows important value.

Abnormal epigenetic regulation is one of the important factors leading to tumorigenesis. Epigenetics refer to changes in gene expression levels caused by non-gene sequence changes, including DNA methylation, histone modification, chromosomal remodeling and non-coding RNA regulation etc., which mainly affect the gene function and feature by regulations of gene transcription and translation process. Histone is the core of chromatin and is involved in post-transcription modification, including acetylation, methylation, phosphorylation and ubiquitination.

The bromodomain is an evolutionarily highly conserved protein consisting of 110 amino acids. It can mediate the protein interaction by recognizing the acetylated lysine residue on the histone, thus further affect the process of transcriptional regulation of genes. 61 types of bromodomains in 46 different proteins were found in the human gene genome. The bromodomain typically has deep hydrophobic pocket with small but tight binding site for its binding with acetylated lysine. Moreover, the conserved water molecules at the bottom of the pocket have a significant effect on its druggability. The binding of the bromodomain to the acetylated protein is generally weak (with a $K_D$ value in micromole to millimole level), which increases the probability of finding potent inhibitors. Evaluation of the druggability of the bromodomain family shows that the BET (bromodomain and additional C-terminal domain) subfamily scores relatively high, which has been proven by revealments of some small molecule inhibitors with different skeleton structures.

Human BET family consist of 4 members: BRD2, BRD3, BRD4 and BRDT. Each member comprises two tandem bromodomains (BD1 and BD2) for recognizing the acetylated lysine residues at the end of the histone, as well as an additional C-terminal domain. Wherein, BRD2 regulates the body’s energy balance, abnormal regulation of dyslipidemia or lipogenesis, inflammation levels and autoimmne diseases; BRD3 binds to the acetylated GATA1, regulating the red blood cell targeted genes; BRD4 marks mitosis and promotes transcription; BRDT only express in the testis and is important for expression of gene that produces sperm. After binding to histone, BRD2 and BRD3 involve in promoting transcriptional elongation, BRD4 could bind to positive transcriptional elongation factor b (P-TEFb), thus induces phosphorylation of RNA polymerase and increases the transcription output. BRD4 bins to different transcription factors and regulates downstream gene expression. Its binding with acetylated RelA induces stimulation of nuclear NF-κB and activate the transcription of inflammatory gene. BRD4 associates with the N-terminal domain of retinoic acid receptor $\alpha$ to regulate a discrete group of gene, and its association with p53 would regulate the p21 expression. BRD4 also interacts with several chromatin modifying enzymes, including histone methylase NSD3 and hydroxylase JMJD6. BRD4 target genes (eg c-Myc, C-Fos, aurora B, cyclin D1 and cyclin D2) take part in the regulation of the cell cycle. Research data show that BRD4 is also involved in the DNA damage signaling. BRD4 takes part in the regulation of the apolipoprotein A1 gene, therefore regulate the high-density lipoprotein level, which is related to the pathology of arteriosclerosis.

The BET family is associated with a variety of diseases. Chromosomal translocation induces BRD4 (or BRD3) fuses and co-expresses with testis nuclear protein (NUT), leading to a rare cancer: NUT midline cancer (NMC). BRD4 plays an important role in many hematological malignancies, including acute myeloid lymphoma, acute lymphoblastic leukemia, lymphoma and many myelomas. Besides, BRD4 also relates to a range of tumors, such as neuroblastoma, malignat glioma, lung cancer and melanoma. BRD4 is also associated with inflammation and the lifecycle of some viruses.

Therefore, the compound that inhibits the binding of bromodomain and acetylated protein indicates a trial of novel methods for treating inflammation and cancer. So far, academic and industrial research groups have found different chemical types of BET inhibitors, some of them already have entered clinical trial phase. A series of patent applications for BET inhibitors have been disclosed by now, including WO2011054553, WO2011054845, WO2013097052, WO2013185284, WO2014139324, WO2014164771, WO2015100282, WO2015075665, WO2015080707, WO2015164480, WO2015195862, WO2016050821 etc.

Wherein, the bromodomain inhibitor **ABBV-075**, developed by Abbive Company, has a promising prospect in the treatment of acute myeloid leukemia (AML), multiple myeloma (MM) and solid tumor, and is now under phase I

clinical trial. Tensha Therapeutics Company devotes to development of small molecule epigenetic protein BET inhibitor, and its first drug candidate Tensha-010 has two phase Ib studies being conducted. Besides, CPI-0610 developed by Constellation Pharmaceuticals company, GSK-525762 developed by GlaxoSmithKline and MK-8628 developed by Merck also have entered phase I/II clinical trial.

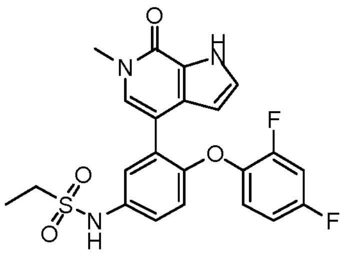
ABBV-075

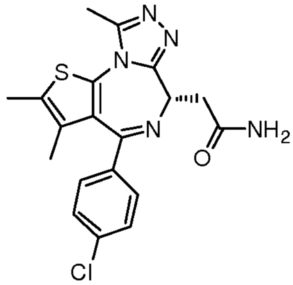
Tensha-010

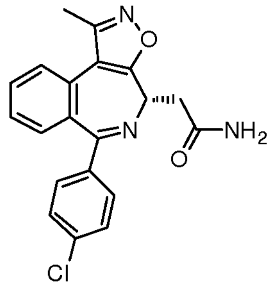
CPI-0610

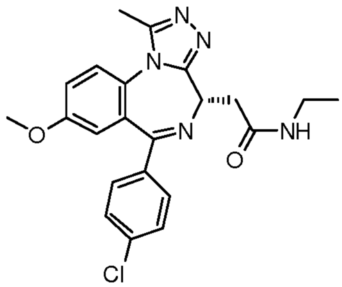
GSK525762

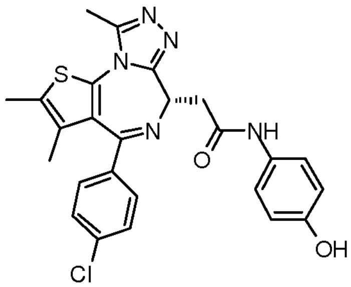
MK-8628

At this stage, there is an urgent need to develop more novel bromodomain inhibitors that could be used for treating diseases related to bromodomain function (including BET domain function) and other adaptation diseases.

**Content of the present invention**

The technical problem to be solved in the present invention is to develop more novel bromodomain inhibitors, thereby providing more therapeutic methods for diseases and adaptation diseases related to bromodomain function (including BET domain function), thus the present invention provides a series of nitrogenous macrocyclic compound with bromodomain inhibitory function, which is completely different from existing techniques. The nitrogenous macrocyclic compounds in the present invention could effectively bind to the bromodomain of the BET family BRD4, BRD3, BRD2 and BRDT, thereby regulating the transcription of the downstream gene c-myc and other related target genes, further regulating the downstream signal pathway and playing its specific role, including treatment of diseases such as inflammatory diseases, cancer and AIDS; some of the compound possess high activity and relatively good cell activity and metabolic stability, thus they could also be an effective drug for treating tumors.

The present invention solves the above technical problems by the following technical solutions.

The present invention provides a nitrogenous macrocyclic compound represented by formula **III-0,** a tautomer thereof, an optical isomer thereof, a hydrate thereof, a solvate thereof, a pharmaceutically acceptable salt thereof (e.g., a formate or a monoformate) or a prodrug thereof;

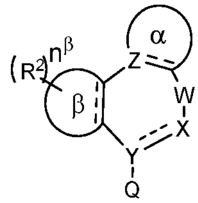

**III-0** ;

wherein, W is $-C(R^1)(R^3)-$ or $-N(R^4)-$;

each of $R^1$, $R^3$, $R^4$ is independently -H, or $C_1$-$C_5$ alkyl (for example $C_1$-$C_3$ alkyl, or for another example methyl, ethyl, n-propyl or isopropyl; also for example methyl, ethyl or isopropyl), $C_3$-$C_7$ cycloalkyl (for example cycloproyl), $C_1$-$C_5$ alkoxyl (for example $C_1$-$C_3$ alkoxyl; or for another example methoxy, ethoxy, n-propoxy or isopropoxy), $C_1$-$C_5$ haloalkyl (the number of halogen may be one or more (such as 2, 3 or 4), when more than one halogen exist, the halogens may be the same or different; the halo groups may be on the same carbon or not; the "$C_1$-$C_5$ haloalkyl" is, for example, "$C_1$-$C_3$ haloalkyl", or for another example trifluoromethyl, difluoromethyl, 1,2-difluoroethyl, also for example trifluoromethyl), $C_1$-$C_5$ alkyl substituted by one or more (e.g. 2, 3 or 4) of $R^{R15}$ (when more than one $R^{R15}$ exist, the $R^{R15}$(s) may be the same or different; the "$C_1$-$C_5$ alkyl" is, for example, methyl or ethyl; the "$C_1$-$C_5$ alkyl substituted by one $R^{R15}$" is, for example, 2-hydroxyethyl, carboxymethyl, hydroxymethyl or methoxymethyl, or for another example 2-hydroxyethyl, carboxymethyl, or methoxymethyl), halogen (for example fluorine or chlorine), $-(CH_2)n_0C(=O)N(R^{R1})(R^{R2})$, $-NH(CH_2)n_1C(=O)N(R^{R3})(R^{R4})$, $-(CH_2)n_2N(R^{R5})(R^{R6})$, $-(CH_2)n_3OC(=O)N(R^{R7})(R^{R8})$, $-(CH_2)_4NHC(=O)R^{R9}$, $-(CH_2)n_5NHC(=O)OR^{R10}$, $-(CH_2)n_6NHS(=O)_2R^{R11}$ or $-(CH_2)n_7S(=O)_2N(R^{R12})(R^{R13})$;

(both of the $R^1$ and $R^3$ may not be -H; the $R^1$ and $R^3$ may not be -H at the same time; at least one of the $R^1$ and $R^3$ (for example 1) may be -H; both of the $R^1$ and $R^3$ may be -H; when the $R^1$ and $R^3$ are different, the carbon atom they attached to is a chiral carbon atom; the configuration of the chiral carbon atom may be R or S)

all of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently -H, $C_1$-$C_5$ alkyl (for example $C_1$-$C_3$ alkyl, or for another example methyl, ethyl, n-propyl or isopropyl; also for example methyl, ethyl or isopropyl), $C_1$-$C_5$ haloalkyl, $-(CH_2)m_1CN$, $-C(CH_3)_2CN$, 3-7 membered cycloalkyl (for example cyclopropyl), or, $C_6$-$C_{10}$ aryl substituted by one or more (for example 2, 3 or 4) of $R^{R14}$ (when more than one $R^{R14}$ exist, the $R^{R14}$ may be the same or different; the "$C_6$-$C_{10}$ aryl" is, for example, phenyl; the $R^{R14}$ may independently attached to the ortho, meta or para position of the $C_6$-$C_{10}$ aryl; the " $C_6$-$C_{10}$ aryl substituted by one $R^{R14}$" is, for example, 4-hydroxyphenyl); $R^{R14}$ is independently -H, $C_1$-$C_5$ alkyl (for example $C_1$-$C_3$ alkyl, or for another example methyl, ethyl, n-propyl or isopropyl), $C_1$-$C_5$ alkoxyl, $C_1$-$C_5$ haloalkyl, halogen, -OH, -CN or $-NH_2$;

each of $n_0$, $n_1$, $n_2$, $n_3$, $n_4$, $n_5$, $n_6$, $n_7$ and $m_1$ is independently 0, 1, 2 or 3;

Alternatively, $R^1$, $R^3$ and the carbon atom they are attached to together form a $C_3$-$C_7$ cycloalkyl (for example cyclopropyl);

"

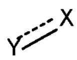

" represents the bond between X and Y is a single or double bond;

X is $-CH_2-$, =N-, -NH-, -O-, $-S(=O)_2-$ or -C(=O)-;

Y is

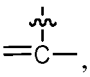, 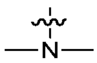 or 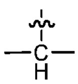;

and at least one of X and Y is a nitrogen atom;

Q is phenyl substituted by one or more (for example 2, 3 or 4) of $R^{Q1}$ (when more than one $R^{Q1}$ exist, the $R^{Q1}$ may be the same or different; each of the $R^{Q1}$ may be independently attached to the ortho, meta or para position of the phenyl; when two $R^{Q1}$ are present, the $R^{Q1}$ may be attached to the ortho and para, or meta and para positions of the phenyl; the "phenyl substituted by one or more of $R^{Q1}$" such as 4-fluorophenyl, 4-hydroxyphenyl, 3-hydroxy-4-fluorophenyl, 4-methoxyphenyl, 4-chlorophenyl, 4-cyanophenyl, 3-chlorophenyl, 3-fluorophenyl, 4-cyanophenyl, 2,4-difluorophenyl, 2,4-dichlorophenyl, 2-fluoro-4-chlorophenyl, 2-chloro-4-fluoro-5-methoxyphenyl, 3-methoxy-4-fluorophenyl, 4-methoxy-3-fluorophenyl, 2-methoxy-4-fluorophenyl, 2-chloro-4-fluorophenyl, or 2-fluoro-4-cyanophenyl), 3-6 membered cyclohydrocarbyl-$(CH_2)_{nQ}$- substituted by one or more (for example, 2, 3 or 4) of $R^{Q2}$ (when more than one $R^{Q2}$ exist, the $R^{Q2}$(s) may be the same or different; the 3-6 membered cyclohydrocarbyl is for example 3-6 membered cycloalkyl or cyclohexyl group; the "3-6 membered cyclohydrocarbyl-$(CH_2)_{nQ}$- substituted by one or more of $R^{Q2}$", is for example cyclohexyl), 3-6 membered heterocyclohydrocarbyl-$(CH_2)_{mQ}$- substituted by one or more (for example, 2, 3 or 4) of $R^{Q3}$ (when more than one $R^{Q3}$ exist, the $R^{Q3}$(s) may be the same or different; the heteroatom in the heterocyclohydrocarbyl is N and/or O, the number of which is 1-3; the 3-6 membered heterocyclohydrocarbyl is for example 5-6 membered heteroaryl or 3-6 membered heterocycloalkyl; the 3-6 membered heterocycloalkyl is for example 2-pyranyl, 3-pyranyl or 2-tetrahydrofuranyl), or, 5-6 membered heteroaryl substituted by one or more (for example, 2, 3 or 4) of $R^{Q4}$ (the heteroatom in the heteroaryl is N and/or O, the number of which

is 1-3; the "5-6 membered heteroaryl" is for example pyridyl or pyrimidinyl; the pyridyl is for example pyridin-2-yl or pyridin-4-yl; the "5-6 membered heteroaryl substituted by one or more of $R^{Q4}$" is for example pyridyl-2-yl, or 5-chloro-pyridin-2-yl);

each of $n_Q$ and $m_Q$ is independently 0, 1 or 2;

each of the all $R^{Q1}$, $R^{Q2}$, $R^{Q3}$ and $R^{Q4}$ is independently -H, -OH, -CN, halogen (for example fluoro or chloro), halogenated or unsubstituted $C_1$-$C_5$ alkyl (the "$C_1$-$C_5$ alkyl" is for example $C_1$-$C_3$ alkyl, or for another example, methyl, ethyl, n-propyl or isopropyl; the number of the "halogen" is one or more (for example 2, 3 or 4), when more than one halogen exist, the halogen(s) may be the same or different; the "halogen" is independently fluorine, chlorine or bromine), $C_1$-$C_5$ alkoxyl (for example, $C_1$-$C_3$ alkoxyl, or for another example, methoxy, ethoxy, n-propoxy or isopropoxy, also for example methoxy) or $C_3$-$C_7$ cycloalkyl (for example cyclopropyl);

the "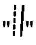" in

"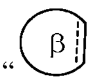"

refers to the bond β ring and the 7-membered ring fused by is a single or double bond;

β ring is 5-6 membered aromatic ring (for example benzene ring), or, "5-6 membered heteroaromatic ring containing 1-3 of heteroatom selected from the group consisting of N, O and S" (for example pyridine ring, pyridazine ring, pyrrole ring, pyrazole ring, furan ring or thiophene ring; the nitrogen atom in the pyridine ring may be located at ortho, meta or para position of the Y, or may be located at ortho position of the Y);

$n^\beta$ is 0, 1, 2, 3 or 4;

each of the all $R^2$ is independently halogenated or unsubstituted $C_1$-$C_5$ alkyl (the "$C_1$-$C_5$ alkyl" is for example "$C_1$-$C_3$ alkyl", or for example methyl, ethyl, n-propyl or isopropyl; the number of the "halogen" is one or more (for example, 2, 3 or 4), when more than one halogen exist, the halogens may be the same or different; each of which is "independently fluorine, chlorine or bromine"), $C_3$-$C_7$ cycloalkyl (e.g. cyclopropyl), hydroxyl, halogen (e.g., fluorine or chlorine), $C_1$-$C_5$ alkoxyl (e.g., $C_1$-$C_3$ alkoxyl, for another example methoxy), $-N(R^{2a})(R^{2b})$ (for example $-NH_2$), $-NHC(R^{2C})H_2$, $-(CH_2)n^{\beta 2}N(R^{2k})S(=O)_2(R^{2d})$ (for example $-N(R^{2k})S(=O)_2(R^{2d})$, for another example

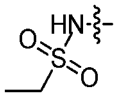 , 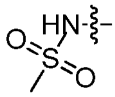 or 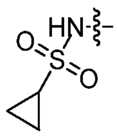

), $-(CH_2)n^{\beta 2}N(R^{2k})S(=O)_2NH(R^{2e})$ (e.g. $-N(R^{2k})S(=O)_2NH(R^{2e})$, for another example $-NHS(=O)_2NH(R^{2e})$, also for example

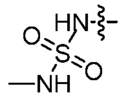

), $-(CH_2)n^{\beta 2}-S(=O)_2N(R^{2f})(R^{2g})$ (for example $-S(=O)_2N(R^{2f})(R^{2g})$ or $-CH_2-S(=O)_2N(R^{2f})(R^{2g})$. The $-S(=O)_2N(R^{2f})(R^{2g})$ is for example $-S(=O)_2N(R^{2g})H$, for another example

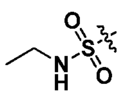.

The $CH_2-S(=O)_2N(R^{2f})(R^{2g})$ is for example

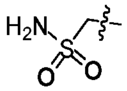 , 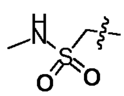 , or 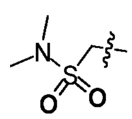 ),

$-(CR^{2x}R^{2y})-S(=O)_2N(R^{2f})(R^{2g})$ (e.g.,

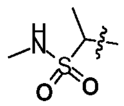 or 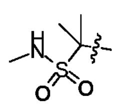

), $-(CH_2)n^{\beta2}-S(=O)(R^{2i})$ (e.g.,

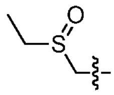

or $-S(=O)(R^{2i})$), $-(CH_2)n^{\beta2}NHC(=O)NH(R^{2e})$ (for example $-NHC(=O)NH(R^{2e})$, for another example

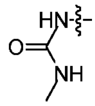

), $-(CH_2)n^{\beta2}NHC(=O)(R^{2e})$ (for example $-NHC(=O)(R^{2e})$, or for another example actamido), $-(CH_2)n^{\beta2}OC(=O)NH(R^{2e})$, $-(CH_2)n^{\beta2}NHC(=O)O(R^{2e})$, $-(CH_2)n^{\beta2}-C(=O)NH(R^{2e})$, $-S(=O)_2(R^{2h})$, $-C_1-C_6$ alkyl-$S(=O)_2(R^{2i})$ (the $C_1-C_6$ alkyl is for example $C_1-C_3$ alkyl, or for another example, methyl, ethyl, n-propyl or isopropyl, also for example methyl; the "$C_1-C_6$ alkyl-$S(=O)_2(R^{2i})$" is for example $-(CH_2)n^{\beta2}-S\ (=O)_2(R^{2i})$, $n^{\beta2}$ is 1, 2, 3, or 4, or for another example $-CH_2-S(=O)_2(R^{2i})$, also for example

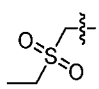, 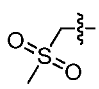, 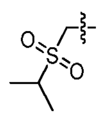, 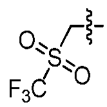

or

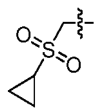

), or, "a 5-6 membered heteroaryl substituted by one or more (e.g., 2, 3 or 4) of $R^{2j}$" (the "5-6 membered heteroaryl" is for example 5-6 membered heteroaryl containing 1-3 of heteroatom(s) selected from the group consisting of N, O and S);

(each $R^2$ may be independently located at ortho, meta or para position of the Y; for example, when $n^{\beta2}$ is 1, and the $\beta$ ring is a six-membered ring, $R^2$ may be located at ortho, para or meta position of the Y; for another example, when $n^{\beta2}$ is 2 and the $\beta$ ring is a six-membered ring, two $R^2$(s) may be located at meta and para, ortho and para, or, ortho and meta position)

each of the all $n^{\beta2}$ is independently 0, 1, 2, 3 or 4;

wherein, each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently -H, $C_1-C_6$ alkyl (for example $C_1-C_3$ alkyl, or for another example methyl, ethyl, n-propyl or isopropyl; also for example methyl, ethyl or isopropyl), $C_1-C_3$ haloalkyl (the number of the halogen may be one or more (e.g., 2, 3 or 4); when more than one halogen exist, the halogens may be the same or different; the "$C_1-C_3$ haloalkyl" is for example trifluoromethyl), 3-7 membered cycloalkyl (e.g., cyclopropyl, cyclobutyl, cyclopentyl or cyclohexyl, for another example cyclopropyl), $-N(CH_3)_2$, or, 5-6 membered nitrogen-containing heteroaryl (e.g., pyridinyl, pyrazinyl, pyridazinyl, pyrimidinyl, pyrrolyl, pyrazolyl or imidazolyl); each $R^{2j}$ is independently -H, $C_1-C_3$ alkyl, or halogen; each of $R^{2x}$ and $R^{2y}$ is independently $C_1-C_6$ alkyl (for example $C_1-C_3$ alkyl, for another example methyl, ethyl, n-propyl or isopropyl, also for example methyl);

"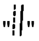" in

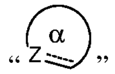

refers to the bond α ring and 7-membered ring fused by is a single or double bond;
Z is

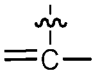 or 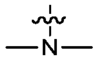;

α ring is a 5-10 membered heteroaromatic ring substituted by one or more (for example, 2, 3 or 4) of $R^{\alpha}$; (the "5-10 membered heteroaromatic ring" is for example "5-10 membered heteroaromatic ring containing 1-6 heteroatom(s) selected from the group consisting of N, O and S. The "5-10 membered heteroaromatic ring" may be a 5-6 membered heteroaromatic ring, or a fused ring formed by two 5-6 membered heteroaromatic rings. The "5-6 membered heteroaromatic ring" may be "5-6 membered heteroaromatic ring containing 1-4 heteroatom(s) selected from the group consisting of N, O and S, which may also be pyridine, pyridazine, pyrimidine, pyrazine, furan, thiophene, pyrrole, pyrazole, imidazole, oxazole, thiazole, pyrimidinone, oxadiazole, pyridone or triazole, or also may be

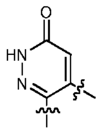, 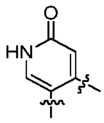, 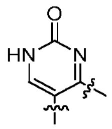, 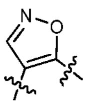, 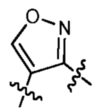, 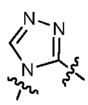,

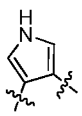, 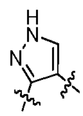 or 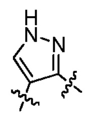.

The "fused ring formed by two 5-6 membered heteroaromatic rings" may be "a fused ring formed by two 5-6 membered heteroaromatic rings containing 1-6 heteroatom(s) selected from the group consisting of N, O and S", also may be a fused ring formed by any two heterocyclic rings selected from the group consisting of pyridine, pyridazine, pyrimidine, pyrazine, furan, thiophene, pyrrole, pyrazole, imidazole, oxazole, isoxazole, thiazole, pyrimidinone, oxadiazole, pyridone and triazole, also may be

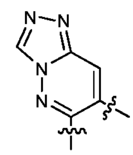, 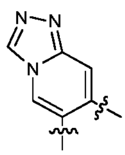, 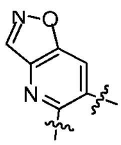

or

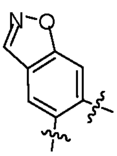.

The "5-10 membered heteroaromatic ring substituted by one or more of $R^{\alpha}$" may be

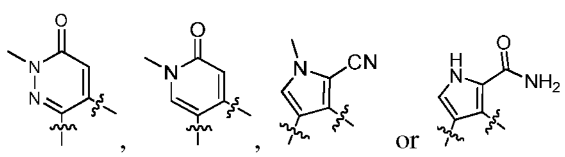

);
each of the all $R^{\alpha}$ is independently -H, -CN, $C_1$-$C_5$ alkyl (for example $C_1$-$C_3$ alkyl, or for another example methyl, ethyl, n-propyl or isopropyl; also for example methyl), $C_3$-$C_7$ cycloalkyl (for example cycloproyl), $C_1$-$C_5$ alkoxyl (for example $C_1$-$C_3$ alkoxyl; or for another example methoxy, ethoxy, n-propoxy or isopropoxy), $C_1$-$C_5$ haloalkyl, halogen (for example fluoro or chloro), -C(=O)N($R^{\alpha1}$)($R^{\alpha2}$) (for example formamyl), or -N($R^{\alpha1}$)($R^{\alpha2}$); (each of $R^{\alpha}$ is independently located at ortho, meta or para position of Z; when the α ring contains -NH-, the $R^{\alpha}$ may bonded to the -NH-) wherein, each of the all $R^{\alpha1}$, $R^{\alpha2}$, $R^{\alpha4}$ and $R^{\alpha4}$ is independently -H, $C_1$-$C_5$ alkyl (for example $C_1$-$C_3$ alkyl, the $C_1$-$C_3$ alkyl is for example methyl, ethyl, n-propyl or isopropyl), or $C_1$-$C_5$ haloalkyl (the number of the halogen may be one or more (for example 2, 3 or 4); when more than one halogen exist, the halogens may be the same or different; the "$C_1$-$C_5$ haloalkyl" is for example trifluoromethyl).

In one embodiment, the definition of each group in the compound **III-0** is defined as follows (undefined definition as described above):
The compound **III-0** may be

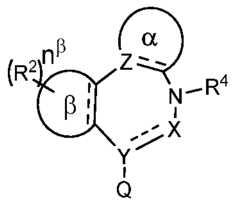
**III-1** or 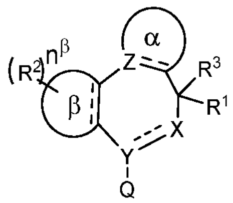
**III-2** .

In one embodiment, the definition of each group in the compound **III-0** is defined as follows (undefined definition as described above):
The compound **III-0** may be

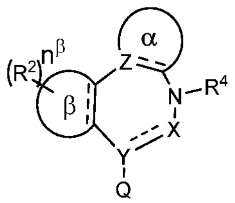
**III-1** or 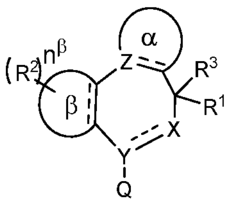
**III-2** ;

when $R^3$ is H, the

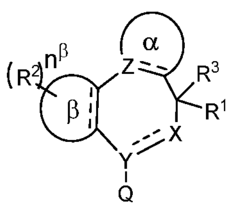
**III-2**

may be

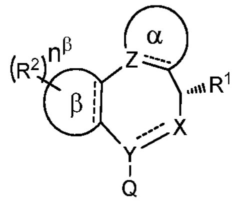

**III-2-1**

In one embodiment, the definition of each group in the compound **III-0** is defined as follows (undefined definition as described above):
$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$.

In one embodiment, the definition of each group in the compound **III-0** is defined as follows (undefined definition as described above):
All of the $R^{R1}$ and $R^{R2}$ are each independently -H, 4-hydroxyphenyl or $C_1$-$C_5$ alkyl, or may be -H or a $C_1$-$C_5$ alkyl.

In one embodiment, the definition of each group in the compound **III-0** is defined as follows (undefined definition as described above):

$R^1$ and $R^4$ are each independently -H, $C_1$-$C_5$ alky, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$;
All of the $R^{R1}$ and $R^{R2}$ are each independently -H or $C_1$-$C_5$ alkyl.

In one embodiment, the definition of each group in the compound **III-0** is defined as follows (undefined definition as described above):
$n_0$, $n_1$, $n_2$, $n_3$, $n_4$, $n_5$, $n_6$, $n_7$ and $m_1$ are each independently 0 or 1.

In one embodiment, the definition of each group in the compound **III-0** is defined as follows (undefined definition as described above):
The

“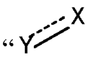”

between X and Y is a single bond.

In one embodiment, the definition of each group in the compound **III-0** is defined as follows (undefined definition as described above):
The

“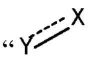”

between X and Y is a single bond; X is $-CH_2-$, -C(=O)- or -NH-.

In one embodiment, the definition of each group in the compound **III-0** is defined as follows (undefined definition as described above):
The

“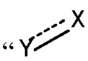”

between X and Y is a double bond.

In one embodiment, the definition of each group in the compound **III-0** is defined as follows (undefined definition as described above):
The

“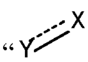”

between X and Y is a double bond; X is =N-.

In one embodiment, the definition of each group in the compound **III-0** is defined as follows (undefined definition as described above):

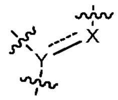

may be

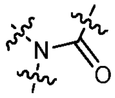, 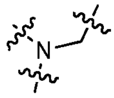, 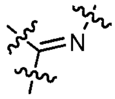 or 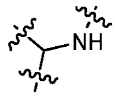.

In one embodiment, the definition of each group in the compound **III-0** is defined as follows (undefined definition as described above):
Q is phenyl substituted by one or more of $R^{Q1}$, a 3-6 membered cyclohydrocarbyl substituted by one or more of $R^{Q2}$ or a 3-6 membered heterocyclohydrocarbyl substituted by one or more of $R^{Q3}$, or also may be a phenyl substituted by one or more (for example, 2, 3 or 4) of $R^{Q1}$.

In one embodiment, the definition of each group in the compound **III-0** is defined as follows (undefined definition as described above):
$R^{Q1}$, $R^{Q2}$, $R^{Q3}$ and $R^{Q4}$ are each independently -H, -OH, -CN, halogen, halogenated or unsubstituted $C_1$-$C_5$ alkyl, $C_3$-$C_7$ cycloalkyl, or $C_1$-$C_5$ alkoxyl, or also may be -H, -OH, -CN, halogen or $C_1$-$C_5$ alkoxyl.

In one embodiment, the definition of each group in the compound **III-0** is defined as follows (undefined definition as described above):

Q is phenyl substituted by one or more (e.g., 2, 3 or 4) of $R^{Q1}$, a 3-6 membered cyclohrdrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$, or may be phenyl substituted by one or more (for example, 2, 3 or 4) of $R^{Q1}$;
All of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, $C_1$-$C_5$ alkoxyl, or also may be -H, -OH, -CN, halogen or $C_1$-$C_5$ alkoxyl.

In one embodiment, the definition of each group in the compound **III-0** is defined as follows (undefined definition as described above):
The "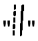" of

“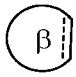”

β ring and 7-membered ring fused by is a double bond.

In one embodiment, the definition of each group in the compound **III-0** is defined as follows (undefined definition as described above):
β ring is benzene.

In one embodiment, the definition of each group in the compound **III-0** is defined as follows (undefined definition as described above):
$n^{\beta}$ is 0, 1 or 2, or may be 1 or 2, or may be 0. When $n^{\beta}$ is 1 or 2, at least one of $R^2$ is located at para or meta (or para) position of Y.

In one embodiment, the definition of each group in the compound **III-0** is defined as follows (undefined definition as described above):

All of the $R^2$ are each independently hydroxyl, halogen, halogenated or unsubstituted $C_1$-$C_5$ alkyl, $C_3$-$C_7$ cycloalkyl, $C_1$-$C_5$ alkoxyl, $-N(R^{2a})(R^{2b})$, $-NHC(R^{2c})H_2$, $-(CH_2)n^{\beta2}N(R^{2k})S(=O)_2(R^{2d})$, $-(CH_2)n^{\beta2}N(R^{2k})S(=O)_2NH(R^{2e})$, $-(CH_2)n^{\beta2}-S(=O)_2N(R^{2f})(R^{2g})$, $-(CR^{2x}R^{2y})-S(=O)_2N(R^{2f})(R^{2g})$, $-(CH_2)n^{\beta2}-S(=O)(R^{2i})$, $-(CH_2)n^{\beta2}NHC(=O)NH(R^{2e})$, $-(CH_2)n^{\beta2}NHC(=O)(R^{2e})$, or, $-C_1$-$C_6$ alkyl-$S(=O)_2(R^{2i})$,

or also may be hydroxyl, halogen, $C_1$-$C_5$ alkoxyl, -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -(C$R^{2x}R^{2y}$)-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$),

or also may be hydroxyl, halogen, $C_1$-$C_5$ alkyoxyl, -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -($CH_2$)$n^{\beta2}$-S(=O)($R^{2i}$), ($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$).

In one embodiment, the definition of each group in the compound **III-0** is defined as follows (undefined definition as described above):

When $n^\beta$ is 1, $R^2$ may be located at the para or meta (or para) position of Y, it may be -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -(C$R^{2x}R^{2y}$)-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$),

Also may be -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$).

In one embodiment, the definition of each group in the compound **III-0** is defined as follows (undefined definition as described above):
When $n^\beta$ is 2, one of $R^2$ may be located at the para position of Y, the other $R^2$ may be located at the meta position of Y.

In one embodiment, the definition of each group in the compound **III-0** is defined as follows (undefined definition as described above):

When $n^\beta$ is 2, one of $R^2$ is located at the para position of the Y, it may be -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -(C$R^{2x}R^{2y}$)-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$), or also may be -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl-S(=O)$_2$($R^{2i}$);

The other $R^2$ is located at the meta position of the Y, it may be hydroxyl, halogenated or unsubstituted $C_1$-$C_5$ alkyl, $C_3$-$C_7$ cycloalkyl, halogen, $C_1$-$C_5$ alkoxyl, or also may be hydroxyl, halogen or $C_1$-$C_5$ alkoxyl.

In one embodiment, the definition of each group in the compound **III-0** is defined as follows (undefined definition as described above):
all $n^{\beta2}$ are each independently 0, 1 or 2, or may each independently be 0 or 1.

In one embodiment, the definition of each group in the compound **III-0** is defined as follows (undefined definition as described above):
$R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_6$ alkyl, $C_1$-$C_3$ haloalkyl, or 3-7 membered cycloalkyl.

In one embodiment, the definition of each group in the compound **III-0** is defined as follows (undefined definition as described above):

The "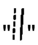" of

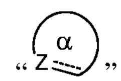

$\alpha$ ring and 7-membered ring fused by is a double bond.

In one embodiment, the definition of each group in the compound **III-0** is defined as follows (undefined definition as described above):
Z is

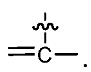

In one embodiment, the definition of each group in the compound **III-0** is defined as follows (undefined definition as described above):

All of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha1}$)($R^{\alpha2}$), also may be -H, -CN, $C_1$-$C_5$ alkyl, or -C(=O)N($R^{\alpha1}$)($R^{\alpha2}$), even may be -H or $C_1$-$C_5$ alkyl.

In one embodiment, the definition of each group in the compound **III-0** is defined as follows (undefined definition as described above):

α ring is

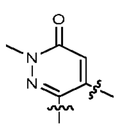 , 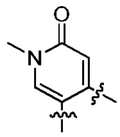 , 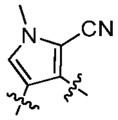 or 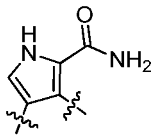 .

In one embodiment, the definition of each group in the compound **III-0** is defined as follows (undefined definition as described above):

The compound **III-0** may be

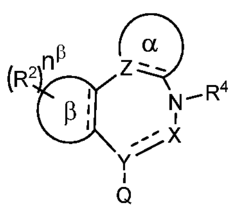

**III-1**

or

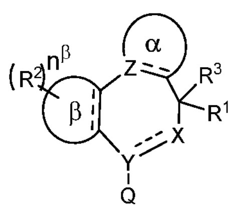

**III-2**

(when $R^3$ is H, the

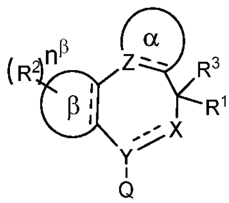

**III-2**

may be

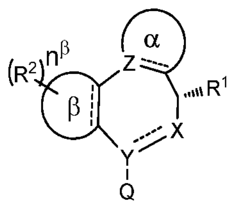

**III-2-1**

); $R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or -$(CH_2)n_0$C(=O)N($R^{R1}$)($R^{R2}$); $R^3$ is -H;

each $R^{R15}$ is independently $C_1$-$C_5$ alkoxyl(for example methoxyl), -COOH or -OH;

All of the $R^{R1}$ and $R^{R2}$ are each independently -H, 4-hydroxyphenyl or $C_1$-$C_5$ alkyl;

$N_0$ is 0 or 1;

or, $R_1$, $R_3$ and the carbon atom they attached to form together a $C_3$-$C_7$ cycloalkyl(for example cyclopropyl);

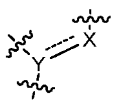

may be

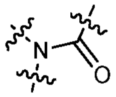 , 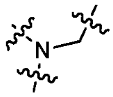 , 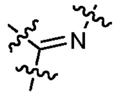 or 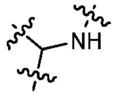 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;

All of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, $C_1$-$C_5$ alkoxyl;

The "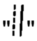" in

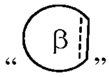

β ring and 7-membered ring fused by is a double bond;

β ring is benzene ring;

$n^{\beta}$ is 0, 1 or 2, or may be 1 or 2, or may be 0. When $n^{\beta}$ is 1 or 2, at least one of $R^2$ is located at the para or meta (or para) position of Y;

all of the $R^2$ are each independently hydroxyl, halogen, halogenated or unsubstituted $C_1$-$C_5$ alkyl, $C_3$-$C_7$ cycloalkyl, $C_1$-$C_5$ alkoxyl, $-N(R^{2a})(R^{2b})$, $-NHC(R^{2c})H_2$, $-(CH_2)n^{\beta2}N(R^{2k})S(=O)_2(R^{2d})$, $-(CH_2)n^{\beta2}N(R^{2k})S(=O)_2NH(R^{2e})$, $-(CH_2)n^{\beta2}$-$S(=O)_2N(R^{2f})(R^{2g})$, $-(CR^{2x}R^{2y})$-$S(=O)_2N(R^{2f})(R^{2g})$, $-(CH_2)n^{\beta2}$-$S(=O)(R^{2i})$, $-(CH_2)n^{\beta2}$ $NHC(=O)NH(R^{2e})$, $-(CH_2)n^{\beta2}NHC(=O)(R^{2e})$, or, $-C_1$-$C_6$ alkyl-$S(=O)_2(R^{2i})$;

(In one case, when $n^{\beta}$ is 1, $R^2$ may be located at the para or meta (or para) position of Y, it may be $-N(R^{2a})(R^{2b})$, $-NHC(R^{2c})H_2$, $-(CH_2)n^{\beta2}N(R^{2k})S(=O)_2(R^{2d})$, $-(CH_2)n^{\beta2}N(R^{2k})S(=O)_2NH(R^{2e})$, $-(CH_2)n^{\beta2}$-$S(=O)_2N(R^{2f})(R^{2g})$, $-(CR^{2x}R^{2y})$-$S(=O)_2N(R^{2f})(R^{2g})$, $-(CH_2)n^{\beta2}$-$S(=O)(R^{2i})$, $-(CH_2)n^{\beta2}NHC(=O)NH(R^{2e})$, $-(CH_2)n^{\beta2}NHC(=O)(R^{2e})$, or, $-C_1$-$C_6$ alkyl-$S(=O)_2(R^{2i})$; in one case,when $n^{\beta}$ is 2, one of $R^2$ may be located at the para position of Y, the other $R^2$ may be located at the meta position of the Y; in one case, when $n^{\beta}$ is 2, one of $R^2$ is located at the para position of Y, it may be $-N(R^{2a})(R^{2b})$, $-NHC(R^{2c})H_2$, $-(CH_2)n^{\beta2}N(R^{2k})S(=O)_2(R^{2d})$, $-(CH_2)n^{\beta2}N(R^{2k})S(=O)_2NH(R^{2e})$, $-(CH_2)n^{\beta2}$-$S(=O)_2N(R^{2f})(R^{2g})$, $-(CR^{2x}R^{2y})$-$S(=O)_2N(R^{2f})(R^{2g})$, $-(CH_2)n^{\beta2}$-$S(=O)(R^{2i})$, $-(CH_2)n^{\beta2}NHC(=O)NH(R^{2e})$, $-(CH_2)n^{\beta2}NHC(=O)(R^{2e})$, or, $-C_1$-$C_6$ alkyl-$S(=O)_2(R^{2i})$; the other $R^2$ is located at the meta position of Y, it may be hydroxyl, halogen, halogenated or unsubstituted $C_1$-$C_5$ alkyl, $C_3$-$C_7$ cycloalkyl, $C_1$-$C_5$ alkoxyl)

All of the $n^{\beta2}$ may each independently be 0, 1 or 2, or may independently be 0 or 1;

$R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_6$ alkyl, $C_1$-$C_3$ haloalkyl, or 3-7 membered cycloalkyl;

The "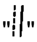" in

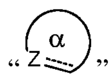

α ring and 7-membered ring fused by is a double bond;

Z is

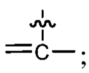

α ring is 5-10 membered heteroaryl substituted by one or more (e.g., 2, 3 or 4) of $R^{\alpha}$ (for example,

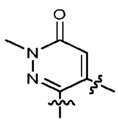, 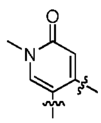, 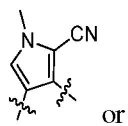 or 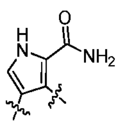

);

All of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or $-C(=O)N(R^{\alpha1})(R^{\alpha2})$ (also may be -H, or $C_1$-$C_5$ alkyl), all of the $R^{\alpha1}$ and $R^{\alpha2}$ are each independently -H, $C_1$-$C_5$ alkyl.

In one embodiment, the definition of each group in the compound **III-0** is defined as follows (undefined definition as described above):

The compound **III-0** may be

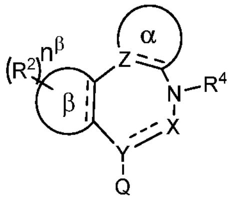

**III-1** or 

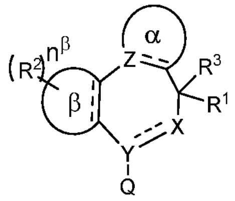

**III-2**

(when $R^3$ is H, the

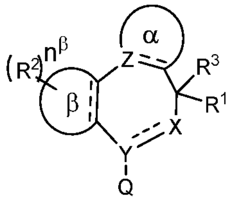

**III-2** may be 

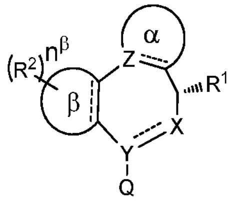

**III-2-1**

);

$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H;

each $R^{R15}$ is independently $C_1$-$C_5$ alkoxyl(for example methoxyl), -COOH or -OH;

All of the $R^{R1}$ and $R^{R2}$ are each independently -H, 4-hydroxyphenyl or $C_1$-$C_5$ alkyl;

$N_0$ is 0 or 1 (e.g., 1);

or, $R_1$, $R_3$ and the carbon atom they attached to form together a $C_3$-$C_7$ cycloalkyl(for example cyclopropyl);

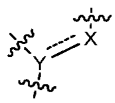

may be

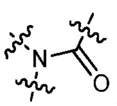, 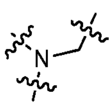, 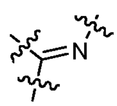 or 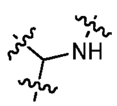 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;

all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;

The "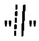" in

“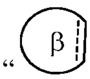”

β ring and 7-membered ring fused by is a double bond;

β ring may be benzene ring or "a 5-6 membered heteroaromatic ring containing 1-3 of heteroatom(s) selected from the group consisting of N, O and S"; or may be benzene ring;

$n^\beta$ is 1, $R^2$ is $-C_1-C_6$ alkyl-$S(=O)_2(R^{2i})$; or, $n^\beta$ is 2, one of $R^2$ is $-C_1-C_6$ alkyl-$S(=O)_2(R^{2i})$, the other $R^2$ is hydroxyl, halogen, halogenated or unsubstituted $C_1-C_5$ alkyl, $C_3-C_7$ cycloalkyl, or $C_1-C_5$ alkoxyl; (the $-C_1-C_6$ alkyl-$S(=O)_2(R^{2i})$ may be located at the para or meta position of Y, or located at the para position; the "hydroxyl, halogen, halogenated or unsubstituted $C_1-C_5$ alkyl, $C_3-C_7$ cycloalkyl, or $C_1-C_5$ alkoxyl" may be located at the meta position of Y);

$R^{2i}$ is $C_1-C_6$ alkyl, $C_1-C_3$ haloalkyl or 3-7 membered cycloalkyl;

The "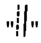" in

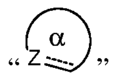

α ring and 7-membered ring fused by is a double bond;

Z is

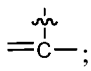

α ring is 5-10 membered heteroaromatic ring substituted by one or more (e.g., 2, 3 or 4) of $R^\alpha$ (for example,

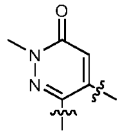 , 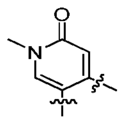 , 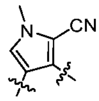 or 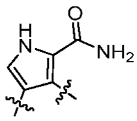

);

all of the $R^\alpha$ are each independently -H, -CN, $C_1-C_5$ alkyl, $C_1-C_5$ alkoxyl, $C_1-C_5$ haloalkyl, $C_3-C_7$ cycloalkyl, or $-C(=O)N(R^{\alpha1})(R^{\alpha2})$ (also may be -H, or $C_1-C_5$ alkyl), all of the $R^{\alpha1}$ and $R^{\alpha2}$ are each independently -H or $C_1-C_5$ alkyl.

The following is the range of the sulfoxide compound to be deleted:

In one embodiment, the definition of each group in the compound **III-0** is defined as follows (undefined definition as described above):

The compound **III-0** may be

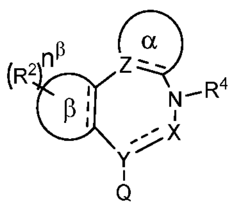

**III-1** or

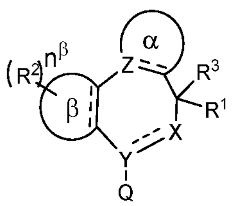

**III-2**

(when $R^3$ is H, the

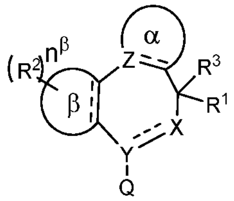

**III-2**

may be

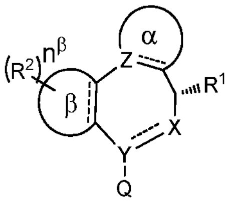

**III-2-1** );

$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H;
each $R^{R15}$ is independently $C_1$-$C_5$ alkoxyl(for example methoxyl), -COOH or -OH;
all of the $R^{R1}$ and $R^{R2}$ are each independently -H, 4-hydroxyphenyl or $C_1$-$C_5$ alkyl;
$N_0$ is 0 or 1 (or e.g., 1);
or, $R_1$, $R_3$ and the carbon atom they attached to form together a $C_3$-$C_7$ cycloalkyl(for example cyclopropyl);

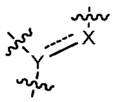

may be

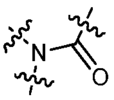 , 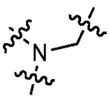 , 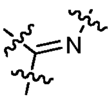 or 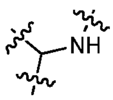 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) $R^{Q3}$;
all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;
the "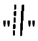" in

“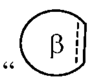”

β ring and 7-membered ring fused by is a double bond;
β ring may be benzene ring or "a 5-6 membered heteroaromatic ring containing 1-3 of heteroatom(s) selected from the group consisting of N,O and S"; or may be benzene ring;
$n^{\beta}$ is 1, $R^2$ is $-(CH_2)n^{\beta 2}-S(=O)(R^{2i})$; or, $n^{\beta}$ is 2, one of $R^2$ is $-(CH_2)n^{\beta 2}-S(=O)(R^{2i})$, the other $R^2$ is hydroxyl halogen halogenated or unsubstituted $C_1$-$C_5$ alkyl, $C_3$-$C_7$ cycloalkyl or $C_1$-$C_5$ alkoxyl; (the $-(CH_2)n^{\beta 2}-S(=O)(R^{2i})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);

$n^{\beta 2}$ may be 0, 1, or 2 (or may be independently 0 or 1, or may be 0);
$R^{2i}$ is $C_1$-$C_6$ alkyl, $C_1$-$C_3$ haloalkyl or 3-7 membered cycloalkyl;
the "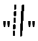" in

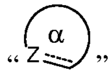

α ring and 7-membered ring fused by is a double bond;
Z is

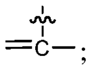

α ring is a 5-10 membered heteroaromatic ring substituted by one or more (e.g., 2, 3 or 4) of $R^{\alpha}$ (for example,

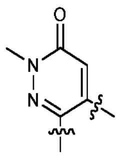 , 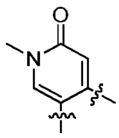 , 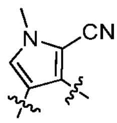 or 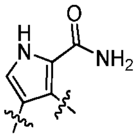 );

all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha 1}$)($R^{\alpha 2}$) (also may be -H, or $C_1$-$C_5$ alkyl), all of the $R^{\alpha 1}$ and $R^{\alpha 2}$ are each independently -H or $C_1$-$C_5$ alkyl.

The following is the range of the sulfonamides compound to be deleted:
In one embodiment, the definition of each group in the compound **III-0** is defined as follows (undefined definition as described above):

The compound **III-0** may be

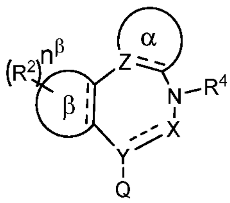

**III-1** or

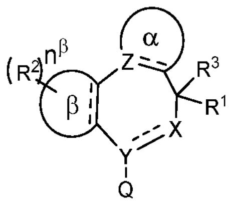

**III-2**

(when $R^3$ is H, the

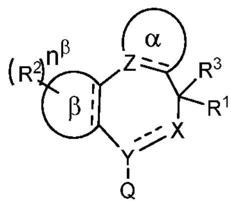

**III-2**

may be

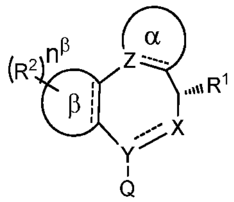

**III-2-1** );

$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H;
each $R^{R15}$ is independently $C_1$-$C_5$ alkoxyl(for example methoxyl), -COOH or -OH;
all of the $R^{R1}$ and $R^{R2}$ are each independently -H, 4-hydroxyphenyl or $C_1$-$C_5$ alkyl;
$N_0$ is 0 or 1 (or e.g., 1);
or, $R_1$, $R_3$ and the carbon atom they attached to form together a $C_3$-$C_7$ cycloalkyl(for example cyclopropyl);

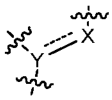

may be

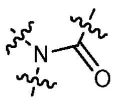 , 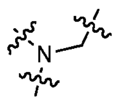 , 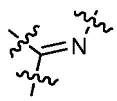 or 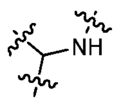 ,

or may be

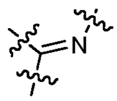 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;
all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;
the "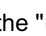" in

“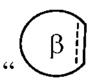”

β ring and 7-membered ring fused by is a double bond;
the β ring is benzene ring;
$n^\beta$ is 1, $R^2$ is $-(CH_2)n^{\beta 2}N(R^{2k})S(=O)_2(R^{2d})$; or, $n^\beta$ is 2, one of $R^2$ is $-(CH_2)n^{\beta 2}N(R^{2k})S(=O)_2(R^{2d})$, the other $R^2$ is hydroxyl, halogen, halogenated or unsubstituted $C_1$-$C_5$ alkyl, $C_3$-$C_7$ cycloalkyl, $C_1$-$C_5$ alkoxyl(the $-(CH_2)n^{\beta 2}N(R^{2k})S(=O)_2(R^{2d})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);
$n^{\beta 2}$ may independently be 0, 1 or 2, or may independently be 0 or 1, or may be 0;
$R^{2d}$ and $R^{2k}$ are each independently -H, $C_1$-$C_6$ alkyl, $C_1$-$C_3$ haloalkyl or 3-7 membered cycloalkyl(or also may be -H, $C_1$-$C_6$ alkyl, or 3-7 membered cycloalkyl);

The "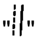" in

“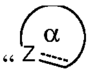”

α ring and 7-membered ring fused by is a double bond;
Z is

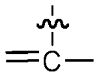;

α ring is a 5-10 membered heteroaromatic ring substituted by one or more (e.g., 2, 3 or 4) of $R^{\alpha}$ (for example,

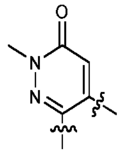, 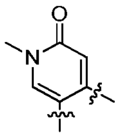, 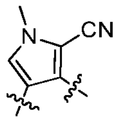 or 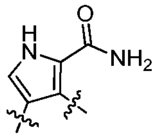);

all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha1}$)($R^{\alpha2}$) (also may be -H, or $C_1$-$C_5$ alkyl), all of the $R^{\alpha1}$ and $R^{\alpha2}$ are each independently -H or $C_1$-$C_5$ alkyl.

The following is the range of the aminosulfonyl compound to be deleted:
In one embodiment, the definition of each group in the compound **III-0** is defined as follows (undefined definition as described above):

The compound **III-0** may be

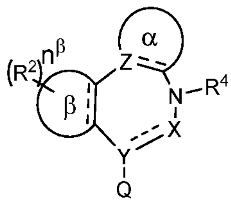

**III-1**

or

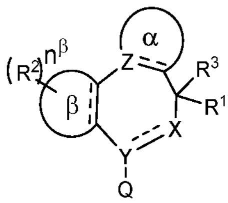

**III-2**

(when $R^3$ is H, the

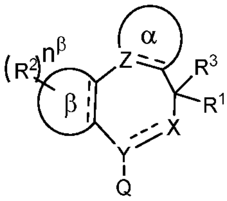

**III-2**

may be

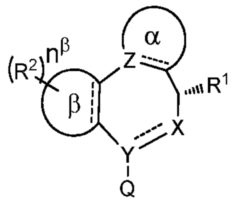

**III-2-1** );

$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H;
$R^{R15}$ is independently $C_1$-$C_5$ alkoxyl(for example methoxyl), -COOH or -OH;
all of the $R^{R1}$ and $R^{R2}$ are each independently -H, 4-hydroxyphenyl or $C_1$-$C_5$ alkyl;
$N_0$ is 0 or 1 (or for example 1);
or, $R^1$, $R^3$ and the carbon atom they attached to form together a $C_3$-$C_7$ cycloalkyl(for example cyclopropyl);

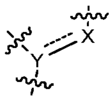

may be

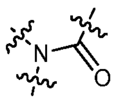 , 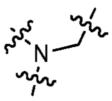 , 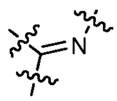 or 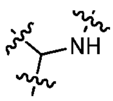 ,

or may be

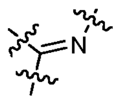 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;
all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;
The "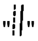" in

"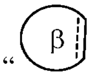"

β ring and 7-membered ring fused by is a double bond;
The β ring is benzene ring;
$n^\beta$ is 1, $R^2$ is $-(CH_2)n^{\beta2}$-$S(=O)_2N(R^{2f})(R^{2g})$ or $-(CR^{2x}R^{2y})$-$S(=O)_2N(R^{2f})(R^{2g})$; or, $n^\beta$ is 2, one of $R^2$ is $-(CH_2)n^{\beta2}$-$S(=O)_2N(R^{2f})(R^{2g})$ or $-(CR^{2x}R^{2y})$-$S(=O)_2N(R^{2f})(R^{2g})$, the other $R^2$ is hydroxyl, halogen, halogenated or unsubstituted $C_1$-$C_5$ alkyl, $C_3$-$C_7$ cycloalkyl, or $C_1$-$C_5$ alkoxyl(the $-(CH_2)n^{\beta2}$-$S(=O)_2N(R^{2f})(R^{2g})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);
$n^{\beta2}$ may independently be 0, 1 or 2 (may be 0 or 1, or may be 0);
$R^{2f}$ and $R^{2g}$ are each independently -H, $C_1$-$C_6$ alkyl, $C_1$-$C_3$ haloalkyl or 3-7 membered cycloalkyl(or also may be -H, $C_1$-$C_6$ alkyl, or 3-7 membered cycloalkyl);

the "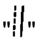" in

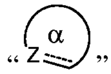

α ring and 7-membered ring fused by is a double bond;
Z is

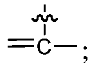

α ring is a 5-10 membered heteroaromatic ring substituted by one or more (e.g., 2, 3 or 4) of $R^{\alpha}$ (for example,

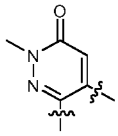, 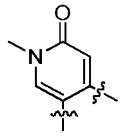, 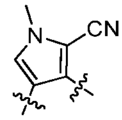 or 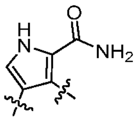);

all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha 1}$)($R^{\alpha 2}$) (also may be -H, or $C_1$-$C_5$ alkyl), all of the $R^{\alpha 1}$ and $R^{\alpha 2}$ are each independently -H or $C_1$-$C_5$ alkyl.

The following is the range of the aminosulfonamido compound to be deleted:

In one embodiment, the definition of each group in the compound **III-0** is defined as follows (undefined definition as described above):

The compound **III-0** may be

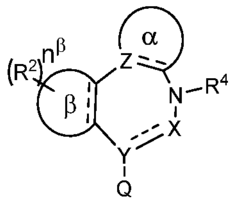

III-1

or

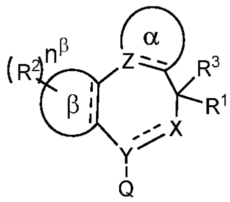

III-2

(when $R^3$ is H, the

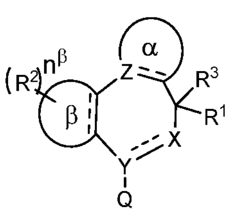

III-2

may be

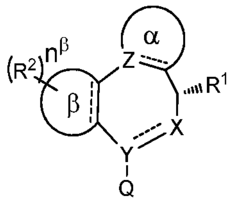

III-2-1 );

$R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H;

$R^{R15}$ is independently $C_1$-$C_5$ alkoxyl(for example methoxyl), -COOH or -OH;

all of the $R^{R1}$ and $R^{R2}$ are each independently -H, 4-hydroxyphenyl or $C_1$-$C_5$ alkyl;

$N_0$ is 0 or 1 (or for example 1);

or, $R^1$, $R^3$ and the carbon atom they attached to form together a $C_3$-$C_7$ cycloalkyl(for example cyclopropyl);

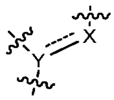

may be

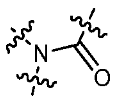 , 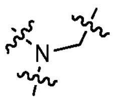 , 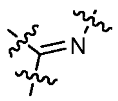 or 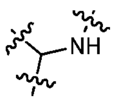 ,

or may be

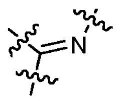 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;

all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;

the "" in

“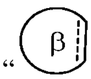”

β ring and 7-membered ring fused by is a double bond;

the β ring is benzene ring;

$n^\beta$ is 1, $R^2$ is $-(CH_2)n^{\beta 2}N(R^{2k})S(=O)_2NH(R^{2e})$; or, $n^\beta$ is 2, one of $R^2$ is $-(CH_2)n^{\beta 2}N(R^{2k})S(=O)_2NH(R^{2e})$, the other $R^2$ is hydroxyl, halogen, halogenated or unsubstituted $C_1$-$C_5$ alkyl, $C_3$-$C_7$ cycloalkyl, or $C_1$-$C_5$ alkoxyl; (the $-(CH_2)n^{\beta 2}N(R^{2k})S(=O)_2NH(R^{2e})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);

$n^{\beta 2}$ may independently be 0, 1 or 2 (may be 0 or 1, or may be 0);

$R^{2e}$ and $R^{2k}$ are each independently -H, $C_1$-$C_6$ alkyl, $C_1$-$C_3$ haloalkyl or 3-7 membered cycloalkyl(or also may be -H, $C_1$-$C_6$ alkyl, or 3-7 membered cycloalkyl);

the "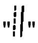" in

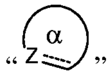

$\alpha$ ring and 7-membered ring fused by is a double bond;
Z is

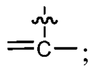

$\alpha$ ring is a 5-10 membered heteroaromatic ring substituted by one or more (e.g., 2, 3 or 4) of $R^{\alpha}$ (for example,

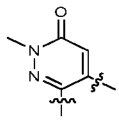, 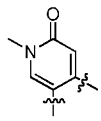, 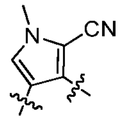 or 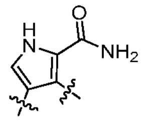 );

all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha1}$)($R^{\alpha2}$) (also may be -H, or $C_1$-$C_5$ alkyl), all of the $R^{\alpha1}$ and $R^{\alpha2}$ are each independently -H, $C_1$-$C_5$ alkyl.

The following is the range of the amido compound to be deleted:

In one embodiment, the definition of each group in the compound **III-0** is defined as follows (undefined definition as described above):

The compound **III-0** may be

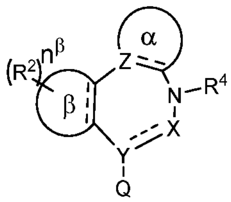

III-1

or

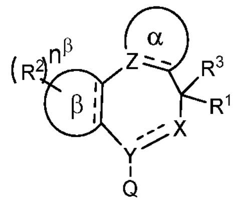

III-2

(when $R^3$ is H, the

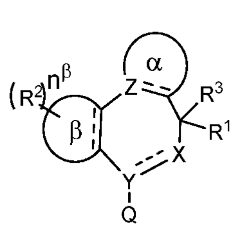

III-2

may be

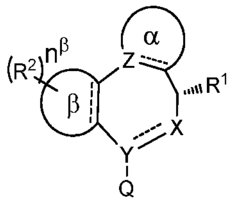

**III-2-1** );

$R^1$ and $R^4$ are each independently-H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or $-(CH_2)n_0C(=O)N(R^{R1})(R^{R2})$; $R^3$ is -H.
each $R^{R15}$ is independently $C_1$-$C_5$ alkoxyl(for example methoxyl), -COOH or -OH;
all of the $R^{R1}$ and $R^{R2}$ are each independently -H, 4-hydroxyphenyl or $C_1$-$C_5$ alkyl;
$N_0$ is 0 or 1 (or for example 1);
or, $R^1$, $R^3$ and the carbon atom they attached to form together a $C_3$-$C_7$ cycloalkyl(for example cyclopropyl);

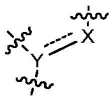

may be

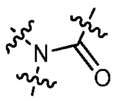 , 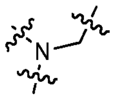 , 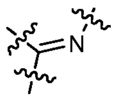 or 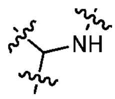 ,

or may be

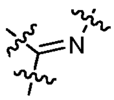 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;
all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;
the "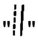" in

“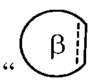”

β ring and 7-membered ring fused by is a double bond;
the β ring is benzene ring;
$n^\beta$ is 1, $R^2$ is $-(CH_2)n^{\beta2}NHC(=O)(R^{2e})$; or, $n^\beta$ is 2, one of $R^2$ is $-(CH_2)n^{\beta2}NHC(=O)(R^{2e})$, the other $R^2$ is hydroxyl, halogen, halogenated or unsubstituted $C_1$-$C_5$ alkyl, $C_3$-$C_7$ cycloalkyl, or $C_1$-$C_5$ alkoxyl; (the $-(CH_2)n^{\beta2}NHC(=O)(R^{2e})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);
$n^{\beta2}$ may be 0, 1 or 2 (may be 0 or 1, or may be 0);
$R^{2e}$ is $C_1$-$C_6$ alkyl, $C_1$-$C_3$ haloalkyl or 3-7 membered cycloalkyl(or also may be $C_1$-$C_6$ alkyl, or 3-7 membered cycloalkyl);

[00234] the "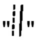" in

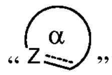

α ring and 7-membered ring fused by is a double bond;
Z is

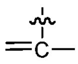;

the α ring is a 5-10 membered heteroaromatic ring substituted by one or more (e.g., 2, 3 or 4) of $R^{\alpha}$ (for example,

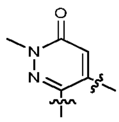, 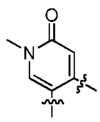, 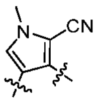 or 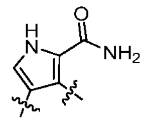 );

all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha 1}$)($R^{\alpha 2}$) (also may be -H, or $C_1$-$C_5$ alkyl), all of the $R^{\alpha 1}$ and $R^{\alpha 2}$ are each independently -H, $C_1$-$C_5$ alkyl.

The following is the range of the ureido compound to be deleted:

In one embodiment, the definition of each group in the compound **III-0** is defined as follows (undefined definition as described above):

the compound **III-0** may be

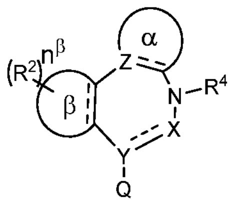

**III-1**

or

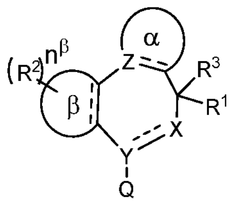

**III-2**

(when $R^3$ is H, the

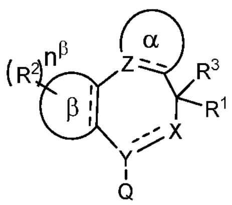

**III-2**

may be

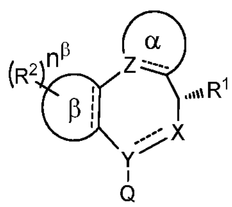

**III-2-1**

); $R^1$ and $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more (for example 2, 3 or 4) of $R^{R15}$, or -$(CH_2)n_0$C(=O)N($R^{R1}$)($R^{R2}$); $R^3$ is -H.
each $R^{R15}$ is independently $C_1$-$C_5$ alkoxyl(for example methoxyl), -COOH or -OH;
all of the $R^{R1}$ and $R^{R2}$ are each independently -H, 4-hydroxyphenyl or $C_1$-$C_5$ alkyl;
$N_0$ is 0 or 1 (or for example 1);
or, $R^1$, $R^3$ and the carbon atom they attached to form together a $C_3$-$C_7$ cycloalkyl(for example cyclopropyl);

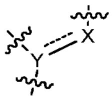

may be

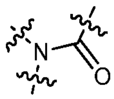 , 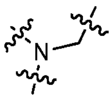 , 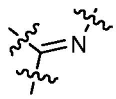 or 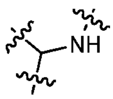 ,

or may be

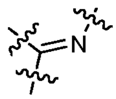 ;

Q is phenyl substituted by one or more (e.g., 2, 3 or 4) of $R^{Q1}$, a 3-6 membered cyclohydrocarbyl substituted by one or more (e.g., 2, 3 or 4) of $R^{Q2}$ or a 3-6 membered heterocyclohydrocarbyl substituted by one or more (e.g., 2, 3 or 4) of $R^{Q3}$;

all of the $R^{Q1}$, $R^{Q2}$ and $R^{Q3}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;

the "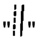" in

“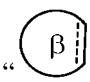”

β ring and 7-membered ring fused by is a double bond;

the β ring is benzene ring or "a 5-6 heteroaromatic ring containing 1-3 heteroatom(s) selected from the group consisting of N, O and S" or may be benzene ring;

$n^{\beta}$ is 1, $R^2$ is $-(CH_2)n^{\beta2}NHC(=O)NH(R^{2e})$; or, $n^{\beta}$ is 2, one of $R^2$ is $-(CH_2)n^{\beta2}NHC(=O)NH(R^{2e})$, the other $R^2$ is hydroxyl, halogen, halogenated or unsubstituted $C_1$-$C_5$ alkyl, $C_3$-$C_7$ cycloalkyl, or $C_1$-$C_5$ alkoxyl; (the $-(CH_2)n^{\beta2}NHC(=O)NH(R^{2e})$ may be located at the meta or para position of Y, or may be located at the para position; the "hydroxyl, halogen, halogenated or unsubstituted $C_1$-$C_5$ alkyl, $C_3$-$C_7$ cycloalkyl, or $C_1$-$C_5$ alkoxyl" may be located at the meta position of Y);

$n^{\beta2}$ may independently be 0, 1 or 2 (or may be 0 or 1, or may be 0);

each $R^{2e}$ is independently $C_1$-$C_6$ alkyl, $C_1$-$C_3$ haloalkyl or 3-7 membered cycloalkyl(or also may be $C_1$-$C_6$ alkyl, or 3-7 membered cycloalkyl);

the "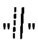" in

“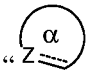”

α ring and 7-membered ring fused by is a double bond;

Z is

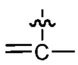 ;

the α ring is a 5-10 membered heteroaromatic ring substituted by one or more (e.g., 2, 3 or 4) of $R^{\alpha}$ (for example,

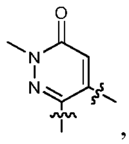, 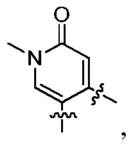, 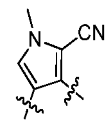 or 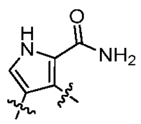

);
All of the $R^{\alpha}$ is each independently -H, -CN, $C_1$-$C_5$ alkyl, $C_1$-$C_5$ alkoxyl, $C_1$-$C_5$ haloalkyl, $C_3$-$C_7$ cycloalkyl, or -C(=O)N($R^{\alpha 1}$)($R^{\alpha 2}$) (also may be -H, or $C_1$-$C_5$ alkyl), all of the $R^{\alpha 1}$ and $R^{\alpha 2}$ are each independently -H, $C_1$-$C_5$ alkyl.

The present invention provides a nitrogeneous macrocyclic compound represented by formula **III-0**, a tautomer thereof, an optical isomer, a hydrate, a solvate thereof, a pharmaceutically acceptable salt thereof (for example formate) or a prodrug thereof:

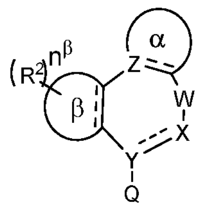

**III-0**

wherein, W is -C($R^1$)($R^3$)- or -N($R^4$)-(e.g.,

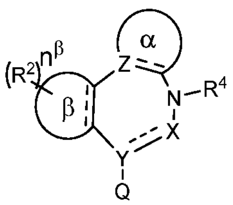

**III-1**

or

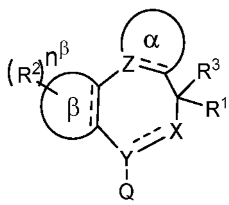

**III-2**

);
$R^1$, $R^3$ and $R^4$ are each independently -H, $C_1$-$C_5$ alkyl(the $C_1$-$C_5$ alkyl is for example methyl, ethyl, n-propyl or isopropyl), $C_3$-$C_7$ cycloalkyl(for example cyclopropyl), $C_1$-$C_5$ alkoxyl(the $C_1$-$C_5$ alkoxyl is for example $C_1$-$C_3$ alkoxyl, the $C_1$-$C_3$ alkoxyl is for example methoxy, ethoxy, n-propoxy or isopropoxy), $C_1$-$C_5$ haloalkyl(the number of halogen may be one or more (such as 2, 3 or 4), when more than one halogen exist, the halogen(s) may be the same or different; the halo groups may be on the same carbon or not; the "$C_1$-$C_5$ haloalkyl" is, for example, "$C_1$-$C_3$ haloalkyl", the "$C_1$-$C_3$ haloalkyl" is for example trifluoromethyl, difluoromethyl, or 1,2-difluoroethyl), $C_1$-$C_5$ alkyl substituted by one or more (e.g., 2, 3 or 4) of $R^{R15}$ (when more than one $R^{R15}$ exist, the $R^{R15}$(s) may be the same or different; the "$C_1$-$C_5$ alkyl" is, for example, methyl or ethyl; the "$C_1$-$C_5$ alkyl substituted by one $R^{R15}$" is, for example, 2-hydroxyethyl, carboxymethyl, or methoxymethyl), halogen (for example fluorine or chlorine), -$(CH_2)n_0$C(=O)N($R^{R1}$)($R^{R2}$), -NH$(CH_2)$n,C(=O)N($R^{R3}$)($R^{R4}$), -$(CH_2)n_2$N($R^{R5}$)($R^{R6}$), -$(CH_2)n_3$OC(=O)N($R^{R7}$)($R^{R8}$), -$(CH_2)n_4$NHC(=O)$R^{R9}$, -$(CH_2)n_5$NHC(=O)O$R^{R10}$, -$(CH_2)n_6$NHS$(=O)_2R^{R11}$ or -$(CH_2)n_7$S$(=O)_2$N($R^{R12}$)($R^{R13}$); (both of the $R^1$ and $R^3$ may not be -H; the $R^1$ and $R^3$ may not be -H at the same time; at least one (for example one) of the $R^1$ and $R^3$ may be -H; both of the $R^1$ and $R^3$ may be -H; when the $R^1$ and $R^3$ are different, the carbon atom they attached to is a chiral carbon atom; the configuration of the chiral carbon atom may be R or S)
each $R^{R15}$ is independently $C_1$-$C_5$ alkoxyl(for example methoxy) or -OH;
all of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently -H, $C_1$-$C_5$ alkyl(the $C_1$-$C_5$ alkyl is for example $C_1$-$C_3$ alkyl, the $C_1$-$C_3$ alkyl is for example methyl, ethyl, n-propyl; or isopropyl), $C_1$-$C_5$ haloalkyl, -$(CH_2)m_1$CN, -C$(CH_3)_2$CN, 3-7 membered cycloalkyl(for example cyclopropyl), or, a $C_6$-$C_{10}$ aryl substituted by one or more (for example 2, 3 or 4) of $R^{R14}$ (when more than one $R^{R14}$ exist, the $R^{R14}$(s) may be the same or different; the "$C_6$-$C_{10}$ aryl" is, for example, phenyl; the $R^{R14}$ may independently be attached to the ortho, meta or para position of the $C_6$-$C_{10}$ aryl; the "$C_6$-$C_{10}$ aryl substituted by one $R^{R14}$" is, for example, 4-hydroxyphenyl); $R^{R14}$ is each independently -H, $C_1$-$C_5$ alkyl(for example $C_1$-$C_3$ alkyl, the $C_1$-$C_3$ alkyl is for example

methyl, ethyl, n-propyl, or isopropyl), $C_1$-$C_5$ alkoxyl, $C_1$-$C_5$ haloalkyl, halogen,-OH, -CN or $NH_2$;
$n_0$, $n_1$, $n_2$, $n_3$, $n_4$, $n_5$, $n_6$, $n_7$ and $m_1$ are each independently to be 0, 1, 2 or 3 (or for another example 0 or 1);
or, $R^1$, $R^3$ and the carbon atom they attached to together form a $C_3$-$C_7$ cycloalkyl(for example cyclopropyl);

“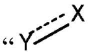”

refers to the bond between X and Y is a single or double bond;
X is $-CH_2-$, =N-, -NH-, -O-, $-S(=O)_2-$ or -C=O-;
Y is

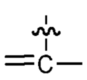, 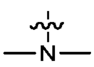 or 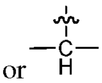;

and at least one of X and Y is a nitrogen atom; (

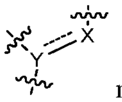 may be 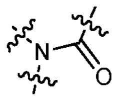,

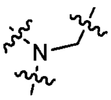, 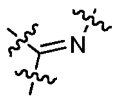 or 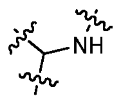

)
Q is a phenyl substituted by one or more (for example 2, 3 or 4) of $R^{Q1}$ (when more than one $R^{Q1}$ exist, the $R^{Q1}$(s) may be the same or different; the $R^{Q1}$ can be independently attached to the ortho, meta or para position of the phenyl; when two $R^{Q1}$(s) are present, the $R^{Q1}$(s) may be attached to the ortho and para, or meta and para positions of the phenyl; the "phenyl substituted by one or more of $R^{Q1}$" such as 4-fluorophenyl, 4-methoxyphenyl, 4-chlorophenyl, 3-chlorophenyl, 3-fluorophenyl, 4-cyanophenyl, 2,4-difluorophenyl, 2,4-dichlorophenyl, 2-fluoro-4-chlorophenyl, 3-methoxyl-4-fluorophenyl, 2-methoxyl-4-fluorophenyl, 2-chloro-4-fluorophenyl, or 2-fluoro-4-cyanophenyl), a 3-6 membered cyclohydrocarbyl-$(CH_2)_{nQ}$-substituted by one or more (for example, 2, 3 or 4) of $R^{Q2}$ (when more than one $R^{Q2}$ exist, the $R^{Q2}$(s) may be the same or different; the 3-6 membered cyclohydrocarbyl is, for example, 3-6 membered cycloalkyl, the 3-6 membered cycloalkyl is, for example, cyclohexyl; the "3-6 membered cyclohydrocarbyl-$(CH_2)_{nQ}$-" substituted by one or more of $R^{Q2}$, such as cyclohexyl), a 3-6 membered heterocyclohydrocarbyl-$(CH_2)_{mQ}$-substituted by one or more (for example, 2, 3 or 4) of $R^{Q3}$ (when more than one $R^{Q3}$ exist, the $R^{Q3}$(s) may be the same or different; the heteroatom in the heterocyclohydrocarbyl is N and/or O, the number of which is 1-3; the 3-6 membered heterocyclohydrocarbyl is, for example, 5-6 membered heteroaryl or 3-6 membered heterocycloalkyl; the 3-6 membered heterocycloalkyl is, for example, 2-pyranyl, 3-pyranyl or 2-tetrahydrofuranyl), or, 5-6 membered heteroaryl substituted by one or more (for example, 2, 3 or 4) of $R^{Q4}$ (the heteroatom in the heteroaryl is N and/or O, the number of which is 1-3; the "5-6 membered heteroaryl" is, for example, pyridyl or pyrimidinyl; the pyridyl is, for example, pyridin-2-yl; the "5-6 membered heteroaryl substituted by one or more of $R^{Q4}$" is for example, pyridyl-2-yl, or 5-chloro-pyridin-2-yl);
$n_Q$ and $m_Q$ are each independently to be 0, 1 or 2;
all of $R^{Q1}$, $R^{Q2}$, $R^{Q3}$ and $R^{Q4}$ are each independently -H, -CN, halogen (for example fluorine or chlorine), $C_1$-$C_5$ alkyl(the "$C_1$-$C_5$" alkyl is, for example, $C_1$-$C_3$ alkyl, the $C_1$-$C_3$ alkyl is for example, methyl, ethyl, n-propyl or isopropyl); $C_1$-$C_5$ alkoxyl(the $C_1$-$C_5$ alkoxyl is for example methoxyl, ethoyxl, n-propoxyl or isopropoxyl) or $C_3$-$C_7$ cycloalkyl(for example cyclopropyl);
the "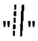" in

“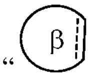”

β ring and 7-membered ring fused by is a single or double bond;

β ring is 5-6 membered aromatic ring (for example benzene ring), or, "5-6 membered heteroaromatic ring containing 1-3 heteroatom(s) selected from the group consisting of N, O and S" (for example pyridine ring, pyridazine ring, pyrrole ring, pyrazole ring, furan ring or thiophene ring; the nitrogen atom in the pyridine ring may be located at the ortho, meta or para position of Y);
$n^\beta$ is 0, 1, 2, 3 or 4 (e.g., 1 or 2);
all of $R^2$ is each independently $C_1$-$C_5$ alkyl, halogen (e.g., fluorine or chlorine), $C_1$-$C_5$ alkoxyl(e.g., methoxyl), $-N(R^{2a})(R^{2b})$, $-NHC(R^{2c})H_2$, $-N(R^{2k})S(=O)_2(R^{2d})$, $-(CH_2)n^{\beta 2}N(R^{2k})S(=O)_2(R^{2d})$, $-NHS(=O)_2NH(R^{2e})$, $-N(R^{2k})S(=O)_2NH(R^{2e})$, $-(CH_2)n^{\beta 2}N(R^{2k})S(=O)_2NH(R^{2e})$, $-NHC(=O)NH(R^{2e})$, $-(CH_2)n^{\beta 2}NHC(=O)NH(R^{2e})$, $-NHC(=O)(R^{2f})$, $-(CH_2)n^{\beta 2}NHC(=O)(R^{2e})$, $-(CH_2)n^{\beta 2}OC(=O)NH(R^{2e})$, $-(CH_2)n^{\beta 2}NHC(=O)O(R^{2e})$, $-(CH_2)n^{\beta 2}-C(=O)NH(R^{2e})$, $-S(=O)_2N(R^{2g})H$, $-(CH_2)n^{\beta 2}-S(=O)_2N(R^{2g})H$, $-S(=O)(R^{2h})$, $-(CH_2)n^{\beta 2}-S(=O)(R^{2i})$, $-S(=O)_2(R^{2h})$, $-(CH_2)n^{\beta 2}-S(=O)_2(R^{2i})$, $-CH_2S(=O)_2(R^{2i})$, $-C_1$-$C_6$ alkyl-$S(=O)_2(R^{2i})$ (the $C_1$-$C_6$ alkyl is, for example $C_1$-$C_3$ alkyl, the $C_1$-$C_3$ alkyl is for example methyl, ethyl, propyl or isopropyl), or a 5-6 membered heteroaryl substituted by one or more (for example 2, 3 or 4) of $R^{2j}$ (the "5-6 membered heteroaryl" is, for example, 5-6 membered heteroaryl containing 1-3 heteroatom(s) selected from the group consisting of N, O or S);
(any one of the $R^2$ may independently be located at the ortho, meta or para position of Y; for example, when $n^\beta$ is 1 and β ring is a six-membered ring, $R^2$ is located at the para or meta position of Y; or for another example, when $n^\beta$ is 2 and β ring is a six-membered ring, the two $R^2$ are located at the meta and para, ortho and para or ortho and meta position of Y. For example when $n^\beta$ is 1, 2, 3 or 4, at least one $R^2$ (for example one, which may be located at the ortho, para or meta position) is $-N(R^{2k})S(=O)_2(R^{2d})$, $-(CH_2)n^{\beta 2}N(R^{2k})S(=O)_2(R^{2d})$, $-NHS(=O)_2NH(R^{2e})$, $-N(R^{2k})S(=O)_2NH(R^{2e})$, $-(CH_2)n^{\beta 2}N(R^{2k})S(=O)_2NH(R^{2e})$, $-NHC(=O)NH(R^{2e})$, $-(CH_2)n^{\beta 2}NHC(=O)NH(R^{2e})$, $-NHC(=O)(R^{2f})$, $-(CH_2)n^{\beta 2}NHC(=O)(R^{2e})$, $-(CH_2)n^{\beta 2}OC(=O)NH(R^{2e})$, $-(CH_2)n^{\beta 2}NHC(=O)O(R^{2e})$, $-(CH_2)n^{\beta 2}-C(=O)NH(R^{2e})$, $-S(=O)_2N(R^{2g})H$, $-(CH_2)n^{\beta 2}-S(=O)_2N(R^{2g})H$, $-S(=O)(R^{2h})$, $-(CH_2)n^{\beta 2}-S(=O)(R^{21})$, $-S(=O)_2(R^{2h})$, $-(CH_2)n^{\beta 2}-S(=O)_2(R^{2i})$, $-CH_2S(=O)_2(R^{2i})$ or "5-6 membered heteroaryl substituted by one or more of $R^{2j}$"; for another example, when $n^\beta$ is 1, 2, 3 or 4, ate least one $R^2$ (for example one, which may be located in the ortho, para or meta position) is $-N(R^{2k})S(=O)_2(R^{2d})$, $-(CH_2)n^2-S(=O)_2(R^{2i})$, 或, $-CH_2S(=O)_2(R^{2i})$)
all of the $n^{\beta 2}$ is each independently 0, 1, 2, 3 or 4;
wherein, $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_6$ alkyl(the $C_1$-$C_6$ alkyl is for example $C_1$-$C_3$ alkyl, the $C_1$-$C_3$ alkyl is for example methyl, ethyl, n-propyl or isopropyl), $C_1$-$C_3$ haloalkyl( the number of halogen may be one or more (e.g., 2, 3 or 4); when more than one halogen exist, the halogen(s) may be the same or different; the "$C_1$-$C_3$ haloalkyl" is, for example, trifluoromethyl), 3-7 membered cycloalkyl(e.g., cyclopropyl, cyclobutyl, cyclopentyl or cyclohexyl), $-N(CH_3)_2$, or 5-6 membered N-containing heteroaryl(e.g., pyridinyl, pyrazinyl, pyridazinyl, pyrimidinyl, pyrrolyl, pyrazolyl or imidazolyl), $R^{2j}$ is independently -H, $C_1$-$C_3$ alkyl, or halogen;
"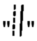" in

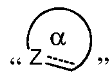

refers to the bond α ring and 7-membered ring fused by is a single or double bond;
Z is

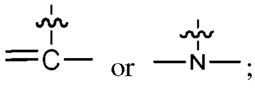

α ring is a 5-10 membered heteroaryl substituted by one or more (for example, 2, 3 or 4) of $R^\alpha$; (the "5-10 membered heteroaryl" is for example "5-10 membered heteroaryl containing 1-6 heteroatom(s) selected from the group consisting of N, O and S; the "5-10 membered heteroaryl" may be a 5-6 membered heteroaryl, or a fused ring formed by two 5-6 membered heteroaryls; the "5-6 membered heteroaryl" may be "5-6 membered heteroaryl containing 1-4 heteroatom(s) selected from the group consisting of N, O and S; the "fused ring formed by two 5-6 membered heteroaryls" may be "fused ring formed by two 5-6 membered heteroaryls containing 1-6 heteroatom(s) selected from the group consisting of N, O and S"; the "5-6 membered heteroaryl containing 1-4 heteroatom(s) selected from the group consisting of N, O and S" may be pyridine, pyridazine, pyrimidine, pyrazine, furan, thiophene, pyrrole, pyrazole, imidazole, oxazole, thiazole, pyrimidinone, oxadiazole, pyridone and triazole, also may be

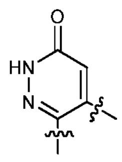 , 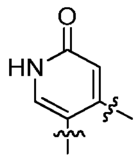 ,

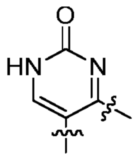 , 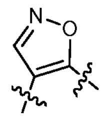 , 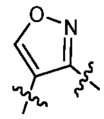 , 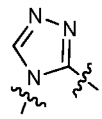 , 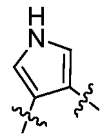 , 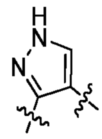 or 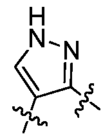 ;

the "fused ring formed by two 5-6 membered heteroaryls containing 1-6 heteroatom(s) selected from the group consisting of N, O and S" may be any fused ring formed by two heterocyclic rings selected from the group consisting of pyridine, pyridazine, pyrimidine, pyrazine, furan, thiophene, pyrrole, pyrazole, imidazole, oxazole, isoxazole, thiazole, pyrimidinone, oxadiazole, pyridone and triazole, also may be

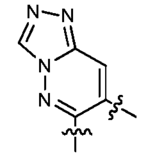 , 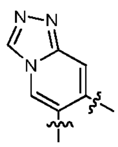 , 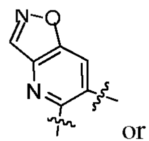 or 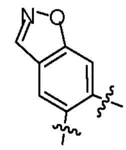 );

all of the $R^{\alpha}$ is each independently -H, -CN, $C_1$-$C_5$ alkyl(for example $C_1$-$C_3$ alkyl, the $C_1$-$C_3$ alkyl is for example methyl, ethyl , n-propyl or isospropyl), $C_1$-$C_5$ alkoxyl(for example $C_1$-$C_3$ alkoxyl; the $C_1$-$C_3$ alkoxyl is for example methoxy, ethoxy, n-propoxy or isopropoxy), $C_1$-$C_5$ haloalkyl, halogen (for example fluorine or chlorine), -C(=O)N($R^{\alpha1}$)($R^{\alpha2}$), or -N($R^{\alpha1}$)($R^{\alpha2}$); (each of the $R^{\alpha}$ is independently located at the ortho, meta or para position of Z; when the $\alpha$ ring contains -NH-, the $R^{\alpha}$ may be bonded to the -NH-)

wherein, all of the $R^{\alpha1}$, $R^{\alpha2}$, $R^{\alpha4}$ and $R^{\alpha4}$ are each independently -H, $C_1$-$C_5$ alkyl(for example $C_1$-$C_3$ alkyl, the $C_1$-$C_3$ alkyl for example is methyl, ethyl, n-propyl or isopropyl), or $C_1$-$C_5$ haloalkyl(the number of halogen may be one or more (for example 2, 3 or 4); when more than one halogen exist, the halogens may be the same or different; the "$C_1$-$C_5$ haloalkyl" is, for example trifluoromethyl).

In one embodiment, the definition of each group in the compound **III-0** is defined as follows (undefined definition as described above):

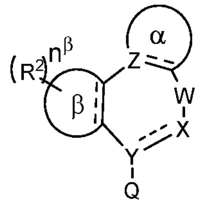

**III-0**

wherein, W is -C($R^1$)($R^3$)-;

$R^3$ is -H; $R^1$ is -H, $C_1$-$C_5$ alkyl(the $C_1$-$C_5$ alkyl is for example $C_1$-$C_3$ alkyl, the $C_1$-$C_3$ alkyl for example is methyl, ethyl, n-propyl or isopropyl), $C_1$-$C_5$ haloalkyl(the number of halogen may be one or more (for example 1, 2, 3 or 4); when more than one halogen exist, the halogens may be the same or different; the halogens may be attached to the same or different carbon atoms; the "$C_1$-$C_5$ haloalkyl" is for example "$C_1$-$C_3$ haloalkyl"; the"$C_1$-$C_3$ haloalkyl" is for example trifluoromethyl, difluoromethyl, 1,2-difluoroethyl), $C_3$-$C_7$ cycloalkyl(for example cyclopropyl), -$(CH_2)_{n0}$C(=O)N($R^{R1}$)($R^{R2}$) or -$(CH_2)_{n7}$S$(=O)_2$N($R^{R12}$)($R^{R13}$); (both of the $R^1$ and $R^3$ may not be -H; the $R^1$ and $R^3$ may not be -H at the same time; at least one (for example 1) of the $R^1$ and $R^3$ may be -H; the $R^1$ and $R^3$ may be -H at the same time; when the $R^1$ and $R^3$ are different, the carbon atom they attached to is a chiral carbon atom; the configuration of the chiral carbon atom may be R or S; for example,

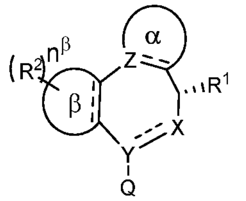 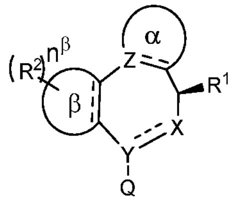

III-2-1 or III-2-2

)

$R^{R1}$, $R^{R2}$, $R^{R12}$ and $R^{R13}$ are each independently -H, $C_1$-$C_5$ alkyl(the $C_1$-$C_5$ alkyl is for example $C_1$-$C_3$ alkyl, the $C_1$-$C_3$ alkyl is for example methyl, ethyl, n-propyl or isopropyl), $C_1$-$C_5$ haloalkyl, or 3-7 membered cycloalkyl;
or, $R^1$, $R^3$ and the carbon atom they attached to together form a $C_3$-$C_7$ cycloalkyl(for example cyclopropyl);
$n_0$ and $n_7$ are each independently 0, 1, 2 or 3 (or for example 0 or 1);

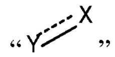

refers to the bond between X and Y is a single or double bond;
X is -$CH_2$-, =N-, -NH-, or -C(=O)-;
Y is

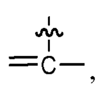, 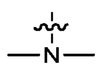 or 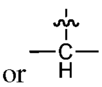;

and at least one of X and Y is a nitrogen atom; (

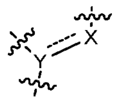

may be

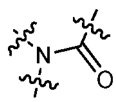,

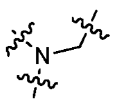, 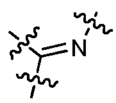 or 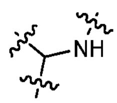

)

Q is a phenyl substituted by one or more (for example 2, 3 or 4) of $R^{Q1}$ (when more than one $R^{Q1}$ exist, the $R^{Q1}$ may be the same or different; the $R^{Q1}$ may be independently attached to the ortho, meta or para position of the phenyl; when two $R^{Q1}$ are present, the $R^{Q1}$ may be attached to the ortho and para, or meta and para positions of the phenyl; the "phenyl substituted by one or more (for example 2, 3 or 4) of $R^{Q1}$" such as 4-fluorophenyl, 4-chlorophenyl, 4-cyanophenyl, 2,4-difluorophenyl, 2,4-dichlorophenyl, 2-fluoro-4-chlorophenyl, 2-chloro-4-fluorophenyl, or 2-fluoro-4-cyanophenyl), or a 5-6 membered heteroaryl substituted by one or more (e.g., 2, 3 or 4) of $R^{Q4}$ (the heteroatom in the heteroaryl is N and/or O, the number of which is 1-3; the "5-6 membered heteroaryl" is for example pyridinyl or pyrimidinyl; the pyridinyl is for example pyridin-2-yl; the "5-6 membered heteroaryl substituted by one or more (e.g., 2, 3 or 4) of $R^{Q4}$" is for example pyridin-2-yl, or 5-chloro-pyridin-2-yl);
$R^{Q1}$ and $R^{Q4}$ is each independently -H, -CN or halogen (e.g., fluorine or chlorine);
the "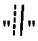" in

“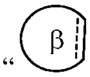”

β ring and 7-membered ring fused by is a single or double bond;
β ring is 5-6 membered aromatic ring (for example benzene ring), or, "5-6 membered heteroaromatic ring containing 1-3 heteroatoms selected from the group consisting of N, O and S" (for example pyridine ring, pyridazine ring, pyrrole ring, pyrazole ring, furan ring or thiophene ring; the nitrogen atom in the pyridine ring may be located at the ortho, meta or para position of Y, or may be located at the ortho position of Y);
$n^{\beta}$ is 0, 1, or 2;
each $R^2$ is independently halogen (e.g., fluorine or chlorine), $C_1$-$C_5$ alkoxyl(e.g., methoxyl), $-N(R^{2k})S(=O)_2(R^{2d})$, $-NHS(=O)_2NH(R^{2e})$, $-S(=O)_2N(R^{2g})H$, $-S(=O)_{1-2}(R^{2h})$, or $-(CH_2)n^{\beta 2}-S(=O)_{1-2}(R^{2i})$; (any one of the $R^2$ may be independently located at the ortho, meta or para position of Y; for example, when $n^{\beta}$ is 1 and β ring is a six-membered ring, $R^2$ is located at the para or meta position of Y; or for another example, when $n^{\beta}$ is 2 and β ring is a six-membered ring, the two $R^2$ are located at the meta and para, ortho and para or ortho and meta position of Y)
$n^{\beta}$ is 1 or 2;
wherein, $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_3$ alkyl(e.g., methyl, ethyl, n-propyl or isopropyl), or 3-7 membered cycloalkyl(e.g., cyclopropyl, cyclobutyl, cyclopentyl or cyclohexyl);
"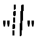" in

“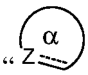”

refers to the bond α ring and 7-membered ring fused by is a single or double bond;
Z is

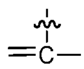;

α ring is a 5-10 membered heteroaromatic ring substituted by one or more (for example, 2, 3 or 4) of $R^{\alpha}$; (the "5-10 membered heteroaromatic ring" is for example "5-10 membered heteroaromatic ring containing 1-6 heteroatoms selected from the group consisting of N, O and S; the "5-10 membered heteroaromatic ring" may be a 5-6 membered heteroarylaromatic ring, or a fused ring formed by two 5-6 membered heteroaromatic rings; the "5-6 membered heteroaromatic ring" may be "5-6 membered heteroaromatic ring containing 1-4 heteroatoms selected from the group consisting of N, O and S"; the "fused ring formed by two 5-6 membered heteroaromatic rings" may be a "fused ring formed by two 5-6 membered heteroaromatic rings containing 1-6 heteroatoms selected from the group consisting of N, O and S"; the "5-6 membered heteroaromatic ring containing 1-4 heteroatoms selected from the group consisting of N, O and S" may also be pyridine, pyridazine, pyrimidine, pyrazine, furan, thiophene, pyrrole, pyrazole, imidazole, oxazole, thiazole, pyrimidinone, oxadiazole, pyridone and triazole, also may be

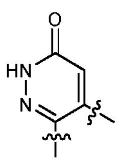, 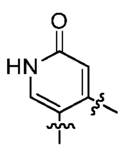, 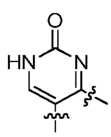, 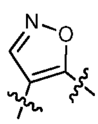, 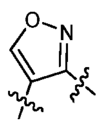, 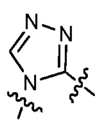, 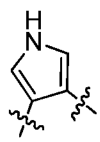, 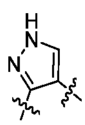

or

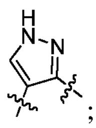;

the "fused ring formed by two 5-6 membered heteroaromatic rings containing 1-6 heteroatoms selected from the

group consisting of N, O and S" also may be any fused ring formed by two heteroaromatic rings selected from the group consisting of pyridine, pyridazine, pyrimidine, pyrazine, furan, thiophene, pyrrole, pyrazole, imidazole, oxazole, isoxazole, thiazole, pyrimidinone, oxadiazole, pyridone and triazole, also may be

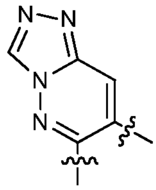
,
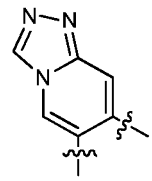
,

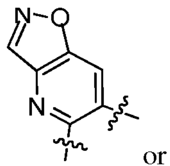
or
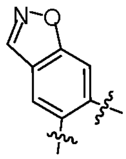

);
all of the $R^{\alpha}$ is each independently -H, -CN, $C_1$-$C_5$ alkyl(for example $C_1$-$C_3$ alkyl, the $C_1$-$C_3$ alkyl for example is methyl, ethyl, n-propyl and isopropyl), halogen (for example fluorine or chlorine) or -C(=O)N($R^{\alpha1}$)($R^{\alpha2}$) (each $R^{\alpha}$ is independently located at the ortho, meta or para position of Z; when the $\alpha$ ring contains -NH-, the $R^{\alpha}$ may be bonded to the -NH-)
wherein the $R^{\alpha1}$ and $R^{\alpha2}$ are each independently -H or $C_1$-$C_5$ alkyl(for example $C_1$-$C_3$ alkyl; the $C_1$-$C_3$ alkyl for example is methyl, ethyl, n-propyl or isopropyl).

In one embodiment, the definition of each group in the compound **III-0** is defined as follows (undefined definition as described above):

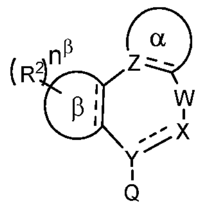

**III-0**

wherein, W is -C($R^1$)($R^3$)-;
$R^3$ is -H; $R^1$ is -H, $C_1$-$C_5$ alkyl(the $C_1$-$C_5$ alkyl is for example $C_1$-$C_3$ alkyl, the $C_1$-$C_3$ alkyl for example is methyl, ethyl, n-propyl or isopropyl), $C_1$-$C_5$ haloalkyl(the number of halogen may be one or more, for example 1, 2, 3 or 4); when more than one halogen exist, the halogens may be the same or different; the halogens may be attached to the same or different carbon atom; the "$C_1$-$C_5$ haloalkyl" is for example "$C_1$-$C_3$ haloalkyl"; the"$C_1$-$C_3$ haloalkyl" is for example trifluoromethyl, difluoromethyl, 1,2-difluoroethyl), $C_3$-$C_7$ cycloalkyl(for example cyclopropyl), $-(CH_2)_{n_0}C(=O)N(R^{R1})(R^{R2})$ or $-(CH_2)_{n_7}S(=O)_2N(R^{R12})(R^{R13})$; (both of the $R^1$ and $R^3$ may not be -H; the $R^1$ and $R^3$ may not be -H at the same time; at least one (for example 1) of the $R^1$ and $R^3$ may be -H; the $R^1$ and $R^3$ may be -H at the same time; when the $R^1$ and $R^3$ are different, the carbon atom they attached to is a chiral carbon atom; the configuration of the chiral carbon atom may be R or S; for example,

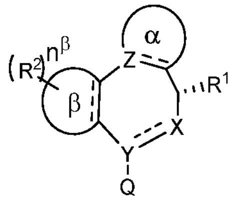
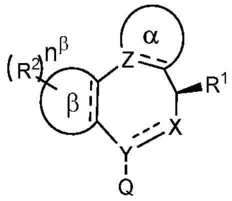

III-2-1 or III-2-2 )

$R^{R1}$, $R^{R2}$, $R^{R12}$ and $R^{R13}$ are each independently -H, $C_1$-$C_5$ alkyl(the $C_1$-$C_5$ alkyl is for example $C_1$-$C_3$ alkyl, the $C_1$-$C_3$ alkyl is for example methyl, ethyl, n-propyl and isopropyl), $C_1$-$C_5$ haloalkyl, or 3-7 membered cycloalkyl; or, $R^1$, $R^3$ and the carbon atom they are attached to together form a $C^3$-$C^7$ cycloalkyl(for example cyclopropyl);
$n_0$ and $n_7$ are each independently 0, 1, 2, or 3 (e.g., 0 or 1);

“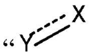”

refers to the bond between X and Y is a single or double bond;
X is -$CH_2$-, =N-, -NH-, or -C(=O)-;
Y is

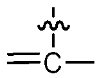, 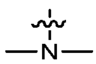 or 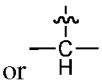;

and at least one of X and Y is a nitrogen atom; (

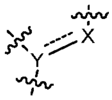

may be

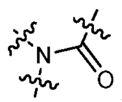,

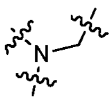, 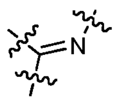 or 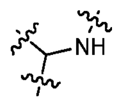

)
Q is a phenyl substituted by one or more (for example 2, 3 or 4) of $R^{Q1}$ (when more than one $R^{Q1}$ exist, the $R^{Q1}$ may be the same or different; the $R^{Q1}$ may be independently attached to the ortho, meta or para position of the phenyl; when two $R^{Q1}$ are present, the $R^{Q1}$ may be attached to the ortho and para positions of the phenyl; the "phenyl substituted by one or more (for example 2, 3 or 4) of $R^{Q1}$" such as 4-fluorophenyl, 4-chlorophenyl, 4-cyanophenyl, 2,4-difluorophenyl, 2,4-dichlorophenyl, 2-fluoro-4-chlorophenyl, 2-chloro-4-fluorophenyl, or 2-fluoro-4-cyanophenyl), or a 5-6 membered heteroaryl substituted by one or more (e.g., 2, 3 or 4) of $R^{Q4}$ (the heteroatom in the heteroaryl is N and/or O, the number of which is 1-3; the "5-6 membered heteroaryl" for example is pyridinyl or pyrimidinyl; the pyridinyl for example is pyridin-2-yl; the "5-6 membered heteroaryl substituted by one or more (e.g., 2, 3 or 4) of $R^{Q4}$" for example is pyridin-2-yl, or 5-chloro-pyridin-2-yl);
each $R^{Q1}$ and $R^{Q4}$ is independently -H, -CN or halogen (e.g., fluorine or chlorine);
the"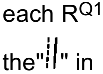" in

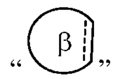

β ring and 7-membered ring fused by is a single or double bond;

β ring is 5-6 membered aromatic ring (for example benzene ring), or, "5-6 membered heteroaromatic ring containing 1-3 heteroatoms selected from the group consisting of N, O and S" (for example pyridine ring, pyridazine ring, pyrrole ring, pyrazole ring, furan ring or thiophene ring; the nitrogen atom in the pyridine ring may be located at the ortho, meta or para position of Y);

$n^{\beta}$ is 0, 1, or 2;

each $R^2$ is independently $-N(R^{2a})(R^{2b})$, $-NHC(R^{2c})H_2$, $-N(R^{2k})S(=O)_2(R^{2d})$, $-NHS(=O)_2NH(R^{2e})$, $-NHC(=O)(R^{2f})$, $-S(=O)_2N(R^{2g})H$, $-S(=O)(R^{2h})$, $-CH_2S(=O)_2(R^{2i})$, $-CH_2N(R^{2a})(R^{2b})$, $-CH_2NHC(R^{2c})H_2$, $-CH_2N(R^{2k})S(=O)_2(R^{2d})$, $-CH_2NHS(=O)_2NH(R^{2e})$, $-CH_2NHC(=O)(R^{2f})$, or $-CH_2S(=O)_2N(R^{2g})H$; (any one of the $R^2$ may be independently located at the ortho, meta or para position of Y; for example, when $n^{\beta}$ is 1 and β ring is a six-membered ring, $R^2$ is located at the para or meta position of Y; or for another example, when $n^{\beta}$ is 2 and β ring is a six-membered ring, the two $R^2$ are located at the meta and para, ortho and para or ortho and meta positions)

$n^{\beta 2}$ is 1 or 2;

wherein, $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_3$ alkyl(e.g., methyl, ethyl, n-propyl or isopropyl), or 3-7 membered cycloalkyl(e.g., cyclopropyl, cyclobutyl, cyclopentyl or cyclohexyl);

"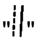" in

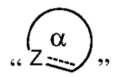

refers to the bond α ring and 7-membered ring fused by is a single or double bond;

Z is

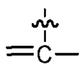;

α ring is a 5-10 membered heteroaromatic ring substituted by one or more (for example, 2, 3 or 4) of $R^{\alpha}$; (the "5-10 membered heteroaromatic ring" is for example "5-10 membered heteroaromatic ring containing 1-6 heteroatoms selected from the group consisting of N, O and S; the "5-10 membered heteroaromatic ring" may be a 5-6 membered heteroarylaromatic ring, or a fused ring formed by two 5-6 membered heteroaromatic rings; the "5-6 membered heteroaromatic ring" may be "5-6 membered heteroaromatic ring containing 1-4 heteroatoms selected from the group consisting of N, O and S"; the "fused ring formed by two 5-6 membered heteroaromatic rings" may be a "fused ring formed by two 5-6 membered heteroaromatic rings containing 1-6 heteroatoms selected from the group consisting of N, O and S"; the "5-6 membered heteroaromatic ring containing 1-4 heteroatoms selected from the group consisting of N, O and S" may also be pyridine, pyridazine, pyrimidine, pyrazine, furan, thiophene, pyrrole, pyrazole, imidazole, oxazole, thiazole, pyrimidinone, oxadiazole, pyridone and triazole, also may be

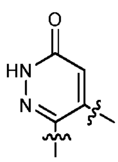, 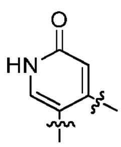, 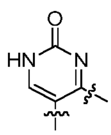, 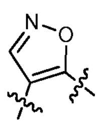, 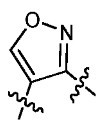, 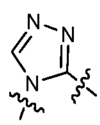, 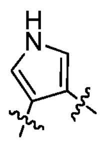, 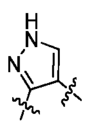

or

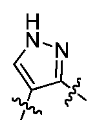;

the "fused ring formed by two 5-6 membered heteroaromatic rings containing 1-6 heteroatoms selected from the group consisting of N, O and S" also may be any fused ring formed by two heteroaromatic rings selected from the group consisting of pyridine, pyridazine, pyrimidine, pyrazine, furan, thiophene, pyrrole, pyrazole, imidazole, oxazole, isoxazole, thiazole, pyrimidinone, oxadiazole, pyridone and triazole, also may be

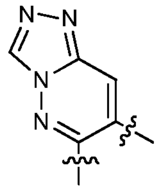 , 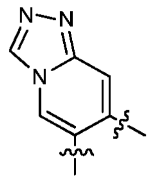 ,

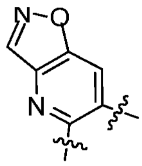 or 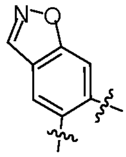 );

all of the $R^{\alpha}$ is each independently -H, -CN, $C_1$-$C_5$ alkyl(for example $C_1$-$C_3$ alkyl, the $C_1$-$C_3$ alkyl for example is methyl, ethyl, n-propyl and isopropyl), halogen (for example fluorine or chlorine) or -C(=O)N($R^{\alpha1}$)($R^{\alpha2}$) (each $R^{\alpha}$ is independently located at the ortho, meta or para position of Z; when the $\alpha$ ring contains -NH-, the $R^{\alpha}$ may be bonded to the -NH-)

wherein the $R^{\alpha1}$and $R^{\alpha2}$ are each independently -H or $C_1$-$C_5$ alkyl(for example $C_1$-$C_3$ alkyl; the $C_1$-$C_3$ alkyl for example is methyl, ethyl, n-propyl or isopropyl).

In one embodiment, the definition of each group in the compound **III-0** is defined as follows (undefined definition as described above):

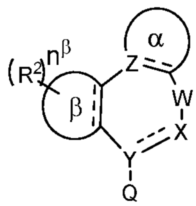

**III-0**

wherein, W is -C($R^1$)($R^3$)-;

$R^3$ is -H; $n^{\beta}$ is 2; Z is

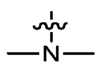 .

In one embodiment, the definition of each group in the compound **III-0** is defined as follows (undefined definition as described above):

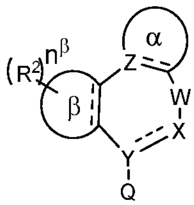

**III-0**

wherein, W is -C($R^1$)($R^3$)-;

$R^1$ and $R^3$ are both not -H; $n^\beta$ is 2; Z is

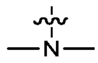.

In one embodiment, the definition of each group in the compound **III-0** is defined as follows (undefined definition as described above):

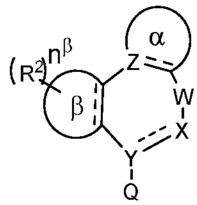

**III-0**

wherein, W is -C($R^1$)($R^3$)-;

$R^1$ and $R^3$ are both not -H; $n^\beta$ is 1; Z is

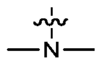.

In one embodiment, the definition of each group in the compound **III-0** is defined as follows (undefined definition as described above):

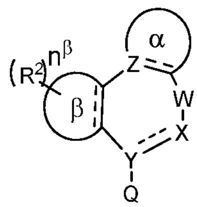

**III-0**

wherein, W is -C($R^1$)($R^3$)-;

$R^3$ is -H; $n^\beta$ is 1; Z is

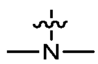.

In one embodiment, the definition of each group in the compound **III-0** is defined as follows (undefined definition as described above):

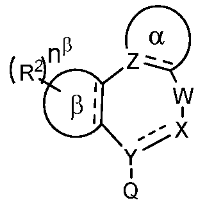

**III-0**

wherein, W is -C($R^1$)($R^3$)-;

$R^3$ is -H; $n^\beta$ is 2; Z is

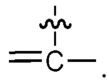

In one embodiment, the definition of each group in the compound **III-0** is defined as follows (undefined definition as described above):

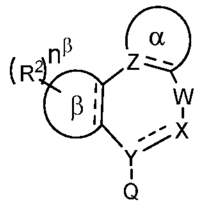

**III-0**

wherein, W is -C($R^1$)($R^3$)-;

$R^1$ and $R^3$ are both not -H; $n^\beta$ is 2; Z is

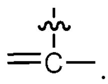

In one embodiment, the definition of each group in the compound **III-0** is defined as follows (undefined definition as described above):

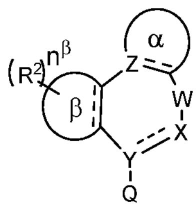

**III-0**

wherein, W is -C($R^1$)($R^3$)-;

$R^1$ and $R^3$ are both not -H; $n^\beta$ is 1; Z is

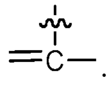

In one embodiment, the definition of each group in the compound **III-0** is defined as follows (undefined definition as described above):

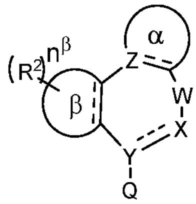

**III-0**

wherein, W is $-C(R^1)(R^3)-$;

$R^3$ is -H; $n^\beta$ is 1; Z is

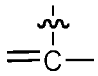.

In one embodiment, the definition of each group in the compound **III-0** is defined as follows (undefined definition as described above):

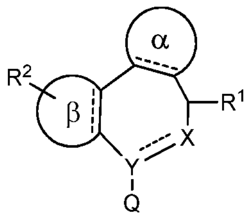

III

$R^1$ is selected from -H, $C_1$-$C_5$ linear or branched alkyl, $C_1$-$C_5$ linear or branched alkoxyl, $C_1$-$C_5$ linear or branched haloalkyl, halogen, $-(CH_2)n_0C(=O)N(R^{R1})(R^{R2})$, $-NH(CH_2)n_1C(=O)N(R^{R3})(R^{R4})$, $-(CH_2)n_2N(R^{R5})(R^{R6})$, $-(CH_2)n_3OC(=O)N(R^{R7})(R^{R8})$, $-(CH_2)n_4NHC(=O)R^{R9}$, $-(CH_2)n_5NHC(=O)OR^{R10}$, $-(CH_2)n_6NHS(=O)_2R^{R11}$ or $-(CH_2)n_7S(=O)_2N(R^{12})(R^{13})$;

Wherein, $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently selected from -H, $C_1$-$C_5$ linear or branched alkyl, $C_1$-$C_5$ linear or branched haloalkyl, $-(CH_2)m_1CN$, $-C(CH_3)_2CN$, 3-7 membered cycloalkyl, or $C_6$-$C_{10}$ aryl substituted by $R^{R14}$; $n_0$, $n_1$, $n_2$, $n_3$, $n_4$, $n_5$, $n_6$, $n_7$ and $m_1$ are each independently 0, 1, 2 or 3; $R^{R14}$ is selected from -H, $C_1$-$C_5$ linear or branched alkyl, $C_1$-$C_5$ linear or branched alkoxyl, linear or branched haloalkyl, halogen, -OH, -CN or $-NH_2$; the substitution may be mono- or poly-substitution;

X is selected from $-CH_2-$, =N-, -NH-, -O-, $-S(=O)_2-$ or -C(=O)-;

Y is selected from

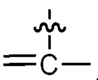, 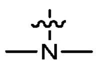 or 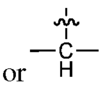;

and at least one of X and Y is a nitrogen atom;

Q is selected from phenyl substituted by $R^{Q1}$, 3-6 membered cyclohydrocarbyl-$(CH_2)n_Q$-substituted by $R^{Q2}$, 3-6 membered heterocyclohydrocarbyl-$(CH_2)n_Q$-substituted by $R^{Q3}$, or 5-6 membered heteroaryl substituted by $R^{Q4}$; each of $n_Q$, $m_Q$ is independently selected from 0, 1 or 2; each of $R^{Q1}$, $R^{Q2}$, $R^{Q3}$ or $R^{Q4}$ is independently selected from -H, -CN or halogen; the substitution may be a mono- or poly-substitution; each of the heteroatom in the heterocyclohydrocarbyl and the heteroaryl is independently selected from N or O, each of the number of the heteroatom is independently 1-3;

β ring is 5-6 membered aromatic ring or heteroaromatic ring, the heteroaromatic ring contains 1-3 heteroatom, the heteroatom is selected from N, O or S;

$R^2$ is selected from $-N(R^{2a})(R^{2b})$, $-NHC(R^{2c})H_2$, $-N(R^{2k})S(=O)_2(R^{2d})$, $-NHS(=O)_2NH(R^{2e})$, $-NHC(=O)(R^{2f})$, $-S(=O)_2N(R^{2g})H$, $-S(=O)_2(R^{2h})$, $-CH_2S(=O)_2(R^{2i})$, or 5-6 membered heteroaryl substituted by $R^{2j}$; the substitution may be mono- or poly-substitution;

wherein, $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently selected from -H, $C_1$-$C_3$ linear or branched alkyl, 3-7 membered cycloalkyl, $-N(CH_3)_2$, or 5-6 membered nitrogenous heteroaryl; $R^{2j}$ is selected from -H, $C_1$-$C_3$ linear or branched alkyl or halogen;

$\alpha$ ring is 5-6 membered heteroaromatic ring or a fused ring formed by two 5-6 membered heteroaromatic rings, each of the 5-6 membered heteroaromatic ring and the fused ring formed by two 5-6 membered heteroaromatic rings is independently substituted by $R^{\alpha}$; the substitution may be mono- or poly-substitution;

wherein, $R^{\alpha}$ is selected from -H, -CN, $C_1$-$C_5$ linear or branched alkyl, $C_1$-$C_5$ linear or branched alkoxyl, $C_1$-$C_5$ linear or branched haloalkyl, halogen, $-C(=O)N(R^{a1})(R^{a2})$, or$-N(R^{a3})(R^{a4})$; wherein, each of $R^{a1}$, $R^{a2}$, $R^{a3}$ and $R^{a4}$ is independently selected from -H, $C_1$-$C_5$ linear or branched alkyl, or $C_1$-$C_5$ linear or branched haloalkyl;

wherein, when $\alpha$ ring is 5-6 membered heteroaromatic ring, the 5-6 membered heteroaromatic ring is not isoxazole ring.

In one embodiment, the definition of each group in the compound III is defined as follows (undefined definition as described above):

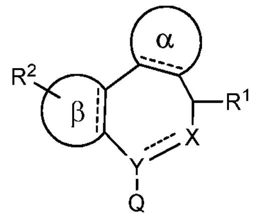

III

when $R^1$ is not -H, the cyclocarbon atom the $R^1$ directly is attached to is preferably in the configuration of S;

when $R^1$ is $C_1$-$C_5$ linear or branched alkyl, the $C_1$-$C_5$ linear or branched alkyl is preferably $C_1$-$C_3$ linear or branched alkyl, more preferably methyl, ethyl, n-propyl or isopropyl;

when $R^1$ is $C_1$-$C_5$ linear or branched alkoxyl, the $C_1$-$C_5$ linear or branched alkoxyl is preferably $C_1$-$C_3$ linear or branched alkoxyl, more preferably methoxyl, ethoxyl, n-propoxyl or isopropoxyl;

when $R^1$ is $C_1$-$C_5$ linear or branched haloalkyl, the $C_1$-$C_5$ linear or branched haloalkyl is $C_1$-$C_5$ linear or branched alkyl substituted by one or more same or different halogens, the halogenation may be on the same or different carbon atoms; the $C_1$-$C_5$ linear or branched haloalkyl is preferably $C_1$-$C_3$ linear or branched haloalkyl, more preferably trifluoromethyl, difluoromethyl or 1,2-difluoethyl etc.;

when $R^1$ is halogen, the halogen is preferably fluorine or chlorine;

each of $n_0$, $n_1$, $n_2$, $n_3$, $n_4$, $n_5$, $n_6$, $n_7$ and $m_1$ is independently preferably to be 0 or 1;

when each of $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently to be $C_1$-$C_5$ linear or branched alkyl, the $C_1$-$C_5$ linear or branched alkyl is referably to be $C_1$-$C_3$ linear or branched alkyl, more preferably to be methyl, ethyl, n-propyl or isopropyl;

when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently $C_1$-$C_5$ linear or branched haloalkyl, the $C_1$-$C_5$ linear or branched haloalkyl is a $C_1$-$C_3$ linear or branched alkyl substituted by one or more same or different halogens, the halogenation may be on the same of different carbon atoms; the $C_1$-$C_5$ linear or branched haloalkyl is preferably $C_1$-$C_3$ linear or branched haloalkyl, more preferably trifluoromethyl, difluoromethyl or 1,2-difluoethyl etc.;

when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently 3-7 membered cycloalkyl, the 3-7 membered cycloalkyl is preferably cyclopropyl, cyclobutyl, cyclopentyl or cyclohexyl;

when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently $C_6$-$C_{10}$ aryl substituted by $R^{R14}$, the $C_6$-$C_{10}$ aryl is preferably phenyl; the $R^{R14}$ is selected from -H, $C_1$-$C_5$ linear or branched alkyl, $C_1$-$C_5$ linear or branched alkoxyl, $C_1$-$C_5$ linear or branched haloalkyl, halogen, -OH, -CN, or $-NH_2$; wherein, the $C_1$-$C_5$ linear or branched alkyl is preferably $C_1$-$C_3$ linear or branched alkyl, more preferably methyl, ethyl, n-propyl or isopropyl; the $C_1$-$C_5$ linear or branched alkoxyl is preferably $C_1$-$C_3$ linear or branched alkoxyl, more preferably methoxyl, ethoxyl, n-propoxyl or isopropoxyl; the $C_1$-$C_5$ linear or branched haloalkyl is preferably $C_1$-$C_3$ linear or branched haloalkyl, more preferably trifluoromethyl, difluoromethyl or 1,2-difluoethyl etc.; the substitution may be mono- or di-substitution;

X is preferably $-CH_2$-, =N-, -NH-, or -C(=O)-;

Y is preferably

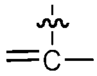 or 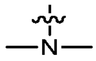 ;

when Q is phenyl substituted by $R^{Q1}$, the substitution is preferably mono- or di-substitution, more preferably para-substitution or 2,4-disubstitution; $R^{Q1}$ is preferably halogen, more preferably fluorine or chlorine;

when Q is a 3-6 membered cyclohydrocarbyl-$(CH_2)_{nQ}$-substituted by $R^{Q2}$, the 3-6 membered cyclohydrocarbyl is preferably 3-6 membered cycloalkyl; $n_Q$ preferably is 0; the substitution is preferably di-substitution, more preferably on the same carbon; $R^{Q2}$ preferably is -H or halogen (e.g., fluorine or chlorine);

when Q is a 3-6 membered heterocyclohydrocarbyl-$(CH_2)m_Q$-substituted by $R^{Q3}$, the 3-6 membered heterocyclohydrocarbyl is preferably 3-6 membered heterocycloalkyl; $m_Q$ preferably is 0; the heteroatom preferably is O; the number of heteroatom is preferably 1, $R^{Q3}$ preferably is -H; the 3-6 membered heterocycloalkyl preferably is attached to the rest moiety of the general formula III by the carbon on the ring; the 3-6 membered heterocycloalkyl preferably is 2-pyperidyl, 3-piperidyl, 2-tetrahydrofuranyl etc.,

when Q is 5-6 membered heteroaryl substituted by $R^{Q4}$, the 5-6 membered heteroaryl preferably is pyridyl or pyrimidyl; $R^{Q4}$ preferably is halogen (e.g., fluorine or chlorine);

$\beta$ ring preferably is benzene ring, pyridine ring, pyridazine ring, pyrrole ring, furan ring or thiophene ring; further preferably is benzene ring or pyridine ring;

when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_3$ linear or branched alkyl, the $C_1$-$C_3$ linear or branched alkyl preferably is methyl, ethyl, n-propyl or isopropyl;

when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 3-7 membered cycloalkyl, the 3-7 membered cycloalkyl preferably is cyclopropyl, cyclobutyl, cyclopentyl or cyclohexyl;

when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 5-6 membered nitrogenous heteroaryl, the 5-6 membered nitrogenous heteroaryl preferably is pyridyl, pyrazinyl, pyridazinyl, pyrimidinyl, pyrrolyl, pyrazolyl or imidazolyl; more preferably is attached to the rest moiety of the general formula **III** by the carbon atom on the ring;

when $R^2$ is 5-6 membered heteroaryl substituted by $R^{2j}$, $R^{2j}$ preferably is methyl, ethyl, n-propyl or isopropyl; the heteroatom of the 5-6 membered heteroaryl preferably is nitrogen atom, more preferably is pyridyl, pyrazinyl, pyridazinyl, pyrimidinyl, pyrrolyl, pyrazolyl or imidazolyl;

when $\alpha$ ring is 5-6 membered heteroaromatic ring substituted by $R^\alpha$, the 5-6 membered heteroaromatic ring preferably is pyridine, pyridazine, pyrimidine, pyrazine, furan, thiophene, pyrrole, pyrazole, imidazole, oxazole, thiazole, pyrimidinone, oxadiazole, pyridone, triazole; the substitution is preferably mono- or di-substitution;

when $\alpha$ ring is $R^\alpha$ substituted fused ring formed by two 5-6 membered heteroaromatic rings, the fused ring formed by two 5-6 membered heteroaromatic rings preferably is formed by any two selected from the group consisting of pyridine, pyridazine, pyrimidine, pyrazine, furan, thiophene, pyrrole, pyrazole, imidazole, oxazole, thiazole, pyrimidinone, oxadiazole, pyridone, triazole; the substitution preferably is mono- or di-substitution;

when $R^\alpha$ is $C_1$-$C_5$ linear or branched alkyl, the $C_1$-$C_5$ linear or branched alkyl is preferably $C_1$-$C_3$ linear or branched alkyl, more preferably methyl, ethyl, n-propyl or isopropyl;

when $R^\alpha$ is $C_1$-$C_5$ linear or branched alkoxyl, the $C_1$-$C_5$ linear or branched alkoxyl is preferably $C_1$-$C_3$ linear or branched alkoxyl, more preferably methoxyl, ethoxyl, n-propoxyl or isopropoxyl;

when $R^\alpha$ is $C_1$-$C_5$ linear or branched haloalkyl, the $C_1$-$C_5$ linear or branched haloalkyl is $C_1$-$C_5$ linear or branched alkyl that substituted by one or more same or different halogen atoms, the halogenation may be on the same of different carbon atoms; the $C_1$-$C_5$ linear or branched haloalkyl is preferably $C_1$-$C_3$ linear or branched haloalkyl, more preferably trifluoromethyl, difluoromethyl or 1,2-difluoethyl etc.;

when $R^\alpha$ is halogen, the halogen is preferably fluorine or chlorine;

when $R^\alpha$ is -C(=O)N($R^{a1}$)($R^{a2}$), each of the $R^{a1}$ and $R^{a2}$ is preferably -H, $C_1$-$C_5$ linear or branched alkyl, more preferably methyl, ethyl, n-propyl or isopropyl;

when $R^\alpha$ is -N($R^{a3}$)($R^{a4}$), each of the $R^{a3}$ and $R^{a4}$ is preferably -H, $C_1$-$C_5$ linear or branched alkyl, more preferably methyl, ethyl, n-propyl or isopropyl.

In one embodiment, the definition of each group in the compound **III** is defined as follows (undefined definition as described above):

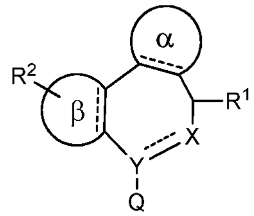

III

α ring is preferably $R^{\alpha}$ substituted

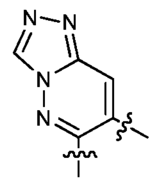 , 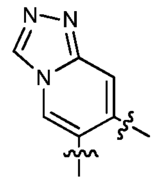 , 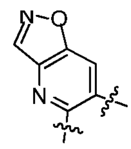 ,

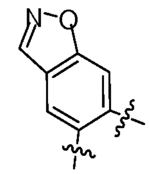 , 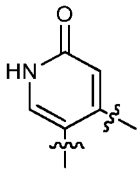 , 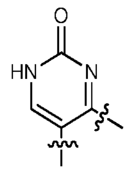 , 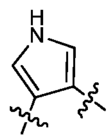 , 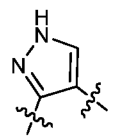 or 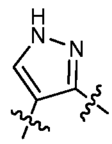 ;

the substitution preferably is mono- or di-substitution;
wherein, $R^{\alpha}$ is as given above; preferably is methyl, methoxyl, ethylamino, carbamoyl or cyano.

In one embodiment, the compound **III** or **III-0** is any one of the compounds as follows:

6-(4-fluorophenyl)-2-methyl-9-(methylsulfonemethyl)-5-oxo-2,4,5,6-tetrahydrobenzo[b]pyrrolo[3,4-d]azepin-3-nitrile (**4003**)
6-(4-fluorophenyl)-2-methyl-9-(methylsulfonemethyl)-5-oxo-2,4,5,6-tetrahydrobenzo[*b*]pyrrolo[3,4-d]azepin-3-carboxamide (**4001**)
6-(4-fluorophenyl)-2-methyl-9-(methylsulfonemethyl)-2,4,5,6-tetrahydrobenzo[b]pyrrolo[3,4-*d*]azepin-3-nitrile (**4004**)
6-(4-fluorophenyl)-2-methyl-9-(methylsulfonemethyl)-2,4,5,6-tetrahydrobenzo[*b*]pyrrolo[3,4-d]azepin-3-carboxamide (**4002**)
9-fluoro-6-(4-fluorophenyl)-2-methyl-2,4,5,6-tetrahydropyrido[2,3-*b*]pyrrolo[3,4-*d*]azepine-3-carboxamide (**4005**)
7-(4-fluorophenyl)-2-methyl-10-(methylsulfonemethyl)-2*H*-benzo[*c*]pyrido[3,4-*e*]azepin-3(*5H*)-ketone (**4007**)
7-(4-fluorophenyl)-2-methyl-10-(methylsulfonemethyl)-6,7-dihydro-2*H*-benzo[c]pyrido[3,4-*e*]azepin-3(5*H*)-ketoformate (**4008A**);
7-(4-Fluorophenyl)-2-methyl-10-(methylsulfonemethyl)-6,7-dihydro-2*H*-benzo[*c*]pyrido[3,4-*e*]azepin-3(5*H*)-ketone (**4008B**);
7-(4-Fluorophenyl)-2-methyl-10-(ethylsulfonemethyl)-2*H*-benzo[c]pyrido[3,4-e]azepin-3(5*H*)-ketone (**4009**);
7-(4-Chlorophenyl)-2-methyl-2,5-dihydro-3*H*-benzo[*c*]pyrido[3,4-*e*]azepin-3-one (**4010**);
7-(4-Chlorophenyl)-10-((ethylsulfone)methyl)-2-methyl-2,5-dihydro-3*H*-benzo[*c*]pyrido[3,4-*e*]azepin-3-one (**4011**);
7-(2,4-Difluorophenyl)-2-methyl-10-(ethylsulfonemethyl)-2*H*-benzo[*c*]pyrido[3,4-*e*]azepin-3(5*H*)-ketone (**4012**);
7-(4-Chloro-2-fluorophenyl)-10-((ethylsulfone)methyl)-2-methyl-2,5-dihydro-3*H*-benzo[*c*]pyrido[3,4-e]azepin-3-one (**4013**);
(*S*)-2-(7-(4-fluorophenyl)-2-methyl-10-(methylsulfonylmethyl)-3-oxo-3,5-dihydro-2*H*-benzo[*c*]Pyrido[3,4-*e*]azepin-5-yl)acetamide (**4014**);
(*S*)-*N*-ethyl-2-(7-(4-fluorophenyl)-2-methyl-10-(methylsulfonemethyl)-3-oxo-3,5-dihydro-2*H*-benzo[*c*]pyrido[3,4-*e*]azepin-5-yl)acetamide (**4015**);
(*S*)-2-(7-(4-chlorophenyl)-2-methyl-3-oxo-3,5-dihydro-2*H*-benzo[*c*]pyrido[3,4-*e*]azepin-5-yl)-*N*-ethylacetamide (**4016**);

(*S*)-2-(7-(4-chlorophenyl)-2-methyl-3-oxo-3,5-dihydro-2*H*-benzo[*c*]pyrido[3,4-*e*]azepin-5-yl)-acetamide (**4017**);
(*S*)-2-(7-(4-chlorophenyl)-2-methyl-3-oxo-3,5-dihydro-2*H*-benzo[*c*]pyrido[3,4-*e*]azepin-5-yl)-*N*-isopropylacetamide (**4032**);
*N*-(7-(4-fluorophenyl)-2-methyl-3-oxo-3,5-dihydro-2*H*-benzo[*c*]pyrido[3,4-*e*]azepin-10-yl)ethanesulfonamide (**4018**);
7-(4-Fluorophenyl)-10-methoxy-2-methyl-2,5-dihydro-3*H*-benzo[*c*]pyrido[3,4-*e*]azepin-3-ketone (**4019**);
7-(4-Fluorophenyl)-9-methoxy-2-methyl-2,5-dihydro-3*H*-benzo[*c*]pyrido[3,4-*e*]azepin-3-ketone (**4020**);
(*S*)-*N*-ethyl-2-(7-(4-fluorophenyl)-10-methoxy-2-methyl-3-oxo-3,5-dihydro-2*H*-benzo[*c*]pyrido[3,4-*e*]azepin-5-yl)acetamide (**4021**);
10-((Ethylsulfonyl)methyl)-7-(4-fluorophenyl)-2-methyl-2,5-dihydro-3*H*-dipyrido[2,3-*c*:3',4'-*e*]azepin-3-one (**4022**);
(*S*)-*N*-ethyl-2-(7-(4-fluorophenyl)-9-methoxy-2-methyl-3-oxo-3,5-dihydro-2*H*-benzo[*c*]pyrido[3,4-*e*]azepin-5-yl)acetamide (**4023**);
*N*-(7-(4-chlorophenyl)-2-methyl-3-oxo-3,5-dihydro-2*H*-benzo[*c*]pyrido[3,4-*e*]azepin-10-yl)ethanesulfonamide (**4024**);
(*S*)-7-(4-chlorophenyl)-2,5-dimethyl-2,5-dihydro-3*H*-benzo[*c*]pyrido[3,4-*e*]azepin-3-ketone (**4025**);
(*S*)-10-((ethylsulfone)methyl)-7-(4-fluorophenyl)-2,5-dimethyl-2,5-dihydro-3*H*-benzo[*c*]pyrido[3,4-*e*]azepin-3-one (**4026**);
(*R*)-10-((ethylsulfone)methyl)-7-(4-fluorophenyl)-2,5-dimethyl-2,5-dihydro-3*H*-benzo[*c*]pyrido[3,4-e]azepin-3-one (**4033**)
7-(4-Chlorophenyl)-10-((ethylsulfone)methyl)-2-methyl-2,5-dihydro-3*H*-dipyrido[3,4-*c*:3',4'-*e*]azepin-3-one (**4027**);
7-(2-Chloro-4-fluorophenyl)-10-((ethylsulfone)methyl)-2-methyl-2,5-dihydro-3*H*-benzo[*c*]pyrido[3,4-*e*]azepin-3-one (**4028**);
(*S*)-2-(7-(4-chlorophenyl)-2-methyl-3-oxo-3,5-dihydro-2*H*-dipyrido[2,3-*c*:3',4'-*e*]azepin-5-yl)acetamide (**4029**);
(*S*)-2-(7-(4-chlorophenyl)-2-methyl-3-oxo-3,5-dihydro-2*H*-dipyrido[2,3-*c*:3',4'-*e*]azepin-5-yl)-*N*-ethylacetamide (**4030**);
*N*-(9-chloro-7-(4-fluorophenyl)-2-methyl-3-oxo-3,5-dihydro-2*H*-benzo[*c*]pyrido[3,4-*e*]azepin-10-yl)ethanesulfonamide (**4031**);
(*S*)-2-(7-(4-chlorophenyl)-9-fluoro-2-methyl-3-oxo-3,5-dihydro-2*H*-benzo[*c*]pyrido[3,4-*e*]azepin-5-yl)-*N*-ethylacetamide (**4034**);
(*S*)-*N*-ethyl-2-(8-fluoro-7-(4-fluorophenyl)-9-methoxy-2-methyl-3-oxo-3,5-dihydro-2*H*-benzo[*c*]pyrido[3,4-*e*]azepin-5-yl)acetamide (**4035**);
(*S*)-*N*-ethyl-2-(10-fluoro-7-(4-fluorophenyl)-9-methoxy-2-methyl-3-oxo-3,5-dihydro-2*H*-benzo[*c*]pyrido[3,4-*e*]azepin-5-yl)acetamide (**4036**);
(*S*)-10-((ethylsulfone)methyl)-7-(4-chlorophenyl)-2,5-dimethyl-2,5-dihydro-3*H*-benzo[*c*]pyrido[3,4-*e*]azepin-3-one (**4037**);
(*S*)-5-ethyl-10-((ethylsulfone)methyl)-7-(4-fluorophenyl)-2-methyl-2,5-dihydro-3*H*-benzo[*c*]pyrido[3,4-*e*]azepin-3-one (**4038**);
9-((Ethylsulfonyl)methyl)-7-(4-fluorophenyl)-2-methyl-2,5-dihydro-3*H*-benzo[*c*]pyrido[3,4-*e*]azepin-3-one (**4039**);
(*R*)-10-((ethylsulfone)methyl)-7-(4-fluorophenyl)-2-methyl-5-(trifluoromethyl)-2,5-dihydro-3*H*-benzo[*c*]pyrido[3,4-*e*]azepin-3-one (**4040**);
10-((Ethylsulfonyl)methyl)-7-(4-fluorophenyl)-2-methylspiro[benzo[*c*]pyrido[3,4-*e*]azepin-5,1'-cyclopropane]-3(2*H*)-one (**4041**);
(*S*)-5-cyclopropyl-10-((ethylsulfone)methyl)-7-(4-fluorophenyl)-2-methyl-2,5-dihydro-3*H*-benzo[*c*]pyrido[3,4-*e*]azepin-3-one (**4042**);
(*S*)-7-(4-fluorophenyl)-2,5-dimethyl-10-((trifluoromethylsulfone)methyl)-2,5-dihydro-3*H*-benzo[*c*]pyrido[3,4-*e*]azepin-3-one (**4043**);
(*S*)-10-((isopropylsulfone)methyl)-7-(4-fluorophenyl)-2,5-dimethyl-2,5-dihydro-3*H*-benzo[*c*]pyrido]3,4-*e*]azepin-3-one (**4044**);
(*S*)-7-(4-fluorophenyl)-2,5-dimethyl-10-((cyclopropylsulfone)methyl)-2,5-dihydro-3*H*-benzo[*c*]pyrido[3,4-e]azepin-3-one (**4045**);
(*S*)-7-(2,4-difluorophenyl)-10-((ethylsulfone)methyl)-2,5-dimethyl-2,5-dihydro-3*H*-benzo[*c*]pyrido[3,4-*e*]azepin-3-one (**4046**);
(*S*)-5-ethyl-10-((ethylsulfone)methyl)-7-(4-chlorophenyl)-2-methyl-2,5-dihydro-3*H*-benzo[*c*]pyrido[3,4-*e*]azepin-3-one (**4047**);
(5*S*)-10-((ethylsulfoxide)methyl)-7-(4-fluorophenyl)-2,5-dimethyl-2,5-dihydro-3*H*-benzo[*c*]pyrido[3,4-e]azepin-3-one (**4048**);
(S*)-N*-(7-(4-fluorophenyl)-2,5-dimethyl-3-oxo-3,5-dihydro-2*H*-benzo[*c*]pyrido[3,4-*e*]azepin-10-yl)ethanesulfonamide (**4049**);

(*S*)-*N*-(7-(4-fluorophenyl)-2,5-dimethyl-3-oxo-3,5-dihydro-2*H*-benzo[*c*]pyrido[3,4-*e*]azepin-10-yl)methylaminosulfonamide (**4050**);
(*S*)-1-ethyl-3-(7-(4-fluorophenyl)-2,5-dimethyl-3-oxo-3,5-dihydro-2*H*-benzo[*c*]pyrido[3,4-*e*]azepin-10-yl)urea (**4051**);
(*S*)-*N*-ethyl-7-(4-fluorophenyl)-2,5-dimethyl-3-oxo-3,5-dihydro-2*H*-benzo[*c*]pyrido[3,4-*e*]azepin-10-sulfonamide (**4052**);
(*S*)-10-((ethylsulfone)methyl)-7-(4-methoxyphenyl)-2,5-dimethyl-2,5-dihydro-3*H*-benzo[*c*]pyrido[3,4-e]azepin-3-one (**4053**);
(*S*)-10-((ethylsulfone)methyl)-7-(4-hydroxyphenyl)-2,5-dimethyl-2,5-dihydro-3*H*-benzo[*c*]pyrido[3,4-*e*]azepin-3-one (**4061**);
(*S*)-10-((ethylsulfone)methyl)-7-(4-fluoro-3-methoxyphenyl)-2,5-dimethyl-2,5-dihydro-3*H*-benzo[*c*]pyrido[3,4-*e*]azepin-3-one (**4054**);
(*S*)-10-((ethylsulfone)methyl)-7-(4-fluoro-3-hydroxyphenyl)-2,5-dimethyl-2,5-dihydro-3*H*-benzo[*c*]pyrido[3,4-*e*]azepin-3-one (**4064**);
(*S*)-*N*-(9-fluoro-7-(4-fluorophenyl)-2,5-dimethyl-3-oxo-3,5-dihydro-2*H*-benzo[*c*]pyrido[3,4-*e*]azepin-10-yl)ethanesulfonamide (**4055**);
(*S*)-*N*-ethyl-2-(10-((ethylsulfonyl)methyl)-7-(4-fluorophenyl)-2-methyl-3-oxo-3,5-dihydro-2*H*-benzo[*c*]pyrido[3,4-*e*]azepin-5-yl)acetamide (**4056**);
(*S*)-2-(10-((ethylsulfonyl)methyl)-7-(4-fluorophenyl)-2-methyl-3-oxo-3,5-dihydro-2*H*-benzo[*c*]pyrido[3,4-e]azepin-5-yl)aceticacid (**4067**);
(*S*)-2-(10-((ethylsulfonyl)methyl)-7-(4-fluorophenyl)-2-methyl-3-oxo-3,5-dihydro-2*H*-benzo[*c*]pyrido[3,4-*e*]azepin-5-yl)acetamide (**4076**);
(*S*)-10-((ethylsulfone)methyl)-7-(4-fluorophenyl)-9-methoxy-2,5-dimethyl-2,5-dihydro-3*H*-benzo[*c*]pyrido[3,4-e]azepin-3-one (**4057**);
(*S*)-10-((ethylsulfone)methyl)-7-(4-fluorophenyl)-9-hydroxy-2,5-dimethyl-2,5-dihydro-3*H*-benzo[*c*]pyrido[3,4-*e*]azepin-3-one (**4063**);
(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)ethylsulfonamide (**4058**);
(*S*)-7-(4-fluorophenyl)-2,5-dimethyl-10-((methylsulfone)methyl)-2,5-dihydro-3*H*-benzo[*c*]pyrido[3,4-*e*]azepin-3-one (**4059**);
(*S*)-10-((ethylsulfone)methyl)-7-(4-fluorophenyl)-5-isopropyl-2-methyl-2,5-dihydro-3*H*-benzo[c]pyrido[3,4-e]azepin-3-one (**4060**);
(*S*)-*N*-(7-(4-chlorophenyl)-9-fluoro-2,5-dimethyl-3-oxo-3,5-dihydro-2*H*-benzo[*c*]pyrido[3,4-*e*]azepin-10-yl)ethanesulfonamide (**4062**);
(*S*)-*N*-(9-chloro-5-ethyl-7-(4-fluorophenyl)-2-methyl-3-oxo-3,5-dihydro-2*H*-benzo[*c*]pyrido[3,4-*e*]azepin-10-yl)ethanesulfonamide (**4065**);
(*S*)-*N*-(5-ethyl-9-fluoro-7-(4-fluorophenyl)-2-methyl-3-oxo-3,5-dihydro-2*H*-benzo[*c*]pyrido[3,4-*e*]azepin-10-yl)ethanesulfonamide (**4066**);
(*S*)-*N*-(9-chloro-7-(4-chlorophenyl)-2,5-dimethyl-3-oxo-3,5-dihydro-2*H*-benzo[*c*]pyrido[3,4-*e*]azepin-10-yl)ethanesulfonamide (**4068**);
(*S*)-10-amino-9-chloro-7-(4-fluorophenyl)-2,5-dimethyl-2,5-dihydro-3*H*-benzo[*c*]pyrido[3,4-*e*]azepin-3-one (**4069**);
(*S*)-10-amino-9-fluoro-7-(4-fluorophenyl)-2,5-dimethyl-2,5-dihydro-3H-benzo[*c*]pyrido[3,4-*e*]azepin-3-one (**4070**);
(S)-(7-(4-fluorophenyl)-2,5-dimethyl-3-oxo-3,5-dihydro-2H-benzo[*c*]pyrido[3,4-e]azepin-10-yl)methanesulfonamide (**4071**);
(*S*)-1-(7-(4-fluorophenyl)-2,5-dimethyl-3-oxo-3,5-dihydro-2*H*-benzo[*c*]pyrido[3,4-*e*]azepin-10-yl)-*N*-methylmethanesulfonamide (**4072**);
(*S*)-1-(7-(4-fluorophenyl)-2,5-dimethyl-3-oxo-3,5-dihydro-2*H*-benzo[*c*]pyrido[3,4-*e*]azepin-10-)-*N*,*N*-dimethylmethanesulfonamide (**4073**);
(*S*)-*N*-(9-fluoro-7-(4-fluorophenyl)-2,5-dimethyl-3-oxo-3,5-dihydro-2*H*-benzo[*c*]pyrido[3,4-*e*]azepin-10-yl)methanesulfonamide (**4074**);
(*S*)-4-(10-((ethylsulfonyl)methyl)-2,5-dimethyl-3-oxo-3,5-dihydro-2*H*-benzo[*c*]pyrazolo[3,4-*e*]azepin-7-yl)benzonitrile (**4075**);
(*S*)-*N*-(9-chloro-5-cyclopropyl-7-(4-fluorophenyl)-2-methyl-3-oxo-3,5-dihydro-2*H*-benzo[*c*]pyrido[3,4-e]azepin-10-yl)ethanesulfonamide (**4077**);
10-((Ethylsulfonyl)methyl)-7-(4-fluorophenyl)-2,5-dimethyl-2,5-dihydro-3*H*-benzo[e]pyrido[4,3-*c*][1,2]diazepin-3-one (**4078**);
(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)methanesulfonamide (**4079**);

(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)cyclopropylsulfonamide (**4080**);
10-((Ethylsulfonyl)methyl)-7-(4-fluorophenyl)-2,5-dimethyl-2,5-dihydro-3*H*-benzo[*c*]pyridazino[3,4-e]azepin-3-one (**4081**);
10-((Ethylsulfonyl)methyl)-7-(4-fluorophenyl)-5-(methoxymethyl)-2-methyl-2,5-dihydro-3*H*-benzo[*c*]pyrido[3,4-*e*]azepin-3-one (**4082**);
(*S*)-*N*-(9-fluoro-7-(4-fluorophenyl)-2,5-dimethyl-3-oxo-3,5-dihydro-2*H*-benzo[*c*]pyrido[3,4-*e*]azepin-10-yl)propanamide (**4083**);
(*S*)-*N*-(5-cyclopropyl-9-fluoro-7-(4-fluorophenyl)-2-methyl-3-oxo-3,5-dihydro-2*H*-benzo[*c*]pyrido[3,4-*e*]azepin-10-yl)ethanesulfonamide (**4084**);
(*S*)-10-((ethylsulfone)methyl)-9-fluoro-7-(4-fluorophenyl)-2,5-dimethyl-2,5-dihydro-3*H*-benzo[*c*]pyrido[3,4-*e*]azepin-3-one (**4085**);
(*S*)-9-chloro-10-((ethylsulfone)methyl)-7-(4-fluorophenyl)-2,5-dimethyl-2,5-dihydro-3*H*-benzo[*c*]pyrido[3,4-*e*]azepin-3-one (**4086**);
(*S*)-10-((ethylsulfone)methyl)-7-(4-fluoro-2-methoxyphenyl)-2,5-dimethyl-2,5-dihydro-3*H*-benzo[*c*]pyrido[3,4-*e*]azepin-3-one (**4087**);
(*S*)-10-((ethylsulfone)methyl)-7-(4-fluorophenyl)-5-(2-hydroxyethyl)-2-methyl-2,5-dihydro-3*H*-benzo[*c*]pyrido[3,4-*e*]azepin-3-one (**4088**);
10-((Ethylsulfonyl)methyl)-7-(4-fluoro-3-methoxyphenyl)-2-methyl-2,5-dihydro-3*H*-benzo[*c*]pyrido[3,4-e]azepin-3-one (**4089**);
(*S*)-2-(7-(4-fluorophenyl)-2,5-dimethyl-3-oxo-3,5-dihydro-2*H*-benzo[*c*]pyrido[3,4-*e*]azepin-10-yl)-*N*-methylpropane-2-sulfonamide (**4090**);
1-((*S*)-7-(4-fluorophenyl)-2,5-dimethyl-3-oxo-3,5-dihydro-2*H*-benzo[*c*]pyrido[3,4-*e*]azepin-10-yl)-*N*-methylethane-1-sulfonamide (**4091**);
*N*-(9-fluoro-7-(4-fluorophenyl)-2,5-dimethyl-3-oxo-3,5-dihydro-2*H*-benzo[*e*]pyrido[4,3-*c*][1,2]diazepin-10-yl)ethanesulfonamide (**4092**);
7-(2-Chloro-4-fluorophenyl)-10-((ethylsulfone)methyl)-2,5-dimethyl-2,5-dihydro-3*H*-benzo[*e*]pyrido[4,3-*c*][1,2]diazepin-3-one (**4093**);
10-((ethylsulfone)methyl)-7-(4-fluorophenyl)-9-methoxy-2-methyl-2,5-dihydro-3*H*-benzo[*c*]pyrido[3,4-*e*]azepin-3-one (**4094**);
*N*-(9-fluoro-7-(4-fluorophenyl)-2,5-dimethyl-3-oxo-3,5-dihydropyrido-2*H*-benzo[*c*]pyridazino[3,4-*e*]azepin-10-yl)ethanesulfonamide (**4095**);
7-(2-Chloro-4-fluoro-5-methoxyphenyl)-10-((ethylsulfone)methyl)-2-methyl-2,5-dihydro-3*H*-benzo[*c*]pyrido[3,4-*e*]azepin-3-one (**4096**);
7-(2-Chloro-4-fluorophenyl)-10-((ethylsulfone)methyl)-2,5-dimethyl-2,5-dihydro-3*H*-benzo[*c*]pyridazino[3,4-*e*]azepin-3-one (**4097**);
7-(2-Chloro-4-fluorophenyl)-2-methyl-10-((methylsulfone)methyl)-2,5-dihydro-3*H*-benzo[*c*]pyrido[3,4-e]azepin-3-one (**4098**);
1-(7-(4-fluorophenyl)-2,5-dimethyl-3-oxo-3,5-dihydro-2*H*-benzo[*e*]pyrido[4,3-*c*][1,2]diazepin-10-yl)-*N*-methylmethanesulfonamide (**4099**);
10-((Ethylsulfonyl)methyl)-7-(4-fluoro-3-methoxyphenyl)-2,5-dimethyl-2,5-dihydro-3*H*-benzo[*c*]pyridazino[3,4-e]azepin-3-one (**4100**);
(*S*)-7-(2-chloro-4-fluorophenyl)-10-((ethylsulfone)methyl)-2,5-dimethyl-2,5-dihydro-3*H*-benzo[*c*]pyrido[3,4-e]azepin-3-one (**4101**);
(S)-7-(2-chloro-4-fluoro-5-methoxyphenyl)-10-((ethylsulfone)methyl)-2,5-dimethyl-2,5-dihydro-3 *H*-benzo[*c*]pyrido[3,4-*e*]azepin-3-one (**4102**);
(*S*)-1-(7-(4-fluoro-3-methoxyphenyl)-2,5-dimethyl-3-oxo-3,5-dihydro-2*H*-benzo[*c*]pyrido[3,4-*e*]azepin-10-yl)-*N*-methylmethanesulfonamide (**4103**);
(*S*)-10-((ethylsulfone)methyl)-7-(3-fluoro-4-methoxyphenyl)-2,5-dimethyl-2,5-dihydro-3*H*-benzo[*c*]pyrido[3,4-*e*]azepin-3-one (**4104**);
(*S*)-10-((ethylsulfone)methyl)-2,5-dimethyl-7-(pyrido-4-yl)-2,5-dihydro-3*H*-benzo[*c*]pyrido[3,4-*e*]azepin-3-one (**4105**).

The present invention also provides a preparation method for the nitrogenous macrocyclic compound represented by formula (**III-0-A**), comprising: carrying out an amide reduction on the intermediate compound represented by formula (**III-0-B**):

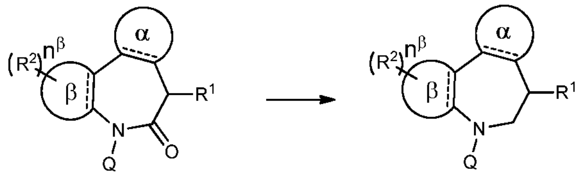

III-0-B III-0-A ;

wherein, the substituents are as described above.

Wherein, the amide reduction may be carried out by using conditions and parameters that commonly used in conventional amide reduction in the art, the present invention preferably to be carried out by treatment with borane in tetrahydrofuran and at room temperature.

The present invention also provides a preparation method for the nitrogenous macrocyclic compound represented by formula (**III-0-B**), comprising: carrying out an Ullmann coupling reaction on the intermediate compound represented by formula (**III-0-C**) and Q':

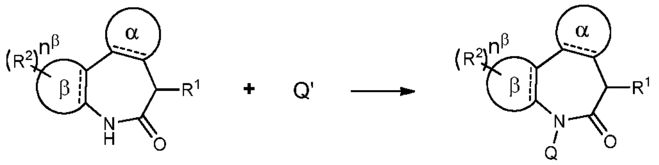

III-0-C III-0-B ;

wherein, the substituents are as described above, Q' is the corresponding halide on the reaction site of the structural fragment Q;

The Ullmann coupling reaction is carried out by using conditions and parameters that commonly used in conventional such reactions in the art. The present invention is preferably to be carried out in solvent, in the presence of a palladium catalyst and a base; or promoted by microwave irradiation.

Wherein, the solvent is a conventional solvent that commonly used in Ullmann coupling reaction in the art, including but not limited to 1,4-dixane, acetonitrile, water or a mixture thereof.

The reaction temperature of the Ullmann coupling reaction is the conventional reaction temperature in the art, e.g., 80°C-110°C.

The copper catalyst is a conventional catalyst for Ullmann coupling reaction in the art, including but not limited to cuprous iodide.

The copper catalyst also may, before catalyzation, coordinate with conventional ligands commonly used for Ullmann coupling reaction in the art. The ligand includes but not limited to N1,N2-dimethylethane-1,2-diamine.

The base is a conventional base for Suzuki coupling reaction in the art, includes but limited to carbonates or phosphates of sodium, potassium and cesium.

The preparation method for compound **III-0-B**, also comprises the steps bellow: carrying out a cyclization reaction on the intermediate compound represented by formula (**III-0-L**):

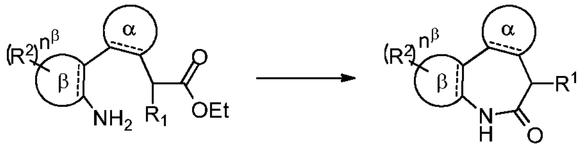

III-0-L III-0-C .

The preparation method for compound **III-0-B**, also comprises the steps bellow: carrying out a Suzuki coupling reaction on the intermediate compound represented by formula (**III-0-J**) and the intermediate compound represented by formula (**III-0-K**):

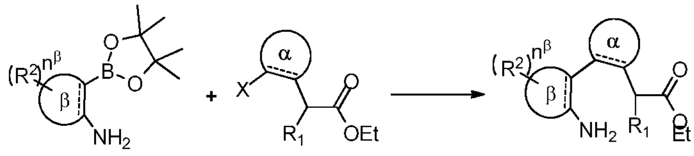

III-0-J III-0-K III-0-L

.

The present invention also provides a preparation method for the nitrogenous macrocyclic compound represented by formula (**III-0-D**), comprising: carrying out a cyclization reaction on the intermediate compound represented by formula (**III-0-E**);

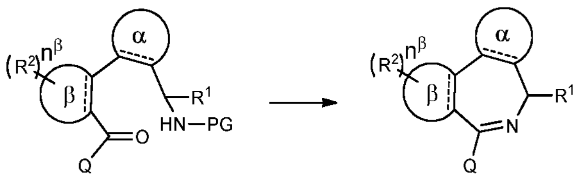

III-0-E III-0-D ;

wherein, the substituents are as described above.

Wherein, the cyclization reaction is carried out by using conditions and parameters that commonly used in conventional such reactions in the art. The present invention preferably to be carried out by treatment with trifluoroacetic acid in dichloroethane and at room temperature.

The preparation method for the nitrogenous macrocyclic compound represented by formula (**III-0-D**), also comprises the steps bellow: carrying out Suzuki coupling reaction on the intermediate compound represented by formula (**III-0-F**) and the intermediate compound represented by formula (**III-0-G**):

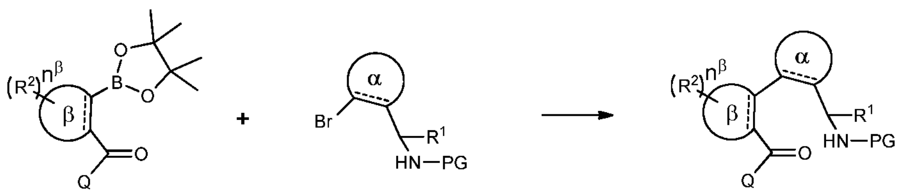

III-0-F III-0-G III-0-E ;

wherein, the substituents are as described above.

The Suzuki coupling reaction may be carried out by using conditions and parameters that commonly used in conventional such reaction in the art. The present invention is preferably to be carried out in a solvent with the presence of a palladium catalyst and a base; or promoted by microwave radiation.

Wherein, the solvent is a conventional solvent that commonly used in Suzuki coupling reaction in the art, including but not limited to 1,4-dioxane, acetonitrile, water or the mixture thereof.

The reaction temperature of the Suzuki coupling reaction is the conventional reaction temperature for such reaction in the art, e.g., 80°C-110°C.

The palladium catalyst is a conventional catalyst for Suzuki coupling reaction in the art, including but not limited to tetrakis(triphenylphosphosphine)palladium, tris(dibenzylideneacetone)palladium(0) (0), or 1,1'-bis(diphenylphosphino)ferrocene] dichloropalladium (II).

The palladium catalyst also may, before catalyzation, coordinate with conventional ligands commonly used for Suzuki coupling reaction in the art. The ligand includes but not limited to 2-(Dicyclohexylphosphino)-2',4',6'-triisopropyl-1,1'-biphenyl(XPhos).

The base is conventional base for Suzuki coupling reaction in the art, includes but not limited to carbonates or phosphates of sodium, potassium and cesium.

In the present invention, based on the mechanism of the Suzuki coupling reaction, the reactant of the above reaction, which is the intermediate compound represented by formula **(III-0-F),** may be replaced by a boronic acid or other boric acid ester derivative thereof.

The preparation method for compound **III-0-D**, also may comprise the steps below: carrying out a Suzuki

coupling reaction on the intermediate compound represented by the formula (**III-0-H**) with the intermediate compound represented by formula (**III-0-I**):

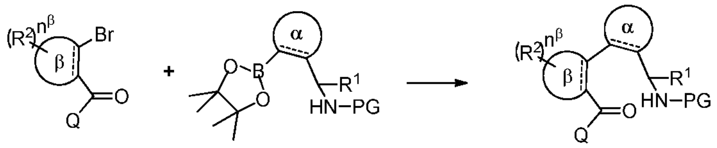

III-0-H III-0-I III-0-E .

The present invention also provides the intermediate compounds represented by formula (III-0-C), (**III-0-E**) or (III-0-F),

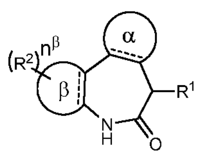

III-0-C

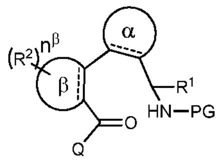

III-0-E

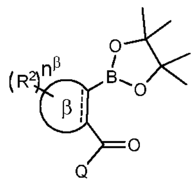

III-0-F ;

wherein, all of the substituents are as described above.

The present invention also provides the intermediate compounds represented by formula (**III-0-H**), (**III-0-J**) or (**III-0-L**),

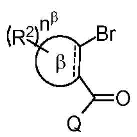

III-0-H

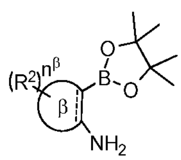

III-0-J

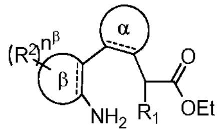

III-0-L ;

wherein, all of the substituents are as described above.

The present invention also provides a preparation method for the nitrogenous macrocyclic compound represented by formula (**III-A**), comprising: carrying out an amide reduction on the intermediate compound represented by the formula (**III-B**);

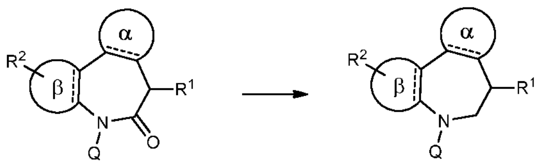

III-B III-A ;

wherein, all of the substituents are as described above.

Wherein, the amide reduction may be carried out by using conditions and parameters that commonly used in conventional amide reduction in the art, the present invention preferably to be carried out by treatment with borane in tetrahydrofuran and at room temperature.

The present invention also provides a preparation method for the nitrogenous macrocyclic compound represented by formula (**III-B**), comprising: carrying out an Ulmann coupling reaction on the intermediate compound represented by formula (**III-C**) and Q';

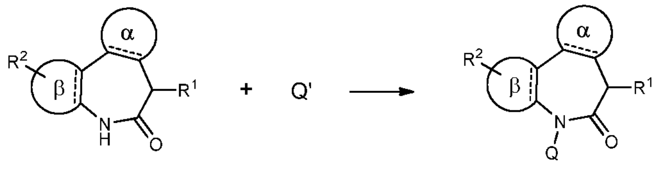

III-C III-B ;

wherein, all of the substituents are as described above, Q' is the corresponding halide on the reaction site of the structural fragment Q;

The Ullmann coupling reaction is carried out by using conditions and parameters that commonly used in conventional such reactions in the art. The present invention is preferably to be carried out in solvent, in the presence of a palladium catalyst and a base; or promoted by microwave irradiation.

Wherein, the solvent is a conventional solvent that commonly used in Ullmann coupling reaction in the art, including but not limited to 1,4-dixane, acetonitrile, water or a mixture thereof.

The reaction temperature of the Ullmann coupling reaction is the conventional reaction temperature in the art, e.g., 80°C-110°C.

The copper catalyst is a conventional catalyst for Ullmann coupling reaction in the art, including but not limited to cuprous iodide.

The copper catalyst also may, before catalyzation, coordinate with conventional ligands commonly used for Ullmann coupling reaction in the art. The ligand includes but not limited to N1,N2-dimethylethane-1,2-diamine.

The base is a conventional base for Suzuki coupling reaction in the art, includes but limited to carbonates or phosphates of sodium, potassium and cesium.

The preparation method for compound **III-B**, also comprises the steps bellow: carrying out a cyclization reaction on the intermediate compound represented by formula (**III-L**):

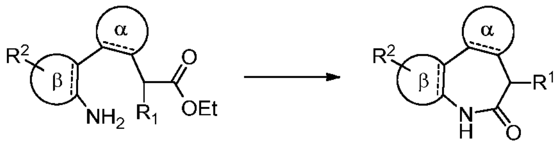

III-L III-C .

The preparation method for compound **III-B**, also comprises the steps bellow: carrying out a Suzuki coupling reaction on the intermediate compound represented by formula (**III-0-J**) and the intermediate compound represented by formula (**III-K**):

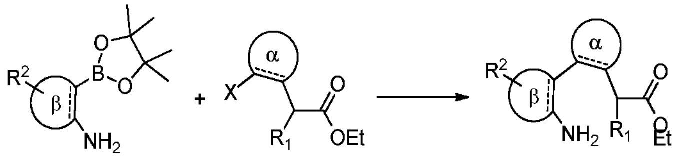

III-J III-K III-L .

The preparation method for compound **III-B**, also comprises the steps bellow: carrying out a Suzuki coupling reaction on the intermediate compound represented by formula (**III-J**) and the intermediate compound represented by formula (**III-K**):

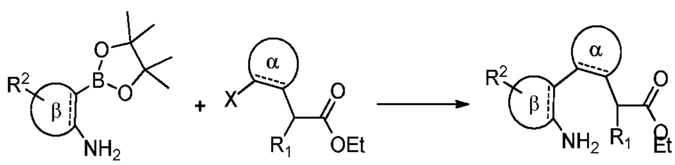

III-J III-K III-L .

The present invention also provides a preparation method for the nitrogenous macrocyclic compound represented by formula (**III-D**), comprising: carrying out a cyclization reaction on the intermediate compound represented by formula (**III-E**);

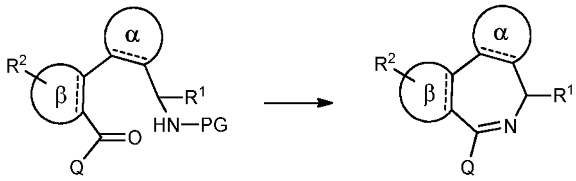

III-E III-D ;

wherein, the substituents are as described above.

Wherein, the cyclization reaction is carried out by using conditions and parameters that commonly used in conventional such reactions in the art. The present invention preferably to be carried out by treatment with trifluoroacetic acid in dichloroethane and at room temperature.

The preparation method for the nitrogenous macrocyclic compound represented by formula (**III-D**), also comprises the steps bellow: carrying out Suzuki coupling reaction on the intermediate compound represented by formula (**III-F**) and the intermediate compound represented by formula (**III-G**):

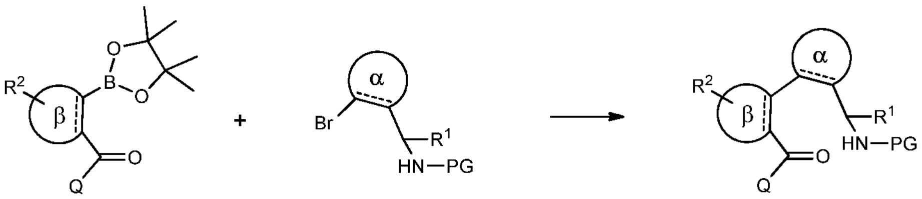

III-F III-G III-E ;

wherein, the substituents are as described above.

The Suzuki coupling reaction may be carried out by using conditions and parameters that commonly used in conventional such reaction in the art. The present invention is preferably to be carried out in a solvent with the presence of a palladium catalyst and a base; or promoted by microwave radiation.

Wherein, the solvent is a conventional solvent that commonly used in Suzuki coupling reaction in the art, including but not limited to 1,4-dioxane, acetonitrile, water or the mixture thereof.

The reaction temperature of the Suzuki coupling reaction is the conventional reaction temperature for such reaction in the art, e.g., 80°C-110°C.

The palladium catalyst is a conventional catalyst for Suzuki coupling reaction in the art, including but not limited to tetrakis(triphenylphosphosphine)palladium, tris(dibenzylideneacetone)palladium(0) (0), or [1,1'-bis(diphenylphosphino)ferrocene] dichloropalladium (II).

The palladium catalyst also may, before catalyzation, coordinate with conventional ligands commonly used for Suzuki coupling reaction in the art. The ligand includes but not limited to 2-(Dicyclohexylphosphino)-2',4',6'-triisopropyl-1,1'-biphenyl(XPhos).

The base is conventional base for Suzuki coupling reaction in the art, includes but not limited to carbonates or phosphates of sodium, potassium and cesium.

In the present invention, based on the mechanism of the Suzuki coupling reaction, the reactant of the above reaction, which is the intermediate compound represented by formula (**III-F**), may be replaced by a boronic acid or other boric acid ester derivative thereof.

The preparation method for compound **III-D**, also may comprise the steps below: make carrying out a Suzuki coupling reaction on the intermediate compound represented by the formula (**III-H**) with the intermediate compound represented by formula (**III-I**):

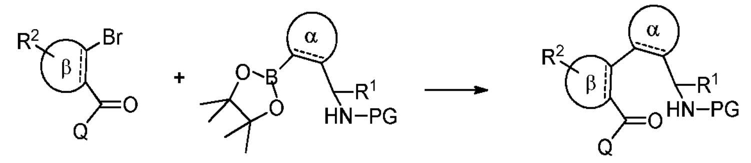

III-H III-I III-E .

The present invention also provides the intermediate compounds represented by formula (**III-C**), (**III-E**), (**III-F**) or (**III-G**),

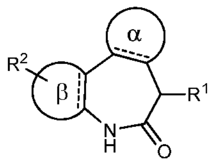

III-C

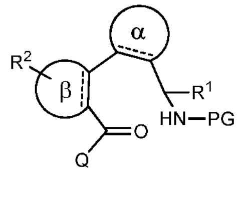

III-E

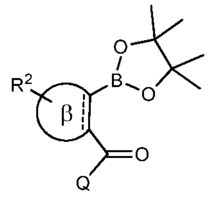

III-F

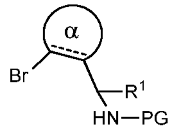

III-G ;

wherein, all of the substituents are as described above.

The present invention also provides the intermediate compounds represented by formula (III-H), (**III-I**), (**III-J**), (**III-K**) or (**III-L**),

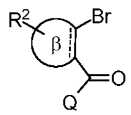

III-H

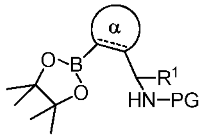

III-I

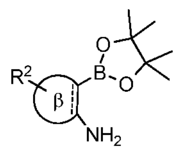

III-J

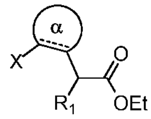

III-K

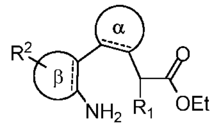

III-L ;

wherein, all of the substituents are as described above.

In the present invention, the preparation method for the nitrogeneous microcyclic compound represented by formula **III** also preferably comprises the reaction route as follows:

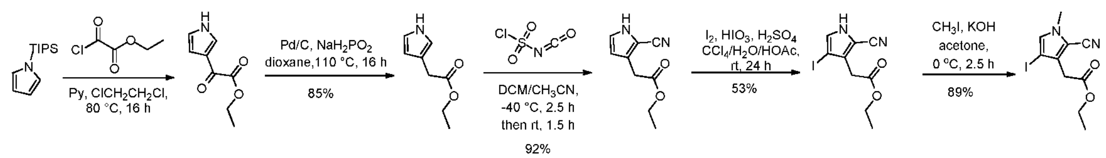


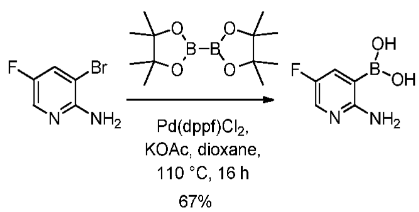


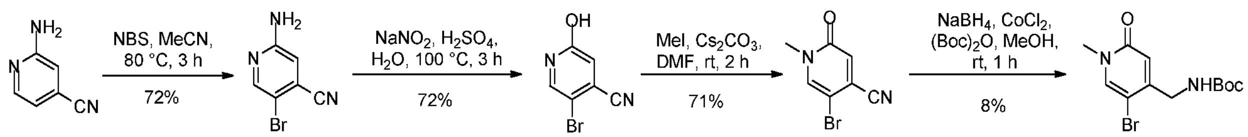

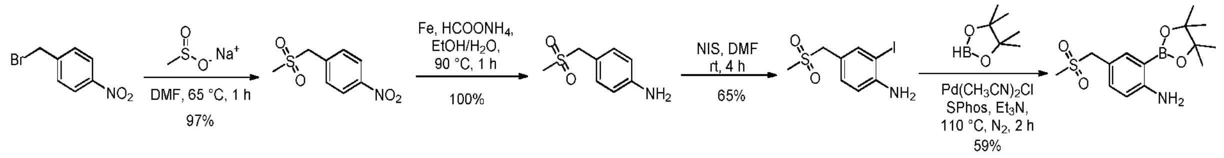


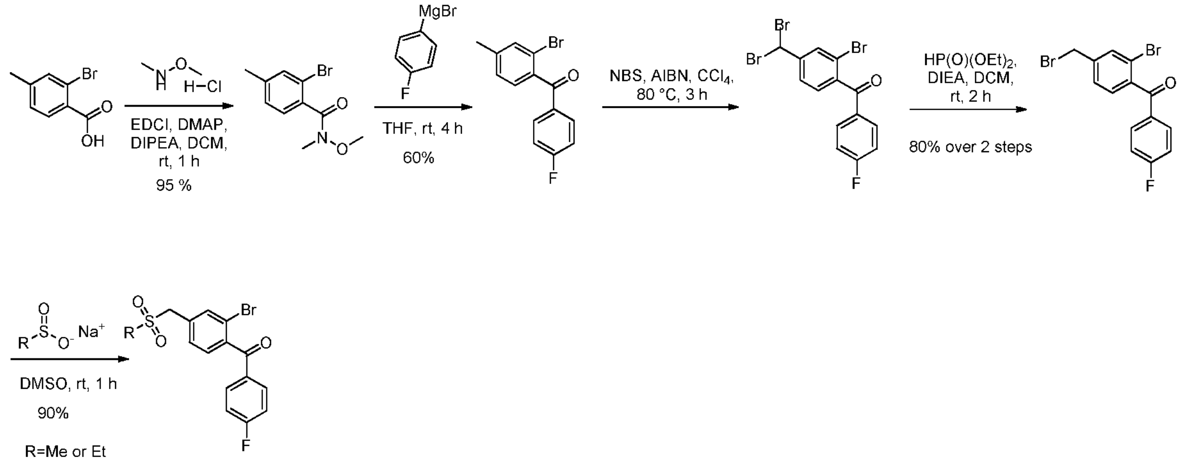


According to the preparation methods disclosed by the present invention, the man skilled in the art may use the same principles and methods to prepare the specific compounds relateed to the compound represented by the general formula **III**.

The present invention also provides a use of the nitrogeneous macrocyclic compound represented by formula **III-0,** the tautomer thereof, the optical isomer thereof, the hydrate, the solvate, the pharmaceutical acceptable salt thereof or the prodrug thereof in manufacturing a bromodomain inhibitor.

The present invention also provides a use of the nitrogeneous macrocyclic compound represented by formula **III-0,** the tautomer thereof, the optical isomer thereof, the hydrate, the solvate, the pharmaceutical acceptable salt thereof or the prodrug thereof in manufacturing a medicament for treating and/or preventing a disease requiring modulation of the binding ability of bromodomain and acetylated protein for treating and/or preventing.

The diseases require modulation of the binding ability of bromodomain and acetylated protein for treating and/or preventing are, for example, tumor (for another example cancer), pulmonary disease, inflammatory disease or autoimmune disease, for another example (includes but not limited to) acoustic neuroma, acute leukemia, acute lymphocytic leukemia, acute myeloid leukemia (monocytic, myeloblastic, adenocarcinoma, angiosarcoma, astrocytoma, myelomonocytic and promyelocytic), acute t-cell leukemia, basal cell carcinoma, cholangiocarcinoma, bladder cancer, brain cancer, breast cancer, bronchial cancer, cervical cancer, chondrosarcoma, chordoma, choriocarcinoma, chronic leukemia, chronic lymphocytic leukemia, chronic myeloid (granulocytic) leukemia, chronic myelogenous leukemia, colon cancer, colorectal cancer, craniopharyngioma, cystadenocarcinoma, diffuse large B-cell lymphoma, dysproliferativechanges (dysplasia and metaplasis), embryonic cancer, endometrial cancer, endothelial sarcoma, ependymoma, epithelial cancer, erythroleukemia, esophageal cancer, estrogen receptor-positive breast cancer, essential thrombocytosis, Ewing's sarcoma, Fibrosarcoma, follicular lymphoma, germ cell testicular cancer, glioma, glioblastoma, glioma, heavy chain disease, hemangioblasts , liver cancer, hepatocellular carcinoma, hormone-insensitive prostate cancer, leiomyosarcoma, leukemia, liposarcoma, lung carcinoma, lymphangioendotheliosarcoma, lymphatic sarcoma, lymphoblastic leukemia, lymphoma (Hodgkin and non-Hodgkin), bladder, breast, colon, lung, ovary, pancreas, prostate, skin and uterus malignant tumors and hyperproliferative disorders, T- Cell or B-cell derived lymphoid malignancy, leukemia, lymphoma, medullary carcinoma, medulloblastoma, melanoma, meningioma, mesothelioma, multiple myeloma, myeloid leukemia, myeloma, myxosarcoma, Neuroblastoma, NUT midline cancer (NMC), non-small cell lung cancer, oligodendroglioma, oral cancer, osteogenic sarcoma, ovarian cancer, pancreatic cancer, papillary adenocarcinoma, papillary carcinoma, pineal gland Tumor, polycythemia vera, prostate cancer, rectal cancer, renal cell carcinoma, retinoblastoma, rhabdomyosarcoma, sarcoma, sebaceous gland cancer, seminoma, skin cancer, small cell lung cancer, solid tumor (cancer and sarcoma), Small cell lung cancer, gastric cancer, squamous cell carcinoma, synovial tumor, sweat adenoma, thyroid cancer, primary macroglobulinemia, testicular tumor, uterine cancer, and nephroblastoma etc.

The pulmonary disease, inflammatory disease or autoimmune disease preferably includes: Addison's disease, acute gout, ankylosing spondylitis, asthma, atherosclerosis, Behcet's disease, bullous skin disease, chronic obstructive pulmonary disease (COPD), Crohn's disease, dermatitis, eczema, giant cell arteritis, glomerulonephritis, hepatitis, hypopituitaritis, inflammatory bowel disease, Kawasaki disease, lupus nephritis, multiple sclerosis, myocarditis, muscle

Inflammation, nephritis, organ transplant rejection, osteoarthritis, pancreatitis, pericarditis, nodular polyarteritis, localized pneumonia, primary biliary cirrhosis, psoriasis, psoriatic arthritis, rheumatoid Arthritis, scleritis, sclerosing cholangitis, sepsis, systemic lupus erythematosus, high-angstrom arteritis, toxic shock, thyroiditis, type I diabetes, ulcerative colitis, uveitis, vitiligo, vasculature Inflammation and Wegener's granuloma etc.

The present invention further provides a use of the nitrogeneous macrocyclic compound represented by formula **III-0**, the tautomer thereof, the optical isomer thereof, the hydrate, the solvate, the pharmaceutical acceptable salt thereof or the prodrug thereof in manufacturing a medicament for treating and/or preventing pulmonary disease, inflammatory disease or autoimmune diseases.

The pulmonary disease, inflammatory disease or autoimmune disease preferably includes: Addison's disease, acute gout, ankylosing spondylitis, asthma, atherosclerosis, Behcet's disease, bullous skin disease, chronic obstructive pulmonary disease (COPD), Crohn's disease, dermatitis, eczema, giant cell arteritis, glomerulonephritis, hepatitis, hypopituitaritis, inflammatory bowel disease, Kawasaki disease, lupus nephritis, multiple sclerosis, myocarditis, muscle Inflammation, nephritis, organ transplant rejection, osteoarthritis, pancreatitis, pericarditis, nodular polyarteritis, localized pneumonia, primary biliary cirrhosis, psoriasis, psoriatic arthritis, rheumatoid Arthritis, scleritis, sclerosing cholangitis, sepsis, systemic lupus erythematosus, high-angstrom arteritis, toxic shock, thyroiditis, type I diabetes, ulcerative colitis, uveitis, vitiligo, vasculature Inflammation and Wegener's granuloma etc.

The present invention also provides a pharmaceutical composition, comprising the nitrogenous macrocyclic compound represented by formula **III-0**, the tautomer thereof, the optical isomer thereof, the hydrate, the solvate, the pharmaceutical acceptable salt thereof or the prodrug thereof, as well as at least one pharmaceutical excipient. The percentage of mass of the nitrogenous macrocyclic compound represented by formula **III-0**, the tautomer thereof, the optical isomer thereof, the hydrate, the solvate, the pharmaceutical acceptable salt thereof or the prodrug thereof is 0.1%-99.99%, the mass percentage refers to the nitrogenous macrocyclic compound represented by formula **III-0**, the tautomer thereof, the optical isomer thereof, the hydrate, the solvate, the pharmaceutical acceptable salt thereof or the prodrug thereof as a percentage of the total mass of the pharmaceutical composition. The sum of the mass percentage of the nitrogenous macrocyclic compound represented by formula III-0, the tautomer thereof, the optical isomer thereof, the hydrate, the solvate, the pharmaceutical acceptable salt thereof or the prodrug thereof and the pharmaceutical excipient is 100%. The pharmaceutical excipient is selected by the route of administration and the function characteristics, and generally is a filler, a diluent, a binder, a wetting agent, a disintegrant, a lubricant, an emulsifier or a suspending agent.

The present invention also provides a method for treating a disease or condition in a subject in need thereof, comprising: administrating to the subject a therapeutically effective amount of the nitrogenous macrocyclic compound represented by formula **III-0**, the tautomer thereof, the optical isomer thereof, the hydrate, the solvate, the pharmaceutical acceptable salt thereof or the prodrug thereof, wherein the disease or condition is selected from the diseases described above, requiring for modulations of bromodomain and acetylated protein for treating and/or preventing, pulmonary disease, inflammatory disease or autoimmune disease (the definitions are as defined above).

The present invention also provides a use of the nitrogenous macrocyclic compound represented by formula **III**, the tautomer thereof, the optical isomer thereof, the hydrate, the solvate, the pharmaceutical acceptable salt thereof or the prodrug thereof in manufacturing a bromodomain inhibitor.

The present invention also provides a use of the nitrogenous macrocyclic compound represented by formula **III**, the tautomer thereof, the optical isomer thereof, the hydrate, the solvate, the pharmaceutical acceptable salt thereof or the prodrug thereof in manufacturing a medicament for treating and/or preventing a disease requiring modulation of the binding ability of bromodomain and acetylated protein for treating and/or preventing.

The diseases requiring modulation of the binding ability of bromodomain and acetylated protein for treating/ preventing preferably include acoustic neuroma, acute leukemia, acute lymphocytic leukemia, acute myeloid leukemia (monocytic, myeloblastic, adenocarcinoma, angiosarcoma, astrocytoma, myelomonocytic and promyelocytic), acute t-cell leukemia, basal cell carcinoma, cholangiocarcinoma, bladder cancer, brain cancer, breast cancer, bronchial cancer, cervical cancer, chondrosarcoma, chordoma, choriocarcinoma, chronic leukemia, chronic lymphocytic leukemia, chronic myeloid (granulocytic) leukemia, chronic myelogenous leukemia, colon cancer, colorectal cancer, craniopharyngioma, cystadenocarcinoma, diffuse large B-cell lymphoma, dysproliferativechanges (dysplasia and metaplasis), embryonic cancer, endometrial cancer, endothelial sarcoma, ependymoma, epithelial cancer, erythroleukemia, esophageal cancer, estrogen receptor-positive breast cancer, essential thrombocytosis, Ewing's sarcoma, Fibrosarcoma, follicular lymphoma, germ cell testicular cancer, glioma, glioblastoma, glioma, heavy chain disease, hemangioblasts , liver cancer, hepatocellular carcinoma, hormone-insensitive prostate cancer, leiomyosarcoma, leukemia, liposarcoma, lung carcinoma, lymphangioendotheliosarcoma, lymphatic sarcoma, lymphoblastic leukemia, lymphoma (Hodgkin and non-Hodgkin), bladder, breast, colon, lung, ovary, pancreas, prostate, skin and uterus malignant tumors and hyperproliferative disorders, T- Cell or B-cell derived lymphoid malignancy, leukemia, lymphoma, medullary carcinoma, medulloblastoma, melanoma, meningioma, mesothelioma, multiple myeloma, myeloid leukemia, myeloma, myxosarcoma, Neuroblastoma, NUT midline cancer (NMC), non-small cell lung cancer, oligodendroglioma, oral cancer, osteogenic sarcoma, ovarian cancer, pancreatic cancer, papillary adenocarcinoma, papillary carcinoma, pineal gland Tumor, polycythemia vera, prostate

cancer, rectal cancer, renal cell carcinoma, retinoblastoma, rhabdomyosarcoma, sarcoma, sebaceous gland cancer, seminoma, skin cancer, small cell lung cancer, solid tumor (cancer and sarcoma), Small cell lung cancer, gastric cancer, squamous cell carcinoma, synovial tumor, sweat adenoma, thyroid cancer, primary macroglobulinemia, testicular tumor, uterine cancer, and nephroblastoma etc.

The present invention further provides a use of the nitrogeneous macrocyclic compound represented by formula III, the tautomer thereof, the optical isomer thereof, the hydrate, the solvate, the pharmaceutical acceptable salt thereof or the prodrug thereof in manufacturing a medicament for treating and/or preventing pulmonary disease, inflammatory disease or autoimmune diseases.

The pulmonary disease, inflammatory disease or autoimmune disease preferably includes: Addison's disease, acute gout, ankylosing spondylitis, asthma, atherosclerosis, Behcet's disease, bullous skin disease, chronic obstructive pulmonary disease (COPD), Crohn's disease, dermatitis, eczema, giant cell arteritis, glomerulonephritis, hepatitis, hypopituitaritis, inflammatory bowel disease, Kawasaki disease, lupus nephritis, multiple sclerosis, myocarditis, muscle Inflammation, nephritis, organ transplant rejection, osteoarthritis, pancreatitis, pericarditis, nodular polyarteritis, localized pneumonia, primary biliary cirrhosis, psoriasis, psoriatic arthritis, rheumatoid Arthritis, scleritis, sclerosing cholangitis, sepsis, systemic lupus erythematosus, high-angstrom arteritis, toxic shock, thyroiditis, type I diabetes, ulcerative colitis, uveitis, vitiligo, vasculature Inflammation and Wegener's granuloma etc.

The present invention also provides a pharmaceutical composition, comprising the nitrogenous macrocyclic compound represented by formula **III**, the tautomer thereof, the optical isomer thereof, the hydrate, the solvate, the pharmaceutical acceptable salt thereof or the prodrug thereof, as well as at least one pharmaceutical excipient. The percentage of mass of the nitrogenous macrocyclic compound represented by formula **III**, the tautomer thereof, the optical isomer thereof, the hydrate, the solvate, the pharmaceutical acceptable salt thereof or the prodrug thereof is 0.1%-99.99%, the mass percentage refers to the nitrogenous macrocyclic compound represented by formula **III**, the tautomer thereof, an optical isomer thereof, the hydrate, the solvate, the pharmaceutical acceptable salt thereof or the prodrug thereof as a percentage of the total mass of the pharmaceutical composition. The sum of the mass percentage of the nitrogenous macrocyclic compound represented by formula **III**, the tautomer thereof, the optical isomer thereof, the hydrate, the solvate, the pharmaceutical acceptable salt thereof or the prodrug thereof and the pharmaceutical excipient is 100%. The pharmaceutical excipient is selected by the route of administration and the function characteristics, and generally is a filler, a diluent, a binder, a wetting agent, a disintegrant, a lubricant, an emulsifier or a suspending agent.

The present invention also provides a use of the pharmaceutical composition in manufacturing a bromodomain inhibitor.

The present invention also provides a use of the pharmaceutical composition in manufacturing a medicament for treating and/or preventing a disease requiring modulation of the binding ability of bromodomain and acetylated protein for treating and/or preventing.

The diseases requiring modulation of the binding ability of bromodomain and acetylated protein for treating/ preventing are as described above.

The present invention also provides a method for treating a disease or condition in a subject in need thereof, comprising: administrating to the subject a therapeutically effective amount of the nitrogenous macrocyclic compound represented by formula III, the tautomer thereof, the optical isomer thereof, the hydrate, the solvate, the pharmaceutical acceptable salt thereof or the prodrug thereof, wherein the disease or condition is selected from the diseases described above, requiring for modulations of bromodomain and acetylated protein for treating/ preventing, pulmonary disease, inflammatory disease or autoimmune disease (the definitions are as defined above).

Based on the common knowledge in the art, the above preferable conditions may be arbitrarily combined to obtain preferable embodiments of the present invention.

Unless otherwise indicated, the following terms and phrases used herein are intended to have the following meanings:

The term "pharmaceutically acceptable salt" refers to a salt of the compound of the present invention that is prepared by reacting the compound having a specific substituent of the present invention with a relatively non-toxic acid or base. When the compound of the present invention contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound of the present invention contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid,

benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like (refer to Berge et al., "Pharmaceutical Salts", Journal of Pharmaceutical Science 66: 1-19 (1977*)).* Certain specific compounds of the present invention that contain both basic and acidic functional groups can be converted to any base or acid addition salt.

Preferably, through bringing the salt into contact with a base or an acid in a conventional manner, then separating the parent compound, the neutral form of the compound is thereby regenerated. The difference between the parent form of the compound and its various salt forms lies in specific physical properties, such as different solubility in a polar solvent.

"Pharmaceutically acceptable salt" used herein belongs to a derivative of the compound of the present invention, wherein, the parent compound is modified by forming a salt with an acid or a base. Examples of the pharmaceutically acceptable salt include but are not limited to an inorganic acid or organic acid salt of a basic moiety such as amine, an alkali metal salt or an organic salt of an acidic moiety such as carboxylic acid, and the like. The pharmaceutically acceptable salt includes conventional non-toxic salt or quaternary ammonium salt of the parent compound, such as a salt formed by a non-toxic inorganic acid or an organic acid. The conventional non-toxic salt includes but is not limited to the salt derived from an inorganic acid and an organic acid, wherein the inorganic acid or organic acid is selected from the group consisting of 2-acetoxybenzoic acid, 2-hydroxyethanesulfonic acid, acetic acid, ascorbic acid, benzenesulfonic acid, benzoic acid, bicarbonate, carbonic acid, citric acid, edetic acid, ethanedisulfonic acid, ethanesulfonic acid, fumaric acid, glucoheptose, gluconic acid, glutamic acid, glycolic acid, hydrobromic acid, hydrochloric acid, hydroiodide, hydroxyl, hydroxynaphthalene, isethionic acid, lactic acid, lactose, dodecylsulfonic acid, maleic acid, malic acid, mandelic acid, methanesulfonic acid, nitric acid, oxalic acid, pamoic acid, pantothenic acid, phenylacetic acid, phosphoric acid, polygalactanal acid, propionic acid, salicylic acid, stearic acid, subacetic acid, succinic acid, sulfamic acid, sulfanilic acid, sulfuric acid, tannin, tartaric acid and p-toluenesulfonic acid.

The pharmaceutically acceptable salt of the present invention can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof. Generally, non-aqueous media such as ether, ethyl acetate, ethanol, isopropanol or acetonitrile are preferred.

In addition to the salt form, the compound provided by the present invention also exists in prodrug form. The prodrug of the compound described herein is the compound that readily undergoes chemical change under physiological condition to be converted into the compound of the present invention. Additionally, the prodrug can be converted to the compound of the present invention by a chemical or biochemical method *in vivo* environment.

Certain compounds of the present invention can exist in an unsolvated form or a solvated form, including hydrated form. Generally, the solvated form is equivalent to the unsolvated form, and both are encompassed within the scope of the present invention.

The term "pharmaceutically acceptable carrier" refers to any agent or carrier medium which is capable of delivering an effective amount of the active substance of the present invention, does not interfere with the biological activity of the active substance and has no toxic side effect on the host or patient. The representative carrier includes water, oil, vegetable and mineral, cream base, lotion base, ointment base and the like. The base includes a suspending agent, a thickener, a penetration enhancer and the like. Their formulations are well known to the skilled in the cosmetic field or the topical pharmaceutical field. The additional information about the carrier can be referred to Remington: The Science and Practice of Pharmacy, 21st Ed, Lippincott, Williams & Wilkins (2005*)*, the disclosure of which is incorporated herein by reference.

The term "excipient" generally refers to a carrier, a diluent and/or a medium required for formulating an effective pharmaceutical composition.

For a medicament or a pharmacologically active agent, the term "effective amount" or "therapeutically effective amount" refers to a nontoxic but sufficient amount to achieve a desired effect of the medicament or the agent. For the oral dosage form of the present invention, an "effective amount" of the active substance in the composition refers to an amount required for achieving a desired effect when combining with another active substance in the composition. The effective amount varies from person to person and is determined depending on the age and general condition of the recipient as well as the specific active substance. The appropriate effective amount in an individual case can be determined by the skilled in the art based on routine experiment.

Certain compounds of the present invention can have an asymmetric carbon atom (optical center) or a double bond. The racemate, diastereomer, geometric isomer and individual isomer are all encompassed within the scope of the present invention.

The compound of the present invention may have a specific geometric or stereoisomeric form. The present invention contemplates all such compounds, including *cis* and *trans* isomer, (-)- and (+)-enantiomer, (*R*)- and (*S*)-enantiomer, diastereoisomer, (*D*)-isomer, (*L*)-isomer, and racemic mixture and other mixtures, for example, an enantiomer or diastereoisomer enriched mixture, all of which are encompassed within the scope of the present invention. The

substituent such as alkyl may have an additional asymmetric carbon atom. All these isomers and mixtures thereof are encompassed within the scope of the present invention.

Graphical representation used herein for racemates, ambiscalemic and scalemic or pure enantiomer compounds are from Maehr, J. Chem. Ed. 1985, 62: 114-120. *1985, 62: 114-120.* Unless otherwise stated, the absolute configuration of a stereocenter is indicated by wedge bonds and dashed bond. When the compound described herein comprises olefinic double bonds or other centers of geometric asymmetry, unless otherwise specified, they includes the E and Z geometric isomers. Likewise, all tautomeric forms are included within the scope of the invention.

The chemical formula of the present invention may exhibit tautomerism, structural isomerism, and stereoisomerism. The invention includes any interconversion or structural or stereoisomeric forms thereof, and mixtures thereof, the ability of which is not limited to any one or a mixture thereof.

Optically active (*R*)- and (*S*)-isomer, or *D* and *L* isomer can be prepared using chiral synthesis or chiral reagents or other conventional techniques. If one kind of enantiomer of certain compound of the present invention is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (for example, carbamate generated from amine).

The compound of the present invention may contain an unnatural proportion of atomic isotope at one or more than one atom(s) that constitute the compound. For example, the compound can be radiolabeled with a radioactive isotope, such as tritium ($^3H$), iodine-125 ($^{125}I$) or C-14 ($^{14}C$). All isotopic variations of the compound of the present invention, whether radioactive or not, are encompassed within the scope of the present invention.

Unless otherwise defined, the term "substituted" means one or more than one hydrogen atom(s) on a specific atom are substituted with the substituent, including deuterium and hydrogen variants, as long as the valence of the specific atom is normal and the substituted compound is stable. When the substituent is a ketone (i.e. =O), it means two hydrogen atoms are substituted. Positions on an aromatic ring can not be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When one of the variable is selected from a single bond, it means that the two groups linked by the single bond are connected directly. For example, when L in A-L-Z represents a single bond, the structure of A-L-Z is actually A-Z.

In the present invention, when the type and range of the substituent is defined in form similar to "$R_1$-$R_2$-$R_3$-", it means that the substituent as represented by "$R_1$-$R_2$-$R_3$-" is entirely bonded to the compound by $R_3$, and the $R_1$, $R_2$ $R_3$ is bonded sequentially to each other by chemical bonds; besides, "$R_1$-$R_2$-$R_3$-" also may be desfied by text description, as in the present invention, "$R_1$-$R_2$-$R_3$-" and "$R_2$-$R_3$-substituted by $R_1$" have the same meaning.

Unless otherwise specified, when a group or a bond of a substituent may be cross-linked to two atoms on a ring, such group or substituent may be bonded to any atom on the ring. When the recited group or substituent does not indicate which atom is attached to the compound included but not specified in the chemical structural formula, such group or substituent may be bonded through any of its atoms. Combinations of groups or substituents and/or variants thereof are permissible only if such combinations result in stable compounds.

Unless otherwise specified, the term "hydrocarbyl" or its hyponyms (e.g. alkyl, alkenyl, alkynyl, and aryl, etc.), by itself or as part of another substituent, refers to a linear, branched chain or cyclic hydrocarbon radical or any combination thereof. They can be fully saturated, mono- or polyunsaturated, can be mono-, di- or poly-substituted, can be monovalent, divalent or multivalent, can also include a divalent or multivalent group, have a specified number of carbon atom (for example, $C_1$-$C_{10}$ indicates 1 to 10 carbon atoms, The term "hydrocarbyl" includes, but is not limited to aliphatic hydrocarbyl and aromatic hydrocarbyl. The aliphatic hydrocarbyl includes linear and cyclic hydrocarbyl, specifically includes but not limited to alkyl, alkenyl, and alkynyl. The aromatic hydrocarbyl includes but is not limited to 6-12 membered aromatic hydrocarbyl such as phenyl, naphthyl and the like. In some embodiments, the term "hydrocarbyl" refers to a linear or branched group or a combination thereof which can be fully saturated, mono- or polyunsaturated, and can include a divalent or multivalent group. Examples of the saturated hydrocarbyl group include, but are not limited to, methyl, ethyl, *n*-propyl, isopropyl, *n*-butyl, *tert*-butyl, isobutyl, *sec*-butyl, cyclohexyl, (cyclohexyl)methyl, cyclopropylmethyl, and the homolog or isomer of *n*-amyl, *n*-hexyl, *n*-heptyl, *n*-octyl and other atom groups. The unsaturated hydrocarbyl has one or more than one double or triple bonds. Examples of the unsaturated alkyl include but are not limited to, vinyl, 2-propenyl,

butenyl, crotyl, 2-isopentenyl, 2-(butadienyl), 2,4-pentadienyl, 3-(1,4-pentadienyl), ethynyl, 1- and 3-propynyl, 3-butynyl, and more higher homologs and isomers.

Unless otherwise specified, the term heterohydrocarbyl, heterocyclic, hydrocyclyl-hetero, cyclohetero, hetero heteroalkyl, hetero heterocylic are refer to a specific group contains heteroatom or heterogroup, heteroatom or heterogroup comprise but not limited to N, NH, substituted or protected NH, O, S, S(=O), $S(=O)_2$, C(=O),C(=O)O, for cyclic group, heteroatom or heterogroup may located within or outside the ring system(eg. cyclopropylsulfone or cyclopropyl), wherein the so-called heterohydrocarbyl group or heterocyclic group is bonded to the rest of the molecule through a carbon atom, that is, the heteroatom could located any position of the group (except of the position where the group is attached to the rest of the molecule); the so-called hydrocyclyl-hetero and cyclohetero are attached to the other part of the molecule by heteroatom, that is, the heteroatom located in the place where the group attached to the rest of the molecule; the so-called heterohydrocarbyl-hetero group and heterocyclic hetero groupe is attached to the rest part of the molecule by a heterogroup, where the heteroatom may be located in any position of the group (including the position where the group has been attached to the rest of the molecule).

Unless otherwise specified, the term "heterohydrocarbyl" or its hyponyms (such as heteroalkyl, heteroalkenyl, heteroalkynyl, and heteroaryl, etc.), by itself or as part of another substituent, refers to a stable linear, branched or cyclohydrocarbyl or any combination thereof, which has a specified number of carbon atoms and at least one heteroatom. In some embodiments, the term "heteroalkyl" by itself or in combination with another term refers to a stable linear chain, branched hydrocarbon radical or a combination thereof which has a specified number of carbon atoms and at least one heteroatom. In a specific embodiment, a heteroatom is selected from B, O, N and S, wherein nitrogen and sulfur atoms are optionally oxidized and the nitrogen atom is optionally quaternized. The heteroatom or heteroatom group can be located at any interior position of a heterohydrocarbyl, including the position where the hydrocarbyl attaches to the rest part of the molecule. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-CH_2-CH=N-OCH_3$ and $-CH=CH-N(CH_3)-CH_3$. Up to two consecutive heteroatoms can be present, such as, $-CH_2-NH-OCH_3$.

Unless otherwise specified, the terms "alkoxy", "alkylamino" and "alkylthio" (or thioalkyl) are used by the conventional meaning and refer to an alkyl group connected to the rest part of the molecule via an oxygen atom, an amino or a sulfur atom respectively.

Unless otherwise specified, the term "cyclohydrocarbyl", "heterocyclohydrocarbyl" or its hyponyms (such as aryl, heteroaryl, cycloalkyl, heterocycloalkyl, cycloalkenyl, heterocycloalkenyl, cycloalkynyl, heterocycloalkynyl, etc.) by itself or in combination with another term refers to cyclized "hydrocarbyl" or "heterohydrocarbyl". Examples of the cycloalkyl include, but are not limited to, cyclopentyl, cyclohexyl, 1-cyclohexenyl, 3-cyclohexenyl, cycloheptyl and the like. Non-limiting examples of heterocycloalkyl include 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydro-thiophen-2-yl, tetrahydro-thiophen-3-yl, 1-piperazinyl and 2-piperazinyl.

Unless otherwise specified, the term "halo" or "halogen" by itself or as part of another substituent refers to fluorine, chlorine, bromine or iodine atom. Furthermore, the term "haloalkyl" is meant to include monohaloalkyl and polyhaloalkyl. For example, the term "halo($C_1$-$C_4$)alkyl" is meant to include, but not limited to, trifluoromethyl, 2,2,2-trifluoroethyl, 4-chlorobutyl, 3-bromopropyl and the like.

Unless otherwise specified, the term "aryl" refers to a polyunsaturated aromatic substituent, can be mono-, di- or poly-substituted, can be a monovalent, divalent or multivalent, can be a single ring or a multiple ring (e.g. one to three rings; wherein at least one ring is aromatic), which are fused together or connected covalently. The term "heteroaryl" refers to an aryl(or ring) containing one to four heteroatoms. In an illustrative example, the heteroatom is selected from B, O, N and S, wherein nitrogen and sulfur atoms are optionally oxidized and nitrogen atom is optionally quaternized. A heteroaryl may attach to the rest part of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furanyl, thienyl, pyridyl, pyrimidinylbenzothiazolyl, purinyl, benzimidazolyl, indolyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl. The substituent of any of the above aryl and heteroaryl ring system is selected from the acceptable substituent described below.

Unless otherwise specified, when combined with other terms (such as aryloxy, arylthio, arylalkyl), the aryl includes the aryl and heteroaryl ring as defined above. Thus, the term "aralkyl" is meant to include the group (e.g. benzyl, phenethyl, pyridylmethyl, etc.) where an aryl is attached to an alkyl, including an alkyl where the carbon atom (e.g. methylene) has been replaced by an atom such as oxygen, for example, phenoxymethyl, 2-pyridyloxy, 3-(1-naphthyloxy)propyl, and the like.

Unless otherwise specified, the term "ring" refers to a substituted or unsubstituted cycloalkyl, heterocycloalkyl,

cycloalkenyl, heterocycloalkenyl, cycloalkynyl, heterocycloalkynyl, aryl or heteroaryl. The so-called ring includes a single ring, a ring assembly, a spiral ring, a fused ring or a bridged ring. The number of the atom on the ring is usually defined as the member number of the ring, for example, a "5-7 membered ring" means that 5 to 7 atoms are arranged on a ring. Unless otherwise specified, the ring optionally contains 1 to 3 heteroatoms. Therefore, a "5-7 membered ring" includes, for example, phenyl, pyridinyl and piperidinyl; on the other hand, the term "5-7 membered heterocycloalkyl ring" includes pyridyl and piperidinyl, but excluding phenyl. The term "ring" also includes a ring system containing at least one ring, wherein each ring independently meets the above definition.

Unless otherwise specified, the term "heteroatom" comprise other atoms except of carbon (C) and hydrogen (H), eg. oxygen (O), nitrogen (N), Sulfur (S), silicon (Si), germanium (Ge), aluminium (Al) and boron (B) etc.

Unless otherwise specified, the term "leaving group" refers to a functional group or atom which can be replaced by another functional group or atom through a substitution reaction (such as affinity substitution reaction). For example, representative leaving groups include triflate; chlorine, bromine and iodine; sulfonate group, such as mesylate, tosylate, *p*-bromobenzenesulfonate, *p*-toluenesulfonates and the like; acyloxy, such as acetoxy, trifluoroacetoxy and the like.

Unless otherwise specified, the term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl(e.g. acetyl, trichloroacetyl or trifluoroacetyl); alkoxycarbonyl, such as *tert*-butoxycarbonyl(Boc); arylmethoxycarbonyl such as benzyloxycarbonyl(Cbz) and 9-fluorenylmethoxycarbonyl(Fmoc); arylmethyl such as benzyl(Bn), trityl(Tr), 1,1-bis-(4'-methoxyphenyl)methyl; silyl such as trimethylsilyl(TMS) and tert-butyldimethylsilyl(TBS) and the like. The term "hydroxy protecting group" refers to a protecting group suitable for blocking the side reaction on hydroxy. Representative hydroxy protecting groups include, but are not limited to: alkyl such as methyl, ethyl and *tert*-butyl; acyl such as alkanoyl(e.g. acetyl); arylmethyl such as benzyl(Bn), *p*-methoxybenzyl(PMB), 9-fluorenylmethyl(Fm), and diphenylmethyl(benzhydryl, DPM); silyl such as trimethylsilyl(TMS) and *tert*-butyl dimethyl silyl(TBS) and the like.

Unless otherwise specified, the term haloalkyl is for example, and is not limited to, trifluoromethyl, trichloromethyl, pentafluoroethyl, and pentachloroethyl. The term "alkoxy" represents any alkyl defined above having a specified number of carbon atoms attached by an oxygen bridge. Unless otherwise specified, $C_{1-6}$ alkoxy includes $C_1$, $C_2$, $C_3$, $C_4$, $C_5$ and $C_6$ alkoxy. Examples of alkoxy include, but not limited to methoxy, ethoxy, *n*-propoxy, isopropoxy, *n*-butoxy, *sec*-butoxy, *tert*-butoxy, *n*-pentyloxy and *S*-pentoxy. "cycloalkyl" comprises saturated cyclic groups such as cyclopropyl, cyclobutyl or cyclopentyl. 3-7 membered cycloalkyl comprise $C_3$, $C_4$, $C_5$, $C_6$ and $C_7$ cycloalkyl. "Alkenyl" comprises linear or branched hydrocarbon chains, wherein one or more carbon-carbon double bonds, such as vinyl and propylene groups, are present at any stable site on the chain.

Unless otherwise specified, the term "halogen" is refer to fluorine, chlorine, bromine and iodine.

Unless otherwise specified, the term "heterocycle" or "heterocyclo" refers to a stable monocyclic, bicyclic or tricyclic ring containing a heteroatom or a heteroatom group, which can be saturated, partially unsaturated or unsaturated (aromatic) and can contain carbon atoms and 1, 2, 3 or 4 ring heteroatoms independently selected from N, O and S, wherein any of the above heterocycle can be fused to a benzene ring to form a bicyclic ring.

Unless otherwise specified, examples of the heterocyclic compound include, but are not limited to: acridinyl, azocinyl, benzimidazolyl, benzofuranyl, benzomercaptofuranyl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-*b*]tetrahydrofuranyl, furanyl, furazanyl, imidazolidinyl, imidazolinyl, imidazolyl, 1*H*-indazolyl, indolenyl, indolinyl, indolizinyl, indolyl, 3*H*-indolyl, isobenzofuranyl, isoindolyl, isoindolinyl, isoquinolinyl, isothiazolyl, isoxazolyl, methylenedioxyphenyl, morpholinyl, naphthyridinyl, octahydro-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, hydroxindolyl, pyrimidinyl, phenanthridinyl, phenanthrolinyl, phenazine, phenothiazine, benzoxanthinyl, phenoloxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyrido-oxazolyl, pyrido-imidazolyl, pyrido-thiazolyl, pyridinyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thienyl, thieno-oxazolyl, thieno-thiazolyl, thieno-imidazolyl, thienyl, triazinyl, 1*H*-1,2,3-triazolyl, 2*H*-1,2,3-triazolyl, 1*H*-1,2,4-triazolyl, 4*H*-1,2,4-triazolyl,and xanthenyl. Also included are fused-ring compounds and spiro compounds.

Unless otherwise specified, the compound of the present invention can be prepared by a variety of synthetic methods well known to the skilled in the art, including the following enumerative embodiment, the embodiment formed by the following enumerative embodiment in combination with other chemical synthesis methods and the equivalent replacement well known to the skilled in the art. The preferred embodiment includes, but is not limited to the embodiment of the present invention.

Unless otherwise specified, the optimum reaction conditions and reaction durations for each individual step may vary depending on the particular reactant employed and the substituents present in the reactants employed. Unless otherwise specified, solvents, temperature and other conditions may be easily selected by the man skilled in the art. Specified methods are provided in the synthesis embodiments part. The reactions may be post-treated in conventional manners, for example by removing the solvent from the residue and further purifying according to methods known in the art, the methodology for example, but not limited to, crystallization, distillation, extraction, trituration and chromatography. Unless otherwise specified, the material and reagents are either commercially available or can be prepared from the commercially available materials by the man skilled in the art using the methods described in the chemical literature.

Unless otherwise specified, the conventional experiment, including appropriate operation for conditions, reagents and synthesis routes, protection of any chemical functional groups that are incompatible with the reaction conditions, and the deprotection at appropriate point in the reaction sequence of the process are all included in the scope of the present invention. Appropriate protecting groups and methods using these protecting groups to protect and deprotect different substituent are known by the art, examples refer to T. Greene and P. Wuts, Protecting Groups in Organic Synthesis (3rd ed.), John Wiley & Sons, NY (1999*)*, the content of which is hereby incorporated by reference in its entirety. The synthesis of the compound of the present invention may be accomplished by methods similar to the synthetic reaction schemes described above and the methods described in specific embodiments.

Unless otherwise specified, if the starting material is not commercially available, preparation may be done by using standard organic chemistry techniques, or using techniques that synthesizing known compound which is similar in structure, or using similar methods described in reaction scheme above or in the synthesis embodiments.

Unless otherwise specified, when an optically active form of the compound is needed, it may be prepared by performing the methods described herein with optically active starting material (e.g., by asymmetrically inducing a suitable reaction step), or by using standard methods (e.g., chromatography, recrystallization or enzymatic resolution) to seperate the stereoisomer mixture of compounds or intermediates thereof.

Unless otherwise specified, when a pure geometric isomer of a compound is needed, it may be prepared by performing the methods described herein by using pure geometric isomer as starting material, or using standard methods (e.g., chromagraphy) to seperate the geometric isomer mixture of compounds or intermediates thereof.

Unless otherwise specified, the reagents and material used in the present invention are commercially available.

Unless otherwise specified, the present invention employed the following abbreviations: aq represents water; HATU represents *O*-(7-azabenzotriazol-1-yl)-*N*,*N*,*N*',*N*'-tetramethyluronium hexafluorophosphate; EDC represents *N*-(3-dimethylaminopropyl)-*N*'-ethylcarbodiimide hydrochloride; *m*-CPBA represents 3-chloroperoxybenzoic acid; eq represents equivalent or equivalence; CDI represents carbonyl diimidazole; DCM represents dichloromethane; PE represents petroleum ether; DIAD represents diisopropyl azodicarboxylate; DMF represents *N*,*N*-dimethylformamide; DMSO represents dimethylsulfoxide; EtOAc represents ethyl acetate; EtOH represents ethanol; MeOH represents methanol; CBz represents benzyloxycarbonyl, which is an amino protecting group; BOC represents *tert*-butylcarbonyl, which is an amino protecting group; HOAc represents acetic acid; $NaCNBH_3$ represents sodium cyanoborohydride; r.t. represents room temperature; O/N represents overnight; THF represents tetrahydrofuran; $Boc_2O$ represents di-*tert*-butyldicarbonate; TFA represents trifluoroacetic acid; DIPEA represents diisopropylethylamine; $SOCl_2$ represents thionyl chloride; $CS_2$ represents carbon disulfide; TsOH represents *p*-toluenesulfonic acid; NFSI represents N-fluoro-N-(benzenesulfonyl)-benzsulfamide; NCS represents 1-chloropyrrolidine-2,5-dione; n-$Bu_4NF$ represents tetrabutylammonium fluoride; iPrOH represents 2-propanol; mp represents melting point.

Unless otherwise specified, compounds are named manually or by ChemDraw® software, the commercially available compounds use their vendor directory names.

The positive progress of the present invention is that the nitrogenous macrocyclic compound in the present invention may effectively bond to the bromodomain of BRD4, BRD3, BRD2 and BRDT from the BET family to regulate the downstream gene c-myc and the transcription of targeted gene thereof, therefore modulate the downstream signaling pathway and perform a specific effect, including treatment of diseases such as inflammatory diseases, cancer and AIDS; wherein some of the compounds possess high activity and relatively good cell activity and metabolic stability, so that they may be used as an effective medicament for treating tumor.

## Brief description of the drawings

Figure 1 shows the content of 4009 in tumors of MV-4/11 mice.
Figure 2 shows the content of 4009 in the plasma of MV-4/11 mice.
Figure 3 shows the amount of MYC mRNA expression versus time in tumor.
Figure 4 shows the mice's body weight data of mice.
Figure 5 shows the mice's tumor volume change data.

Figure 6 shows the relative volume change of the mice's tumor.
Figure 7 shows the change of the mice-tumor's weight.

**Detailed description of the preferred embodiment**

The following embodiments further illustrate the present invention, but by all means the invention is not limited thereto. The present invention has been described in detail herein, the embodiments of the present invention are disclosed herein, and modification of the embodiments may be made by technicians in this field without departing from the intention and scope of the present intention.

The following embodiments with no specific conditions should be preformed according to conventional conditions and methods, or according to the description of the product. Starting materials may be obtained commercially, by performing methods known in the art or methods described herein. The structure of the compound is deterimined by $^1$H NMR or MS, while NMR measurement is done by using BrukerAV-300 type NMR apparatus, with solvent of DMSO-$D_6$ or $CDCl_3$, internal standard TMS.

**Emobodiment 4003: 6-(4-Fluorophenyl)-2-methyl-9-(methylsulfonemethyl)-5-oxo-2,4,5,6-tetrahydrobenzo[b]pyrr olo[3,4-*d*]azepin-3-nitrile**

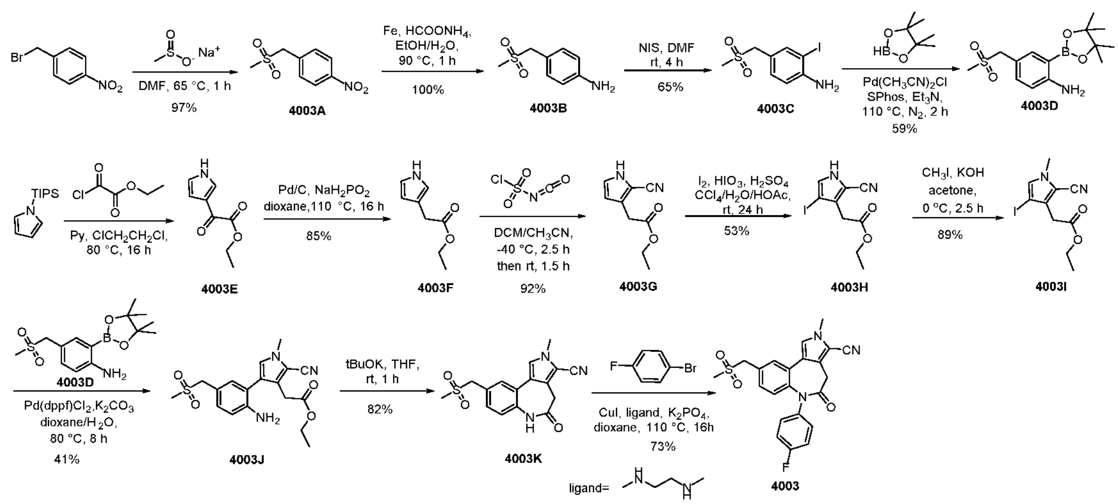


Step 1: 1-(Methylsulfonemethyl)-4-nitrobenzene

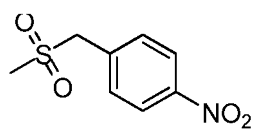

4-Nitrobenzyl bromide (10.0 g, 46.4 mmol) was dissolved in *N*,*N*-dimethylformamide (25 mL) followed by addition of sodium methanesulfinate (7.10 g, 69.6 mmol). The reaction mixture was stirred at 65°C for 1 hours, then cooled down to r.t., diluted by water and keep stirring for 10 minutes, filtered and dried to obtain the title compound (9.7g, 97%) as white solid.

Step 2: 4-(Methylsulfonemethyl)-4-phenylamine

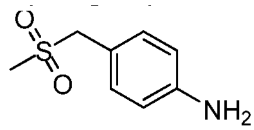

1-(Methylsulfonemethyl)-4-nitrobenzene (9.7 g, 45.1 mmol), iron powder (12.6 g, 225.6 mmol) and ammonium chloride (28.4 g, 451.1 mmol) was dissolved in ethanol (120 mL) and water (60 mL) and stirred at 90°C for 2 hours. The solid was filtered off and the filtrate was concentrated. The residue was dissolved in EtOAc, washed by dicarbonate and brine, dried over anhydrous sodium sulfate then concentrated to obtain the title compound (8.54 g, 100%), which is in form of white solid. LCMS (ESI) $[M+H]^+$ = 186.1.

Step 3: 2-Iodo-4-(methylsulfonemethyl)phenylamine

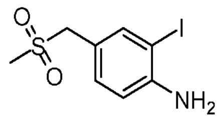

4-(Methylsulfonylmethyl)phenylamine (8.54 g, 46.1 mmol) was dissolved in *N*,*N*-dimethylformamide (460 mL) and *N*-iodosuccinimide (11.4 g, 50.7 mmol), the reaction solution was stirred at room temperature for 4 hours. The reaction was quenched with 10% hyposulphite and saturated sodium carbonate, then extracted by EtOAc (50 mL*3), the organic phase was combined and washed by brine, then dried over anhydrous magnesium sulfate, then filtered and concentrated. The residue was separated and purified with flash chromatography (dicloromethane/ methanol = 20/1) to obtain the title compound (9.3 g, 65%), which is in form of orange solid. LCMS (ESI) $[M+H]^+$ = 312.0.

Step 4: 4-(methylsulfonemethyl)-2-(4,4,5,5-tetramethyl-1,3,2-dioxoborolan-2-yl)phenylamine

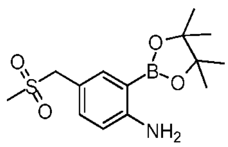

2-Iodo-4-(methylsulfonylmethyl)phenylamine (1.87 g, 6.0 mmol), pinacol borate (2.31 g, 18.0 mmol), bis(acetonitrile)palladium chloride (II) (156 mg, 0.6 mmol), 2-cyclohexylphosphine-2,6-dimethoxybiphenyl(985 mg, 2.4 mmol) and triethylamine (3.04 g, 30 mmol) dissolved in 1,4-dioxane (18 mL) and stirred at 110°C for 2 hours under nitrogen protection. The reaction solution was concentrated and the residue was purified by flash chromatography (dicloromethane/ EtOAc = 10/1) to obtain the title compound (1.1 g, 59%) in form of pale yellow solid. LCMS (ESI) $[M+H]^+$ = 312.2.

Step 5: 2-Oxo-2-(1*H*-pyrrol-3-yl)ethyl acetate

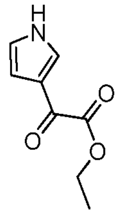

1-(Triisopropylsilyl)-1*H*-pyrrole (16.0 g, 71.7 mmol), 2-chloro-2-oxoethylacetate (14.6 g, 107.6 mmol) was dissolved in pyridine (17 mL, 215.1 mmol) and 1,2-dichloroethane (75 mL), and stirred at 80°C under nitrogen protection

overnight. The reaction solution was concentrated, the residue was separated and purified with flash chromatography (PE/ EtOAc = 1/1) to obtain the title compound (7.4 g, 62%) in form of pale yellow solid. LCMS (ESI) $[M+H]^+$ = 168.1.

Step 6: 2-(1*H*-pyrrol-3-yl)ethyl acetate

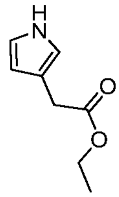

2-Oxo-2-(1*H*-pyrrol-3-yl)ethyl acetate (2.4 g, 14.4 mmol), palladium/carbon (7.2 g) and sodium hypophosphite (25.3 g, 287 mmol). 1,4-dioxane (120 mL) was stirred at 110°C under nitrogen protection overnight. After filtration, the filtrate was concentrated and the residue was separated and purified with flash chromatography (PE/ EtOAc = 10/1) to obtain the tilte compound (1.88 g, 85%) in form of pale yellow oil. LCMS (ESI) $[M+H]^+$ = 154.1.

Step 7: 2-(2-Cyano-1*H*-pyrrol-3-yl)ethyl acetate

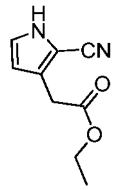

2-(1*H*-pyrrol-3-yl)ethyl acetate (1.88 g, 12.3 mmol) was dissolved in acetonitrile (80 mL) and dichloromethane (20 mL), cooled down to -42°C, then chlorosulfonyl isocyanate (1.74 g, 12.3 mmol) was slowly added dropwise with intense agitation under nitrogen protection, the addition was done in 30 minutes, and stirring was continued at this temperature for 2.5 hours. *N*,*N*-dimethylformamide (5 mL) was quickly added to the reaction solution, and the mixture was gradually warmed to room temperature over 1.5 hours. The reaction solution was washed with diluted sodium hydrogen carbonate solution, dried over anhydrous sodium sulfate and concentrated. The residue was separated and purified with flash chromatography (PE/ EtOAc = 10/1) to obtain the title compound (2.0 g, 92%) in form of pale yellow oil. LCMS (ESI) $[M+H]^+$ = 179.1.

Step 8: 2-(2-Cyano-4-iodo-1*H*-pyrrol-3-yl)ethyl acetate

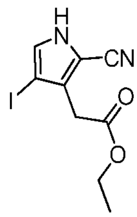

2-(2-Cyano-1*H*-pyrrol-3-yl)ethyl acetate (1.99 g, 11.2mmol), iodic acid (396 mg, 2.24 mmol) and iodine (1.14 g, 4.48 mmol) was disoloved in tetrachloromethane (80 mL), water (2.4 mL), acetic acid (5.6 mL) and concentrated sulfuric acid (0.4 mL), the mixture was stirred at room temperature overnight under nitrogen protection. The reaction solution was washed by diluted sodium dicarbonate, dried over anhydrous sodium sulfate and then concentrated. The residue was separated and purified with flash chromatography (PE/ EtOAc = 10/1) to obtain the title compound (2.62 g,

53%) in form of pale yellow solid. LCMS (ESI) $[M+H]^+$ = 305.0.

Step 9: 2-(2-Cyano-4-iodo-1-methyl-1*H*-pyrrol-3-yl)ethyl acetate

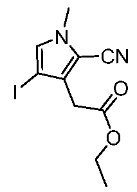

2-(2-Cyano-4-iodo-1*H*-pyrrol-3-yl)ethyl acetate (2.06 g, 6.78 mmol) and potassium hydroxide (949 mg, 16.94 mmol) were dissolved in acetone (50 mL), then methyl iodide (1.92 g, 13.56 mmol) was slowly added dropwise at 0°C. The mixture was continued to be stirred at 0°C for 2.5 hours. EtOAc was added and washed by water, dried over anhydrous sodium sulfate and then filtered and concentrated. The residue was separated and purified with flash chromatography (PE/ EtOAc = 10/1) to obtain the title compound (2.63 g, 89%) in form of pale yellow oil. LCMS (ESI) $[M+H]^+$ = 319.0.

Step 10:

2-(4-(2-Amino-5-(methylsulfonylmethyl)phenyl)-2-cyano-1-methyl-1*H*-pyrrol-3-yl)ethyl acetate.

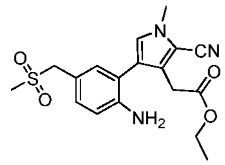

2-(2-Cyano-4-iodo-1-methyl-1*H*-pyrrol-3-yl)ethyl acetate (477 mg, 1.50 mmol), 4-(methylsulfonemethyl)-2-(4,4,5,5-tetramethyl-1,3,2-dioxoborolan-2-yl)phenylaime (444 mg, 1.43 mmol), [1,1'-bis (diphenylphosphino) ferrocene] dichloropalladium (II) (110 mg, 0.15 mmol) and potassium carbonate (1.04 g, 7.50 mmol) was dissolved in 1,4-dioxane (8 mL) and water (2 mL) and stirred at 80° C under Nitrogen protection for 8 hours. The reaction solution was cooled down, filtered and concentrated. The residue was separated and purified with flash chromatography (PE/ EtOAc = 1/2) to obtain the title compound (288 mg, 41%) in form of yellow solid. LCMS (ESI) $[M+H]^+$ = 376.2.

Step 11: 2-Methyl-9-(methylsulfonemethyl)-5-oxo-2,4,5,6-tetrahydrobenzo[*b*]pyrrole[3,4-*d*]azepin-3-nitril e

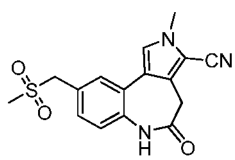

2-(4-(2-Amino-5-(methylsulfonylmethyl)phenyl)-2-cyano-1-methyl-1*H*-pyrrol-3-yl)ethy l acetate (285 mg, 0.76 mmol) was dissolved in tetrahydrofuran (10 mL), then potassium *tert*-butoxide (255 mg, 2.28) was added in portions, then the reaction solution was stirred for 1 hours. Dichloromethane was added to the reaction mixture, which was washed with water, dried over anhydrous sodium sulfate, filtered and concentrated. The residue was separated and purified with flash chromatography (PE/ EtOAc = 10/1) to obtain the title compound (204 mg, 82%) in form of white solid. LCMS (ESI) $[M+H]^+$ = 330.1.

Step 12: 6-(4-Fluorophenyl)-2-methyl-9-(methylsulfonemethyl)-5-oxo-2,4,5,6-tetrahydrobenzo[*b*]pyrrolo[3 ,4-*d*]azein-3-carbonitrile

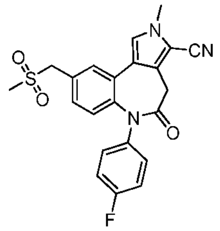

2-Methyl-9-(methylsulfonemethyl)-5-oxo-2,4,5,6-tetrahydrobenzo[*b*]pyrrole[3,4-*d*]azepi n-3-nitrile (53 mg, 0.161 mmol), 1-bromo-4-fluorobenzene (42 mg, 0.242 mmol), *N1*,*N2*-dimethylethane-1,2-diamine (11 mg, 0.129 mmol), potassium phosphate (170 mg, 0.805 mmol) and cuprous iodide (12 mg, 0.065 mmol) was dissolved in 1,4-dioxane (3 mL) and stirred at 110°C overnight under nitrogen protection. The reaction solution was filtered and the filtrate was concentrated. The residue was purified by preparative-HPLC to obtain the tilte compound (17.5 mg, 26%) in form of white solid. MS (ESI): m/z = 424.0 $[M+1]^+$; $^1$H NMR (400 MHz, DMSO-$d_6$) $\delta$ 7.71 (s, 1H), 7.57 (d, *J* = 1.2 Hz, 1H), 7.25 (dd, *J* = 1.2 Hz, 6.8 Hz, 1H), 7.22-7.16 (m, 2H), 7.12-7.05 (m, 2H), 6.98 (d, *J* = 6.8 Hz, 1H), 4.49 (s, 2H), 3.87 (s, 3H), 3.55(s, 2H), 2.94 (s, 3H).

**Embodiment 4001: 6-(4-fluorophenyl)-2-methyl-9-(methylsulfonemethyl)-5-oxo-2,4,5,6-tetrahydrobenzo[*b*]pyrro lo[3,4-*d*]azepin-3-carboxamide**

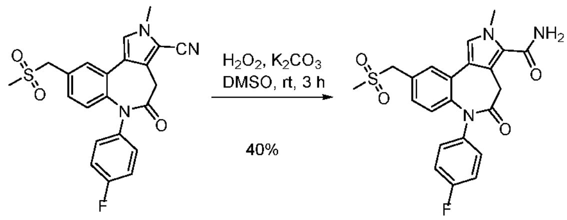


4003 4001

6-(4-Fluorophenyl)-2-methyl-9-(methylsulfonemethyl)-5-oxo-2,4,5,6-tetrahydrobenzo[*b* ]pyrrolo[3,4-*d*]azepin-3-nitrile (42 mg, 0.099 mmol) and potassium carbonate (41 mg, 0.297 mmol) were dissolved in dimethylsulfoxide (2 mL), then 30% aqueous hydrogen peroxide (0.5 mL) was added into the solution, the reaction mixture was stirred at room temperature for 3 hours. The reaction was quenched with water and extracted with EtOAc, the EtOAc phase was separated and then water and saturated brine was used to wash the solution, followed by desiccation with anhydrous sodium sulfate and filtered and concentrated in vacuum, the residue was purified by reverse phase preparative-HPLC to obtain the title compound (17.7 mg, 40%) in form of white solid. LCMS (ESI) $[M+H]^+$= 442.1; $^1$H NMR (400 MHz, DMSO-$d_6$) $\delta$ 7.54 (s, 1H), 7.46-7.25 (m, 3H), 7.23-7.13 (m, 3H), 7.10-7.01 (m, 2H), 6.94-6.87 (m, 1H), 4.48 (s, 2H), 3.84 (s, 3H), 2.93 (s, 3H), 2.54 (s, 2H).

Embodiment 4004: **6-(4-Fluorophenyl)-2-methyl-9-(methylsulfonylmethyl)-2,4,5,6-tetrahydrobenzo[*b*]pyrrolo[3, 4-*d*]azepin-3-nitrile**

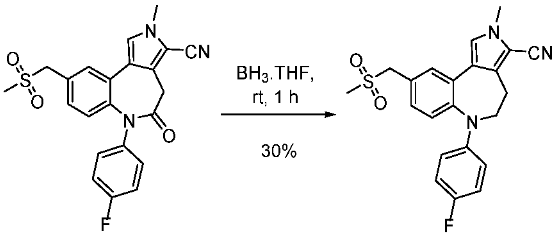


4003 4004

6-(4-Fluorophenyl)-2-methyl-9-(methylsulfonylmethyl)-5-oxo-2,4,5,6-tetrahydrobenzo[ *b*]pyrrolo[3,4-*d*]azepin-3-nitrile (50 mg, 0.118 mmol) was dissolved in tetrahydrofuran (5 mL), borane (1 M in tetrahydrofuran, 0.5 mL) was added into the mixture at 0°C under nitrogen protection, then the mixture was warmed to room temperature and stirred for 1 hour. Methanol (5 mL) was added into the reaction mixture then concentrated, the residue was purified by preparative-HPLC to obtain the title compound (14.6 mg, 30%) in form of white solid. LCMS (ESI) $[M+H]^+$= 410.1; $^1H$ NMR (400 MHz, DMSO-$d_6$) $\delta$ 7.84 (s, 1H), 7.62 (s, 1H), 7.25-7.19 (m, 2H), 6.98 (t, *J* = 7.2 Hz, 3H), 6.76-6.70 (m, 2H), 4.48 (s, 2H), 3.88 (t, *J* = 4.4 Hz, 2H), 3.75 (s, 3H), 3.01 (t, *J* = 4.4 Hz, 2H), 2.96 (s, 3H).

**Embodiment 4002: 6-(4-fluorophenyl)-2-methyl-9-(methylsulfonylmethyl)-2,4,5,6-tetrahydrobenzo[*b*]pyrrolo[3,4-*d*]azepin-3-carboxamide**

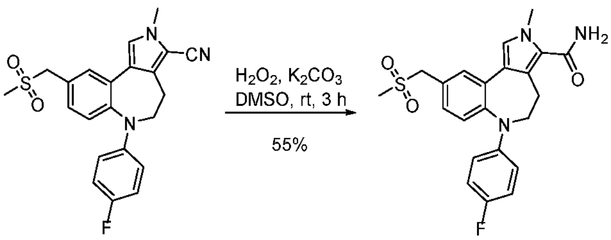


4004 4002

6-(4-Fluorophenyl)-2-methyl-9-(methylsulfonemethyl)-2,4,5,6-tetrahydrobenzo[*b*]pyrrol o[3,4-*d*]azepin-3-nitrile (99 mg, 0.240 mmol) and potassium carbonate (50 mg, 0.362 mmol) was dissolved in dimethylsulfoxide (5 mL), then added 30% aqueous hydrogen peroxide (0.5 mL), the reaction mixture was stirred at room temperature for 3 hours. The reaction was quenched with water and extracted with EtOAc, the EtOAc phase was separated and then water and saturated brine was used to wash the solution, followed by desiccation with anhydrous sodium sulfate and filtered and concentrated in vacuum, the residue was purified by reverse phase preparative-HPLC to obtain the title compound (57 mg, 55%) in form of white solid. LCMS (ESI) $[M+H]^+$= 428.1; $^1H$ NMR (400 MHz, DMSO-$d_6$) $\delta$ 7.81 (s, 1H), 7.33 (s, 1H), 7.27-7.09 (m, 3H), 7.23-7.13 (m, 4H), 6.97 (t, *J* = 7.2 Hz, 2H), 6.80-6.73 (m, 2H), 4.45 (s, 2H), 3.82 (t, *J* = 4.0 Hz, 2H), 3.74 (s, 3H), 3.08 (t, *J* = 4.0 Hz, 2H), 2.95 (s, 3H).

**Embodiment 4005: 9-fluoro-6-(4-fluorophenyl)-2-methyl-2,4,5,6-tetrahydropyrido[2,3-*b*]pyrrolo[3,4-*d*]azepin-3-carboxamide**

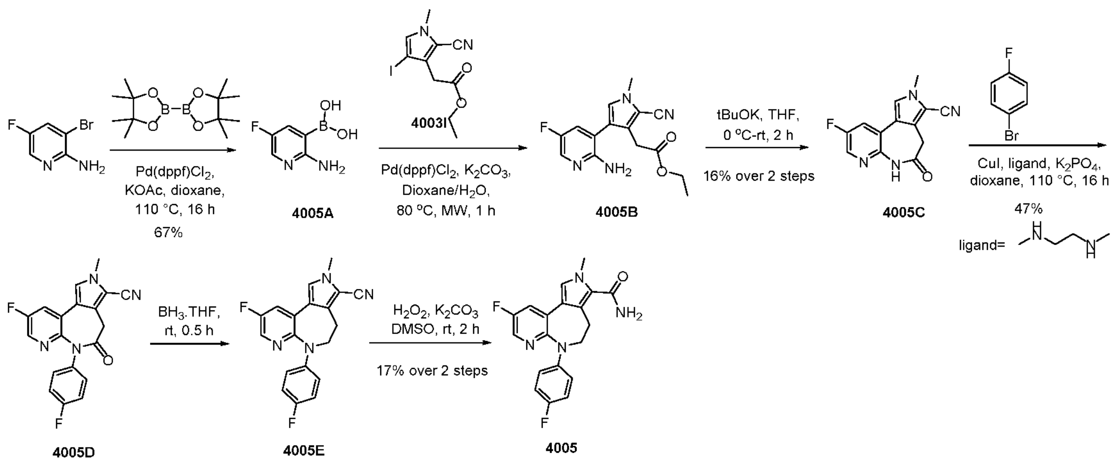


Step 1: 2-Amino-5-fluoropyridin-3-yl boronic acid

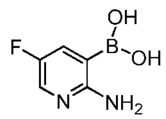

3-Bromo-5-fluoro-pyridin-2-amine (1 g, 5.2 mmol), boranoic acid pinacol ester (2 g, 7.8 mmol), [1,1'-bis(diphenylphosphino)Ferrocene]dichloropalladium (II) (0.38 g, 0.52 mmol) and potassium acetate (1.53 g, 15.6 mmol) were dissolved in 1,4-dioxane (50 mL) and stirred at 110°C under nitrogen protection overnight. The reaction mixture was filtered, the filtrate was concentrated in vacuum, The residue was separated and purified with flash chromatography (PE/ EtOAc = 1/99) to obtain the title compound (0.55 g, 67%) in form of yellow solid. LCMS (ESI) $[M+H]^+$ = 157.1.

Step 2: 2-(4-(2-Amino-5-fluoropyridin-3-yl)-2-cyano-1-methyl-1*H*-pyrrol-3-yl)ethyl acetate

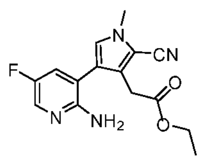

2-Amino-5-fluoropyridin-3-ylboronic acid (200 mg, 1.28 mmol), [1,1'-bis (diphenylphosphino) ferrocene]dichloropalladium(II) (93.6 mg, 0.13 mmol), potassium carbonate (529.9 mg, 3.84 mmol) and 2-(2-cyano-4-iodo-1-methyl-1*H*-pyrrol-3-yl)ethyl acetate (448.4 mg, 1.41 mmol) was dissolved in 1,4-dioxane/ water (2 mL/ 0.5 mL), the mixture was heated to 80°C by microwave and stirred under nitrogen for 1 hour. Then filtered and concentrated, the residue was separated and purified with flash chromatography (PE/EtOAc = 4/1) to obtain the title compound.

Step 3: 9-Fluoro-2-methyl-5-oxo-2,4,5,6-tetrahydropyrido[2,3-*b*]pyrrolo[3,4-*d*]azepin-3-nitrile

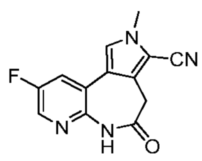

2-(4-(2-Amino-5-fluoropyridin-3-yl)-2-cyano-1-methyl-1*H*-pyrrol-3-yl)ethyl acetate was dissolved in tetrahydrofuran (4 mL), potassium *tert*-butoxide (286.7 mg, 2.56 mmol) was added at 0°C, then warmed to room temperature and stirred for 2 hours. Then filtered and the filtrate was concentrated to obtain the title compound (55 mg, yield of the two steps 16%) in form of yellow solid. LCMS (ESI) $[M+H]^+$ = 257.1.

Step 4: 9-Fluoro-6-(4-fluorophenyl)-2-methyl-5-oxo-2,4,5,6-tetrahydropyrido[2,3-*b*]pyrrolo[3,4-*d*]azepin-3-nitrile

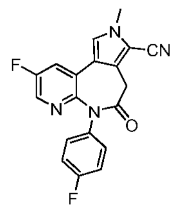

9-Fluoro-2-methyl-5-oxo-2,4,5,6-tetrahydropyrido[2,3-*b*]pyrrolo[3,4-*d*]azepin-3-nitrile (55 mg, 0.21 mmol), 1-bromo-4-fluorobenzene (45.1 mg, 0.26 mmol), *N*,*N'*-dimethylethane-1,2-diamine (20 mg, 0.27 mmol), iodine Cuprous (4.05 mg, 0.021 mmol) and potassium phosphate (134 mg, 0.63 mmol) were dissolved in 1,4-dioxane (2 mL), sealed and stirred at 110°C overnight. Then filtered and the filtrate was concentrated the residue was separated and purified with flash chromatography (PE/EtOAc = 5/1) to obtain the title compound (35 mg, 47%) in form of white solid. LCMS (ESI) $[M+H]^+$ = 351.1.

Step 5: 9-Fluoro-6-(4-fluorophenyl)-2-methyl-2,4,5,6-tetrahydropyrido[2,3-*b*]pyrrolo[3,4-*d*]azepin-3-nitri le

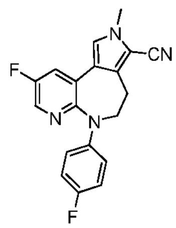

A mixture of 9-fluoro-6-(4-fluorophenyl)-2-methyl-5-oxo-2,4,5,6-tetrahydropyrido[2,3-*b*]pyrrolo[3,4-*d*]azepin-3-nitrile (35 mg, 0.10 mmol) and borane (1 M in tetrahydrofuran, 4 mL) was stirred at room temperature for 0.5 h. Methanol (2 mL) was added to quench the reaction and then concentrated the reaction solution to obtain a crude product of the tilte compound (40 mg) in form of yellow solid. LCMS (ESI) $[M+H]^+$ = 337.1.

Step 6: 9-Fluoro-6-(4-fluorophenyl)-2-methyl-2,4,5,6-tetrahydropyrido[2,3-*b*]pyrrolo[3,4-*d*]azepin-3-carb oxamide

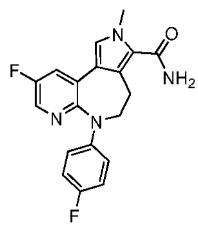

9-Fluoro-6-(4-fluorophenyl)-2-methyl-2,4,5,6-tetrahydropyrido[2,3-*b*]pyrrolo[3,4-*d*]aze pin-3-nitrile (40 mg crude product) and potassium carbonate (49.7mg, 0.36 mmol) were dissolved in dimethylsulfoxide (2 mL), then 30% aqueous hydrogen peroxide (2 mL) was added, the reaction mixture was stirred at room temperature for 2 hours. The reaction was quenched with water, then extracted with EtOAc, the EtOAc phase was then separated and washed with

water and saturated brine, dried with anhydrous sodium sulfate, filttered and concentrated in vaccum. The residue was separated and purified with reverse phase preparative-HPLC to obtain the title compound (7.0 mg, yield of the two steps 17%) in form of white solid. (ESI): $R_T$(min)=4.955, [M+H]+ = 355.1; $^1$H NMR (400 MHz, DMSO-d6) $\delta$ 8.47 (s, 1H), 8.08-8.04 (m, 2H), 7.61 (s, 1H), 7.22 (brs, 2H), 7.04-7.00 (m, 2H), 6.96-6.93 (m, 2H), 3.82-3.80 (m, 2H), 3.74 (s, 3H), 3.05-3.03 (m, 2H).

**Embodiment 4007: 7-(4-Fluorophenyl)-2-methyl-10-(methylsulfonemethyl)-2*H*-benzo[*c*]pyrido[3,4-*e*]azepin-3(5 *H*)-ketone**

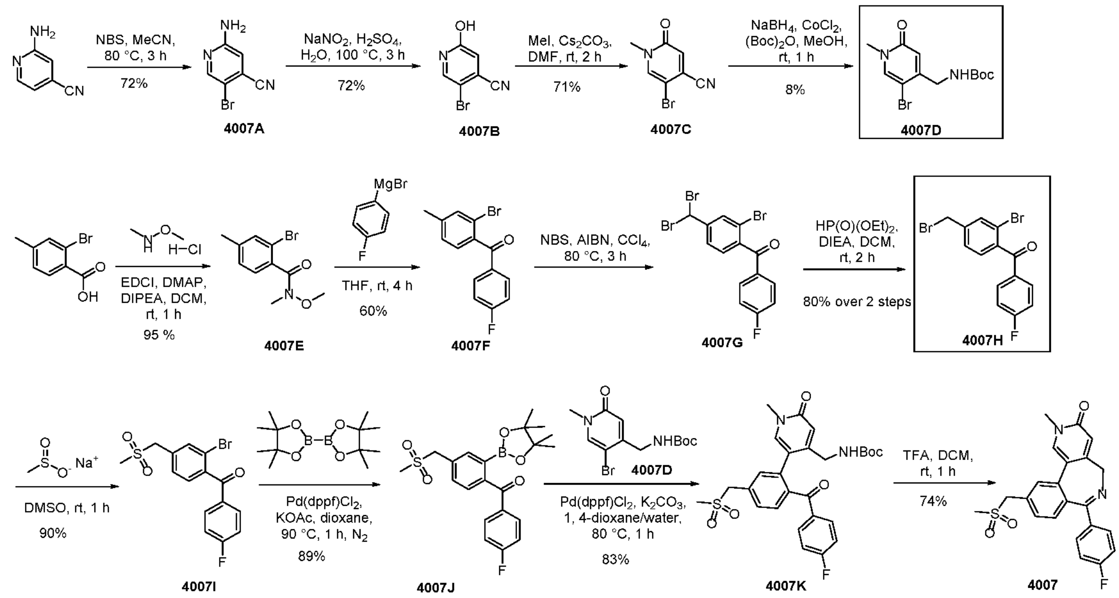


Step 1: 2-amino-4-cyano-5-bromopyridine

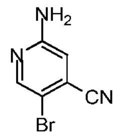

*N*-bromosuccinimide (3.8 g, 21.38 mmol) was added to a solution of 2-amino-4-cyanopyridine (2.12 g, 17.8 mmol) in acetonitrile (60 mL). The reaction mixture was stirred at 80°C for 3 hour. Evaporate the solvent under reduced pressure, the residue was separated and purified with flash chromatography (PE/EtOAc = 2/1) to obtain the title compound (3.05 g, 72%). LCMS (ESI) $[M+H]^+$ = 197.9.

Step 2: 5-Bromo-2-hydroxy-4-cyanopyridine

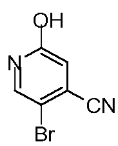

2-Amino-4-cyano-5-bromopyridine (3.05 g, 15.4 mmol) was dissolved in concentrated sulfuric acid (10 mL),

and sodium nitrite (2.15 g, 31 mmol) in water (60 mL) was slowly added dropwise at 0°C. After the dropwise addition was completed, the mixture was stirred at 100°C for 3 hours. The reaction mixture was poured into ice water (200 mL) and ethyl acetate (50 mL*3) was used to extract. Combine the organic phases and then dried it with anhydrous sodium sulfate, filtered and concentrated to obtain the crude product of the title compound (2.2 g, 72%), which is to be used directly in the next step. LCMS (ESI) $[M+H]^+$ =199.0.

Step 3: 5-bromo-1-methyl-2-oxo-4-cyano-1,2-dihydropyridine

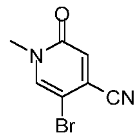

Methyl iodide (3.15 g, 22.2 mmol) was added dropwise to 5-bromo-2-hydroxy-4-cyanopyridine (2.2 g, 11.1 mmol) and cesium carbonate (7.24 g, 22.2 mmol) in *N*,*N*-dimethylformamide solution (100 mL). The reaction mixture was stirred at room temperature for 2 hours, filtered and concentrated. The residue was separated and purified with flash chromatography (PE/EtOAc = 3/1) to obtain the title compound (1.68 g, 71%). LCMS (ESI) $[M+H]^+$= 213.1.

Step 4: (5-bromo-1-methyl-2-oxo-1,2-dihydropyridin-4-yl) tert-butylcarbamate

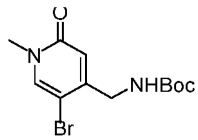

5-Bromo-1-methyl-2-oxo-4-cyano-1,2-dihydropyridine (1000 mg, 4.7 mmol), di-tert-butyl dicarbonate ester (1.025 g, 4.7 mmol) and cobalt chloride hexahydrate (111 mg, 0.47 mmol) was mixed in methanol (30 mL), then sodium borohydride (1.78 g, 47 mmol) was added in portions. The reaction mixture was stirred at room temperature for 1 hour and then concentrated. The residue was layered by adding EtOAc (50 mL) and saturated ammonium chloride solution (100 mL), the organic phase was then separated. The aqueous phase was extracted by EtOAc (20 mL*3). The organic phase was combined and dried over anhydrous sodium sulfate, filtered and concentrated. The crude product obtained was then separated and purified by preparative-TLC (PE/EtOAc = 4/1) to obtain the title compound (110 mg, 8%) in form of white solid. LCMS (ESI) $[M+H]^+$= 317.0.

Step 5: 2-bromo-*N*-methoxy-*N*,4-dimethylbenzamide

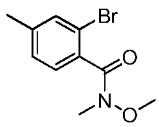

2-bromo-4-methylbenzoic acid (10.75 g, 50 mmol), *N*,*O*-dimethylhydroxylamine hydrochloride (5.85 g, 60 mmol) and *N*-ethyl-*N'*-(3-methylaminopropyl) dimethyl-amino-propylcarbodiimide (11.5 g, 60 mmol), *N*,*N*-dimethylaminopyridine (0.73 g, 60 mmol), diisopropylethylamine (12.9 g, 100 mmol) was mixed with dichloromethane (200 mL), and the mixture was stirred at 20°C for 1 hour and then concentrated. The residue was layered by adding EtOAc (100 mL) and water (200 mL), the organic phase was then separated. The aqueous phase was extracted by EtOAc (30 mL*3). The organic phase was combined and dried over anhydrous sodium sulfate, filtered and concentrated. The residue was then separated and purified by flash chromatography (PE/EtOAc = 6/1) to obtain the title compound (12.3 g, 95%) in form of yellow oil. LCMS (ESI) $[M+H]^+$ = 258.0.

Step 6: (2-bromo-4-methylphenyl)(4-fluorophenyl)methanone

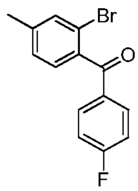

4-fluorophenylmagnesium bromide (1 M in tetrahydrofuran, 95 mL) was added to 2-bromo-*N*-methoxy-*N*,4-dimethylbenzamide (12.3 g, 47 mmol) in tetrahydrofuran (300 mL) at 0°C under nitrogen protection. The reaction mixture was stirred at room temperature for 4 hours. The reaction was quenched with water (30 mL) then extracted with EtOAc (100 mL*3). The organic phases were combined, then washed with brine, dried with anhydrous sodium sulfate, filtered and concentrated. The residue was then separated and purified by flash chromatography (PE/EtOAc = 6/1) to obtain the title compound (8.2 g, 60%) in form of yellow solid.

Step 7: (2-bromo-4-(dibromomethyl)phenyl)(4-fluorophenyl)methanone

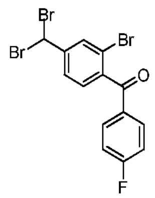

(2-bromo-4-methylphenyl)(4-fluorophenyl)methanone (8.2 g, 28 mmol), *N*-bromosuccinimide (15 g, 84 mmol) and azodiisobutyronitrile (460 mg, 2.8 mmol) were mixed in carbon tetrachloride (100 mL). The reaction solution was stirred at 80°C for 3 hours. The solvent was spin dried, the crude product(13 g, 103%) obtained was directly used in the next step.

Step 8: (2-bromo-4-(bromomethyl)phenyl)(4-fluorophenyl)methanone

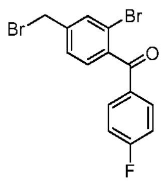

Diethyl phosphite (7.7 g, 56 mmol) and diisopropylethylamine (7.25 g, 56 mmol) were added into (2-bromo-4-(dibromomethyl)phenyl)(4-fluorobenzene) methanone (13 g, 28.8 mmol) in dichloromethane (100 mL). The reaction mixture was stirred at 20°C for 2 hours. Concentration of the reaction mixture, the residue was then separated and purified by flash chromatography to obtain the title compound (8.3 g, yield of the two steps 80%) in form of yellow solid.

Step 9: (2-bromo-4-(methylsulfonylmethyl)phenyl)(4-fluorophenyl)methanone

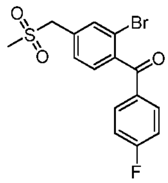

Sodium methylsulfinate (816 mg, 8 mmol) was added to (2-bromo-4-(bromomethyl)phenyl)(4-fluorophenyl)methanone (1.5 g, 4 mmol) in dimethylsulfoxide solution (15 mL). The reaction mixture was stirred at room temperature for 1 hour. EtOAc (50 mL) and water (150 mL) was used to layer the reacture mixture, the organic phase was washed by saturated sodium chloride(50 mL*3), dried over anhydrous sodium sulfate, filtered and concentrated to obtaine the title compound (1.35 g, 90%). LCMS (ESI) $[M+H]^+$ = 370.9.

Step 10: (4-(methylsulfonylmethyl)-2-(4,4,5,5-tetramethyl-1,3,2-dioxoborolan-2-yl)phenyl(4-fluorophenyl) methanone

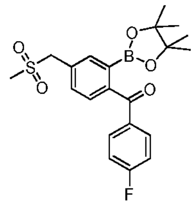

(2-bromo-4-(methylsulfonylmethyl)phenyl)(4-fluorophenyl)methanone (1.35 g, 3.64 mmol), boronic acid pinacol ester (1.84 g, 7.28 mmol),
[1,1'-bis(diphenylphosphino)ferrocene]palladium(II) dichloride (266 mg, 0.36 mmol) and potassium carbonate (713 mg, 7.28 mmol) were mixed in 1,4-dioxane (40 mL). The reaction mixture was heated to 90°C and stirred for 1 hour under protection of nitrogen. Then the mixture was filtered and filtrate was concentrated, the residue was then separated and purified by preparative-TLC (PE/EtOAc = 8/1) to obtain the title compound (1.3 g, 89%) in form of yellow solid. LCMS (ESI) $[M+H]^+$= 418.1.

Step 11: (5-(2-(4-fluorobenzoyl)-5-(methylsulfonylmethyl)phenyl)-1-methyl-2-oxo-1,2-dihydropyridine-4-yl)*tert*-butylmethylcarbamate

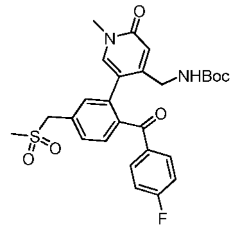

(4-(methylsulfonylmethyl)-2-(4,4,5,5-tetramethyl-1,3,2-dioxoborolan-2-yl)phenyl(4-flu oro Phenyl)methanone (200 mg, 0.5 mmol), *tert*-butyl(5-bromo-1-methyl-2-oxo-1,2-dihydropyridin-4-yl)methylcarbamate (79 mg , 0.25 mmol), [1,1'-bis(diphenylphosphino)ferrocene]palladium(II) dichloride (18 mg, 0.025 mmol) and potassium carbonate (69 mg, 0.5 mmol) was mixed in 1,4-dioxane (2 mL) and water (0.2 mL). The reaction mixture was heated to 80°C and stirred for 1 hour under protection of nitrogen. Then filtered and spin dryed, the residue was then separated and purified by preparative-TLC (PE/ EtOAC =3/1) to obtain the title compound (110 mg, 83%) in form of yellow solid. LCMS (ESI) $[M+H]^+$=529.2.

Step 12: 7-(4-fluorophenyl)-2-methyl-10-(methylsulfonemethyl)-2*H*-benzo[*c*]pyrido[3,4-*e*]azepin-3(5*H*)-k etone

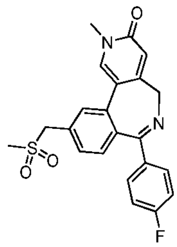

Trifluoroacetic acid (1 mL) and (5-(2-(4-fluorobenzoyl)-5-(methylsulfonylmethyl)phenyl)-1-methyl-2-oxo-1,2-dihydropyridin-4-y l)methyl *tert*-butylcarbamate (113 mg, 0.214 mmol) was dissolved in dichloromethane (4 mL) and stirred at room temperature for 1 hour, the reaction solution was concentrated and the residue was purified by preparative-HPLC to obtain the title compound (65 mg, 74%) in form of white solid. (ESI): $R_T$ (min) = 3.815, [M+H]+ = 411.0 ; $^1$H NMR (400 MHz, DMSO-$d_6$) $\delta$ 8.08 (s, 1H), 7.71 (d, *J* = 1.2 Hz, 1H), 7.48 (m, 3H), 7.32 (d, *J* = 8 Hz, 1H), 7.23 (t, *J* = 8.8 Hz, 2H), 6.52 (s, 1H), 4.74 (d, J = 10.4 Hz, 1H), 4.62 (dd, *J* = 14.0 Hz, *J* = 25.6 Hz, 2H), 3.89 (d, *J* = 10.0 Hz, 1H), 3.50 (s, 3H), 3.02 (s, 3H).

**Embodiment 4008: 7-(4-fluorophenyl)-2-methyl-10-(methylsulfonemethyl)-6,7-dihydro-2*H*-benzo[*c*]pyrido[3,4-*e* ]azepin-3(5*H*)-ketone formate**

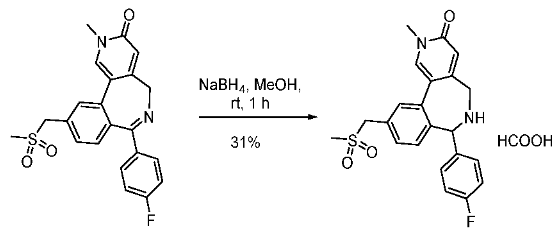

4007 4008

Sodium borohydride (32.4 mg, 0.85 mmol) was added to 7-(4-fluorophenyl)-2-methyl-10-(methylsulfonylmethyl)-2*H*-benzo[*c*]pyrido[3,4-*e*]azepin-3(5*H*)-k etone (35 mg, 0.085 mmol) in MeOH (10 mL) solution. The reaction solution was stirred at room temperature for 1 hour, then concentrated. The residue was purified by preparative-HPLC to obtain the title compound (12 mg, 31%) in form of yellow solid. (ESI): $R_T$ (min)=3.912, [M+H]+ = 413.1; $^1$H NMR (400 MHz, DMSO-$d_6$) $\delta$ 8.25 (br, 1H), 7.85 (s, 1H), 7.39-7.35 (m, 3H), 7.24 (d, *J* = 8 Hz, 1H), 7.14 (t, *J* = 8.8 Hz, 2H), 6.64 (d, *J* = 8 Hz, 1H), 6.49 (s, 1H), 4.89 (s, 1H), 4.45 (d, *J* = 5.6 Hz, 2H), 3.64 (d, *J* = 13.6 Hz, 1H), 3.51 (s, 3H), 3.24 (d, J = 13.6 Hz, 1H), 3.90 (s, 3H).

Embodiment 4009: **7-(4-fluorophenyl)-2-methyl-10-(ethylsulfonemethyl)-2*H*-benzo[*c*]pyrido[3,4-*e*]azepin-3(5*H*)**-ketone

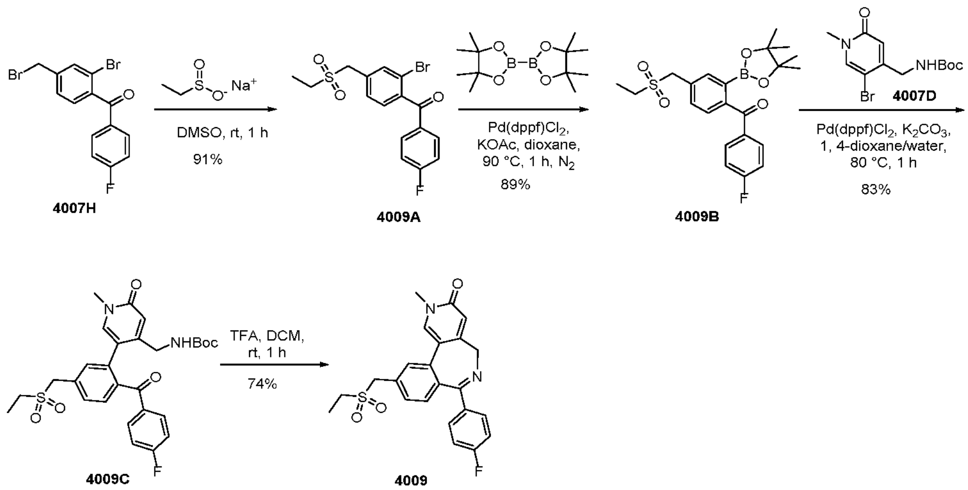


Step 1: (2-Bromo-4-(ethylsulfonylmethyl)phenyl)(4-fluorophenyl)methanone

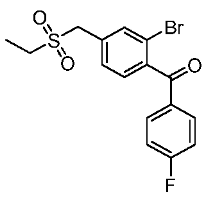

Sodium ethylsulfinate (929 mg, 8 mmol) was added to (2-bromo-4-(bromomethyl)phenyl)(4-fluorophenyl)methanone (1.5 g, 4 mmol) in dimethylsulfoxide solution (15 mL). The reaction mixture was stirred at room temperature for 1 hour. EtOAc(50 mL) and water (150 mL) is used to layer the mixture, organic phase was then washed by saturated brine (50 mL*3), dried over anhydrous sodium sulfate, filtered and concentrated to obtain the title compound (1.39 g, 91%). LCMS (ESI) $[M+H]^+$= 385.0.

Step 2: (4-(ethylsulfonylmethyl)-2-(4,4,5,5-tetramethyl-1,3,2-dioxoborolan-2-yl)phenyl(4-fluorophenyl)m ethanone

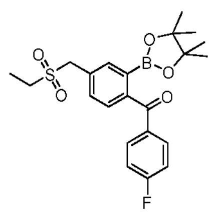

(2-bromo-4-(ethylsulfonylmethyl)phenyl)(4-fluorophenyl)methanone (1.39 g, 3.64 mmol), boronic acid pinacol ester (1.84 g, 7.28 mmol), [1,1'-bis(diphenylphosphino)ferrocene]palladium(II) dichloride (266 mg, 0.36 mmol) and potassium carbonate (713 mg, 7.28 mmol) were mixed in 1,4-dioxane (40 mL). The reaction mixture was heated to 90°C under nitrogen for 1 hour. Then the mixture was filtered and filtrate was concentrated, the residue was then separated and purified by preparative-TLC (PE/EtOAc = 8/1) to obtain the title compound (1.34g, 89%) in form of yellow solid. LCMS (ESI) $[M+H]^+$= 432.2.

Step 3: (5-(2-(4-fluorobenzoyl)-5-(ethylsulfonylmethyl)phenyl)-1-methyl-2-oxo-1,2-dihydropyridine-4-yl )*tert*-butylmethylcarbamate

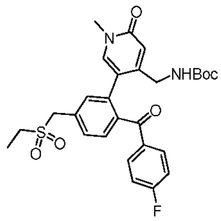

(4-(ethylsulfonylmethyl)-2-(4,4,5,5-tetramethyl-1,3,2-dioxoborolan-2-yl)phenyl(4-fluor o Phenyl)methanone (207 mg, 0.5 mmol), (5-bromo-1-methyl-2-oxo-1,2-dihydropyridin-4-yl) *tert*-butylmethylcarbamate (79 mg , 0.25 mmol), [1,1'-bis(diphenylphosphino)ferrocene]palladium(II) dichloride (18 mg, 0.025 mmol) and potassium carbonate (69 mg, 0.5 mmol) in 1,4-dioxane (2 mL) and water (0.2 mL). The mixture was heated to 80°C and stirred for 1 hour under protection of nitrogen. Filtered and spin dry, the residue was separated and purified with preparative-TLC (PE/EtOAc = 3/1) to obtain the title compound (112 mg, 83%). LCMS (ESI) $[M+H]^+$ = 543.2.

Step 4: 7-(4-fluorophenyl)-2-methyl-10-(ethylsulfonemethyl)-2*H*-benzo[*c*]pyrido[3,4-*e*]azepin-3(5*H*)-ket one

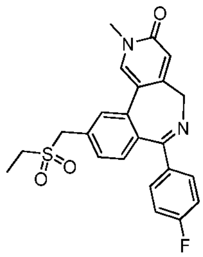

Trifluoroacetic acid (1 mL) and (5-(2-(4-fluorobenzoyl)-5-(ethylsulfonylmethyl)phenyl)-1-methyl-2-oxo-1,2-dihydropyridin-4-yl) methyl *tert*-butyl arbamate (112 mg, 0.214 mmol) were dissolved in dichloromethane (4 mL) and stirred at room temperature for 1 hours, then the solution was concentrated, the residue was separated and purified with preparative-HPLC to obtain the title compound (65 mg, 74%) in form of white solid. (ESI): $R_T$ (min)=3.06 min, [M+H]+ = 425.1; $^1$H NMR (400 MHz, DMSO-$d_6$) $\delta$ 8.07 (s, 1H), 7.71 (d, *J* = 1.2 Hz, 1H), 7.48 (m, 3H), 7.31 (d, *J* = 8.0 Hz, 1H), 7.22 (t, *J* = 8.8 Hz, 2H), 6.52 (s, 1H), 4.73 (d, *J* = 10.4 Hz, 1H), 4.60 (dd, *J* = 13.6 Hz, *J* = 36.8 Hz, 2H), 3.89 (d, *J* = 10.4 Hz, 1H), 3.50 (s, 3H), 3.14 (q, *J* = 7.2 Hz, 2H), 1.26 (t, *J* = 7.2 Hz, 3H).

**Embodiment 4010: 7-(4-chlorophenyl)-2-methyl-2*H*-benzo[*c*]pyrido[3,4-*e*]azepin-3(5*H*)-ketone**

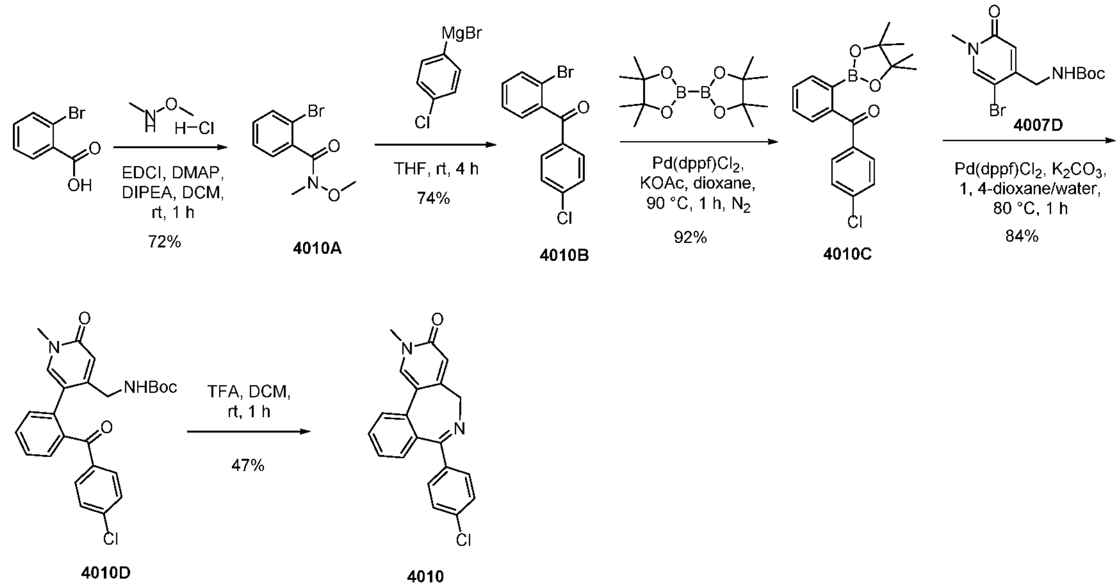


Step 1: 2-bromo-*N*-methoxy-*N*-methylbenzamide

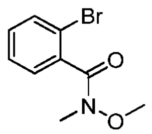

2-bromobenzoic acid (5.0 g, 25.0 mmol), *O*,*N*-dimethylhydroxylamine hydrochloride (4.88 g, 50.0 mmol), *N*-ethyl-*N*-(3-dimethylaminopropyl) Carbodiimide hydrochloride (14.4 g, 75.0 mmol), *N*,*N*-dimethylaminopyridine (0.31 g, 2.5 mmol) and diisopropylethylamine (25.8 g, 200 mmol) were dissolved in dichloromethane (300 mL), then stirred at room temperature for 1 hour, then concentrated. The residue was layered by EtOAc (100 mL) and water (200 mL), the organic phase was separated and the aquoues phase was extracted by EtOAc (30 mL*3). Combine the organic phases, then dried with anhydrous sodium sulfate, filtered and concentrated. The residue was separated and purified with flash chromatography (PE/EtOAc = 6/1) to obtain the title compound (4.4 g, 72%) in form of yellow oil. LCMS (ESI) $[M+H]^+$ = 244.0.

Step 2: (2-bromophenyl)(4-chlorophenyl)methanone

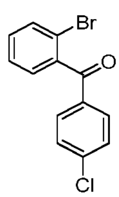

4-fluorophenylmagnesium bromide (1 M in tetrahydrofuran, 36 mL) was added to 2-bromo-*N*-methoxy-*N*-methylbenzamide (4.4 g, 18 mmol) in tetrahydrofuran (80 mL) solution at 0°C under nitrogen protection. The reaction solution was stirred at r.t. for 4 hours. The reaction was then quenched by water (30 mL), extracted by EtOAc (50 mL*3). Then combine the organic phases and washed by brine, dried over anhydrous sodium sulfate, filtered and concentrated, the crude product was separated and purified by flash chromatography (PE/EtOAc = 2/1) to obtain the title compound (3.92 g, 74%) in form of yellow oil.

Step 3: (4-Chlorophenyl)(2-(4,4,5,5-tetramethyl-1,3,2-dioxoborolan-2-yl)phenyl)methanone

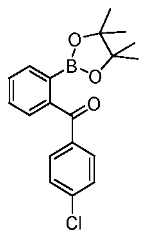

(2-bromophenyl)(4-chlorophenyl)methanone (1.0 g, 3.34 mmol), boronic acid pinacol ester (1.84 g, 7.28 mmol), [1,1'-bis(diphenyl)phosphyl)ferrocene]palladium(II) dichloride (266 mg, 0.36 mmol) and potassium carbonate (713 mg, 7.28 mmol) were mixed in 1,4-dioxane (40 mL). The reaction solution was stirred at 90°C for 1 hour under protection of nitrogen. Then filtered and concentrated, the residue was purified by preparative-TLC (PE/EtOAc = 10/1) to obtain the title compound (1.05 g, 92%) in form of yellow oil. LCMS (ESI) $[M+H]^+$= 343.1.

Step 4: (5-(2-(4-chlorobenzoyl)phenyl)-1-methyl-2-oxo-1,2-dihydropyridin-4-yl)methylcarbamic acid *tert*-butyl ester

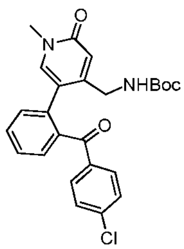

(4-Chlorophenyl)(2-(4,4,5,5-tetramethyl-1,3,2-dioxoborolan-2-yl)phenyl)methanone (200 mg, 0.58 mmol), (5-bromo-1-methyl-2-oxo-1,2-dihydropyridin-4-yl) *tert*-butylmethylcarbamate (92.0 mg, 0.29 mmol), [1,1'-bis(diphenylphosphino)ferrocene]palladium(II) dichloride (23 mg, 0.028 mmol) and potassium carbonate (80.5 mg, 0.58 mmol) were mixed in 1,4-dioxane (2 mL) and water (0.2 mL). The reaction mixture was heated to 80°C and stirred for 1 hour under nitrogen protection. Then filtered and spin dried, the residue was purified by preparative-TLC (PE/EtOAc = 1/1) to obtain the title compound (110 mg, 84%) in form of yellow solid. LCMS (ESI) $[M+H]^+$= 453.1.

Step 5: 7-(4-chlorophenyl)-2-methyl-2*H*-benzo[*c*]pyrido[3,4-*e*]azepin-3(5*H*)-ketone

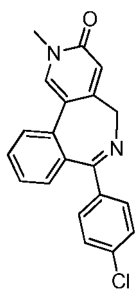

Trifluoroacetic acid (1 mL) and (5-(2-(4-chlorobenzoyl)phenyl)-1-methyl-2-oxo-1,2-dihydropyridin-4-yl) *tert*-butylmethylacarbamate (20 mg, 0.044 mmol) was dissolved in dichloromethane (4 mL) and stirred at r.t. for 2 hours. The reaction solution was then concentrated, the residue was purified by preparative-HPLC to obtain the title compound (7 mg, 47%) in form of white solid. (ESI): $R_T$ (min) = 7.486 min, $[M+H]^+$ = 335.1; $^1$H NMR (400 MHz, DMSO-$d_6$) $\delta$ 8.15

(s, 1H), 7.70 (d, *J* = 8.0 Hz, 1H), 7.63 (t, *J* = 7.2 Hz, 1H), 7.45 (m, 5H), 7.26 (d, *J* = 7.6 Hz, 1H), 6.51 (s, 1H), 4.74 (d, *J* = 10.0 Hz, 1H), 3.87 (d, *J* = 10.0 Hz, 1H), 3.49 (s, 3H).

**Embodiment 4011: 7-(4-chlorophenyl)-10-((ethylsulfone)methyl)-2-methyl-2,5-dihydro-3*H*-benzo[*c*]pyrido[3,4-*e*] Azazale-3-ketone**

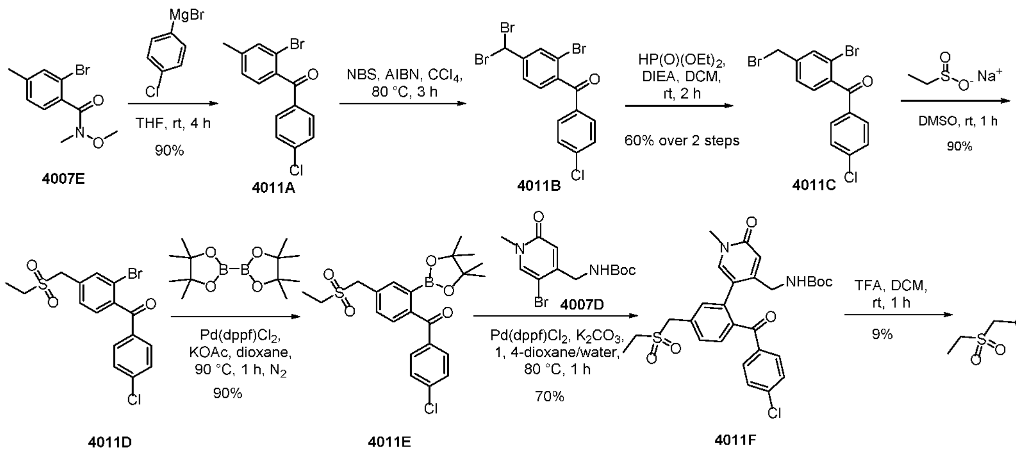


Step 1: (2-bromo-4-methylphenyl)(4-chlorophenyl)methanone

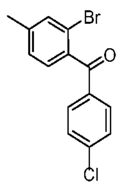

4-chlorophenylmagnesium bromide (1 M in tetrahydrofuran, 15.8 mL) was added to 2 - bromo - N - methoxy - N,4 - dimethylbenzamide (2g, 8 mmol) in tetrahydrofuran solution (100 mL) at 0°C under nitrogen protection. The reaction mixture was stirred at room temperature for 4 hours. The reaction was quenched with water (30 mL) and extracted by EtOAc (50 mL*3). Combine the organic phases and washed by brine, dried over anhydrous sodium sulfate, filtered and concentrated, the crude product was separated and purified by flash chromatography to obtain the title compound (1.8 g, 90%) in form of yellow solid.

Step 2: (2-bromo-4-(dibromomethyl)phenyl)(4-chlorophenyl)methanone

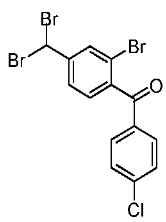

*N*-bromosuccinimide (1 g, 3 mmol), azobisisobutyronitrile (100 mg, 0.6 mmol) and (2-bromo-4-methylphenyl)(4-chlorophenyl)methanone (1.6 g, 9 mmol) was mixed in tetrachloride (30 mL), and the reaction was stirred at 80°C for 3 hours. The reaction mixture was spin dried and the obtained crude material (1 g, 100%) was used directly in the next step.

Step 3: (2-bromo-4-(bromomethyl)phenyl)(4-chlorophenyl)methanone

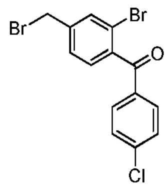

Diethyl phosphite (1.1 g, 4.5 mmol) and diisopropylethylamine (1.2 g, 9 mmol) were added to (2-bromo-4-(dibromomethyl)phenyl)(4-chloro phenyl)methanone (1 g, 2 mmol) in dichloromethane (30 mL). The reaction mixture was stirred at 20°C for 2 hours. Concentration of the reaction mixture, the residue was purified by flash chromatography to obtain the title compound (0.6 g, yield of the two steps 60%) in form of yellow solid. LCMS (ESI) $[M+H]^+$= 388.9.

Step 4: (2-bromo-4-(ethylsulfonylmethyl)phenyl)(4-chlorophenyl)methanone

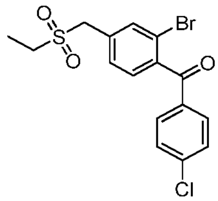

Sodium ethylsulfinate (180 mg, 1.55 mmol) was added to (2-bromo-4-(bromomethyl)phenyl)(4-chlorophenyl)methanone (300 mg, 0.77 mmol) in methylsulfoxide (2 mL). The reaction mixture was stirred at room temperature for 1 hour. EtOAc (15 mL) and water (15 mL) was added to layer the reaction mixture, organic phase was washed by brine (10 mL*3), dried over anhydrous sodium, filtered and concentrated to obtain the title compound (270 mg, 90%). LCMS (ESI) $[M+H]^+$= 400.9.

Step 5: (4-(ethylsulfonylmethyl)-2-(4,4,5,5-tetramethyl-1,3,2-dioxoborolan-2-yl)phenyl(4-chlorophenyl) methanone

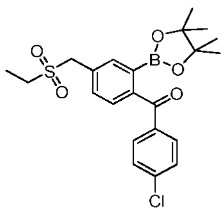

The compound (2-bromo-4-(ethylsulfonylmethyl)phenyl)(4-chlorophenyl)methanone (270 mg, 0.67 mmol), boronic acid pinacol ester (342 mg, 1.35 mmol), [1,1'-bis(diphenylphosphino)ferrocene]palladium(II) dichloride (40 mg, 0.05 mmol) and potassium acetate (198 mg, 2.01 mmol) were mixed in 1,4-dioxane (2 mL). The reaction mixture was heated to 90°C and stirred for 1 hour under nitrogen protection. Then filtered and spin dried, the residue was purified by preparative-TLC (PE/EtOAc = 8/1) to obtain the title compound (243 mg, 90%) in form of yellow solid. LCMS (ESI) $[M+H]^+$ = 449.1.

Step 6: (5-(2-(4-chlorobenzoyl)-5-(ethylsulfonylmethyl)phenyl)-1-methyl-2-oxo-1,2-dihydropyridine-4-yl) *tert*-butylmethylcarbamate

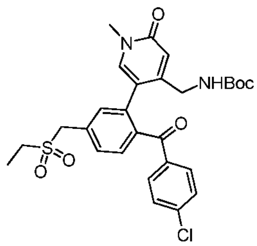

The compound (4-(ethylsulfonylmethyl)-2-(4,4,5,5-tetramethyl-1,3,2-dioxoborolan-2-yl)phenyl(4-Chlorophenyl) methanone (107 mg, 0.16 mmol), (5-bromo-1-methyl-2-oxo-1,2-dihydropyridin-4-yl) *tert*-butyl-*N*-methylacarbamate (50 mg, 0.16 mmol), [1,1'-bis(diphenylphosphino)ferrocene]palladium(II) dichloride (10 mg, 0.02 mmol) and potassium carbonate (66 mg, 0.48 mmol) were mixed in 1,4-dioxane (1 mL) and water (0.1 mL). The reaction mixture was heated to 80°C and stirred for 1hour under nitrogen protection. Then filtered and spin dried, the residue was purified by preparative-TLC (PE/EtOAc = 3/1) to obtain the title compound (37 mg, 70%) in form of yellow solid. LCMS (ESI) $[M+H]^+$ =559.2.

Step 7: 7-(4-chlorophenyl)-10-((ethylsulfone)methyl)-2-methyl-2,5-dihydro-3*H*-benzo[*c*]pyrido[3,4-*e*]aze pin-3-ketone

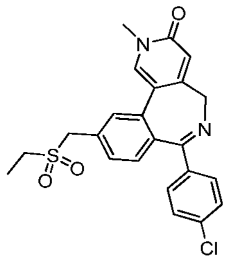

Trifluoroacetic acid (1 mL) and the compound (5-(2-(4-chlorobenzoyl)-5-(ethylsulfonylmethyl)phenyl)-1-methyl-2-oxo-1,2-dihydropyridin-4-yl) *tert*-butyl-*N*-methylacarbamate (37 mg, 0.07 mmol) was dissolved in dichloromethane (4 mL), the reaction mixture was stirred at r.t. for 1 hour, the reaction mixture was concentrated, the residue was purified by preparative-HPLC to obtain the title compound (3.5 mg, 9%) in form of white solid. (ESI): $R_T$ (min) = 4.011, $[M+H]^+$ = 441.1; $^1$H NMR (400 MHz, DMSO - $d_6$) $\delta$ 8.08 (s, 1H), 7.718 - 7.715 (d, *J* = 1.2 Hz, 1H), 7.49 - 7.43 (m, 5H), 7.32 - 7.31 (d, *J* = 8 Hz, 1H), 6.53 (s, 1H), 4.77 - 4.75 (d, *J* = 10.4 Hz, 1H), 4.67 - 4.54 (m, 2H), 3.91 - 3.89 (d, *J* = 18 Hz, 1H), 3.51 (s, 3H), 3.18 - 3.12 (m, 2H), 1.29 - 1.24 (m, 3H).

**Embodiment 4012: 7-(2,4-difluorophenyl)-2-methyl-10-(ethylsulfonemethyl)-2*H*-benzo[*c*]pyrido[3,4-*e*]azepin-3(5 *H*)-ketone**

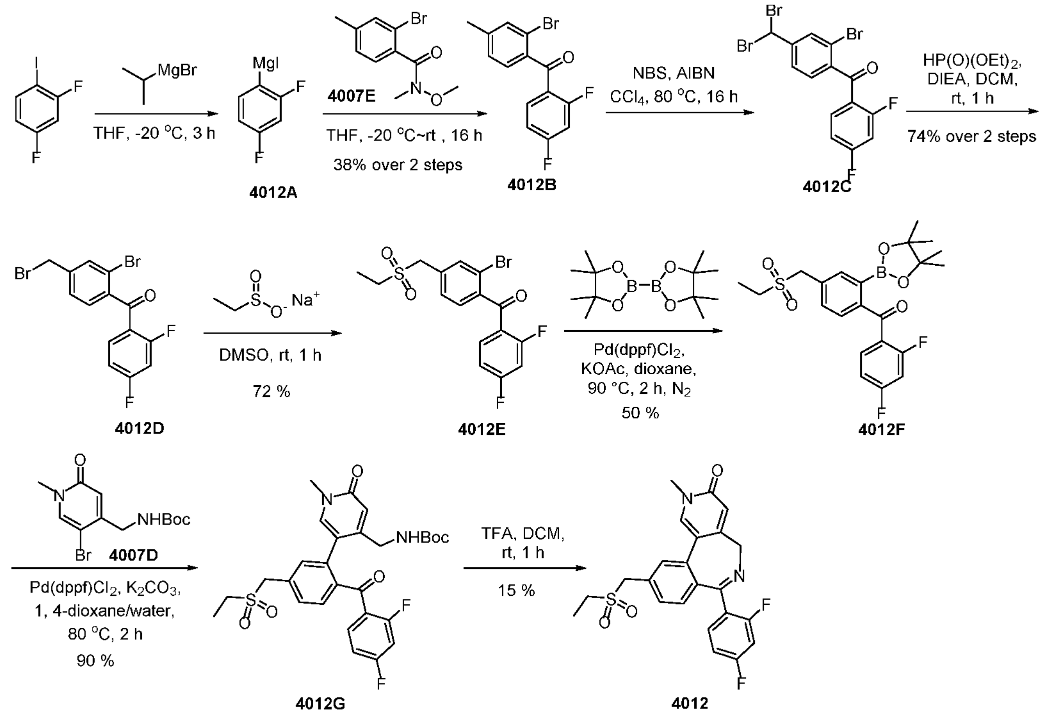


Step 1: (2,4-difluorophenyl)magnesium iodide

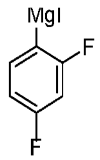

Isopropylmagnesium bromide (2 M in tetrahydrofuran, 16 mL, 32 mmol) was slowly added dropwise to a solution of 2,4-difluoro-1-iodobenzene (7.68 g, 32 mmol) in anhydrous tetrahydrofuran (16 mL) at -20°C under nitrogen protection. The reaction mixture was stirred at -20°C for 3 hours, (2,4-difluorophenyl)magnesium iodide (1 M in tetrahydrofuran) was obtained and used directly in the next step. GCMS [M-I] = 114.0.

Step 2: (2-Bromo-4-methylphenyl)(2,4-difluorophenyl)methanone

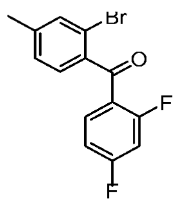

(2,4-difluorophenyl)magnesium iodide (1 M in tetrahydrofuran, 32 mL) was added to 2-bromo-*N*-methoxy-*N*,4-dimethylbenzamide (1.028 g, 4 mmol) in tetrahydrofuran (100 mL) at -20°C under nitrogen protection. The reaction mixture was stirred overnight. The reaction was quenched by saturated ammonium chloride (20 mL) and extracted by EtOAc (20 mL*3). The combined organic phases were washed by brine, dried over anhydrous sodium sulfate, the crude product was separated and purified by flash chromatography (PE/EtOAc = 20/1) to obtain the title compound (476 mg, yield of the two steps 38%) in form of yellow solid. GCMS (ESI) [M] = 310.0,312.0.

Step 3: (2-bromo-4-(dibromomethyl)phenyl)(2,4-difluorophenyl)methanone

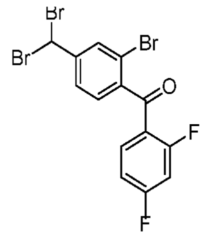

(2-bromo-4-methylphenyl)(2,4-difluorophenyl)methanone (400 mg, 1.5 mmol), *N*-bromosuccinimide (420 mg, 4.6 mmol) and azodiisobutyronitrile (25 mg, 0.15 mmol) were mixed in carbon tetrachloride (10 mL). The reaction mixture was stirred at 80°C for 16 hours, then the solvent was spin dried and obtained the crude product of the title compound (700 mg) in form of yellow solid, which may be used directly in the next step without further purification. LCMS (ESI) $[M+H]^+$= 467.0.

Step 4: (2-bromo-4-(bromomethyl)phenyl)(2,4-difluorophenyl)methanone

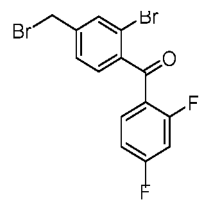

Diethyl phosphite (310 mg, 2.25 mmol) and diisopropylethylamine (580 mg, 4.5 mmol) were added to (2-bromo-4-(dibromomethyl)phenyl)(2,4-difluorophenyl)methanone (1 g, 2 mmol) in dichloromethane (10 mL). The reaction mixture was stirred at 20°C for 1 hour. Concentration of the reaction mixture, the crude product was separated and purified by flash chromatography (PE/EtOAc = 1/1) to obtain the title compound (432 mg, yield of the two steps 74%) in form of yellow solid. LCMS (ESI) $[M+H]^+$ = 389.0, 341.0.

Step 5: (2-bromo-4-(ethylsulfonylmethyl)phenyl)(2,4 - difluorophenyl)methanone

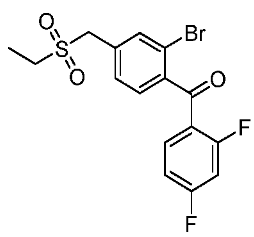

Sodium ethylsulfinate (387 mg, 3.339 mmol) was added to (2-bromo-4-(bromomethyl)phenyl)(2,4-difluorophenyl)methanone (432 mg, 1.113 mmol) in dimethylsulfoxide solution (10 mL). The reaction miture was stirred at r.t. for 1 hour. EtOAc (20 mL) and water (50 mL) was added to layer the mixture, the organic phase was washed by brine (50 mL*3), dried over anhydrous sodium sulfate, filtered and concentrated, the crude product was separated and purified by flash chromatography (PE/EtOAc = 1/1) to obtain the title compound (320 mg, 72%) in form of brown solid. LCMS (ESI) $[M+H]^+$= 403.0, 405.0.

Step 6: (4-(ethylsulfonylmethyl)-2-(4,4,5,5-tetramethyl-1,3,2-dioxoborolan-2-yl)phenyl(2,4-difluorophen yl)methanone

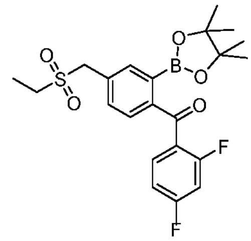

(2-bromo-4-(ethylsulfonylmethyl)phenyl)(2,4-difluorophenyl)methanone (320 mg, 0.8 mmol), bis(pinacolato)diboron (608 mg, 2.4 mmol), [1,1'-bis(diphenylphosphino)ferrocene]palladium(II) dichloride (58 mg, 0.08 mmol) and potassium acetate (235 mg, 2.4 mmol) are mixed in 1,4-dioxane (5 mL). The reaction mixture was heated to 90°C and stirred for 2hours under nitrogen protection. Then filtered and the fitrat was concentrate, the residu was separated and purified by flash chromatography (PE/EtOAc = 30/1) to obtain the title compound (180 mg, 50%) in form of yellow solid. LCMS (ESI) $[M+H]^+$ = 451.0.

Step 7: (5-(2-(2,4-difluorobenzoyl)-5-(ethylsulfonylmethyl)phenyl)-1-methyl-2-oxo-1,2-dihydropyridin-4 -yl)methyl *tert*-butylcarbamate

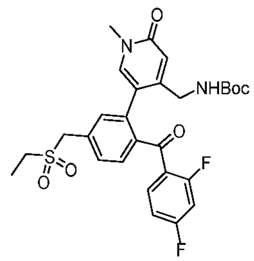

(4-(ethylsulfonylmethyl)-2-(4,4,5,5-tetramethyl-1,3,2-dioxoborolan-2-yl)phenyl(2,4-difl uorophenyl)methanone (154 mg, 0.348 mmol), (5-bromo-1-methyl-2-oxo-1,2-dihydropyridin-4-yl)methyl *tert*-butylcarbamate (55 mg, 0.174 mmol), [1,1'-bis(diphenylphosphino)ferrocene]palladium(II) dichloride (20 mg, 0.0174 mmol) and potassium carbonate (110 mg, 0.522 mmol) were mixed in 1,4-dioxane (4 mL) and water (0.4 mL). The reaction mixture was heated to 80°C and stirred for 1 hour under nitrogen protection. After filtration and spin dried, the residue was purified by flash chromatography (EtOAc/methanol = 30/1) to obtain the title compound (78 mg, 90%) in form of yellow solid. LCMS (ESI) $[M+H]^+$ = 561.0.

Step 8: 7-(2,4-difluorophenyl)-2-methyl-10-(ethylsulfonemethyl)-2*H*-benzo[c]pyrido[3,4-*e*]azepin-3(5*H*)-ketone

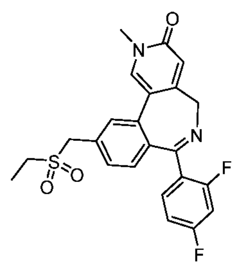

Trifluoroacetic acid (1 mL) and (5-(2-(2,4-difluorobenzoyl)-5-(ethylsulfonylmethyl)phenyl)-1-methyl-2-oxo-1,2-dihydropyridin-4 -yl)methyl *tert*-butylcarbamate (78 mg, 0.139 mmol) was dissolved in dichloromethane (4 mL), the reaction mixture was stirred at r.t. for 1 hour, the reaction mixture was then concentrated, the residue was purified by preparative-HPLC to obtain the title compound (9.2 mg, 15%) in form of white solid. LCMS (ESI): $[M+H]^+$ = 442.0; $^1H$ NMR (400 MHz, DMSO - $d_6$) $\delta$ 8.12 (s, 1H), 7.70 (s, 1H), 7.63 (dd, *J* = 8.4 Hz, *J* = 14.8 Hz, 1H), 7.43 (d, *J* = 8.0 Hz, 1H), 7.23 (m, 3H), 6.53 (s, 1H), 4.80 (d, *J* = 10.4 Hz, 1H), 4.61 (d, *J* = 13.2 Hz, 1H), 4.54 (d, *J* = 13.6 Hz, 1H), 3.91 (d, *J* = 9.6 Hz, 1H), 3.12 (q, *J* = 7.6 Hz, 2H), 1.24 (t, *J* = 7.6 Hz, 3H).

**Embodiment 4013: 7-(4-chloro-2-fluorophenyl)-10-((ethylsulfone)methyl)-2-methyl-2,5-dihydro-3*H*-benzo[*c*]pyrido[3,4-*e*]azepin-3-ketone**

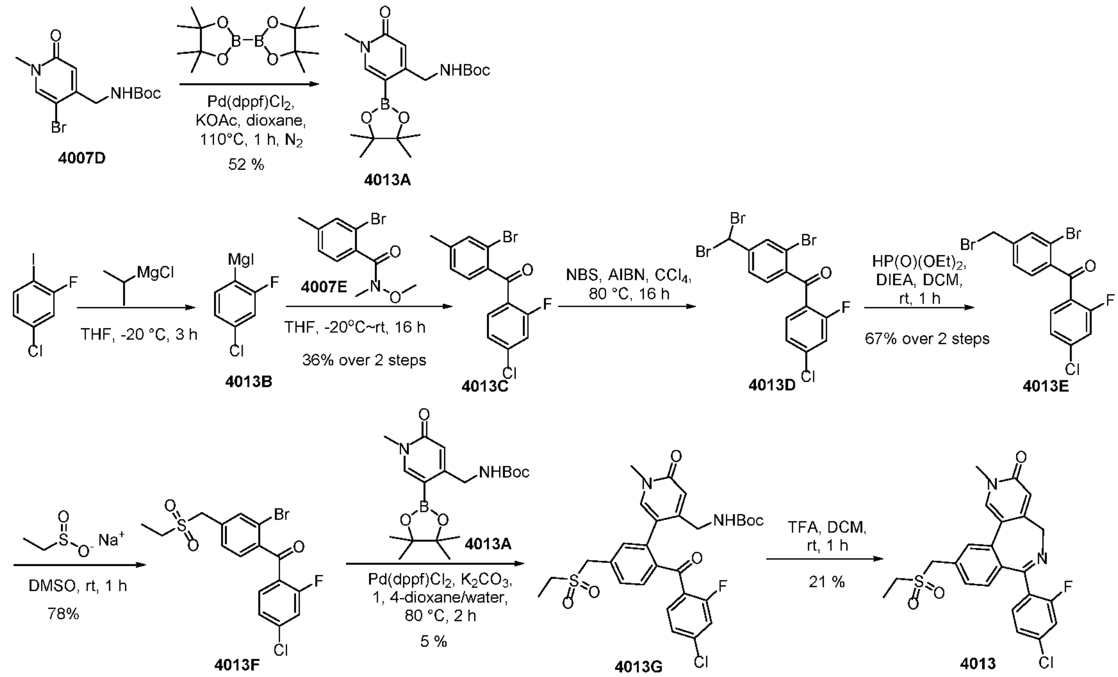


Step 1: (1-methyl-2-oxo-5-(4,4,5,5-tetramethyl-1,3,2-dioxaborolan-2-yl)-1,2-dihydropyridin-4-yl)methyl *tert*-butylcarbamate

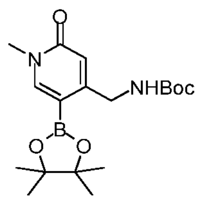

(5-Bromo-1-methyl-2-oxo-1,2-dihydropyridin-4-yl) *tert*-butyl-*N*-methylacarbamate (252 mg, 0.8 mmol), bis(pinacolato)diboron (608 mg, 2.4 mmol), [1,1'-bis(diphenylphosphino)ferrocene]palladium(II) dichloride (58 mg, 0.08 mmol) and potassium acetate (235 mg, 2.4 mmol) were mixed in 1,4-dioxane (5 mL). The reaction mixture was heated to 90°C and stirred for 2 hours under nitrogen protection. After filtration and concentration of the filtrate, the residue was purified by flash chromatography (EtOAc 100%) to obtain the title compound (151 mg, 52%) in form of yellow oil. LCMS (ESI) $[M+H]^+$ = 364.8.

Step 2: (4-chloro-2-fluorophenyl)magnesium iodide

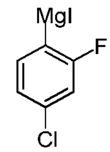

Isopropylmagnesium chloride (2 M in tetrahydrofuran, 16 mL, 32 mmol) was slowly added dropwise to 4-chloro-2-fluoro-1-iodobenzene (8.20 g, 32 mmol) in anhydrous tetrahydrofuran solution (16 mL) at -20°C under nitrogen protection. The reaction solution was stirred at -20°C for 3 hours to give (4-chloro-2-fluorophenyl)magnesium iodide (1M in tetrahydrofuran), which was used directly for the next reaction. GCMS [M-I] = 129.9.

Step 3: (2-Bromo-4-methylphenyl)(4-chloro-2-fluorophenyl)methanone

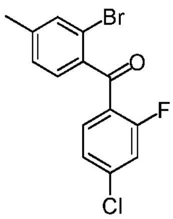

(4-chloro-2-fluorophenyl)magnesium iodide (1 M in tetrahydrofuran, 32 mL) was added to 2-bromo-*N*-methoxy-*N*,4-dimethylbenzamide (1.03 g, 4 mmol) in tetrahydrofuran (100 mL). The reaction mixture was stirred at room temperature overnight. The reaction was quenched by saturated ammonium chloride (20 mL) and extracted by EtOAc (20 mL*3). The combined organic phases were washed by brine, dried over anhydrous sodium sulfate, after filtration and concentration of the filtrate, the crude product was separated and purified by flash chromatography (PE/EtOAc = 20/1) to obtain the title compound (600 mg, yield of the two steps 36%) in form of yellow solid. GCMS (ESI) [M] = 326.0, 328.0.

Step 4: (2-bromo-4-(dibromomethyl)phenyl)(4-chloro-2-fluorophenyl)methanone

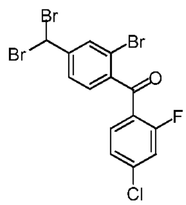

(2-bromo-4-methylphenyl)(4-chloro-2-fluorophenyl)methanone (600 mg, 1.83 mmol), *N*-bromosuccinimide (977 mg, 5.49 mmol) and azobisisobutyronitrile (29.5 mg, 0.18 mmol) were mixed in carbon tetrachloride (20 mL). The reaction mixture was stirred at 80°C for 16 hours, spin dried to obtain the crude product of the title compound (900 mg) in form of yellow solid, which is used directly without further purification in the nest step. LCMS (ESI) $[M+H]^+$ = 481.0,479.0

Step 5: (2-bromo-4-(bromomethyl)phenyl)(4-chloro-2-fluorophenyl)methanone

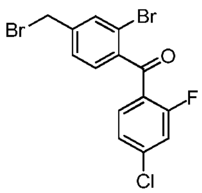

Diethyl phosphite (252.5 mg, 2.73 mmol) and diisopropylethylamine (708.3 mg, 5.49 mmol) were added into (2-bromo-4-(dibromomethyl)phenyl)(4-chloro-2-fluorophenyl)methanone (900 mg, 1.83 mmol) in dichloromethane (20 mL). The reaction mixture was stirred at 20°C for 1 hour. Concentration of the reaction mixture, the residue was purified by flash chromatography (PE/EtOAc = 1/1)to obtain the title compound (500 mg, yield of the two steps 67%) in form of yellow solid. LCMS (ESI) $[M+H]^+$ = 408.0, 406.0.

Step 6: (2-bromo-4-(ethylsulfonylmethyl)phenyl)(4-chloro-2-fluorophenyl)methanone

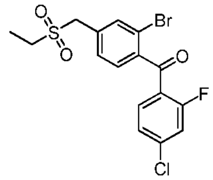

Sodium ethylsulfinate (428 mg, 3.69 mmol) was added to (2-bromo-4-(bromomethyl)phenyl)(4-chloro-2-fluorophenyl)methanone (500 mg, 1.23 mmol) ) in dimethylsulfoxide (10 mL). The reaction mixture was stirred at room temperature for 1 hour. EtOAc (20 mL) and water (50 mL) was added to layer the mixture, the organic phase was washed by brine (50 mL*3), dried over anhydrous sodium sulfate, after filtration and concentration of the filtrate, the residue was purified by flash chromatography (PE/EtOAc = 1/1) to obtain the title compound (400 mg, 78%) in form of brown solid. LCMS (ESI) $[M+H]^+$= 436.0, 438.0.

Step 7: (5-(2-(4-Chloro-2-fluorobenzoyl)-5-(ethylsulfonylmethyl)phenyl)-1-methyl-2-oxo-1,2-dihydrobut yridin-4-yl)methyl *tert*-butylcarbamate

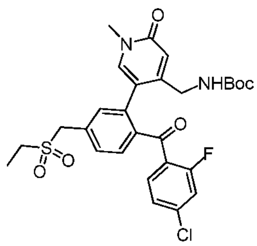

(2-bromo-4-(ethylsulfonylmethyl)phenyl)(4-chloro-2-fluorophenyl)methanone (90 mg, 0.21 mmol), (1-methyl-2-oxo-5-(4,4,5,5-tetramethyl-1,3,2-dioxaborolan-2-yl)-1,2-dihydropyridin-4-yl)methyl *tert*-butylcarbamate (151 mg, 0.41 mmol), [1,1'-bis(diphenylphosphino)ferrocene]palladium(II) dichloride (16 mg, 0.021 mmol) and potassium carbonate (89 mg, 0.36 mmol) were mixed in 1,4-dioxane (4 mL) and water (0.4 mL). The reaction mixture was heated to 80°C and stirred for 1 hour under nitrogen protection. After filtration and spin dried, the residue was purified by flash chromatography (PE/EtOAc = 30/1)to obtain the title compound (10 mg, 5%) in form of yellow solid. LCMS (ESI) $[M+H]^+$ = 576.1.

Step 8: 7-(4-chloro-2-fluorophenyl)-10-((ethylsulfonyl)methyl)-2-methyl-2,5-dihydro-3*H*-benzo[*c*]pyrido [3,4-*e*]azepin-3-ketone

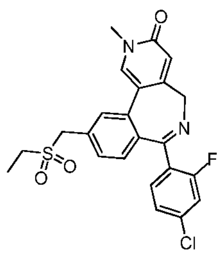

Trifluoroacetic acid (0.5 mL) and (5-(2-(4-chloro-2-fluorobenzoyl)-5-(ethylsulfonylmethyl)phenyl)-1-methyl-2-oxo-1,2-dihydropyri din-4-yl)methyl *tert*-butylcarbamate (10 mg, 0.017 mmol) were dissolved in dichloromethane (2 mL) and stirred at room temperature for 1 hour. After concentration, the residue was purified by preparative-HPLC to obtain the title compound (1.7 mg, 22%) in form of white solid. LCMS (ESI) $[M+H]^+$ = 458.0; $^1H$ NMR (400 MHz, $CD_3OD$) $\delta$ 8.16 (s, 1H), 7.78 (s, 1H), 7.59 - 7.55 (m, 1H), 7.53 - 7.51 (d, *J* = 8.0 Hz, 1H), 7.36 - 7.33 (m, 1H), 7.30 - 7.28 (d, *J* = 8.0 Hz, 1H), 7.25 - 7.22 (m, 1H), 6.65 (s, 1H), 4.82 - 4.79 (d, *J* = 12 Hz, 1H), 4.57 (s, 2H), 4.13 - 4.10 (d, *J* = 12 Hz, 1H), 3.69 (s, 3H), 3.17 - 3.12 (q, J = 7.6 Hz, 2H), 1.41 - 1.37 (t, *J* = 7.6 Hz, 3H).

**Embodiment 4014: (*S*)-2-(7-(4-fluorophenyl)-2-methyl-10-(methylsulfonylmethyl)-3-oxo-3,5-dihydro-2*H*-benzo[*c* ]pyrido[3,4-*e*]azepin-5-yl)acetamide**

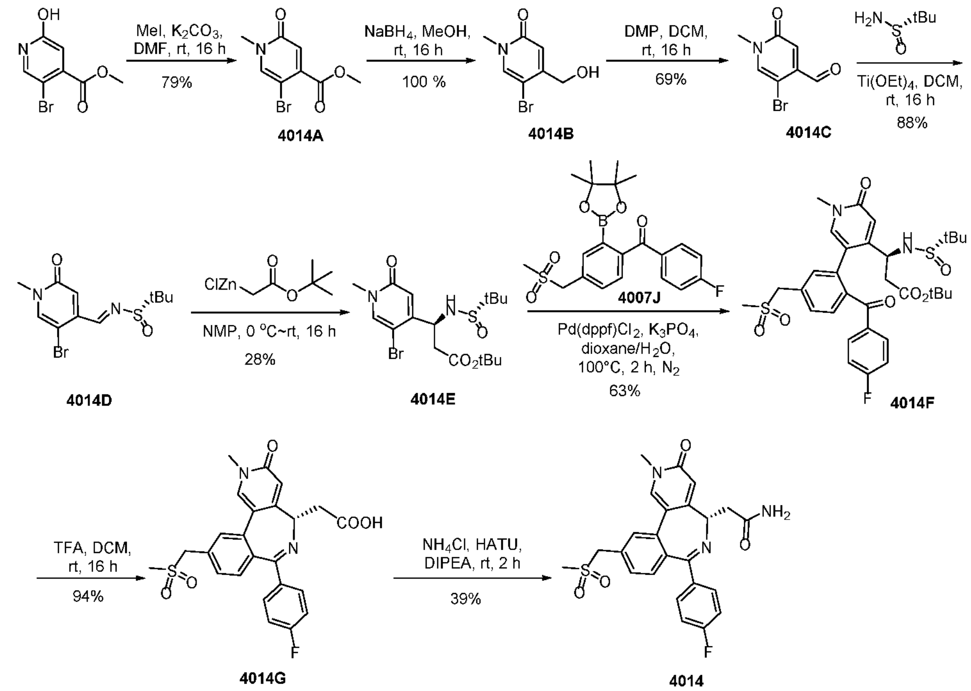


Step 1: 5-Bromo-1-methyl-2-oxo-1,2-dihydropyridine-4-methyl formate

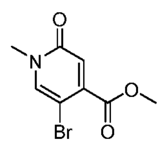

5-Bromo-2-hydroxyisonicotinate formate (3.00 g, 12.90 mmol), potassium carbonate (5.30 g, 38.70 mmol) and methyl iodide (2.00 g, 14.20 mmol) in *N,N*-dimethylformamide (20 mL) were mixed and stirred at room temperature overnight. After filtration and concentration of the filtrate. The residue was purified by reverse phase preparative-HPLC to obtain the title compound (2.5 g, 79%) in form of yellow solid. MS (ESI): m/z = 248.0 $[M+1]^+$

Step 2: 5-Bromo-4-(hydroxymethyl)-1-methylpyridine-2(1*H*)-ketone

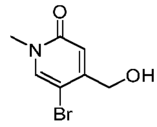

5-Bromo-1-methyl-2-oxo-1,2-dihydropyridine-4-methyl formate (2.46 g, 10 mmol) and sodium borohydride (1.14 g, 30 mmol) were mixed in methanol (20 mL) and stirred at room temperature overnight. Acetic acid (2 mL) was added to the reaction mixture and concentrated. The residue was purified by flash chromatography (PE/EtOAc = 10/1) to obtain the title compound (2.20 g, 100%) in form of white solid. MS (ESI): m/z = 218.0 $[M+1]^+$.

Step 3: 5-Bromo-1-methyl-2-oxo-1,2-dihydropyridine-4-aldehyde

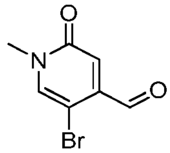

5-bromo-4-(hydroxymethyl)-1-methylpyridine-2(1*H*)-ketone (2.20 g, 10 mmol) and Dess-Martin periodinane (8.48 g, 20 mmol) were mixed in dichloromethane (50 mL), and stirred at room temperature overnight. The residue was purified by flash chromatography (PE/EtOAc = 2/1) to obtain the title compound (1.50 g, 69%) in form of yellow solid. b$^1$H NMR (400 MHz, $CDCl_3$) $\delta$ 10.13 (s, 1H), 7.61 (s, 1H), 7.04 (s, 1H), 3.59 (s, 3H).

Step 4: (*S*)-*N*-((5-bromo-1-methyl-2-oxo-1,2-dihydropyridin-4-yl)methylene)-2-methylpropan-2-Sulfona mide

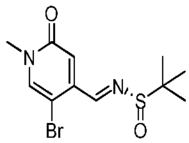

5-bromo-1-methyl-2-oxo-1,2-dihydropyridine-4-aldehyde (648 mg, 3 mmol), tetraethoxytitanium (1.37 g, 6 mmol) and (S)-2-methylpropane-2-sulfinamide (544.5 mg, 4.5 mmol) were dissolved in dichloromethane (30 mL), the reaction mixture was stirred at room temperature overnight. After concentration, the residue was purified by flash chromatography (dichloromethane/EtOAc = 10/1) to obtain the title compound (1.5 g, 88%) in form of yellow solid. MS (ESI): m/z = 321.0 $[M+1]^+$.

Step 5: (*S*)-3-(5-bromo-1-methyl-2-oxo-1,2-dihydropyridin-4-yl)-3-((*S*)-1,1-dimethyl ethylsulfonamide) *tert*-butylpropionate

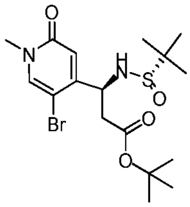

At 0°C, 2-*tert*-butoxy-2-carbonylethylzinc chloride (1 M in diethyl ether, 3 mL, 3 mmol) was slowly added dropwise to (*S*)-*N*-((5-bromo-1-methyl-2-oxo-1,2-dihydropyridin-4-yl)methylene)-2-methylpropane-2-sulfina mide (319 mg, 1 mmol) in 1-methyl-2-pyrrolidone (5 mL), the reaction solution was slowly warmed to room temperature and stirred

overnight. The reaction was quenched by water and extracted by EtOAc (20 mL*3). The organic phase was separated, dried over anhydrous sodium sulfate, filtered and concentrated. The residue was purified by flash chromatography (PE/EtOAc = 1/1), to obtain (*S*)-3-(5-bromo-1-methyl-2-oxo-1,2-dihydropyridin-4-yl)-3-((*S*)-1,1-dimethylethylsulfonamide) *tert*-butylpropionate (168 mg, 44%) in form of yellow solid, as well as its diastereomer (*R*)-3-(5-bromo-1-methyl-2-oxo-1,2-dihydropyridin-4-yl)-3-((*S*)-1,1-dimethylethylsulfonamide) *tert*-butylpropionate (108 mg, 28%) in form of yellow solid. MS (ESI): *m*/*z* = 381.0 [M+1]+.

Step 6: (*S*)-3-((*S*)-1,1-dimethylethylsulfinamide)-3-(5-(2-(4-fluorobenzoyl)-5-(methyl)sulfone methyl)phenyl)-1-methyl-2-oxo-1,2-dihydropyridin-4-yl)*tert*-butylpropionate

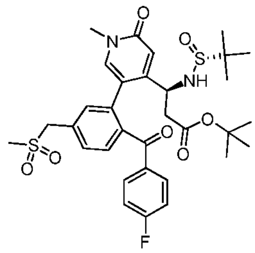

(*S*)-3-(5-bromo-1-methyl-2-oxo-1,2-dihydropyridin-4-yl)-3-((*S*)-1,1-dimethylethyl *tert*-butylpropionate (168 mg, 0.39 mmol), (4-fluorophenyl)(4-(methylsulfonylmethyl)-2-(4,4,5,5-tetramethyl)-1,3,2-dioxoborolan-2-yl)pheny l ketone (326 mg, 0.78 mmol), [1,1'-bis(diphenylphosphino)ferrocene]dichloride palladium(II) dichloride (29 mg, 0.039 mmol) and potassium phosphate (229 mg, 1.08 mmol) were mixed in 1,4-dioxane (5 mL) and water (1 mL). The reaction mixture was heated to 100°C and stirred for 2 hours under nitrogen protection. EtOAc and water was added to layer the mixture, the organic phase was separated and dried over anhydrous sodium sulfate, after filtration and concentration of the filtrate, the residue was purified by flash chromatography (PE/EtOAc = 1/10) to obtain the title compound (160 mg, 63%) in form of white solid. MS (ESI): *m*/*z* = 647.2 [M+1]+.

Step 7: (*S*)-2-(7-(4-fluorophenyl)-2-methyl-10-(methylsulfonemethyl)-3-oxo-3,5-dihydro-2*H*-benzeno[*c*]p yrido[3,4-*e*]azepin-5-yl)acetic acid

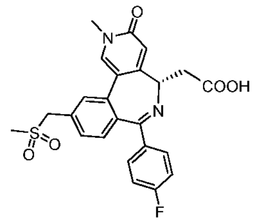

(*S*)-3-((*S*)-1,1-dimethylethylsulfinamide)-3-(5-(2-(4-fluorobenzoyl)-5-(methylsulfone)ph enyl)-1-methyl-2-oxo-1,2-dihydropyridin-4-yl) *tert*-butylpropanoate (160 mg, 0.25 mmol) was dissolved in trifluoroacetic acid (5 mL) and dichloromethane (5 mL), then stirred at room temperature overnight. After concentration, the crude product of the title compound is obtained in form of brown oil (110 mg, 94%), which was used directly in the next step without further purification. MS (ESI): m/z = 469.1 [M+1]+.

Step 8: (*S*)-2-(7-(4-fluorophenyl)-2-methyl-10-(methylsulfonemethyl)-3-oxo-3,5-dihydro-2*H*-benzeno[*c*]p yrido[3,4-*e*]azepin-5 -yl)acetamide

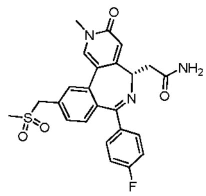

(*S*)-2-(7-(4-fluorophenyl)-2-methyl-10-(methylsulfonylmethyl)-3-oxo-3,5-dihydro-2*H*-b enzo[c]pyrido[3,4-e]azepin-5- yl)acetic acid (50 mg, 0.11 mmol), anhydrous ammonium chloride (12 mg, 0.22 mmol), 2-(7-azabenzene Triazole)-*N,N,N',N'*-tetramethylurea hexafluorophosphate (84 mg, 0.22 mmol) and *N*-ethyl-*N*-isopropylpropan-2-amine (43 mg, 0.33 mmol) were mixed in *N,N*-dimethylformamide (5 mL), the reaction mixture was stirred at r.t. for 2 hours. After filtration and concentration of the filtrate, the residue was purified by preparative-HPLC to obtain the title compound (20 mg, 39%) in form of white solid. MS (ESI): m/z = 468.1 [M+H]$^+$; $^1$H NMR (400 MHz, DMSO - $d_6$) $\delta$ 8.05 (s, 1H), 7.74 (s, 1H), 7.66 (s, 1H), 7.52 (d, *J* = 8.4 Hz, 1H), 7.50 - 7.44 (m, 2H), 7.34 (d, *J* = 8.0 Hz, 1H), 7.24 (d, *J* = 8.8 Hz, 2H), 6.97 (s, 1H), 6.42 (s, 1H), 4.65 (q, *J* = 14.0, 42.8 Hz, 2H), 4.24 (t, *J* = 6.8 Hz, 1H), 3.50 (s, 3H), 3.04 (s, 3H), 2.99 - 2.95 (m, 2H).

**Embodiment 4015: (S)-*N*-ethyl-2-(7-(4-fluorophenyl)-2-methyl-10-(methylsulfonemethyl)-3-oxo-3,5-dihydro-2*H*-benzo[*c*]pyrido[3,4-*e*]azepin-5-yl)acetamide**

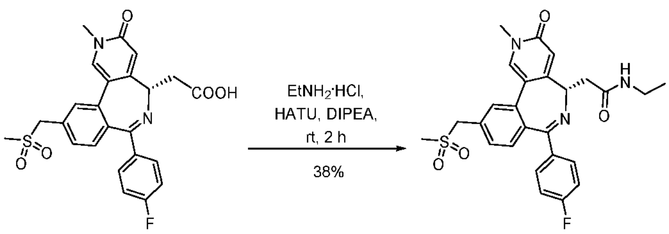


4014G 4015

Step 1: (*S*)-*N*-ethyl-2-(7-(4-fluorophenyl)-2-methyl-10-(methylsulfonemethyl)-3-oxo-3,5-dihydrogen-2*H*-benzo[*c*]pyrido[3,4-*e*]azepin-5-yl)acetamide

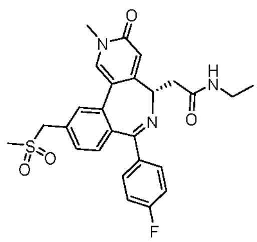

(*S*)-2-(7-(4-fluorophenyl)-2-methyl-10-(methylsulfonylmethyl)-3-oxo-3,5-dihydro-2*H*-b enzo[c]pyrido[3,4-e]azepin-5-yl)acetic acid (50 mg, 0.11 mmol), ethylamine hydrochloride (18 mg, 0.22 mmol), 2-(7-azabenzene Triazole)-*N,N,N',N'*-tetramethylurea hexafluorophosphate (84 mg, 0.22 mmol) and *N*-ethyl-*N*-isopropylpropan-2-amine (71 mg, 0.55 mmol) were mixed in *N,N*-dimethylformamide (5 mL) and stirred at room temperature for 2 hours. After filtration and concentration of the filtrate, the residue was purified by preparative-HPLC to obtain the title compound (20 mg, 38%) in form of white solid. MS (ESI): m/z = 496.2 [M+H]$^+$; $^1$H NMR (400 MHz, DMSO - $d_6$) $\delta$ 8.21 (t, *J* = 5.2 Hz, 1H), 8.05 (s, 1H), 7.74 (s, 1H), 7.52 (d, *J* = 8.4 Hz, 1H), 7.49 - 7.42 (m, 2H), 7.34 (d, *J* = 8.0 Hz, 1H), 7.24 (t, *J* = 8.0 Hz, 2H), 6.42 (s, 1H), 4.65 (q, *J* = 13.6, 41.4 Hz, 2H), 4.24 (t, *J* = 6.8 Hz, 1H), 3.50 (s, 3H), 3.14 - 3.06 (m, 2H), 3.04 (s, 3H), 2.99 - 2.95 (m, 2H), 1.04 (t, *J* =7.2 Hz, 3H) .

**Embodiment 4016: (*S*)-2-(7-(4-chlorophenyl)-2-methyl-3-oxo-3,5-dihydro-2H-benzo[*c*]pyrido[3,4-e]azepin-5-yl)-N-ethylacetamide**

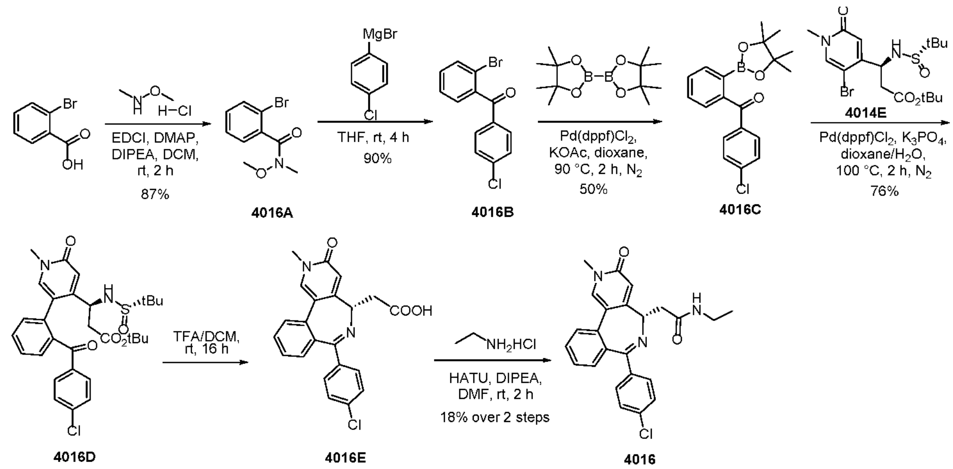


Step 1: 2-bromo-*N*-methoxy-*N*-methylbenzamide

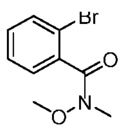

2-bromo-benzoic acid (4.02 g, 20.00 mmol), *N,O*-dimethylhydroxylamine hydrochloride (5.85 g, 60 mmol) and *N*-ethyl-*N'*-(3-dimethylaminopropyl) carbodiimide hydrochloride (7.71 g, 40.00 mmol), N,N - dimethylaminopyridine (244 mg, 2.00 mmol), diisopropylethylamine (5.17 g, 40.00 mmol) were mixed in dichloromethane (100 mL), stirred at 20°C for 1 hour and then concentrated. EtOAc (100 mL) and water (100 mL) was added to layer the mixture, the organic phase was separated and the aqueous phase then extracted by EtOAc (30 mL*3), the combined organic phases were washed by brine, dried over anhydrous sodium sulfate, filtered and concentrated. The residue was purified by flash chromatography (PE/EtOAc = 6/1) to obtain the title compound (4.25 g, 87%) in form of yellow oil. LCMS (ESI) $[M+H]^+$= 246.0.

Step 2: (2-bromo-phenyl)(4-chlorophenyl)methanone

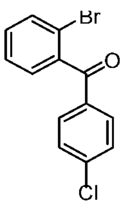

4-chlorophenylmagnesium bromide (1 M in tetrahydrofuran, 30 mL) was slowly added dropwise to 2-bromo-*N*-methoxy-*N*-methylbenzamide (3.66 g, 15.00 mmol) in tetrahydrofuran (100 mL) in solution at 0°C under nitrogen protection. The reaction was quenched by water (30 mL) and extracted by EtOAc (50 mL*3). The combined organic phases were washed by brine, dried over anhydrous sodium sulfate, after filtration and concentration of the filtrate, the crude product was separated and purified by flash chromatography to obtain the title compound (4 g, 90%) in form of yellow solid.

Step 3: (4-chlorophenyl)(2-(4,4,5,5-tetramethyl-1,3,2-dioxaborolan-2-yl)phenyl)methanone

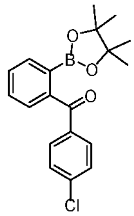

(2-bromo-phenyl)(4-chlorophenyl)methanone (2.00 g, 6.77 mmol), boronic acid pinacol ester (5.16 g, 20.31 mmol), [1,1'-bis(diphenyl)phosphyl)ferrocene]palladium(II) dichloride (498 mg, 0.68 mmol) and potassium carbonate (1.99 g, 20.31 mmol) were mixed in 1,4-dioxane (20 mL). The reaction mixture was heated to 90°C and stirred for 2 hours under nitrogen protection. After filtration and concentration of the filtrate, the residue was purified by flash chromatography (PE/EtOAc = 7/1) to obtain the title compound (1.2 g, 50%) in form of yellow solid. LCMS (ESI) $[M+H]^+$ = 343.1.

Step 4: (*S*)-3-(5-(2-(4-chlorobenzoyl)phenyl)-1-methyl-2-oxo-1,2-dihydropyridin-4-yl)-3-((*S*)-1,1-dimeth ylethylsulfinamide) *tert*-butylpropionate

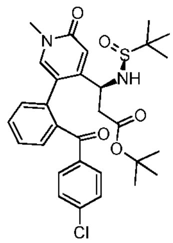

(*S*)-3-(5-bromo-1-methyl-2-oxo-1,2-dihydropyridin-4-yl)-3-((*S*)-1,1-dimethylethyl *tert*-butylpropionate (180 mg, 0.41 mmol), (4-chlorophenyl)(2-(4,4,5,5-tetramethyl-1,3,2-dioxaboron)-2-yl)phenyl)methanone (281 mg, 0.82 mmol), [1,1'-bis(diphenylphosphino)ferrocene]palladium(II) dichloride (30 mg, 0.042mmol) and potassium phosphate (229 mg, 1.08 mmol) were mixed in 1,4-dioxane (10 mL) and water (1 mL). The reaction mixture was heated to 100°C and stirred for 2 hours under nitrogen protection. EtOAc and water was added to layer the mixture, the organic phase was dried over anhydrous sodium sulfate, after filtration and concentration of the filtrate, the residue was purified by flash chromatography (PE/EtOAc = 1/10) to obtain the title compound (180 mg, 76%) in form of white solid. MS (ESI): *m/z* = 571.2 $[M+1]^+$.

Step 5: (*S*)-2-(7-(4-chlorophenyl)-2-methyl-3-oxo-3,5-dihydro-2*H*-benzo[*c*]pyrido[3,4-*e*]azepin-5-yl)aceti c acid

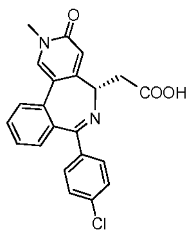

(*S*)-3-(5-(2-(4-chlorobenzoyl)phenyl)-1-methyl-2-oxo-1,2-dihydropyridin-4-yl)-3-((*S*)-1, 1-dimethylethylsulfinamide) *tert*-butylpropionate (180 mg, 0.32 mmol) were dissolved in trifluoroacetic acid (5 mL) and dichloromethane (5 mL), the reaction mixture was stirred at r.t. overnigh. After concentration of the mixture, the title compound was obtained in form of brown oil (160 mg), which is used diredctly in the next step without further purification. MS (ESI): m/z = 393.1

$[M+1]^+$.

Step 6: (*S*)-2-(7-(4-chlorophenyl)-2-methyl-3-oxo-3,5-dihydro-2*H*-benzo[*c*]pyrido[3,4-*e*]azepin-5-yl)-*N*-et hylacetamide

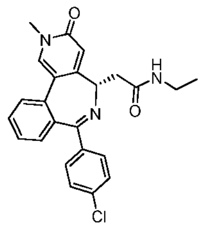

(*S*)-2-(7-(4-chlorophenyl)-2-methyl-3-oxo-3,5-dihydro-2H-benzo[*c*]pyrido[3,4-*e*]azepin-5-yl)acetic acid (50 mg, 0.13 mmol), ethylamine hydrochloride (21 mg, 0.26 mmol), 2-(7-azabenzotriazole)-*N,N,N',N'*-tetramethylurea hexafluorophosphate (99 mg, 0.26 mmol) and *N*-ethyl-*N*-isopropylpropan-2-amine (34 mg, 0.26 mmol) were mixed in *N,N*-dimethylcarbamide (5 mL), the reaction mixture was stirred at r.t. for 2 hours. After filtration and concentration of the filtrate, the residue was purified by preparative-HPLC to obtain the title compound (10 mg, 18%) in form of white solid. MS (ESI): m/z = 420.1 $[M+H]^+$; $^1H$ NMR (400 MHz, DMSO - $d_6$) $\delta$ 8.26 - 8.17 (m, 1H), 8.11(s, 1H), 7.76 - 7.62 (m, 2H), 7.54 - 7.35(m, 5H), 7.29 (d, *J* = 6.8 Hz, 1H), 6.40 (s, 1H), 4.30 - 4.19 (m, 1H), 3.48 (s, 3H), 3.16 - 3.02 (m, 2H), 3.00 - 2.90 (m, 2H), 1.09 - 0.97 (m, 3H).

**Embodiment 4017: (*S*)-2-(7-(4-chlorophenyl)-2-methyl-3-oxo-3,5-dihydro-2*H*-benzo[*c*]pyrido[3,4-*e*]azepin-5-yl)a cetamide**

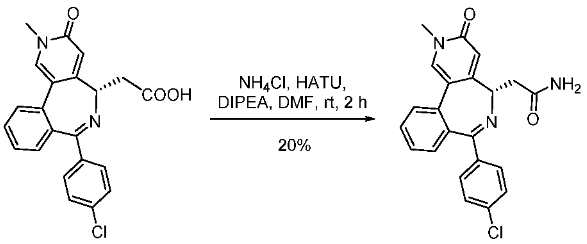


**4016E** **4017**

Step 1: (*S*)-2-(7-(4-chlorophenyl)-2-methyl-3-oxo-3,5-dihydro-2*H*-benzo[*c*]pyrido[3,4-*e*]azepin-5-yl)aceta mide

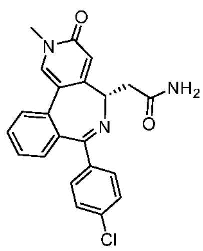

(*S*)-2-(7-(4-chlorophenyl)-2-methyl-3-oxo-3,5-dihydro-2*H*-benzo[*c*]pyrido[3,4-*e*]azepin-5-yl)acetic acid (50 mg, 0.11 mmol), anhydrous ammonium chloride (14 mg, 0.26 mmol), 2-(7-azabenzotriazole)-*N,N,N',N'*-tetramethylurea hexafluorophosphate (99 mg, 0.26 mmol) and *N*-ethyl-*N*-isopropylpropan-2-amine (34 mg, 0.26 mmol) were mixed in *N,N*-dimethylcarbamide (5 mL), then sterred at r.t. for 2 hours. After filtration and concentration of the filtrate, the residue was purified by preparative-HPLC to obtain the title compound (10 mg, 20%) in form of white solid. MS (ESI): m/z = 392.1 $[M+H]^+$; $^1H$ NMR (400 MHz, DMSO - $d_6$) $\delta$ 8.08 (s, 1H), 7.76 - 7.63(m, 2H), 7.54 - 7.35 (m, 5H), 7.32 (d, *J* = 7.6

Hz, 1H), 6.59 (s, 1H), 4.50 - 4.40 (m, 1H), 3.66 (s, 3H), 3.28 - 3.20 (m, 1H), 3.07 - 2.95 (m, 1H), 1.17 - 1.08 (m, 2H).

**Embodiment 4032: (*S*)-2-(7-(4-chlorophenyl)-2-methyl-3-oxo-3,5-dihydro-2*H*-benzo[*c*]pyrido[3,4-*e*]azepin-5-yl)-*N*-isopropylacetamide**

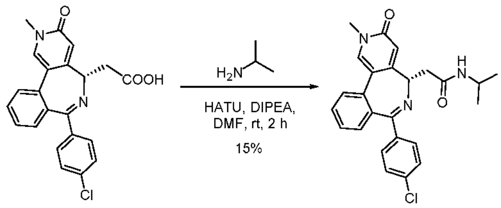


4016E 4032

Step 1: (*S*)-2-(7-(4-chlorophenyl)-2-methyl-3-oxo-3,5-dihydro-2*H*-benzo[*c*]pyrido[3,4-*e*]azepin-5-yl)-*N*-isopropylacetamide

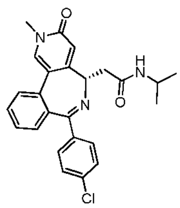

(*S*)-2-(7-(4-chlorophenyl)-2-methyl-3-oxo-3,5-dihydro-2*H*-benzo[*c*]pyrido[3,4-*e*]azepin-5-yl)acetic acid (90 mg, 0.23 mmol), isopropylamine (27 mg, 0.46 mmol), 2-(7-azabenzotriazole)-*N,N,N',N'*-tetramethylurea hexafluorophosphate (175 mg, 0.46 mmol) and *N*-ethyl-*N*-isopropylpropan-2-amine (119 mg, 0.92 mmol) were mixed in *N,N*-dimethyl formamide (5 mL) and stirred at room temperature for 2 hours. The reaction mixture was then poured into wate (30 mL) and extracted by EtOAc (50 mL*2). The combined organic phases were washed by brine, dried over anhydrous sodium sulfate, filtered and concentrated. The residue was purified by preparative-HPLC to obtain the title compound (15 mg, 15%) in form of white solid. MS (ESI): m/z = 434.0 $[M+H]^+$; $^1H$ NMR (400 MHz, DMSO - $d_6$) $\delta$ 8.13 (s, 1H), 8.11 (s, 1H), 7.73 (d, *J* = 8.0 Hz, 1H), 7.67 (t, *J* = 7.6 Hz, 1H), 7.51 - 7.38 (m, 5H), 7.29 (d, *J* = 7.6 Hz, 1H), 6.40 (s, 1H), 4.24 (t, *J* = 6.8 Hz, 1H), 3.89 - 3.78 (m, 1H), 3.49 (s, 3H), 3.00 - 2.86 (m, 2H), 1.10 (d, *J* =6.8 Hz, 3H), 1.05 (t, *J* = 6.4 Hz, 3H).

**Comparative embodiment 4018: *N*-(7-(4-fluorophenyl)-2-methyl-3-oxo-3,5-dihydro-2*H*-benzo[*c*]pyrido[3,4-*e*]azapin-10-yl)eth anesulfonamide**

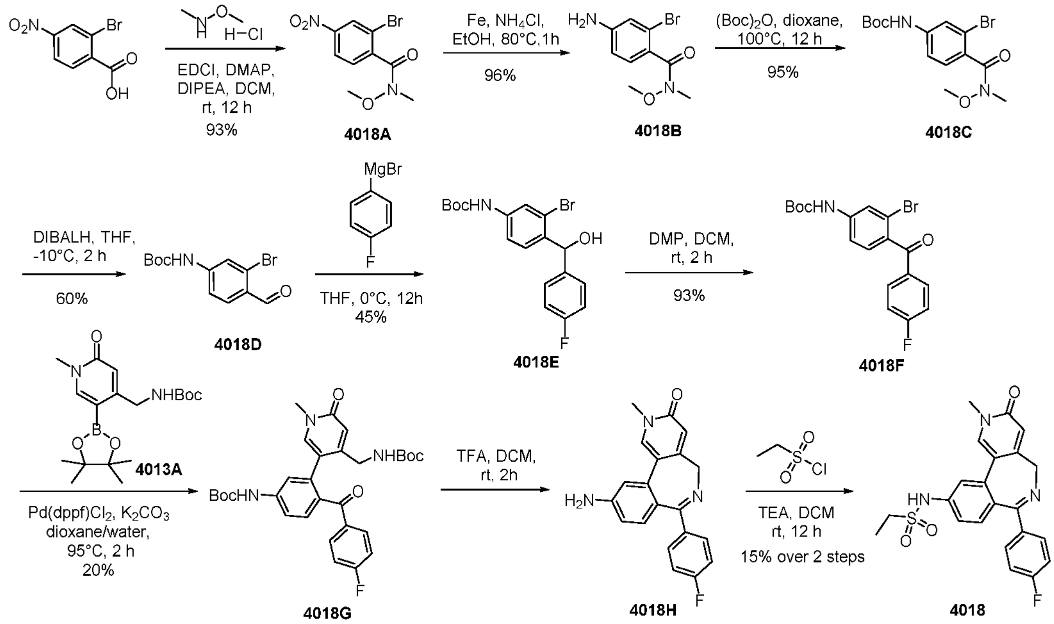


Step 1: 2-bromo-*N*-methoxy-*N*-methyl-4-nitrobenzamide

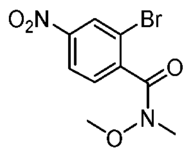

2-bromo-4-nitrobenzoic acid (2 g, 8.1 mmol), *N,O*-dimethylhydroxylamine hydrochloride (2.43 g, 24.3 mmol) and *N*-ethyl-*N'*-(3-dimethylaminopropyl)carbodiimide hydrochloride (3.1 g, 16.2 mmol), *N,N*-dimethylaminopyridine (99 mg, 0.81 mmol), diisopropylethylamine (2.1 g, 16.2 mmol) were mixed in dichloromethane (50 mL) and stir at 20 °C for 1 hour then concentrated. EtOAc (30 mL) and water (20 mL) was added to layer the mixture, the organic phase was separated and the aqueous phase was extracted by EtOAc (20 mL*3). The combined organic phases were washed by brine, dried over anhydrous sodium sulfate. The residue was purified by flash chromatography (PE/EtOAc = 6/1) to obtain the title compound (2.2 g, 93%) in form of yellow solid. LCMS (ESI) $[M+H]^+$ = 289.0.

Step 2: 4-amino-2-bromo-*N*-methoxy-*N*-methylbenzamide

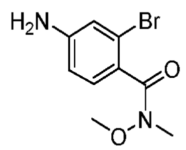

2-bromo-*N*-methoxy-*N*-methyl-4-nitrobenzamide (2.2 g, 7.6 mmol), iron powder (2.12 g, 38.1 mmol) and ammonium chloride (814 mg, 15.2 mmol)) were mixed in ethanol (40 ml), then heated to 80°C and stirred for 1 hour. The reaction mixture was quenched with water (20 mL) and extracted by EtOAc (30 mL*3). The combined organic phases were washed by brine (20 mL), dried over anhydrous sodium sulfate, filtered and concentrated, the residue was purified by flash chromatography (PE/EtOAc = 3/1) to obtain the title compound (1.9 g, 96%) in form of yellow solid. LCMS (ESI) $[M+H]^+$ = 259.0.

Step 3: 3-bromo-4-(methoxy(methyl)carbamoyl)phenyl-*tert*-butyl-carbamate

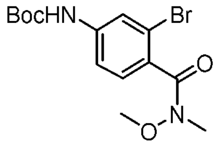

4-amino-2-bromo-*N*-methoxy-*N*-methylbenzamide (1.9 g, 7.3 mmol) and di-*tert*-butyl dicarbonate (15.9 g, 73 mmol) were dissolved in dioxane (30 mL), heated to 100°C and stirred for 12 hours. After filtration and concentration of the filtrate, the residue was purified by flash chromatography (PE/EtOAc = 6/1) to obtain the title compound (2.5 g, 95%) in form of yellow solid. LCMS (ESI) $[M+H]^+$ = 259.0.

Step 4: *Tert* - Butyl 3-bromo-4-formaldehyde-benzyl-amino-*tert* butyl formate

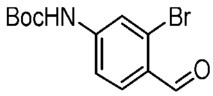

Isobutylaluminum hydride (1 M in tetrahydrofuran, 11.2 mL, 11.2 mmol) was added slowly into a solution of 3-bromo -4-(methoxy(methyl)carbamoyl) *tert*-butylphenylcarbamate (1 g, 2.8 mmol) in tetrahydrofuran (40 mL) at -10°C, and the mixture was stirred at this temperature for 2 hours. The reaction was quenched by the addition of sodium sulfate decahydrate (5 g), stirring was continued for half an hour, filtered, and the filtrate was concentrated. The residue was purified by flash chromatography (PE/EtOAc = 8/1) to obtain the title compound (500 mg, 60%) in form of yellow solid. LCMS (ESI) $[M+H]^+$ = 300.0.

Step 5: 3-bromo-4-((4-fluorophenyl)(hydroxymethyl) *tert*-butylphenylcarbamate

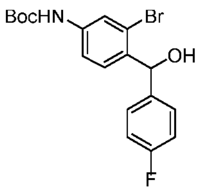

4-fluorophenylmagnesium bromide (1 M in tetrahydrofuran, 8.3 mL) was slowly added dropwise into to 3-bromo-4- formaldehyde-tert butyl-phenylcarbamate (500 mg, 1.7 mmol) in tetrahydrofuran (2 mL). The reaction mixture was stirred at r.t. overnight, the reaction was quenched by saturated ammonium chloride (20 mL) and extracted by EtOAc (20 mL*3). The combined organic phases were washed by brine, dried over anhydrous sodium sulfate, after filtration and concentration of the filtrate, the residue was purified by flash chromatography (PE/EtOAc = 5/1) to obtain the title compound (300 mg, 45%) in form of yellow oil. LCMS (ESI) $[M+H]^+$= 395.0.

Step 6: 3-bromo-4-(4-fluorobenzoyl) *tert*-butylphenylcarbamate

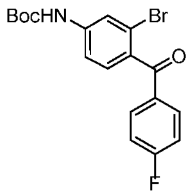

3-Bromo-4-((4-fluorophenyl)(hydroxymethyl) *tert*-butylphenylcarbamate (300 mg, 0.76 mmol) and Dess-Martin periodinane (482 mg, 1.14 mmol) were mixed in dichloromethane (5 mL), and stirred at r.t. for 2 hours. After filtration and concentration of the filtrate, the residue was purified by preparative-TLC (PE/EtOAc = 15/1) to obtain the title compound (277 mg, 93%) in form of yellow oil. LCMS (ESI) [M+H]+ =393.0.

Step 7: ((5-(5-((*tert*-butoxycarbonyl)amino)-2-(4-fluorobenzoyl)phenyl)-1-methyl-2-oxo-1,2-dihydropyri din-4-yl)methyl) *tert*-butylcarbamate

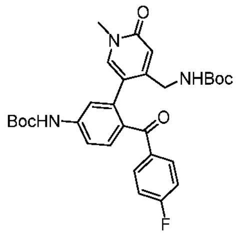

3-Bromo-4-(4-fluorobenzoyl) *tert*-butylphenylcarbamate (277 mg, 0.70 mmol), (1-methyl-2-oxo-5-(4,4,5,5-tetramethyl-1,3,2-dioxaborolan-2-yl)-1,2-dihydropyridin-4-yl)methylc arbamate (386 mg, 1.05 mmol), [1,1'-bis(diphenylphosphino) ferrocene]palladium(II) dichloride (51 mg, 0.07 mmol) and potassium carbonate (291 mg, 2.1 mmol) were mixed in 1,4-dioxane (3 mL) and water (0.3 mL). The reaction mixture was heated to 90°C and stirred for 1 hour under nitrogen protection. After filtration and concentration of the filtrate, the residue was purified by preparative-TLC (PE/EtOAc = 1/1) to obtain the title compound (78 mg, 20%) in form of yellow oil. LCMS (ESI) [M+H]+ =552.3.

Step 8: 10-amino-7-(4-fluorophenyl)-2-methyl-2,5-dihydro-3*H*-benzo[*c*]pyrido[3,4-*e*]azepin-3-ketone

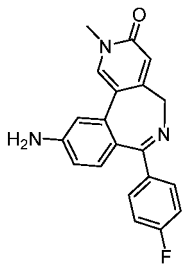

Trifluoroacetic acid (1 mL) was added dropwise to ((5-(5-((tert-butoxycarbonyl)amino)-2-(4-fluorobenzoyl)phenyl)-1-methyl-2-oxo-1,2-dihydropyrid in-4-yl)methyl) *tert*-butylcarbamate (78 mg, 0.07 mmol) in dichloromethane (4 mL), the reaction mixture was stirred at r.t. for 1 hour under nitrogen protection, then concentrated, there residue (95mg) was used directly in the next step. LCMS (ESI) $[M+H]^+$= 334.1.

Step 9: *N*-(7-(4-fluorophenyl)-2-methyl-3-oxo-3,5-dihydro-2*H*-benzo[*c*]pyrido[3,4-*e*]azepin-10-yl)ethanes ulfonamide

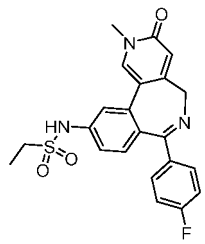

Ethylsulfonyl chloride (180 mg, 1.4mmol) was added into 10-amino-7-(4-fluorophenyl)-2-methyl-2,5-dihydro-3*H*-benzo[*c*]pyrido[3,4-*e*]azepin-3-keone (95 mg, 0.28 mmol) and triethylamine (283 mg, 2.8 mmol) in dichloromethane (3 mL). The reaction solution was stirred at r.t. overnight. Reaction mixture was washed by water and brine, dried over anhydrous sodium sulfate, filtered and concentrated. The residue was purified by preparative-HPLC to obtain the title compound (8.9 mg, yield of the two steps 15%) in form of white solid. LCMS (ESI) $[M+H]^+$ = 426.0; $^1H$ NMR (400 MHz, $CD_3OD$) $\delta$8.02(s, 1H), 7.50-7.47 (m, 2H), 7.25 (s, 1H), 7.22-7.18 (d, *J* = 8.8 Hz, 2H), 7.13 (s, 2H), 6.47 (s, 1H), 4.68-4.65(d, *J* = 9.2 Hz, 1H), 3.88-3.86 (d, *J* =10 Hz, 1H), 3.49 (s, 3H), 3.18-3.14 (m, 2H), 1.25-1.21 (t, *J* = 6.8 Hz, 3H).

**Comparative embodiment 4019: 7-(4-fluorophenyl)-10-methoxy-2-methyl-2,5-dihydro-3*H*-benzo[*c*]pyrido[3,4-*e*]azepin-3-one**

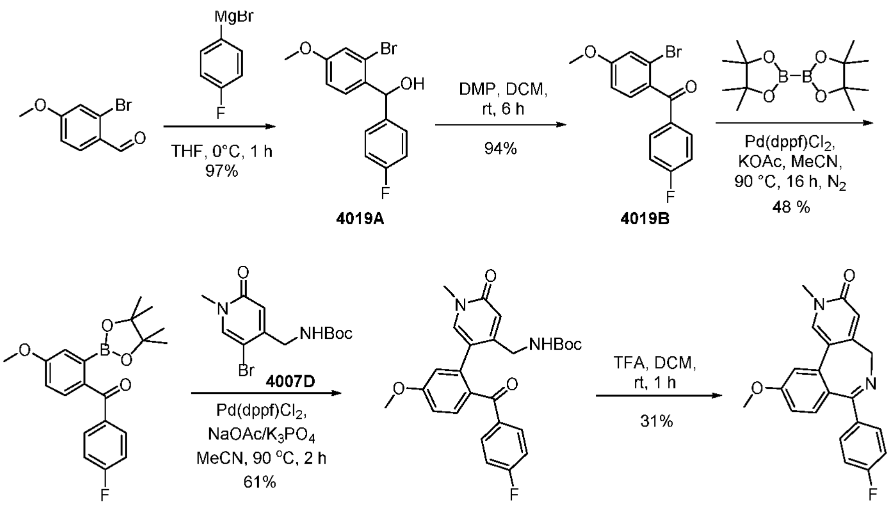


4019A 4019B 4019C 4019D 4019

Step 1: 2-bromo-4-methoxyphenyl)4-fluorophenyl)methanol

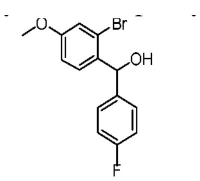

4-fluorophenylmagnesium bromide (1 M in tetrahydrofuran, 10 mL) was slowly added dropwise into to a solution of 2-bromo-4-methoxybenzaldehyde (2.15 g, 10.0 mmol) in tetrahydrofuran (20 mL) under nitrogen protection at 0°C. The reaction mixture was stirred at 0°C for 1 hour, and was quenched by saturated ammonium chloride (20 mL) and extracted by EtOAc (100 mL*3). The combined organic phases were washed by brine, dried over anhydrous sodium sulfate, filtered and concentrated, the residue was purified by flash chromatography (PE/EtOAc = 5/1) to obtain the title compound (3.0 g, 97%) in form of yellow oil, and used directly in the next step.

Step 2: (2-bromo-4-methoxyphenyl)(4-fluorophenyl)methanone

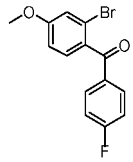

(2-bromo-4-methoxyphenyl)(4-fluorophenyl)methanol (1.5 g, 4.84 mmol) and Dess-Martin periodinane (2.05 g, 4.84 mmol) were mixed in dichloromethane (20 mL) and stirred at room temperature for 6 hours. Then followed by filtration and concentration of the filtrate. The residue was purified by preparative-TLC (PE/EtOAc = 5/1) to obtain the title compound (1.4 g, 94%) in form of yellow oil. LCMS (ESI) [M+H]+ =308.9.

Step 3: (4-fluorophenyl)(4-methoxy-2-(4,4,5,5-tetramethyl-1,3,2-dioxaborolan-2-yl)phenyl)ketone

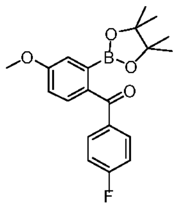

(2-bromo-4-methoxyphenyl)(4-fluorophenyl)methanone (600 mg, 1.94 mmol), bis(pinacolato)diboron (493 mg, 1.94 mmol), [1,1'-bis(diphenylphosphino) ferrocene]palladium(II) dichloride (28 mg, 0.04 mmol) and potassium acetate (318 mg, 3.88 mmol) were mixed in acetonitrile (10 mL). The reaction mixture was heated to 90°C and stirred for 16 hours under nitrogen protection. Then followed by filtration and concentration of the filtrate, the residue was purified by flash chromatography (PE/EtOAc = 15/1) to obtain the title compound (330 mg, 48%) in form of yellow solid. LCMS (ESI) $[M+H]^+$ = 357.1.

Step 4: (5-(2-(4-fluorobenzoyl)-5-methoxyphenyl)-1-methyl-2-oxo-1,2-dihydropyridin-4-yl)aminomethyl *tert*-butyl ester

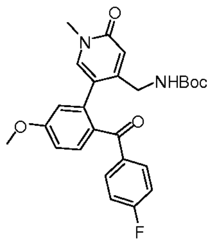

(4-fluorophenyl)(4-methoxy-2-(4,4,5,5-tetramethyl-1,3,2-dioxaborolan-2-yl)phenyl) ketone (150 mg, 0.42 mmol), (5-bromo-1-methyl-2-oxo-1,2-dihydropyridin-4-yl) tert-butylmethylcarbamate (133 mg, 0.42 mmol), [1,1'-bis(diphenylphosphino) ferrocene]palladium(II) dichloride (15 mg, 0.02 mmol), sodium acetate (69 mg, 0.84 mmol) and potassium phosphate (178 mg, 0.84 mmol) were mixed in acetonitrile (5 mL). The reaction mixture was heated to 90°C and stirred for 2 hours under nitrogen protection. Then filtered and spin dried, the residue was purified by preparative-TLC (100% EtOAc) to obtain the title compound (120 mg, 61%) in form of yellow solid. LCMS (ESI) [M+H]+ =467.3.

Step 5: 7-(4-fluorophenyl)-10-methoxy-2-methyl-2,5-dihydro-3*H*-benzo[*c*]pyrido[3,4-*e*]azepin-3-ketone

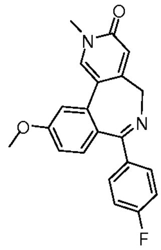

Trifluoroacetic acid (1 mL) and (5-(2-(4-fluorobenzoyl)-5-methoxyphenyl)-1-methyl-2-oxo-1,2-dihydropyridine-4-yl)methylamin omethyl *tert-butyl* ester (100 mg, 0.21 mmol) was dissolved in dichloromethane (4 mL), then stirred at r.t. for 1 hour, concentrated, the residue was purified by preparative-HPLC to obtain the title compound (30 mg, 31%) in form of yellow solid. LCMS (ESI): $R_T$ (min) = 1.406, $[M+H]^+$ = 349.1; $^1H$ NMR (400 MHz, $CD_3OD$) $\delta$ 8.17 (s, 1H), 7.52-7.48 (m, 2H), 7.27-7.15 (m, 4H), 7.06-7.03 (m, 1H), 6.62 (s, 1H), 4.70 (d, $J$ = 10.8 Hz, 1H), 4.11 (d, $J$ = 10.8 Hz, 1H), 3.98 (s, 3H), 3.68 (s, 3H).

**Comparative embodiment 4020: 7-(4-fluorophenyl)-9-methoxy-2-methyl-2,5-dihydro-3*H*-benzo[*c*]pyrido[3,4-*e*]azepin-3-one**

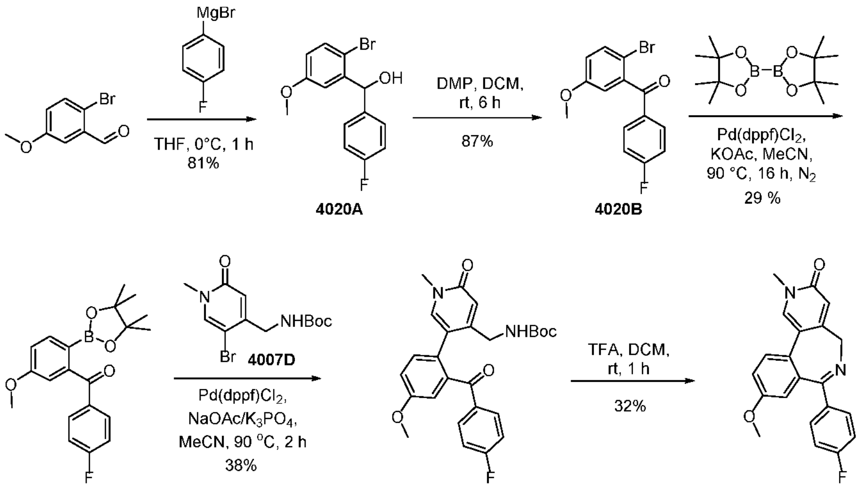


4020A 4020B 4020C 4020D 4020

Step 1: (2-bromo-5-methoxyphenyl)(4-fluorophenyl)methanol

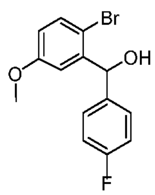

4-fluorophenylmagnesium bromide (1 M in tetrahydrofuran, 10 mL, 10 mmol) was slowly added dropwise into to 2-bromo-5-methoxybenzaldehyde (2.15 g, 10.0 mmol) in THF (20 mL)) at 0°C under nitrogen protection. The reaction was then stirred for 1 hour at 0°C. The reaction was quenched by saturated ammonium chloride (20 mL) and extracted by EtOAc (100 mL*3). The combined organic phases were washed by brine, dried over anhydrous sodium sulfate, then followed by filtration and concentration of the filtrate, the residue was purified by flash chromatography (PE/EtOAc = 15/1) to obtain the title compound (2.5 g, 81%) in form of yellow solid, and was used directly in the next step.

Step 2: (2-bromo-5-methoxyphenyl)(4-fluorophenyl)methanone

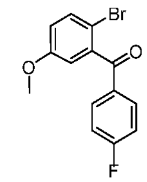

(2-bromo-5-methoxyphenyl)(4-fluorophenyl)methanol (1.5 g, 4.84 mmol) and Dess-Martin periodinane (2.46 g, 5.81 mmol) were mixed in dichloromethane (15 mL) and stirred at r.t. for 6 hours. Then followed by filtration and concentration of the filtrate. The residue was purified by flash chromatography (PE/EtOAc = 15/1) to obtain the title compound (1.3 g, 87%) in form of yellow oil. LCMS (ESI) $[M+H]^+$ = 308.9.

Step 3: (4-fluorophenyl)(5-methoxy-2-(4,4,5,5-tetramethyl-1,3,2-dioxaborolan-2-yl)phenyl)methanone

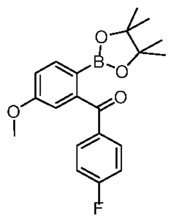

(2-bromo-5-methoxyphenyl)(4-fluorophenyl)methanone (600 mg, 1.94 mmol), bis(pinacolato)diboron (493 mg, 1.94 mmol), [1,1'-bis(diphenylphosphino) ferrocene]palladium(II) dichloride (28 mg, 0.04 mmol) and potassium acetate (318 mg, 3.88 mmol) were mixed in acetonitrile (10 mL). The reaction mixture was heated to 90°C and stirred for 16 hours under nitrogen protection. Then followed by filtration and concentration of the filtrate, the residue was purified by flash chromatography (PE/EtOAc = 20/1) to obtain the title compound (200 mg, 29%) in form of blue solid. LCMS (ESI) $[M+H]^+$ = 357.2.

Step 4: (5-(2-(4-fluorobenzoyl)-4-methoxyphenyl)-1-methyl-2-oxo-1,2-dihydropyridin-4-yl)aminomethyl *tert*-butyl ester

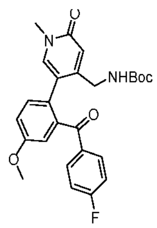

(4-fluorophenyl)(5-methoxy-2-(4,4,5,5-tetramethyl-1,3,2-dioxaborolan-2-yl)phenyl)met hanone (60 mg, 0.17 mmol), (5-bromo-1-methyl-2-oxo-1,2-dihydropyridin-4-yl) *tert*-butylmethylcarbamate (53 mg, 0.17 mmol), [1,1'-bis(diphenylphosphino) ferrocene]palladium(II) dichloride (7 mg, 0.01 mmol), sodium acetate (28 mg, 0.34 mmol) and potassium phosphate (72 mg), 0.34 mmol) were mixed in acetonitrile (3 mL). The reaction mixture was heated to 90°C and stirred for 2 hours under nitrogen protection. Then filtered and spin dried, the residue was purified by preparative-TLC (100% EtOAc) to obtain the title compound (30 mg, 38%) in form of yellow solid. LCMS (ESI) $[M+H]^+$ =467.2.

Step 5: 7-(4-fluorophenyl)-9-methoxy-2-methyl-2,5-dihydro-3H-benzo[c]pyrido[3,4-e]azepin-3-ketone

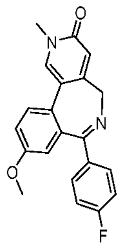

Trifluoroacetic acid (0.5 mL) and (5-(2-(4-fluorobenzoyl)-4-methoxyphenyl)-1-methyl-2-oxo-1,2-dihydropyridine-4-yl)methylamin omethyl *tert*-butyl ester (30 mg, 0.06 mmol) were dissolved in dichloromethane (2 mL), and stirred at r.t. for 1 hour then concentrated, the residue was purified by preparative-HPLC to obtain the title compound (6.6 mg, 32%) in form of white solid. (ESI): $R_T$ (min) = 1.629, $[M+H]^+$ = 349.1; $^1H$ NMR (400 MHz, $CD_3OD$) $\delta$ 8.04 (s, 1H), 7.65 (d, *J* = 8.8 Hz, 1H), 7.52-7.48 (m, 2H), 7.27-7.24 (m, 1H), 7.17-7.12 (m, 2H), 6.78-6.77 (m, 1H), 6.62 (s, 1H), 4.71 (d, *J* = 10.4 Hz, 1H), 4.03 (d, *J* = 10.4 Hz, 1H), 3.78 (s, 3H), 3.66 (s, 3H).

**Embodiment 4021: (*S*)-*N*-ethyl-2-(7-(4-fluorophenyl)-10-methoxy-2-methyl-3-oxo-3,5-dihydro-2*H*-benzo[*c*]pyrido[3,4-*e*]azepin-5-yl)acetamide**

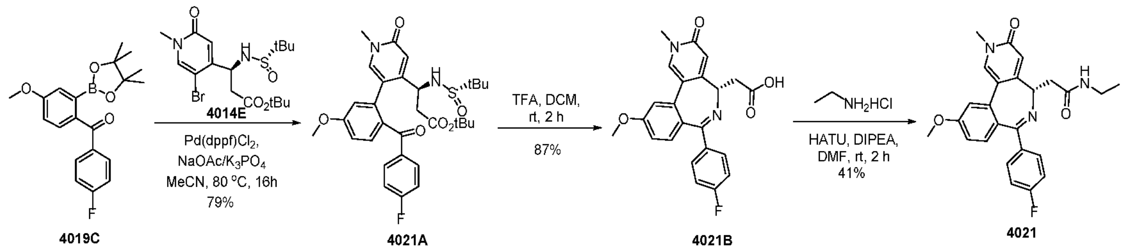


Step 1: (*S*)-3-(((*S*)-*tert*-butylsulfenylamido)-3-(5-(2-(4-fluorobenzoyl)-5-methoxyphenyl)-1-methyl-2-oxo -1,2-dihydropyridin-4-yl) *tert*-butylpropanoate

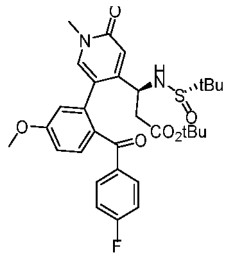

(4-fluorophenyl)(4-methoxy-2-(4,4,5,5-tetramethyl-l,3,2-dioxaborolan-2-yl)phenyl) ketone (100 mg, 0.28 mmol), (*S*)-3-(5-bromo-1-methyl-2-oxo-1,2-dihydropyridin-4-yl)-3-(((*S*)-*tert*-butylsulfinamide) *tert*-butylpropionate (122 mg, 0.28 mmol), [1,1'-bis(diphenylphosphino) ferrocene]palladium(II) dichloride (II), (10 mg, 0.01 mmol), sodium acetate (46 mg, 0.56 mmol) and potassium phosphate (119 mg, 0.56 mmol) were mixed in acetonitrile (3 mL). The reaction mixture was heated to 80°C and stirred for 16 hours under nitrogen protection. EtOAc and water was added to the mixture and then organic phases were washed by brine, dried over anhydrous sodium sulfate, filtered and concentrated, the residue was purified by flash chromatography (dichloromethane/methanol = 10/1) to obtain the title compound (130 mg, 79%) in form of yellow oil. LCMS (ESI) $[M+H]^+$= 585.3.

Step 2: (*S*)-2-(7-(4-fluorophenyl)-10-methoxy-2-methyl-3-oxo-3,5-dihydro-2*H*-benzo[c]pyrido[3,4-*e*]azep in-5-yl)acetic acid

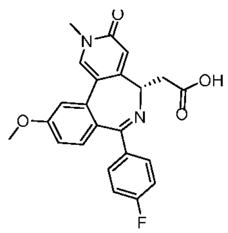

(*S*)-3-(((*S*)-*tert*-butylsulfenylamido)-3-(5-(2-(4-fluorobenzoyl)-5-methoxyphenyl)-1-*tert*-butylmethyl-2-oxo-1,2-dihydropyridin-4-yl)propanoate (100 mg, 0.17 mmol) was dissolved in trifluoroacetic acid (1 mL) and dichloromethane (4 mL), the reaction mixture was stirred at r.t. for 2 hours. Concentrated the mixture to obtain crude product of the title compound (130 mg, 87%) in form of brown oil, which was used directly in the next step without further purification. LCMS (ESI) $[M+H]^+$= 407.1.

Step 3: (*S*)-2-(7-(4-fluorophenyl)-10-methoxy-2-methyl-3-oxo-3,5-dihydro-2*H*-benzo[*c*]pyrido[3,4-*e*]azep in-5-yl)-*N*-ethylacetamide

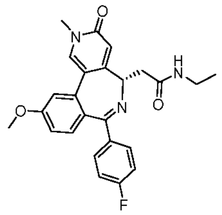

(*S*)-2-(7-(4-fluorophenyl)-10-methoxy-2-methyl-3-oxo-3,5-dihydro-2*H*-benzo[*c*]pyrido[ 3,4-*e*]azepin-5-yl)acetic acid (55 mg, 0.14 mmol), ethylamine hydrochloride (16 mg, 0.20 mmol), 2-(7-azabenzotriazole)-*N,N,N',N'*-tetramethylurea hexafluorophosphate (103 mg, 0.27 mmol) and *N*-ethyl-*N*-isopropylpropan-2-amine (52 mg, 0.41 mmol) were mixed in *N,N*-dimethylformamide (3 mL), and stirred at r.t. for 2 hours. Then followed by filtration and concentration of the filtrate, the residue was purified by preparative-HPLC to obtain the title compound (25 mg, 41%) in form of white solid. (ESI): $R_T$ (min) = 1.585, $[M+H]^+$ = 434.2; $^1H$ NMR (400 MHz, $CD_3OD$) $\delta$ 8.10 (s, 1H), 7.49-7.45 (m, 2H), 7.25-7.23 (m, 2H), 7.14-7.09 (m, 2H), 7.05-7.02 (m, 1H), 6.55 (s, 1H), 4.47-4.45 (m, 1H), 3.97 (s, 3H), 3.66 (s, 3H), 3.28-3.14 (m, 3H), 3.01-2.98 (m, 1H), 1.20-1.16 (m, 3H).

**Embodiment 4022: 10-((ethylsulfonyl)methyl)-7-(4-fluorophenyl)-2-methyl-2,5-dihydro-3*H*-dipyrido[2,3-*c*: 3',4'-*e*]azepin-3-ketone**

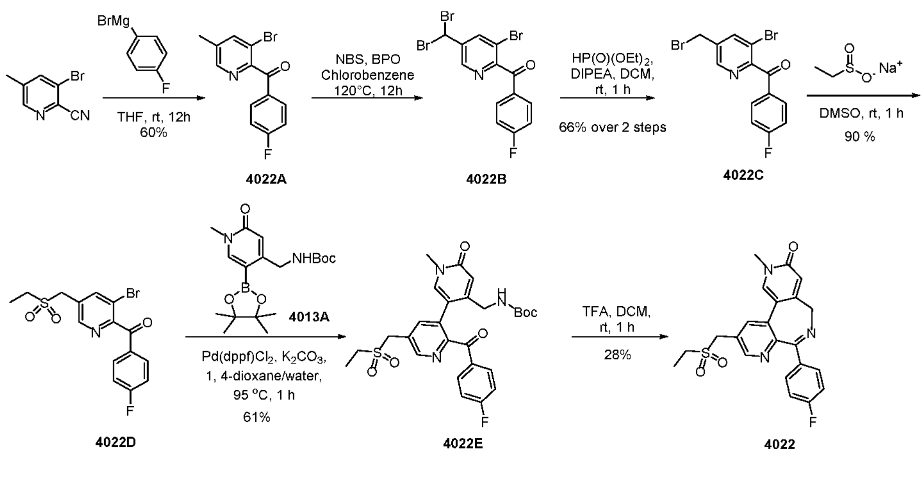

Step 1: (1-methyl-2-oxo-5-(4,4,5,5-tetramethyl-1,3,2-dioxoborolan-2-yl)-1,2-dihydropyridin-4-yl)methylc arbamic *tert*-butyl ester

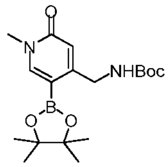

(5-bromo-1-methyl-2-oxo-1,2-dihydropyridin-4-yl)methylcarbamic tert-butyl ester (1 g, 3.2 mmol), bis(pinacolato)diboron (2.4 g, 9.6 mmol), [1,1'-bis(diphenylphosphino) ferrocene]palladium(II) dichloride (240 mg, 0.32 mmol) and potassium acetate (960 mg, 9.6 mmol) were mixed in 1,4-dioxane (20 mL). The reaction mixture was heated to 110°C and stirred for 1 hour under nitrogen protection. Then followed by filtration and concentration of the filtrate, the residue was purified by flash chromatography (100% EtOAc) to obtain the title compound (500 mg, 43%) in form of yellow oil. LCMS (ESI) $[M+H]^+$ = 364.8.

Step 2: (3-bromo-5-methylpyridin-2-yl)(4-fluorophenyl)methanone

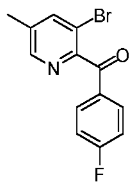

(4-fluorophenyl)magnesium bromide (1 M in tetrahydrofuran, 25 mL) was slowly added dropwise into to 3-bromo-5-methyl-2-cyanopyridine (1 g, 5.0 mmol) in THF(10 mL) at -20°C under nitrogen protection, the reaction solution was slowly warmed to room temperature and stirred overnight. The combined organic phases were washed by brine, dried over anhydrous sodium sulfate, then followed by filtration and concentration of the filtrate, the crude product was separated and purified by flash chromatography (PE/EtOAc = 8/1) to obtain the title compound (900 mg, yield of the two steps 60%) in form of yellow solid. LCMS (ESI) $[M+H]^+$ = 294.0.

Step 3: (3-bromo-5-(dibromomethyl) pyridin-2-yl)(4-fluorophenyl)methanone

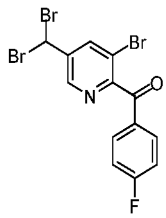

(3-bromo-5-methylpyridin-2-yl)(4-fluorophenyl)methanone (900 mg, 3.0 mmol), *N*-bromosuccinimide (1.6 g, 9.0 mmol) and dibenzoyl peroxide (73 mg, 0.3 mmol) were mixed in chlorobenzene (20 mL). The reaction mixture was stirred at 120°C for 12 hours, spin dried and then obtained the crude product of the title compound (1.1g) in form of yellow solid, which was used directly in the next step without purification. LCMS (ESI) $[M+H]^+$ = 449.8.

Step 4: (3-bromo-5-(bromomethyl) pyridin-2-yl)(4-fluorophenyl)methanone

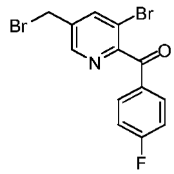

Diethyl phosphite (504 mg, 3.6 mmol) and diisopropylethylamine (929 mg, 7.2 mmol) were added to (3-bromo-5-(dibromomethyl)pyridin-2-yl)(4-fluorophenyl)methanone (1.1 g, 2.4 mmol) in dichloromethane (20 mL). The reaction mixture was stirred at 20°C for 2 hour. The solvent was then evaporated, the residue was purified by flash chromatography (PE/EtOAc = 10/1) to obtain the title compound (700 mg, yield of the two steps 66%) in form of yellow solid. LCMS (ESI) $[M+H]^+$= 372.0.

Step 5: (3-bromo-5-(ethylsulfonylmethyl) pyridin-2-yl)(4-fluorophenyl)methanone

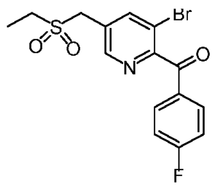

Sodium ethylsulfinate (441 mg, 3.8 mmol) was added to (3-bromo-5-(bromomethyl) pyridin-2-yl)(4-fluorophenyl)methanone (700 mg, 1.8 mmol) in of dimethylsulfoxide (10 mL), the reaction mixture was stirred at r.t. for 1 hour. EtOAc (20 mL) and water (50 mL) was added to layer the mixture, the organic phase was washed by brine (10 mL*3), dried over anhydrous sodium sulfate, filtered and concentrated, the residue was purified by flash chromatography (PE/EtOAc = 2/1) to obtain the title compound (650 mg, 90%) in form of yellow solid. LCMS (ESI) $[M+H]^+$= 386.0.

Step 6: (5-(5-(ethylsulfonylmethyl)-2-(4-fluorobenzoyl)pyridin-3-yl)-1-methyl-2-oxo-1,2-dihydropyridin-4-yl)methyl *tert*-butylcarbamate

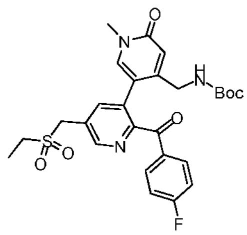

(3-bromo-5-(ethylsulfonylmethyl) pyridin-2-yl)(4-fluorophenyl)methanone (100 mg, 0.26 mmol), (1-methyl-2-oxo-5-(4,4,5,5-tetramethyl-1,3,2-dioxoborolan-2-yl)-1,2-dihydropyridin-4-yl)methyl *tert*-butylcarbamate (124 mg, 0.4 mmol), [1,1'-bis(diphenylphosphino) ferrocene]palladium(II) dichloride (20 mg, 0.03 mmol) and potassium carbonate (108 mg, 0.78 mmol) were mixed in 1,4-dioxane (5 mL) and water (0.5 mL). The reaction mixture was heated to 95°C and stirred for 1 hour under nitrogen protection. Then filtered and spin dried, the residue was purified by flash chromatography (PE/methanol = 30/1) to obtain the title compound (86 mg, 61%) in form of yellow solid. LCMS (ESI) $[M+H]^+$ = 544.1.

Step 7: 10-((ethylsulfonyl)methyl)-7-(4-fluorophenyl)-2-methyl-2,5-dihydro-3*H*-dipyrido[2,3-*c*:3',4'-*e*]aze pin-3-one

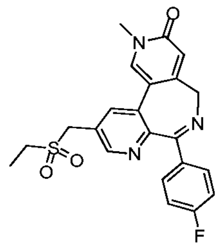

Trifluoroacetic acid (1 mL) and (5-(5-(ethylsulfonylmethyl)-2-(4-fluorobenzoyl) pyridin-3-yl)-1-methyl-2-oxo-1,2-dihydropyridin-4-yl)methyl *tert*-butylcarbamate (86 mg, 0.16 mmol) was dissolved in dichloromethane (4 mL), and stirred at r.t. for 1 hour, concentrated the reaction mixture, the residue was purified by preparative-HPLC to obtain the title compound (19.0 mg, 28%) in form of white solid. LCMS (ESI) $[M+H]^+$ = 426.1; $^1H$ NMR (400 MHz, DMSO-$d_6$) $\delta$ 8.65 (s, 1H), 8.16 (s, 2H), 7.48-7.45 (m, 2H), 7.21-7.16 (m, 2H), 6.57 (s, 1H), 4.81-4.73 (m, 2H), 4.63-4.60 (d, *J* = 13.6 Hz, 2H), 4.02-3.99 (d, *J* = 10.4 Hz, 1H), 3.51 (s, 3H), 3.23-3.18 (m, 2H), 1.30-1.27 (m, 3H).

**Comparative Embodiment 4023: (*S*)-*N*-ethyl-2-(7-(4-fluorophenyl)-9-methoxy-2-methyl-3-oxo-3,5-dihydro-2*H*-benzo[*c*]pyrido [3,4-*e*]azepin-5-yl)acetamide**

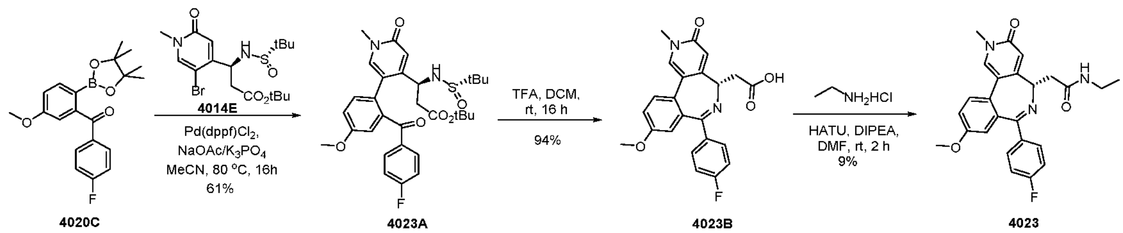


Step 1: (*S*)-3-(((*S*)-*tert*-butylsulfenylamido)-3-(5-(2-(4-fluorobenzoyl)-4-methoxyphenyl)-1-methyl-2-oxo -1,2-dihydropyridin-4-yl) *tert*-butylpropanoate

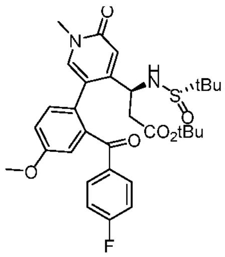

(4-fluorophenyl)(5-methoxy-2-(4,4,5,5-tetramethyl-1,3,2-dioxaborolan-2-yl)phenyl) ketone (100 mg, 0.28 mmol), (*S*)-3-(5-bromo-1-methyl-2-oxo-1,2-dihydropyridin-4-yl)-3-(((*S*)-*tert*-butylsulfinamide) *tert*-butylpropionate (122 mg, 0.28 mmol), [1,1'-bis(diphenylphosphino) ferrocene]palladium(II) dichloride (10 mg, 0.01 mmol), sodium acetate (46 mg, 0.56 mmol) and potassium phosphate (119 mg, 0.56 mmol) were mixed in acetonitrile (3 mL). The reaction mixture was heated to 80°C and stirred for 16 hours under nitrogen protection. Water and EtOAc were added into the raction mixture, organic phase was then separated and dried over anhydrous sodium sulfate, then followed by filtration and concentration of the filtrate, the residue was purified by flash chromatography (dichloromethane/methanol = 10/1) to obtain the title compound (100 mg, 61%) in form of yellow solid. LCMS (ESI) $[M+H]^+$ = 585.3.

Step 2: (*S*)-2-(7-(4-fluorophenyl)-9-methoxy-2-methyl-3-oxo-3,5-dihydro-2H-benzo[*c*]pyrido[3,4-*e*]azepi n-5-yl)acetic acid

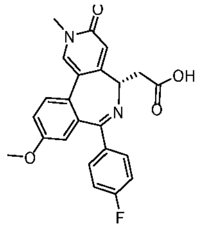

(*S*)-3-(((*S*)-*tert*-butylsulfenylamido)-3-(5-(2-(4-fluorobenzoyl)-4-methoxyphenyl)-1-met hyl-2-oxo-1,2-dihydropyridin-4-yl) *tert*-butylpropanoate (100 mg, 0.17 mmol) dissolved in trifluoroacetic acid (3 mL) and dichloromethane (3 mL), the mixture was concentrated to obtain crude product of the title compound (100 mg, 94%) in form of brown oil, which was used directly in the next step without purification. LCMS (ESI) $[M+H]^+$= 407.1.

Step 3: (*S*)-2-(7-(4-fluorophenyl)-9-methoxy-2-methyl-3-oxo-3,5-dihydro-2H-benzo[*c*]pyrido[3,4-*e*]azepi n-5-yl)-*N*-ethylacetamide

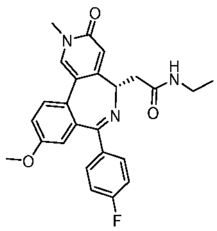

(S)-2-(7-(4-fluorophenyl)-9-methoxy-2-methyl-3-oxo-3,5-dihydro-2*H*-benzo[*c*]pyrido[3, 4-*e*]azepin-5-yl)acetic acid (90 mg, 0.21 mmol), ethylamine hydrochloride (27 mg, 0.33 mmol), 2-(7-azabenzotriazole)-*N,N,N',N'*-tetramethylurea hexafluorophosphate (168 mg, 0.44 mmol) and *N*-ethyl-*N*-isopropylpropan-2-amine (86 mg, 0.67 mmol) were mixed in *N,N*-dimethylformamide (3 mL), and then stirred at r.t. for 2 hours. Then followed by filtration and concentration of the filtrate, the residue was purified by preparative-HPLC to obtain the title compound (9.2 mg, 9%) in form of yellow solid. (ESI): $R_T$ (min) = 1.571, $[M+H]^+$ = 434.1; $^1H$ NMR (400 MHz, $CD_3OD$) $\delta$ 8.01 (s, 1H), 7.67-7.65 (m, 1H), 7.53-7.49 (m, 2H), 7.27-7.25 (m, 1H), 7.15-7.11 (m, 2H), 6.81-6.80 (m, 1H), 6.56 (s, 1H), 4.46-4.44 (m, 1H), 3.79 (s, 3H), 3.65 (s, 3H), 3.25-3.16 (m, 3H), 3.00-2.95 (m, 1H), 1.20-1.17 (m, 3H).

**Embodiment 4024: *N*-(7-(4-chlorophenyl)-2-methyl-3-oxo-3,5-dihydro-2*H*-benzo[*c*]pyrido[3,4-*e*]azepin-3-yl)etha nesulfonamide**

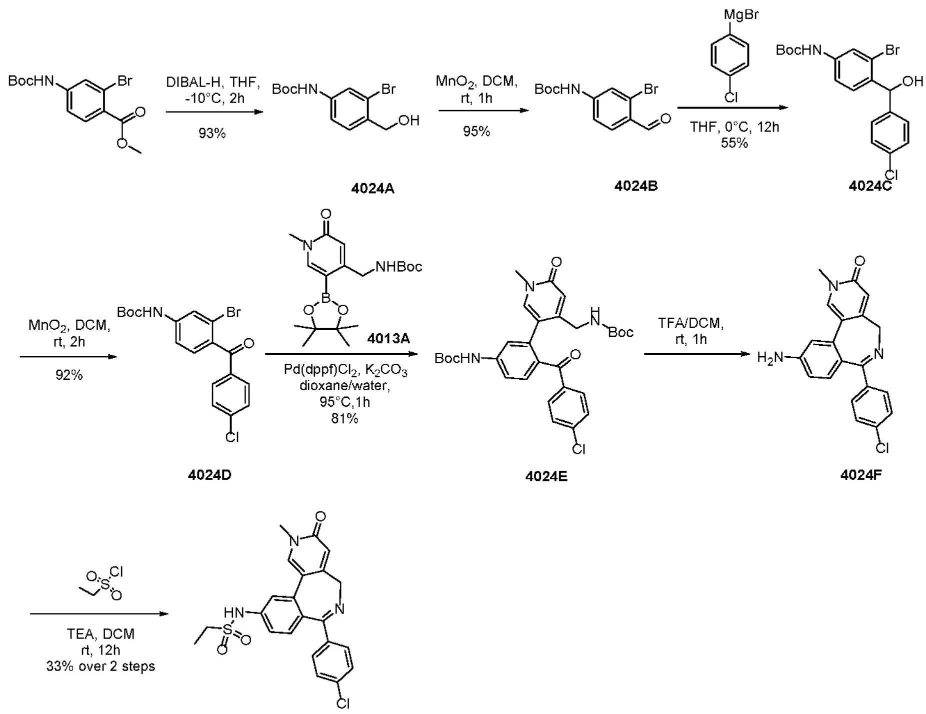


4024A 4024B 4024C 4024D 4024E 4024F

4024

Step 1: (3-bromo-4-(hydroxymethyl)phenyl) *tert*-butylcarbamate

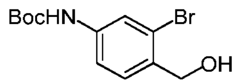

Diisobutyl aluminium hydrid (1 M in tetrahydrofuran, 12.0 mL, 12.0 mmol) was slowly added dropwise into to methyl 2-bromo-4-(tert-butoxycarbonylamino) benzoate (1 g, 3.0 mmol) in THF (40 mL) at -10°C. The reaction mixture was stirred at r.t. for 2 hours, then sodium sulfate decahydrate (5 g) was used to quench the reaction, then continue stirring the mixture for half hour. Then followed by filtration and concentration of the filtrate, the residue was purified by flash chromatography (PE/EtOAc = 5/1) to obtain the title compound (850 mg, 93%) in form of yellow solid. LCMS (ESI) $[M+H]^+$ = 302.0.

Step 2: (3-bromo-4-carbaldehydephenyl) *tert*-butylcarbamate

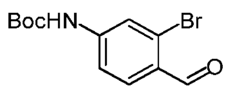

(3-bromo-4-(hydroxymethyl)phenyl) carbamate (850 mg, 2.8 mmol) and manganese dioxide (2.4 g, 28.0 mmol) were combined in dichloromethane (10 mL). It was stirred at room temperature for 1 hour. The reaction mixture was filtered through a pad of celite and washed by dicholoromethane (10 mL*2). Combine the filtrate and washing solution, concentrate. The residue was purified by flash chromatography (PE/EtOAc = 10/1) to obtain the title compound (800 mg, 95%) in form of yellow solid. LCMS (ESI) $[M+H]^+$= 300.0.

Step 3: 3-bromo-4-4-chlorophenyl)hydroxy)methyl) *tert*-butylphenylcarbamate

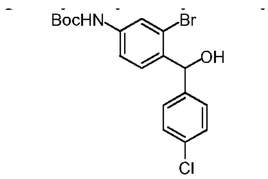

(4-chlorophenyl)magnesium bromide (1 M in tetrahydrofuran, 13.3 mL, 13.3 mmol). was slowly added dropwise into to a solution of (3-bromo-4-carbaldehydephenyl) *tert*-butylcarbamate (800 mg, 2.6 mmol) in tetrahydrofuran (40 mL). The reaction was quenched by saturated ammonium chloride (10 mL) and extracted by EtOAc (20 mL*3). The combined organic phases were washed by brine (20 mL), dried over anhydrous sodium sulfate, filtered and concentrated. The residue was purified by flash chromatography (PE/EtOAc = 8/1) to obtain the title compound (600 mg, 55%) in form of yellow solid. LCMS (ESI) $[M+H]^+$ = 412.0.

Step 4; 3-bromo-4-(4-chlorobenzoyl) *tert*-butylphenylcarbamate

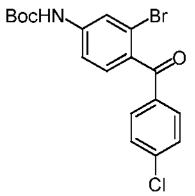

3-bromo-4-((4-chlorophenyl)(hydroxy)methyl)phenylcarbamate (600 mg, 1.4 mmol) and manganese dioxide (1.2 g, 14.0 mmol) were mixed in dichloromethane (10 mL), then stirred at r.t. for 2 hour. The reaction mixture was filtered through a pad of celite and washed by dicholoromethane (10 mL*2). Combine the filtrate and washing solution, concentrate. The residue was purified by flash chromatography (PE/EtOAc = 10/1) to obtain the title compound (550 mg, 92%) in form of yellow solid. LCMS (ESI) $[M+H]^+$= 410.0.

Step 5: (3-(4-((*tert*-butoxycarbonyl)amino)methyl)-1-methyl-6-oxo-1,6-dihydropyridin-3-yl)-4-(4-chlorob enzoyl)phenyl) *tert*-butylcarbamate

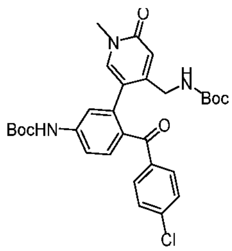

3-bromo-4-(4-chlorobenzoyl) *tert*-butylphenylcarbamate (150 mg, 0.36 mmol), (1-methyl-2-oxo-5-(4,4,5,5-tetramethyl-1,3,2-dioxoborolan-2-yl)-1,2-dihydropyridin-4-yl)methylc arbamate (173 mg, 0.54 mmol), [1,1'-bis(diphenylphosphino) ferrocene]palladium(II) dichloride (30 mg, 0.04 mmol) and potassium carbonate (149 mg, 1.1 mmol) were mixed in 1,4-dioxane (3 mL) and water (0.3 mL). The reaction mixture was heated to 90°C and stirred for 1 hour under nitrogen protection. Then filtered and spin dried, the residue was purified by preparative-TLC (PE/EtOAc = 20/1) to obtain the title compound (168 mg, 81%) in form of yellow oil. LCMS (ESI) [M+H]+ =568.2.

Step 6: 10-amino-7-(4-chlorophenyl)-2-methyl-2,5-dihydro-3*H*-benzo[*c*]pyrido[3,4-*e*]azepin-3-ketone

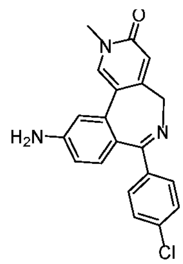

Trifluoroacetic acid (1 mL) and (3-(4-(((*tert*-butoxycarbonyl)amino)methyl)-1-methyl-6-oxo-1,6-dihydropyridin-3-yl)-4-(4-chloro benzoyl)phenyl) *tert*-butylcarbamate (168 mg, 0.29 mmol) were dissolved in dichloromethane (4 mL), then stirred at r.t. for 1 hour. The mixture was then concentrated, residue (170mg) was used directly in the next step without further purification. LCMS (ESI) $[M+H]^+$ = 350.1.

Step 7: *N*-(7-(4-chlorophenyl)-2-methyl-3-oxo-3,5-dihydro-2*H*-benzo[*c*]pyrido[3,4-*e*]azepin-10-yl)ethane sulfonamide

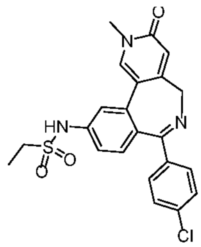

Ethylsulfonyl chloride was added dropwise into 10-amino-7-(4-chlorophenyl)-2-methyl-2,5-dihydro-3*H*-benzo[*c*]pyrido[3,4-*e*]azepin-3-ketone (170 mg, 0.48 mmol) and triethylamine (484 mg, 4.8 mmol) in dichloromethane (5 mL), the reaction mixture was stirred overnight. Water and brine was used to wash the reaction mixture, followed by desiccation by anhydrous sodium sulfate, filtration and concentration. The residue was purified by preparative-HPLC to obtain the title compound (43.1 mg, yield of the two steps 33%) in form of yellow solid. LCMS (ESI) $[M+H]^+$ = 442.1; $^1H$ NMR (400 MHz, $CD_3OD$) $\delta$ 8.06 (s, 1H), 7.45-7.40 (m, 5H), 7.27-7.25 (m, 1H), 7.21-7.19 (d, *J* = 8.4 Hz, 1H), 6.62 (s, 1H), 4.72-4.69 (d, *J* = 10.4 Hz, 1H), 4.08-4.06 (d, *J* = 10.4 Hz, 1H), 3.67 (s, 3H), 3.28-3.23 (m, 2H), 1.39-1.36 (m, 3H).

**Embodiment 4025: (*S*)-7-(4-chlorophenyl)-2,5-dimethyl-2,5-dihydro-3*H*-benzo[*c*]pyrido[3,4-*e*]azepin-3-ketone**

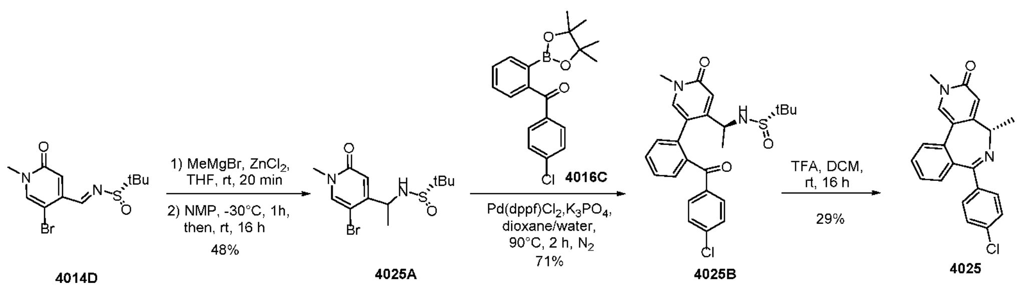


Step 1: (S)-*N*-((S)-1-(5-bromo-1-methyl-2-oxo-1,2-dihydropyridin-4-yl)ethyl)-2-methylpropane-2-sulfina mide

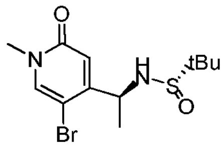

To a solution of zinc chloride (852 mg, 6.25 mmol) in anhydrous tetrahydrofuran (5 mL) was added dropwise methyl magnesium bromide (3 N in diethyl ether, 2.08 mL, 6.24 mmol). The reaction mixture was stirred at r.t. for 20 mins under nitrogen protection. The solution was then slowly added dropwise to (S)-*N*-((5-bromo-1-methyl-2-oxo-1,2-dihydropyridine-4-yl)methylene)-2-methylpropane-2-sulfen amide (400 mg, 1.25 mmol) in 1-methyl-2-pyrrolidone (10 mL) solution, the reaction mixture was stirred at -30°C for 1 hour, then slowly warmed to r.t. and stirred overnight. The reaction was quenched by adding water (50 mL) and extracted with EtOAc (20 mL*3). The organic phase was then separated and dried over anhydrous sodium sulfate, the residue was purified by flash chromatography (EtOAc /methanol = 100/3) to obtain the title compound (200 mg, 48%) in form of brown oil. LCMS (ESI) $[M+H]^+$ = 153.1.

Step 2: (*S*)-*N*-((*S*)-1-(5-(2-(4-chlorobenzoyl)phenyl)-1-methyl-2-oxo-1,2-dihydropyridine-4-yl)ethyl)-2-m ethylpropane-2-sulfenamide

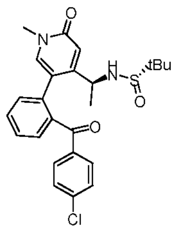

(S)-*N*-((*S*)-1-(5-bromo-1-methyl-2-oxo-1,2-dihydropyridin-4-yl)ethyl)-2-methylpropane -2-sulfenamide (70 mg, 0.21 mmol), (4-chlorophenyl)(2-(4,4,5,5-tetramethyl-1,3,2-dioxaborolan-2-yl)phenyl)methanone (110 mg, 0.32 mmol), [1,1'-bis(diphenylphosphino)ferrocene]palladium(II) dichloride (31 mg, 0.042 mmol) and potassium phosphoate (134 mg, 0.63 mmol) were mixed in 1,4-dioxane (10 mL) and water (1 mL). The reaction mixture was stirred at 90°C for 2 hours under nitrogen protection. Water and EtOAc were added into the reation mixture, organic phase was then separated and dried over anhydrous sodium sulfate, filtered and concentrated, the residue was purified by flash chromatography (PE/EtOAc = 100/3) to obtain the title compound (70 mg, 71%) in form of white solid. MS (ESI): *m*/*z* = 472.1 $[M+H]^+$.

Step 3: (*S*)-7-(4-chlorophenyl)-2,5-dimethyl-2,5-dihydro-3*H*-benzo[*c*]pyrido[3,4-*e*]azepin-3-ketone

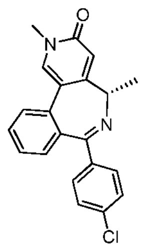

(S)-*N*-((*S*)-1-(5-(2-(4-chlorobenzoyl)phenyl)-1-methyl-2-oxo-1,2-dihydropyridine-4-yl)e thyl)-2-methylpropane-2-sulfenamide (70 mg, 0.15 mmol) was dissolved in dichloromethane (6 mL) and trifluoroacetic acid (2 mL), the reaction mixture was then stirred at r.t. overnight, followed by concentration. The residue was purified by preparative-HPLC to obtain the title compound (15 mg, 29%) in form of white solid. LCMS (ESI) $[M+H]^+$ =349.2; $^1H$ NMR (400 MHz, $CD_3OD$) $\delta$ 8.07 (s, 1H), 7.72 (d, *J* = 7.6 Hz, 1H), 7.66 (d, *J* = 8.0 Hz, 1H), 7.51-7.38 (m, 5H), 7.30 (d, *J* = 7.6 Hz, 1H), 6.63 (s, 1H), 4.10 (q, *J* = 6.4, 13.2 Hz, 1H), 3.67 (s, 3H), 1.77 (t, *J* = 6.8 Hz, 3H).

**Embodiment 4026: (*S*)-10-((ethylsulfone)methyl)-7-(4-fluorophenyl)-2,5-dimethyl-2,5-dihydro-3*H*-benzo[*c*]pyrido[3,4-*e*]azepin-3-ketone**

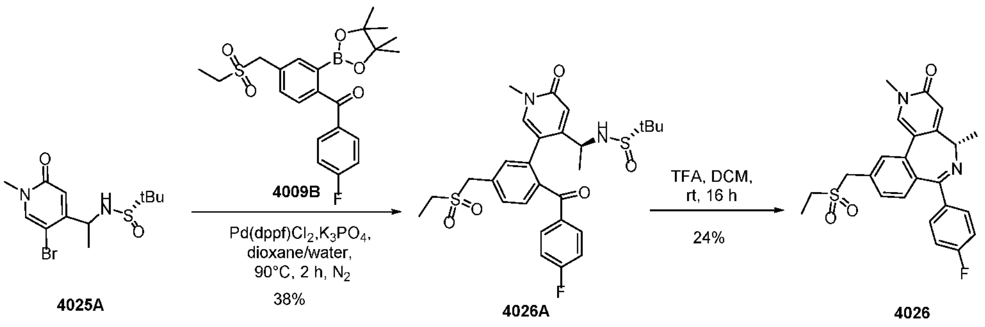


Step 1: (*S*)-*N*-((*S*)-1-(5-(5-((ethylsulfonyl)methyl)-2-(4-fluorobenzoyl)phenyl)-1-methyl-2-oxo-1,2-dihydr opyridin-4-yl)ethyl)-2-methylpropane-2-sulfenamide

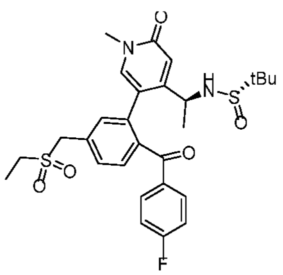

(*S*)-*N*-(1-(5-bromo-1-methyl-2-oxo-1,2-dihydropyridin-4-yl)ethyl)-2-methylpropane-2-s ulfenamide (350 mg, 1.04 mmol), (4-(ethylsulfonylmethyl)-2-(4,4,5,5-tetramethyl-1,3,2-dioxoborolan-2-yl)phenyl)(4-fluorophenyl) methanone (674 mg, 1.56 mmol), [1,1'-bis(diphenylphosphino)ferrocene]palladium(II) dichloride (II) (73 mg, 0.10 mmol) and potassium phosphate (662 mg, 3.12 mmol) were mixed in 1,4-dioxane (10 mL) and water (1 mL). The reaction mixture was heated to 90°C and stirred for 2 hours under nitrogen protection. Water and EtOAc were added into the reaction mixture, organic phase was then separated and dried over anhydrous sodium sulfate, filtered and concentrated, the residue was purified by preparative-HPLC to obtain the title compound (S)-*N*-((S)-1-(5-(5-((ethylsulfonyl)methyl)-2-(4-fluorobenzoyl)phenyl)-1-methyl-2-oxo-1,2-dihydr opyridin-4-yl)ethyl)-2-methylpropane-2-sulfinamide (220 mg, 38 %) in form of white solid; also obtained
(S)-*N*-((R)-1-(5-(5-((ethylsulfonyl)methyl)-2-(4-fluorobenzoyl)phenyl)-1-methyl)-2-oxo-1,2-dihy dropyridin-4-yl)ethyl)-2-methylpropane-2-sulfinamide (45 mg, 8 %) in form of white solid. MS (ESI): *m*/*z* = 561.1 $[M+H]^+$.

Step 2: (S)-10-((ethylsulfonyl)methyl)-7-(4-fluorophenyl)-2,5-dimethyl-2,5-dihydro-3H-benzo[*c*]pyrido[3 ,4-*e*]azepin-3-ketone

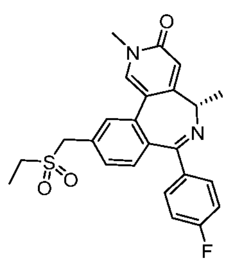

(S)-*N*-((*S*)-1-(5-(5-((ethylsulfonyl)methyl)-2-(4-fluorobenzoyl)phenyl)-1-methyl-2-oxo-1,2-dihydropyridin-4-yl)ethyl)-2-methylpropane-2-sulfenamide (80 mg, 0.14 mmol) was dissolved in dichloromethane (6 mL) and trifluoroacetic acid (2 mL) and stirred at r.t. overnight. The reaction mixture was then concentrated, the residue was purified by preparative-HPLC to obtain the title compound (15 mg, 24%) in form of white solid. LCMS (ESI) $[M+H]^+$ = 439.2; $^1H$ NMR (400 MHz, DMSO-$d_6$) $\delta$ 8.03 (s, 1H), 7.71 (s, 1H), 7.53-7.43 (m, 3H), 7.34 (d, *J* = 8.0 Hz, 1H), 7.23 (d, *J* = 8.8 Hz, 2H), 6.42 (s, 1H), 4.61 (q, *J* = 13.6, 38.0 Hz, 2H), 3.97 (q, *J* = 5.6, 12.4 Hz, 1H), 3.51 (s, 3H), 3.14 (q, *J* = 7.6, 14.8 Hz, 2H), 1.66 (d, *J* = 6.8 Hz, 3H), 1.26 (t, *J* = 8.4 Hz, 3H).

**Embodiment 4033: (*R*)-10-((ethylsulfone)methyl)-7-(4-fluorophenyl)-2,5-dimethyl-2,5-dihydro-3H-benzo[*c*]pyrid o[3,4-*e*]azepin-3-ketone**

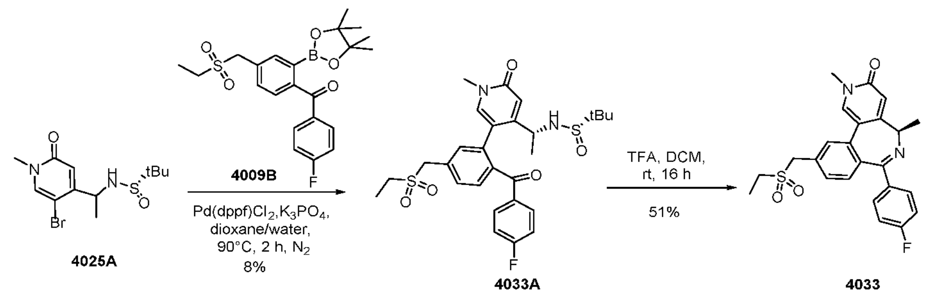


Step 1: (S)-*N*-((R)-1-(5-(5-((ethylsulfonyl)methyl)-2-(4-fluorobenzoyl)phenyl)-1-methyl)-2-oxo-1,2-dihy dropyridin-4-yl)ethyl)-2-methylpropane-2-sulfinamide

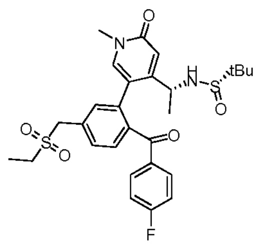

(S)-*N*-(1-(5-bromo-1-methyl-2-oxo-1,2-dihydropyridin-4-yl)ethyl)-2-methylpropane-2-s ulfonamide (350 mg, 1.04 mmol), (4-(ethylsulfonylmethyl)-2-(4,4,5,5-tetramethyl-1,3,2-dioxoborolan-2-yl)phenyl)(4-fluorophenyl) methanone (674 mg, 1.56 mmol), [1,1'-bis(diphenylphosphino)ferrocene]palladium(II) dichloride (II) (73 mg, 0.10 mmol) and potassium phosphate (662 mg, 3.12 mmol) were mixed in 1,4-dioxane (10 mL) and water (1 mL). The reaction mixture was heated to 90°C and stirred for 2 hours under nitrogen protection. Water and EtOAc were added into the reaction mixture, organic phase was then separated and dried over anhydrous sodium sulfate, filtered and concentrated, the residue was purified by preparative-HPLC to obtain the title compound (S)-*N*-((R)-1-(5-(5-((ethylsulfonyl)methyl)-2-(4-fluorobenzoyl)phenyl)-1-methyl)-2-oxo-1,2-dihy dropyridin-4-yl)ethyl)-2-methylpropane-2-sulfinamide (45 mg, 8%) in form of white solid, also obtained was (S)-N-((S)-1-(5-(5-((ethylsulfonyl)methyl)-2-(4-fluorobenzoyl)phenyl)-1-methyl)-2-oxo-1,2-dihyd ropyridin-4-yl)ethyl)-2-methylpropane-2-sulfinamide (220 mg, 38%) in form of white solid. MS (ESI): *m*/*z* = 561.1.

Step 2: (R)-10-((ethylsulfone)methyl)-7-(4-fluorophenyl)-2,5-dimethyl-2,5-dihydro-3*H*-benzo[*c*]pyrido[3, 4-*e*]azepin-3 -ketone

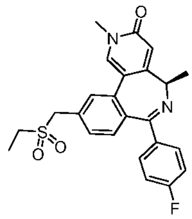

(S)-*N*-((R)-1-(5-(5-((ethylsulfonyl)methyl)-2-(4-fluorobenzoyl)phenyl)-1-methyl-2-oxo-1,2-dihydropyridin-4-yl)ethyl)-2-methylpropane-2-sulfenamide (45 mg, 0.08 mmol) was dissolved in dichloromethane (3 mL) and TFA (3 mL), the reaction mixture was stirred at r.t. overnight. After concentration, the residue was purified by preparative-HPLC to obtain the title compound (18 mg, 51%) in form of white solid. LCMS (ESI) $[M+H]^+ = 439.0$; $^1H$ NMR (400 MHz, DMSO-$d_6$) $\delta$ 8.04 (s, 1H), 7.71 (s, 1H), 7.50-7.46 (m, 3H), 7.34 (d, *J* = 8.0 Hz, 1H), 7.23 (d, *J* = 8.8 Hz, 2H), 6.42 (s, 1H), 4.62 (q, *J* = 13.6, 38.0 Hz, 2H), 3.97 (q, *J* = 6.0, 12.4 Hz, 1H), 3.51 (s, 3H), 3.14 (q, *J* = 7.6, 14.8 Hz, 2H), 1.66 (d, *J* =6.4 Hz, 3H), 1.26 (t, *J* = 8.0 Hz, 3H).

**Embodiment 4027: 7-(4-chlorophenyl)-10-((ethylsulfonyl)methyl)-2-methyl-2,5-dihydro-3H-dipyrido[3,4-*c*:3',4'-*e*]azepin-3-ketone**

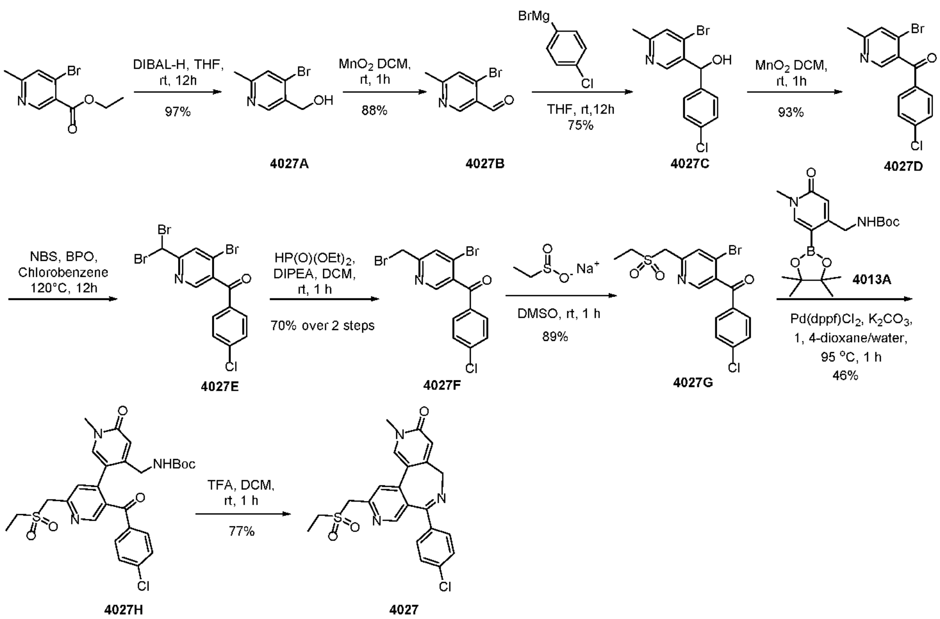


Step 1: (4-bromo-6-methylpyridin-3-yl)methanol

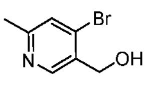

Diisobutylaluminum hydride (1 M in THF, 16.0 mL, 16.0 mmol) was added dropwise into ethyl 4-bromo-6-methyl-3-pyridine carboxylate (1 g, 4.0 mmol) in tetrahydrofuran (30 mL) at -10 °C. The reaction mixture was stirred at r.t. for 12 hours under nitrogen protection, sodium sulfate decahydrate was used to quench the reaction and further stirred at r.t. for 0.5 hour. After filtration and concentration of the filtrate, the residue was purified by flash chromatography (PE/EtOAc = 5/1) to obtain the title compound (800 mg, 97%) in form of yellow solid. LCMS (ESI) $[M+H]^+$= 202.0.

Step 2: 4-bromo-6-methyl-3-pyridinaldehyde

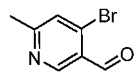

(4-bromo-methylpyridin-3-yl)methanol (800 mg, 3.9 mmol) and manganese dioxide (3.4, 39.0 mmol) were mixed in dichloromethane (20 mL), and then stirred at r.t. for 1 hour. The reaction mixture was filtered through a pad of celite, and washed with dicholoromethane (10 mL*2). The filtrate and washing solution were combined and concentrated. The residue was purified by flash chromatography (PE/EtOAc = 10/1) to obtain the title compound (600 mg, 88%) in form of yellow solid. LCMS (ESI) $[M+H]^+$ = 199.9.

Step 3: (4-bromo-6-methylpyridin-3-yl)(4-chlorophenyl)methanol

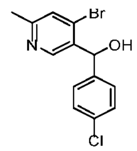

Under nitrogen protection, (4-chlorophenyl)magnesium bromide (1 M in tetrahydrofuran, 15 mL, 15 mmol) was added dropwise into 4-bromo-6-methyl-3-pyridinaldehyde (600 mg, 3.0 mmol) in THF solution (40 mL) at -20°C, the reaction mixture was stirred at r.t. overnight. The reaction was quenched by saturated ammonium chloride (10 mL) and extracted with EtOAc (20 mL*3). The combined organic phases were washed by brine, dried over anhydrous sodium sulfate, filtered and concentrated. The residue was purified by flash chromatography (PE/EtOAc = 8/1) to obtain the title compound (700 mg, 75%) in form of yellow solid. LCMS (ESI) $[M+H]^+$ = 312.0.

Step 4: (4-bromo-6-methylpyridin-3-yl)(4-chlorophenyl)methanone

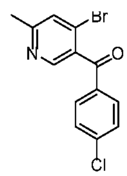

(4-bromo-6-methylpyridin-3-yl)(4-chlorophenyl)methanol (700 mg, 2.2 mmol) and manganese dioxide (1.9 g, 22.0 mmol) were mixed in dichloromethane (20 mL), the reaction mixture was stirred at r.t. for 1 hour. The reaction mixture was filtered through a pad of celite, and washed with dicholoromethane (10 mL*2). The filtrate and washing solution were combined and concentrated. The residue was purified by flash chromatography (PE/EtOAc = 10/1) to obtain the title compound (650 mg, 93%) in form of yellow solid. LCMS (ESI) $[M+H]^+$ = 310.0.

Step 5: (4-bromo-6-(dibromomethyl)pyridin-3-yl)(4-chlorophenyl)methanone

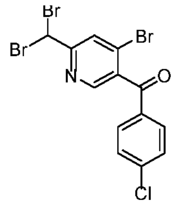

(4-Bromo-6-methylpyridin-3-yl)(4-chlorophenyl)methanone (610 mg, 2.1 mmol), N-bromosuccinimide (1.1 g, 6.3 mmol) and dibenzoyl peroxide (50 mg, 0.21 mmol) were mixed in chlorobenzene (20 mL), then heated to 120°C and stirred for 12 hours, spin dried to obtain the crude product of the title compound (900 mg) in form of yellow solid, which was used directly in the next step without purification. LCMS (ESI) $[M+H]^+$ = 466.0.

Step 6: (4-bromo-6-(bromomethyl)pyridin-3-yl)(4-chlorophenyl)methanone

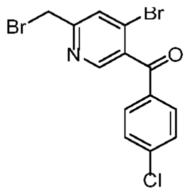

Diethyl phosphite (400 mg, 2.9 mmol) and diisopropylethylamine (736 mg, 5.7 mmol) were added to (4-bromo-6-(dibromomethyl)pyridin-3-yl)(4-chlorophenyl)methanone (900 mg, 1.9 mmol) in dichloromethane (20 mL). The reaction mixture was stirred at 20°C for 1 hour. The solvent was then evaporated and the residue was purified by flash chromatography (PE/EtOAc = 15/1) to obtain the title compound (600 mg, yield of the two steps 70%) in form of yellow solid. LCMS (ESI) $[M+H]^+$= 388.0.

Step 7: (4-bromo-6-(ethylsulfonylmethyl) pyridin-3-yl)(4-chlorophenyl)methanone

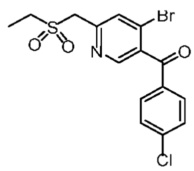

Sodium ethylsulfinate (350 mg, 3.0 mmol) was added to (3-bromo-5-(bromomethyl) pyridin-2-yl)(4-fluorophenyl)methanone (600 mg, 1.5 mmol) in dimethylsulfoxide (10 mL), the reaction mixture was stirred at r.t. for 1 hour. EtOAc (20 mL) and water (20 mL) was added to layer the mixture, the organic phase was washed by brine (50 mL*3), dried over anhydrous sodium sulfate, the residue was purified by flash chromatography (PE/EtOAc = 2/1) to obtain the title compound (550 mg, 89%) in form of brown solid. LCMS (ESI) $[M+H]^+$ = 402.0.

Step 8: (5-(5-(4-chlorobenzoyl)-2-(ethylsulfonylmethyl)pyridin-4-yl)-1-methyl-2-oxo-1,2-dihydropyridin-4-yl) *tert*-methylmethylcarbamate

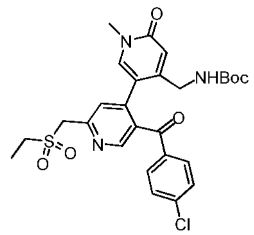

((4-bromo-6-(ethylsulfonylmethyl)pyridin-3-yl)(4-chlorophenyl)methanone (170 mg, 0.39 mmol), (1-methyl-2-oxo-5-(4,4,5,5-tetramethyl-1,3,2-dioxoborolan-2-yl)-1,2-dihydropyridin-4-yl) *tert*-butylmethylcarbamate (285 mg, 0.76 mmol), [1,1'-bis(diphenylphosphino) ferrocene]palladium(II) dichloride (35 mg, 0.04 mmol) and potassium carbonate (175 mg, 0.7 mmol) were mixed in 1,4-dioxane (6 mL) and water (0.6 mL). The reaction mixture was heated to 95 °C and stirred for 1 hour under nitrogen protection. Then filtered and spin dried, the residue was purified by flash chromatography (EtOAc/methanol = 30/1) to obtain the title compound (100 mg, 46%) in form of yellow solid. LCMS (ESI) $[M+H]^+$ = 560.1.

Step 9: 7-(4-chlorophenyl)-10-((ethylsulfonyl)methyl)-2-methyl-2,5-dihydro-3H-dipyrido[3,4-c:3 4'-e] azepin-3 -ketone

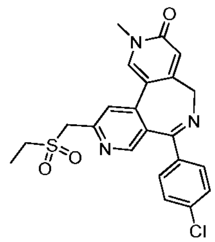

Trifluoroacetic acid (1 mL) and (5-(5-(ethylsulfonylmethyl)-2-(4-fluorobenzoyl)pyridin-3-yl)-1-methyl-2-oxo-1,2-dihydropyridin-4-yl) *tert*-butylmethylcarbamate (110 mg, 0.2 mmol) were dissolved in dichloromethane (4 mL), then stirred at r.t. for 1 hour, the reaction mixture was then concentrated, the residue was purified by preparative-HPLC to obtain the title compound (67.1 mg, 77%) in form of white solid. LCMS (ESI): $[M+H]^+$ = 442.0; $^1H$ NMR (400 MHz, DMSO-$d_6$) $\delta$ 8.44(s, 1H), 8.27 (s, 1H), 7.82 (s, 1H), 7.50-7.44 (m, 4H), 6.56 (s, 1H), 4.84-4.81 (d, $J$ = 10.8 Hz, 1H), 4.77-4.63 (m, 2H), 3.98-3.96 (d, $J$ = 10.4 Hz, 1H), 3.51 (s, 3H), 3.25-3.21 (m, 2H), 1.31-1.23 (m, 3H).

**Embodiment 4028: 7-(2-chloro-4-fluorophenyl)-10-((ethylsulfone)methyl)-2-methyl-2,5-dihydro-3H-benzo[c]pyrido[34-e]azepin-3-ketone**

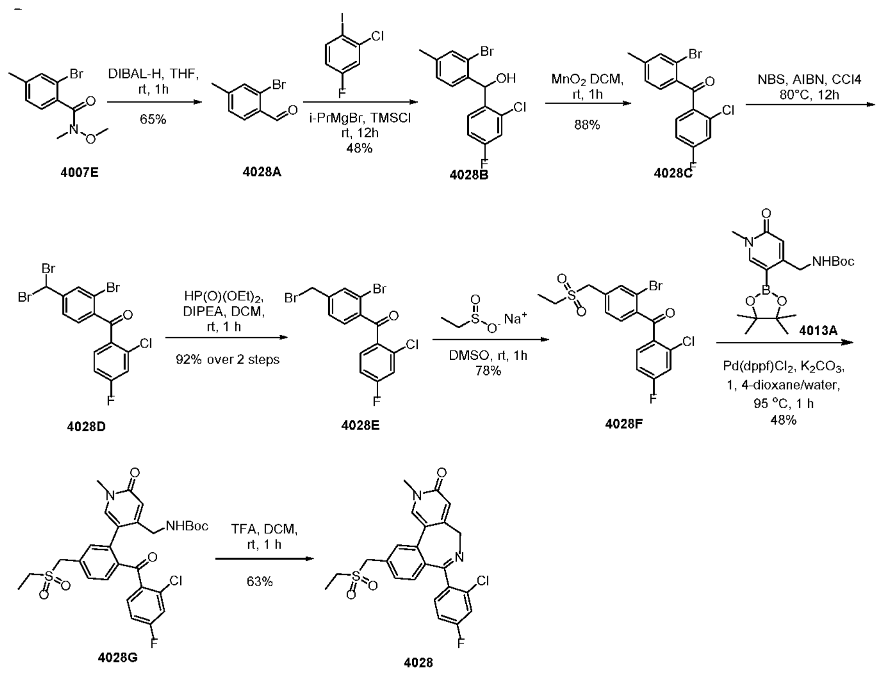


Step 1: 2-bromo-4-methylbenzaldehyde

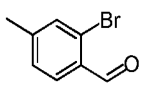

Diisobutyl aluminium hydrid (1 M in THF, 4.7 mL, 4.7 mmol) was slowly added dropwise into 2-bromo-N-methoxy-N,4-dimethylbenzamide (1 g, 3.9 mmol) in tetrahydrofuran (40 mL) solution at -1°C, the reaction mixture was stirred at -10°C for 1 hour under nitrogen protection, then sodium sulfate decahydrate was added to quench to reaction, then continued stirring for half hour. Then followed by filtration and concentration of the filtrate, the residue was purified by flash chromatography (PE/EtOAc = 10/1) to obtain the title compound (500 mg, 65%) in form of yellow solid. LCMS (ESI) $[M+H]^+$ = 199.0.

Step 2: (2-bromo-4-methylphenyl)(2-chloro-4-fluorophenyl)methanol

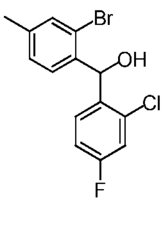

Under nitrogen protection at -20°C, isopropylmagnesium bromide (2 M in tetrahydrofuran, 8 mL, 16 mmol) was slowly added dropwise into to 2-chloro-4-fluoro-1-iodobenzene (4.10 g, 16 mmol) in anhydrous tetrahydrofuran (8 mL). The reaction mixture was stirred at -20°C for 3 hours to give (2-chloro 4-fluorophenyl)magnesium bromide (1M in tetrahydrofuran, 16 mL). Under nitrogen protection at -20°C, the solution was slowly added dropwise into to a solution of 2-bromo-4-methylbenzaldehyde (500 mg, 2.5 mmol) and trimethylchlorosilane (273 mg, 2.5 mmol) in tetrahydrofuran (10 mL), the temperature of the reaction mixture was slowly raised to r.t. and keep stirring overnight. The reaction was quenched by saturated ammonium chloride (10 mL) and extracted by EtOAc (20 mL*3). The combined organic phases were washed by HCl solution (1M, 20 mL) and brine, dried over anhydrous sodium sulfate, filtered and concentrated,

the crude product was separated and purified by flash chromatography (PE/EtOAc = 8/1) to obtain the title compound (400 mg, 48%) in form of yellow solid. LCMS (ESI) $[M+H]^+$ = 329.0.

Step 3: (2-bromo-4-methylphenyl)(2-chloro-4-fluorophenyl)methanone

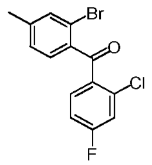

(2-bromo-4-methylphenyl)(2-chloro-4-fluorophenyl)methanol (400 mg, 1.2 mmol) and manganese dioxide (1.05 g, 12.0 mmol) were mixed in dichloromethane (10 mL), and then stirred at r.t. for 1 hour. The reaction mixture was filtered through a pad of celite and washed with dichloromethane (10 mL*2). The filtrate and washing solution was combined and concentrated. The residue was separated and purified by flash chromatography (PE/EtOAc = 10/1) to obtain the title compound (350 mg, 88%) in form of yellow solid. LCMS (ESI) $[M+H]^+$ = 327.0.

Step 4: (2-bromo-4-(dibromomethyl)phenyl)(2-chloro-4-fluorophenyl)methanone

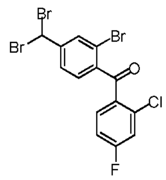

(2-bromo-4-methylphenyl)(2-chloro-4-fluorophenyl)methanone (350 mg, 1.1 mmol), N-bromosuccinimide (567 mg, 3.2 mmol) and azobisisobutyronitrile (17 mg, 0.11 mmol) were mixed in carbon tetrachloride (10 mL), the reaction mixture was heated to 80°C and stirred for 12 hours, spin dried and obtained crude product of the title compound (600 mg) in form of yellow solid, which was used directly in the next step without purification. LCMS (ESI) $[M+H]^+$ = 483.0.

Step 5: (2-bromo-4-(bromomethyl)phenyl)(2-chloro-4-fluorophenyl)methanone

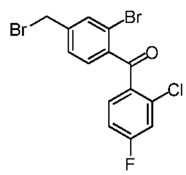

Diethyl phosphite (169 mg, 1.8 mmol) and diisopropylethylamine (475 mg, 3.68 mmol) were added into (2-bromo-4-(dibromomethyl)phenyl)(2-chloro-4-fluorophenyl)methanone (600 mg, 1.2 mmol) in dichloromethane solution (20 mL). NConcentration of the reaction mixture, the residue was purified by flash chromatography (PE/EtOAc = 15/1) to obtain the title compound (400 mg, yield of two steps 92%) in form of yellow solid. LCMS (ESI) $[M+H]^+$ = 405.0.

Step 6: (2-bromo-4-(ethylsulfonylmethyl)phenyl)(2-chloro-4-fluorophenyl)methanone

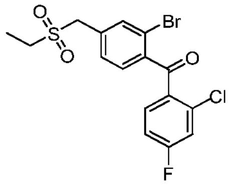

Sodium ethylsulfinate (343 mg, 2.9 mmol) was added into (2-bromo-4-(bromomethyl)phenyl)(2-chloro-4-fluorophenyl)methanone (400 mg, 0.9 mmol) in dimethylsulfoxide (10 mL), the reaction mixture was stirred at r.t. or 1 hour. EtOAc (20 mL) and water (20 mL) was added to layer the mixture, the organic phase was washed by brine (10 mL*3), dried over anhydrous sodium sulfate, filtered and concentrated, the residue was purified by flash chromatography (PE/EtOAc = 3/1) to obtain the title compound (320 mg, 78%) in form of brown solid. LCMS (ESI) $[M+H]^+$ = 419.0.

Step 7: (5-(2-(2-chloro-4-fluorobenzoyl)-5-(ethylsulfonylmethyl)phenyl)-1-methyl-2-oxo-1,2-dihydropyri din-4-yl)methyl *tert*-butylcarbamate

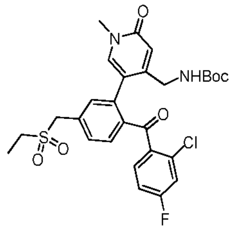

(2-bromo-4-(ethylsulfonylmethyl)phenyl)(2-chloro-4-fluorophenyl)methanone (160 mg, 0.38 mmol), (1-methyl-2-oxo-5-(4,4,5,5-tetramethyl-1,3,2-dioxoborolan-2-yl)-1,2-dihydropyridin-4-yl)methyl *tert*-butylcarbamate (272 mg, 0.73 mmol), [1,1'-bis(diphenylphosphino)ferrocene]palladium(II) dichloride (28 mg, 0.038 mmol) and potassium carbonate (162 mg, 0.6 mmol) were mixed in 1,4-dioxane (6 mL) and water (0.6 mL). The reaction mixture was heated to 95°C and stirred for 1 hour under nitrogen protection. Then filtered and spin dried, the residue was purified by flash chromatography (EtOAc/methanol = 30/1) to obtain the title compound (107 mg, 48%) in form of yellow solid. LCMS (ESI) $[M+H]^+$ = 576.1.

Step 8: 7-(2-chloro-4-fluorophenyl)-10-((ethylsulfone)methyl)-2-methyl-2,5-dihydro-3H-benzo[c]pyrido[ 3,4-e]azepin-3 -ketone

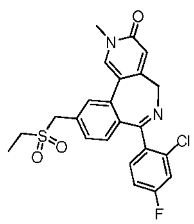

Trifluoroacetic acid (1 mL) and (5-(2-(2-chloro-4-fluorobenzoyl)-5-(ethylsulfonylmethyl)phenyl)-1-methyl-2-oxo-1,2-dihydropyri din-4-yl) *tert*-butylmethylcarbamate (120 mg, 0.2 mmol) were dissolved in dichloromethane (4 mL), and the mixture was stirred at r.t. for 1 hour, then concentrated, the residue was purified by preparative-HPLC to obtain the title compound (69.4 mg, 63%) in form of yellow solid. LCMS (ESI): $[M+H]^+$ = 458.0; $^1H$ NMR (400 MHz, $CD_3OD$) $\delta$ 8.10 (s, 1H), 7.71 (s, 1H), 7.61-7.58 (m, 1H), 7.44-7.39 (m, 2H), 7.36-7.31 (m, 1H), 7.07-7.05 (d, *J* = 8.0 Hz, 1H), 6.53 (s, 1H), 4.78-4.76 (d, *J* = 8 Hz, 1H), 4.63-4.50 (m, 2H), 3.97-3.95 (d, *J* = 8 Hz, 1H), 3.54 (s, 3H), 3.12-3.07 (m, 2H), 1.25-1.21 (m, 3H).

**Embodiment 4029: (S)-2-(7-(4-chlorophenyl)-2-methyl-3-oxo-3,5-dihydro-2H-dipyrido[2,3-c:3',4'-e]azepin-5-yl)acetamide**

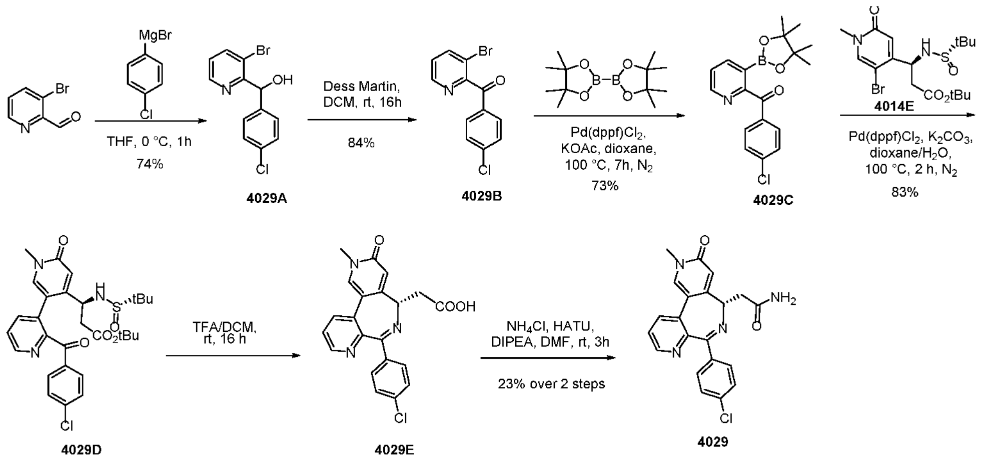


Step 1: (3-bromopyridin-2-yl)(4-chloropyridine)methanol

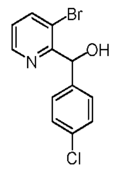

Under nitrogen protection at 0°C, (4-chlorophenyl)magnesium bromide (1 M in THF solution, 6 mL, 6 mmol) was added dropwise into 3-bromo-2-pyridinal (1.0 g, 5.38 mmol) in THF (20 mL), the reaction mixture was stirred at r.t. for 1 hour. The reaction was quenched by saturated ammonium chloride(30 mL), then extracted by EtOAc (50 mL*2). The organic phase was combined, then washed with brine (50 mL), dried over anhydrous sodium sulfate, filtered and concentrated, the title compound was obtained in form of white solid (1.2 g, 74.5%). LCMS (ESI) $[M+H]^+$ = 299.9.

Step 2: (3-bromopyridin-2-yl)(4-chlorophenyl)methanone

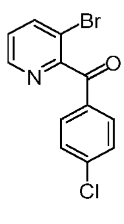

(3-bromopyridin-2-yl)(4-chloropyridin)methanol (1.2 g, 4.02 mmol) and Dess-Martin periodinane (2.56 g, 6.02 mmol) were mixture in dichloromethane (30 mL), then stirred at r.t. overnight. Then filtered and the filtrate was concentrated. The residue was purified by flash chromatography(PE/EtOAc=5/1), the title compound was obtained in form of white solid (1.0 g, 84%). LCMS (ESI) $[M+H]^+$ = 297.9.

Step 3 : (4-chlorophenyl)(3-(4,4,5,5-tetramethyl-1,3,2-dioxoborolan-2-yl)pyridin-2-yl)methanone

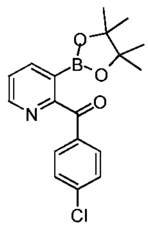

(3-bromopyridin-2-yl)(4-chlorophenyl)methanone (1.0 g, 3.37 mmol), bis(pinacolato)diboron (1.28 g, 5.06 mmol), [1,1'-bis(diphenylphosphino)ferrocene]palladium(II) dichloride (247 mg, 0.34 mmol) and sodium acetate (691 mg, 8.43 mmol) were mixed in 1,4-dioxane (20 mL). The reaction mixture was heated to 100°C and stirred for 7 hours under nitrogen protection. Then filtered and the filtrate was concentrated, the residue was purified by flash chromatography(PE/EtOAc=5/1), the title compound was obtained (640 mg, 73%), in form of yellow solid. LCMS (ESI) $[M+H]^+$ = 262.0.

step 4 : (*S*)-3-(5-(2-(4-chlorobenzoyl)pyridin-3-yl)-1-methyl-2-oxo-1,2-dihydropyridin-4-yl)-3-((*S*)-1,1-dimethylethylsulfin amide)*tert*-butylpropionate

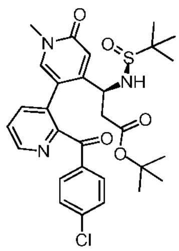

(*S*)-3-(5-bromo-1-methyl-2-oxo-1,2-dihydropyridin-4-yl)-3-((*S*)-1,1-dimethylethylsulfin amide)*tert*-butylpropionate(110 mg, 0.25 mmol), (4-chlorophenyl)(3-(4,4,5,5-tetramethyl-1,3,2-dioxoborolan-2-yl)pyridin-2-yl)methanone (132 mg, 0.38 mmol), [1,1'-bis(diphenylphosphino)ferrocene]palladium (II) dichloride (22 mg, 0.025 mmol) and potassium phosphate(132 mg, 0.625 mmol) were mixed in 1,4-dioxane(10 mL) and water(1 mL). The reaction mixture was heated to 100°C then stirred for 2 hours under nitrogen protection. Water and EtOAc were added into the reaction mixture, organic phase was then separated, dried over anhydrous sodium sulfate, filtered and concentrated, the residue was purified by flash chromatography(100% EtOAc), the title compound was obtained (120 mg, 83%), which was in form of yellow solid. LCMS (ESI) $[M+H]^+$ = 572.0.

step 5 : (*S*)-2-(7-(4-chlorophenyl)-2-methyl-3-oxo-3,5-dihydro-2*H*-dipyrido[2,3-*c*:3',4'-*e*]azepin-5-yl)aceti c acid

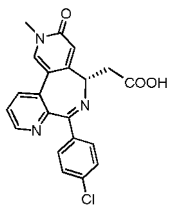

(*S*)-3-(5-(2-(4-chlorobenzoyl)pyridin-3-yl)-1-methyl-2-oxo-1,2-dihydropyridin-4-yl)-3-( (*S*)-1,1-dimethylethylsulfin amide) *tert*-butylpropionate (120 mg, 0.21 mmol) was dissolved in TFA (3 mL) and dichloromethane (3 mL), the reaction mixture was stirred at r.t. overnight. The reaction mixture was concentrated, the crude product of the title compound was obtained, which was in form of yellow oil (100 mg), and was used directly in the next step without purification. LCMS (ESI) $[M+H]^+$= 394.1.

Step 6 : (*S*)-2-(7-(4-chlorophenyl)-2-methyl-3-oxo-3,5-dihydro-2*H*-dipyrido[2,3-*c*:3',4'-*e*]azepin-5-yl)aceta mide

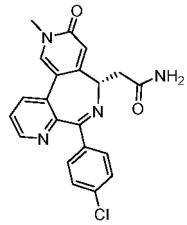

(*S*)-2-(7-(4-chlorophenyl)-2-methyl-3-oxo-3,5-dihydro-2*H*-dipyrido[2,3-*c*:3',4'-*e*]azepin-5-yl)acetic acid (50 mg, 0.105 mmol)) anhydrous ammonium chloride (12 mg, 0.21 mmol), 2-(7-azabenzotriazole)-*N*,*N*,*N'*,*N'*-tetramethylurea hexafluorophosphate (80 mg, 0.21 mmol) and *N*-ethyl-*N*-isopropyl propane-2-amine (41 mg, 0.315 mmol) were mixed in N,N-dimethylformamide (3 mL), and stirred at r.t. for 3 hours. The reaction mixture was filtered and the filtrate was concentrated. The residue was purified by preparative-HPLC, the title compound was obtained in form of white solid (9.5 mg, 23%). LCMS (ESI) $[M+H]^+$ = 393.1; $^1$H NMR (400 MHz, $CD_3OD$) $\delta$ 8.68-8.66 (m, 1H). 8.23-8.20 (m, 1H). 8.17 (s, 1H). 7.72-7.69 (m, 1H). 7.48-7.46 (m, 2H). 7.38-7.36 (m, 2H). 6.62 (s, 1H). 4.52-4.48 (m, 1H). 3.67 (s, 3H). 3.33-3.32 (m, 1H). 3.08-3.04 (m, 1H).

**Embodiment 4030** : **(*S*)-2-(7-(4-chlorophenyl)-2-methyl-3-oxo-3,5-dihydro-2*H*-dipyrido[2,3-*c*:3',4'-*e*]azepin-5-yl)-*N*-ethyl acetamide**

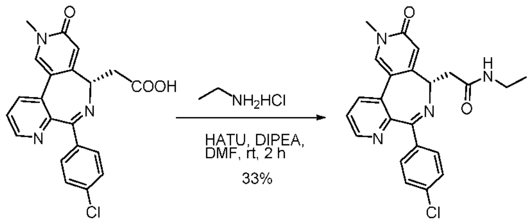


**4029E** **4030**

Step 1 : (*S*)-2-(7-(4-chlorophenyl)-2-methyl-3-oxo-3,5-dihydro-2*H*-dipyrido[2,3-*c*:3',4'-*e*]azepin-5-yl)-*N*-et hyl acetyl

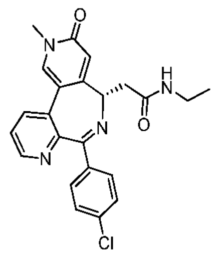

(*S*)-2-(7-(4-chlorophenyl)-2-methyl-3-oxo-3,5-dihydro-2*H*-dipyrido[2,3-*c*:3',4'-*e*]azepin-5-yl)acetic acid (50 mg, 0.105 mmol), ethylamine hydrochlorid (17 mg, 0.21 mmol), 2-(7-azabenzotriazole)-N,N,N',N'-tetramethylurea hexafluorophosphate (80 mg, 0.21 mmol) and *N*-ethyl-*N*-isopropyl propane-2-amine (41 mg, 0.315 mmol) were mixed in N,N-dimethylformamide (3 mL), and stirred at r.t. for 3 hours. The reaction mixture was filtered and the filtrate was concentrated. The residue was purified by preparative-HPLC, the title compound was obtained in form of white solid (14.6 mg, 33%). LCMS (ESI) $[M+H]^+$= 421.1; $^1$H NMR (400 MHz, $CD_3OD$) $\delta$ 8.68-8.66 (m, 1H). 8.23-8.20 (m, 1H). 8.17 (s, 1H). 7.72-7.69 (m, 1H). 7.47-7.45 (m, 2H). 7.38-7.36 (m, 2H). 6.60 (s, 1H). 4.52-4.49 (m, 1H). 3.67 (s, 3H). 3.26-3.20 (m, 3H). 3.04-2.99

(m, 1H). 1.20 (t, 3H).

**Embodiment 4031** : ***N*-(9-chloro-7-(4-flurophenyl)-2-methyl-3-oxo-3,5-dihydro-2*H*-benzo[*c*]pyrido[3,4-*e*]azepin-10-yl)ethylsulfonamide**

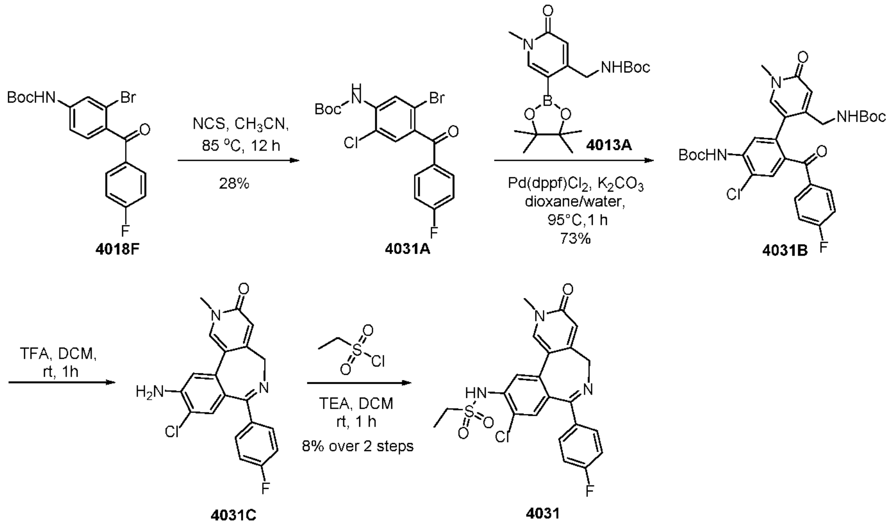


Step 1: 5-bromo-2-chloro-4-(4-fluorobenzoyl) *tert*-butylphenylcarbamate

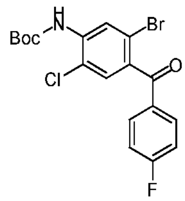

3-bromo-4-(4-fluorobenzoyl)*tert*-butylphenylcarbamate (200 mg, 0.5 mmol) and *N*-chlorosuccinimide (134 mg, 1.0 mmol) were mixed in acetonitrile (5 mL), and heated to 85°C and stirred for 12 hours, then concentrated. The residue was purified by flash chromatography (PE/EtOAc= 8/1), the title compound was obtained in form of yellow solid (60 mg, 27.5%). LCMS (ESI) $[M+H]^+$= 427.0.

Step 2 : ((5-(5-((*t*-butyloxycarboryl)amino)-4-chloro-2-(4-fluorobenzoyl)phenyl)-1-methyl-2-oxo-1,2-dihy dropyridin-4-yl)methyl)*tert*-butylcarbamate

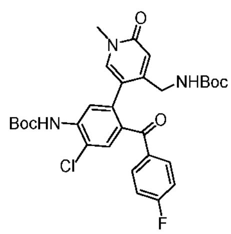

5-bromo-2-chloro-4-(4-fluorobenzoyl)*tert*-butylphenylcarbamate (60 mg, 0.14 mmol), (1-methyl-2-oxo-5-(4,4,5,5-tetramethyl-1,3,2-dioxaborolan-2-yl)-1,2-dihydropyridin-4-yl)methyl *tert*-butylcarbamate (77 mg, 0.21 mmol),

[1,1'-bis (diphenylphosphino)ferrocene]palladium (II) dichloride (10 mg, 0.014 mmol) and potassium carbonate (58 mg, 0.42 mmol) were mixed in 1,4-dioxane (2 mL) and water (0.2 mL). The reaction mixture was heated to 90°C under nitrogen protection then stirred for 1 hour. After filtrateion, the filtratee was spin dried, the residue was purified by preparative-TLC (EtOAc/PE=1/1), the title compound was obtained (60 mg, 73.1%), in form of yellow oil. LCMS (ESI) $[M+H]^+$ = 585.2.

Step 3 : 10-amino-9-chloro-7-(4-flurophenyl)-2-methyl-2,5-dihydro-3*H*-benzo[*c*]pyrido[3,4-*e*]azepin-3-ket one

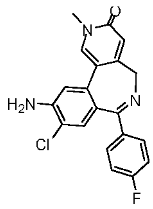

TFA (1 mL) was added dropwise ((5-(5-((t-butyloxycarboryl)amino)-4-chloro-2-(4-fluorobenzoyl)phenyl)-1-methyl-2-oxo-1,2-dihy dropyridin-4-yl)methyl)tert-butylcarbamate (60 mg, 0.1 mmol) in dichloromethane (4 mL), the reaction mixture was stirred at r.t. for 1 hour, and then concentrated to obtain the crude production of the title compound (80 mg), which was used directly in the next step. LCMS (ESI) $[M+H]^+$ = 367.1.

Step 4 : *N*-(9-chloro-7-(4-flurophenyl)-2-methyl-3-oxo-3,5-dihydro-2*H*-benzo[*c*]pyrido[3,4-*e*]azepin-10-yl )ethylsulfonamide

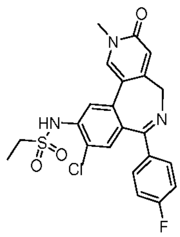

To 10-amino-9-chloro-7-(4-flurophenyl)-2-methyl-2,5-dihydro-3*H*-benzo[*c*]pyrido[3,4-*e*]azepin-3-ket one (80 mg, 0.22 mmol) and triethylamine (223 mg, 2.2 mmol) in dichloromethane solution (5 mL), ethylsulfonyl chloride (85 mg, 0.66 mmol) was added. The reaction mixture was stirred at r.t. overnight. The reaction mixture was washed by water and brine, dried over anhydrous sodium sulfate, filtered and concentrated. The residue was purified by preparative-HPLC, the title compound was obtained in form of white solid (3.9 mg, yield of the two steps 8%). LCMS (ESI): $[M+H]^+$ = 459.0; $^1H$ NMR (400 MHz, DMSO-$d_6$) $\delta$9.85 (s, 1H). 8.11 (s, 1H). 7.68 (s, 1H). 7.51-7.47 (m, 2H). 7.33 (s, 1H). 7.26-7.21 (d, *J* = 8.4 Hz, 2H). 6.51 (s, 1H). 4.74-4.72 (d, *J* = 10.4 Hz, 1H). 3.93-3.90 (d, *J* = 10.4 Hz, 1H). 3.49 (s, 3H). 3.18-3.14 (m, 2H). 1.25-1.21 (t, *J* = 6.8 Hz, 3H).

**Embodiment 4034** : **(*S*)-2-(7-(4-chlorophenyl)-9-fluoro-2-methyl-3-oxo-3,5-dihydro-2*H*-benzo[*c*]pyrido[3,4-*e*]azep in-5-yl)*N*-ethyl acetamide**

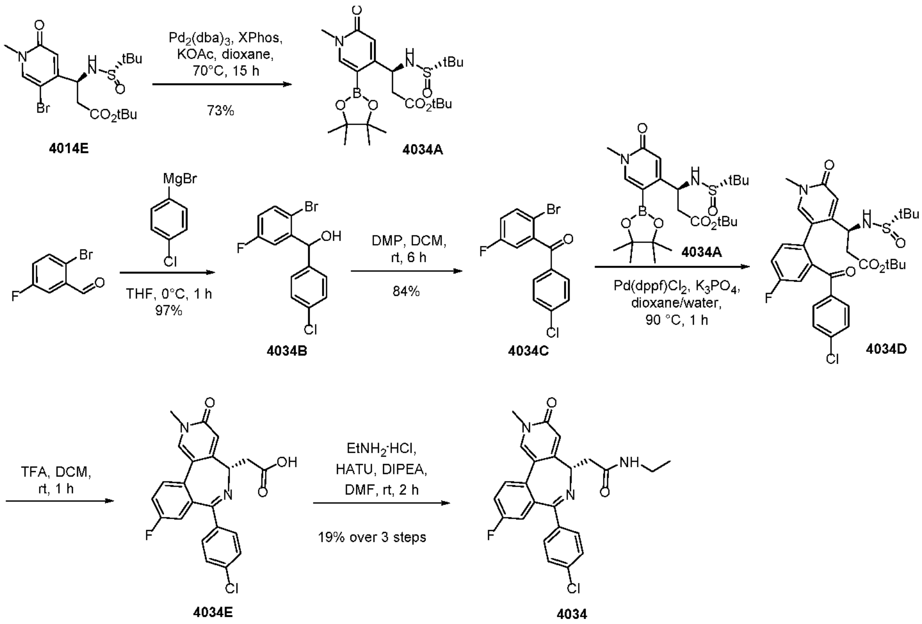


Step 1 : (*S*)-3-((*S*)-tert-butylsulfinamide)-3-(1-methyl-2-oxo-5-(4,4,5,5-tetramethyl-1,3,2-dioxaborolan-2-y l)-1,2-dihydropyridin-4-yl)tert-butylpropionate

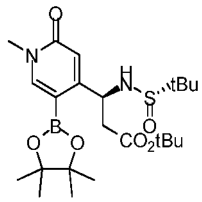

(*S*)-3-(5-bromo-1-methyl-2-oxo-1,2-dihydropyridin-4-yl)-3-(((*S*)-tert-butylsulfinamide) tert-butylpropionate (210 mg, 0.48 mmol), bis(pinacolato)diboron (371 mg, 1.45 mmol), tris(dibenzylideneacetone) palladium (88 mg, 0.096 mmol), 2-bicyclohexylphosphine-2',4',6'-triisopropyl biphenyl(91 mg, 0.192 mmol) and potassium acetate (94 mg, 0.96 mmol) were mixed in 1,4-dioxane (15 mL). The reaction mixture was heated to 70°C and stirred for 15 hours under nitrogen protection. Then filtered and the filtrate was concentrated, the residue was then dissolved in EtOAc, then washed with water and brine, dried over anhydrous sodium sulfate, filtered and concentrated. The residue was purified by flash chromatography (methanol /EtOAc=6/94), the title compound was obtained (170 mg, 73%), which was in form of brownish solid. LCMS (ESI) $[M+H]^+$ = 483.3.

Step 2: (2-bromo-5-flurophenyl)(4-chlorophenyl)methanol

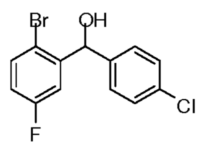

Under nitrogen protection at 0°C, 4-chlorophenylmagnesium bromide (1 M in THF solution, 5 mL, 5 mmol) was slowly added dropwise into 2-bromo-5-fluoro benzaldehyde (1.0 g, 4.93 mmol) in THF (10 mL). The reaction mixture was then stirred at 0°C for 1 hour, the reaction was quenched by saturated ammonium chloride (20 mL), and extracted by EtOAc (50 mL*3). The combined organic phases were washed by brine, dried over anhydrous sodium sulfate, filtered and concentrated, the crude product was separated and purified by flash chromatography (PE/EtOAc=5/1), the title

compound was obtainedin form of white solid (1.5 g, 97%), which was used directly in the next step

Step 3: (2-bromo-5-flurophenyl)(4-chlorophenyl)methanone

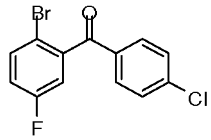

(2-bromo-5-flurophenyl)(4-chlorophenyl)methanol (1.5 g, 4.76 mmol) and Dess-Martin periodinane (2.02 g, 4.76 mmol) were mixed in dichloromethane (15 mL), and was stirred at r.t. for 6 hours. Then filtered and the filtrate was concentrated. The residue was purified by flash chromatography (PE/EtOAc=10/1), the title compound was obtained in form of white solid (1.25 g, 84%).

Step 4 : (*S*)-3-(((S)-tert-butylsulfinamide)-3-(5-(2-(4-chlorobenzoyl)-4-flurophenyl)-1-methyl-2-oxo-1,2-di hydropyridin-4-yl)tert-butylpropionate

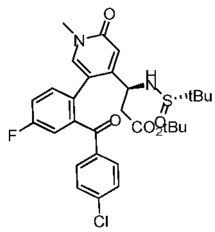

(*S*)-3-((*S*)-tert-butylsulfinamide)-3-(1-methyl-2-oxo-5-(4,4,5,5-tetramethyl-1,3,2-dioxab orolan-2-yl)-1,2-dihydropyridin-4-yl)tert-butylpropionate (170 mg, 0.35 mmol), (2-bromo-5-flurophenyl)(4-chlorophenyl)methanone (108 mg, 0.35 mmol), [1,1'-bis(diphenylphosphino) ferrocene]palladium (II) dichloride (26 mg, 0.034 mmol), and potassium phosphate (72 mg, 0.34 mmol) were mixed in 1,4-dioxane (10 mL) and water (1 mL). The reaction mixture was heated to 90°C then stirred for 1 hour under nitrogen protection. Water and EtOAc were added into the reaction mixture, organic phase was then separated, dried over anhydrous sodium sulfate, filtered and concentrated, the residue was purified by flash chromatography (dichloromethane/methanol=10/1), the title compound was obtained (130 mg, crude product), in form of brownish oil. LCMS (ESI) $[M+H]^+$= 589.2.

Step 5 : (*S*)-2-(7-(4-chlorophenyl)-9-fluoro-2-methyl-3-oxo-3,5-dihydro-2*H*-benzo[*c*]pyrido[3,4-*e*]azepin-5-yl)acetic acid

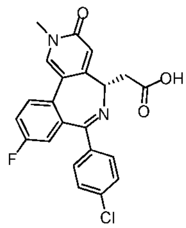

(*S*)-3-(((*S*)-tert-butylsulfinamide)-3-(5-(2-(4-chlorobenzoyl)-4-flurophenyl)-1-methyl-2-oxo-1,2-dihydropyridin-4-yl)tert-butylpropionate (130 mg, 0.22 mmol) were disolved in TFA (2 mL) and dichloromethane (6 mL), the reaction mixture was stirred at r.t. for 16 hours. The reaction mixture was concentrated, the crude product of the title compound was obtained, in form of brownish oil (90 mg, crude product), and was used directly in the next step without purification. LCMS (ESI) $[M+H]^+$= 410.9.

Step 6 : (*S*)-2-(7-(4-chlorophenyl)-9-fluoro-2-methyl-3-oxo-3,5-dihydro-2*H*-benzo[*c*]pyrido[3,4-*e*]azepin-5-yl)-*N*-ethyl acetamide

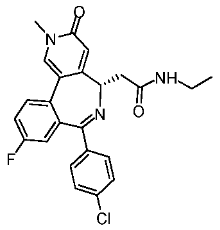

(*S*)-2-(7-(4-chlorophenyl)-9-fluoro-2-methyl-3-oxo-3,5-dihydro-2*H*-benzo[*c*]pyrido[3,4-*e*]azepin-5-yl)acetic acid (90 mg, 0.22 mmol), ethylamine hydrochlorid (35 mg, 0.44 mmol), 2-(7-azabenzotriazole)-N,N,N',N'-tetramethylurea hexafluorophosphate (168 mg, 0.44 mmol) and *N*-ethyl-*N*-isopropylpropane-2-amine (142 mg, 1.10 mmol) were mixed in N,N-dimethylformamide (5 mL), and stirred for 2 hours at rt. The reaction mixture was filtered and the filtrate was concentrated. The residue was purified by preparative-HPLC, the title compound was obtained in form of white solid (18.7 mg, yield of the three steps 19%). LCMS (ESI) $[M+H]^+$= 438.1; $^1H$ NMR (400 MHz, DMSO-$d_6$) $\delta$ 8.20 (t, *J* = 5.2 Hz, 1H). 8.10 (s, 1H). 7.78 (dd, *J* = 8.8, 5.2 Hz, 1H). 7.57 (dt, *J* = 8.8, 2.8 Hz, 1H). 7.49-7.41 (m, 4H). 7.13 (dd, *J* = 9.2, 2.8 Hz, 1H). 6.39 (s, 1H). 4.24 (t, *J* = 7.2 Hz, 2H). 3.47 (s, 3H). 3.08 (q, *J* = 7.6 Hz, 2H). 2.97 (d, *J* = 7.6 Hz, 1H). 1.03 (t, *J* = 7.4 Hz, 3H).

**Embodiment 4035 : (*S*)-*N*-ethyl-2-(8-fluoro-7-(4-flurophenyl)-9-methoxyl-2-methyl-3-oxo-3,5-dihydro-2*H*-benzo[ *c*]pyrido[3,4-*e*]azepin-5-yl)acetamide**

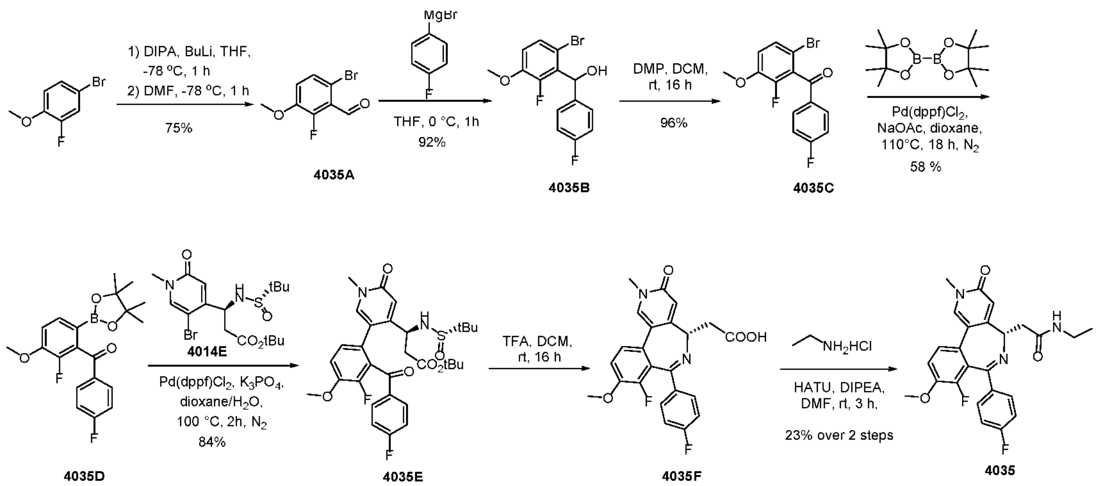


Step 1: 6-bromo-2-fluoro-3-methoxylbenzaldehyde

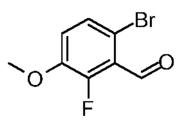

At -78°C and under nitrogen protectioN,N-butyllithium (2.5 M in THF solution, 5.0 mL, 12.5 mmol) was slowly added dropwise into diisopropylamine (1.28 g, 12.68 mmol) in anhydrous THF (20 mL) solution, the reaction mixture was stirred at -78°C for 10 minitues, the solution was slowly added dropwise into 4-bromo-2-fluoro-1-methoxylbenzene

(2.0 g, 9.75 mmol) in anhydrous THF (5 mL) solution, and then under this temperature continue stirring for 1 hour. *N,N*-dimethylformamide (1.07 g, 14.63 mmol) was slowly added dropwise into in the solution, and continue stirring for 1 hour. The reaction was quenched by saturated ammonium chloride (30 mL), and then extracted by EtOAc (60 mL*2). The organic phase was combined, then washed with brine (50 mL), dried over anhydrous sodium sulfate, filtered and concentrated, the crude product was separated and purified by flash chromatography (PE/EtOAc=10/1), the title compound was obtained (1.7 g, 75%), which was in form of white solid.

Step 2: (6-bromo-2-fluoro-3-methoxylbenzyl)(4-flurophenyl)methanol

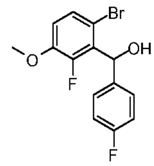

Under nitrogen protection at 0°C, (4-flurophenyl)magnesium bromide (1 M in THF solution, 4 mL, 4 mmol) was slowly added dropwise into 6-bromo-2-fluoro-3-methoxylbenzaldehyde (850 mg, 3.65 mmol) in THF (20 mL) solution. The reaction mixture at 0°C then stirred for 1 hour. The reaction was quenched by water (30 mL), and extracted by EtOAc (50 mL*3). The organic phase was combined, then washed with brine (50 mL), dried over anhydrous sodium sulfate, filtered and concentrated, the title compound was obtained (1.1 g, 92%), in form of yellow oil. LCMS (ESI) $[M+H]^+$= 311.0.

Step 3: (6-bromo-2-fluoro-3-methoxylbenzyl)(4-flurophenyl)methanone

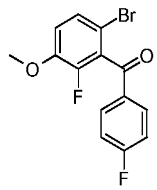

(6-bromo-2-fluoro-3-methoxylbenzyl)(4-flurophenyl)methanol (1.1 g, 3.34 mmol) and Dess-Martin periodinane (2.8 g, 6.68 mmol) were mixed in dichloromethane (30 mL), and was stirred at r.t. for 16 hours. Then filtered and the filtrate was concentrated. The residue was purified by flash chromatography (PE/EtOAc=10/1), the title compound was obtained in form of white solid (1.05 g, 96%). LCMS (ESI) $[M+H]^+$ = 327.0.

Step 4 : (2-fluoro-3-methoxyl-6-(4,4,5,5-tetramethyl-1,3,2-dioxaborolan-2-yl)phenyl)(4-flurophenyl)meth anone

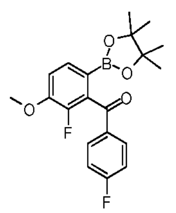

(6-bromo-2-fluoro-3-methoxylbenzyl)(4-flurophenyl)methanone (1.05 g, 3.21 mmol), bis(pinacolato)diboron (1.63 g, 6.42 mmol), [1,1'-bis(diphenylphosphino)ferrocene]palladium (II) dichloride (117 mg, 0.16 mmol) and sodium acetate (789 mg, 9.63 mmol) were mixed in dioxane (20 mL). Under nitrogen protection, the reaction mixture was heated to 110°C and stirred for 18 hours. Then filtered and the filtrate was concentrated, the residue was purified by flash

chromatography (PE/EtOAc=10/1), the title compound was obtained (700 mg, 58%), which was in form of white solid. LCMS (ESI) $[M+H]^+$= 375.1.

Step 5 : (*S*)-3-(((S)-tert-butylsulfinamide)-3-(5-(3-fluoro-2-(4-fluorobenzoyl)-4-methoxylphenyl)-1-methyl -2-oxo-1,2-dihydropyridin-4-yl)tert-butylpropionate

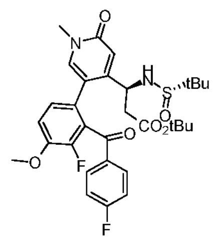

(*S*)-3-(5-bromo-1-methyl-2-oxo-1,2-dihydropyridin-4-yl)-3-(((*S*)-tert-butylsulfinamide)t ert-butylpropionate (120 mg, 0.276 mmol), (2-fluoro-3-methoxyl-6-(4,4,5,5-tetramethyl-1,3,2-dioxaborolan-2-yl)phenyl)(4-flurophenyl)methanone (155 mg, 0.413 mmol), [1,1'-bis(diphenylphosphino)ferrocene]palladium (II) dichloride (10 mg, 0.014 mmol) and potassium phosphate (146 mg, 0.69 mmol) were mixed in 1,4-dioxane (4 mL) and water (0.4 mL). Under nitrogen protection, the reaction mixture was heated to 100°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=19/1), the title compound was obtained (140 mg, 84%), which was in form of yellow solid. LCMS (ESI) $[M+H]^+$ = 603.2.

Step 6 : (*S*)-2-(8-fluoro-7-(4-flurophenyl)-9-methoxyl-2-methyl-3-oxo-3,5-dihydro-2*H*-benzo[*c*]pyrido[3,4 -*e*]azepin-5-yl)acetic acid

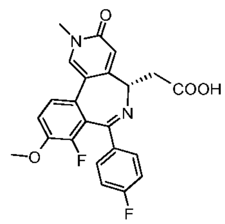

(S)-3-(((S)-tert-butylsulfinamide)-3-(5-(3-fluoro-2-(4-fluorobenzoyl)-4-methoxylphenyl) -1-methyl-2-oxo-1,2-dihydropyridin-4-yl)tert-butylpropionate (140 mg, 0.23 mmol) was dissolved in TFA (3 mL) and dichloromethane (3 mL), the reaction mixture was stirred at r.t. overnight. The reaction mixture was concentrated, the crude product of the title compound was obtained, in form of brownish oil (130 mg), and was used directly in the next step without purification. LCMS (ESI) $[M+H]^+$= 425.1.

Step 7 : (*S*)-2-(8-fluoro-7-(4-flurophenyl)-9-methoxyl-2-methyl-3-oxo-3,5-dihydro-2*H*-benzo[*c*]pyrido[3,4 -*e*]azepin-5-yl)-*N*-ethyl acetamide

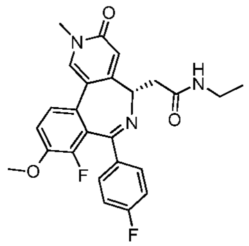

(*S*)-2-(8-fluoro-7-(4-flurophenyl)-9-methoxyl-2-methyl-3-oxo-3,5-dihydro-2*H*-benzo[*c*] pyrido[3,4-*e*]azepin-5-yl)acetic acid (130 mg crude product), ethylamine hydrochlorid (38 mg, 0.464 mmol), 2-(7-azabenzotriazole)-N,N,N',N'-tetramethylurea hexafluorophosphate (176 mg, 0.464 mmol) and *N*-ethyl-*N*-isopropylpropane-2-amine (90 mg, 0.696 mmol) were mixed in N,N-dimethylformamide (3 mL), and stirred at r.t. for 3 hours. The reaction mixture was filtered and the filtrate was concentrated. The residue was purified by preparative-HPLC, the title compound was obtained in form of white solid (24 mg, yield of the two steps 23%). LCMS (ESI) $[M+H]^+$= 452.1; $^1$H NMR (400 MHz, $CD_3OD$) $\delta$ 8.07 (s, 1H). 7.51-7.46 (m, 4H). 7.11-7.07 (m, 2H). 6.56 (s, 1H). 4.52-4.51 (m, 1H). 3.97 (s, 2H). 3.64 (s, 3H). 3.28-3.17 (m, 3H). 3.02-2.98 (m, 1H). 1.20 (t, 3H).

**Embodiment 4036: (*S*)-*N*-ethyl-2-(10-fluoro-7-(4-flurophenyl)-9-methoxyl-2-methyl-3-oxo-3,5-dihydro-2*H*-benzo [*c*]pyrido[3,4-*e*]azepin-5-yl)acetamide**

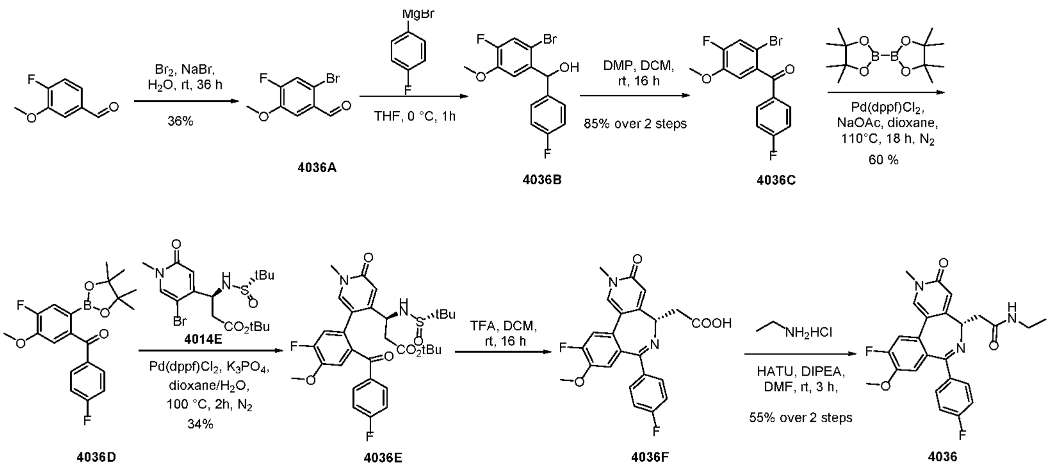


Step 1: 2-bromo-4-fluoro-5-methoxylbenzaldehyde

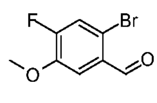

4-fluoro-3-methoxylbenzaldehyde (2.0 g, 12.98 mmol), sodium bromide (6.68 g, 64.88 mmo) and bromine (5.18 g, 32.44 mmol) were mixed in water (50 mL), then stirred at r.t. for 36 hours, white precipitation appeared and then it was filtered and washed by water, the title compound was obtained (1.1 g, 36%) in form of white solid. LCMS (ESI) $[M+H]^+$ = 234.9

Step 2: (2-bromo-4fluoro-5-methoxylbenzyl)(4-flurophenyl)methanol

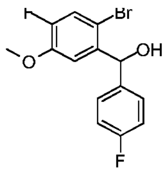

Under nitrogen protection at 0°C, (4-flurophenyl)magnesium bromide (1 M in THF solution, 2.6 mL, 2.6 mmol) was slowly added dropwise inot 2-bromo-4-fluoro-5-methoxylbenzaldehyde (550 mg, 2.36 mmol) in THF (10 mL) solution. At 0°C, the reaction mixture was then stirred for 1 hour. The reaction was quenched by water (30 mL), and extracted by EtOAc (50 mL*3). The organic phase was combined, then washed with brine (50 mL), dried over anhydrous sodium sulfate, filtered and concentrated, the title compound was obtained (900 mg, crude product), in form of yellow oil. LCMS (ESI) $[M+H]^+$= 312.9.

Step 3: (2-bromo-4fluoro-5-methoxylbenzyl)(4-flurophenyl)methanone

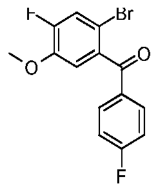

(2-bromo-4fluoro-5-methoxylbenzyl)(4-flurophenyl)methanol (900 mg, crude product) and Dess-Martin periodinane (1.5 g, 3.54 mmol) were mixed in dichloromethane (20 mL), and was stirred at r.t. for 16 hours. Then filtered and the filtrate was concentrated. The residue was purified by flash chromatography (PE/EtOAc=10/1), the title compound was obtained in form of white solid (660 mg, yield of the two steps 85%). LCMS (ESI) $[M+H]^+$ = 327.0.

Step 4: (4-fluoro-5-methoxyl-6-(4,4,5,5-tetramethyl-1,3,2-dioxaborolan-2-yl)phenyl)(4-flurophenyl)meth anone

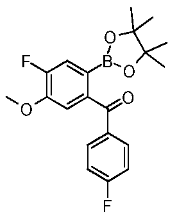

(2-bromo-4fluoro-5-methoxylbenzyl)(4-flurophenyl)methanone (660 mg, 2.02 mmol), Bis(pinacolato)diboron (1.02 g, 4.04 mmol), [1,1'-bis(diphenylphosphino)ferrocene]palladium (II) dichloride (74 mg, 0.1 mmol) and sodium acetate (496 mg, 6.05 mmol) were mixed in dioxane (20 mL). Under nitrogen protection, the reaction mixture was heated to 110°C and stirred for 18 hours. Then filtered and the filtrate was concentrated, the residue was purified by flash chromatography (PE/EtOAc=10/1), the title compound was obtained (450 mg, 60%), which was in form of white solid. LCMS (ESI) $[M+H]^+$= 375.1.

Step 5: (*S*)-3-(((S)-tert-butylsulfinaniide)-3-(5-(5-fluoro-2-(4-fluorobenzoyl)-4-methoxylphenyl)-1-methyl -2-oxo-1,2-dihydropyridin-4-yl) tert-butylpropionate

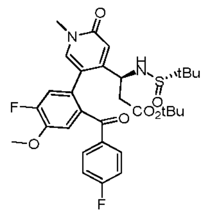

(*S*)-3-(5-bromo-1-methyl-2-oxo-1,2-dihydropyridin-4-yl)-3-(((*S*)-tert-butylsulfinamide) tert-butylpropionate (120 mg, 0.276 mmol), (4-fluoro-5-methoxyl-6-(4,4,5,5-tetramethyl-1,3,2-dioxaborolan-2-yl)phenyl)(4-flurophenyl)methanone (155 mg, 0.413 mmol), [1,1'-bis(diphenylphosphino)ferrocene]palladium (II) dichloride (10 mg, 0.014 mmol) and potassium phosphate (146 mg, 0.69 mmol) were mixed in 1,4-dioxane (4 mL) and water (0.4 mL). Under nitrogen protection, the reaction mixture was heated to 100°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=19/1), the title compound was obtained (80 mg, 34%), which was in form of yellow solid. LCMS (ESI) $[M+H]^+$ = 603.2.

Step 6: (*S*)-2-(10-fluoro-7-(4-flurophenyl)-9-methoxyl-2-methyl-3-oxo-3,5-dihydro-2*H*-benzo[*c*]pyrido[3, 4-*e*]azepin-5-yl)acetic acid

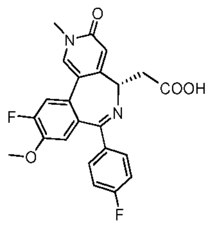

(*S*)-3-(((*S*)-tert-butylsulfinamide)-3-(5-(5-fluoro-2-(4-fluorobenzoyl)-4-methoxylphenyl) -1-methyl-2-oxo-1,2-dihydropyridin-4-yl) tert-butylpropionate (80 mg, 0.13 mmol) was dissolved in TFA (3 mL) and dichloromethane (3 mL), the reaction mixture was stirred at r.t. overnight. The reaction mixture was concentrated, the crude product of the title compound was obtained, which was in form of yellow oil (70 mg), and was used directly in the next step without purification. LCMS (ESI) $[M+H]^+$ = 425.1.

Step 7: (*S*)-2-(10-fluoro-7-(4-flurophenyl)-9-methoxyl-2-methyl-3-oxo-3,5-dihydro-2*H*-benzo[*c*]pyrido[3, 4-*e*]azepin-5-yl)-*N*-ethyl acetamide

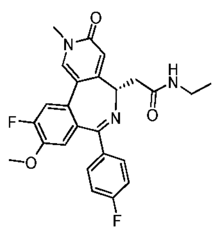

(*S*)-2-(10-fluoro-7-(4-flurophenyl)-9-methoxyl-2-methyl-3-oxo-3,5-dihydro-2*H*-benzo[*c* ]pyrido[3,4-*e*]azepin-5-yl)acetic acid (70 mg crude product), ethylamine hydrochlorid (22 mg, 0.266 mmol), 2-(7-azabenzotriazole)-N,N,N',N'-tetramethylurea hexafluorophosphate (101 mg, 0.266 mmol) and *N*-ethyl-*N*-isopropylpropane-2-amine (52 mg, 0.399 mmol) were mixed in N,N-dimethylformamide (3 mL), and stirred at r.t. for 3 hours. The reaction mixture was filtered and the filtrate was concentrated. The residue was purified by preparative-HPLC, the title compound was obtained in form of white solid (29 mg, yield of the two steps 55%). LCMS (ESI) $[M+H]^+$= 452.1; $^1H$ NMR (400 MHz, $CD_3OD$) $\delta$ 8.06 (s, 1H). 7.56-7.50 (m, 3H). 7.16-7.12 (m, 2H). 6.98 (d, *J* = 8.4 Hz, 1H). 6.55 (s, 1H). 4.46-4.45 (m, 1H). 3.80 (s, 2H).

3.65 (s, 3H). 3.28-3.17 (m, 3H). 3.00-2.96 (m, 1H). 1.21 (t, 3H).

**Embodiment 4037: (*S*)-10-((ethylsulfonyl)methyl)-7-(4-chlorophenyl)-2,5-dimethyl-2,5-dihydro-3*H*-benzo[*c*]pyrido[3,4-*e*]azepin-3-ketone**

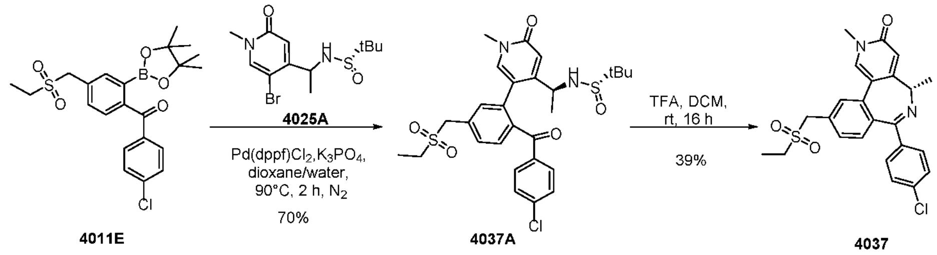


Step 1: (*S*)-*N*-((*S*)-1-(5-(5-((ethylsulfonyl)methyl)-2-(4-chlorobenzoyl)phenyl)-1-methyl-2-oxo-1,2-dihydr opyridin-4-yl)ethyl)-2-methylpropane-2-sulfenamide

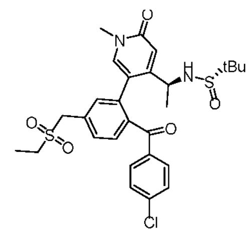

(*S*)-*N*-(1-(5-bromo-1-methyl-2-oxo-1,2-dihydropyridin-4-yl)ethyl)-2-methylpropane-2-s ulfenamide (50 mg, 0.15 mmol), (4-(ethylsulfonylmethyl)-2-(4,4,5,5-tetramethyl-1,3,2-dioxoborolan-2-yl)phenyl)(4-chlorophenyl) methanone (103 mg, 0.23 mmol), [1,1'-bis(diphenylphosphino)ferrocene]palladium (II) dichloride (22 mg, 0.030 mmol) and potassium phosphate (95 mg, 0.45 mmol) were mixed in 1,4-dioxane (10 mL) and water (1 mL). Under nitrogen protection, the reaction mixture was heated to 90°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=97/3), the title compound was obtained (60 mg, 70%), which was pale-brownish solid. MS (ESI): $m/z$ = 577.2 $[M+H]^+$.

Step 2: (*S*)-10-((ethylsulfonyl)methyl)-7-(4-chlorophenyl)-2,5-dimethyl-2,5-dihydro-3*H*-benzo[*c*]pyrido[3 ,4-*e*]azepin-3-ketone

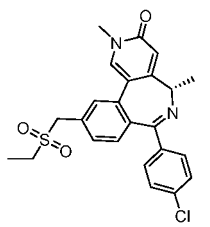

(*S*)-*N*-((*S*)-1-(5-(5-((ethylsulfonyl)methyl)-2-(4-chlorobenzoyl)phenyl)-1-methyl-2-oxo-1,2-dihydropyridin-4-yl)ethyl)-2-methylpropane-2-sulfenamide (60 mg, 0.10 mmol) was dissolved in dichloromethane (3 mL) and TFA (3 mL), the reaction mixture was stirred at r.t. overnight. The reaction mixture was concentrated, the residue was purified by preparative-HPLC, the title compound was obtained in form of white solid (18 mg, 39%). LCMS (ESI) $[M+H]^+$ =454.9;

$^1$H NMR (400 MHz, DMSO-$d_6$) $\delta$ 8.03 (s, 1H). 7.71 (s, 1H). 7.56-7.41 (m, 5H). 7.34 (d, $J$ = 8.4 Hz, 1H). 6.42 (s, 1H). 4.61 (q, $J$ = 13.6, 39.2 Hz, 2H). 4.01-3.94 (m, 1H). 3.50 (s, 3H). 3.13 (q, $J$ = 7.6, 14.8 Hz, 2H). 1.66 (d, $J$ = 6.4 Hz, 3H). 1.26 (t, $J$ = 7.6 Hz, 3H).

**Embodiment 4038: (*S*)-5-ethyl-10-((ethylsulfonyl)methyl)-7-(4-flurophenyl)-2-methyl-2,5-dihydro-3*H*-benzo[c]pyrido[3,4-*e*]azepin-3-ketone**

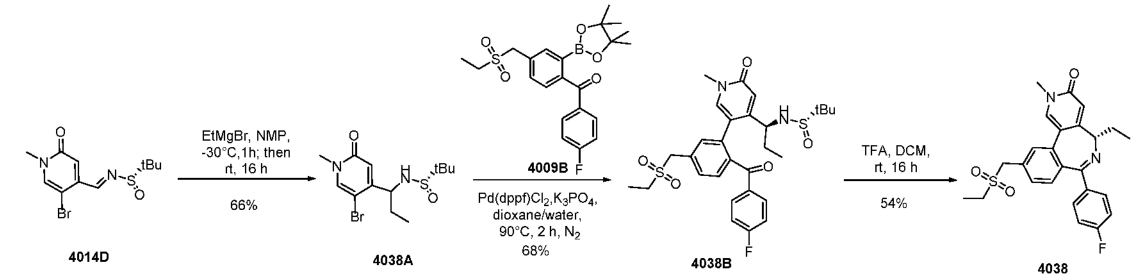


Step 1: (*S*)-*N*-(1-(5-bromo-1-methyl-2-oxo-1,2-dihydropyridin-4-yl)propyl)-2-methylpropane-2-sulfenami de

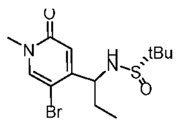

Under nitrogen protection and at -30°C, to (*S*)-*N*-((5-bromo-1-methyl-2-oxo-1,2-dihydropyridin-4-yl)methyl)-2-methylpropane-2-sulfenamid e (320 mg, 1.00 mmol) in 1-methyl-2-pyrrolidone (20 mL) solution, ethylmagnesium bromide (1 M in THF solution, 5 mL, 5 mmol) was slowly added dropwisely, then the reaction mixture was stirred at 30°C for 1 hour, and slowly raised the temperature to r.t. and stirred overnight. The reaction mixture the reaction was quenched by water (50 mL), then extrated by EtOAc (50 mL*4). Then the organic phase was combined, dried over anhydrous sodium sulfate, filtered and concentrated. The residue was purified by preparative-HPLC, the title compound was obtained (230 mg, 66%) in form of white solid. LCMS (ESI) [M+H]$^+$ = 349.0.

Step 2: (*S*)-*N*-((*S*)-1-(5-(5-((ethylsulfonyl)methyl)-2-(4-fluorobenzoyl)phenyl)-1-methyl-2-oxo-1,2-dihydr opyridin-4-yl)propyl)-2-methylpropane-2-sulfenamide

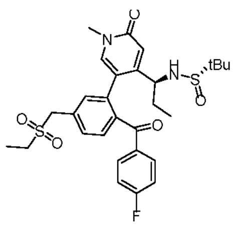

(*S*)-*N*-(1-(5 -bromo-1 -methyl-2-oxo-1,2-dihydropyridin-4-yl)propyl)-2-methylpropane-2-sulfenamide (80 mg, 0.23 mmol), (4-(ethylsulfonylmethyl)-2-(4,4,5,5-tetramethyl-1,3,2-dioxoborolan-2-yl)phenyl)(4-flurophenyl)m ethanone (151 mg, 0.35 mmol), [1,1'-bis(diphenylphosphino)ferrocene]palladium (II) dichloride (17 mg, 0.023 mmol) and potassium phosphate (146 mg, 0.69 mmol) were mixed in 1,4-dioxane (10 mL) and water (1 mL). Under nitrogen protection, the reaction mixture was heated to 90°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=97/3), the title compound was obtained (90 mg, 68%), which was pale-brownish solid. LCMS (ESI) [M+H]$^+$ = 575.2.

Step 3: ((*S*)-5-ethyl-10-((ethylsulfonyl)methyl)-7-(4-flurophenyl)-2-methyl-2,5-dihydro-3*H*-benzo[*c*]pyrid o[3,4-*e*]azepin-3-ketone

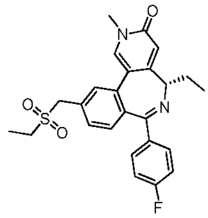

(*S*)-*N*-((*S*)-1-(5-(5-((ethylsulfonyl)methyl)-2-(4-fluorobenzoyl)phenyl)-1-methyl-2-oxo-1,2-dihydropyridin-4-yl)propyl)-2-methylpropane-2-sulfenamide (90 mg, 0.16 mmol) was dissolved in dichloromethane (3 mL) and TFA (3 mL), the reaction mixture was stirred at r.t. overnight. The reaction mixture was concentrated, the residue was purified by preparative-HPLC, the title compound was obtained in form of white solid (39 mg, 54%). LCMS (ESI) $[M+H]^+$ =453.2; $^1H$ NMR (400 MHz, DMSO-$d_6$) $\delta$ 8.03 (s, 1H). 7.71 (s, 1H). 7.52-7.45 (m, 3H). 7.34 (d, *J* = 8.0 Hz, 1H). 7.23 (t, *J* = 8.8 Hz, 2H). 6.39 (s, 1H). 4.61 (q, *J* = 13.2, 37.6 Hz, 2H). 3.63-3.58 (m, 1H). 3.50 (s, 3H). 3.15 (q, *J* = 7.2, 14.8 Hz, 2H). 2.26-2.16 (m, 1H). 2.06-1.96 (m, 1H). 1.27 (t, *J* = 7.2 Hz, 3H). 1.01 (t, *J* = 7.2 Hz, 3H).

**Embodiment 4039: 9-((ethylsulfonyl)methyl)-7-(4-flurophenyl)-2-methyl-2,5-dihydro-3*H*-benzo[*c*]pyrido[3,4-*e*]azepin-3-ketone**

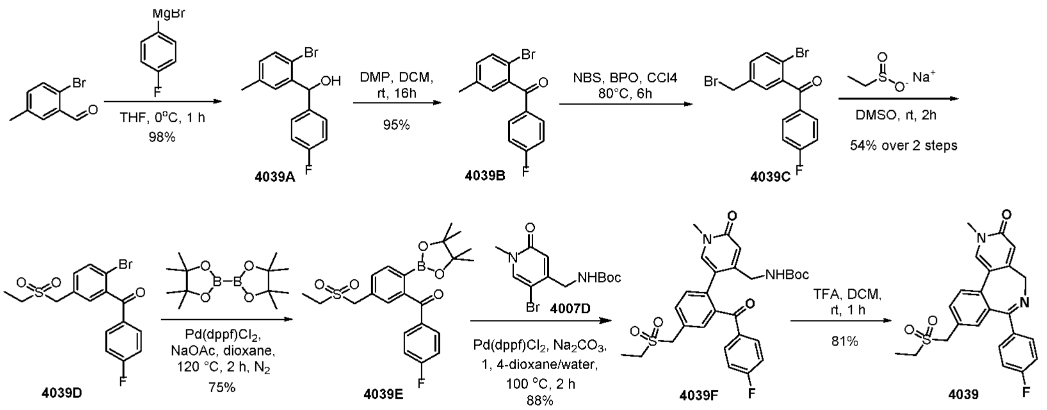


Step 1: (2-bromo-5-methylphenyl)(4-flurophenyl)methanol

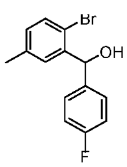

At 0°C and under nitrogen protection, (4-flurophenyl)magnesium bromide (1 M in THF solution, 11 mL, 11 mmol) was slowly added dropwise into 2-bromo-5-methylbenzaldehyde (2.0 g, 10 mmol) in THF solution (20 mL), the reaction mixture was then stirred for 1 hour at 0°C. The reaction was quenched by water (60 mL) and extracted by EtOAc (100 mL*2). The organic phase was combined, then washed with brine (50 mL), dried over anhydrous sodium sulfate, filtered and concentrated, the title compound was obtainedcrude product (2.9 g, 98%), in form of yellow oil. LCMS (ESI) $[M-18+H]^+$ = 276.9.

Step 2: (2-bromo-5-methylphenyl)(4-flurophenyl)methanone

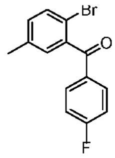

(2-bromo-5-methylphenyl)(4-flurophenyl)methanol (2.9 g, 10 mmol) and Dess-Martin periodinane (6.36 g, 15 mmol) were mixed in dichloromethane (30 mL), and was stirred at r.t. for 16 hours, then concentrated. The residue was purified by flash chromatography (PE/EtOAc=20/1), the title compound was obtained in form of white solid (2.8 g, 94.9%). LCMS (ESI) $[M+H]^+$= 295.0.

Step 3: (2-bromo-5-(bromomethyl)phenyl)(4-flurophenyl)methanone

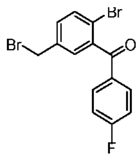

(2-bromo-5-methylphenyl)(4-flurophenyl)methanone (1.4 g, 4.78 mmol), *N*-bromosuccinimide (935 mg, 5.25 mmol) and benzoyl peroxide (116 mg, 0.48 mmol) were mixed in tetrachloromethane (20 mL), and heated to 80°C then stirred for 6 hours, the reaction mixture was cooled down to r.t. and diluted with EtOAc (100 mL), washed by 5% sodium carbonate aq. (40 mL*2) and brine (40 mL), dried over anhydrous sodium sifate, filtered and concentrated, the title compound was obtainedcrude product (1.8 g) which was in form of yellow solid, and was used directly in the next step without purification. LCMS (ESI) $[M+H]^+$ = 372.7.

Step 4: (2-bromo-5-(ethylsulfonylmethyl)phenyl)(4-flurophenyl)methanone

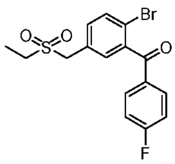

sodium ehtylsulfinate (1.67 g, 14.34 mmol) was added to (2-bromo-5-(bromomethyl)phenyl)(4-flurophenyl)methanone (1.8 g, crude product) in dimethylsulfoxide solution (20 mL), the reaction mixture then stirred at r.t. for 2 hours. EtOAc (100 mL) and water (20 mL) were added into the reaction mixture, then the organic phase was combined, washed by saturated brine (10 mL*3), dried over anhydrous sodium sulfate, filtered and concentrated, the residue was purified by flash chromatography (PE/EtOAc=2/1), the title compound was obtained in form of brownish solid (1 g, yield of the two steps 54%). LCMS (ESI) $[M+H]^+$= 384.9.

Step 6: (5-(ethylsulfonylmethyl)-2-(4,4,5,5-tetramethyl-1,3,2-dioxoborolan-2-yl)phenyl(4-flurophenyl)me thanone

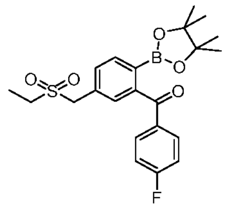

(2-bromo-5-(ethylsulfonylmethyl)phenyl)(4-flurophenyl)methanone (950 mg, 2.49 mmol), bis(pinacolato)diboron (1.26 g, 4.97 mmol), [1,1'-bis(diphenylphosphino)ferrocene]palladium (II) dichloride (88 mg, 0.12 mmol) and sodium acetate (613 mg, 7.49 mmol) were mixed in 1,4-dioxane (20 mL). Under nitrogen protection, the reaction mixture was heated to 120°C then stirred for 2 hours. Then filtered and the filtrate was concentrated, the residue was purified by flash chromatography (PE/EtOAc=10/1), the title compound was obtained (850 mg, 75%), in form of yellow oil. LCMS (ESI) $[M+H]^+$ = 433.1.

Step 6: (5-(4-(ethylsulfonylmethyl)-(2-(4-fluorobenzoyl)phenyl)-1-methyl-2-oxo-1,2-dihydropyridin-4-yl) methylamino tert-butylcarboxylate

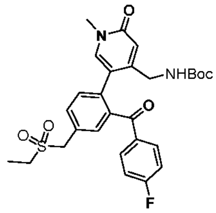

(5-bromo-1-methyl-2-oxo-1,2-dihydropyridin-4-yl)methylamino tert-butyl formate (100 mg, 0.315 mmol), (5-(ethylsulfonylmethyl)-2-(4,4,5,5-tetramethyl-1,3,2-dioxoborolan-2-yl)phenyl(4-flurophenyl)me thanone (204 mg, 0.473 mmol), [1,1'-bis(diphenylphosphino)ferrocene]palladium (II) dichloride (22 mg, 0.03 mmol) and sodium carbonate (83 mg, 0.79 mmol) were mixed in 1,4-dioxane (4 mL) and water (0.4 mL). Under nitrogen protection, the reaction mixture was heated to 100°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=19/1), the title compound was obtained (150 mg, 88%), which was in form of yellow solid. LCMS (ESI) $[M+H]^+$= 543.2.

Step 7:9-((ethylsulfonyl)methyl)-7-(4-flurophenyl)-2-methyl-2,5-dihydro-3*H*-benzo[c]pyrido[3,4-*e*]aze pin-3-ketone

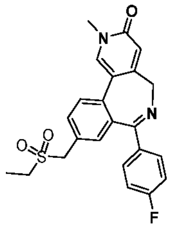

(5-(4-(ethylsulfonylmethyl)-(2-(4-fluorobenzoyl)phenyl)-1-methyl-2-oxo-1,2-dihydropy ridin-4-yl)methylamino tert-butylcarboxylate (150 mg, 0.276 mmol) was dissolved in TFA (2 mL) and dichloromethane (4 mL), then stirred at r.t. for 1 hour, the reaction mixture was concentrated, the residue was purified by preparative-HPLC, the title compound was obtainedin form of white solid (95 mg, 81%). LCMS (ESI) $[M+H]^+$= 425.1; $^1H$ NMR (400 MHz, $CD_3OD$) $\delta$ 8.16 (s, 1H). 7.76-7.74 (m. 2H). 7.54-7.50 (m, 2H). 7.40 (s, 1H). 7.16-7.12 (m, 2H). 6.64 (s, 1H). 4.76 (d, *J* = 10.4 Hz, 1H). 4.46 (s, 2H). 4.08 (d, *J* = 10.4 Hz, 1H). 3.67 (s, 3H). 3.08 (q, *J* = 7.2 Hz, 2H). 1.37 (t, *J* = 7.2 Hz, 3H).

**Embodiment 4040: (R)-10-((ethylsulfonyl)methyl)-7-(4-flurophenyl)-2-methyl-5-(trifluoromethyl)-2,5-dihydro-3 *H*-benzo[c]pyrido[3,4-e]azepin-3-ketone**

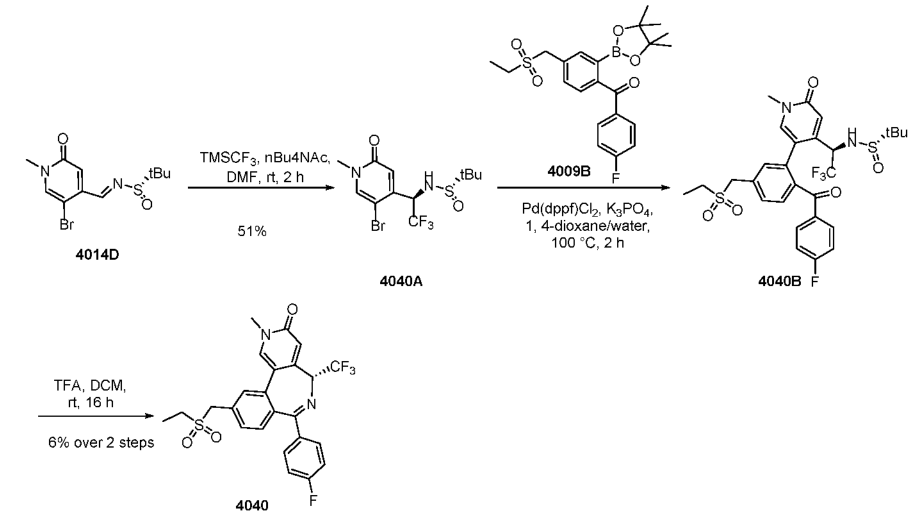


Step 1: (*S*)-*N*-((*R*)-1-(5-bromo-1-methyl-2-oxo-1,2-dihydropyridin-4-yl)-2,2,2-trifluoroethyl)-2-methylpro pane-2-sulfenamide

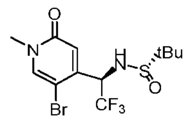

At rt, in (*S*)-*N*-((5-bromo-1-methyl-2-oxo-1,2-dihydropyridin-4-yl)methyl)-2-methylpropane-2-sulfenamid e (80 mg, 0.25 mmol) and tetrabutylammonium acetate (113 mg, 0.37 mmol) in *N,N*-dimethylformamide (4 mL) solution, (trifluoromethyl)trimethylsilane (89 mg, 0.63 mmol) was added slowly added dropwise. The reaction solution was stirred at r.t. for 2 hours, then concentrated. The residue was purified by preparative-HPLC, the title compound was obtained in form of white solid (50 mg, 51%). LCMS (ESI) $[M+H]^+$= 389.0.

Step 2: (*S*)-*N*-((*R*)-1-(5-(5-((ethylsulfonyl)methyl)-2-(4-fluorobenzoyl)-phenyl)-1-methyl-2-oxo-1,2-dihyd ropyridin-4-yl)-2,2,2-trifluoroethyl)-2-methylpropane-2-sulfenamide

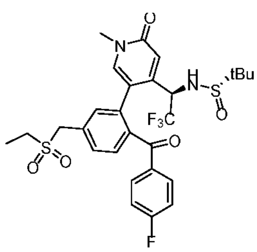

(*S*)-*N*-((*R*)-1-(5-bromo-1-methyl-2-oxo-1,2-dihydropyridin-4-yl)-2,2,2-trifluoroethyl)-2-methylpropane-2-sulfenamide (50 mg, 0.13 mmol), (4-(ethylsulfonylmethyl)-2-(4,4,5,5-tetramethyl-1,3,2-dioxoborolan-2-yl)phenyl)(4-flurophenyl)m ethanone (61 mg, 0.14 mmol), [1,1'-bis(diphenylphosphino)ferrocene]palladium (II) dichloride (10 mg, 0.013 mmol) and potassium phosphate (82 mg, 0.39 mmol) were mixed in 1,4-dioxane (5 mL) and water (1 mL). Under nitrogen protection, the reaction mixture was heated to 100°C then stirred for 2 hours. Water and EtOAc were added

into the reaction mixture, organic phase was then separated, dried over anhydrous sodium sulfate, filtered and concentrated, the residue was purified by flash chromatography (EtOAc/methanol=10/1), the title compound was obtained (100 mg, crude product), which was in form of brownish solid. LCMS (ESI) $[M+H]^+$= 615.2.

Step 3: (*R*)-10-((ethylsulfonyl)methyl)-7-(4-flurophenyl)-2-methyl-5-(trifluoromethyl)-2,5-dihydro-3*H*-be nzo[*c*]pyrido[3,4-*e*]azepin-3-ketone

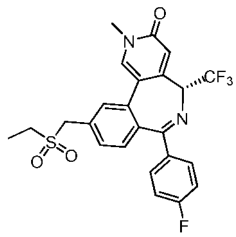

(*S*)-*N*-((*R*)-1-(5-(5-((ethylsulfonyl)methyl)-2-(4-fluorobenzoyl)phenyl)-1-methyl-2-oxo-1,2-dihydropyridin-4-yl)-2,2,2-trifluoroethyl)-2-methylpropane-2-sulfenamide (100 mg, 0.25 mmol) was dissolved in dichloromethane (4 mL) and TFA (2 mL), the reaction mixture was stirred at r.t. overnight. The reaction mixture was concentrated, the residue was purified by preparative-HPLC, the title compound was obtained in form of white solid (4 mg, yield of the two steps 6%). LCMS (ESI) $[M+H]^+$= 492.9; $^1$H NMR (400 MHz, DMSO-$d_6$) $\delta$ 8.15 (s, 1H). 7.78 (s, 1H). 7.55-7.51 (m, 3H).7.41 (d, *J* = 8.0 Hz, 1H). 7.31-7.26 (m, 2H). 6.40 (s, 1H). 4.86-4.84 (m, 1H). 4.65 (q, *J* = 13.6 Hz, 2H). 3.14 (q, *J* = 7.6 Hz, 2H). 1.26 (t, *J* = 7.2 Hz, 3H).

**Embodiment 4041:10-((ethylsulfonyl)methyl)-7-(4-flurophenyl)-2-methyl spiro[benzo[*c*]pyrido[3,4-*e*]azepin-5,1'-cyclopropane]-3(2*H*)-ketone**

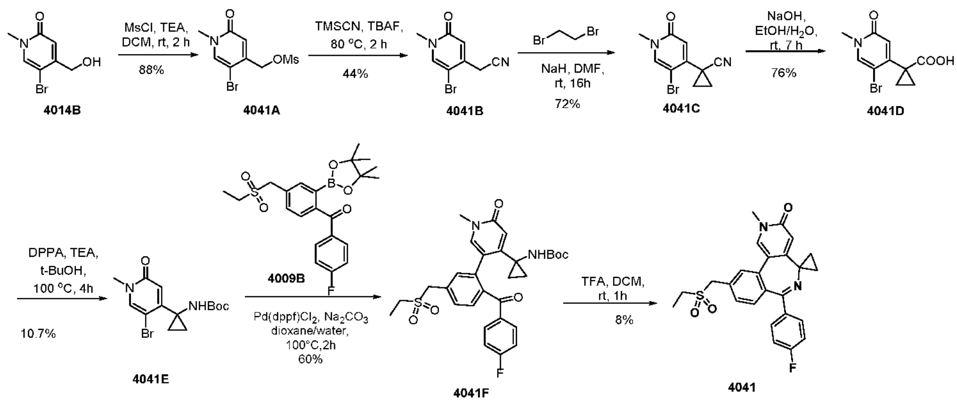


4014B 4041A 4041B 4041C 4041D

4009B 4041E 4041F 4041

Step 1: (5-bromo-1-methyl-2-oxo-1,2-dihydropyridin-4-yl)methylmethanesulfonate

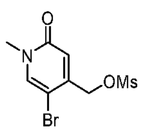

At 0°C, to 5-bromo-4-(hydroxymethyl)-1-methylpyridin-2(1*H*)-ketone (1.0 g, 4.59 mmol) and methylsulfonyl chloride (578 mg, 5.04 mmol) in dichloromethane (20 mL) solution, triethylamine (697 mg, 6.89 mmol) was added. The reaction solution was stirred at r.t. for 2 hours, then concentrated. The residue was then dissolved in EtOAc (100 mL), then washed with water and brine, dried over anhydrous sodium sulfate, filtered and concentrated, the title compound

was obtained in form of white solid (1.2 g, 88%). LCMS (ESI) $[MH]^+$ = 296.0.

Step 2:2-(5-bromo-1-methyl-2-oxo-1,2-dihydropyridin-4-yl)acetonitrile

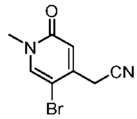

(5-bromo-1-methyl-2-oxo-1,2-dihydropyridin-4-yl)methylmethanesulfonate (1.2 g, 4.05 mmol), trimethylsilyl cyanide (804 mg, 8.1 mmol) and tetrabutylammonium fluoride (2.12 g, 8.1 mmol) in acetonitrile (30 mL) were mixed, then heated to and stirred for 2 hours, then concentrated. The residue was purified by flash chromatography (PE/EtOAc=1/10), the title compound was obtained in form of yellow solid (400 mg, 44%). LCMS (ESI) $[M+H]^+$ = 226.9.

Step 3:1-(5-bromo-1-methyl-2-oxo-1,2-dihydropyridin-4-ylcyclopropylcyanide

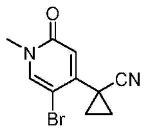

2-(5-bromo-1-methyl-2-oxo-1,2-dihydropyridin-4-yl)acetonitrile (400 mg, 1.76 mmol) and sodium hydride (60% oil dispersed liquid, 211 mg, 0.528 mmol) were mixed in *N,N*-dimethylformamide (4 mL), and stirred at r.t. for 20 minutes, then added 1, 2-dibromoethane (496 mg, 2.64 mmol), the reaction mixture was stirred at r.t. for 16 hours. The reaction was quenched by water (20 mL), and extracted by EtOAc (60 mL*2). The organic phase was combined, then washed with water and brine, dried over anhydrous sodium sulfate, filtered and concentrated, the title compound was obtained in form of yellow solid (320 mg, 72%). LCMS (ESI) $[M+H]^+$= 253.0.

Step 4:1-(5-bromo-1-methyl-2-oxo-1,2-dihydropyridin-4-yl)cyclopropylcarboxylic acid

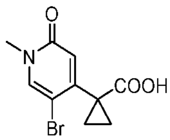

1-(5-bromo-1-methyl-2-oxo-1,2-dihydropyridin-4-ylcyclopropylcyanide (320 mg, 1.26 mmoL) and sodium hydroxide (404 mg, 10.1 mmol) were mixed in ethanol/water (2.5 mL/2.5 mL), and stirred at r.t. for 7 hours, then concentrated. The residue was dissolved in water (10 mL). and extracted by EtOAc (20 mL), the organic phase was discareded, aqueous phase was acidated by 2N HCl to pH=2, and then extracted by EtOAc (50 mL*3). The organic phase was combined, dried over anhydrous sodium sulfate, filtered and concentrated, the title compound was obtained in form of yellow solid (260 mg, 76%). LCMS (ESI) $[M+H]^+$ = 273.8.

Step 5:1-(5-bromo-1-methyl-2-oxo-1,2-dihydropyridin-4-yl)cyclopropyl tert-butylcarbamate

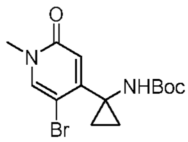

1-(5-bromo-1-methyl-2-oxo-1,2-dihydropyridin-4-yl)cyclopropylcarboxylic acid (260 mg, 0.96 mmol), dephenylphosphoryl azide (394 mg, 1.43 mmol) and triethylamine (193 mg, 1.91 mmol) were mixed in tert-butanol (10 mL), the reaction solution was heated under nitrogen protection to 100°C and stirred for 4 hours, then concentrated. The residue was purified by flash chromatography (PE/EtOAc=1/19), the title compound was obtained in form of yellow solid (35 mg, 11%). LCMS (ESI) $[M+H]^+$= 344.9.

Step 6:1-(5 -(5 -((ethylsulfonyl)methyl)-2-(4-fluorobenzoyl)phenyl)-1-methyl-2-oxo-1,2-dihydropyridin-4-yl)cyclopropyl tert-butylcarbamate

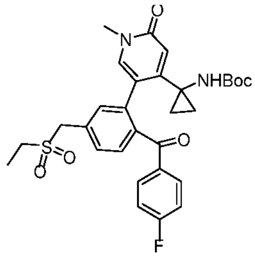

1-(5-bromo-1-methyl-2-oxo-1,2-dihydropyridin-4-yl)cyclopropyl tert-butylcarbamate (35 mg, 0.1 mmol), (4-(ethylsulfonylmethyl)-2-(4,4,5,5-tetramethyl-1,3,2-dioxoborolan-2-yl)phenyl)(4-flurophenyl)m ethanone (66 mg, 0.15 mmol), [1,1'-bis(diphenylphosphino)ferrocene]palladium (II) dichloride (7 mg, 0.01 mmol) and sodium carbonate (27 mg, 0.26 mmol) were mixed in 1,4-dioxane (3 mL) and water (0.3 mL). Under nitrogen protection, the reaction mixture was heated to 100°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=96/4), the title compound was obtained (35 mg, 60%), which was in form of yellow solid. LCMS (ESI) $[M+H]^+$ = 569.0.

Step 7:10-((ethylsulfonyl)methyl)-7-(4-flurophenyl)-2-methylspiro[benzo[*c*]pyrido[3,4-*e*]azepin-5,1'-c yclopropane]-3(2*H*)-ketone

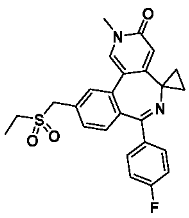

1-(5-(5-((ethylsulfonyl)methyl)-2-(4-fluorobenzoyl)phenyl)-1-methyl-2-oxo-1,2-dihydropyridin-4-yl)cyclopropyl tert-butylcarbamate (35 mg, 0.062 mmol) was dissolved in dichloromethane (2 mL) and TFA (1 mL), the reaction mixture was stirred at r.t. for 1 hour. The reaction mixture was concentrated, the residue was purified by preparative-HPLC, the title compound was obtained in form of white solid (2.1 mg, 8%). LCMS (ESI) $[M+H]^+$ = 450.7; $^1H$ NMR (400 MHz, $CD_3OD$) $\delta$ 8.12 (s, 1H). 7.76 (s, 3H). 7.53-7.48 (m, 3H). 7.30-7.27 (m, 1H). 7.17-7.13 (m, 2H). 6.61 (s, 1H). 4.59 (s, 2H). 3.69 (s, 3H). 3.21 (q, 2H). 1.43 (m, 1H). 1.41 (t, 3H). 1.27 (m, 1H). 0.60 (m, 2H).

**Embodiment 4042: (*S*)-5-cyclopropyl-10-((ethylsulfonyl)methyl)-7-(4-flurophenyl)-2-methyl-2,5-dihydro-3*H*-ben zo[*c*]pyrido[3,4-*e*]azepin-3-ketone**

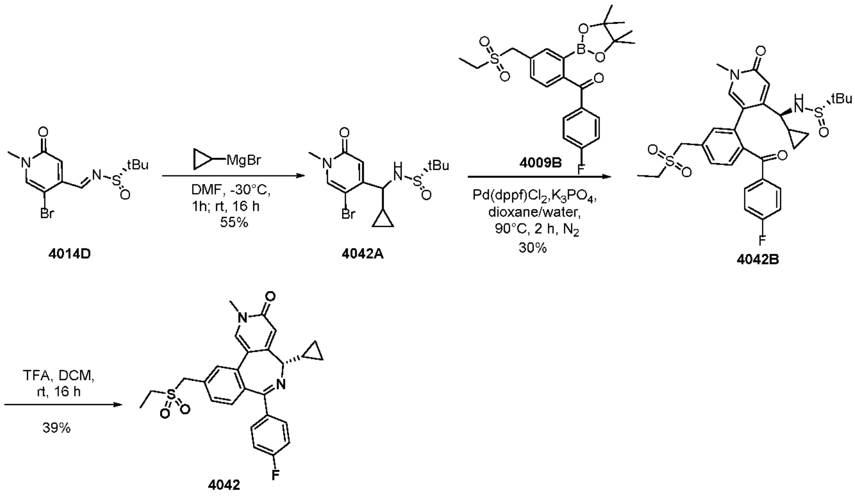


4014D 4042A 4042B 4042

Step 1: (*S*)-*N*-(1-(5-bromo-1-methyl-2-oxo-1,2-dihydropyridin-4-yl)cyclopropylmethyl)-2-methylpropane -2-sulfenamide

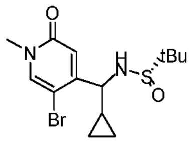

Under nitrogen protection and at -30°C, propylmagnesium bromide (1 M in THF solution, 5 mL, 5 mmol) was slowly added dropwise in (*S*)-*N*-((5-bromo-1-methyl-2-oxo-1,2-dihydropyridin-4-yl)methyl)-2-methylpropane-2-sulfenamid e (320 mg, 1.00 mmol) in *N,N*-dimethylformamide (10 mL) solution, the reaction mixture was then stirred at 30°C for 1 hour, and slowly raised the temperature to r.t. and stirred overnight. The reaction was quenched by water (50 mL), then extracted by dichloromethane (50 mL*3). Then the organic phase was combined, dried over anhydrous sodium sulfate, filtered and concentrated. The residue was purified by flash chromatography (EtOAc/methanol=97/3), the title compound was obtained (200 mg, 55%) in form of white solid. LCMS (ESI) $[M+H]^+$ = 361.0.

Step 2: (*S*)-*N*-((*S*)-cyclopropyl(5-(5-((ethylsulfonyl)methyl)-2-(4-fluorobenzoyl)phenyl)-1-methyl-2-oxo-1,2-dihydropyridin-4-yl)methyl)-2-methylpropane-2-sulfenamide

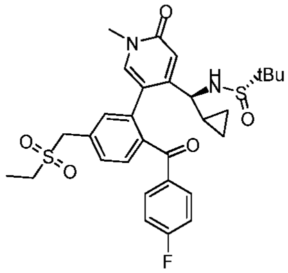

(*S*)-*N*-(1-(5-bromo-1-methyl-2-oxo-1,2-dihydropyridin-4-yl)cyclopropylmethyl)-2-methylpropane-2-sulfenamide (100 mg, 0.28 mmol), (4-(ethylsulfonylmethyl)-2-(4,4,5,5-tetramethyl-1,3,2-dioxoborolan-2-yl)phenyl)(4-flurophenyl)methanone (173 mg, 0.4 mmol), [1,1'-bis(diphenylphosphino)ferrocene]palladium (II) dichloride (20 mg, 0.028 mmol)

and potassium phosphate (178 mg, 0.84 mmol) were mixed in 1,4-dioxane (6 mL) and water (1 mL). Under nitrogen protection, the reaction mixture was heated to 90°C then stirred for 2 hours. Water and EtOAc were added into the reaction mixture, organic phase was then separated, dried over anhydrous sodium sulfate, filtered and concentrated, the residue was purified by flash chromatography (EtOAc/methanol=97/3), the title compound was obtained (50 mg, 30%), which was pale-brownish solid. LCMS (ESI) $[M+H]^+$ = 587.2.

Step 3: (*S*)-5-cyclopropyl-10-((ethylsulfonyl)methyl)-7-(4-flurophenyl)-2-methyl-2,5-dihydro-3*H*-benzo[*c* ]pyrido[3,4-*e*]azepin-3-ketone

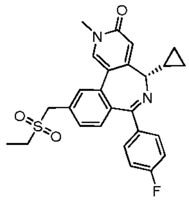

(*S*)-*N*-((*S*)-cyclopropyl(5-(5-((ethylsulfonyl)methyl)-2-(4-fluorobenzoyl)phenyl)-1-methyl-2-oxo-1,2-dihydropyridin-4-yl)methyl)-2-methylpropane-2-sulfenamide (50 mg, 0.085 mmol) was dissolved in dichloromethane (3 mL) and TFA (3 mL), the reaction mixture was stirred at r.t. overnight. The reaction mixture was concentrated, the residue was purified by preparative-HPLC, the title compound was obtained in form of white solid (15 mg, 38%). LCMS (ESI) $[M+H]^+$ = 465.2; $^1H$ NMR (400 MHz, DMSO-$d_6$) $\delta$ 8.03 (s, 1H). 7.70 (d, *J* = 1.6 Hz 1H). 7.50-7.43 (m, 3H). 7.33 (d, *J* = 8.4 Hz, 1H). 7.23 (t, *J* = 8.8 Hz, 2H). 6.73 (s, 1H). 4.60 (dd, *J* = 13.6, 35.6 Hz, 2H). 3.50 (s, 3H). 3.47 (d, *J* = 6.0 Hz 1H). 3.15 (q, *J* = 7.6, 2H). 1.60-1.52 (m, 1H). 1.26 (t, *J* = 7.2 Hz, 3H). 0.79-0.72 (m, 1H). 0.63-0.51 (m, 2H). 0.35-0.27 (m, 2H).

**Embodiment 4043: (*S*)-7-(4-flurophenyl)-2,5-dimethyl-10-((trifluoromethylsulphone)methyl)-2,5-dihydro-3*H*-benzo[*c*]pyrido[3,4-*e*]azepin-3-ketone**

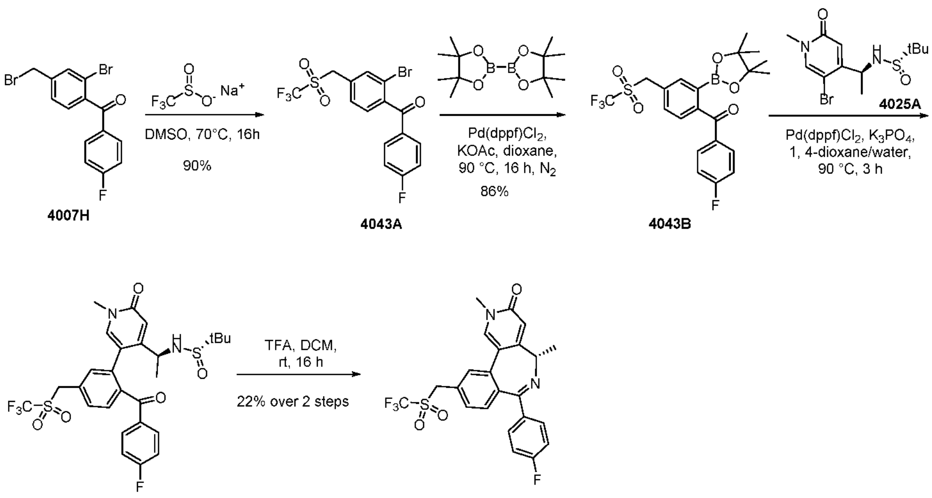


4007H 4043A 4043B 4043C 4043

Step 1: (2-bromo-4-((trifluoromethylsulphone)methyl)phenyl)(4-flurophenyl)methanone

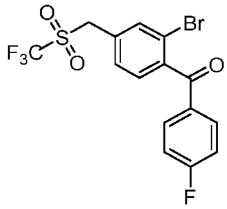

Sodium trifluoromethylsulphinate (500 mg, 3.2 mmol) was added into (2-bromo-4-(bromomethyl)phenyl)(4-flurophenyl)methanone (751 mg, 2.03 mmol) in dimethylsulfoxide solution (6 mL). The reaction mixture was heated to 70°C and stirred for 16 hours. EtOAc (50 mL) and water (150 mL) were added to layer the reaction mixtur, the organic phase was washed by saturated brine (50 mL*3), dried over anhydrous sodium sulfate, filtered and concentrated, the residue was purified by flash chromatography (EtOAc/PE=1/4), the title compound was obtainedin form of white solid (520 mg, 90%). LCMS (ESI) $[M+H]^+$ = 424.6.

Step 2: (4-flurophenyl) 2-(4,4,5,5-tetramethyl-1,3,2-dioxoborolan-2-yl)(4-(trifluoromethylsulphonemethyl)phenyl)methan one

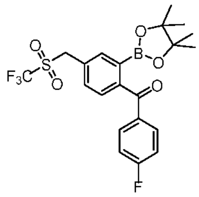

Compound (2-bromo-4-((trifluoromethylsulphone)methyl)phenyl)(4-flurophenyl)methanone (520 mg, 1.23 mmol), bis(pinacolato)diboron (629 mg, 2.46 mmol), [1,1'-bis(diphenylphosphino)ferrocene]palladium (II) dichloride (93 mg, 0.12 mmol) and potassium carbonate (241 mg, 2.46 mmol) were mixed in 1,4-dioxane (10 mL). Under nitrogen protection, the reaction mixture was heated to 90°C and stirred for 16 hours. Then filtered and the filtrate was concentrated, the residue was purified by flash chromatography (PE/EtOAc=1/4), the title compound was obtained (500 mg, 86%), which was in form of brownish solid. LCMS (ESI) [M+H]+ = 472.9.

Step 3: (*S*)-*N*-((*S*)-1-(5-(2-(4-fluorobenzoyl)-5-((trifluoromethylsulphone)methyl)phenyl)-1-methyl-2-oxo -1,2-dihydropyridin-4-yl)ethyl)-2-methylpropane-2-sulfenamide

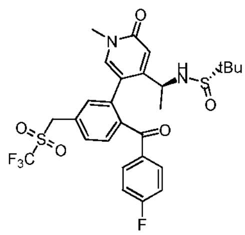

(*S*)-*N*-((*S*)-1-(5-bromo-1-methyl-2-oxo-1,2-dihydropyridin-4-yl)ethyl)-2-methylpropane -2-sulfenamide (50 mg, 0.15 mmol), (4-flurophenyl) 2-(4,4,5,5-tetramethyl-1,3,2-dioxoborolan-2-yl)(4-(trifluoromethylsulphonemethyl)phenyl)methan one (105 mg, 0.22 mmol), [1,1'-bis(diphenylphosphino)ferrocene]palladium (II) dichloride (16 mg, 0.022 mmol) and potassium phosphate (95 mg, 0.45 mmol) were mixed in 1,4-dioxane (8 mL) and water (2 mL). Under nitrogen protection, the reaction mixture was heated to 90°C then stirred for 3 hours. Water and EtOAc were added into the reaction mixture, organic phase was then separated, dried over anhydrous sodium sulfate, filtered and concentrated, the residue was purified by flash chromatography (EtOAc/methanol=10/1), the title compound was obtained (100 mg, crude product), which was in form of brownish solid. LCMS (ESI) $[M+H]^+$= 601.2.

Step 4: (*S*)-7-(4-flurophenyl)-2,5-dimethyl-10-((trifluoromethylsulphone)methyl)-2,5-dihydro-3*H*-benzo[*c* ]pyrido[3,4-*e*]azepin-3-ketone

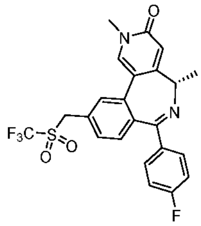

(*S*)-*N*-((*S*)-1-(5-(2-(4-fluorobenzoyl)-5-((trifluoromethylsulphone)methyl)phenyl)-1-met hyl-2-oxo-1,2-dihydropyridin-4-yl)ethyl)-2-methylpropane-2-sulfenamide (100 mg, 0.16 mmol) was dissolved in dichloromethane (4 mL) and TFA (2 mL), the reaction mixture was stirred at r.t. overnight. The reaction mixture was concentrated, the residue was purified by preparative-HPLC, the title compound was obtained in form of yellow solid (16.3 mg, yield of the two steps 22%). LCMS (ESI) $[M+H]^+$= 479.2; $^1H$ NMR (400 MHz, DMSO-$d_6$) $\delta$ 7.98 (s, 1H). 7.82 (s, 1H). 7.57 (dd, *J* = 2.0, 8.0 Hz, 1H). 7.48-7.40 (m, 3H). 7.24 (t, d, *J* = 8.8 Hz, 1H). 6.42 (s, 1H). 5.43 (d, *J* = 13.6 Hz, 1H). 5.32 (d, *J* = 13.6 Hz, 1H). 3.94 (q, *J* = 7.6 Hz, 1H). 3.50 (s, 3H). 1.65 (d, *J* = 6.4 Hz, 3H).

**Embodiment 4044: (*S*)-10-((isopropylsulfone)methyl)-7-(4-flurophenyl)-2,5-dimethyl-2,5-dihydro-3*H*-benzo[*c*]pyrido[3,4-*e*]azepin-3-ketone**

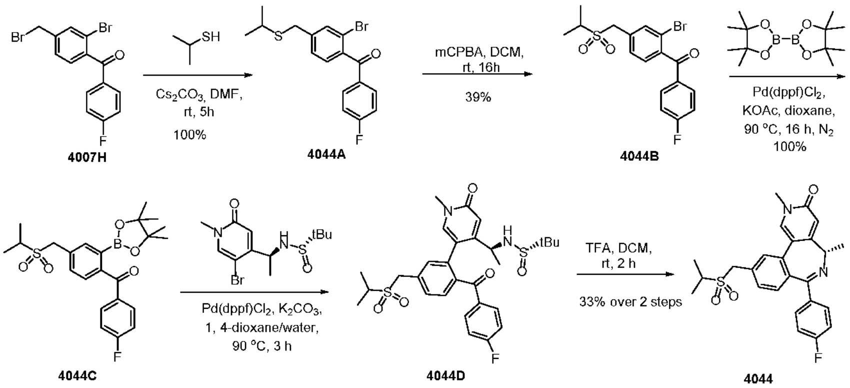


Step 1 : (2-bromo-4-(isopropylthiomethyl)phenyl)(4-flurophenyl)methanone

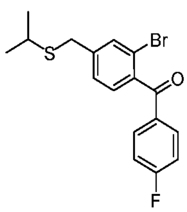

(2-bromo-4-(bromomethyl)phenyl)(4-flurophenyl)methanone (520 mg, 1.41 mmol), barium carbonate (919 mg, 2.82 mmol) and 2-propyl mercaptan (160 mg, 2.11 mmol) were mixed in N,N-dimethylformamide (6 mL), then stirred at r.t. for 5 hours. EtOAc (50 mL) and water (150 mL) were added into the reaction mixture, then the organic phase was combined, and washed by brine (50 mL*3). Dried over anhydrous sodium sulfate, filtered and concentrated, the title compound was obtained in form of brownish solid (520 mg, 100%). and was used directly in the next step without

purification. LCMS (ESI) $[M+H]^+$ = 367.0.

Step 2: (2-bromo-4-(isopropylsulfonemethyl)phenyl)(4-flurophenyl)methanone

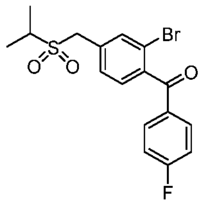

At 0°C, metachloroperbenzoic acid (1.0 g, 4.97 mmol) was added into (2-bromo-4-(isopropylthiomethyl)phenyl)(4-flurophenyl)methanone (520 mg, 1.42 mmol) in dichloromethane solution (20 mL). The reaction mixture was stirred at r.t. overnight, then concentrated. The residue was purified by flash chromatography (PE/EtOAc=1/1), the title compound was obtained in form of white solid (220 mg, 39%). LCMS (ESI) $[M+H]^+$ = 399.8.

Step 3: (4-(isopropylsulfonylmethyl)-2-(4,4,5,5-tetramethyl-1,3,2-dioxaborolan-2-yl)phenyl)(4-flurophen yl)methanone

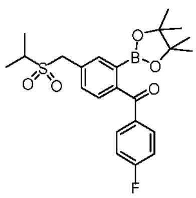

Compound (2-bromo-4-(isopropylsulfonemethyl)phenyl)(4-flurophenyl)methanone (220 mg, 0.55 mmol), bis(pinacolato)diboron (283 mg, 1.10 mmol), [1,1'-bis(diphenylphosphino)ferrocene]palladium (II) dichloride (41 mg, 0.055 mmol) and potassium acetate (108 mg, 1.10 mmol) were mixed in 1,4-dioxane (10 mL). Under nitrogen protection, the reaction mixture was heated to 90°C and stirred for 16 hours. Then filtered and the filtrate was concentrated, the residue was purified by flash chromatography (PE/EtOAc=4/1), the title compound was obtained (260 mg, 100%), which was in form of white solid. LCMS (ESI) $[M+H]^+$ = 447.2.

Step 4: (*S*)-*N*-((*S*)-1-(5-(5-((isopropylsulfonyl)methyl)-2-(4-fluorobenzoyl)phenyl)-1-methyl-2-oxo-1,2-di hydropyridin-4-yl)ethyl)-2-methylpropane-2-sulfenamide

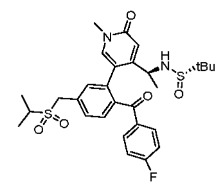

(*S*)-*N*-((*S*)-1-(5-bromo-1-methyl-2-oxo-1,2-dihydropyridin-4-yl)ethyl)-2-methylpropane -2-sulfenamide (50 mg, 0.15 mmol), (4-(isopropylsulfonylmethyl)-2-(4,4,5,5-tetramethyl-1,3,2-dioxaborolan-2-yl)phenyl)(4-flurophen yl)methanone (133 mg, 0.30 mmol), [1,1'-bis(diphenylphosphino)ferrocene]palladium (II) dichloride (11 mg, 0.015 mmol) and potassium phosphate (95 mg, 0.45 mmol) were mixed in 1,4-dioxane (8 mL) and water (2 mL). Under nitrogen protection, the reaction mixture was heated to 90°C then stirred for 3 hours. Water and EtOAc were added into the reaction mixture, organic phase was then separated, dried over anhydrous sodium sulfate, filtered and concentrated, the residue was purified by flash chromatography (EtOAc/methanol=10/1), the title compound was obtained (100 mg, crude product), brownish solid. LCMS (ESI) $[M+H]^+$= 575.2.

Step 4: (*S*)-10-((isopropylsulfone)methyl)-7-(4-flurophenyl)-2,5-dimethyl-2,5-dihydro-3*H*-benzo[*c*]pyrido [3,4-*e*]azepin-3-ketone

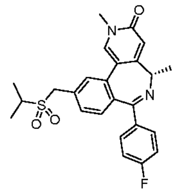

(*S*)-*N*-((1*S*)-1-(5-(5-((isopropylsulfonyl)methyl)-2-(4-fluorobenzoyl)phenyl)-1-methyl-2 -oxo-1,2-dihydropyridin-4-yl)ethyl)-2-methylpropane-2-sulfenamide (100 mg, 0.17 mmol) was dissolved in dichloromethane (4 mL) and TFA (2 mL), the reaction mixture was stirred at r.t. overnight. The reaction mixture was concentrated, the residue was purified by preparative-HPLC, the title compound was obtained in form of white solid (23.3 mg, yield of the two steps 33%). LCMS (ESI) $[M+H]^+$= 453.2; $^1H$ NMR (400 MHz, DMSO-$d_6$) $\delta$ 8.01 (s, 1H). 7.82 (d, *J* = 1.6 Hz, 1H). 7.49-7.45 (m, 3H). 7.33 (d, *J* = 8.0 Hz, 1H). 7.25-7.20 (m, 2H). 6.41 (s, 1H). 4.64 (d, *J* = 13.6 Hz, 1H). 4.53 (d, *J* = 13.6 Hz, 1H). 3.95 (q, *J* = 9.6 Hz, 1H). 3.50 (s, 3H). 3.32-3.27 (m, 1H). 1.65 (d, *J* = 6.4 Hz, 3H). 1.33 (d, *J* = 3.2 Hz, 3H). 1.31 (d, *J* = 3.2 Hz, 3H).

**Embodiment 4045: (*S*)-7-(4-flurophenyl)-2,5-dimethyl-10-((cyclopropylsulfone)methyl)-2,5-dihydro-3*H*-benzo[*c*] pyrido[3,4-*e*]azepin-3-ketone**

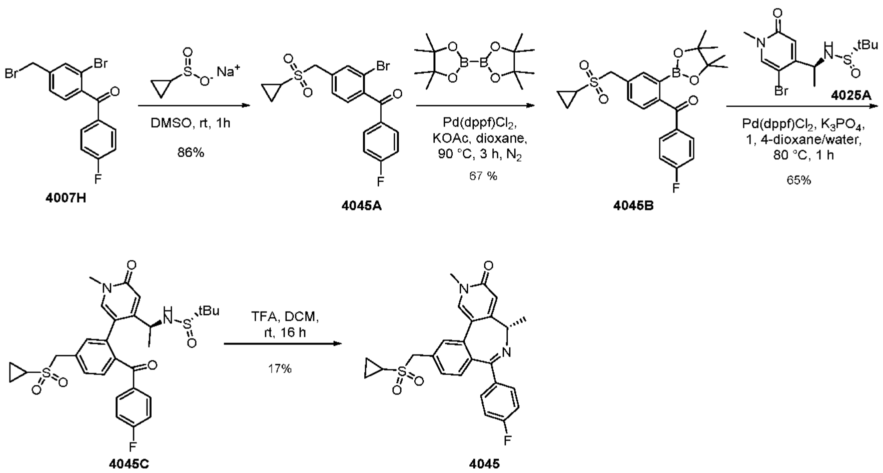


4007H 4045A 4045B 4045C 4045

Step 1: (2-bromo-4-((cyclopropylsulfone)methyl)phenyl)(4-flurophenyl)methanone

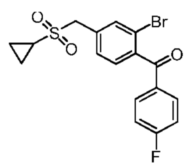

Sodium cylclopropyl fulfinate (517 mg, 4.03 mmol) was added into compound (2-bromo-4-(bromomethyl)phenyl)(4-flurophenyl)methanone (1.0 g, 2.69 mmol) in dimethylsulfoxide (10 mL) solution. The reaction mixture was then stirred for 1 hour at rt. EtOAc (30 mL) and water (100 mL) was added to layer the reaction mixture, the organic phase was washed by saturated brine (50 mL*3), dried over anhydrous sodium sulfate, filtered and concentrated, the title compound was obtainedin form of white solid (923 mg, 86%). LCMS (ESI) $[M+H]^+$= 414.0.

Step 2:

(4-(cyclopropylsulfonemethyl)-2-(4,4,5,5-tetramethyl-1,3,2-dioxoborolan-2-yl)phenyl)(4-flurophe nyl)methanone

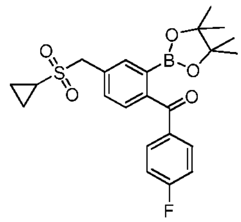

Compound (2-bromo-4-((cyclopropylsulfone)methyl)phenyl)(4-flurophenyl)methanone (100 mg, 0.25 mmol), bis(pinacolato)diboron (96 mg, 0.38 mmol), [1,1'-bis(diphenylphosphino)ferrocene]palladium (II) dichloride (37 mg, 0.05 mmol) and potassium acetate (74 mg, 0.75 mmol) were mixed in 1,4-dioxane (6 mL). Under nitrogen protection, the reaction mixture was heated to 90°C then stirred for 3 hours. filtered and the filtrate was concentrated, the residue was purified by flash chromatography (PE/EtOAc=8/1), the title compound was obtained (74 mg, 67%), which was in form of yellow solid. LCMS (ESI) $[M+H]^+$= 418.1.

Step 3: (*S*)-*N*-((*S*)-1-(5-(2-(4-fluorobenzoyl)-5-((cyclopropylsulfone)methyl)phenyl)-1-methyl-2-oxo-1,2-dihydropyridin-4-yl)ethyl)-2-methylpropane-2-sulfenamide

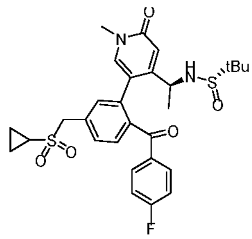

(4-flurophenyl)-2-(4,4,5,5-tetramethyl-1,3,2-dioxoborolan-2-yl)(4-(cyclopropylsulfonemethyl)phenyl)methanone (54 mg, 0.12 mmol), (*S*)-*N*-((*S*)-1-(5-bromo-1-methyl-2-oxo-1,2-dihydropyridin-4-yl)ethyl)-2-methylpropane-2-sulfena mide (27 mg, 0.08 mmol), [1,1'-bis(diphenylphosphino)ferrocene]palladium (II) dichloride (12 mg, 0.016 mmol) and potassium phosphate (51 mg, 0.24 mmol) were mixed in 1,4-dioxane (2 mL) and water (0.2 mL). Under nitrogen protection, the reaction mixture was heated to then stirred for 1 hour. Water and EtOAc were added into the reaction mixture, organic phase was then separated, dried over anhydrous sodium sulfate, filtered and concentrated, the residue was purified by flash chromatography (PE/EtOAc=3/1), the title compound was obtained (30 mg, 65%), which was in form of yellow solid. LCMS (ESI) $[M+H]^+$ = 573.2.

Step 4: (*S*)-7-(4-flurophenyl)-2,5-dimethyl-10-((cyclopropylsulfone)methyl)-2,5-dihydro-3*H*-benzo[*c*]pyrido[3,4-*e*]azepin-3-ketone

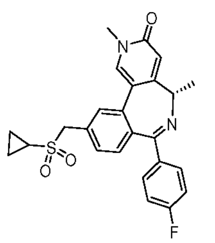

(*S*)-*N*-((*S*)-1-(5-(2-(4-fluorobenzoyl)-5-((cyclopropylsulfone)methyl)phenyl)-1-methyl-2 -oxo-1,2-dihydropyridin-4-yl)ethyl)-2-methylpropane-2-sulfenamide (14 mg, 0.024 mmol) was dissolved in dichloromethane (2 mL) and TFA (1 mL), the reaction mixture was stirred at r.t. overnight. The reaction mixture was concentrated, the residue was purified by preparative-HPLC, the title compound was obtained in form of yellow solid (1.9 mg, 17%). LCMS (ESI): $R_T$ (min) = 4.127, $[M+H]^+$ = 451.2; $^1H$ NMR (400 MHz, DMSO-$d_6$) $\delta$ 8.00 (s, 1H). 7.73 (s, 1H). 7.51-7.45 (m, 3H). 7.33-7.31 (d, *J* = 7.2 Hz, 1H). 7.25-7.20 (m, 2H). 6.41 (s, 1H). 4.73-4.58 (m, 2H). 3.96-3.94 (d, *J* = 6.4 Hz, 1H). 3.50 (s, 3H). 2.68 (s, 1H). 1.68-1.64 (d, *J* = 14.4 Hz, 3H). 1.01-0.87 (m, 4H).

**Embodiment 4046: (*S*)-7-(2,4-dichlorophenyl)-10-((ethylsulfonyl)methyl)-2,5-dimethyl-2,5-dihydro-3*H*-benzo[*c*] pyrido[3,4-*e*]azepin-3-ketone**

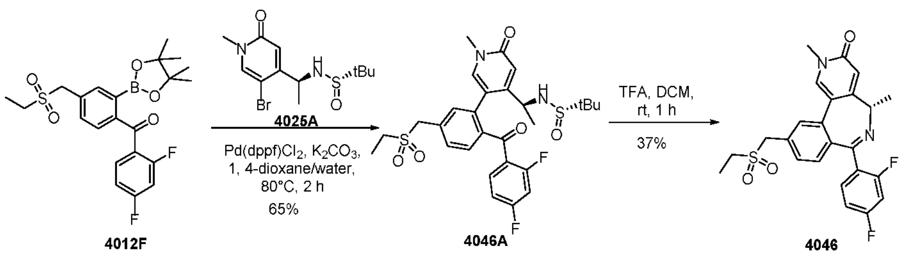


Step 1: (*S*)-*N*-((*S*)-1-(5-(2-(2,4-dichlorobenzoyl)-5-((ethylsulfonyl)methyl)phenyl)-1-methyl-2-oxo-1,2-di hydropyridin-4-yl)ethyl)-2-methylpropane-2-sulfenamide

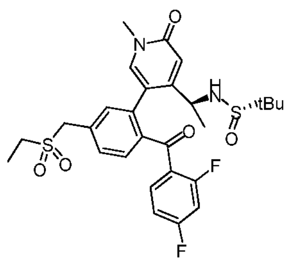

(*S*)-*N*-((*S*)-(5-bromo-1-methyl-2-oxo-1,2-dihydropyridin-4-yl)ethyl)-2-methylpropane-2 -sulfenamide (100 mg, 0.28 mmol), (4-(ethylsulfonylmethyl)-2-(4,4,5,5-tetramethyl-1,3,2-dioxoborolan-2-yl)phenyl)(2,4-dichlorophen yl)methanone (188 mg, 0.42 mmol), [1,1'-bis(diphenylphosphino)ferrocene]palladium (II) dichloride (21 mg, 0.028 mmol) and potassium phosphate (181 mg, 0.84 mmol) were mixed in 1,4-dioxane (4 mL) and water (0.4 mL). Under nitrogen protection, the reaction mixture was heated to 90°C then stirred for 2 hours. Water and EtOAc were added into the reaction mixture, organic phase was then separated, dried over anhydrous sodium sulfate, filtered and concentrated, the residue was purified by flash chromatography (EtOAc/methanol= 30/1), the title compound was obtained, in form of yellow solid (110 mg, 65%). LCMS (ESI) $[M+H]^+$= 578.0.

Step 2: (*S*)-7-(2,4-dichlorophenyl)-10-((ethylsulfonyl)methyl)-2,5-dimethyl-2,5-dihydro-3*H*-benzo[*c*]pyri do[3,4-*e*]azepin-3-ketone

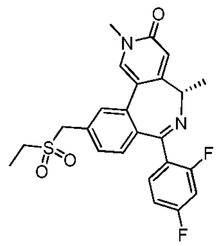

(*S*)-*N*-((*S*)-1-(5-(2-(2,4-dichlorobenzoyl)-5-((ethylsulfonyl)methyl)phenyl)-1-methyl-2-o xo-1,2-dihydropyridin-4-yl)ethyl)-2-methylpropane-2-sulfenamide (110 mg, 0.19 mmol) was dissolved in dichloromethane (4 mL) and TFA (1 mL), the reaction mixture was stirred at r.t. for 1 hour. The reaction mixture was concentrated, the residue was purified by preparative-HPLC, the title compound was obtained in form of white solid (32.2 mg, 37%). LCMS (ESI): [M+H]+ = 456.0; $^1$H NMR (400 MHz, DMSO-$d_6$) $\delta$ 8.08 (s, 1H). 7.70-7.69 (d, *J* = 0.16 Hz, 1H). 7.65-7.59 (m, 1H). 7.45-7.42 (m, 1H). 7.25-7.16 (m, 3H). 6.42 (s, 1H). 4.64-4.51 (m, 2H). 3.99-3.96 (m, 1H). 3.52 (s, 3H). 3.13-3.07 (m, 2H). 1.66-1.64 (d, *J* = 6.4 Hz, 3H). 1.25-1.21 (m, 3H).

**Embodiment 4047: (*S*)-5-ethyl-10-((ethylsulfonyl)methyl)-7-(4-chlorophenyl)-2-methyl-2,5-dihydro-3*H*-benzo[*c*]pyrido[3,4-*e*]azepin-3-ketone**

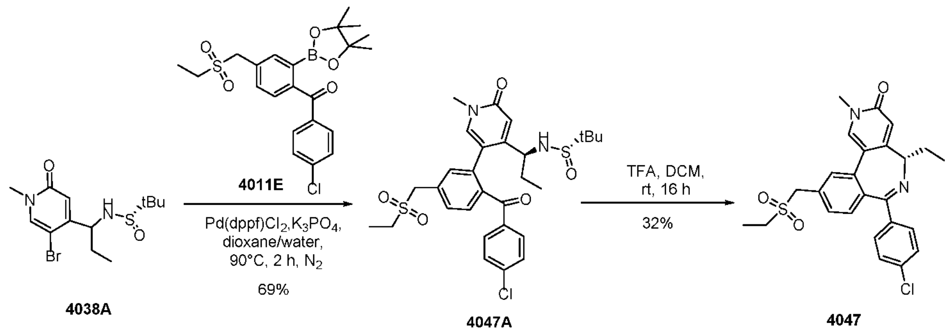


4038A 4047A 4047

Step 1: (*S*)-*N*-((*S*)-1-(5-(5-((ethylsulfonyl)methyl)-2-(4-chlorobenzoyl)phenyl)-1-methyl-2-oxo-1,2-dihydr opyridin-4-yl)propyl)-2-methylpropane-2-sulfenamide

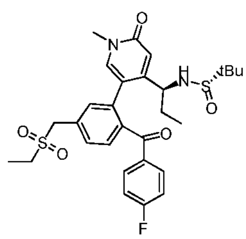

(*S*)-*N*-(1-(5-bromo-1-methyl-2-oxo-1,2-dihydropyridin-4-yl)propyl)-2-methylpropane-2-sulfenamide (70 mg, 0.2 mmol), (4-(ethylsulfonylmethyl)-2-(4,4,5,5-tetramethyl-1,3,2-dioxoborolan-2-yl)phenyl)(4-chlorophenyl) methanone (135 mg, 0.3 mmol), [1,1'-bis(diphenylphosphino)ferrocene]palladium (II) dichloride (29 mg, 0.04 mmol) and potassium phosphate (127 mg, 0.6 mmol) were mixed in 1,4-dioxane (3 mL) and water (0.6 mL). Under nitrogen protection, the reaction mixture was heated to then stirred for 1 hour. Water and EtOAc were added into the reaction mixture, organic phase was then separated, dried over anhydrous sodium sulfate, filtered and concentrated, the residue was purified by flash chromatography (PE/EtOAc=3/1), the title compound was obtained (81 mg, 69%, *ee* = 91%), which was in form of yellow solid. LCMS (ESI) [M+H]$^+$ = 590.6.

Step 2: (*S*)-5-ethyl-10-((ethylsulfonyl)methyl)-7-(4-chlorophenyl)-2-methyl-2,5-dihydro-3*H*-benzo[*c*]pyri do[3,4-*e*]azepin-3-ketone

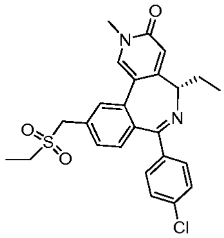

(*S*)-*N*-((*S*)-1-(5-(5-((ethylsulfonyl)methyl)-2-(4-chlorobenzoyl)phenyl)-1-methyl-2-oxo-1,2-dihydropyridin-4-yl)propyl)-2-methylpropane-2-sulfenamide (81 mg, 0.14 mmol) was dissolved in dichloromethane (2 mL) and TFA (1 mL), the reaction mixture was stirred at r.t. overnight. The reaction mixture was concentrated, the residue was purified by preparative-HPLC, the title compound was obtained in form of white solid (21.3 mg, 32%). LCMS (ESI): $R_T$ (min) = 4.49, $[M+H]^+$ = 469.2 ; $^1H$ NMR (400 MHz, DMSO-$d_6$) $\delta$ 8.03 (s, 1H). 7.72 (s, 1H). 7.50-7.43 (m, 5H). 7.35-7.33 (d, *J* = 8.0 Hz, 1H). 6.39 (s, 1H). 4.67-4.54 (m, 2H). 3.63-3.59 (m, 1H). 3.50 (s, 3H). 3.18-3.12 (q, 2H). 2.21-2.19 (m, 1H). 2.02-1.99 (m, 1H). 1.29-1.25 (t, 3H). 1.02-0.99 (t, 3H)

**Embodiment 4048: (5*S*)-10-((ethylsulfoxide)methyl)-7-(4-flurophenyl)-2,5-dimethyl-2,5-dihydro-3*H*-benzo[*c*]pyr ido[3,4-*e*]azepin-3-ketone**

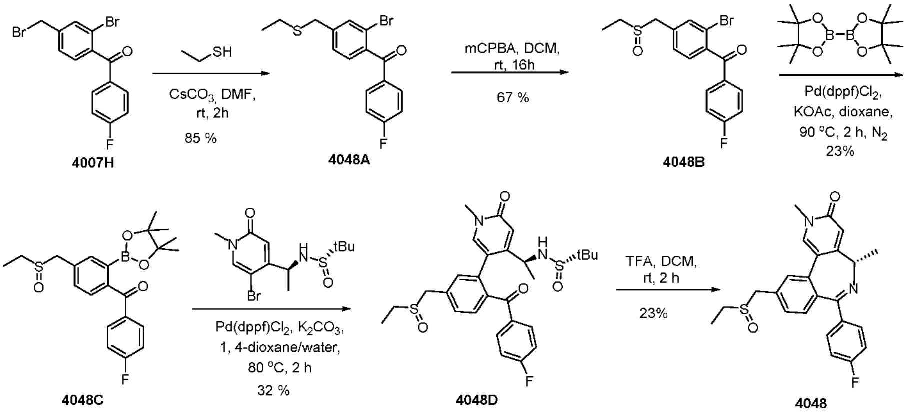


Step 1 : (2-bromo-4-(ethylsulfomethyl)phenyl)(4-flurophenyl)methanone

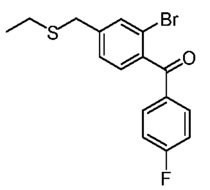

(2-bromo-4-(bromomethyl)phenyl)(4-flurophenyl)methanone (744 mg, 2 mmol). barium carbonate (1.96 g, 6 mmol) and ethanethiol (149 mg, 2.4 mmol) were mixture in *N,N*-dimethylformamide (10 mL), and stirred for 2 hours at rt. EtOAc (20 mL) and water (50 mL) was added into the reaction solution, then the organic phase was combined, washed by brine (50 mL*3). Dried over anhydrous sodium sulfate, filtered and concentrated, the tiltle compound was obtained in form of yellow oil (600 mg, 85%), and was used directly in the next step without purification. LCMS (ESI) $[M+H]^+$ =

353.0, 355.0.

Step 2: (2-bromo-4-(ethylsulfoxidemethyl)phenyl)(4-flurophenyl)methanone

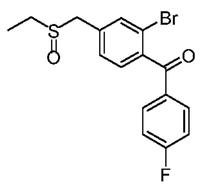

At 0°C, metachloroperbenzoic acid (294 mg, 1.7 mmol) was added into (2-bromo-4-(ethylsulfomethyl)phenyl)(4-flurophenyl)methanone (600 mg, 1.7 mmol) in dichloromethane solution (10 mL). The reaction mixture was stirred at r.t. overnight, then concentrated. The residue was purified by flash chromatography (PE/EtOAc=8/1), the tiltle compound was obtained in form of brownish solid (420 mg, 67%). LCMS (ESI) $[M+H]^+$ = 368.8, 370.8.

Step 3: (4-(ethylsulfoxidemethyl)-2-(4,4,5,5-tetramethyl-1,3,2-dioxaborolan-2-yl)phenyl)(4-flurophenyl) methanone

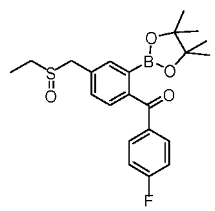

compound (2-bromo-4-(ethylsulfoxidemethyl)phenyl)(4-flurophenyl)methanone (420 mg, 1.14 mmol), bis(pinacolato)diboron (580 mg, 2.28 mmol), [1,1'-bis(diphenylphosphino)ferrocene]palladium (II) dichloride (80 mg, 0.11 mmol) and potassium carbonate (335 mg, 3.42 mmol) were mixed in 1,4-dioxane (5 mL). Under nitrogen protection, the reaction mixture was heated to 90°C then stirred for 2 hours. Then filtered and the filtrate was concentrated, the residue was purified by flash chromatography (PE/EtOAc=3/1), the title compound was obtained (110 mg, 23%), which was in form of yellow solid. LCMS (ESI) $[M+H]^+$= 417.0.

Step 4: (*S*)-*N*-((1*S*)-1-(5-(5-((ethylsulfoxide)methyl)-2-(4-fluorobenzoyl)phenyl)-1-methyl-2-oxo-1,2-dihydropyridin-4-yl)ethyl)-2-methylpropane-2-sulfenamide

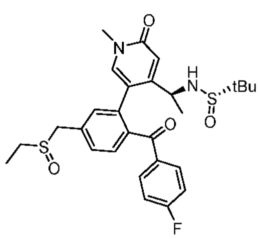

(4-(ethylsulfoxidemethyl)-2-(4,4,5,5-tetramethyl-1,3,2-dioxaborolan-2-yl)phenyl)(4-flurophenyl)methanone (110 mg, 0.264 mmol), (*S*)-*N*-((S)-1-(5-bromo-1-methyl-2-oxo-1,2-dihydropyridin-4-yl)ethyl)-2-methylpropane-2-sulfena mide (44 mg, 0.132 mmol), [1,1'-bis(diphenylphosphino)ferrocene]palladium (II) dichloride (10 mg, 0.0132 mmol) and potassium carbonate (55 mg, 0.396 mmol) were mixed in 1,4-dioxane (4 mL) and water (0.4 mL). Under nitrogen protection, the reaction mixture was heated to then stirred for 2 hours. Water and EtOAc were added into the reaction mixture, organic phase was then separated, dried over anhydrous sodium sulfate, filtered and concentrated, the residue was purified by flash chromatography (EtOAc/methanol=30/1), the title compound was obtained (23 mg, 32%), which

was in form of yellow solid. LCMS (ESI) [M+H]+ = 544.7.

Step 5: (5*S*)-10-((ethylsulfoxide)methyl)-7-(4-flurophenyl)-2,5-dimethyl-2,5-dihydro-3*H*-benzo[*c*]pyrido[ 3,4-*e*]azepin-3-ketone

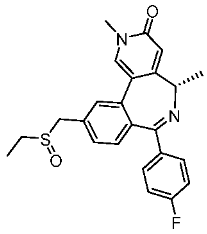

(*S*)-*N*-((1*S*)-1-(5-(5-((ethylsulfoxide)methyl)-2-(4-fluorobenzoyl)phenyl)-1-methyl-2-ox o-1,2-dihydropyridin-4-yl)ethyl)-2-methylpropane-2-sulfenamide (23 mg, 0.042 mmol) was dissolved in dichloromethane (4 mL) and TFA (1 mL), the reaction solution was stirred at r.t. for 2 hours. The reaction mixture was concentrated, the residue was purified by preparative-HPLC, the title compound was obtained in form of white solid (4 mg, yield of the two steps 22%). LCMS (ESI): [M+H]+ = 422.8; $^1$H NMR (400 MHz, DMSO-$d_6$) $\delta$ 8.04 (d, *J* = 14.8 Hz, 1H). 7.63 (d, *J* = 2.4 Hz, 1H). 7.49 (d, *J* = 5.6 Hz, 2H). 7.40 (dd, *J* = 1.6 Hz, 8.4 Hz, 1H). 7.31 (d, *J* = 7.6 Hz, 1H). 7.23 (t, *J* = 8.8 Hz, 2H). 6.40 (s, 1H). 4.27-4.05 (m, 2H). 3.94 (q, *J* = 6.0, 12.4 Hz, 1H). 3.50 (s, 3H). 2.90-2.60 (m, 2H). 1.66 (d, *J* = 6.8 Hz, 3H). 1.21 (t, *J* = 7.6, 14.0 Hz, 3H).

**Embodiment 4049: (*S*)-*N*-(7-(4-flurophenyl)-2,5-dimethyl-3-oxo-3,5-dihydro-2*H*-benzo[*c*]pyrido[3,4-*e*]azepin-10-yl)ethylsulfonamide**

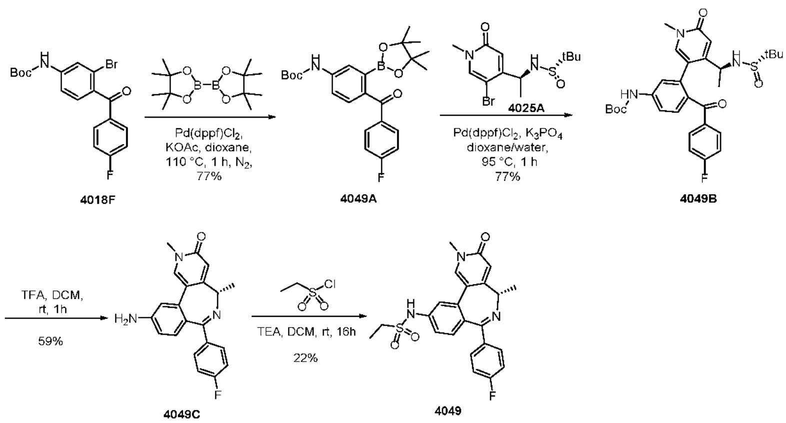


4018F 4049A 4049B 4049C 4049

Step 1: (4-(4-fluorobenzoyl)-3-(4,4,5,5-tetramethyl-1,3,2-dioxaborolan-2-yl)phenyl) tert-butylcarbamate

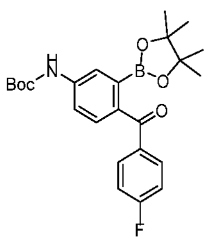

Compound (3-bromo-4-(4-fluorobenzoyl)phenyl) tert-butylcarbamate (1.7 g, 4.33 mmol), bis(pinacolato)diboron

(2.2 g, 8.66 mmol), [1,1'-bis(diphenylphosphino)ferrocene]palladium (II) dichloride (317 mg, 0.433 mmol) and potassium acetate (1.27 g, 12.99 mmol) were mixed in 1,4-dioxane (6 mL), heated to 110°C under nitrogen protection then stirred for 1 hour. The reaction mixture was filtered and the filtrate was concentrated, the residue was purified by flash chromatography (PE/EtOAc=8/1), the tiltle compound was obtained (1.47 g, 77%), which was in form of yellow solid. LCMS (ESI) $[M+H]^+$ = 442.0

Step 2: (3-(4-(1-(((*S*)-tert-butyl-sulfinyl)amino)ethyl)-1-methyl-6-oxo-1,6-dihydropyridin-3-yl)-4-(4-fluor obenzenesulfonyl)phenyl)tert-butlcarbamate

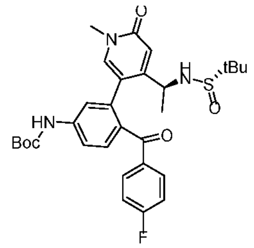

(*S*)-*N*-((*S*)-1-(5-bromo-1-methyl-2-oxo-1,2-dihydropyridin-4-yl)ethyl)-2-methylpropane -2-sulfenamide (190 mg, 0.57 mmol), (4-(4-fluorobenzoyl)-3-(4,4,5,5-tetramethyl-1,3,2-dioxaborolan-2-yl)phenyl) tert-butylcarbamate (500 mg, 1.14 mmol), [1,1'-bis(diphenylphosphino)ferrocene]palladium (II) dichloride (42 mg, 0.057 mmol) and potassium phosphate (236 mg, 1.71 mmol) were mixed in 1,4-dioxane (6 mL) and water (0.6 mL), then heated to 95°C under nitrogen protection then stirred for 1 hour. The reaction mixture was filtered and the filtrate was concentrated, the residue was purified by flash chromatography (EtOAc/methanol=30/1). The title compound was obtained (251 mg, 77%), which was in form of yellow solid. LCMS (ESI) $[M+H]^+$=569.8.

Step 3: (*S*)-10-amino-7-(4-flurophenyl)-2,5-dimethyl-2,5-dihydro-3*H*-benzo[*c*]pyrido[3,4-*e*]azepin-3-keto ne

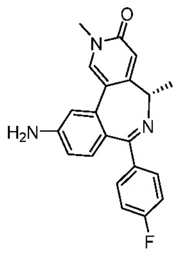

TFA (2 mL) was added into 3-(4-(1-(((*S*)-tert-butyl-sulfinyl)amino)ethyl)-1-methyl-6-oxo-1,6-dihydropyridin-3-yl)-4-(4-fluoro benzoyl)phenyl) tert-butylcarbamate (251 mg, 0.44 mmol) in dichloromethane (8 mL) solution, then stirred at r.t. for 1 hour. After concentration, the residue was purified by flash chromatography (EtOAc/methanol=10/1). The title compound was obtained (90 mg, 59%), which was in form of yellow solid. LCMS (ESI) $[M+H]^+$=347.8.

Step 4: (*S*)-*N*-(7-(4-flurophenyl)-2,5-dimethyl-3-oxo-3,5-dihydro-2*H*-benzo[*c*]pyrido[3,4-*e*]azepin-10-yl)e thylsulfonamide

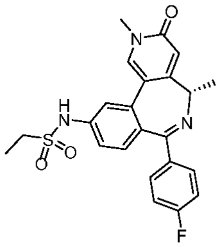

Ethylsulfonyl chloride (33 mg, 0.258 mmol) was added into (*S*)-10-amino-7-(4-flurophenyl)-2,5-dimethyl-2,5-dihydro-3*H*-benzo[*c*]pyrido[3,4-*e*]azepin-3-keto ne (30 mg, 0.086 mmol) and triethylamine (87 mg, 0.86 mmol) in dichloromethane (5 mL) solution, the reaction mixture was stirred at r.t. overnight. The reaction mixture then washed with water and brine, dried (anhydrous sodium sulfate), filtered and concentrated. The residue was purified by preparative-HPLC, the title compound was obtained (8.4 mg, 22%), which was in form of white solid. LCMS (ESI) $[M+H]^+$ = 439.8; $^1H$ NMR (400 MHz, DMSO-$d_6$) $\delta$ 8.00 (s, 1H). 7.49-7.46 (m, 2H). 7.34 (s, 1H). 7.23-7.18 (m, 4H). 6.38 (s, 1H). 3.96-3.94 (m, 1H). 3.49 (s, 3H). 3.30-3.21 (m, 2H). 1.64-1.62 (d, *J* = 6.4 Hz, 3H). 1.26-1.23 (d, *J* = 7.2 Hz, 3H).

**Embodiment 4050: (*S*)-*N*-(7-(4-flurophenyl)-2,5-dimethyl-3-oxo-3,5-dihydro-2*H*-benzo[*c*]pyrido[3,4-*e*]azepin-10-yl)methylamine sulfonamide**

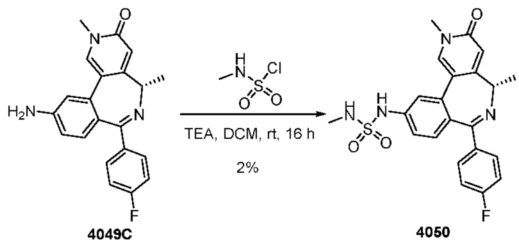


4049C 4050

Step 1: (*S*)-*N*-(7-(4-flurophenyl)-2,5-dimethyl-3-oxo-3,5-dihydro-2*H*-benzo[*c*]pyrido[3,4-*e*]azepin-10-yl) methylamine sulfonamide

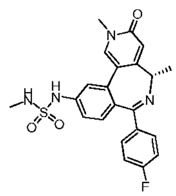

N-methylaminosulfonyl chloride (34 mg, 0.258 mmol) was added into (*S*)-10-amino-7-(4-flurophenyl)-2,5-dimethyl-2,5-dihydro-3*H*-benzo[*c*]pyrido[3,4-*e*]azepin-3-keto ne (30 mg, 0.086 mmol) and triethylamine (87 mg, 0.86 mmol) in dichloromethane (5 mL) solution, the reaction mixture was stirred at r.t. overnight. The reaction mixture then washed with water and brine, dried (anhydrous sodium sulfate), filtered and concentrated. The residue was purified by preparative-HPLC, the title compound was obtained (0.6 mg, 2%), which was in form of white solid. LCMS (ESI): $[M+H]^+$ = 441.2; $^1H$ NMR (400 MHz, $CD_3OD$) $\delta$ 8.04 (s, 1H). 7.50-7.45 (m, 3H). 7.424-7.422 (d, *J* = 0.8 Hz, 1H). 7.35-7.34 (d, *J* = 7.6 Hz, 1H). 7.22-7.21 (d, *J* = 2.0 Hz, 2H). 7.15-7.11 (dd, *J* = 8.8 Hz, 2H). 6.62 (s, 1H). 5.36 (m, 1H). 4.12-4.11 (m, 1H). 3.67 (s, 3H). 2.65 (s, 3H). 1.77-1.75 (d, *J* = 6.8 Hz, 3H).

Embodiment 4051: **(*S*)-1-ethyl-3-(7-(4-flurophenyl)-2,5-dimethyl-3-oxo-3,5-dihydro-2*H*-benzo[*c*]pyrido[3,4-*e*]aze pin-10-yl)urea**

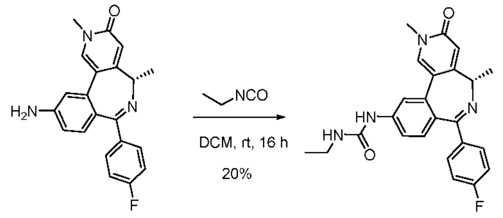


4049C 4051

Step 1: (*S*)-1-ethyl-3-(7-(4-flurophenyl)-2,5-dimethyl-3-oxo-3,5-dihydro-2*H*-benzo[*c*]pyrido[3,4-*e*]azepin -10-yl)urea

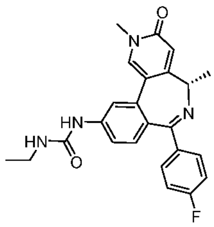

Ethyl isocyanate (37 mg, 0.52 mmol) was added into (*S*)-10-amino-7-(4-flurophenyl)-2,5-dimethyl-2,5-dihydro-3*H*-benzo[*c*]pyrido[3,4-*e*]azepin-3-keto ne (60 mg, 0.17 mmol) in dichloromethane (4 mL) solution. The reaction mixture was stirred at r.t. overnight, after concentration, the residue was purified by preparative-HPLC to obtain the title compound (14.4 mg, 20%), which was in form of white solid. LCMS (ESI) $[M+H]^+$= 419.0; $^1$H NMR (400 MHz, DMSO-$d_6$) $\delta$ 8.87 (s, 1H). 7.98 (s, 1H). 7.89 (s, 1H). 7.49-7.40 (m, 3H). 7.22-7.12 (m, 3H). 6.37 (s, 1H). 6.32-6.30 (m, 1H). 3.96-3.94 (d, *J* = 6.4 Hz, 1H) 3.49 (s, 3H). 3.15-3.12 (q, 2H). 1.64-1.62 (m, 3H). 1.09-1.05 (t, *J* = 7.2 Hz, 3H).

**Embodiment 4052: (*S*)-*N*-ethyl-7-(4-flurophenyl)-2,5-dimethyl-3-oxo-3,5-dihydro-2*H*-benzo[*c*]pyrido[3,4-e]azepin-10-sulfamide**

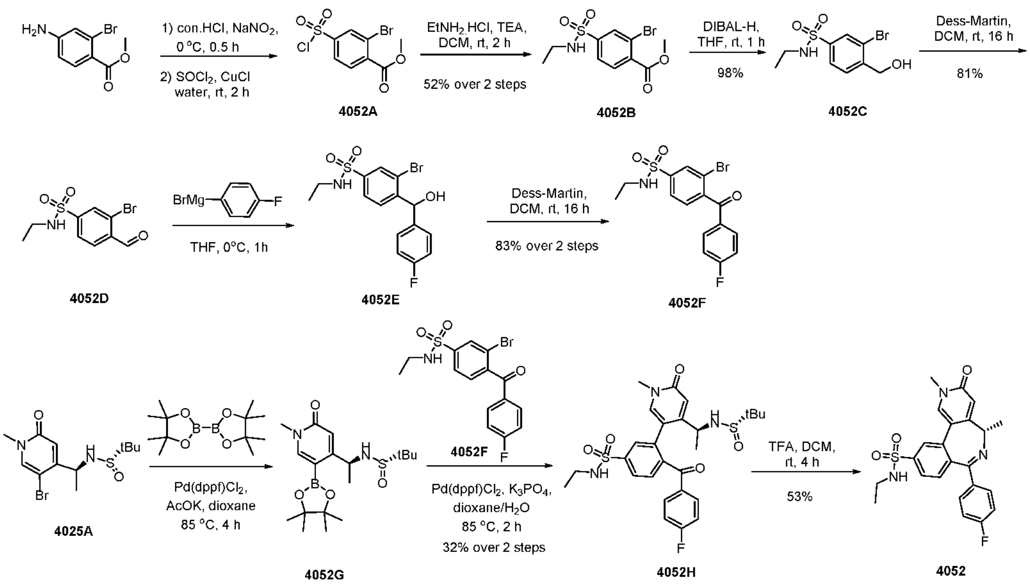


Step 1:2-bromo-4-(chlorosulfonyl)methylbenzoate

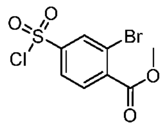

At 0°C, sodium nitrite (660 mg, 9.56 mmol) in water solution (20 mL) was added into 4-amino-2-bromomethylbenzoate (2.0 g, 8.69 mmol) in concentrated HCl solution (15 mL). The reaction mixture was stirred at 0°C for 0.5 hour, the reaction mixture 1 was obtained. In another reaction bottle, water (20 mL) was added, cooled down to 0°C, then slowly and sequently added sulfoxide chloride (5.17 g, 43.47 mmol) and cuprous chloride (43 mg, 0.43 mmol), the reaction mixture was stirred at r.t. for 10 minutes, then slowly added the reaction mixture 1. The reaction mixture was then continued stirring for 2 hours, then filtered, the residue was washed by cold water and dried in vacuum, the crude product of the tiltle compound was obtained (1.8 g) which was in form of yellow solid, and was used directly in the next step without purification. LCMS (ESI) $[MH]^+$= 312.7.

Step 2:2-bromo-4-(*N*-ethylsulfamide)methylbenzoate

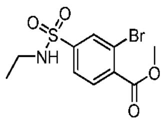

compound 2-bromo-4-(chlorosulfonyl)methylbenzoate crude product (1.8 g), triethylamine (2.64 g, 26.07 mmol) and ethylamine hydrochlorid (1.4 g, 17.38 mmol) were mixed in dichloromethane (20 mL), and stirred for 2 hours at rt. After concentration, the residue was purified by flash chromatography (PE/EtOAc=3/1), the title compound was obtained (1.46 g, yield of the two steps 52.1%), which was in form of yellow solid. LCMS (ESI) $[M+H]^+$= 321.8.

Step 3:3-bromo-*N*-ethyl-4-(hydroxymethyl)benzylsulfamide

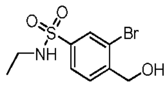

Diisobutylaluminum hydride (1 M in THF solution, 13.6 mL, 13.6 mmol) was added dropwise into 2-bromo-4-(*N*-ethylsulfamide)methylbenzoate (1.46 g, 4.53 mmol) in THF (20 mL) solution. The reaction mixture was stirred at r.t. for 1 hour. After concentration, the residue was dissolved by EtOAc (100 mL), sequently washed by 2N HCl in solution and saturated sodium bicarbonate solution, dried over anhydrous sodium sulfate, filtered and concentratedthe tiltle compound was obtained (1.3 g, 98%), in form of yellow oil. LCMS (ESI) $[M+H]^+$= 293.8.

Step 4:3-bromo-*N*-ethyl-4-formylbenzylsulfamide

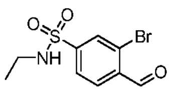

3-bromo-*N*-ethyl-4-(hydroxymethyl)benzylsulfamide (1.3 g, 4.42 mmol) and Dress-Martin agent (2.8 g, 6.63 mmol) were mixed in dichloromethane (20 mL), then stirred at r.t. overnight. After concentration, the residue was purified by flash chromatography (PE/EtOAc=3/1), the title compound was obtained (1.05 g, 81.4%), which was in form of yellow solid. LCMS (ESI): no ion flow.

Step 5:3-bromo-*N*-ethyl-4-((4-flurophenyl)(hydroxyl)methyl)benzylsulfamide

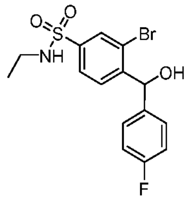

At 0°C, (4-flurophenyl)magnesium bromide (1 M in THF solution, 5.2 mL, 5.2 mmol) was slowly added dropwise into 3-bromo-*N*-ethyl-4-formylbenzylsulfamide (1.05 g, 3.42 mmol) in THF (20 mL) solution, then stirred at 0°C for 1 hour. The reaction was quenched by water (60 mL), then extracted by EtOAc (100 mL*2). The organic phase was combined, then washed with brine (50 mL), dried over anhydrous sodium sulfate, filtered and concentrated, the crude product of the tiltle compound was obtained (1.4 g), which was in form of yellow solid, and was used directly in the next step without purification. LCMS (ESI) $[M+H]^+$ = 389.7.

Step 6:3-bromo-*N*-ethyl-4-(4-fluorobenzoyl)benzylsulfamide

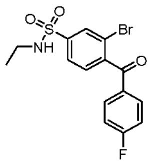

3-bromo-*N*-ethyl-4-((4-flurophenyl)(hydroxyl)methyl)benzylsulfamide crude product (1.4 g) and Dess-Martin periodinane (2.18 g, 5.13 mmol) were mixed in dichloromethane (20 mL), then stirred at r.t. overnight. After concentration, the residue was purified by flash chromatography (PE/EtOAc=3/1), the tiltle compound was obtained (1.1 g, yield of the two steps 83%), which was in form of yellow solid. LCMS (ESI) $[M+H]^+$ = 385.7.

Step 7: (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide

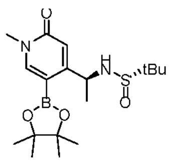

Compound

(*S*)-*N*-((*S*)-1-(5-bromo-1-methyl-2-oxo-1,2-dihydropyridin-4-yl)ethyl)-2-methylpropane-2-sulfena mide (100 mg, 0.298 mmol), bis(pinacolato)diboron (91 mg, 0.358 mmol), [1,1'-bis(diphenylphosphino)ferrocene]palladium (II) dichloride (22 mg, 0.03 mmol) and potassium acetate (73 mg, 0.745 mmol) were mixed in 1,4-dioxane (4 mL). Under nitrogen protection, the reaction mixture was heated to 85°C and stirred for 4 hours. Then filtered and the filtrate was concentrated, the crude product of the tiltle compound was obtained (120 mg), in form of black oil, and was used directly in the next step without purification. LCMS (ESI) $[M+H]^+$ = 382.9.

Step 8:3-(4-((*S*)-1-(((*S*)-tert-butyl-sulfinyl)amino)ethyl)-1-methyl-6-oxo-1,6-dihydropyridin-3-yl)-*N*-et hyl-4-(4-fluorobenzoyl)benzylsulfamide

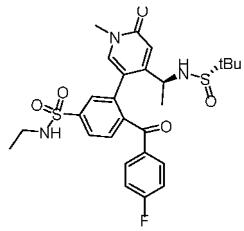

(*S*)-2-methyl-*N*-((*S*)-1-(1-methyl-2-oxo-5-(4,4,5,5-tetramethyl-1,3,2-dioxoperane-2-yl)-1,2-dihydropyridin-4-yl)ethyl)propane-2-sulfenamidecrude product (120 mg), 3-bromo-*N*-ethyl-4-(4-fluorobenzoyl)benzylsulfamide (92 mg, 0.238 mmol), [1,1'-bis(diphenylphosphino)ferrocene]palladium (II) dichloride (22 mg, 0.03 mmol) and potassium phosphate (158 mg, 0.745 mmol) were mixed in 1,4-dioxane (5 mL) and water (0.5 mL). Under nitrogen protection, the reaction mixture was heated to 85°C then stirred for 2 hours. After filtrateion, the filtratee was spin dried, the residue was purified by flash chromatography (PE/EtOAc/methanol=100/5/7). The title compound was obtained (53 mg, yield of the two steps 32%), which was in form of yellow solid. LCMS (ESI) $[M+H]^+$ = 561.7.

Step 9: (*S*)-*N*-ethyl-7-(4-flurophenyl)-2,5-dimethyl-3-oxo-3,5-dihydro-2*H*-benzo[*c*]pyrido[3,4-*e*]azepin-1 0-sulfamide

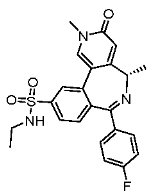

3-(4-((*S*)-1-(((*S*)-tert-butyl-sulfinyl)amino)ethyl)-1-methyl-6-oxo-1,6-dihydropyridin-3-yl)-*N*-ethyl-4-(4-fluorobenzoyl)benzylsulfamide (53 mg, 0.094 mmol) was dissolved in dichloromethane (4 mL) and TFA (1 mL), the reaction mixture was stirred at r.t. for 4 hours. The reaction mixture was concentrated, the residue was purified by preparative-HPLC, the title compound was obtained (22 mg, 53%), which was in form of white solid. LCMS (ESI) $[M+H]^+$ = 439.8; $^1$H NMR (400 MHz, $CD_3OD$) $\delta$ 8.14 (s, 1H). 7.91 (d, *J* = 8.0 Hz, 1H). 7.53-7.49 (m, 3H). 7.17-7.13 (m, 2H). 6.65 (s, 1H). 4.11 (q, 1H). 3.68 (s, 3H). 3.05 (q, 2H). 1.79 (t, 3H). 1.16 (t, 3H).

**Embodiment 4053: (*S*)-10-((ethylsulfonyl)methyl)-7-(4-methoxylphenyl)-2,5-dimethyl-2,5-dihydro-3*H*-benzo[*c*]p yrido[3,4-*e*]azepin-3-ketone**

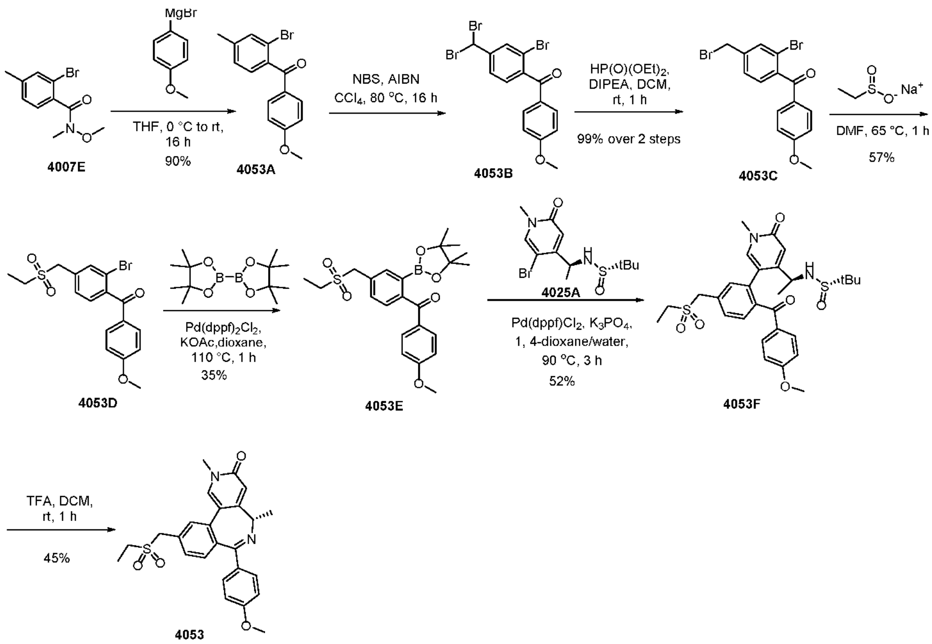


Step 1: (2-bromo-4-methylphenyl)(4-methoxylphenyl)methanone

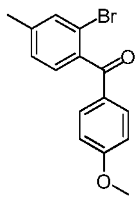

At 0°C, (4-methoxyl)magnesium bromide (1 M in THF solution, 46.7 mL, 46.7 mmol) was slowly added dropwise into 2-bromo-*N*-methoxyl-*N*, 4-dimethylbenzyl formamide (1.5 g, 5.84 mmol) in THF (10 mL) solution, the reaction mixture was stirred at r.t. overnight under nitrogen protection. The reaction was quenched by saturated ammonium chloride, then extracted by EtOAc. The organic phase was washed by brine, dried over anhydrous sodium sulfate, filtered and concentrated. The residue was purified by flash chromatography (PE/EtOAc=20/1). The title compound was obtained (1.6 g, 90%), in form of yellow oil. LCMS (ESI) $[M+H]^+$ = 306.8.

Step 2: (2-bromo-4-(dibromomethyl)phenyl)(4-methoxylphenyl)methanone

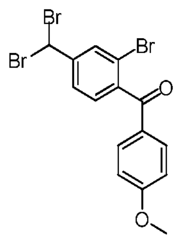

Compound *N*-bromodesuccinimide (2.8 g, 15.78 mmol), azobisisobutyronitrile (87 mg, 0.526 mmol) and (2-bromo-4-methylphenyl)(4-methoxylphenyl)methanone (1.60 g, 5.26 mmol) was dissolved in tetrachloromethane (20 mL), then stirred at for 16 hours, concentrated and the crude product of the title compound was obtained, which was in form of yellow oil, and was used directly in the next step without purification. LCMS (ESI) $[M+H]^+$= 464.6.

Step 3: (2-bromo-4-(bromomethyl)phenyl)(4-methoxylphenyl)methanone

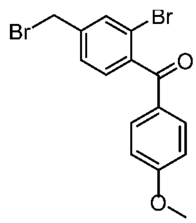

Diethyl phosphite (773 mg, 5.6 mmol) and disopropyl ethylamine (13 g, 10.1 mmol) were added into (2-bromo-4-(dibromomethyl)phenyl)(4-methoxylphenyl)methanone in dichloromethane (20 mL) solution, then stirred at r.t. for 1 hour. Concentration of the reaction mixture, the residue was purified by flash chromatography (PE/EtOAc=1/1). The title compound was obtained (2.0 g, yield of the two steps 99%), in form of yellow oil. LCMS (ESI) $[M+H]^+$=384.7.

Step 4: (2-bromo-4-((ethylsulfonyl)methyl)phenyl)(4-methoxylphenyl)methanone

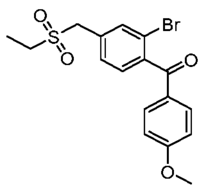

Sodium ethylsulfinate (1.80 g, 15.69 mmol) was added into (2-bromo-4-(bromomethyl)phenyl)(4-methoxylphenyl)methanone (2.0 g, 5.23 mmol) in *N,N*-dimethylformamide (10 mL) solution. The reaction mixture and heated to 65°C then stirred for 1 hour. Water (50 mL) was added into the reaction mixture, then extracted EtOAc (20 mL), the organic phase was washed by brine (50 mL*3), dried over anhydrous sodium sulfate, filtered and concentrated. The residue was purified by flash chromatography (PE/EtOAc=1/1). The title compound was obtained (1.2 g, 58%), which was in form of brownish solid. LCMS (ESI) $[M+H]^+$= 397.0.

Step 5: (4-((ethylsulfonyl)methyl)-2-(4,4,5,5-tetramethyl-1,3,2-dioxaborolan-2-yl)phenyl)(4-methoxyl)me thanone

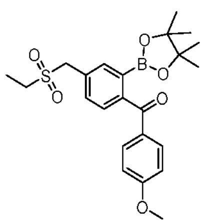

(2-Bromo-4-((ethylsulfonyl)methyl)phenyl)(4-methoxylphenyl)methanone (1.2 g, 3.03 mmol), bis(pinacolato)diboron (1.54 g, 6.06 mmol), [1,1'-bis(diphenylphosphino)ferrocene]palladium (II) dichloride (219 mg, 0.3mmol) and potassium acetate (890 mg, 9.09 mmol) were mixed in 1,4-dioxane (10 mL), heated to 110°C under nitrogen protection then stirred for 1 hour. The reaction mixture was filtered and the filtrate was concentrated, the residue was purified by flash chromatography (PE/EtOAc=10/1). The title compound was obtained (470 mg, 35%), which was in form of yellow solid. LCMS (ESI) $[M+H]^+$= 444.8.

Step 6: (*S*)-*N*-(1-(5-(5-((ethylsulfonyl)methyl)-2-(4-methoxylbenzoyl)phenyl)-1-methyl-2-oxo-1,2-dihydropyridin-4-yl)ethyl)-2-methylpropane-2-sulfenamide

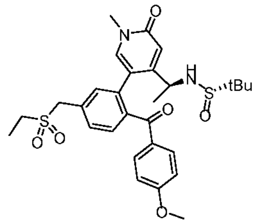

(4-((ethylsulfonyl)methyl)-2-(4,4,5,5-tetramethyl-1,3,2-dioxaborolan-2-yl)phenyl)(4-methoxylphenyl)methanone (124 mg, 0.28 mmol), (*S*)-*N*-((*S*)-1-(5-bromo-1-methyl-2-oxo-1,2-dihydropyridin-4-yl)ethyl)-2-methylpropane-2-sulfena mide (47 mg, 0.14 mmol), [1,1'-bis(diphenylphosphino)ferrocene]palladium (II) dichloride (10 mg, 0.014 mmol) and potassium phosphate (89 mg, 0.42 mmol) were mixed in 1,4-dioxane (4 mL) and water (0.4 mL), then heated to 90°C and stirred for 3 hours under nitrogen protection. The reaction mixture was filtered and the filtrate was concentrated, the residue was purified by flash chromatography (EtOAc/methanol=30/1). The title compound was obtained (42 mg, 52%), which was in form of yellow solid. LCMS (ESI) $[M+H]^+$ = 573.0.

Step 7: (*S*)-10-((ethylsulfonyl)methyl)-7-(4-methoxylphenyl)-2,5-dimethyl-2,5-dihydro-3*H*-benzo[*c*]pyrid o[3,4-*e*]azepin-3-ketone

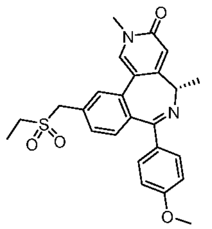

TFA (1 mL) was added into (*S*)-*N*-(1-(5-(5-((ethylsulfonyl)methyl)-2-(4-methoxylbenzoyl)phenyl)-1-methyl-2-oxo-1,2-dihydr opyridin-4-yl)ethyl)-2-methylpropane-2-sulfenamide (42 mg, 0.07 mmol) in dichloromethane (4 mL), the reaction mixture was stirred at r.t. for 1 hour. The reaction mixture was concentrated, the residue was purified by preparative-HPLC to obtain the title compound (14.2 mg, 45%), which was in form of white solid. LCMS (ESI): $[M+H]^+$ = 450.8; $^1H$ NMR (400 MHz, DMSO-$d_6$) $\delta$ 8.02 (s, 1H). 7.69 (s, 1H). 7.50-7.46 (d, *J* = 8.0 Hz, 1H). 7.40-7.32 (m, 3H). 1H). 6.96-6.91 (d, *J* = 8.8 Hz, 2H). 7.23 (m, 3H). 6.40 (s, 1H). 4.70-4.51 (m, 2H). 3.97-3.88 (m, 1H). 3.77 (s, 3H). 3.49 (s, 3H). 3.18-3.10 (q, *J* = 7.2 Hz, 2H). 1.64-1.63 (d, *J* = 6.8 Hz, 3H). 1.27-1.24 (t, *J* = 7.6 Hz, 3H).

Embodiment 4061: **(*S*)-10-((ethylsulfonyl)methyl)-7-(4-hydroxylphenyl)-2,5-dimethyl-2,5-dihydro-3*H*-benzo[*c*]p yrido[3,4-*e*]azepin-3-ketone**

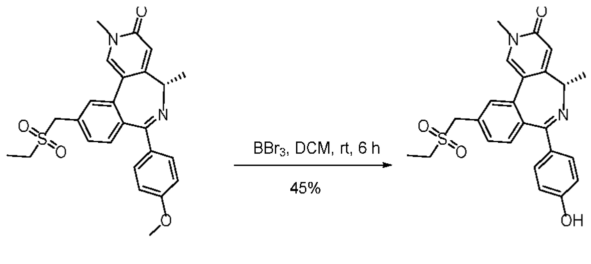


4053 4061

The mixture of (*S*)-10-((ethylsulfonyl)methyl)-7-(4-methoxylphenyl)-2,5-dimethyl-2,5-dihydro-3*H*-benzo[*c*]pyrido[3,4-*e*]azepin-3-ketone (6 mg, 0.013 mmol) and tribromo boron (17% in dichloromethane solution, 1 mL) in was strred at r.t. for 6 hours. Concentration of the reaction mixture, the residue was purified by preparative-HPLC, the title compound was obtained (2.6 mg, 45%), which was in form of yellow solid. LCMS (ESI) [M+H]$^+$ = 436.7; $^1$H NMR (400 MHz, $CD_3OD$) $\delta$ 8.08 (s, 1H). 7.75 (s, 1H). 7.56 (d, *J* = 8.0 Hz, 1H). 7.40 (d, *J* = 8.0 Hz, 1H). 7.31 (s, H). 7.29 (s, 1H). 6.79 (s, 1H). 6.77 (s, 1H). 6.63 (s, 1H). 4.59 (s, 2H). 4.08 (q, 1H). 3.68 (s, 3H). 3.19 (q, 2H). 1.76 (d, *J* = 6.4 Hz, 3H). 1.44 (t, 3H).

**Embodiment 4054: (*S*)-10-((ethylsulfonyl)methyl)-7-(4-fluoro-3-methoxylphenyl)-2,5-dimethyl-2,5-dihydro-3*H*-benzo[*c*]pyrido[3,4-*e*]azepin-3-ketone**

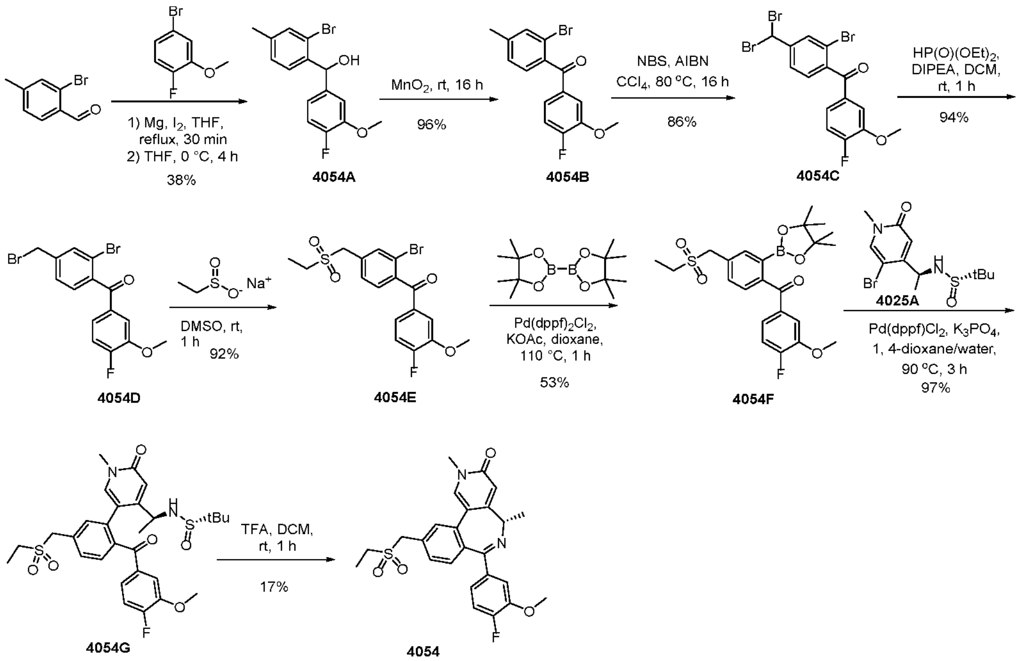


4054A 4054B 4054C 4054D 4054E 4054F 4054G 4054

Step 1: (2-bromo-4-methylphenyl)(4-fluoro-3-methoxylphenyl)methanol

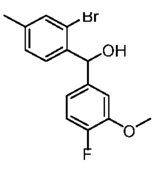

Under nitrogen protection, THF (5 mL) was added into magnissium spalls (864 mg, 36 mmol) and a piece of

$I_2$, then slowly added a small amount of 4-bromo-1-fluoro-2-methoxylbenzyl (7.4 g, 36 mmol) in THF (30 mL) solution. When the reaction was initiated, the temperature of the reaction mixture was raised to 60°C, and the rest material was slowly added. The reaction mixture continued to reflux for 30 minutes to get the Griganard reagent solution. 2-Bromo-4-methylbenzaldehyde (3.6 g, 18 mmol) in THF (10 mL) solution was cooled down to 0°C, the Griganard reagent was slowly added. The reaction mixture was stirred at r.t. for 4 hours, then added EtOAc and saturated ammonium chloride solution to layer the reaction mixture. The organic phase was washed by brine, dried over anhydrous sodium sulfate, filtered and concentrated. The residue was purified by flash chromatography (PE/EtOAc=20/1). The title compound was obtained (2.2 g, 38%), in form of yellow oil. $^1$H NMR (400 MHz, DMSO-$d_6$) $\delta$ 7.38 (d, $J$ = 8.4 Hz, 2H). 7.14 (d, $J$ = 7.2 Hz, 1H). 7.08 (dd, $J$ = 2.0, 8.4 Hz, 3H). 7.01 (m, 1H). 6.88 (m, 1H). 6.14 (d, $J$ = 3.6 Hz, 1H).

Step 2: (2-Bromo-4-methylphenyl)(4-fluoro-3-methoxylphenyl)methanone

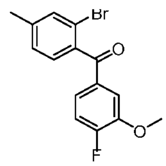

(2-Bromo-4-methylphenyl)(4-fluoro-3-methoxylphenyl)methanol (2.2 g, 6.8 mmol) and manganese dioxide (3.0 g, 34 mmol) were mixed in dichloromethane (20 mL), then stirred at r.t. overnight. The reaction mixture was filtered and the filtrate was concentrated, the tiltle compound was obtained (2.1 g, 96%), which was in form of yellow solid, and was used directly in the next step without purification.

Step 3: (2-Bromo-4-(dibromomethyl)phenyl)(4-fluoro-3-methoxylphenyl)methanone

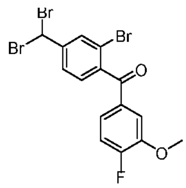

*N*-Bromodesuccinimide (3.47 g, 19.5 mmol), azobisisobutyronitrile (108 mg, 0.65 mmol) and (2-bromo-4-methylphenyl)(4-fluoro-3-methoxylphenyl)methanone (2.1 g, 6.5 mmol) was dissolved in tetrachloromethane (20 mL), and raised the temperature to 80°C and stirred for 16 hours. The reaction mixture was concentrated and the tiltle compound was obtained (2.7 g, 86%), which was in form of yellow solid, and was used directly in the next step without purification.

Step 4: (2-Bromo-4-(bromomethyl)phenyl)(4-fluoro-3-methoxylphenyl)methanone

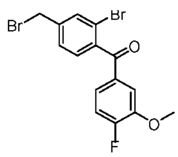

Diethyl phosphite (773 mg, 5.6 mmol) and disopropyl ethylamine (13 g, 10.1 mmol) were added into (2-bromo-4-(dibromomethyl)phenyl)(4-fluoro-3-methoxylphenyl)methanone (2.7 g, 5.6 mmol) in dichloromethane (20 mL) solution, then stirred at r.t. for 1 hour. Concentration of the reaction mixture, the residue was purified by flash chromatography (PE/EtOAc=1/1). The title compound was obtained (2.1 g, 94%), which was in form of yellow solid. LCMS (ESI) $[M+H]^+$= 402.6.

Step 5: (2-bromo-4-((ethylsulfonyl)methyl)phenyl)(4-fluoro-3-methoxylphenyl)methanone

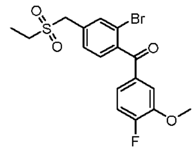

Sodium ethylsulfinate (1.83 g, 15.76 mmol) was added into 2-bromo-4-(bromomethyl)phenyl)(4-fluoro-3-methoxylphenyl)methanone (2.1 g, 5.25 mmol) in dimethylsulfoxide (10 mL) solution, then stirred at r.t. for 1 hour. EtOAc (20 mL) and water (50 mL) was added into the reaction solution, the organic phase was washed by brine (50 mL*3), dried over anhydrous sodium sulfate, filtered and concentrated. The residue was purified by flash chromatography (PE/EtOAc=1/1). The title compound was obtained (2.0 g, 92%), which was in form of brownish solid. LCMS (ESI) $[M+H]^+$ = 414.7.

Step 6: (4-((ethylsulfonyl)methyl)-2-(4,4,5,5-tetramethyl-1,3,2-dioxaborolan-2-yl)phenyl)(4-fluoro-3-met hoxylphenyl)methanone

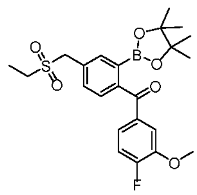

Compound (2-bromo-4-((ethylsulfonyl)methyl)phenyl)(4-fluoro-3-methoxylphenyl)methanone (220 mg, 0.53 mmol), bis(pinacolato)diboron (269 mg, 1.06 mmol), [1,1'-bis(diphenylphosphino)ferrocene]palladium (II) dichloride (37 mg, 0.05mmol) and potassium acetate (156 mg, 1.59 mmol) were mixed in 1,4-dioxane (6 mL), and heated to 110°C under nitrogen protection then stirred for 1 hour. The reaction mixture was filtered and the filtrate was concentrated, the residue was purified by flash chromatography (EtOAc/methanol=30/1). The title compound was obtained (130 mg, 53%), which was in form of yellow solid. LCMS (ESI) $[M+H]^+$ = 462.8.

Step 7: (*S*)-*N*-(1-(5-(5-((ethylsulfonyl)methyl)-2-(4-fluoro-3-methoxylbenzoyl)phenyl)-1-methyl-2-oxo-1,2-dihydropyridin-4-yl)ethyl)-2-methylpropane-2-sulf enamide

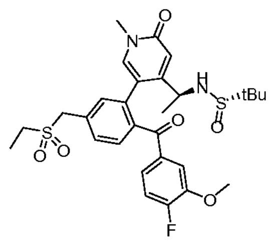

(4-((ethylsulfonyl)methyl)-2-(4,4,5,5-tetramethyl-1,3,2-dioxaborolan-2-yl)phenyl)(4-fluoro-3-methoxylbenzoyl)methanone (130 mg, 0.28 mmol), (*S*)-*N*-((*S*)-1-(5-bromo-1-methyl-2-oxo-1,2-dihydropyridin-4-yl)ethyl)-2-methylpropane-2-sulfena mide (47 mg, 0.14 mmol), [1,1'-bis(diphenylphosphino)ferrocene]palladium (II) dichloride (10 mg, 0.014 mmol) and potassium carbonate (58 mg, 0.42 mmol) were mixed in 1,4-dioxane (4 mL) and water (0.4 mL), the reaction mixture was heated to 90°C and stirred for 3 hours under nitrogen protection. The reaction mixture was filtered and the filtrate was concentrated, the residue was purified by flash chromatography (EtOAc/methanol=30/1). The title compound was obtained (80 mg, 97 %), which was in form of yellow solid. LCMS (ESI) $[M+H]^+$ = 590.6.

Step 8: (*S*)-10-((ethylsulfonyl)methyl)-7-(4-fluoro-3-methoxylbenzoyl)-2,5-dimethyl-2,5-dihydro-3*H*-benzo[*c*]pyrido[3,4-*e*]azepin-3-ketone

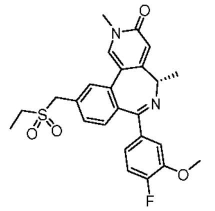

TFA (1 mL) was added into (*S*)-*N*-(1-(5-(5-((ethylsulfonyl)methyl)-2-(4-fluoro-3-methoxylbenzoyl)phenyl)-1-methyl-2-oxo-1,2-dihydropyridin-4-yl)ethyl)-2-methylpropane-2-sulf enamide (80 mg, 0.136 mmol) in dichloromethane (4 mL) solution, then stirred for 1 hour at rt. Concentration of the reaction mixture, the residue was purified by preparative-HPLC to obtain the title compound (10.6 mg, 17%), in form of white solid. LCMS (ESI) $[M+H]^+$ = 469.2; $^1H$ NMR (400 MHz, DMSO-$d_6$) 8.04 (s, 1H). 7.71-7.70 (d, *J* = 1.2 Hz, 1H). 7.50-7.47 (m, 1H). 7.36-7.32 (dd, *J* = 8.0, 15.6 Hz, 2H). 7.23-7.18 (m, 1H). 6.81-6.77 (m, 1H). 6.42 (s, 1H). 4.67-4.53 (q, *J* = 13.6 Hz, 2H). 3.98-3.93 (m, 1H). 3.82 (s, 3H). 3.50 (s, 3H). 3.16-3.10 (q, *J* = 7.6 Hz, 2H). 1.67-1.65 (d, *J* = 6.8 Hz, 3H). 1.2-1.23 (t, *J* = 9.5 Hz, 3H).

**Embodiment 4064: (*S*)-10-((ethylsulfonyl)methyl)-7-(4-fluoro-3-hydroxylphenyl)-2,5-dimethyl-2,5-dihydro-3*H*-benzo[*c*]pyrido[3,4-*e*]azepin-3-ketone**

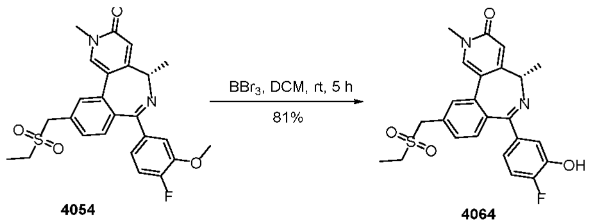


The mixture of (*S*)-10-((ethylsulfonyl)methyl)-7-(4-fluoro-3-methoxylphenyl)-2,5-dimethyl-2,5-dihydro-3*H*-benzo[c]pyrido[3,4-e]azepin-3-ketone (7.6 mg, 0.016 mmol) and tribromo boron (17% in dichloromethane solution, 1 mL) in was stirred for 5 hours at rt. Concentration of the reaction mixture, the residue was purified by preparative-HPLC, the title compound was obtained (6.0 mg, 81%), which was in form of white solid. LCMS (ESI) $[M+H]^+$ = 455.2; $^1H$ NMR (400 MHz, $CD_3OD$) $\delta$ 8.09 (s, 1H). 7.76 (s, 1H). 7.56 (d, *J* = 8.0 Hz, 1H). 7.39 (d, *J* = 8.0 Hz, 1H). 7.09-7.04 (m, 2H). 6.79 (s, 1H). 6.63 (s, 1H). 4.59 (s, 2H). 4.10 (q, 1H). 3.68 (s, 3H). 3.21 (q, 2H). 1.77 (d, *J* = 6.4 Hz, 3H). 1.44 (t, 3H).

**Embodiment 4055: (*S*)-*N*-(9-fluoro-7-(4-flurophenyl)-2,5-dimethyl-3-oxo-3,5-dihydro-2*H*-benzo[*c*]pyrido[3,4-e]azepin-10-yl)ethylsulfonamide**

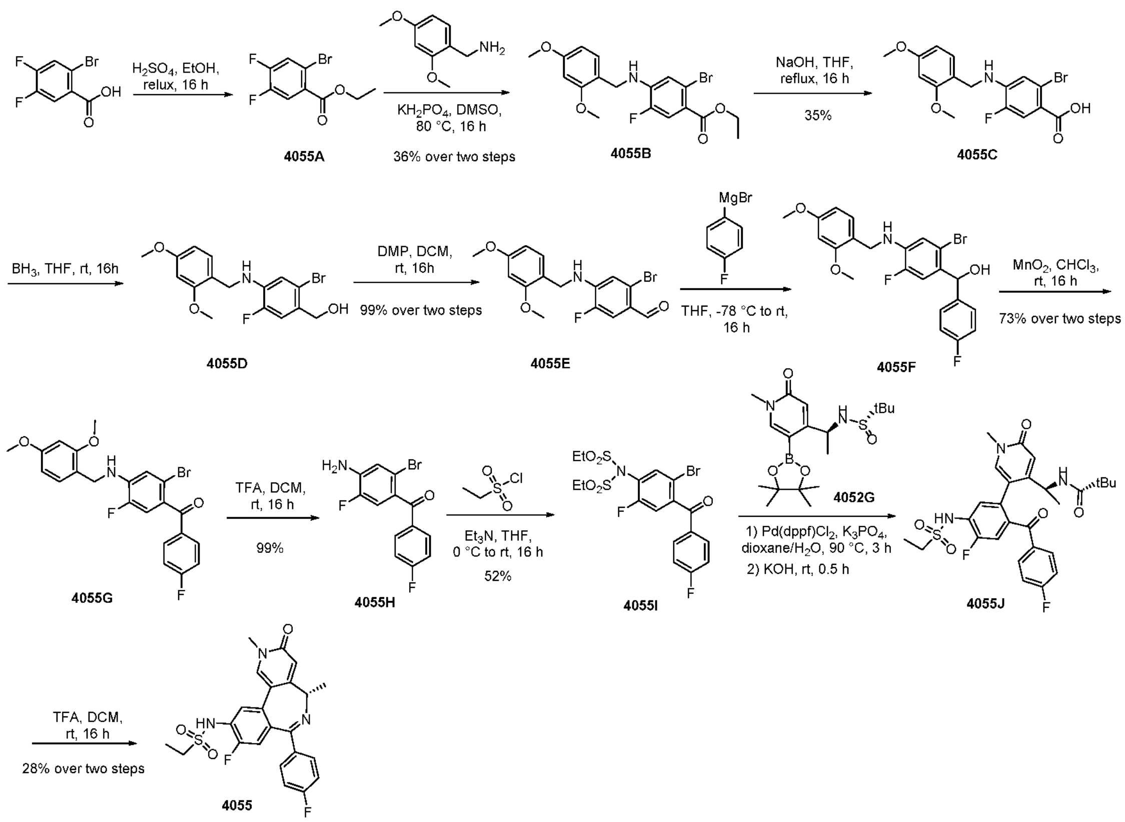


Step 1:2-Bromo-4, 5-dichlorobenzyl ethyl formate

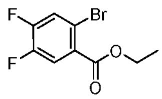

2-Bromo-4, 5-dichlorobenzyl formic acid (5 g, 21.1 mmol) and concentrated sulfuric acid (5 mL) was dissolved in ethanol (50 mL), heated to reflux and stirred overnight. The reaction mixture was cooled down to r.t. and then concentrated, the residue was poured into cold water, extracted by EtOAc (50 mL). The organic phase was dried over anhydrous sodium sulfate, filtered and concentratedthe crude product of the tiltle compound was obtained (5.5 g), in form of colorless liquid, and was used directly in the next step without purification. LCMS (ESI) $[M+H]^+$ = 265.9.

Step 2:2-Bromo-4-((2,4-dimethoxylbenzyl)amino)-5-fluorobenzyl ethyl formate

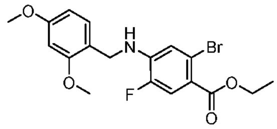

2-Bromo-4, 5-dichlorobenzyl ethyl formate (5.5 g, 21.1 mmol), (2,4-dimethoxylphenyl)methylamine (4.2 g, 25.3 mmol) and monopotassium phosphate (11.48 g, 84.4 mmol) were mixed in dimethylsulfoxide (30 mL), then heated to 80°C and stirred overnight. Water and EtOAc (50 mL) was added into the reaction mixture, the organic phase was separated. The organic phase was dried over anhydrous sodium sulfate, filtered and concentrated. The residue was purified by flash chromatography (PE/EtOAc=4/1). The title compound was obtained (3 g, yield of the two steps 36%),

which was in form of white solid. LCMS (ESI) [M+Na]$^+$ = 433.8.

Step 3:2-Bromo-4-((2,4-dimethoxylbenzyl)amino)-5-fluorobenzylformic acid

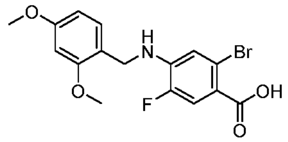

2-Bromo-4-((2,4-dimethoxylbenzyl)amino)-5-fluorobenzylethyl formate (3 g, 7.28 mmol) and sodium hydroxide aqueous solution (10 mL, 2 N) were mixed in THF (30 mL), and refluxed overnight. The reaction mixture HCl (1 N) and, and extracted by EtOAc (30 mL). The organic phase was dried over anhydrous sodium sulfate, filtered and concentrated, the tiltle compound was obtained (1 g, 35%), which was in form of white solid. LCMS (ESI) [M+Na]$^+$ = 405.7.

Step 4: (2-Bromo-4-((2,4-methoxylbenzyl)amino)-5-flurophenyl)methanol

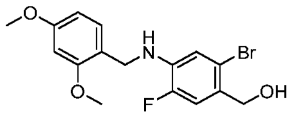

2-Bromo-4-((2,4-dimethoxylbenzyl)amino)-5-fluorobenzyl formic acid (1 g, 2.60 mmol) and borane (1 M in THF solution, 5.2 mL, 5.2 mmol) was dissolved in THF (10 mL) solution, the reaction mixture was stirred at r.t. overnight. The reaction was quenched by methanol (10 mL), then after concentration, the crude product of the tiltle compound was obtained (1 g), which was in form of white solid. LCMS (ESI) [M+Na]$^+$ = 393.9.

Step 5:2-Bromo-4-((2,4-dimethoxylbenzyl)amino)-5-fluoro benzaldehyde

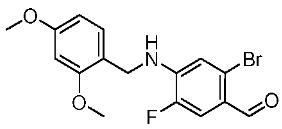

(2-Bromo-4-((2,4-dimethoxylbenzyl)amino)-5-flurophenyl)methanol (1 g, 2.60 mmol) and Dess-Martin periodinane (1.1 g, 2.6mmol) were mixed in dichloromethane (20 mL), the reaction mixture was stirred at r.t. overnight, then concentrated. The residue was purified by flash chromatography (PE/EtOAc=1/1). The title compound was obtained (950 mg, yield of the two steps 99%), which was in form of white solid. LCMS (ESI) [M+H]$^+$ = 369.8.

Step 6: (2-Bromo-4-((2,4-dimethoxylbenzyl)amino)-5-flurophenyl)(4-flurophenyl)methanol

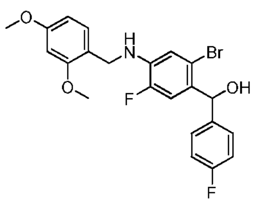

(4-Flurophenyl)magnesium bromide (1 M in THF solution, 25.8 mL, 25.8 mmol) was slowly added dropwise,

at -78°C and under nitrogen protection, to 2-bromo-4-((2,4-dimethoxylbenzyl)amino)-5-fluoro benzaldehyde (950 mg, 2.58 mmol) in THF (10 mL) solution, the reaction mixture was raised to room temperature and stirred overnight. The reaction was quenched by saturated ammonium chloride, then filtered and the filtrate was concentratedthe crude product of the tiltle compound was obtained (1.1 g), which was in form of yellow solid, and was used directly in the next step without purification. LCMS (ESI) $[M+Na]^+$ = 487.0.

Step 7: (2-bromo-4-((2,4-dimethoxylbenzyl)amino)-5-flurophenyl)(4-flurophenyl)methanone

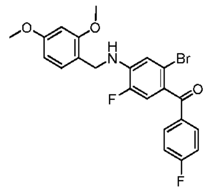

(2-bromo-4-((2,4-methoxylbenzyl)amino)-5-flurophenyl)(4-flurophenyl)methanol (1.1 g, 2.37 mmol) and manganese dioxide (2.06 g, 23.7 mmol) were mixed in formyl trichloride (10 mL), the reaction mixture was stirred at r.t. overnight. Then filtered and the filtrate was concentrated, the residue was purified by flash chromatography (PE/EtOAc=2/1). The title compound was obtained (800 mg, yield of the two steps 73%), which was in form of yellow solid. LCMS (ESI) $[M+Na]^+$ = 485.8.

Step 8: (4-Amino-2-bromo-5-flurohenl)(4-flurohenl)methanone

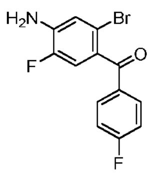

(2-Bromo-4-((2,4-dimethoxylbenzyl)amino)-5-flurophenyl)(4-flurophenyl)methanone (300 mg, 0.65 mmol) and TFA (49 mg, 0.43 mmol) was dissolved in dichloromethane (5 mL), then stirred at r.t. overnight. Concentration of the reaction mixture, the residue was purified by flash chromatography (dichloromethane/methanol=20/1). The title compound was obtained (200 mg, 99%), which was in form of yellow solid. LCMS (ESI) $[M+H]^+$ = 314.0.

Step 9: *N*-(5-bromo-2-fluoro-4-(4-fluorobenzol)henl)-*N*-(ethlsulfonl)ethlsulfonamide

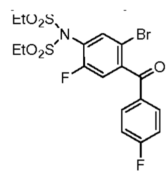

At 0°C, ethylsulfonyl chloride (820 mg, 6.4 mmol) was added into (4-amino-2-bromo-5-flurophenyl)(4-flurophenyl)methanone (200 mg, 0.64 mmol) and triethylamine (646 mg, 6.4 mmol) in dichloromethane (5 mL) solution. The reaction mixture was stirred at r.t. overnight after concentration, the residue was purified by flash chromatography (PE/EtOAc=1/1). The title compound was obtained (167 mg, 52%), which was in form of yellow solid. LCMS (ESI) $[M+H]^+$ = 495.8.

Step 10: *N*-(5-(4-((*S*)-1-(((*S*)-tert-butyl-sulfinyl)amino)ethyl)-1-methyl-6-oxo-1,6-dihydropyridin-3-yl)-2-fl uoro-4-(4-fluorobenzenesulfonyl)phenyl)ethylsulfonamide

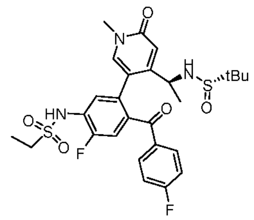

(*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (123 mg, 0.32 mmol), *N*-(5-bromo-2-fluoro-4-(4-fluorobenzoyl)phenyl)-*N*-(ethylsulfonyl)ethylsulfonamide (160 mg, 0.32 mmol), [1,1'-bis(diphenylphosphino)ferrocene]palladium (II) dichloride (24 mg, 0.032 mmol) and potassium phosphate (169 mg, 0.80 mmol) were mixed in 1,4-dioxane (8 mL) and water (2 mL), then heated to 90°C and stirred for 3 hours under nitrogen protection. Concentration of the reaction mixture, the residue was dissolved in potassium hydroxide (10% in aqueous solution, 6 mL) and water (6 mL). The reaction mixture obtained was stirred for 0.5 at rt, then extracted by EtOAc (70 mL). The organic phase was dried over anhydrous sodium sulfate, filtered and concentrated. The residue was purified by flash chromatography (EtOAc/methanol=10/1), the crude product of the tiltle compound was obtained (150 mg), which was in form of brownish solid. LCMS (ESI) $[M+H]^+$ = 580.2.

Step 11: (*S*)-*N*-(9-fluoro-7-(4-flurophenyl)-2,5-dimethyl-3-oxo-3,5-dihydro-2*H*-benzo[*c*]pyrido[3,4-*e*]azepi n-10-yl)ethylsulfonamide

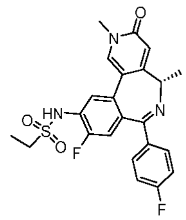

*N*-(5-(4-((*S*)-1-(((*S*)-tert-butyl-sulfinyl)amino)ethyl)-1-methyl-6-oxo-1,6-dihydropyridin -3-yl)-2-fluoro-4-(4-fluorobenzenesulfonyl)phenyl)ethylsulfonamide (150 mg, 0.26 mmol) and TFA (4 mL) was dissolved in dichloromethane (8 mL), then stirred at r.t. overnight. Concentration of the reaction mixture, the residue was purified by preparative-HPLC, the title compound was obtained (41.2 mg, yield of the two steps 28%), which was in form of yellow solid. LCMS (ESI) $[M+H]^+$ = 457.6; $^1H$ NMR (400 MHz, DMSO-$d_6$) $\delta$ 10.09 (s, 1H). 7.63 (d, *J* = 7.6 Hz, 1H). 7.52-7.49 (m, 2H). 7.24-7.17 (m, 3H). 6.39 (s, 1H). 3.98 (q, *J* = 9.6 Hz, 1H). 3.49 (s, 3H). 3.27-3.22 (m, 2H). 1.65 (d, *J* = 6.4 Hz, 3H). 1.30 (t, *J* = 7.6 Hz, 3H).

**Embodiment 4056: (*S*)-*N*-ethyl-2-(10-((ethylsulfonyl)methyl)-7-(4-flurophenyl)-2-methyl-3-oxo-3,5-dihydro-2*H*-b enzo[*c*]pyrido[3,4-*e*]azepin-5-yl)acetamide**

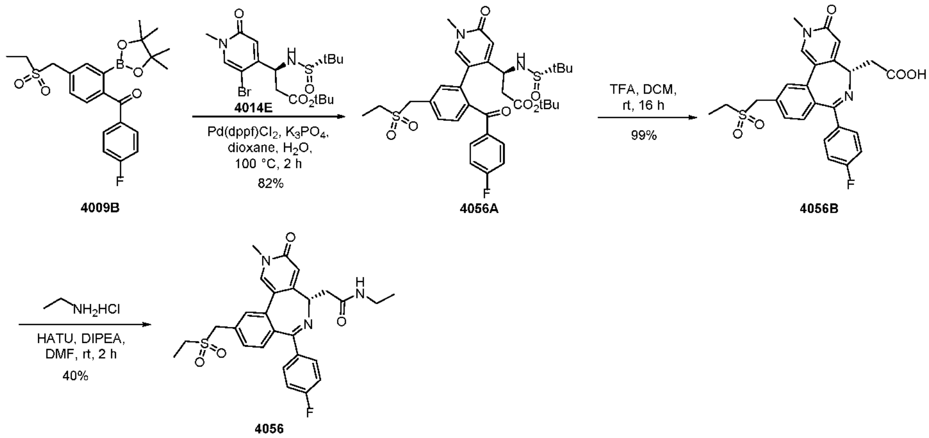


4009B 4056A 4056B

4056

Step 1: (S)-3-(((S)-tert-butyl-sulfinyl)amino)-3-(5-(5-((ethylsulfonyl)methyl)-2-(4-fluorobenzoyl)phenyl)-1-methyl-2-oxo-1,2-dihydropyridin-4-yl) tert-butylpropionate

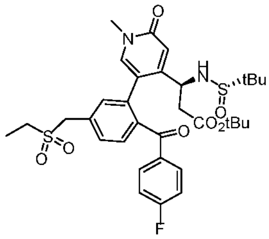

(4-((Ethylsulfonyl)methyl)-2-(4,4,5,5-tetramethyl-1,3,2-dioxaborolan-2-yl)phenyl)(4-flurophenyl)methanone (150 mg, 0.35 mmol), (*S*)-3-(5-bromo-1-methyl-2-oxo-1,2-dihydropyridin-4-yl)-3-(((*S*)-tert-butyl-sulfinyl)amino) tert-butylpropionate (100 mg, 0.23 mmol), [1,1'-bis(diphenylphosphino)ferrocene]palladium (II) dichloride (17 mg, 0.023 mmol) and potassium phosphate (146 mg, 0.69 mmol) were mixed in 1,4-dioxane (6 mL) and water (0.6 mL), and heated to 100°C under nitrogen protection then stirred for 2 hours. The reaction mixture was filtered and the filtrate was concentrated, the residue was purified by flash chromatography (EtOAc/methanol=30/1). The title compound was obtained (190 mg, 82%) which was in form of yellow solid. LCMS (ESI) $[M+H]^+$ = 660.7.

Step 2: (*S*)-2-(10-((ethylsulfonyl)methyl)-7-(4-flurophenyl)-2-methyl-3-oxo-3,5-dihydro-2*H*-benzo[*c*]pyri do[3,4-*e*]azepin-5-yl)acetic acid

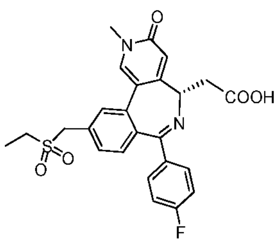

TFA (1 mL) was added into (S)-3-(((S)-tert-butyl-sulfinyl)amino)-3-(5-(5-((ethylsulfonyl)methyl)-2-(4-fluorobenzoyl)phenyl)-1-methyl-2-oxo-1,2-dihydropyridin-4-yl) tert-butylpropionate (190 mg, 0.29 mmol) in dichloromethane (4 mL) solution, the reaction mixture was stirred at r.t. overnight after concentration, the title compound was obtained (140 mg, 99%). in form of white solid, and was used directly in the next step without purification. LCMS (ESI) $[M+H]^+$ = 483.1.

Step 3: (*S*)-*N*-ethyl-2-(10-((ethylsulfonyl)methyl)-7-(4-flurophenyl)-2-methyl-3-oxo-3,5-dihydro-2*H*-benz o[*c*]pyrido[3,4-*e*]azepin-5-yl)acetamide

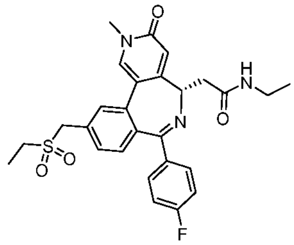

(*S*)-2-(10-((ethylsulfonyl)methyl)-7-(4-flurophenyl)-2-methyl-3-oxo-3,5-dihydro-2*H*-benzo[*c*]pyrido[3,4-*e*]azepin-5-yl)acetic acid (70 mg, 0.145 mmol), ethylamine hydrochlorid (18 mg, 0.22 mmol), *O*-(7-N-benzotri azole)-N,N,N',N'-tetramethylurea hexafluorophosphate (110 mg, 0.29 mmol) and *N,N*-diisopropyl ethylamine (57 mg, 0.435 mmol) was dissolved in *N,N*-dimethylformamide (3 mL), the reaction solution was stirred at r.t. for 2 hours. Water was added into the reaction mixture, then extracted by EtOAc. The organic phase was washed by water and saturated brine, dried over anhydrous sodium sulfate, filtered and concentrated. The residue was purified by preparative-HPLC, the title compound was obtained (29.7 mg, 40%), which was in form of white solid. LCMS (ESI) $[M+H]^+$ = 509.8; $^1H$ NMR (400 MHz, DMSO-$d_6$) $\delta$ 8.18 (t, *J* = 5.2 Hz, 1H). 8.02 (s, 1H). 7.73 (s, 1H). 7.50 (d, *J* = 7.2 Hz, 1H). 7.48-7.42 (m, 2H). 7.33 (d, *J* = 8.0 Hz, 1H). 7.25-7.21 (m, 2H). 6.40 (s, 1H). 4.68-4.54 (m, 2H). 4.24 (t, *J* = 7.2 Hz, 1H). 3.49 (s, 3H). 3.18-3.07 (m, 4H). 2.96-2.50 (m, 2H). 1.27 (t, *J* = 7.2 Hz, 3H). 1.03 (t, *J* = 7.2 Hz, 3H).

**Embodiment 4067: (*S*)-2-(10-((ethylsulfonyl)methyl)-7-(4-flurophenyl)-2-methyl-3-oxo-3,5-dihydro-2*H*-benzo[*c*] pyrido[3,4-*e*]azepin-5-yl)acetic acid**

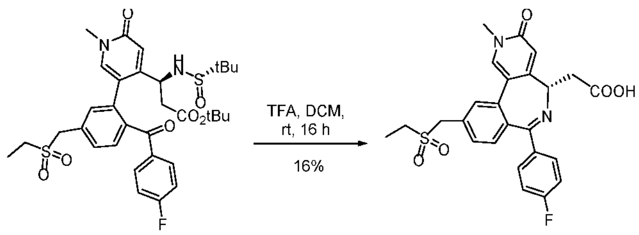


**4056A** **4067**

TFA (1 mL) was added into (*S*)-3-(((*S*)-tert-butyl-sulfinyl)amino)-3-(5-(5-((ethylsulfonyl)methyl)-2-(4-fluorobenzoyl)phenyl)-1-methyl-2-oxo-1,2-dihydropyridin-4-yl) tert-butylpropionate (65 mg, 0.1 mmol) in dichloromethane (2 mL) solution, the reaction mixture was stirred at r.t. overnight then concentrated. The residue was purified by preparative-HPLC, the title compound was obtained (7.9 mg, 16%), which was in form of white solid. LCMS (ESI) $[M+H]^+$ = 483.2; $^1H$ NMR (400 MHz, DMSO-$d_6$) $\delta$ 8.04 (s, 1H). 7.74 (s, 1H). 7.52-7.50 (d, *J* = 8.0 Hz, 1H). 7.46-7.43 (m, 2H). 7.35-7.33 (d, *J* = 8.0 Hz, 1H). 7.25-7.21 (m, 2H). 6.37 (s, 1H). 4.88-4.54 (m, 2H). 4.21-4.17 (m, 1H). 3.49 (s, 3H). 3.19-3.10 (m, 4H). 1.27 (t, *J* = 7.2 Hz, 3H).

**Embodiment 4076: (*S*)-2-(10-((ethylsulfonyl)methyl)-7-(4-flurophenyl)-2-methyl-3-oxo-3,5-dihydro-2*H*-benzo[*c*] pyrido[3,4-*e*]azepin-5-yl)acetamide**

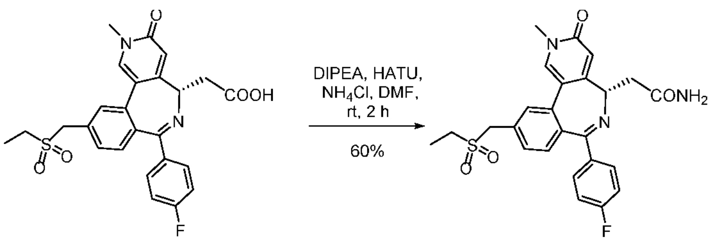


4067 4076

(*S*)-2-(10-((ethylsulfonyl)methyl)-7-(4-flurophenyl)-2-methyl-3-oxo-3,5-dihydro-2*H*-benzo[*c*]pyrido[3,4-*e*]azepin-5-yl)acetic acid (15 mg, 0.03 mmol), 2-(7-azobenzotriazole)-N,N,N',N'-tetramethylurea hexafluorophosphate (16 mg, 0.04 mmol), ammonium chloride (3 mg, 0.04 mmol) and N,N-diisopropyl ethylamine (8 mg, 0.06 mmol) were mixed in N,N-dimethylformamide (5 mL), then stirred at r.t. for 2 hours. The reaction mixture was filtered and the filtrate was concentrated, the residue was purified by preparative-HPLC, the title compound was obtained (9 mg, 60%), which was in form of white solid. LCMS (ESI) $[M+H]^+$ = 482.1; $^1H$ NMR (400 MHz, DMSO-$d_6$) $\delta$8.02 (s, 1H). 7.73 (s, 1H). 7.62 (s, 1H). 7.51-7.45 (m, 3H). 7.46 (s, 4H). 7.34-7.32 (d, *J* = 8.0 Hz, 1H). 7.25-7.21 (t, *J* = 8.8 Hz, 2H). 6.93 (s, 1H). 6.42 (s, 1H). 4.67-4.64 (d, *J* = 12.8 Hz, 1H). 4.58-4.54 (d, *J* = 14 Hz, 1H). 4.24-4.23 (d, *J* = 7.2 Hz, 2H). 3.49 (s, 3H). 3.18-3.12 (m, 2H). 2.97-2.95 (d, *J* = 6.8 Hz, 2H). 1.29-1.25 (t, *J* = 7.6 Hz, 3H).

**Embodiment 4057: (*S*)-10-((ethylsulfonyl)methyl)-7-(4-flurophenyl)-9-methoxyl-2,5-dimethyl-2,5-dihydro-3*H*-benzo[*c*]pyrido[3,4-*e*]azepin-3-ketone**

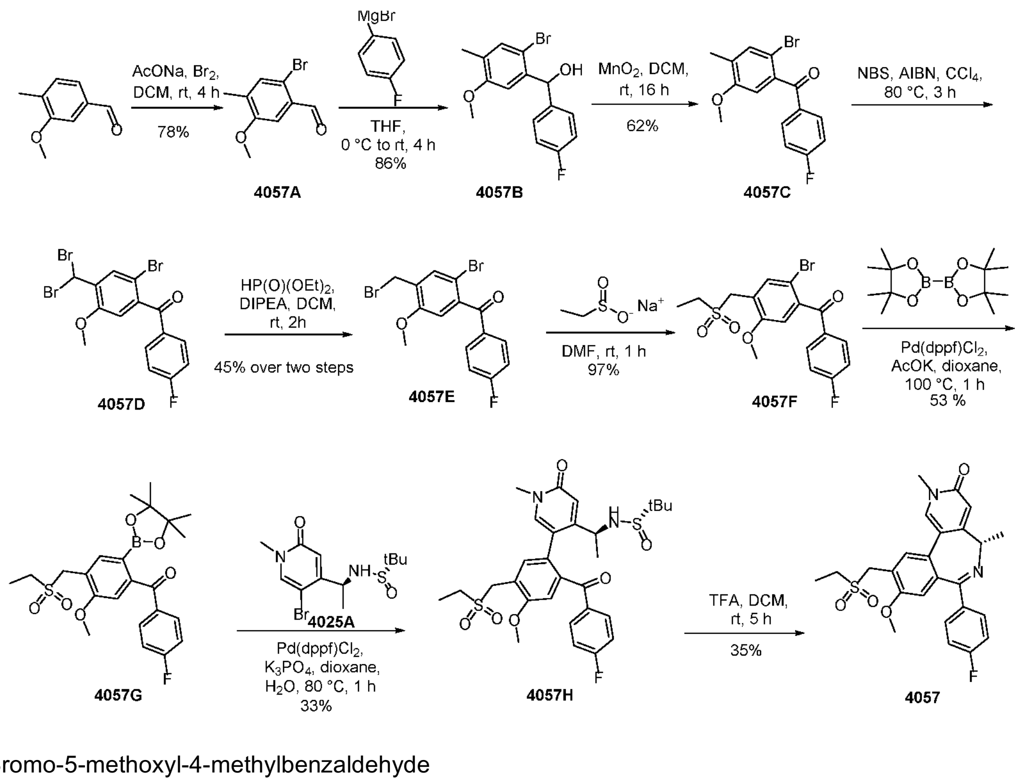


Step 1:2-Bromo-5-methoxyl-4-methylbenzaldehyde

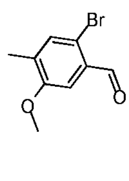

Bromine (3.84 g, 24 mmol) was added into 3-methoxyl-4-methylbenzaldehyde (3.0 g, 20 mmol) and sodium acetate (2.46 g, 30 mmol) in dichloromethane (30 mL) solution. The reaction mixture was stirred at rt for 4 hours and concentrated, the residue was purified by flash chromatography (PE/EtOAc=2/1). The title compound was obtained (3.6 g, 78%). LCMS (ESI) $[M+H]^+$ = 230.9.

Step 2: (2-Bromo-5-methoxyl-4-methylphenyl)(4-flurophenyl)methanol

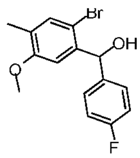

At 0°C and under nitrogen protection, (4-flurophenyl)magnesium bromide (1 M in THF solution, 50 mL) were added into 2-bromo-5-methoxyl-4-methylbenzaldehyde (2.1 g, 9 mmol) in THF (30 mL) solution. The reaction mixture was raised to r.t. and stirred for 4 hours, the reaction was quenched by saturated ammonium chloride (30 mL), and extracted by EtOAc (100 mL). The organic phase was washed by saturated brine (300 mL), dried over anhydrous sodium sulfate, filtered and concentrated. The residue was purified by flash chromatography (EtOAc/PE=5/1). The title compound was obtained (2.5 g, 86%), which was in form of yellow solid. LCMS (ESI) $[M+H]^+$ = 306.9.

Step 3: (2-Bromo-5-methoxyl-4-methylphenyl)(4-flurophenyl)methanone

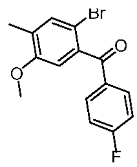

(2-Bromo-5-methoxyl-4-methylphenyl)(4-flurophenyl)methanol (1.8 g, 5.5 mmol) and manganese dioxide (2.4 g, 27.5 mmol) were mixed in dichloromethane (20 mL), was stirred at r.t. for 16 hours. The reaction mixture was filtered and the filtrate was concentrated, the residue was purified by flash chromatography (PE/EtOAc=2/1). The title compound was obtained (1.1 g, 62%), in form of yellow oil. LCMS (ESI) $[M+H]^+$ = 304.8.

Step 4: (2-Bromo-4-(dibromomethyl)-5-methoxylphenyl)(4-flurophenyl)methanone

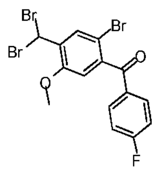

*N*-Bromosuccinimide (2.9 g, 10 mmol), azobisisobutyronitrile (164 mg, 1 mmol) and (2-bromo-5-methoxyl-4-methylphenyl)(4-flurophenyl)methanone (5.34 g, 30 mmol) were mixed in tetrachloromethane (25 mL), the reaction mixture was heated to 80°C and stirred for 3 hours. The reaction mixture was concentrated and the curde product of the tiltle compound was obtained (4.9 g), which was in form of yellow solid, and was used directly in the next step without purification. LCMS (ESI) $[M+H]^+$ = 482.6.

Step 5: (2-Bromo-4-(bromomethyl)-5-methoxylphenyl)(4-flurophenyl)methanone

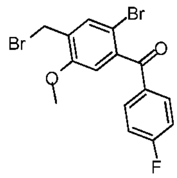

Diethyl phosphite (940 mg, 6.8 mmol), diisopropyl ethylamine (880 mg, 6.8 mmol) and (2-bromo-4-(dibromomethyl)-5-methoxylphenyl)(4-flurophenyl)methanone (1.63 g, 3.4 mmol) was dissolved in dichloromethane (20 mL), the reaction mixture then stirred at r.t. for 2 hours. Concentration of the reaction mixture, the residue was purified by flash chromatography (PE/EtOAc=4/1). The title compound was obtained (1.0 g, yield of the two steps 45%), which was in form of yellow solid. LCMS (ESI) $[M+H]^+$ = 402.7.

Step 6: (2-Bromo-4-((ethylsulfonyl)methyl)-5-methoxylphenyl)(4-flurophenyl)methanone

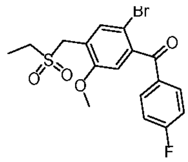

(2-Bromo-4-(bromomethyl)-5-methoxylphenyl)(4-flurophenyl)methanone (1.0 g, 2.49 mmol) and sodium ethylsulfinate (580 mg, 4.98 mmol) was dissolved in dimethylsulfoxide (10 mL), the reaction mixture then stirred at r.t. for 1 hour. EtOAc (30 mL) and water (100 mL) were added into the reaction mixture, the organic phase was separated. The organic phase was washed by saturated brine (30 mL*3), dried over anhydrous sodium sulfate, filtered and concentrated, the crude product of the tiltle compound was obtained (1.0 g, 97%), which was in form of yellow solid. LCMS (ESI) $[M+H]^+$ = 417.0.

Step 7: (4-((ethylsulfonyl)methyl)-5-methoxyl-2-(4,4,5,5-tetramethyl-1,3,2-dioxaborolan-2-yl)phenyl)(4-f lurophenyl)methanone

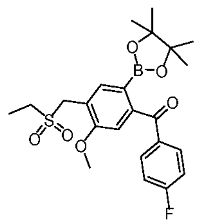

(2-bromo-4-((ethylsulfonyl)methyl)-5-methoxylphenyl)(4-flurophenyl)methanone (600 mg, 1.45 mmol), bis(pinacolato)diboron (737 mg, 2.9 mmol), [1,1'-bis(diphenylphosphino)ferrocene]palladium (II) dichloride (220 mg, 0.29 mmol) and potassium acetate (427 mg, 4.35 mmol) were mixed in 1,4-dioxane (10 mL), under nitrogen protection, the reaction mixture was heated to 100°C and stirred for 1 hour. The reaction mixture was cooled down to r.t. and then filtered and the filtrate was concentrated. The residue was purified by flash chromatography (PE/EtOAc=4/1). The title compound was obtained (320 mg, 53%), which was in form of yellow solid. LCMS (ESI) $[M+H]^+$ = 463.0.

Step 8: (*S*)-*N*-((*S*)-1-(5-(5-((ethylsulfonyl)methyl)-2-(4-fluorobenzoyl)-4-methoxylphenyl)-1-methyl-2-ox o-1,2-dihydropyridin-4-yl)ethyl)-2-methylpropane-2-sulfenamide

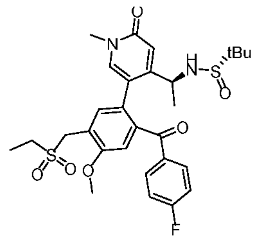

(4-((ethylsulfonyl)methyl)-5-methoxyl-2-(4,4,5,5-tetramethyl-1,3,2-dioxaborolan-2-yl)phenyl)(4-flurophenyl)methanone (160 mg, 0.35 mmol), (*S*)-*N*-((*S*)-1-(5-bromo-1-methyl-2-oxo-1,2-dihydropyridin-4-yl)ethyl)-2-methylpropane-2-sulfena mide (78 mg, 0.23 mmol), [1,1'-bis(diphenylphosphino)ferrocene]palladium (II) dichloride (34 mg, 0.046 mmol) and potassium phosphate (146 mg, 0.69 mmol) were mixed in 1,4-dioxane (2 mL) and water (0.2 mL), under nitrogen protection, the reaction mixture was heated to 80°C then stirred for 1 hour. The reaction mixture was cooled down to r.t. and then filtered and the filtrate was concentrated, the residue was purified by flash chromatography (EtOAc/methanol=15/1). The title compound was obtained (68 mg, 33%), in form of yellow oil. LCMS (ESI) $[M+H]^+$ = 591.0.

Step 9: (*S*)-10-((ethylsulfonyl)methyl)-7-(4-flurophenyl)-9-methoxyl-2,5-dimethyl-2,5-dihydro-3*H*-benzo [*c*]pyrido[3,4-*e*]azepin-3-ketone

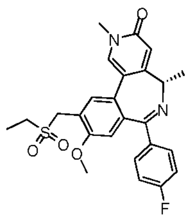

TFA (1 mL) was added into (*S*)-*N*-((*S*)-1-(5-(5-((ethylsulfonyl)methyl)-2-(4-fluorobenzoyl)-4-methoxylphenyl)-1-methyl-2-oxo-1,2-dihydropyridin-4-yl)ethyl)-2-methylpropane-2-sulfenamide (68 mg, 0.115 mmol) in dichloromethane (4 mL). The reaction mixture was stirred at r.t. for 5 hours and then concentrated. The residue was purified by preparative-HPLC, the title compound was obtained (19 mg, 35%), which was in form of white solid. LCMS (ESI) $[M+H]^+$ = 468.8; $^1$H NMR (400 MHz, DMSO-$d_6$) $\delta$ 7.94 (s, 1H). 7.88 (s, 1H). 7.56-7.52 (m, 2H). 7.25-7.20 (m, 2H). 6.90 (s, 1H). 6.40 (s, 1H). 4.63-4.59 (m, 1H). 4.42-4.39 (m, 1H). 3.98-3.96 (m, 1H). 3.74 (s, 3H). 3.49 (s, 3H). 3.12-3.09 (m, 2H). 1.66-1.64 (d, *J* = 6.4 Hz, 3H). 1.28-1.24 (t, *J* = 7.6 Hz, 3H).

**Embodiment 4063: (*S*)-10-((ethylsulfonyl)methyl)-7-(4-flurophenyl)-9-hydroxyl-2,5-dimethyl-2,5-dihydro-3*H*-benzo[*c*]pyrido[3,4-*e*]azepin-3-ketone**

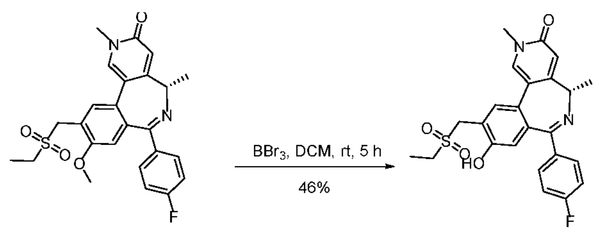


4067 4063

The mixture of (*S*)-10-((ethylsulfonyl)methyl)-7-(4-flurophenyl)-9-methoxyl-2,5-dimethyl-2,5-dihydro-3*H*-benzo [*c*]pyrido[3,4-*e*]azepin-3-ketone (10 mg, 0.021 mmol) and tribromo boron (17% in dichloromethane solution, 1 mL) in was stirred at r.t. for 5 hours. Concentration of the reaction mixture, the residue was purified by preparative-HPLC, the title compound was obtained (4.5 mg, 46%), which was in form of white solid. LCMS (ESI) $[M+H]^+$ = 455.2; $^1$H NMR (400 MHz, $CD_3OD$) $\delta$ 8.00 (s, 1H). 7.67 (s, 1H). 7.54-7.51 (m, 2H). 7.17-7.12 (m, 3H). 6.79 (s, 1H). 6.62 (s, 1H). 4.56 (s, 2H). 4.14 (q, 1H). 3.67 (s, 3H). 3.15 (q, 2H). 1.77 (d, *J* = 6.4 Hz, 3H). 1.42 (t, 3H).

**Embodiment 4058: (*S*)-*N*-(9-chloro-7-(4-flurophenyl)-2,5-dimethyl-3-oxo-3,5-dihydro-2*H*-benzo[*c*]pyrido[3,4-*e*]azepin-10-yl)ethylsulfamide**

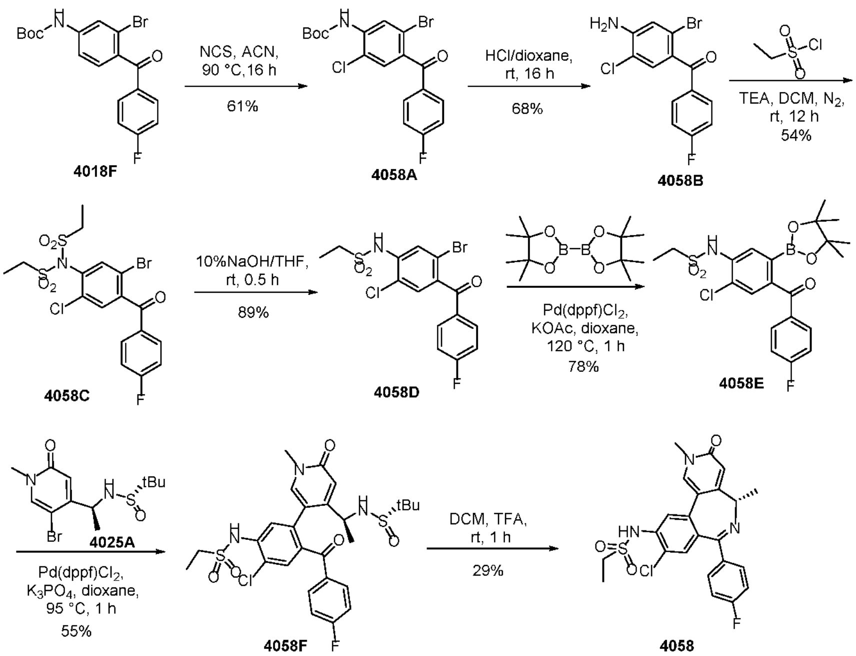


Step 1: (5-Bromo-2-chloro-4-(4-fluorobenzoyl)phenyl) tert-butylcarbamate

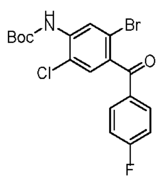

(3-Bromo-4-(4-fluorobenzoyl)phenyl) tert-butylcarbamate (6 g, 15.2 mmol) and *N*-chlorosuccinimide (6.1 g, 45.6 mmol) was dissolved in acetonitrile (50 mL), and heated to 90°C and stirred overnight. Concentration of the reaction mixture, the residue was purified by preparative-HPLC, the title compound was obtained (4 g, 61%), which was in form of yellow solid. LCMS (ESI) $[M+H]^+$ = 428.0.

Step 2: (4-Amino-2-bromo-5-chlorophenyl)(4-flurophenyl)methanone

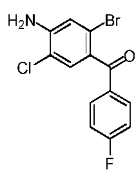

(5-Bromo-2-chloro-4-(4-fluorobenzoyl)phenyl) tert-butylcarbamate (4 g, 9.4 mmol) and HCl (4 M in 1,4-dioxane solution, 20 mL) in the mixture then stirred at r.t. overnight. Concentration of the reaction mixture, the residue was purified by preparative-HPLC, the title compound was obtained (2.1 g, 68%), which was in form of white solid. LCMS (ESI) $[M+H]^+$ = 328.0.

Step 3: *N*-(5-bromo-2-chloro-4-(4-fluorobenzoyl)phenyl)-*N*-(ethylsulfonyl)ethylsulfamide

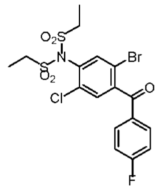

(4-Amino-2-bromo-5-chlorophenyl)(4-flurophenyl)methanone (2.1 g, 6.4 mmol), ethylsulfonyl chloride (8.2 g, 64.0 mmol) and triethylamine (6.46 g, 64.0 mmol) was dissolved in dichloromethane (30 mL), and was stirred at r.t. under nitrogen protection for 12 hours. The reaction mixture was concentrated and the tiltle compound was obtained (1.3 g, 54%), which was in form of yellow solid. LCMS (ESI) $[M+H]^+$ = 530.5.

Step 4:*N*-(5-bromo-2-chloro-4-(4-fluorobenzoyl)phenyl)ethylsulfamide

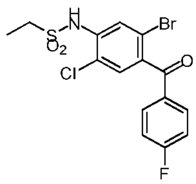

10% NaOH aqueous solution (5 mL) was slowly added dropwise into *N*-(5-bromo-2-chloro-4-(4-fluorobenzoyl)phenyl)-*N*-(ethylsulfonyl)ethylsulfamide (1.3 g, 2.5 mmol) in THF (5 mL) solution, and stirred at r.t. for 0.5 hour. EtOAc (10 mL) and water (10 mL) was added into the reaction mixture, the organic phase was separated. The organic phase was washed by saturated brine (10 mL*3), dried over anhydrous sodium sulfate, filtered and concentrated. The residue was purified by flash chromatography (PE/EtOAc=2/1). The title compound was obtained (800 mg, 89%), which was in form of yellow solid. LCMS (ESI) $[M+H]^+$ = 419.6.

Step 5: *N*-(2-chloro-4-(4-fluorobenzoyl)-5-(4,4,5,5-tetramethyl-1,3,2-dioxaborolan-2-yl)phenyl)ethylsulfa mide

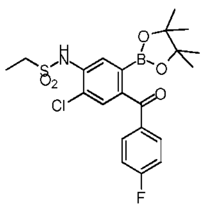

Compound *N*-(5-bromo-2-chloro-4-(4-fluorobenzoyl)phenyl)ethylsulfamide (138 mg, 0.33 mmol), bis(pinacolato)diboron (167 mg, 0.66 mmol), [1,1'-bis(diphenylphosphino)ferrocene]palladium (II) dichloride (24 mg, 0.033 mmol) and potassium acetate (97 mg, 0.99 mmol) were mixed in 1,4-dioxane (4 mL), and heated to 120°C then stirred for 1 hour under nitrogen protection. The reaction mixture was filtered and the filtrate was concentrated, the residue was purified by flash chromatography (100% EtOAc). The title compound was obtained (120 mg, 78%), in form of yellow oil. LCMS (ESI) $[M+H]^+$ = 468.0.

Step 6: *N*-(5-(4-((*S*)-1-(((*S*)-tert-butyl-sulfinyl)amino)ethyl)-1-methyl-6-oxo-1,6-dihydropyridin-3-yl)-2-c hloro-4-(4-fluorobenzenesulfonyl)phenyl)ethylsulfonamide

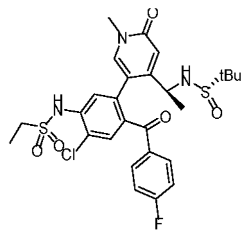

Compound *N*-(2-chloro-4-(4-fluorobenzoyl)-5-(4,4,5,5-tetramethyl-1,3,2-dioxaborolan-2-yl)phenyl)ethylsulfa mide (120 mg, 0.26 mmol), (*S*)-*N*-((*S*)-1-(5-bromo-1-methyl-2-oxo-1 ,2-dihydropyridin-4-yl)ethyl)-2-methylpropane-2-sulfena mide (87 mg, 0.26 mmol), [1,1'-bis(diphenylphosphino)ferrocene]palladium (II) dichloride (19 mg, 0.026 mmol) and potassium phosphate (165 mg, 0.78 mmol) were mixed in 1,4-dioxane (4 mL) and water (0.4 mL), and heated to 95°C then stirred for 1 hour under nitrogen protection. The reaction mixture was filtered and the filtrate was concentrated, the residue was purified by flash chromatography (EtOAc/methanol=30/1). The title compound was obtained (84 mg, 55%), in form of yellow oil. LCMS (ESI) $[M+H]^+$ = 596.2.

Step 7: (*S*)-*N*-(9-chloro-7-(4-flurophenyl)-2,5-dimethyl-3-oxo-3,5-dihydro-2*H*-benzo[*c*]pyrido[3,4-*e*]azepi n-10-yl)ethylsulfamide

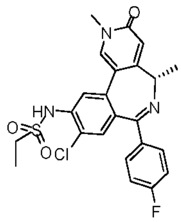

TFA (1 mL) was added into *N*-(5-(4-((*S*)-1-(((*S*)-tert-butyl-sulfinyl)amino)ethyl)-1-methyl-6-oxo-1,6-dihydropyridin-3-yl)-2-c hloro-4-(4-fluorobenzenesulfonyl)phenyl)ethylsulfonamide (84 mg, 0.14 mmol) in dichloromethane (4 mL) solution. The reaction mixture was stirred at r.t. for 1 hour, then concentrated. The residue was purified by preparative-HPLC, the title compound was obtained (19.4 mg, 29%), which was in form of white solid. LCMS (ESI) $[M+H]^+$ = 473.2; $^1H$ NMR (400 MHz, DMSO-$d_6$) $\delta$ 8.05 (s, 1H). 7.67 (s, 1H). 7.47-7.50 (dd, *J* = 2.8 Hz, 5.6 Hz, 2H). 7.33 (s, 1H). 7.25-7.20 (t, *J* = 8.8 Hz, 2H). 3.96-4.01 (q, *J* = 6.4 Hz, 1H). 3.49 (s, 3H). 3.23-3.28 (q, *J* = 7.6 Hz, 6.4 Hz, 2H). 1.63-1.65 (d, *J* = 6.4 Hz, 3H). 1.29-1.32 (t, *J* = 7.2 Hz, 3H).

**Embodiment 4059: (*S*)-7-(4-flurophenyl)-2,5-dimethyl-10-((dimethylfulfone)methyl)-2,5-dihydro-3*H*-benzo[*c*]pyrido[3,4-*e*]azepin-3-ketone**

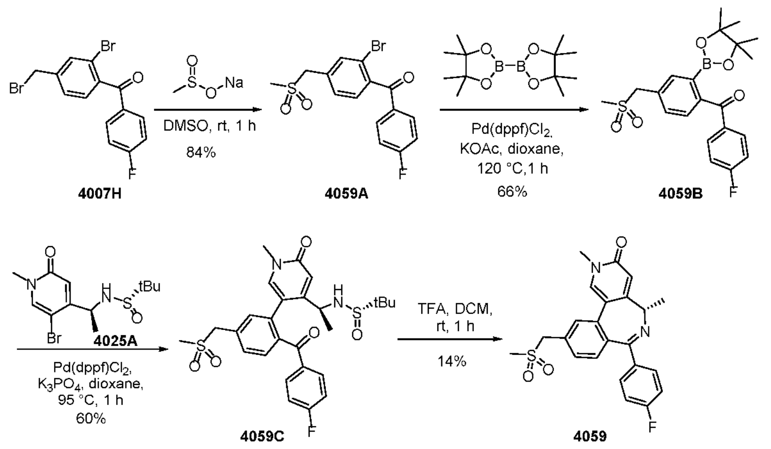


4007H 4059A 4059B 4059C 4059

Step 1: (2-Bromo-4-((dimethylfulfone)methyl)phenyl)(4-flurophenyl)methanone

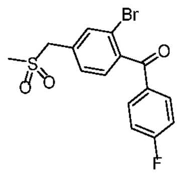

(2-Bromo-4-(bromomethyl)phenyl)(4-flurophenyl)methanone (500 mg, 1.34 mmol) and sodium methanesulphinate (274 mg, 2.69 mmol) was dissolved in dimethylsulfoxide (5 mL), then stirred at r.t. for 1 hour. EtOAc (10 mL) and water (10 mL) was added into the reaction mixture, the organic phase was separated. The organic phase was washed by saturated brine (10 mL*3), dried over anhydrous sodium sulfate, filtered and concentrated. The residue was purified by flash chromatography (PE/EtOAc=2/1). The title compound was obtained (420 mg, 84%), which was in form of yellow solid. LCMS (ESI) $[M+H]^+$ = 370.8.

Step 2: (4-Flurophenyl)(4-((dimethylfulfone)methyl)-2-(4,4,5,5-tetramethyl-1,3,2-dioxaborolan-2-yl)phen yl)methanone

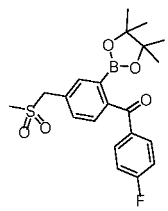

Compound (2-bromo-4-((dimethylfulfone)methyl)phenyl)(4-flurophenyl)methanone (150 mg, 0.40 mmol), bis(pinacolato)diboron (203 mg, 0.80 mmol), [1,1'-bis(diphenylphosphino)ferrocene]palladium (II) dichloride (30 mg, 0.04 mmol) and potassium acetate (117 mg, 1.2 mmol) were mixed in 1,4-dioxane (4 mL), and heated to 120°C then stirred for 1 hour under nitrogen protection. The reaction mixture was filtered and the filtrate was concentrated, the residue was purified by flash chromatography (PE/EtOAc=1/1). The title compound was obtained (112 mg, 66%), in form of yellow oil. LCMS (ESI) $[M+H]^+$ = 419.1.

Step 3: (*S*)-*N*-((*S*)-1-(5-(2-(4-fluorobenzoyl)-5-((dimethylfulfone)methyl)phenyl)-1-methyl-2-oxo-1,2-dih ydropyridin-4-yl)ethyl)-2-methylpropane-2-sulfenamide

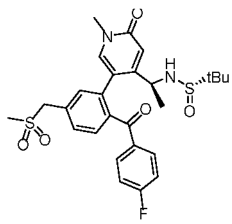

Compound (4-flurophenyl)(4-((dimethylfulfone)methyl)-2-(4,4,5,5-tetramethyl-1,3,2-dioxaborolan-2-yl)phenyl)methanone (112 mg, 0.27 mmol), (*S*)-*N*-((*S*)-1-(5-bromo-1-methyl-2-oxo-1,2-dihydropyridin-4-yl)ethyl)-2-methylpropane-2-sulfena mide (87 mg, 0.26 mmol), [1,1'-bis(diphenylphosphino)ferrocene]palladium (II) dichloride (19 mg, 0.026 mmol) and potassium phosphate (165 mg, 0.78 mmol) were mixed in 1,4-dioxane (4 mL) and water (0.4 mL), and heated to 95°C then stirred for 1 hour under nitrogen protection. The reaction mixture was filtered and the filtrate was concentrated, the residue was purified by flash chromatography (EtOAc/methanol=30/1). The title compound was obtained (87 mg, 60%), in form of yellow oil. LCMS (ESI) $[M+H]^+$ = 547.2.

Step 4: (*S*)-7-(4-flurophenyl)-2,5-dimethyl-10-((dimethylfulfone)methyl)-2,5-dihydro-3*H*-benzo[*c*]pyrido[ 3,4-*e*]azepin-3-ketone

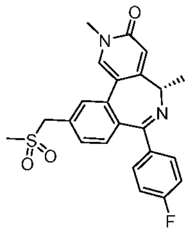

TFA (1 mL) was added into (*S*)-*N*-((*S*)-1-(5-(2-(4-fluorobenzoyl)-5-((dimethylfulfone)methyl)phenyl)-1-methyl-2-oxo-1,2-dih ydropyridin-4-yl)ethyl)-2-methylpropane-2-sulfenamide (87 mg, 0.16 mmol) in dichloromethane (4 mL) solution. The reaction mixture was stirred at r.t. for 1 hour then concentrated concentration, the residue was purified by preparative-HPLC, the title compound was obtained (9.8 mg, 14%), which was in form of white solid. LCMS (ESI) $[M+H]^+$ = 425.2; $^1H$ NMR (400 MHz, DMSO-$d_6$) $\delta$ 8.03 (s, 1H). 7.71 (s, 1H). 7.46-7.49 (m, 3H). 7.32-7.35 (d, *J* = 8.4 Hz, 1H). 7.20-7.24 (m, 3H). 6.41 (s, 1H). 4.55-4.68 (q, *J* = 26.4 Hz, 13.6 Hz, 2H). 3.94-3.99 (q, *J* = 6.4 Hz, 1H). 3.49 (s, 3H). 3.00 (s, 3H). 1.64-1.66 (d, *J* = 6.4 Hz, 3H).

**Embodiment 4060: (*S*)-10-((ethylsulfonyl)methyl)-7-(4-flurophenyl)-5- 或 propyl-2-methyl-2,5-dihydro-3*H*-benzo[*c*]pyrido[3,4-*e*]azepin-3-ketone**

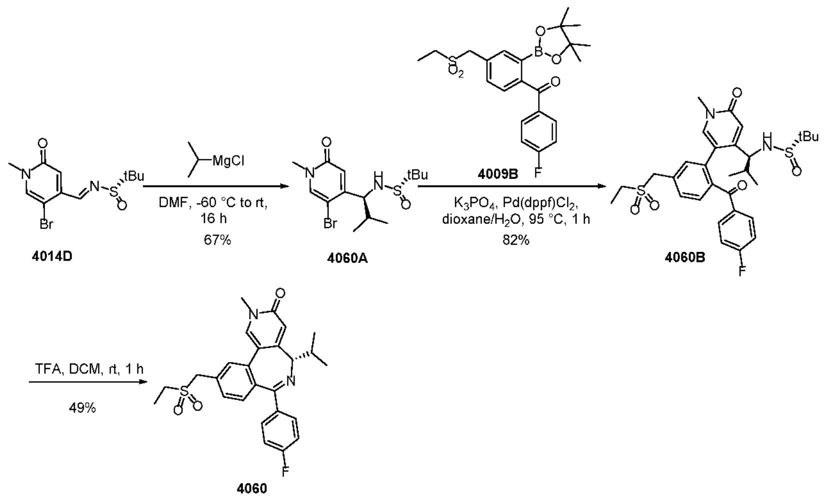


Step 1: (*S*)-*N*-((*S*)-1-(5-bromo-1-methyl-2-oxo-1,2-dihydropyridin-4-yl)-2-methylpropyl)-2-methylpropan e-2-sulfenamide

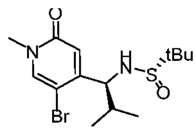

At -60°C and under nitrogen protection, isopropyl magnesium chloride (2 M in THF solution, 0.32 mL) was slowly added dropwise into (*S*,*E*)-*N*-((5-bromo-1-methyl-2-oxo-1,2-dihydropyridin-4-yl)methyl)-2-methylpropane-2-sulfenam ide (50 mg, 0.16 mmol) in THF (5 mL) solution, then continued stirring for 0.5 hours at this temperature, then the reaction mixture was raised to r.t. and stirred overnight. Water (10 mL) was added into the reaction mixture, then extracted by EtOAc (10 mL). The organic phase was washed by saturated brine (10 mL*3), dried over anhydrous sodium sulfate, filtered and concentrated. The residue was purified by flash chromatography (EtOAc). The title compound was obtained (38 mg, 67%) in form of white solid. LCMS (ESI) $[M+H]^+$ = 363.0.

Step 2: (*S*)-*N*-((*S*)-1-(5-(5-((ethylsulfonyl)methyl)-2-(4-fluorobenzoyl)phenyl)-1-methyl-2-oxo-1,2-dihydr opyridin-4-yl)-2-methylpropyl)-2-methylpropane-2-sulfenamide

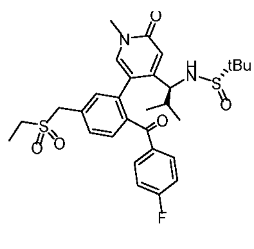

(*S*)-*N*-((*S*)-1-(5-bromo-1-methyl-2-oxo-1,2-dihydropyridin-4-yl)-2-methylpropyl)-2-met hylpropane-2-sulfenamide (38 mg, 0.10 mmol), (4-((ethylsulfonyl)methyl)-2-(4,4,5,5-tetramethyl-1,3,2-dioxaborolan-2-yl)phenyl)(4-flurophenyl) methanone (90 mg, 0.2 mmol), [1,1'-bis(diphenylphosphino)ferrocene]palladium (II) dichloride (7 mg, 0.01 mmol) and potassium phosphate (64 mg, 0.3 mmol) were mixed in 1,4-dioxane (4 mL) and water (0.4 mL), and heated to 95°C then stirred for 1 hour under nitrogen protection. The reaction mixture was filtered and the filtrate was concentrated, the residue was purified by flash chromatography (EtOAc/methanol=30/1). The title compound was obtained (51 mg, 82%), in form of yellow oil. LCMS (ESI) $[M+H]^+$ = 589.2.

Step 3: (*S*)-10-((ethylsulfonyl)methyl)-7-(4-flurophenyl)-5-isopropyl-2-methyl-2,5-dihydro-3*H*-benzo[*c*]p yrido[3,4-*e*]azepin-3-ketone

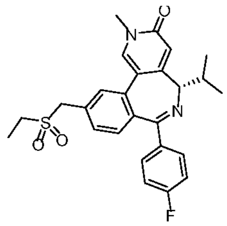

TFA (1 mL) was added into (*S*)-*N*-((*S*)-1-(5-(5-((ethylsulfonyl)methyl)-2-(4-fluorobenzoyl)phenyl)-1-methyl-2-oxo-1,2-dihydr opyridin-4-yl)-2-methylpropyl)-2-methylpropane-2-sulfenamide (51 mg, 0.087 mmol) in dichloromethane (4 mL) solution. The reaction mixture was stirred at r.t. for 1 hour then concentrated. The residue was purified by preparative-HPLC, the title compound was obtained (20.0 mg, 49%), which was in form of white solid. LCMS (ESI) $[M+H]^+$ = 467.1; $^1H$ NMR (400 MHz, DMSO-$d_6$) $\delta$ 8.01 (s, 1H). 7.73-7.73 (d, *J* = 1.6 Hz, 1H). 7.46-7.50 (m, 3H). 7.34-7.36 (d, *J* = 8.0 Hz, 1H). 7.25-7.20 (m, 2H). 4.55-4.67 (q, *J* = 13.2 Hz, 20.8 Hz, 2H). 3.49 (s, 3H). 3.26-3.23 (d, *J* = 10.0 Hz, 1H). 3.14-3.20 (q, *J* = 7.2 Hz, 3H). 2.63-2.66 (m, 1H). 1.26-1.29 (t, *J* = 7.6 Hz, 3H). 1.11-1.13 (d, *J* = 6.4 Hz, 3H). 0.91-0.93 (d, *J* = 6.8 Hz, 3H).

**Embodiment 4062: (*S*)-*N*-(7-(4-chlorophenyl)-9-fluoro-2,5-dimethyl-3-oxo-3,5-dihydro-2*H*-benzo[*c*]pyrido[3,4-*e*] azepin-10-yl)ethylsulfonamide**

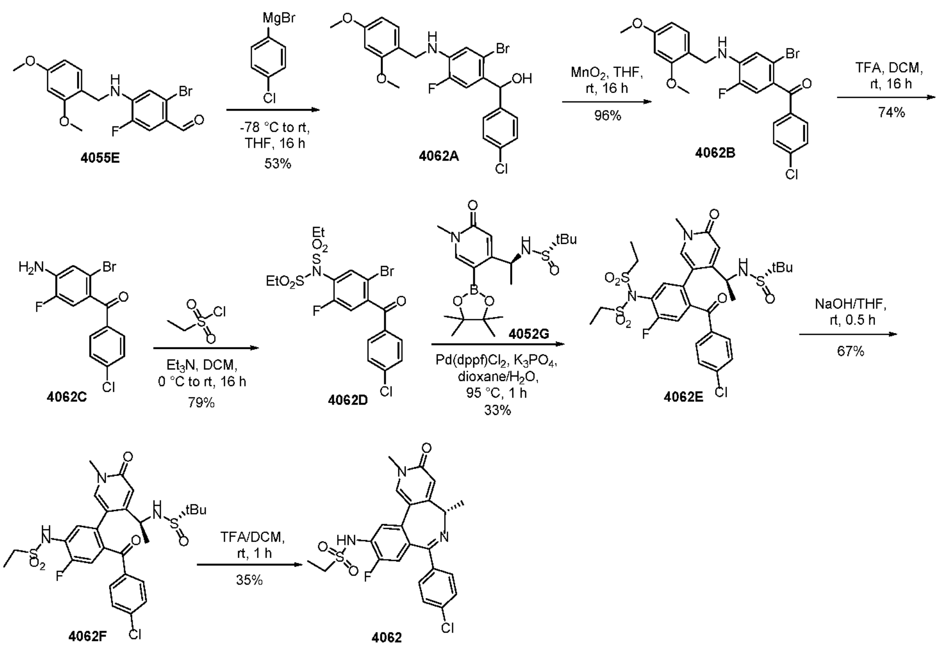


Step 1: (2-bromo-4-((2,4-dimethoxylbenzyl)amino)-5-flurophenyl)(4-chlorophenyl)methanol

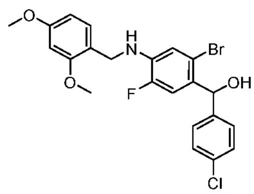

At -78°C and under nitrogen protection, (4-chlorophenyl)magnesium bromide (1 M in THF solution, 27 mL, 27 mmol) was added into 2-bromo-4-((2,4-dimethoxylbenzyl)amino)-5-fluoro benzaldehyde (1 g, 2.7 mmol) in THF (10 mL) solution, the reaction mixture was raised to r.t. and stirred overnight. Ammonium chloride (1 g) was added into the reaction mixture, then filtered and the filtrate was concentrated, the residue was purified by flash chromatography (PE/EtOAc=5/1). The title compound was obtained (690 mg, 53%), which was in form of yellow solid.

Step 2: (2-Bromo-4-((2,4-dimethoxylbenzyl)amino)-5-flurophenyl)(4-chlorophenyl)methanone

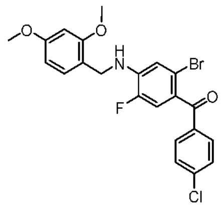

(2-Bromo-4-((2,4-dimethoxybenzyl)amino)-5-flurophenyl)(4-chlorophenyl)methanol (690 mg, 1.44 mmol) and manganese dioxide (1.23 g, 14.4 mmol) were mixed in THF (20 mL), then stirred at r.t. overnight, the reaction mixture was filtered and the filtrate was concentrated, the residue was purified by flash chromatography (PE/EtOAc=10/1). The title compound was obtained (660 mg, 96%), which was in form of yellow solid.

Step 3: (4-Amino-2-bromo-5-flurophenyl)(4-chlorophenyl)methanone

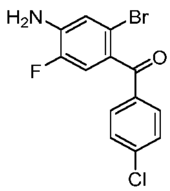

(2-Bromo-4-((2,4-dimethoxylbenzyl)amino)-5-flurophenyl)(4-chlorophenyl)methanone (660 mg, 1.38 mmol) and TFA (1.57 g, 13.8 mmol) was dissolved in dichloromethane (10 mL), the reaction mixture was stirred at r.t. overnight. Concentration of the reaction mixture, the residue was purified by flash chromatography (PE/EtOAc=5/1). The title compound was obtained (335 mg, 74%), which was in form of yellow solid. LCMS (ESI) $[M+H]^+$ = 327.8.

Step 4: *N*-(5-bromo-4-(4-chlorobenzoyl)-2-flurophenyl)-*N*-(ethylsulfonyl)ethylsulfonamide

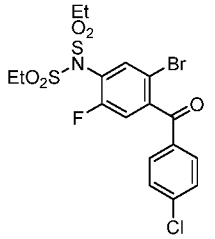

At 0°C, ethylsulfonyl chloride (1.41 g, 10.2 mmol) was added into (4-amino-2-bromo-5-flurophenyl)(4-chlorophenyl)methanone (335 mg, 1.02 mmol) and triethylamine (1.03 g, 10.2 mmol) in dichloromethane (5 mL) solution. The reaction mixture was raised to r.t. and stirred overnight, then concentrated. The residue was purified by flash chromatography (PE/EtOAc=1/1). The title compound was obtained (410 mg, 79%), which was in form of yellow solid. LCMS (ESI) $[M+H]^+$ = 511.8.

Step 5: *N*-(5-(4-((*S*)-1-(((*S*)-tert-butyl-sulfinyl)amino)ethyl)-1-methyl-6-oxo-1,6-dihydropyridin-3-yl)-4-( 4-chlorobenzoyl)-2-flurophenyl)-*N*-(ethylsulfonyl)ethylsulfonamide

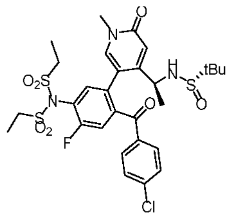

Compound (*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (50 mg, 0.13 mmol), *N*-(5-bromo-4-(4-chlorobenzoyl)-2-flurophenyl)-*N*-(ethylsulfonyl)ethylsulfonamide (55 mg, 0.11 mmol), potassium phosphate (83 mg, 0.39 mmol) and [1,1'-bis(diphenylphosphino)ferrocene]palladium (II) dichloride (10 mg, 0.014 mmol) were mixed in 1,4-dioxane (2 mL) and water (0.2 mL), and heated to 90°C then stirred for 1 hour under nitrogen protection. The reaction mixture was filtered and the filtrate was concentrated, the residue was purified by preparative-TLC (EtOAc/methanol=25/1). The title compound was obtained (30 mg, 33%), in form of yellow oil. LCMS (ESI) $[M+H]^+$ = 687.8.

Step 6: *N*-(5-(4-((*S*)-1-(((*S*)-tert-butyl-sulfinyl)amino)ethyl)-1-methyl-6-oxo-1,6-dihydropyridin-3-yl)-4-( 4-chlorobenzoyl)-2-flurophenyl)ethylsulfonamide

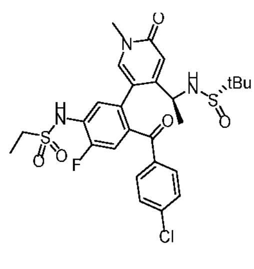

10% NaOH aqueous solution (2 mL) was added into *N*-(5-(4-((*S*)-1-(((S)-tert-butyl-sulfinyl)amino)ethyl)-1-methyl-6-oxo-1,6-dihydropyridin-3-yl)-4-( 4-chlorobenzoyl)-2-flurophenyl)-*N*-(ethylsulfonyl)ethylsulfonamide (30 mg, 0.05 mmol) in THF (2 mL) solution solution. The reaction mixture was continued to be stirred for 0.5 hour. The reaction mixture was added by EtOAc (10 mL) and water (10 mL), the organic phase was separated. The organic phase was washed by saturated brine and water, dried over anhydrous sodium sulfate, filtered and concentrated. The residue was purified by flash chromatography (PE/EtOAc=2/1). The title compound was obtained (20 mg, 67%), which was in form of yellow solid. LCMS (ESI) $[M+H]^+$ = 595.9.

Step 7: (*S*)-*N*-(7-(4-chlorophenyl)-9-fluoro-2,5-dimethyl-3-oxo-3,5-dihydro-2*H*-benzo[c]pyrido[3,4-*e*]aze pin-10-yl)ethylsulfonamide

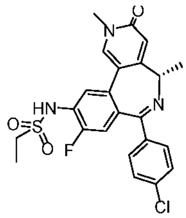

TFA (1 mL) was added into *N*-(5-(4-((*S*)-1-(((*S*)-tert-butyl-sulfinyl)amino)ethyl)-1-methyl-6-oxo-1,6-dihydropyridin-3-yl)-4-( 4-chlorobenzoyl)-2-flurophenyl)ethylsulfonamide (28 mg, 0.05 mmol) in dichloromethane (4 mL) solution. The reaction mixture was stirred at r.t. for 1 hour after concentration, the residue was purified by preparative-HPLC, the title compound was obtained (5.7 mg, 35%), which was in form of white solid. LCMS (ESI) $[M+H]^+$ = 474.1; $^1H$ NMR (400 MHz, DMSO-$d_6$) $\delta$ 8.00 (s, 1H). 7.61-7.59 (d, *J* = 7.6 Hz, 1H). 7.46 (s, 4H). 7.16-7.13 (d, *J* = 10.8 Hz, 1H). 6.3 (s, 1H). 3.48 (s, 1H). 6.51 (s, 1H). 4.01-3.96 (q, *J* = 6.4 Hz, 1H). 3.23-3.19 (q, *J* = 7.6 Hz, 2H). 1.65-1.63 (d, *J* = 6.4 Hz, 3H). 1.30-1.26 (t, *J* = 7.6 Hz, 3H).

**Embodiment 4065: (*S*)-*N*-(9-chloro-5-ethyl-7-(4-flurophenyl)-2-methyl-3-oxo-3,5-dihydro-2*H*-benzo[*c*]pyrido[3,4 -*e*]azepin-10-yl)ethylsulfonamide**

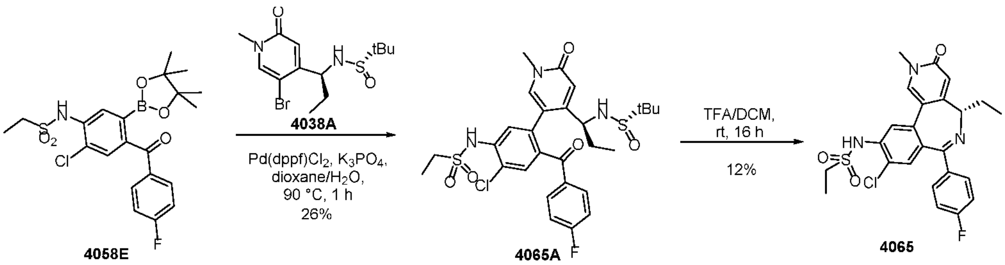


4058E 4065A 4065

Step 1: *N*-(5-(4-((*S*)-1-(((*S*)-tert-butyl-sulfinyl)amino)propyl)-1-methyl-6-oxo-1,6-dihydropyridin-3-yl)-2-chloro-4-(4-fluorobenzoyl)phenyl)ethylsulfonamide

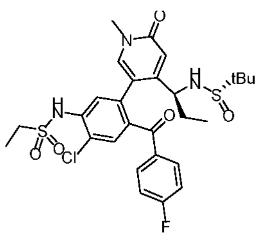

compound *N*-(2-chloro-4-(4-fluorobenzoyl)-5-(4,4,5,5-tetramethyl-1,3,2-dioxaborolan-2-yl)phenyl)ethylsulfo namide (60 mg, 0.13 mmol), (*S*)-*N*-((*S*)-1-(5-bromo-1-methyl-2-oxo-1,2-dihydropyridin-4-yl)propyl)-2-methylpropane-2-sulfen amide (45 mg, 0.13 mmol, potassium phosphate (81 mg, 0.38 mmol) and [1,1'-bis(diphenylphosphino)ferrocene]palladium (II) dichloride (20 mg, 0.03 mmol) were mixed in 1,4-dioxane (4 mL) and water (0.4 mL), and heated to 90°C then stirred for 1 hour under nitrogen protection. The reaction mixture was filtered and the filtrate was concentrated, the residue was purified by preparative-TLC (EtOAc/methanol=25/1). The title compound was obtained (20 mg, 26%), in form of yellow oil. LCMS (ESI) $[M+H]^+$ = 609.1.

Step 2: (*S*)-*N*-(9-chloro-5-methyl-7-(4-flurophenyl)-2-methyl-3-oxo-3,5-dihydro-2*H*-benzo[*c*]pyrido[3,4-*e* ]azepin-10-yl)ethylsulfonamide

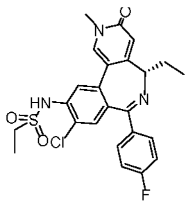

TFA (1 mL) was added into *N*-(5-(4-((*S*)-1-(((*S*)-tert-butyl-sulfinyl)amino)ethyl)-1-methyl-6-oxo-1,6-dihydropyridin-3-yl)-4-( 4-chlorobenzoyl)-2- fluorophenyl)ethylsulfonamide (20 mg, 0.035 mmol) in dichloromethane (4 mL) solution, the reaction mixture was stirred at r.t. overnight, then concentrated. The residue was purified by preparative-HPLC, the title compound was obtained (2 mg, 12%), which was in form of white solid. LCMS (ESI) $[M+H]^+$ = 487.7; $^1H$ NMR (400 MHz, DMSO-$d_6$) $\delta$ 7.97 (s, 1H). 7.59 (s, 1H). 7.52-7.50 (dd, *J* = 6.4 Hz, 1H). 7.50-7.48 (dd, *J* = 6.0 Hz, 1H). 7.24 (s, 1H). 7.22-7.20 (d, *J* = 8.4 Hz, 1H). 6.34 (s, 1H). 3.65-3.61 (q, *J* = 5.6 Hz, 1H). 3.5 (s, 3H). 3.16-3.12 (q, *J* = 3.2 Hz, 2H). 2.19-2.16 (m, 1H). 2.02-1.97 (m, 1H). 1.30-1.26 (t, *J* = 7.2 Hz, 3H). 1.02-0.98 (t, *J* = 6.8 Hz, 3H).

**Embodiment 4066: (*S*)-*N*-(5-ethyl-9-fluoro-7-(4-flurophenyl)-2-methyl-3-oxo-3,5-dihydro-2*H*-benzo[*c*]pyrido[3,4 -*e*]azepin-10-yl)ethylsulfonamide**

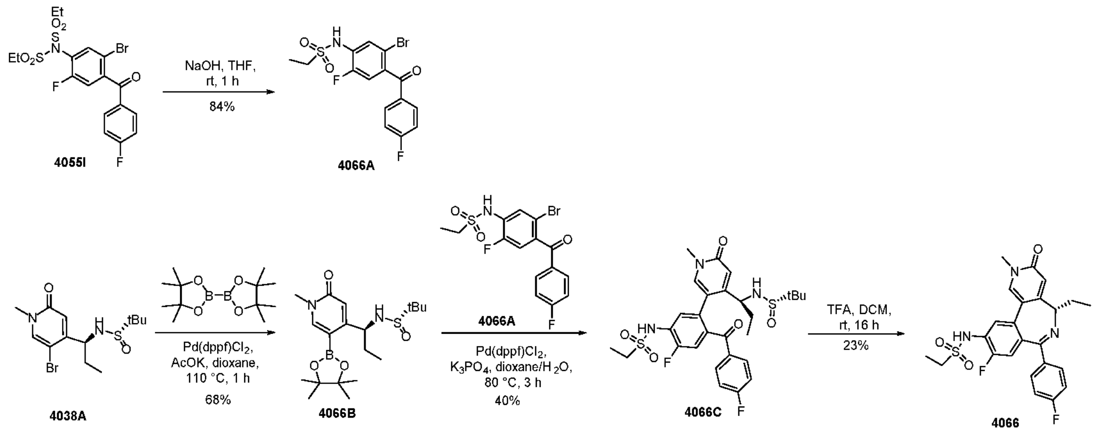


Step 1: *N*-(5-bromo-2-fluoro-4-(4-fluorobenzoyl)phenyl)ethylsulfonamide

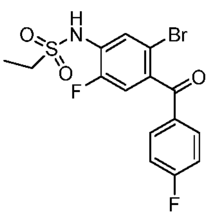

*N*-(5-bromo-2-fluoro-4-(4-fluorobenzoyl)phenyl)-*N*-(ethylsulfonyl)ethylsulfonamide (250 mg, 0.5 mmol) was dissolved in THF (5 mL) and 10% NaOH aqueous solution (5 mL), then stirred at r.t. for 1 hour. Concentration of the reaction mixture, the residue was purified by flash chromatography (PE/EtOAc=10/1), the tiltle compound was obtained (170 mg, 84%), which was in form of yellow solid. LCMS (ESI) $[M+H]^+$ = 405.7.

Step 2: (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)propyl)propane-2-sulfenamide

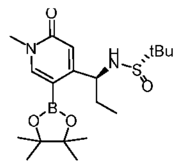

(*S*)-*N*-((*S*)-1-(5-bromo-1-methyl-2-oxo-1,2-dihydropyridin-4-yl)propyl)-2-methylpropan e-2-sulfenamide (100 mg, 0.29 mmol), bis(pinacolato)diboron (147 mg, 0.58 mmol), [1,1'-bis(diphenylphosphino)ferrocene]palladium (II) dichloride (41 mg, 0.058 mmol) and potassium acetate (87 mg, 0.87 mmol) were mixed in 1,4-dioxane (4 mL), heated to 110°C under nitrogen protection then stirred for 1 hour. The reaction mixture was filtered and the filtrate was concentrated, the residue was purified by flash chromatography (PE/EtOAc=10/1), the tiltle compound was obtained (78 mg, 68%), in form of yellow oil. LCMS (ESI) $[M+H]^+$ = 397.1.

Step 3: *N*-(*S*-(4-((*S*)-1-(((*S*)-tert-butyl-sulfinyl)amino)propyl)-1-methyl-6-oxo-1,6-dihydropyridin-3-yl)-2-fluoro-4-(4-fluorobenzoyl)phenyl)ethylsulfonamide

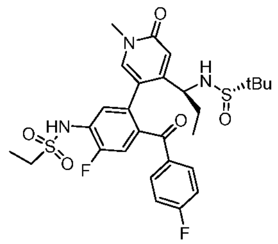

(*S*)-2-Methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (80 mg, 0.2 mmol), *N*-(5-bromo-2-fluoro-4-(4-fluorobenzoyl)phenyl)ethylsulfonamide (120 mg, 0.3 mmol), [1,1'-bis(diphenylphosphino)ferrocene]palladium (II) dichloride (30 mg, 0.04 mmol) and potassium phosphate (128 mg, 0.6 mmol) were mixed in 1,4-dioxane (4 mL) and water (1 mL), and heated to 80°C for 3 hours under nitrogen protection. Concentration of the reaction mixture, the residue was purified by flash chromatography (EtOAc/methanol=10/1), the tiltle compound was obtained (45 mg, 40%), which was in form of brownish solid. LCMS (ESI) $[M+H]^+$ = 593.9.

Step 4: (*S*)-*N*-(5-ethyl-9-fluoro-7-(4-flurophenyl)-2-methyl-3-oxo-3,5-dihydro-2*H*-benzo[*c*]pyrido[3,4-*e*]a zepin-10-yl)ethylsulfonamide

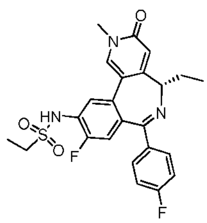

*N*-(5-(4-((*S*)-1-(((*S*)-tert-butyl-sulfinyl)amino)propyl)-1-methyl-6-oxo-1,6-dihydropyridi n-3-yl)-2-fluoro-4-(4-fluorobenzoyl)phenyl)ethylsulfonamide (45 mg, 0.07 mmol) and TFA (1 mL) was dissolved in dichloromethane (4 mL), the reaction mixture was stirred at r.t. overnight then concentrated. The residue was purified by preparative-HPLC to

obtain the title compound (7.6 mg, 23%), which was in form of yellow solid. LCMS (ESI) $[M+H]^+$ = 457.6; $^1H$ NMR (400 MHz, DMSO-$d_6$) $\delta$ 10.10-10.08 (brs, 1H). 8.01 (s, 1H). 7.62 (d, $J$ = 8.0 Hz, 1H). 7.53-7.50 (m, 2H). 7.25-7.15 (m, 3H). 6.36 (s, 1H). 3.64-3.61 (m, 1H). 3.49 (s, 3H). 3.26-3.23 (m, 2H). 3.21-3.17 (m, 1H). 2.03-1.98 (m, 1H). 1.32-1.28 (m, 3H). 1.02-0.98 (m, 3H).

**Embodiment 4068: (*S*)-*N*-(9-chloro-7-(4-chlorophenyl)-2,5-dimethyl-3-oxo-3,5-dihydro-2H-benzo[*c*]pyrido[3,4-*e*] azepin-10-yl)ethylsulfonamide**

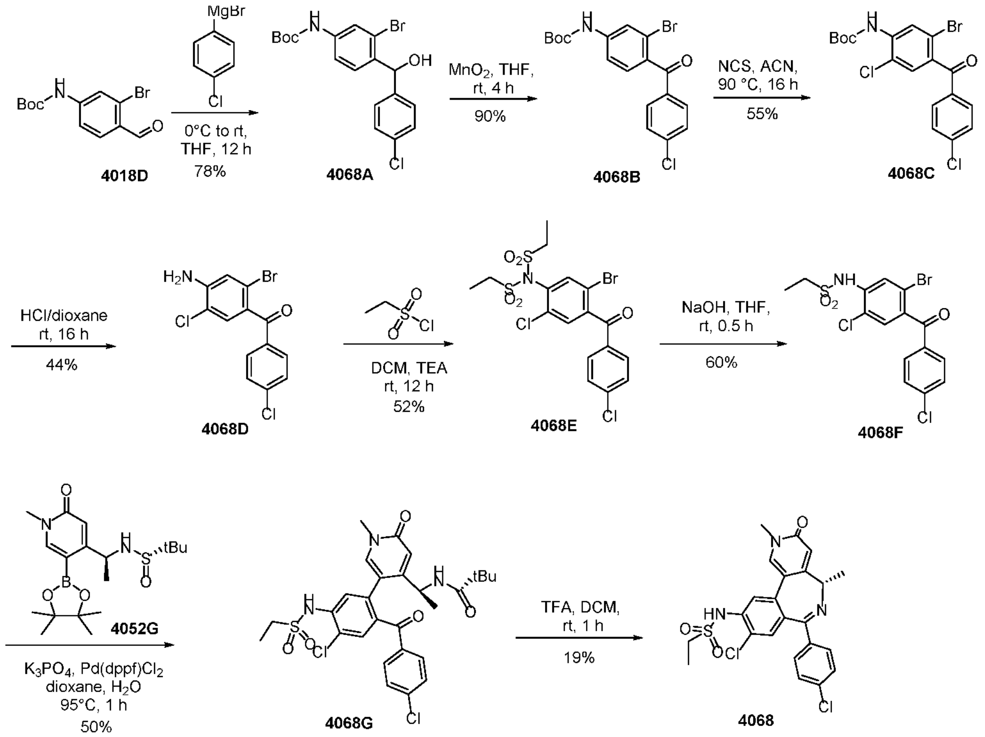


Step 1: (3-Bromo-4-((4-chlorophenyl)(hydroxyl)methyl)phenyl) tert-butylcarbamate

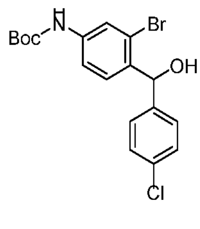

Under nitrogen protection at 0°C, (4-chlorophenyl)magnesium bromide (1 M in THF solution, 43.3 mL) was added into (3-bromo-4-formylphenyl) tert-butylcarbamate (2.6 g, 8.7 mmol) in THF (30 mL) solution. The reaction mixture was stirred at r.t. for 12 hours, the reaction was quenched by saturated ammonium chloride (30 mL), and extracted by EtOAc (100 mL). The organic phase was washed by saturated brine (300 mL), dried over anhydrous sodium sulfate, filtered and concentrated. The residue was purified by flash chromatography (PE/EtOAc=5/1). The title compound was obtained (2.8 g, 78%), in form of yellow oil.

Step 2: (3-Bromo-4-(4-chlorobenzoyl)phenyl) tert-butylcarbamate

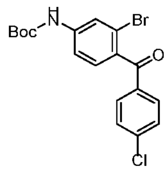

(3-Bromo-4-((4-chlorophenyl)(hydroxyl)methyl)phenyl) tert-butylcarbamate (2.8 g, 6.8 mmol) and manganese dioxide (5.9 g, 68 mmol) were mixed in THF (20 mL), and was stirred at r.t. for 12hours. The reaction mixture was filtered and the filtrate was concentrated, the residue was purified by flash chromatography (PE/EtOAc=8/1). The title compound was obtained (2.5 g, 90%), which was in form of yellow solid.

Step 3: (5-Bromo-2-chloro-4-(4-chlorobenzoyl)phenyl) tert-butylcarbamate

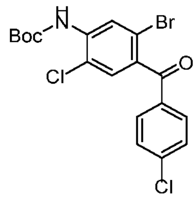

(3-Bromo-4-(4-chlorobenzoyl)phenyl) tert-butylcarbamate (2.5 g, 6.08 mmol) and *N*-chlorosuccinimide (2.45 g, 18.3 mmol) was dissolved in acetonitrile (50 mL), and heated to 90°C and stirred overnight. The reaction mixture was filtered and the filtrate was concentrated, the residue was purified by flash chromatography (PE/EtOAc=10/1). The title compound was obtained (1.5 g, 55%), which was in form of yellow solid. LCMS (ESI) $[M+H]^+$ = 443.0.

Step 4: (4-Amino-2-bromo-5-chlorophenyl)(4-chlorophenyl)methanone

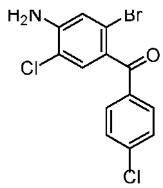

The mixture of (5-bromo-2-chloro-4-(4-chlorobenzoyl)phenyl) tert-butylcarbamate (1.5 g, 3.4 mmol) and HCl (4 M in 1,4-dioxane solution, 20 mL) then was stirred at r.t. overnight. Concentration of the reaction mixture, the residue was purified by flash chromatography (PE/EtOAc=6/1). The title compound was obtained (512 mg, 44%), which was in form of yellow solid. LCMS (ESI) $[M+H]^+$ = 343.7.

Step 5: *N*-(5-bromo-2-chloro-4-(4-chlorobenzoyl)phenyl)-*N*-(ethylsulfonyl)ethylsulfonamide

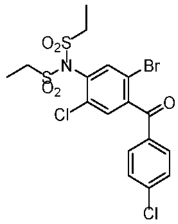

(4-Amino-2-bromo-5-chlorophenyl)(4-chlorophenyl)methanone (250 mg, 0.7 mmol), ethylsulfonyl chloride (932 mg, 7.2 mmol) and triethylamine (733 mg, 7.2 mmol) was dissolved in dichloromethane (4 mL), the reaction mixture and was stirred at r.t. under nitrogen protection for 12 hours. The reaction mixture then concentrated and the crude product of the tiltle compound was obtained (200 mg, 52%), which was in form of yellow solid. LCMS (ESI) [M+H]$^+$ = 528.7.

Step 6: *N*-(5-bromo-2-chloro-4-(4-chlorobenzoyl)phenyl)ethylsulfonamide

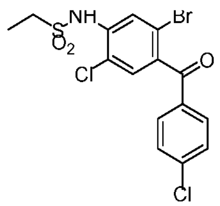

10% NaOH aqueous solution (3 mL) was added dropwise into *N*-(5-bromo-2-chloro-4-(4-chlorobenzoyl)phenyl)-*N*-(ethylsulfonyl)ethylsulfonamide (200 mg, 0.4 mmol) in THF (3 mL) solution. The mixture was stirred at r.t. for 0.5 hour, then added to the reaction mixture EtOAc (10 mL) and water (10 mL), the organic phase was separated. The organic phase was washed by saturated brine (10 mL*3), dried over anhydrous sodium sulfate, filtered and concentrated. The residue was purified by flash chromatography (PE/EtOAc=2/1). The title compound was obtained (100 mg, 60%), which was in form of yellow solid. LCMS (ESI) [M+H]$^+$ = 435.7.

Step 7: *N*-(5-(4-((*S*)-1-(((*S*)-tert-butyl-sulfinyl)amino)ethyl)-1-methyl-6-oxo-1,6-dihydropyridin-3-yl)-2-c hloro-4-(4-chlorobenzoyl)phenyl)ethylsulfonamide

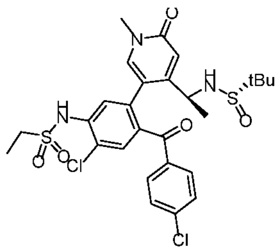

*N*-(5-bromo-2-chloro-4-(4-chlorobenzoyl)phenyl)ethylsulfonamide (100 mg, 0.23 mmol), (*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (88 mg, 0.23 mmol), [1,1'-bis(diphenylphosphino)ferrocene]palladium (II) dichloride (17 mg, 0.02 mmol) and potassium phosphate (146 mg, 0.69 mmol) were mixed in 1,4-dioxane (2 mL) and water (0.1 mL), the reaction mixture was heated to 95°C then stirred for 1 hour under nitrogen protection. The reaction mixture was filtered and the filtrate was concentrated, the residue was purified by flash chromatography (EtOAc/methanol=15/1). The title compound was obtained (70 mg, 50%), in form of yellow oil. LCMS (ESI) [M+H]$^+$ = 612.8.

Step 8: (*S*)-*N*-(9-chloro-7-(4-chlorophenyl)-2,5-dimethyl-3-oxo-3,5-dihydro-2*H*-benzo[c]pyrido[3,4-*e*]aze pin-10-yl)ethylsulfonamide

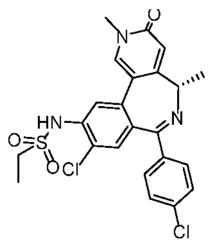

TFA (1 mL) was added into *N*-(5-(4-((*S*)-1-(((*S*)-tert-butyl-sulfinyl)amino)ethyl)-1-methyl-6-oxo-1,6-dihydropyridin-3-yl)-2-chloro-4-(4-chlorobenzoyl)phenyl)ethylsulfonamide (70 mg, 0.11 mmol) in dichloromethane (4 mL) solution. The reaction mixture was stirred at r.t. for 1 hour and then concentrated. The residue was purified by preparative-HPLC, the title compound was obtained (10.6 mg, 19%), which was in form of white solid. LCMS (ESI) $[M+H]^+$ = 490.1; $^1H$ NMR (400 MHz, DMSO-$d_6$) $\delta$ 8.04 (s, 1H). 7.65 (s, 1H). 7.47-7.45 (m, 4H). 7.32 (s, 1H). 6.40 (s, 1H). 4.01-3.99 (q, *J* = 6.8 Hz, 1H). 3.49 (s, 3H). 3.24-3.22 (q, *J* = 6.4 Hz, 2H). 1.65-1.63 (d, *J* = 6.4 Hz, 3H). 1.32-1.28 (t, *J* = 7.6 Hz, 3H).

**Embodiment 4069: (*S*)-10-amino-9-chloro-7-(4-flurophenyl)-2,5-dimethyl-2,5-dihydro-3*H*-benzo[*c*]pyrido[3,4-*e*] azepin-3-ketone**

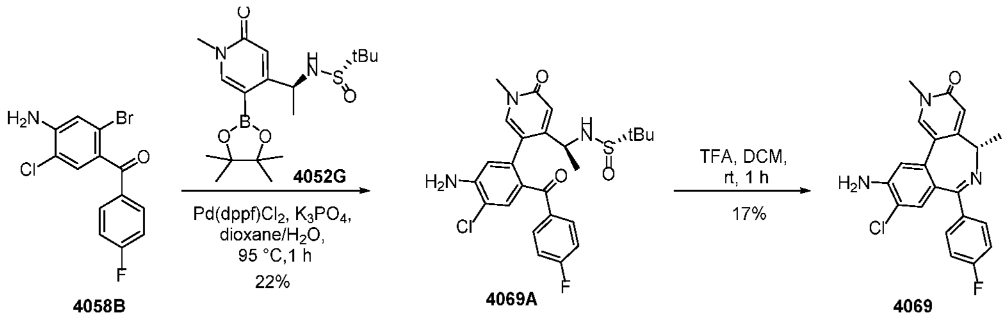


Step 1: (*S*)-*N*-((*S*)-1-(5-(5-amino-4-chloro-2-(4-fluorobenzoyl)phenyl)-1-methyl-2-oxo-1,2-dihydropyridi n-4-yl)ethyl)-2-methylpropane-2-sulfenamide

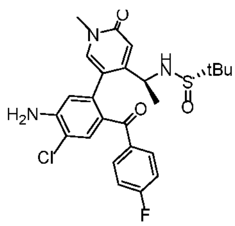

(*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (58 mg, 0.15 mmol), (4-amino-2-bromo-5-chlorophenyl)(4-flurophenyl)methanone (50 mg, 0.15 mmol), potassium phosphate (95 mg, 0.45 mmol) and [1,1'-bis(diphenylphosphino)ferrocene]palladium (II) dichloride (11 mg, 0.015 mmol) were mixed in 1,4-dioxane (2 mL) and water (0.2 mL), and heated to 95°C then stirred for 1 hour under nitrogen protection. The reaction mixture was filtered and the filtrate was concentrated, the residue was purified by preparative-TLC, the tiltle compound was obtained (17 mg, 22%), in form of yellow oil. LCMS (ESI) $[M+H]^+$= 503.9.

Step 2: (*S*)-10-amino-9-chloro-7-(4-flurophenyl)-2,5-dimethyl-2,5-dihydro-3*H*-benzo[*c*]pyrido[3,4-*e*]azep in-3-ketone

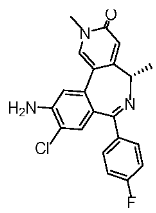

TFA (1 mL) was added into (*S*)-*N*-((*S*)-1-(5-(5-amino-4-chloro-2-(4-fluorobenzoyl)phenyl)-1-methyl-2-oxo-1,2-dihydropyridi n-4-yl)ethyl)-2-methylpropane-2-sulfenamide (17 mg, 0.03 mmol) in dichloromethane (4 mL) solution. The reaction mixture was stirred at r.t. for 1 hour then concentrated. The residue was purified by preparative-HPLC to obtain the title compound (2 mg, 17%), which was in form of white solid. LCMS (ESI) $[M+H]^+$ = 382.1; $^1H$ NMR (400 MHz, DMSO-$d_6$) $\delta$ 7.96 (s, 1H). 7.49-7.45 (m, 2H). 7.23-7.18 (m, 2H). 7.03 (s, 1H). 6.94 (s, 1H). 6.35 (s, 1H). 6.05 (s, 2H). 3.97-3.96 (t, *J* = 6.0 Hz, 1H). 3.47 (s, 3H). 1.62-1.60 (d, *J* = 6.4 Hz 3H).

**Embodiment 4070: (*S*)-10-amino-9-fluoro-7-(4-flurophenyl)-2,5-dimethyl-2,5-dihydro-3*H*-benzo[*c*]pyrido[3,4-*e*]a zepin-3-ketone**

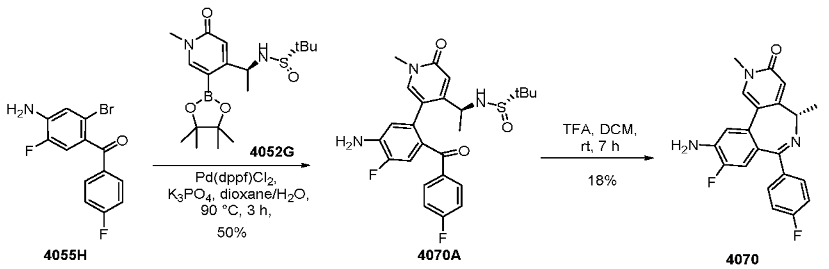


4055H 4070A 4070

Step 1: (*S*)-*N*-((*S*)-1-(5-(5-amino-4-fluoro-2-(4-fluorobenzoyl)phenyl)-1-methyl-2-oxo-1,2-dihydropyridin -4-yl)ethyl)-2-methylpropane-2-sulfenamide

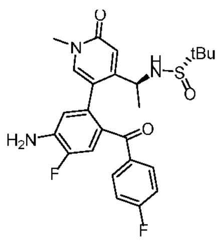

(4-Amino-2-bromo-5-flurophenyl)(4-flurophenyl)methanone (100 mg, 0.32 mmol), (*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (159 mg, 0.41 mmol), [1,1'-bis(diphenylphosphino)ferrocene]palladium (II) dichloride (47 mg, 0.064 mmol) and potassium phosphate (204 mg, 0.96 mmol) were mixed in 1,4-dioxane (3 mL) and water (0.6 mL), the reaction mixture heated to 90°C and stirred for 3 hours under nitrogen protection. The reaction mixture was filtered and the filtrate was concentrated, the residue was purified by flash chromatography (EtOAc/methanol=15/1). The title compound was obtained (78 mg, 50%), in form of yellow oil. LCMS (ESI) $[M+H]^+$ = 488.1.

Step 2: (*S*)-10-amino-9-fluoro-7-(4-flurophenyl)-2,5-dimethyl-2,5-dihydro-3*H*-benzo[*c*]pyrido[3,4-*e*]azep in-3-ketone

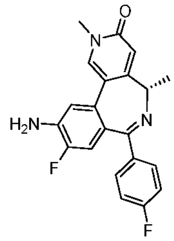

TFA (1 mL) was added into (*S*)-*N*-((*S*)-1-(5-(5-amino-4-fluoro-2-(4-fluorobenzoyl)phenyl)-1-methyl-2-oxo-1,2-dihydropyridin -4-yl)ethyl)-2-methylpropane-2-sulfenamide (78 mg, 0.16 mmol) in dichloromethane (4 mL) solution. The reaction mixture was stirred at r.t. for 7 hours and concentrated. The residue was purified by preparative-HPLC, the title compound was obtained (10.5 mg, 18%), which was in form of white solid. LCMS (ESI) $[M+H]^+$ = 366.2; $^1H$ NMR (400 MHz, DMSO-$d_6$) $\delta$ 7.95 (s, 1H). 7.51-7.47 (m, 2H). 7.16-7.12 (m, 2H). 7.01 (d, *J* = 8.8 Hz, 1H). 6.82 (m, *J* = 12.0 Hz, 1H). 6.60 (s, 1H). 4.13 (q, *J* = 6.4 Hz, 1H). 3.66 (m, 3H). 1.75 (t, *J* = 6.4 Hz, 3H).

**Embodiment 4071: (*S*)-(7-(4-flurophenyl)-2,5-dimethyl-3-oxo-3,5-dihydro-2*H*-benzo[*c*]pyrido[3,4-*e*]azepin-10-yl) methane sulfamide;**

**Embodiment 4072: (*S*)-1-(7-(4-flurophenyl)-2,5-dimethyl-3-oxo-3,5-dihydro-2*H*-benzo[*c*]pyrido[3,4-*e*]azepin-10-yl)-*N*-methylmethane sulfamide** [01236] **and 4073: (*S*)-1-(7-(4-flurophenyl)-2,5-dimethyl-3-oxo-3,5-dihydro-2*H*-benzo[*c*]pyrido[3,4-*e*]azepin-10-yl)-*N*,*N*-dimethylmethane sulfamide**

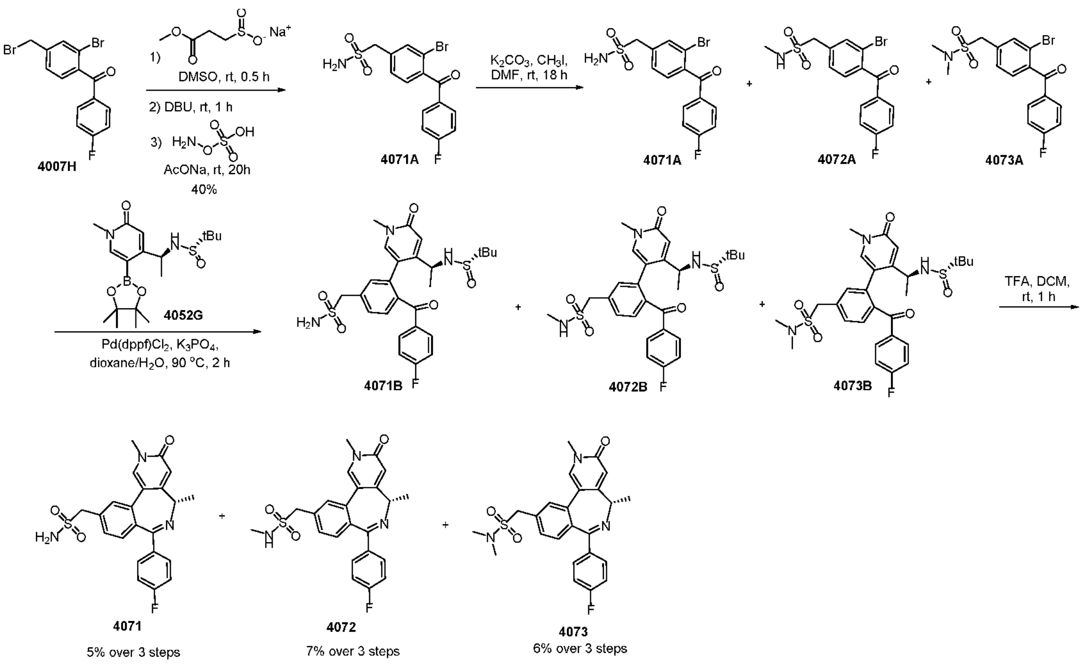


Step 1: (3-Bromo-4-(4-fluorobenzoyl)phenyl)methane sulfamide

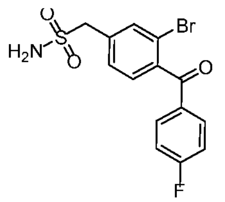

(2-Bromo-4-(bromomethyl)phenyl)(4-flurophenyl)methanone (250 mg, 0.672 mmol) and 3-methoxyl-3-oxo propane sodium sulfinate (140 mg, 0.806 mmol) were mixed in dimethylsulfoxide (3 mL), was stirred at r.t. for 0.5 hour. Then added 1,8-diazabicyclo[5.4.0]undec-7-ene (123 mg, 0.806 mmol), and continued stirring at r.t. for 1 hour. To the reaction mixture added sodium acetate (253 mg, 3.1 mmol) and hydroxylamine-*O*-sulfonic acid (380 mg, 3.36 mmol) in aqueous solution (5 mL), the mixture obtained was stirred at r.t. for 20 hours. The reaction mixture was diluted by EtOAc (80 mL), and washed by saturated brine and water, dried and concentrated, the residue was purified by flash chromatography (PE/EtOAc=1/1). The title compound was obtained (100 mg, 40%), inc form of colorless liquid. LCMS (ESI) $[M+H]^+$ = 371.9.

Step 2: (3-Bromo-4-(4-fluorobenzoyl)phenyl)methane sulfamide; 1-(3-bromo-4-(4-fluorobenzoyl)phenyl)-*N*-methylmethane sulfamide and 1-(3-bromo-4-(4-fluorobenzoyl)phenyl)-*N*,*N*-dimethylmethane sulfamide

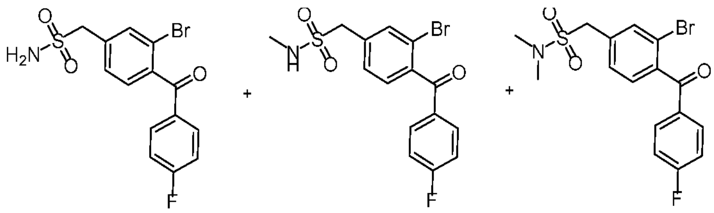

(3-Bromo-4-(4-fluorobenzoyl)phenyl)methane sulfamide (100 mg, 0.269 mmol), methyl iodide (46 mg, 0.322 mmol) and potassium carbonate (56 mg, 0.403 mmol) was dissolved in *N,N*-dimethylformamide (3 mL), was stirred at r.t. for 18 hours. The reaction mixture was diluted by EtOAc (80 mL), and washed by saturated brine and water, then concentrate, the mixture of the tiltle compound was obtained (100 mg), in form of yellow oil. LCMS (ESI) $[M+H]^+$ = 371.8; 385.8; 399.8.

Step 3: (3-(4-((*S*)-1-(((*S*)-tert-butyl-sulfinyl)amino)ethyl)-1-methyl-6-oxo-1,6-dihydropyridin-3-yl)-4-(4-fl uorobenzoyl)phenyl)methane sulfamide; 1-(3-(4-((*S*)-1-(((*S*)-tert-butylsulfinyl)amino) ethyl)-1-methyl-6-oxo-1,6-dihydropyridin-3-yl)-4-(4-fluorobenzoyl)phenyl)-*N*-methylmethane sulfamide and 1-(3-(4-((*S*)-1-(((*S*)-tert-butylsulfinyl)amino) ethyl)-1-methyl-6-oxo-1,6-dihydropyridin-3-yl)-4-(4-fluorobenzoyl)phenyl)-*N*,*N*-dimethy methane sulfamide

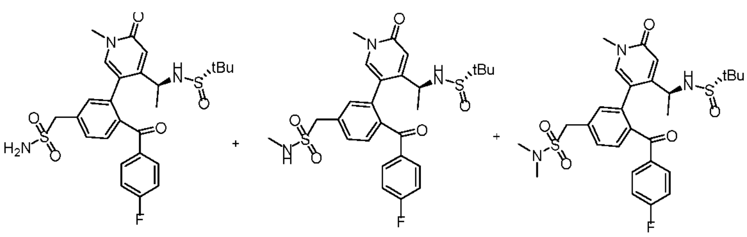

(3-Bromo-4-(4-fluorobenzoyl)phenyl)methane sulfamide, 1-(3-bromo-4-(4-fluorobenzoyl)phenyl)-*N*-methylmethane sulfamide and 1-(3-bromo-4-(4-fluorobenzoyl)phenyl)-*N*,*N*-dimethylmethane sulfamide, (*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (150 mg, 0.39 mmol), [1,1'-bis(diphenylphosphino)ferrocene]palladium (II) dichloride (19 mg, 0.026 mmol) and potassium phosphate (138 mg, 0.65 mmol) were mixed in 1,4-dioxane (6 mL) and water (0.6 mL), and heated to 90°C then stirred for 2 hours under nitrogen protection. The reaction mixture was cooled down to rt, then filtered and the filtrate

was concentrated, the residue was purified by flash chromatography (PE/EtOAc/methanol=5/95/5), the mixture of the tiltle compound was obtained (75 mg), in form of yellow oil. LCMS (ESI) $[M+H]^+$ = 547.9; 561.9; 576.0.

Step 4: (*S*)-(7-(4-flurophenyl)-2,5-dimethyl-3-oxo-3,5-dihydro-2*H*-benzo[*c*]pyrido[3,4-*e*]azepin-10-yl)me thane sulfamide;

(*S*)-1-(7-(4-flurophenyl)-2,5-dimethyl-3-oxo-3,5-dihydro-2*H*-benzo[*c*]pyrido[3,4-*e*]azepin-10-yl)-*N*-methylmethane sulfamide
and
(*S*)-1-(7-(4-flurophenyl)-2,5-dimethyl-3-oxo-3,5-dihydro-2*H*-benzo[*c*]pyrido[3,4-*e*]azepin-10-yl)-*N*,*N*-dimethylmethane sulfamide

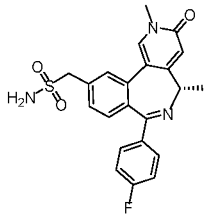
4071

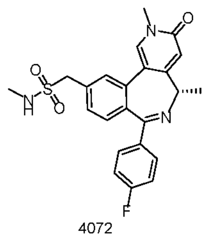
4072

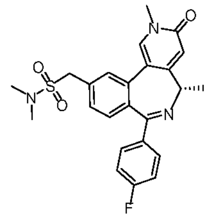
4073

The mixture of (3-(4-((*S*)-1-(((*S*)-tert-butyl-sulfinyl)amino)ethyl)-1-methyl-6-oxo-1,6-dihydropyridin-3-yl)-4-(4-fl uorobenzoyl)phenyl)methane sulfamide, 1-(3-(4-((*S*)-1-(((S)-tert-butylsulfinyl)amino) ethyl)-1-methyl-6-oxo-1,6-dihydropyridin-3-yl)-4-(4-fluorobenzoyl)phenyl)-*N*-methylmethane sulfamide, 1-(3-(4-(-(((*S*)-tert-butylsulfinyl)amino) ethyl)-1-methyl-6-oxo-1,6-dihydropyridin-3-yl)-4-(4-fluorobenzoyl)phenyl)-*N*,*N*-dimethylmethan e sulfamide (75 mg) and TFA (2 mL) were dissolved in dichloromethane (3 mL), and was stirred at r.t. for 16 hours. Concentration of the reaction mixture, the residue was purified by preparative-HPLC, then obtained (*S*)-(7-(4-flurophenyl)-2,5-dimethyl-3-oxo-3,5-dihydro-2*H*-benzo[*c*]pyrido[3,4-*e*]azepin-10-yl) methylsulfamide (6.2 mg, 5%), which was in form of white solid. LCMS (ESI) $[M+H]^+$ = 426.1; $^1H$ NMR (400 MHz, $CD_3OD$) $\delta$ 8.09 (s, 1H). 7.76 (s, 1H). 7.53-7.50 (m, 3H). 7.34 (d, *J* = 7.6 Hz, 1H). 7.16-7.11 (m, 2H). 6.64 (s, 1H). 4.52 (s, 2H). 4.10 (q, 1H). 3.68 (s, 3H). 1.78 (d, 3H).

(*S*)-1-(7-(4-flurophenyl)-2,5-dimethyl-3-oxo-3,5-dihydro-2*H*-benzo[*c*]pyrido[3,4-*e*]azepin-10-yl)-*N*-methylmethane sulfamide (8.7 mg, 7%), which was in form of white solid. LCMS (ESI) $[M+H]^+$ = 440.0; $^1H$ NMR (400 MHz, $CD_3OD$) $\delta$ 8.09 (s, 1H). 7.76 (s, 1H). 7.53-7.50 (m, 3H). 7.35 (d, *J* = 8.0 Hz, 1H). 7.16-7.11 (m, 2H). 6.64 (s, 1H). 4.51 (s, 2H). 4.10 (q, 1H). 3.68 (s, 3H). 2.77 (s, 3H). 1.78 (d, 3H).
and
(*S*)-1-(7-(4-flurophenyl)-2,5-dimethyl-3-oxo-3,5-dihydro-2*H*-benzo[*c*]pyrido[3,4-*e*]azepin-10-yl)-*N*,*N*-dimethylsulfamide (8.0 mg, 6%), which was in form of white solid. LCMS (ESI) $[M+H]^+$ = 454.0; $^1H$ NMR (400 MHz, $CD_3OD$) $\delta$ 8.08 (s, 1H). 7.78 (s, 1H). 7.54-7.48 (m, 3H). 7.35 (d, *J* = 8.0 Hz, 1H). 7.16-7.11 (m, 2H). 6.64 (s, 1H). 4.50 (s, 2H). 4.10 (q, 1H). 3.68 (s, 3H). 2.90 (s, 6H). 1.78 (d, 3H).

**Embodiment 4074: (*S*)-*N*-(9-fluoro-7-(4-flurophenyl)-2,5-dimethyl-3-oxo-3,5-dihydro-2*H*-benzo[*c*]pyrido[3,4-*e*]a zepin-10-yl) methylsulfamide**

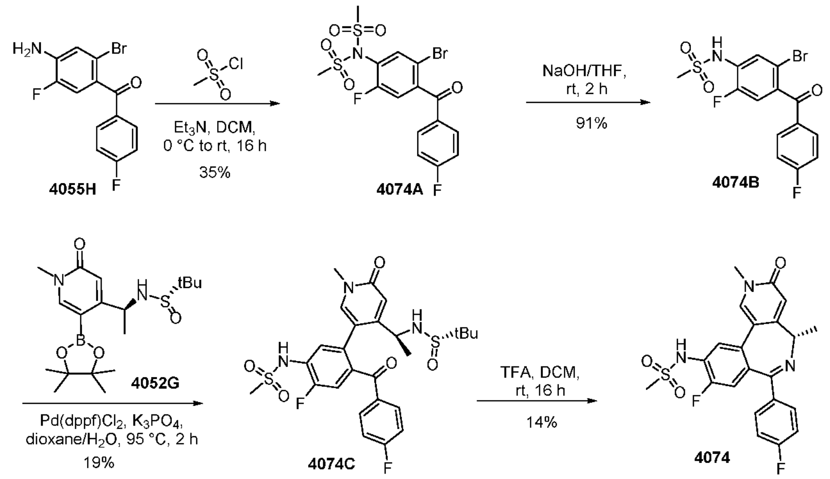


Step 1: *N*-(5-bromo-2-fluoro-4-(4-fluorobenzoyl)phenyl)-*N*-(dimethylfulfone)methylsulfamide

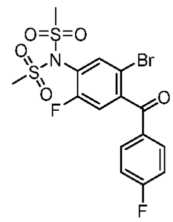

At 0°C, methanesulfonyl chloride (1.5 g, 13.5 mmol) was added into (4-amino-2-bromo-5-flurophenyl)(4-ffluorophenyl)methanone (420 mg, 1.35 mmol) and triethylamine (1.36 g, 13.5 mmol) in dichloromethane (10 mL) solution. The reaction mixture was raised to r.t. and stirred overnight, then concentrated. The residue was purified by flash chromatography (PE/EtOAc=1/1). The title compound was obtained (220 mg, 35%), which was in form of yellow solid. LCMS (ESI) $[M+H]^+$ = 468.0.

Step 2: *N*-(5-bromo-2-fluoro-4-(4-fluorobenzoyl)phenyl)methane sulfamide

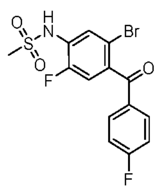

10% NaOH aqueous solution (4 mL) was slowly added dropwise into *N*-(5-bromo-2-fluoro-4-(4-fluorobenzoyl)phenyl)-*N*-(dimethylfulfone)methylsulfamide (220 mg, 0.47 mmol) in THF (4 mL) solution, the mixture was then stirred at r.t. for 2 hours. The reaction mixture was added by EtOAc (10 mL) and water (10 mL), the organic phase was separated. The organic phase was washed by saturated brine and water, dried over anhydrous sodium sulfate, filtered and concentrated. The residue was purified by flash chromatography (PE/EtOAc=2/1). The title compound was obtained (166 mg, 91%), which was in form of yellow solid. LCMS (ESI) $[M+H]^+$ = 391.9.

Step 3: *N*-(5-(4-((*S*)-1-(((*S*)-tert-butyl-sulfinyl)amino)ethyl)-1-methyl-6-oxo-1,6-dihydropyridin-3-yl)-2-fl uoro-4-(4-fluorobenzoyl)phenyl)methylsulfamide

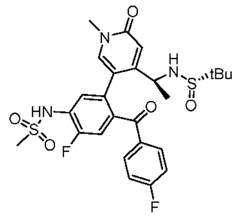

(*S*)-2-Methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (147 mg, 0.39 mmol), *N*-(5-bromo-2-fluoro-4-(4-fluorobenzoyl)phenyl)methane sulfamide (150 mg, 0.39 mmol), [1,1'-bis(diphenylphosphino)ferrocene]palladium (II) dichloride (29 mg, 0.039 mmol) and potassium phosphate (248 mg, 1.17 mmol) were mixed in 1,4-dioxane (8 mL) and water (2 mL), and heated to 95°C then stirred for 2 hours under nitrogen protection. Concentration of the reaction mixture, the residue was purified by flash chromatography (EtOAc/methanol=10/1). The title compound was obtained (41 mg, 19%), which was in form of brownish solid. LCMS (ESI) $[M+H]^+$ = 566.2.

Step 4: (*S*)-*N*-(9-fluoro-7-(4-flurophenyl)-2,5-dimethyl-3-oxo-3,5-dihydro-2*H*-benzo[*c*]pyrido[3,4-*e*]azepi n-10-yl)methane sulfamide

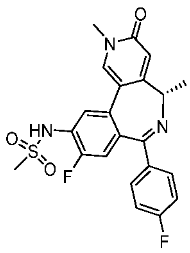

*N*-(5-(4-((*S*)-1-(((*S*)-tert-butyl-sulfinyl)amino)ethyl)-1-methyl-6-oxo-1,6-dihydropyridin -3-yl)-2-fluoro-4-(4-fluorobenzoyl)phenyl)methane sulfamide (41 mg, 0.075 mmol) and TFA (4 mL) was dissolved in dichloromethane (8 mL), then stirred at r.t. overnight. Concentration of the reaction mixture, the residue was purified by preparative-HPLC, the title compound was obtained (4.7 mg, 14%), which was in form of yellow solid. LCMS (ESI) $[M+H]^+$ = 444.1; $^1$H NMR (400 MHz, DMSO-*d6*) $\delta$ 10.08 (brs, 1H). 8.02 (s, 1H). 7.60 (d, *J* = 7.6 Hz, 1H). 7.55-7.49 (m, 2H). 7.27-7.14 (m, 3H). 6.39 (s, 1H). 3.98 (q, *J* = 2.8 Hz, 1H). 3.49 (s, 3H). 3.17 (s, 3H). 1.64 (d, *J* = 6.8 Hz, 3H).

**Embodiment 4075: (*S*)-4-(10-((ethylsulfonyl)methyl)-2,5-dimethyl-3-oxo-3,5-dihydro-2*H*-benzo[*c*]pyrazole[3,4-*e*] azepin-7-yl)benzylnitrile**

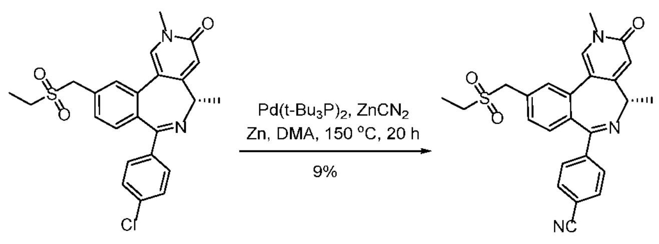


**4037** **4075**

(*S*)-7-(4-Chlorophenyl)-10-((ethylsulfonyl)methyl)-2,5-dimethyl-2,5-dihydro-3*H*-benzo[ *c*]pyrido[3,4-*e*]azepin-3-ketone (65 mg, 0.143 mmol), zinc cyanide (34 mg, 0.286 mmol), di(tritert-butylphosphine) palladium (0) (7 mg, 0.014 mmol) and zinc powder (1 mg, 0.014 mmol) were mixed in *N*,*N*-dimethylbenzylamine (2 mL), and heated to 150°C and stirred for 20 hours. The reaction mixture was cooled down to rt, diluted with EtOAc (10 mL), then filtered and the filtrate was concentrated. The residue was purified by preparative-HPLC, the title compound was obtained (5.5 mg, 9%), which

was in form of white solid. LCMS (ESI) [M+H]+ = 446.2; $^1$H NMR (400 MHz, $CD_3OD$) $\delta$ 8.09 (s, 1H). 7.78-7.76 (m, 3H). 7.66-7.64 (m, 2H). 7.57 (d, *J* = 6.8 Hz, 1H). 7.34 (d, *J* = 8.0 Hz, 1H). 6.65 (s, 1H). 4.60 (s, 2H). 4.17 (q, 1H). 3.68 (s, 3H). 3.21 (q, 2H). 1.80 (d, 3H). 1.44 (t, 3H).

**Embodiment 4077: (*S*)-*N*-(9-chloro-5-cyclopropyl-7-(4-flurophenyl)-2-methyl-3-oxo-3,5-dihydro-2*H*-benzo[*c*]pyr ido[3,4-*e*]azepin-10-yl)ethylsulfonamide**

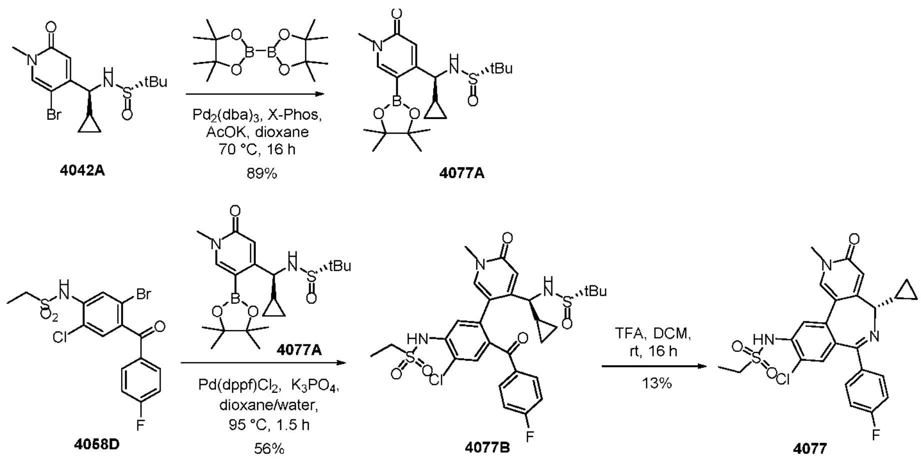


Step 1: (*S*)-*N*-((*S*)-cyclopropyl(1-methyl-2-oxo-5-(4,4,5,5-tetramethyl-1,3,2-dioxaborolan-2-yl)-1,2-dihyd ropiridin-4-l)methyl)-2-methlroane-2-sulfenamide

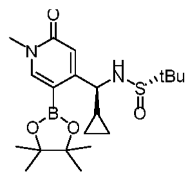

(*S*)-*N*-((*S*)-(5-bromo-1-methyl-2-oxo-1,2-dihydropyridin-4-yl)(cyclopropyl)methyl)-2-m ethylpropane-2-sulfenamide (200 mg, 0.553 mmol), bis(pinacolato)diboron (422 mg, 1.66 mmol), 2-dicyclohexylphosphorus-2,4,6-triisopropyl biphenyl (106 mg, 0.22 mmol), tri(dibenzyl acetylacetone)dipalladium (0) (101 mg, 0.11 mmol) and potassium acetate (136 mg, 1.39 mmol) were mixed in 1,4-dioxane (5 mL), and heated to 70°C and stirred for 16 hours under nitrogen protection. Concentration of the reaction mixture, the residue was purified by flash chromatography (dichloromethane/methanol=10/1). The title compound was obtained (100 mg, 89%), which was in form of yellow solid. LCMS (ESI) [M+H]+ = 409.0.

Step 2: *N*-(5-(4-((*S*)-(((*S*)-tert-butyl-sulfinyl)amino)(cyclopropyl)methyl)-1-methyl-6-oxo-1,6-dihydropyri din-3-yl)-2-chloro-4-(4-fluorobenzoyl)phenyl)ethylsulfonamide

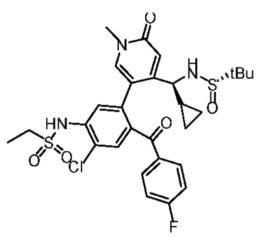

*N*-(5-bromo-2-chloro-4-(4-fluorobenzoyl)phenyl)ethylsulfonamide (230 mg, 0.546 mmol), (*S*)-*N*-((*S*)-cyclopropyl(1-methyl-2-oxo-5-(4,4,5,5-tetramethyl-1,3,2-dioxaborolan-2-yl)-1,2-dihyd ropyridin-4-yl)methyl)-2-methylpropane-2-sulfenamide (230 mg, 0.563 mmol), potassium phosphate (357 mg, 1.68 mmol) and [1,1'-bis(diphenylphosphino)ferrocene]palladium (II) dichloride (115 mg, 0.157 mmol) were mixed in 1,4-dioxane (5 mL) and water (0.5 mL), and heated to 95°C for 1.5 hours under nitrogen protection. The reaction mixture was filtered and the filtrate was concentrated. The residue was purified by flash chromatography (PE/EtOAc=2/1). The title compound was obtained (200 mg, 56%), in form of yellow oil. LCMS (ESI) [M+H]$^+$= 622.2.

Step 3: (*S*)-*N*-(9-chloro-5-cyclopropyl-7-(4-flurophenyl)-2-methyl-3-oxo-3,5-dihydro-2*H*-benzo[*c*]pyrido[ 3,4-*e*]azepin-10-yl)ethylsulfonamide

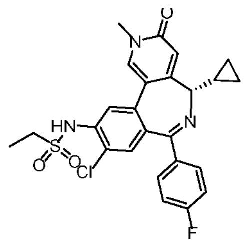

TFA (1 mL) was added into *N*-(5-(4-((*S*)-(((*S*)-tert-butyl-sulfinyl)amino)(cyclopropyl)methyl)-1-methyl-6-oxo-1,6-dihydropyri din-3-yl)-2-chloro-4-(4-fluorobenzoyl)phenyl)ethylsulfonamide (200 mg, 0.322 mmol) in dichloromethane (4 mL) solution, the reaction mixture was stirred at r.t. overnight then concentrated. The residue was purified by preparative-HPLC, the title compound was obtained (21.0 mg, 13%), which was in form of white solid. LCMS (ESI) [M+H]$^+$ = 500.1; $^1$H NMR (400 MHz, DMSO) $\delta$ 9.61 (s, 1H). 8.06 (s, 1H). 7.67 (s, 1H). 7.49-7.45 (m, 2H). 7.33 (s, 1H). 7.22-7.21 (t, *J* = 4.8 Hz, 2H). 6.71 (s, 1H). 3.50-3.49 (m, 1H). 3.31-3.29 (t, *J* = 10.8 Hz, 2H). 1.56-1.54 (d, *J* = 5.2 Hz, 1H). 1.33-1.29 (t, *J* = 7.2 Hz, 3H). 0.75-0.73 (t, *J* = 7.2 Hz, 1H). 0.60-0.58 (t, *J* = 8.4 Hz, 2H). 0.31-0.29 (d, *J* = 4.4 Hz, 1H).

**Embodiment 4078: 10-((ethylsulfonyl)methyl)-7-(4-flurophenyl)-2,5-dimethyl-2,5-dihydro-3*H*-benzo[e]pyrido[4,3-*c*][1,2]diazepin-3-ketone**

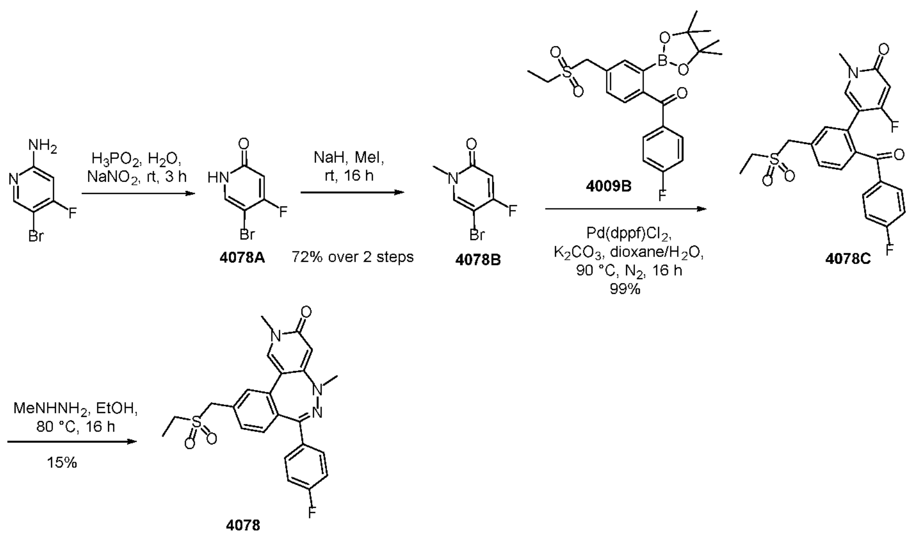


Step 1: 5-Bromo-4-fluoropyridin-2(1*H*)-ketone

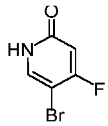

Sodium nitrite (860 mg, 12.46 mmol) in water solution (20 mL) was slowly added into 5-bromo-4-fluoropyridin-2-amine (2 g, 10.47 mmol) and hypophosphory (691 mg, 104.7 mmol) in water solution (30 mL). The obtained mixture was stirred at r.t. for 3 hours. Then the precipition was collected and washed by water, dried, the crude product of the tiltle compound was obtained (1.8 g), which was in form of yellow solid. LCMS (ESI) $[M+1]^+$ = 193.8.

Step 2: 5-Bromo-4-fluoro-1-methylpyridin-2(1*H*)-ketone

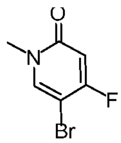

Sodium hydride (300 mg, 8.1 mmol, 60% oily dispersed liquid) was added in portions into 5-bromo-4-fluoropyridin-2(1*H*)-ketone (1.29 g, 6.75 mmol) in THF (10 mL), the reaction mixture was stirred at r.t. for 30 minutes. Then added methyl iodide (1.05 g, 8.1 mmol), and continued stirring overnight at rt. Ammonium chloride (1 g) was added into the reaction mixture, then stirred for 30 minutes and concentrated. The residue was purified by flash chromatography (PE/EtOAc=1/1). The title compound was obtained (1 g, yield of the two steps 72%), which was in form of yellow solid. LCMS (ESI) $[M+1]^+$ = 205.9.

Step 3: 5-(5-((Ethylsulfonyl)methyl)-2-(4-fluorobenzoyl)phenyl)-4-fluoro-1-methylpyridin-2(1*H*)-ketone

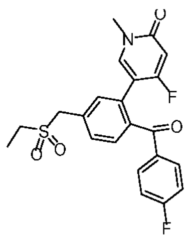

5-Bromo-4-fluoro-1-methylpyridin-2(1*H*)-ketone (206 mg, 1 mmol), (4-((ethylsulfonyl)methyl)-2-(4,4,5,5-tetramethyl-1,3,2-dioxaborolan-2-yl)phenyl)(4-flurophenyl) methanone (648 mg, 1.5 mmol), [1,1'-bis(diphenylphosphino)ferrocene]palladium (II) dichloride (73.1 mg, 0.1 mmol) and potassium carbonate (414 mg, 3 mmol) were mixed in 1,4-dioxane (5 mL) and water (1 mL), and heated to 90°C and stirred overnight under nitrogen protection. Concentration of the reaction mixture, the residue was purified by flash chromatography (PE/EtOAc=1/1). The title compound was obtained (430 mg, 99%), in form of yellow oil. LCMS (ESI) $[M+1]^+$ = 431.9.

Step 4: 10-((Ethylsulfonyl)methyl)-7-(4-flurophenyl)-2,5-dimethyl-2,5-dihydro-3*H*-benzo[*e*]pyrido[4,3-*c*] [1,2]diazein-3-ketone

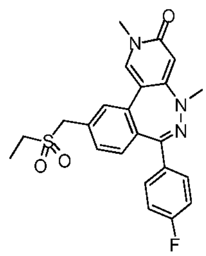

5-(5-((Ethylsulfonyl)methyl)-2-(4-fluorobenzoyl)phenyl)-4-fluoro-1-methylpyridin-2(1 *H*)-ketone (50 mg, 0.116 mmol) and methylhydrazine (4153 mg, 11.6 mmol) was dissolved in ethanol (10 mL), and heated to 80°C and stirred overnight then concentrated. The residue was purified by preparative-HPLC, the title compound was obtained (7.6 mg, 15%), which was in form of yellow solid. LCMS (ESI) $[M+H]^+$ = 440.0; $^1H$ NMR (400 MHz, DMSO-$d_6$) $\delta$ 7.56-7.48 (m, 2H). 7.42-7.37 (m, 2H). 7.31 (d, *J* = 7.6 Hz, 1H). 7.18 (d, *J* = 8.0 Hz, 1H). 7.11-7.01 (m, 2H). 4.28 (s, 2H). 3.58 (s, 3H). 3.02 (q, *J* = 7.2 Hz, 2H). 1.46 (t, *J* = 7.6 Hz, 3H).

**Embodiment 4079: (*S*)-*N*-(9-chloro-7-(4-flurophenyl)-2,5-dimethyl-3-oxo-3,5-dihydro-2*H*-benzo[*c*]pyrido[3,4-*e*]azepin-10-yl)methylsulfamide**

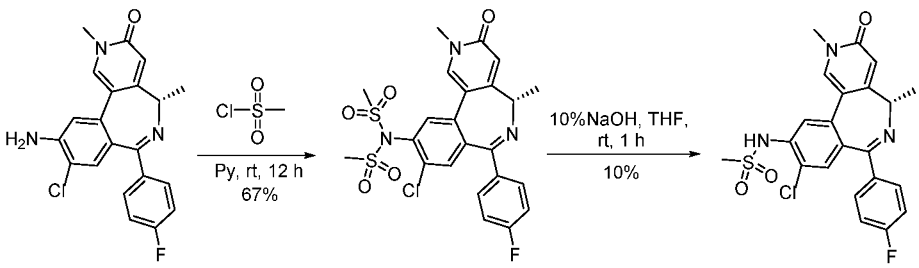


CP-4069 4079A 4079

Step 1: (*S*)-*N*-(9-chloro-7-(4-flurophenyl)-2,5-dimethyl-3-oxo-3,5-dihydro-2*H*-benzo[*c*]pyrido[3,4-*e*]azepi n-10-yl)-*N*-(dimethylfulfone)methane sulfamide

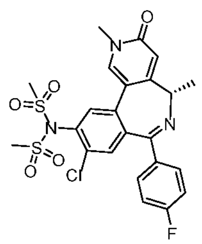

Methane sulfonyl chloride (34 mg, 0.3 mmol) was added into (*S*)-10-amino-9-chloro-7-(4-flurophenyl)-2,5-dimethyl-2,5-dihydro-3*H*-benzo[*c*]pyrido[3,4-*e*]azep in-3-ketone (12 mg, 0.03 mmol) in pyridine (1 mL) solution, the reaction mixture was stirred at r.t. for 12 hours and concentrated, the residue was purified by flash chromatography (PE/EtOAc=15/1). The tiltle compound was obtained (11 mg, 67%), which was in form of yellow solid. LCMS (ESI) $[M+H]^+$ = 537.8.

Step 2: (*S*)-*N*-(9-chloro-7-(4-flurophenyl)-2,5-dimethyl-3-oxo-3,5-dihydro-2*H*-benzo[*c*]pyrido[3,4-*e*]azepi n-10-yl)methane sulfamide

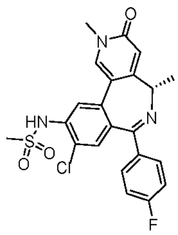

10% NaOH aqueous solution (1 mL) was slowly added into (*S*)-*N*-(9-chloro-7-(4-flurophenyl)-2,5-dimethyl-3-oxo-3,5-dihydro-2*H*-benzo[*c*]pyrido[3,4-*e*]azepi n-10-yl)-*N*-(dimethylfulfone) methane sulfamide (11 mg, 0.02 mmol) in THF (1 mL) solution. The mixture obtained was then stirred at r.t. for 1 hour. EtOAc (10 mL) and water (10 mL) was added into the reaction mixture, the organic phase was separated. The organic phase was washed by saturated brine (10 mL*3), dried over anhydrous sodium sulfate, filtered and concentrated. The residue was purified by preparative-HPLC, the tiltle compound was obtained (0.9 mg, 10%), which was in form of white solid. LCMS (ESI) $[M+H]^+$ = 460.1; $^1H$ NMR (400 MHz, $CD_3OD$) $\delta$ 8.06 (s, 1H). 7.53 (s, 1H). 7.53-7.49 (m, 2H). 7.30 (s, 1H). 7.18-7.13 (m, 2H). 6.62 (s, 1H). 4.16-4.12 (q, *J* = 6.8 Hz, 1H). 3.66 (s, 3H). 3.16-3.13 (s, 3H). 1.78-1.75 (d, *J* = 6.4 Hz, 3H).

**Embodiment 4080: (*S*)-*N*-(9-chloro-7-(4-flurophenyl)-2,5-dimethyl-3-oxo-3,5-dihydro-2*H*-benzo[*c*]pyrido[3,4-*e*]azepin-10-yl)cyclopropylsulfamide**

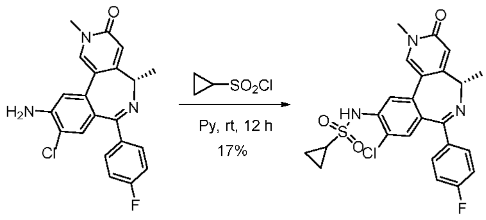


4069 4080

Cyclopropylsulfonyl chloride (44 mg, 0.3 mmol) was added into (*S*)-10-amino-9-chloro-7-(4-flurophenyl)-2,5-dimethyl-2,5-dihydro-3*H*-benzo[*c*]pyrido[3,4-*e*]azep in-3-ketone (12 mg, 0.03 mmol) in pyridine (1 mL) solution, the reaction mixture was stirred at r.t. for 12 hours and then concentrated. The residue was purified by preparative-HPLC, the title compound was obtained (2.6 mg, 17%), which was in form of white solid. LCMS (ESI) $[M+H]^+$ = 486.1; $^1H$ NMR (400 MHz, $CD_3OD$) $\delta$8.06 (s, 1H). 7.83 (s, 1H). 7.53-7.49 (m, 2H). 7.30 (s, 1H). 7.18-7.14 (m, 2H). 6.63 (s, 1H). 4.15-4.13 (q, *J* = 6.4 Hz, 1H). 3.67 (s, 3H). 1.78-1.76 (d, *J* = 6.8 Hz, 3H). 1.32 (s, 1H). 1.14-1.12 (dd, *J* = 4.8 Hz, 2H). 1.03-1.01 (d, *J* = 8.0 Hz, 2H).

**Embodiment 4081: 10-((Ethylsulfonyl)methyl)-7-(4-flurophenyl)-2,5-dimethyl-2,5-dihydro-3*H*-benzo[*c*]pyridazino[3,4-*e*]azepin-3-ketone**

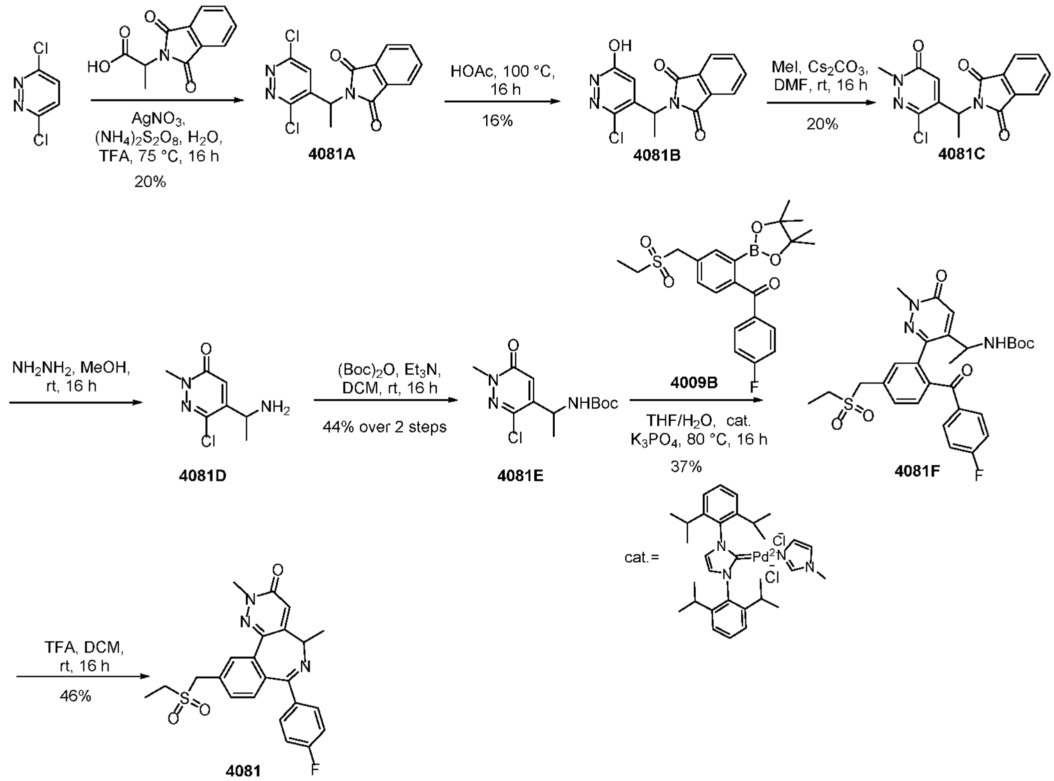


Step 1: 2-(1-(3,6-Dichloropyridazine-4-yl)ethyl)isoindol-1,3-diketone

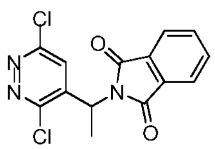

At 70°C, ammonium persulfate (4.1 g, 18 mmol) aqueous solution (6 mL) was slowly added into 3,6-dichloropyridazine (1.5 g, 10 mmol), 2-(1,3-dioxo-isoindol-2-yl)propionic acid (3.72g, 17 mmol), silver nitrite (167mg, 1 mmol) and TFA (230 mg, 2 mmol) aqueous solution (6 mL), the mixutre obtained was stirred at 70°C overnight. The reaction mixture was cooled down to rt, diluted by EtOAc (40 mL), neutralized with ammonium hydroxide (pH = 9.0). The organic phase was dried over anhydrous sodium sulfate, filtered and concentrated. The residue was purified by flash chromatography (PE/EtOAc=1/1). The title compound was obtained (650 mg, 20%), which was in form of yellow solid. LCMS (ESI) $[M+1]^+$ = 321.9.

Step 2: 2-(1-(3-Chloro-6-hydroxylpyridazine-4-yl)ethyl)isoindol-1,3-diketone

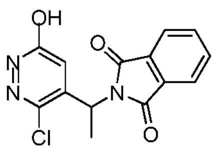

2-(1-(3,6-Dichloropyridazine-4-yl)ethyl)isoindol-1,3-diketone (1.0 g, 3.1 mmol) was dissolved in acetic acid (10 mL), and heated to 100°C and stirred overnight. Concentration of the reaction mixture, the residue was purified by flash chromatography (PE/EtOAc=1/1). The title compound was obtained (150 mg, 16%), which was in form of white solid. LCMS (ESI) $[M+1]^+$ = 303.9.

Step 3: 2-(1-(3-Chloro-1-methyl-6-oxo-1,6-dihydropyridazine-4-yl)ethyl)isoindol-1,3 -diketone

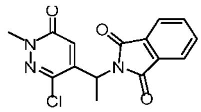

2-(1-(3-chloro-6-hydroxylpyridazine-4-yl)ethyl)isoindol-1,3-diketone (1.21 g, 4 mmol), methyl iodide (681 mg, 4 mmol) and barium carbonate (2.6 g, 12 mmol) were mixed in N,N-dimethylformamide (5 mL), then stirred at r.t. overnight. Water and EtOAc (20 mL) was added into the reaction mixture, the organic phase was separated. The organic phase was dried over anhydrous sodium sulfate, filtered and concentrated, the residue was purified by flash chromatography (PE/EtOAc=1/1). The title compound was obtained (250 mg, 20%), in form of yellow oil. LCMS (ESI) $[M+1]^+$ = 317.9.

Step 4:5-(1-Aminoethyl)-6-chloro-2-methyl pyridazine-3(2*H*)-ketone

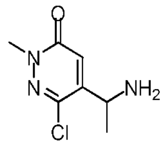

2-(1-(3-Chloro-1-methyl-6-oxo-1,6-dihydropyridazine-4-yl)ethyl)isoindol-1,3-diketone (250 mg, 0.79 mmol) and hydrated dihydrogen (220 mg, 7.9 mmol) was dissolved in ethanol (10 mL), then stirred at r.t. overnight. Concentration of the reaction mixture, the crude product of the tiltle compound was obtained (147 mg), in form of yellow oil, and was used directly in the next step without purification. LCMS (ESI) $[M+H]^+$ = 187.9.

Step 5: 1-(3-Chloro-1-methyl-6-oxo-1,6-dihydropyridazine-4-yl)ethyltert-butylcarbamate

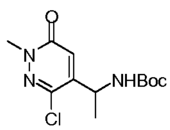

5-(1-Aminoethyl)-6-chloro-2-methylpyridazine-3(2*H*)-ketone (147 mg, 0.79 mmol). di-tert-butyl dicarbonate (343 mg, 1.56 mmol) and triethylamine (239 mg, 2.34 mmol) was dissolved in dichloromethane, then stirred at r.t. overnight. Concentration of the reaction mixture, the residue was purified by flash chromatography (PE/EtOAc=2/1). The title compound was obtained (100 mg, yield of the two steps 44%), which was in form of white solid. LCMS (ESI) $[M+H]^+$ = 288.0.

Step 6: 1-(3-(5-((ethylsulfonyl)methyl)-2-(4-fluorobenzoyl)phenyl)-1-methyl-6-oxo-1,6-dihydropyridazin e-4-yl)ethyl-*tert*-butylcarbamate

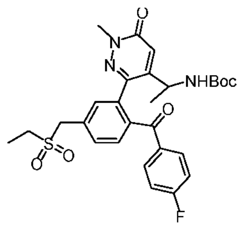

1-(3-Chloro-1-methyl-6-oxo-1,6-dihydropyridazine-4-yl)ethyltert-butylcarbamate (75 mg, 0.26 mmol), (4-((ethylsulfonyl)methyl)-2-(4,4,5,5-tetramethyl-1,3,2-dioxaborolan-2-yl)phenyl)(4-flurophenyl) methanone (168 mg, 0.39 mmol), [1,3-di(2,6-diisopropylphenyl)-1,3-dihydro-2*H*-imidazol-2-ylmethyl]dichloro[3-methyl-1*H*-imida zol]palladium (II) (17 mg, 0.026 mmol) and potassium phosphate (180 mg, 0.78 mmol) were mixed in THF (2 mL) and water (4 mL), and heated to 80°C and stirred overnight. Concentration of the reaction mixture, the residue was purified by flash chromatography (PE/EtOAc=1/2). The title compound was obtained (54 mg, 37%), which was in form of yellow solid. LCMS (ESI) $[M+1]^+$ = 558.0.

Step 7: 10-((Ethylsulfonyl)methyl)-7-(4-flurophenyl)-2,5-dimethyl-2,5-dihydro-3H-benzo[*c*]pyridazino[3, 4-*e*]azepin-3-ketone

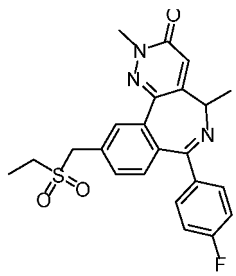

1-(3-(5-((Ethylsulfonyl)methyl)-2-(4-fluorobenzoyl)phenyl)-1-methyl-6-oxo-1,6-dihydr opyridazine-4-yl) tert-butylcarbonate (40 mg, 0.072 mmol) and TFA (1 mL) was dissolved in dichloromethane (10 mL), then stirred at r.t. overnight. Concentration of the reaction mixture, the residue was purified by preparative-HPLC, the title compound was obtained (14.5 mg, 46%) in form of white solid. LCMS (ESI) $[M+1]^+$ = 439.9; $^1$H NMR (400 MHz, *D*MSO-$d_6$) $\delta$ 7.93 (d, *J* = 1.2 Hz, 1H). 7.63 (dd, *J* = 2.0, 8.4 Hz, 1H). 7.54-7.47 (m, 2H). 7.38 (d, *J* = 8.0 Hz, 1H). 7.27-7.19 (m, 2H). 6.95 (d, *J* = 1.2 Hz, 1H). 4.75 (d, *J* = 13.6 Hz, 1H). 4.68 (d, *J* = 13.6 Hz, 1H). 4.07 (q, *J* = 6.8 Hz, 1H). 3.74 (s, 3H). 3.14 (qd, *J* = 1.2, 7.2 Hz, 2H). 1.68 (d, *J* = 6.8 Hz, 3H). 1.26 (t, *J* = 7.6 Hz, 3H).

**Embodiment 4082: 10-((ethylsulfonyl)methyl)-7-(4-flurophenyl)-5-(methoxylmethyl)-2-methyl-2,5-dihydro-3*H*-benzo[*c*]pyrido[3,4-*e*]azepin-3-ketone**

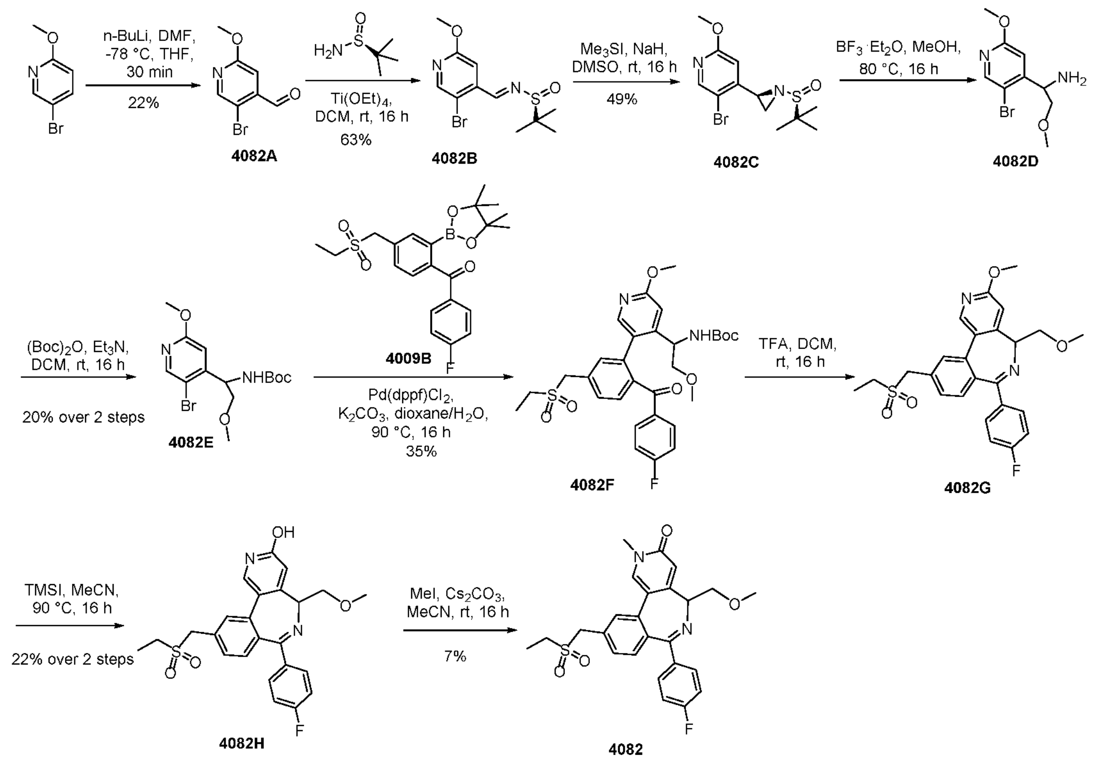


Step 1: 5-Bromo-2-methoxylpyridin-4-aldehyde

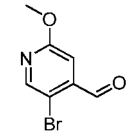

At -78°C and under nitrogen protection, n-butylithium (2.5 M in THF solution, 102.5 mL) was slowly added into diisopropylamine (26.1 g, 26 mmol) in THF (200 mL) solution, then stirred at -78°C for 30 minnutes. Added 5-bromo-2-methoxylpyridine (40 g, 21.27 mmol) in THF (40 mL) solution to the reaction mixture, the mixutre obtained was then stirred for 1 hour at 78°C. Then added *N,N*-dimethylformamide (18.9 g, 21.7 mmol) to the reaction mixture, then continued stirring at -78°C for 30 minutes. The reaction mixture was quenched by saturated ammonium chloride, extracted by EtOAc (200 mL). The organic phase was dried over anhydrous sodium sulfate, filtered and concentrated. The residue was recrystallized by EtOAc/PE (1/5), the title compound was obtained (10 g, 22%), which was in form of white solid. $^1$H NMR (400 MHz, $CDCl_3$) $\delta$ 10.29 (s, 1H). 8.41 (s, 1H). 7.17 (s, 1H). 3.96 (s, 3H).

Step 2: (*S,E*)-*N*-((5-bromo-2-methoxylpyridin-4-yl)methyl)-2-methylpropane-2-sulfenamide

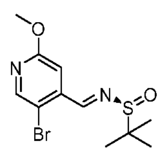

5-Bromo-2-methoxylpyridin-4-aldehyde (1.08 g, 5 mmol), (S)-2-methylpropane-2-sulfenamide (605 mg, 5 mmol) and tetraethyl titanate (110 mg, 0.5 mmol) was dissolved in dichloromethane (20 mL), then stirred at r.t. overnight. Concentration of the reaction mixture, the residue was purified by flash chromatography (PE/EtOAc=2/1). The title

compound was obtained (1 g, 63%), in form of yellow oil. LCMS (ESI) $[M+1]^+$ = 318.8.

Step 3: 5-Bromo-4-((*R*)-1-((*S*)-tert-butyl-sulfinyl)aziridin-2-yl)-2-methoxylpyridine

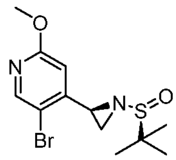

(*S,E*)-*N*-((5-Bromo-2-methoxylpyridin-4-yl)methyl)-2-methylpropane-2-sulfenamide (960 mg, 3 mmol) in dimethyl sulfoxide (5 mL) solution was added into sodium hydride (60% oily dispersed liquid, 360 mg, 9 mmol) and trimethylsulfur iodide (1.84 g, 9 mmol) in dimethylsulfoxide (30 mL) solution, the mixutre obtained was then stirred at r.t. overnight. Water and EtOAc (40 mL) was added into the reaction mixture, the organic phase was then separated. The organic phase was dried over anhydrous sodium sulfate, filtered and concentrated, the residue was purified by flash chromatography (PE/EtOAc=4/1). The title compound was obtained (488 mg, 49%), in form of yellow oil. LCMS (ESI) $[M+1]^+$ = 332.9.

Step 4: 1-(5-Bromo-2-methoxylpyridin-4-yl)-2-methoxylethylamine

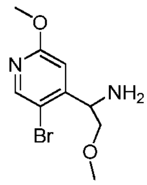

5-Bromo-4-((*R*)-1-((*S*)-tert-butyl-sulfinyl) aziridin-2-yl)-2-methoxylpyridine (90 mg, 0.3 mmol) and boron trifluoride etherate (42.6 mg, 0.3 mmol) was dissolved in methanol (5 mL), and heated to 80°C then stirred overnight. Concentration of the reaction mixture, the crude product of the tiltle compound was obtained (100 mg), in form of yellow oil. LCMS (ESI) $[M+H]^+$ = 260.9.

Step 5: 1-(5-Bromo-2-methoxylpyridin-4-yl)-2-methoxylethyl*tert*-butylcarbamate

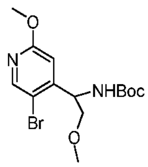

(5-Bromo-2-methoxylpyridin-4-yl)-2-methoxylethylamine (666 mg, 2 mmol), di-tert-butyl dicarbonate (864 mg, 4 mmol) and triethylamine (606 mg, 6 mmol) was dissolved in dichloromethane (5 mL), the reaction mixture was stirred at r.t. overnight, then concentrated. The residue was purified by flash chromatography (PE/EtOAc=4/1). The title compound was obtained (172 mg, yield of the two steps 20%), which was in form of white solid. LCMS (ESI) $[M+H]^+$ = 362.9.

Step 6: 1-(5-(5-((ethylsulfonyl)methyl)-2-(4-fluorobenzoyl)phenyl)-2-methoxylpyridin-4-yl)-2-methoxyle thyl*tert*-butylcarbamate

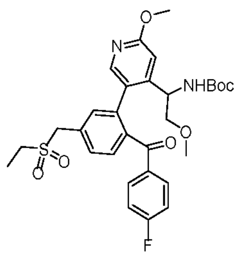

1-(5-bromo-2-methoxylpyridin-4-yl)-2-methoxylethyltert-butylcarbamate (55 mg, 0.15 mmol), (4-((ethylsulfonyl)methyl)-2-(4,4,5,5-tetramethyl-1,3,2-dioxaborolan-2-yl)phenyl)(4-flurophenyl) methanone (108 mg, 0.25 mmol), [1,1'-bis(diphenylphosphino)ferrocene]palladium (II) dichloride (15 mg, 0.15 mmol) and potassium carbonate (68 mg, 0.5 mmol) were mixed in 1,4-dioxane (5 mL) and water (0.5 mL), the reaction mixture was heated to 90°C then stirred overnight under nitrogen protection, then concentrated. The residue was purified by flash chromatography (PE/EtOAc=1/2-1/4). The title compound was obtained (31 mg, 35%), which was in form of yellow solid. LCMS (ESI) $[M+H]^+$ = 586.9.

Step 7: 10-((Ethylsulfonyl)methyl)-7-(4-flurophenyl)-3-methoxyl-5-(methoxylmethyl)-5*H*-benzo[*c*]pyrido [3,4-*e*]azepin

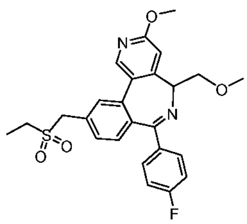

1-(5-(5-((Ethylsulfonyl)methyl)-2-(4-fluorobenzoyl)phenyl)-2-methoxylpyridin-4-yl)-2-methoxylethyltert-butylcarbamate (31 mg, 0.053 mmol) and TFA (1 mL) was dissolved in dichloromethane (10 mL), then stirred at r.t. overnight, the reaction mixture was concentrated, the crude product of the tiltle compound was obtained (50 mg), in form of yellow oil. LCMS (ESI) $[M+H]^+$ = 468.9.

Step 8: 10-((Ethylsulfonyl)methyl)-7-(4-flurophenyl)-5-(methoxylmethyl)-5H-benzo[*c*]pyrido[3,4-*e*]azepi n-3-ol

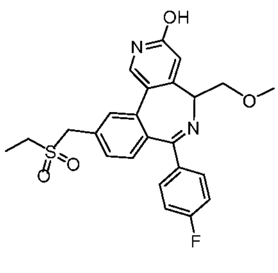

10-((Ethylsulfonyl)methyl)-7-(4-flurophenyl)-3-methoxyl-5-(methoxylmethyl)-5*H*-benz o[c]pyrido[3,4-e]azepine (50 mg, 0.1 mmol) and trimethyl iodosilane(100 mg, 0.5 mmol) was dissolved in acetonitrile (5 mL), and heated to 90°C then stirred overnight. Concentration of the reaction mixture, the residue was purified by flash chromatography (dichloromethane/methanol=10/1). The title compound was obtained (10 mg, yield of the two steps 22%), which was in form of yellow solid. LCMS (ESI) $[M+H]^+$ = 455.0.

Step 9: 10-((Ethylsulfonyl)methyl)-7-(4-flurophenyl)-5-(methoxylmethyl)-2-methyl-2,5-dihydro-3*H*-benz o[*c*]pyrido[3,4-*e*]azepin-3-ketone

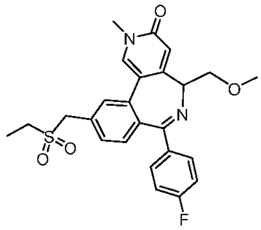

10-((Ethylsulfonyl)methyl)-7-(4-flurophenyl)-5-(methoxylmethyl)-5*H*-benzo[*c*]pyrido[3 ,4-*e*]azepin-3-ol (100 mg, 0.22 mol), methyl iodide (31 mg, 0.22 mmol) and barium carbonate (214.5 mg, 0.66 mmol) were mixed in acetonitrile (5 mL), then stirred at r.t. overnight. The reaction mixture was filtered and the filtrate was concentrated, the residue was purified by preparative-HPLC, the title compound was obtained (7.6 mg, 7%), which was in form of white solid. LCMS (ESI) $[M+H]^+$ = 469.0; $^1H$ NMR (400 MHz, *D*MSO-$d_6$) $\delta$ 8.04 (s, 1H). 7.72 (d, *J* = 1.2 Hz, 1H). 7.53-7.44 (m, 3H). 7.35 (d, *J* = 8.4 Hz, 1H). 7.24 (t, *J* = 8.8 Hz, 1H). 6.37 (s, 1H). 4.66 (d, *J* = 13.6 Hz, 1H). 4.57 (d, *J* = 13.6 Hz, 1H). 4.23-4.12 (m, 3H). 4.04-3.88 (m, 2H). 3.50 (s, 3H). 3.37 (s, 3H). 3.13 (q, *J* = 7.6 Hz, 2H). 1.25 (t, *J* = 7.2 Hz, 3H).

**Embodiment 4083: (*S*)-*N*-(9-fluoro-7-(4-flurophenyl)-2,5-dimethyl-3-oxo-3,5-dihydro-2*H*-benzo[*c*]pyrido[3,4-e]a zepin-10-yl)propanamide**

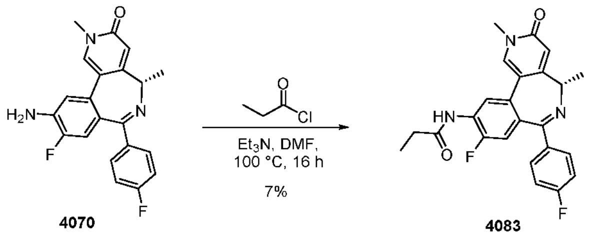


4070 4083

Propionyl chloride (92 mg, 1 mmol) was added into (*S*)-10-amino-9-fluoro-7-(4-flurophenyl)-2,5-dimethyl-2,5-dihydro-3*H*-benzo[*c*]pyrido[3,4-*e*]azep in-3-ketone (35 mg, 0.096 mmol) and triethylamine (100 mg, 1 mmol) in *N,N*-dimethylformamide (3 mL) solution. The reaction mixture and heated to 100°C then stirred overnight and concentrated, the residue was purified by preparative-HPLC, the title compound was obtained (3.0 mg, yield 7%), which was in form of white solid. LCMS (ESI) $[M+H]^+$ = 422.1; $^1H$ NMR (400 MHz, *D*MSO-$d_6$) $\delta$ 8.42 (d, *J* = 7.6 Hz, 1H). 8.07 (s, 1H). 7.53-7.49 (m, 2H). 7.17-7.07 (m, 3H). 6.62 (s, 1H). 4.13 (q, *J* = 6.4 Hz, 1H). 3.67 (s, 3H). 2.56 (q, *J* = 8.0 Hz, 2H). 1.77 (d, *J* = 6.4 Hz, 3H) 1.25 (q, *J* = 7.2 Hz, 3H).

**Embodiment 4084: (*S*)-*N*-(5-cyclopropyl-9-fluoro-7-(4-flurophenyl)-2-methyl-3-oxo-3,5-dihydro-2*H*-benzo[*c*]pyr ido[3,4-*e*]azepin-10-yl)ethylsulfonamide**

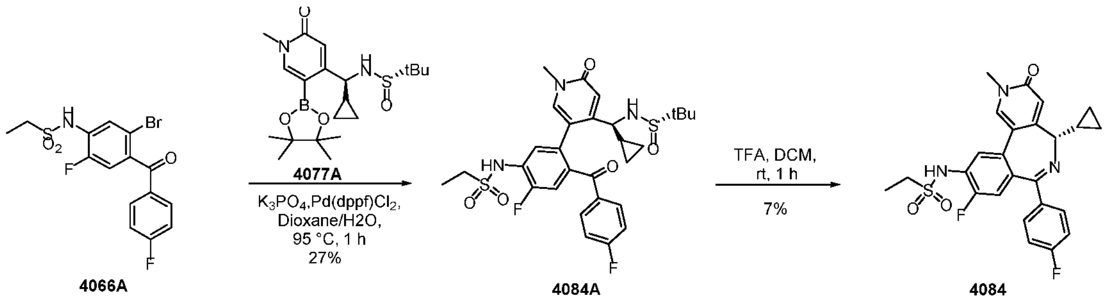


4066A 4084A 4084

Step 1: *N*-(5-(4-((*S*)-(((*S*)-*tert*-butyl-sulfinyl)amino)(cyclopropyl)methyl)-1-methyl-6-oxo-1,6-dihydropyri din-3-yl)-2-fluoro-4-(4-fluorobenzoyl)phenyl)ethylsulfonamide

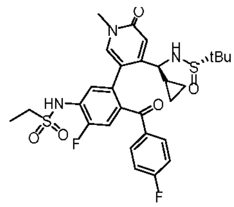

*N*-(5-Bromo-2-fluoro-4-(4-fluorobenzoyl)phenyl)ethylsulfonamide (200 mg, 0.49 mmol), (*S*)-*N*-((*S*)-cyclopropyl(1-methyl-2-oxo-5-(4,4,5,5-tetramethyl-1,3,2-dioxaborolan-2-yl)-1,2-dihyd ropyridin-4-yl)methyl)-2-methylpropane-2-sulfenamide (404 mg, 0.99 mmol), [1,1'-bis(diphenylphosphino)ferrocene]palladium (II) dichloride (36 mg, 0.05 mmol) and potassium phosphate (315 mg, 1.48 mmol) were mixed in 1,4-dioxane (4 mL) and water (0.2 mL), and heated to 95°C then stirred for 1 hour under nitrogen protection. The reaction mixture was filtered and the filtrate was concentrated, the residue was purified by flash chromatography (methanol /EtOAc=3/97). The title compound was obtained (80 mg, 27%), in form of yellow oil. LCMS (ESI) $[M+H]^+$ = 606.0.

Step 2: (*S*)-*N*-(5-cyclopropyl-9-fluoro-7-(4-flurophenyl)-2-methyl-3-oxo-3,5-dihydro-2*H*-benzo[*c*]pyrido[ 3,4-*e*]azepin-10-yl)ethylsulfonamide

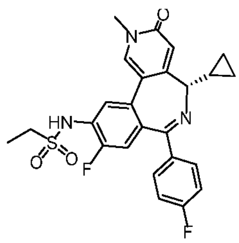

TFA (1 mL) was added into *N*-(5-(4-((*S*)-(((*S*)-tert-butyl-sulfinyl)amino)(cyclopropyl)methyl)-1-methyl-6-oxo-1,6-dihydropyri din-3-yl)-2-fluoro-4-(4-fluorobenzoyl)phenyl)ethylsulfonamide (80 mg, 0.13 mmol) in dichloromethane (4 mL) solution, the reaction mixture was then stirred at r.t. for 1 hour then concentrated. The residue was purified by preparative-HPLC, the title compound was obtained (4.4 mg, 7%), which was in form of white solid. LCMS (ESI) $[M+H]^+$ = 484.2; $^1H$ NMR (400 MHz, $CD_3OD$) $\delta$ 8.53 (s, 1H). 8.02 (s, 1H). 7.81-7.79 (d, *J* = 7.6 Hz, 3H). 7.55-7.51 (m, 2H). 7.18-7.09 (m, 3H). 6.96 (s, 1H). 3.70-3.68 (d, *J* = 6.8 Hz, 3H). 3.45-3.43 (d, *J* = 7.6 Hz, 1H). 3.31-3.12 (m, 2H). 1.71-1.65 (m, 1H). 1.43-1.38 (m, 3H). 0.88-0.75 (m, 2H). 0.68-0.51 (m, 1H). 0.39-0.32 (m, 1H).

**Embodiment 4085:** (*S*)-1**0-((ethylsulfonyl)methyl)-9-fluoro-7**-(**4-flurophenyl)-2,5-dimethyl-2,5-dihydro-3*H*-benzo [*c*]pyrido[3,4-*e*]azepin-3-ketone**

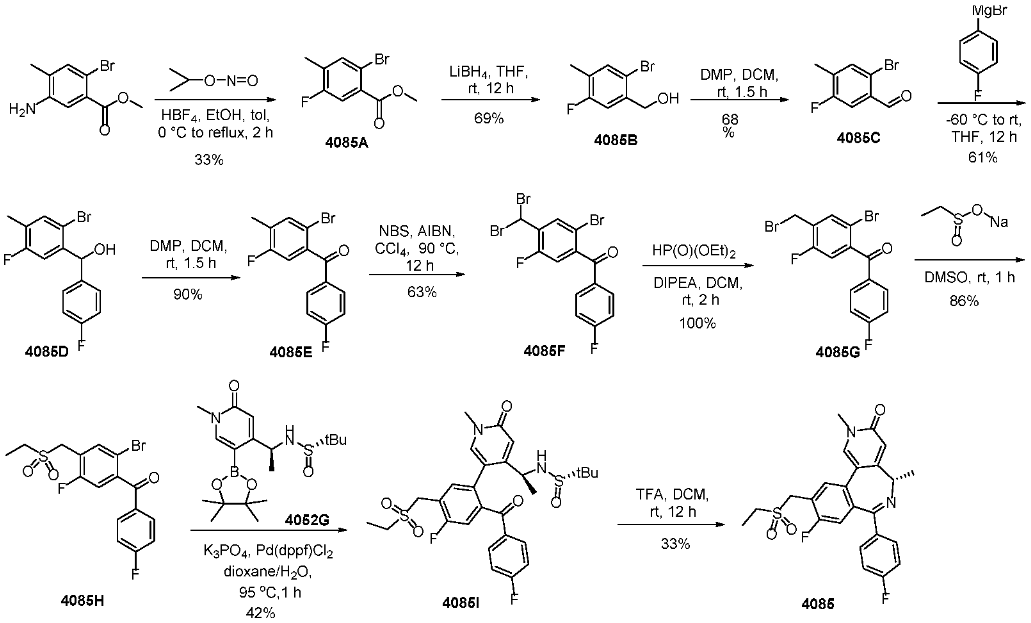


Step 1: 2-Bromo-5-fluoro-4-methylmethylbenzoate

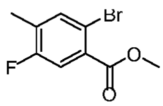

At 0°C, fluoroboric acid (7.2 g, 32.8 mmol) was added into 5-amino-2-bromo-4-methylmethylbenzoate (2.0 g, 8.2 mmol) and isopropyl nitrite (1.5 g, 16.4 mmol) in ethanol (10 mL) solution. The reaction mixture was stirred at 0°C for 30 minutes, then added to refluxing toluene (10 mL), then stirred for 2 hours at this temperature. The reaction mixture was then concentrated, the residue was purified by flash chromatography (PE). The title compound was obtained (650 mg, 33%), which was in form of yellow solid.GCMS [M] = 246.0.

Step 2: (2-Bromo-5-fluoro-4-methylphenyl)methanol

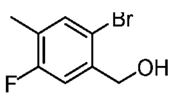

Lithium borohydride (2 M in THF solution, 4 mL, 7.9 mmol) was added slowly into 2-bromo-5-fluoro-4-methylmethylbenzoate (650 mg, 2.6 mmol) in THF (12 mL) solution. The reaction mixture was stirred at r.t. under nitrogen protection for 12 hours, then quenched by saturated ammonium chloride (20 mL), and extracted by EtOAc (20 mL). The organic phase was washed by saturated brine and water, dried over anhydrous sodium sulfate, filtered and concentrated. The residue was purified by flash chromatography (PE/EtOAc=20/1). The title compound was obtained (400 mg, 69%), which was in form of white solid. LCMS (ESI) $[M+H]^+$ = 200.9.

Step 3:2-Bromo-5-fluoro-4-methylbenzaldehyde

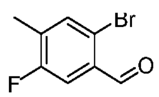

2-Bromo-5-fluoro-4-methylphenyl)methanol (400 mg, 1.83 mmol) and Dess-Martin periodinane (1.2 g, 2.74 mmol) were mixed in dichloromethane (8 mL), was stirred at r.t. for 1.5 hours. The reaction mixture was filtered and the filtrate was concentrated, the residue was purified by flash chromatography (PE/EtOAc=50/1). The title compound was obtained (270 mg, 68%), which was in form of yellow solid. LCMS (ESI) $[M+H]^+$ = 216.8.

Step 4: (2-Bromo-5-fluoro-4-methylphenyl)(4-flurophenyl)methanol

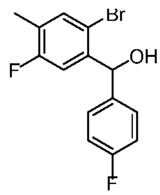

At -60°C and under nitrogen protection, (4-flurophenyl)magnesium bromide (1 M in THF solution, 7 mL) was added into 2-bromo-5-fluoro-4-methylbenzaldehyde (530 mg, 2.44 mmol) in THF (8.0 mL) solution. The reaction mixture was stirred at r.t. for 12 hours, then quenched by saturated ammonium chloride (100 mL), and extracted by EtOAc (20 mL). The organic phase was dried over anhydrous sodium sulfate, filtered and concentrated, the residue was purified by flash chromatography (PE/EtOAc=5/1). The title compound was obtained (470 mg, 61%), which was in form of yellow solid. LCMS (ESI) $[M+H]^+$ = 295.8.

Step 5: (2-Bromo-5-fluoro-4-methylphenyl)(4-flurophenyl)methanone

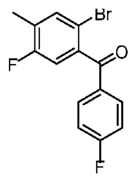

(2-Bromo-5-fluoro-4-methylphenyl)(4-flurophenyl)methanol (470 mg, 1.5 mmol) and Dess-Martin periodinane (955 mg, 2.3 mmol) were mixed in dichloromethane (8 mL), was stirred at r.t. for 1.5 hours. The reaction mixture was filtered and the filtrate was concentrated, the residue was purified by flash chromatography (PE/EtOAc=50/1). The title compound was obtained (420 mg, 90%), which was in form of yellow solid. LCMS (ESI) $[M+H]^+$ = 310.8.

Step 6: (2-Bromo-4-(dibromomethyl)-5-flurophenyl)(4-flurophenyl)methanone

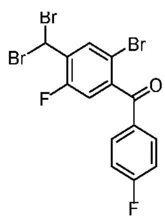

*N*-Bromosuccinimide (721 mg, 4.05 mmol), azobisisobutyronitrile (44 mg, 0.27 mmol) and (2-bromo-5-fluoro-4-methylphenyl)(4-flurophenyl)methanone (420 mg, 1.35 mmol) were mixed in tetrachloromethane (8 mL), and heated to 90°C and stirred for 12 hours. Concentration of the reaction mixture, the residue was purified by flash chromatography (PE/EtOAc=30/1). The title compound was obtained (400 mg, 63%), which was in form of yellow solid. LCMS (ESI) $[M+H]^+$= 468.6.

Step 7: (2-Bromo-4-(bromomethyl)-5-flurophenyl)(4-flurophenyl)methanone

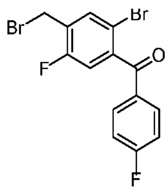

(2-Bromo-4-(dibromomethyl)-5-flurophenyl)(4-flurophenyl)methanone (400 mg, 0.85 mmol), triethyl phosphate (177 mg, 1.28 mmol) and diisopropyl ethylamine (165 mg, 1.28 mmol) were dissolved in dichloromethane (15 mL), then stirred at r.t. for 2 hours. Concentration of the reaction mixture, the residue was purified by flash chromatography (PE/EtOAc=50/1). The title compound was obtained (450 mg, 100%), which was in form of yellow solid. LCMS (ESI) $[M+H]^+$ = 388.7.

Step 8: (2-Bromo-4-((ethylsulfonyl)methyl)-5-flurophenyl)(4-flurophenyl)methanone

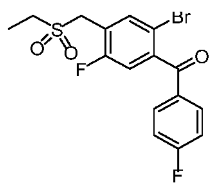

(2-Bromo-4-(bromomethyl)-5-flurophenyl)(4-flurophenyl)methanone (450 mg, 1.15 mmol) and sodium ethanesulfinate (268 mg, 2.31 mmol) were mixed in dimethylsulfoxide (6 mL), then stirred at r.t. for 1 hour. EtOAc (30 mL) and water (100 mL) was added into the reaction mixture, the organic phase was separated. The organic phase was washed by saturated brine (30 mL*3), dried over anhydrous sodium sulfate, filtered and concentrated, the title compound was obtained (400 mg, 86%), which was in form of yellow solid. LCMS (ESI) $[M+H]^+$ = 402.8.

Step 9: (*S*)-*N*-((S)-1-(5-(5-((ethylsulfonyl)methyl)-4-fluoro-2-(4-fluorobenzoyl)phenyl)-1-methyl-2-oxo-1, 2-dihydropyridin-4-yl)ethyl)-2-methylpropane-2-sulfenamide

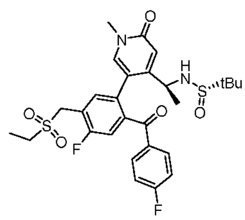

(2-Bromo-4-((ethylsulfonyl)methyl)-5-flurophenyl)(4-flurophenyl)methanone (200 mg, 0.50 mmol), (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide (284 mg, 0.74 mmol), [1,1'-bis(diphenylphosphino)ferrocene]palladium (II) dichloride (36 mg, 0.05 mmol) and potassium phosphate (315 mg, 1.49 mmol) were mixed in 1,4-dioxane (6 mL) and water (0.3 mL), and heated to 95°C then stirred for 1 hour under nitrogen protection. The reaction mixture was filtered and the filtrate was concentrated, the residue was purified by flash chromatography (dichloromethane/methanol=3/97). The title compound was obtained (120 mg, 42%), which was in form of yellow solid. LCMS (ESI) $[M+H]^+$ = 578.9.

Step 10: (*S*)-10-((Ethylsulfonyl)methyl)-9-fluoro-7-(4-flurophenyl)-2,5-methyl-2,5-dihydro-3*H*-benzo[*c*]py rido[3,4-*e*]azein-3-ketone

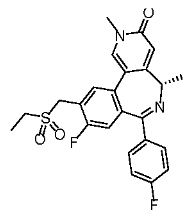

TFA (2 mL) was added into (*S*)-*N*-((*S*)-1-(5-(5-((ethylsulfonyl)methyl)-4-fluoro-2-(4-fluorobenzoyl)phenyl)-1-methyl-2-oxo-1, 2-dihydropyridin-4-yl)ethyl)-2-methylpropane-2-sulfenamide (120 mg, 0.20 mmol) in dichloromethane (8 mL) solution. The reaction mixture was stirred at r.t. for 12 hours and concentrated, the residue was purified by preparative-HPLC, the tiltle compound was obtained (31.4 mg, 33%), which was in form of white solid. LCMS (ESI) $[M+H]^+$ = 457.0; $^1H$ NMR (400 MHz, $CD_3OD$) $\delta$ 8.07 (s, 1H). 7.84-7.82 (d, *J* = 7.2 Hz, 1H). 7.56-7.52 (m, 2H). 7.20-7.13 (m, 3H). 6.64 (s, 1H). 4.65 (s, 2H). 4.16-4.11 (m, 1H). 3.69-3.67 (d, *J* = 7.2 Hz, 3H). 3.27-3.11 (m, 2H). 1.79-1.77 (d, *J* = 6.4 Hz, 3H). 1.47-1.41 (m, 3H).

**Embodiment 4086: (*S*)-9-chloro-10-((ethylsulfonyl)methyl)-7-(4-flurophenyl)-2,5-dimethyl-2,5-dihydro-3*H*-benz o[*c*]pyrido[3,4-*e*]azepin-3-ketone**

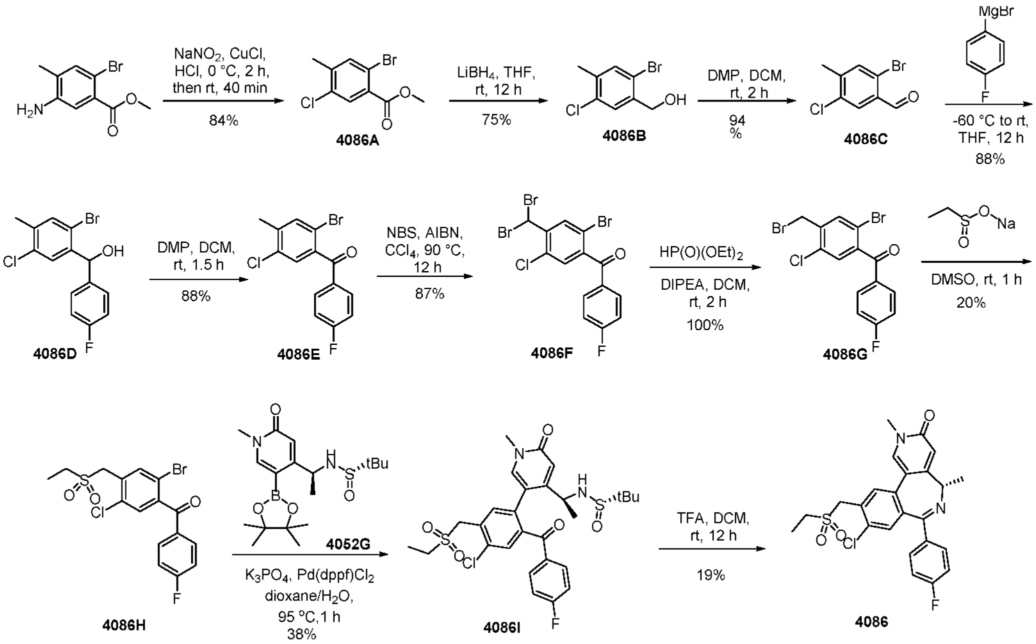


Step 1: 2-Bromo-5-chloro-4-methylmethylbenzoate

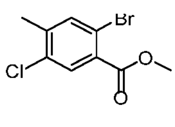

At 15°C, concentrated HCl (4.5 mL) was added into 5-amino-2-bromo-4-methylmethylbenzoate (1 g, 4.1 mmol)

in 1,4-dioxane (7 mL) solution, the mixutre obtained was cooled down to 5°C. sodium nitrite (292 mg, 4.23 mol) in water (5 mL) solution was added into the reaction solution, the temperature was kept under 0°C during the addition. The reaction mixture was then stirred for 2 hours at 0°C, then the reaction mixture was slowly added dropwise to the reaction bottle containing concentrated HCl (2.3 mL) and cuprous chloride (0.49 mg, 0.004 mmol) at rt. The mixutre obtained was continued stirring for 40 minutes. The reaction mixture was poured into ice water (100 mL) and filtered, the residue was dissolved in EtOAc (70 mL), washed by saturated brine (500 mL*3), dried over anhydrous sodium sulfate, filtered and concentrated. The residue was purified by flash chromatography (PE/EtOAc=20/1). The title compound was obtained (900 mg, 84%), which was in form of white solid. GCMS [M] = 262.0.

Step 2: (2-Bromo-5-chloro-4-methlhenl)methanol

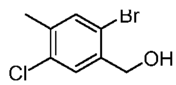

Lithium borohydride (2 M in THF solution, 2.5 mL, 5.1 mmol) was slowly added into 2-bromo-5-chloro-4-methylmethylbenzoate (900 mg, 3.4 mmol) in THF (12 mL) solution. The reaction mixture and was stirred at r.t. under nitrogen protection for 12 hours. The reaction was quenched by saturated ammonium chloride (20 mL), and extracted by EtOAc (20 mL). The organic phase was washed by saturated brine and water, dried over anhydrous sodium sulfate, filtered and concentrated. The residue was purified by flash chromatography (PE/EtOAc=20/1). The title compound was obtained (600 mg, 75%), which was in form of white solid. LCMS (ESI) $[M+H]^+$ = 233.9.

Step 3: 2-Bromo-5-chloro-4-methylbenzaldehyde

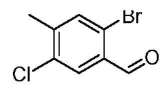

(2-Bromo-5-chloro-4-methylphenyl)methanol (700 mg, 2.97 mmol) and Dess-Martin periodinane (1.5 g, 4.5mmol) were mixed in dichloromethane (10 mL), then stirred at r.t. for 2 hours. The reaction mixture was filtered and the filtrate was concentrated, the residue was purified by flash chromatography (PE/EtOAc=50/1). The title compound was obtained (650 mg, 94%), which was in form of yellow solid. LCMS (ESI) $[M+H]^+$ = 231.9.

Step 4: (2-Bromo-5-chloro-4-methylphenyl)(4-flurophenyl)methanol

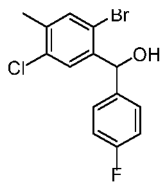

At -60°C and under nitrogen protection, (4-flurophenyl)magnesium bromide (1 M in THF solution, 8.4 mL) was added dropwise into 2-bromo-5-chloro-4-methylbenzaldehyde (650 mg, 2.78 mmol) in THF (8.0 mL). The reaction mixture was raised to r.t. and stirred for 12 hours, then quenched by saturated ammonium chloride (100 mL), extracted by EtOAc (30 mL*3). The organic phase was dried over anhydrous sodium sulfate, filtered and concentrated, the residue was purified by flash chromatography (PE/EtOAc=5/1). The title compound was obtained (800 mg, 88%), which was in form of yellow solid. LCMS (ESI) $[M+H]^+$ = 325.9.

Step 5: (2-Bromo-5-chloro-4-methylphenyl)(4-flurophenyl)methanone

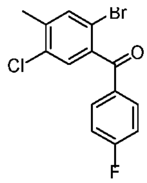

(2-Bromo-5-chloro-4-methylphenyl)(4-flurophenyl)methanol (800 mg, 2.43 mmol) and Dess-Martin periodinane (1.6 g, 3.77 mmol) were mixed in dichloromethane (8 mL), was stirred at r.t. for 1.5 hours. The reaction mixture was filtered and the filtrate was concentrated, the residue was purified by flash chromatography (PE/EtOAc=50/1). The title compound was obtained (700 mg, 88%), which was in form of yellow solid. LCMS (ESI) $[M+H]^+$ = 310.8.

Step 6: (2-Bromo-5-chloro-4-(dibromomethyl)phenyl)(4-flurophenyl)methanone

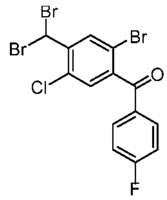

*N*-Bromosuccinimide (1.39 g, 7.8 mmol), azobisisobutyronitrile (73 mg, 0.44 mmol) and (2-bromo-5-chloro-4-methylphenyl)(4-flurophenyl)methanone (700 mg, 2.13 mmol) were dissolved in tetrachloromethane (8 mL), and heated to 90°C then stirred for 12 hours. Concentration of the reaction mixture, the residue was purified by flash chromatography (PE/EtOAc=30/1). The title compound was obtained (900 mg, 87%), which was in form of yellow solid. LCMS (ESI) $[M+H]^+$ = 482.5.

Step 7: (2-Bromo-4-(bromomethyl)-5-chlorophenyl)(4-flurophenyl)methanone

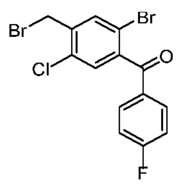

(2-Bromo-5-chloro-4-(dibromomethyl)phenyl)(4-flurophenyl)methanone (900 mg, 1.85 mmol), triethyl phosphate (460 mg, 3.3mmol) and diisopropyl ethylamine (432 mg, 3.3mmol) were dissolved in dichloromethane (15 mL), then stirred at r.t. for 2 hours. Concentration of the reaction mixture, the residue was purified by flash chromatography (PE/EtOAc=50/1). The title compound was obtained (950 mg, 100%), which was in form of yellow solid. LCMS (ESI) $[M+H]^+$= 404.6.

Step 8: (2-Bromo-5-chloro-4-((ethlsulfonl)methyl)phenyl)(4-flurohenyl)methanone

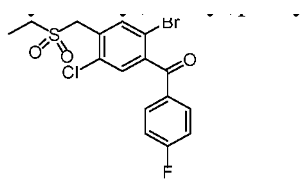

(2-Bromo-4-(bromomethyl)-5-chlorophenyl)(4-flurophenyl)methanone (950 mg, 2.33 mmol) and sodium ethanesulfinate (545 mg, 4.69 mmol) were mixed in dimethylsulfoxide (6 mL), then stirred at r.t. for 1 hour. EtOAc (30 mL) and water (50 mL) were added into the reaction solution, the organic phase was separated. The organic phase was washed by saturated brine (30 mL*3), dried over anhydrous sodium sulfate, filtered and concentrated, the title compound was obtained (200 mg, 20%), which was in form of yellow solid. LCMS (ESI) $[M+H]^+$= 419.8.

Step 9: (*S*)-*N*-((*S*)-1-(5-(4-chloro-5-((ethylsulfonyl)methyl)-2-(4-fluorobenzoyl)phenyl)-1-methyl-2-oxo-1 ,2-dihydropyridin-4-yl)ethyl)-2-methylpropane-2-sulfenamide

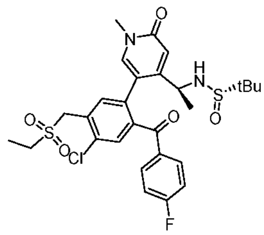

(2-Bromo-5-chloro-4-((ethylsulfonyl)methyl)phenyl)(4-flurophenyl)methanone (200 mg, 0.47 mmol), (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide (207 mg, 0.53 mmol), [1,1'-bis(diphenylphosphino)ferrocene]palladium (II) dichloride (40 mg, 0.05 mmol) and potassium phosphate (342 mg, 1.6 mmol) were mixed in 1,4-dioxane (5 mL) and water (0.5 mL), and heated to 95 °C then stirred for 1 hour under nitrogen protection. The reaction mixture was filtered and the filtrate was concentrated, the residue was purified by flash chromatography (dichloromethane/methanol=3/97). The title compound was obtained (80 mg, 38%), which was in form of yellow solid. LCMS (ESI) $[M+H]^+$ = 595.0.

Step 10: (*S*)-9-Chloro-10-((ethylsulfonyl)methyl)-7-(4-flurophenyl)-2,5-dimethyl-2,5-dihydro-3*H*-benzo[*c*] pyrido[3,4-*e*]azepin-3-ketone

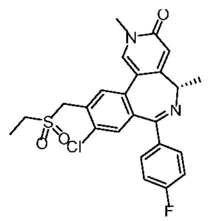

TFA (2 mL) was added into ((*S*)-*N*-((*S*)-1-(5-(4-chloro-5-((ethylsulfonyl)methyl)-2-(4-fluorobenzyl formamide) phenyl)-1-methyl-2-oxo-1,2-dihydropyridin-4-yl)ethyl)-2-methylpropane-2-sulfenamide (80 mg, 0.13 mmol) in dichloromethane (8 mL) solution. The reaction mixture was stirred at r.t. for 12 hours then concentrated. The residue was purified by preparative-HPLC, the title compound was obtained (12 mg, 19%), which was in form of white solid. LCMS (ESI) $[M+H]^+$ = 473.0; $^1H$ NMR (400 MHz, *D*MSO-$d_6$) $\delta$ 8.03 (s, 1H). 7.84 (s, 1H). 7.50-7.47 (m, 2H). 7.40 (s, 1H). 7.24-7.22 (m, 2H). 6.4 (s, 1H). 4.81-4.78 (d, *J* = 13.2 Hz, 1H). 4.65-4.62 (d, *J* = 11.6 Hz, 1H). 4.00-3.99 (q *J* = 6.4 Hz, 1H). 3.50 (s, 3H). 3.42-3.32 (q, *J* = 1.2 Hz, 2H). 1.66-1.65 (d, *J* = 6.0 Hz, 3H). 1.32-1.28 (t, *J* = 7.6 Hz, 3H).

**Embodiment 4087: (*S*)-10-((ethylsulfonyl)methyl)-7-(4-fluoro-2-methoxylphenyl)-2,5-dimethyl-2,5-dihydro-3*H*-benzo[*c*]pyrido[3,4-*e*]azepin-3-ketone**

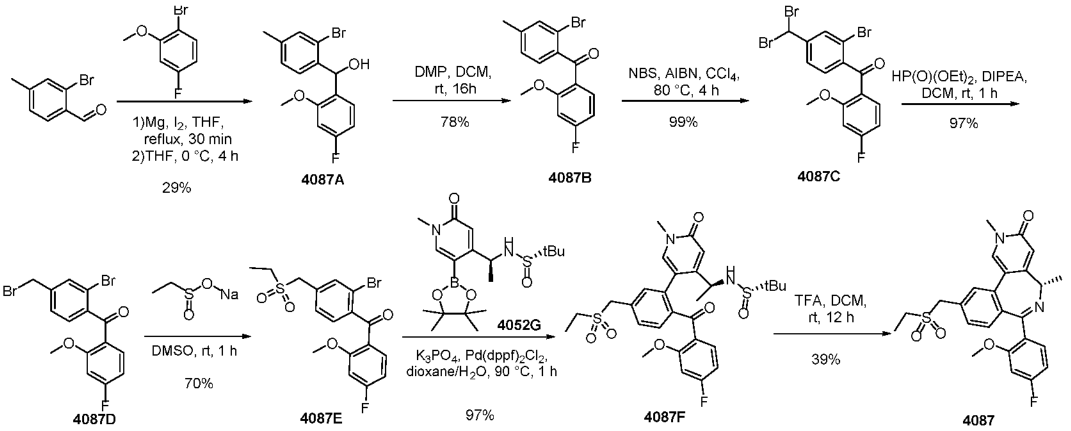


Step 1: (2-Bromo-4-methylphenyl)(4-fluoro-2-methoxylphenyl)methanol

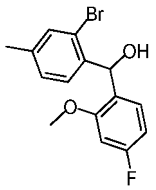

Under nitrogen protection, THF (1 mL) was added into magnesium spalls (173 mg, 7.2 mmol) and a small piece of $I_2$, then slowly added a small amount of 1-bromo-4-fluoro-2-methoxylbenzyl (1.5 g, 7.2 mmol) in THF (6 mL) solution. After the reaction was started, the reaction mixture was heated to 60°C, then added the rest of the materials. The reaction mixture continued to reflux, after 30 minutes to obtaine the Griganard reagent. 2-bromo-4-methylbenzaldehyde (0.7 g, 3.6 mmol) in THF (2 mL) solution then cooled down to 0°C, and slowly added the Griganard reagent. The reaction mixture was stirred at r.t. for 4 hours, then use EtOAc and ammonium chloride solution to separated the phases. The organic phase was washed by brine, dried over anhydrous sodium sulfate, filtered and concentrated. The residue was purified by flash chromatography (PE/EtOAc=20/1). The title compound was obtained (0.3 g, 29%), in form of colorless oil. MS (ESI): *m/z* = 307.0; 309.0 $[M-18]^+$.

Step 2: (2-Bromo-4-methylphenyl)(4-fluoro-2-methoxylphenyl)methanone

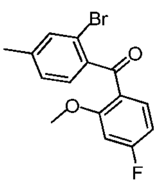

(2-Bromo-4-methylphenyl)(4-fluoro-2-methoxylphenyl)methanol (0.3 g, 1.0 mmol) and Dess-Martin periodinane (0.6 g, 1.5 mmol) were mixed in dichloromethane (15 mL), stirred at r.t. for 12 hours. The reaction mixture was filtered and the filtrate was concentrated, the title compound was obtained (0.25 g, 78%), which was in form of white solid, and was used directly in the next step without purification. MS (ESI): *m/z* = 323.0; 325.0 $[M+H]^+$; $^1H$ NMR (400 MHz, *D*MSO-*d*$_6$) $\delta$7.57 (m, 1H). 7.53 (s, 1H). 7.26 (s, 2H). 7.08 (dd, *J* = 10.4 Hz, 2.0 Hz, 2H). 6.91 (m, 1H). 3.63 (s, 3H). 2.36 (s, 3H).

Step 3: (2-Bromo-4-(dibromomethyl)phenyl)(4-fluoro-2-methoxylphenyl)methanone

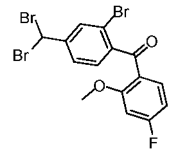

*N*-Bromosuccinimide (0.4 g, 2.3 mmol), azobisisobutyronitrile (13 mg, 0.08 mmol) and (2-bromo-4-methylphenyl)(4-fluoro-2-methoxylphenyl)methanone (0.25 g, 0.8 mmol) was dissolved in tetrachloromethane (5 mL), and heated to 80°C and stirred for 4 hours. Evaporated the solvent under reduced pressure to obtain the tiltle compound (0.37 g, 99%), which was in form of yellow solid, and was used directly in the next step without purification. MS (ESI): *m/z* = 479.0; 481.0; 483.0 $[M+H]^+$.

Step 4: (2-Bromo-4-(bromomethyl)phenyl)(4-fluoro-2-methoxylphenyl)methanone

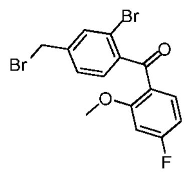

Triethyl phosphate (159 mg, 1.2 mmol), diisopropyl ethylamine (149 mg, 1.2 mmol) and (2-bromo-4-(dibromomethyl)phenyl)(4-fluoro-2-methoxylphenyl)methanone (369 mg, 0.8 mmol) were dissolved in dichloromethane (25 mL), then stirred at r.t. for 1 hour. Evaporated the solvent under reduced pressure, the residue was purified by flash chromatography (PE/EtOAc=1/1). The title compound was obtained (300 mg, 97%), which was in form of white solid. MS (ESI): *m/z* = 401.0; 403.0; 405.0 $[M+H]^+$.

Step 5: (2-Bromo-4-((ethylsulfonyl)methyl)phenyl)(4-fluoro-2-methoxylphenyl)methanone

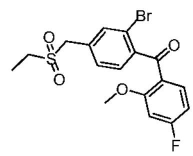

Sodium ethanesulfinate (173 mg, 1.5 mmol) was added into (2-bromo-4-(bromomethyl)phenyl)(4-fluoro-2-methoxylphenyl)methanone (300 mg, 0.8 mmol) in dimethylsulfoxide (3 mL) solution. The reaction mixture was stirred at r.t. for 1 hour. EtOAc (10 mL) and water (25 mL) were added into the reaction mixture, the organic phase was separated. The organic phase was washed by saturated brine (10 mL*3), dried over anhydrous sodium sulfate, filtered and concentrated, the residue was purified by flash chromatography (PE/EtOAc=1/1). The title compound was obtained (220 mg, 70%), in form of colorless oil. MS (ESI): *m/z* = 415.0; 417.0; 418.0 $[M+H]^+$.

Step 6: (*S*)-*N*-((*S*)-1-(5-(5-((ethylsulfonyl)methyl)-2-(4-fluoro-2-methoxylbenzoyl)phenyl)-1-methyl-2-ox o-1,2-dihydropyridin-4-yl)ethyl)-2-methylpropane-2-sulfenamide

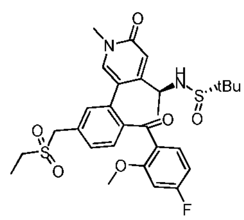

(2-Bromo-4-((ethylsulfonyl)methyl)phenyl)(4-fluoro-2-methoxylphenyl)methanone (220 mg, 0.5 mmol), (*S*)-2-methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (309 mg, 0.8 mmol), [1,1'-bis(diphenylphosphino)ferrocene]palladium (II) dichloride (40 mg, 0.05 mmol) and potassium phosphate (343 mg, 1.6 mmol) were mixed in 1,4-dioxane (5 mL) and water (0.2 mL), and heated to 90°C then stirred for 1 hour under nitrogen protection. The reaction mixture was filtered and the filtrate was concentrated, the residue was purified by flash chromatography (EtOAc/methanol=30/1). The title compound was obtained (60 mg, 20%), in form of yellow oil. MS (ESI): *m/z* = 591.0; 592.0; 593.0 $[M+H]^+$.

Step 7: (*S*)-10-((ethylsulfonyl)methyl)-7-(4-fluoro-2-methoxylphenyl)-2,5-dimethyl-2,5-dihydro-3*H*-benz o[c]pyrido[3,4-*e*]azepin-3-ketone

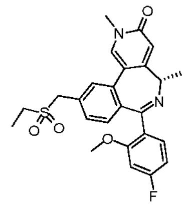

TFA (1 mL) was added into (*S*)-*N*-((*S*)-1-(5-(5-((ethylsulfonyl)methyl)-2-(4-fluoro-2-methoxylbenzoyl)phenyl)-1-methyl-2-oxo -1,2-dihydropyridin-4-yl)ethyl)-2-methylpropane-2-sulfenamide (60 mg, 0.1 mmol) in dichloromethane (4 mL) solution. The reaction mixture was stirred 12 hours at r.t. and then concentrated, the residue was purified by preparative-HPLC, the title compound was obtained (18.5 mg, 39%), which was in form of white solid. MS (ESI): *m/z* = 469.0; 470.0 $[M+H]^+$. $^1H$ NMR (400 MHz, $CD_3OD$) $\delta$ 8.12 (s, 1H). 7.74 (d, *J* = 1.6 Hz, 1H). 7.47 (m, 2H). 7.23 (d, *J* = 7.6 Hz, 1H). 6.81 (m, 2H). 6.64 (s, 1H). 4.56 (s, 2H). 4.15 (d, *J* = 6.0 Hz, 1H). 3.71 (s, 3H). 3.37 (s, 3H). 3.16 (q, *J* = 3.6 Hz, 2H). 1.76 (d, *J* = 6.4 Hz, 3H). 1.41 (t, *J* = 7.2 Hz, 3H).

**Embodiment 4088: (*S*)-10-((ethylsulfonyl)methyl)-7-(4-flurophenyl)-5-(2-hydroxyethyl)-2-methyl-2,5-dihydro-3 *H*-benzo[*c*]pyrido[3,4-*e*]azepin-3-ketone**

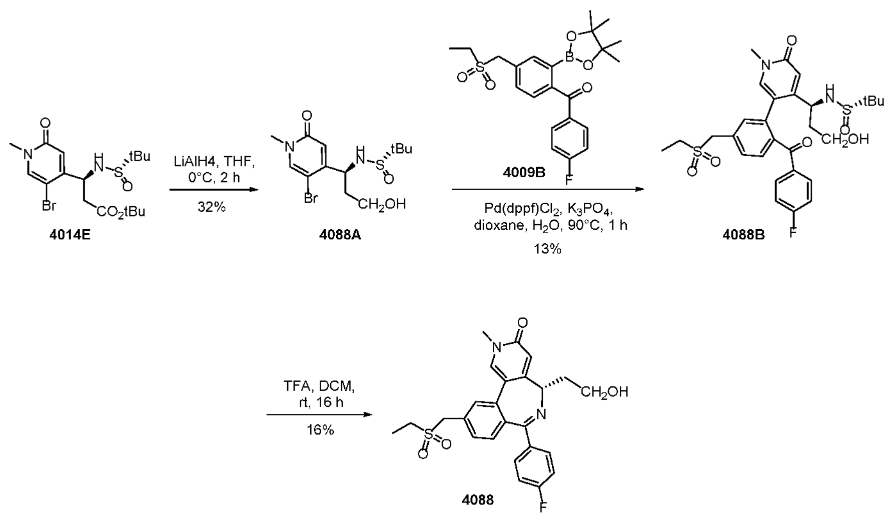


Step 1: (*S*)-*N*-((*S*)-1-(5-bromo-1-methyl-2-oxo-1,2-dihydropyridin-4-yl)-3-hydroxypropyl)-2-methylpropa ne-2-sulfenamide

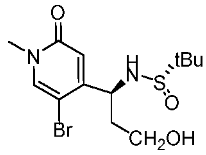

At 0°C, lithium alumiunum hydride (50 mg, 1.38 mmol) was added into (*S*)-3-(5-bromo-1-methyl-2-oxo-1,2-dihydropyridin-4-yl)-3-(((*S*)-tert-butyl-sulfinyl)amino) tert-butylpropionate (200 mg, 0.5 mmol) in THF (60 mL) solution. The reaction mixture was then stirred for 2 hours at 0°C, then quenched by saturated ammonium chloride solution (0.1 mL) and water. Concentration of the reaction mixture, the residue was purified by preparative-TLC (dichloromethane/methanol=10/1). The title compound was obtained (70 mg, 32%), in form of gray solid. MS (ESI): m/z = 367.0; 365.0 $[M+H]^+$.

Step 2: (*S*)-*N*-((*S*)-1-(5-(5-((ethylsulfonyl)methyl)-2-(4-fluorobenzoyl)phenyl)-1-methyl-2-oxo-1,2-dihydr opyridin-4-yl)-3-hydroxypropyl)-2-methylpropane-2-sulfenamide

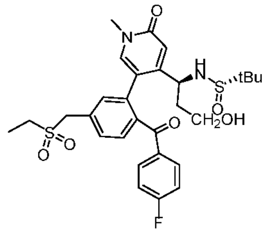

(*S*)-*N*-((*S*)-1-(5-bromo-1-methyl-2-oxo-1,2-dihydropyridin-4-yl)-3-hydroxypropyl)-2-m ethylpropane-2-sulfenamide (100 mg, 0.27 mmol), (4-((ethylsulfonyl)methyl)-2-(4,4,5,5-tetramethyl-1,3,2-dioxaborolan-2-yl)phenyl)(4-flurophenyl) methanone (130 mg, 0.30 mmol), [1,1'-bis(diphenylphosphino)ferrocene]palladium (II) dichloride (20 mg, 0.027 mmol) and potassium phosphate (120 mg, 0.57 mmol) were mixed in 1,4-dioxane (5 mL) and water (0.2 mL), and heated to 90°C then stirred for 1 hour under nitrogen protection. Water (15 mL) and EtOAc (25 mL) was added into the reaction mixture, the organic phase was separated. The organic phase was dried over anhydrous sodium sulfate, filtered and concentrated, the residue was purified by preparative-TLC (dichloromethane/methanol=10/1). The title compound was obtained (20 mg, 13%), in form of yellow oil. MS (ESI): $m/z$ = 591.0 $[M+H]^+$.

Step 3: (*S*)-10-((Ethylsulfonyl)methyl)-7-(4-flurophenyl)-5-(2-Hydroxyethyl)-2-methyl-2,5-dihydro-3*H*-b enzo[*c*]pyrido[3,4-*e*]azepin-3-ketone

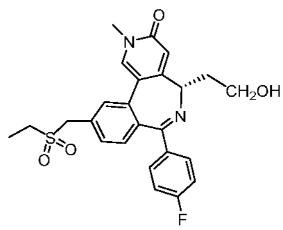

(*S*)-*N*-((*S*)-1-(5-(5-((ethylsulfonyl)methyl)-2-(4-fluorobenzoyl)phenyl)-1-methyl-2-oxo-1,2-dihydropyridin-4-yl)-3-hydroxypropyl)-2-methylpropane-2-sulfenamide (20 mg, 0.034 mmol) was dissolved in dichloromethane (4 mL) and TFA (1 mL), then stirred at r.t. overnight. Concentration of the reaction mixture, the residue was purified by preparative-HPLC, the title compound was obtained (2.5 mg, 16%), which was in form of white solid. MS (ESI): $m/z$ = 469.1 $[M+H]^+$; $^1H$ NMR (400 MHz, $CD_3OD$) $\delta$ 8.10 (s, 1H). 7.78 (s, 1H). 7.57 (m, 1H). 7.37 (d, $J$ = 8.0 Hz, 1H). 7.17 (m, 2H). 6.62 (s, 1H). 4.85 (s, 2H). 4.15 (m, 1H). 3.91 (m, 2H). 3.68 (s, 3H). 3.32 (q, $J$ = 7.6 Hz, 2H). 2.57 (m, 1H). 2.28 (m, 1H). 1.44 (t, $J$ = 6.8 Hz, 3H).

**Embodiment 4089: 10-((ethylsulfonyl)methyl)-7-(4-fluoro-3-methoxylphenyl)-2-methyl-2,5-dihydro-3*H*-be nzo[*c*]pyrido[3,4-*e*]azepin-3-ketone**

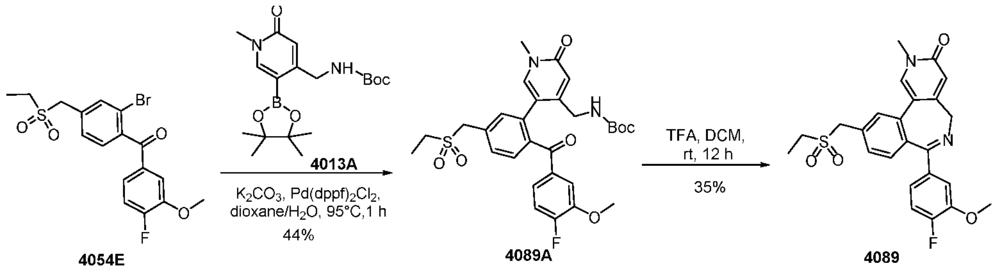


Step 1: ((5-(5-((Ethylsulfonyl)methyl)-2-(4-fluoro-3-methoxylbenzoyl)phenyl)-1-methyl-2-oxo-1,2-dihyd ropyridin-4-yl)methyl)*tert*-butylcarbamate

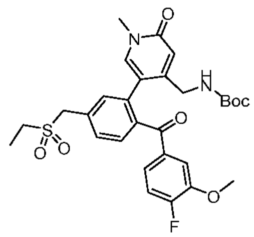

(2-Bromo-4-((ethylsulfonyl)methyl)phenyl)(4-fluoro-3-methoxylphenyl)methanone (250 mg, 0.60 mmol), ((1-methyl-2-oxo-5-(4,4,5,5-tetramethyl-1,3,2-dioxaborolan-2-yl)-1,2-dihydropyridin-4-yl)methyl) *tert*-butylcarbamate (439 mg, 1.2 mmol), potassium carbonate (250 mg, 1.8 mmol) and [1,1'-bis(diphenylphosphino)ferrocene]palladium (II) dichloride (45 mg, 0.06 mmol) were mixed in 1,4-dioxane (10 mL) and water (1 mL), heated to 95°C then stirred for 1 hour under nitrogen protection. The reaction mixture was filtered and the filtrate was concentrated, the residue was purified by preparative-TLC (EtOAc/methanol=25/1). The title compound was obtained (150 mg, 44%), in form of yellow oil. LCMS (ESI) $[M+H]^+$= 573.0.

Step 2: 10-((Ethylsulfonyl)methyl)-7-(4-fluoro-3-methoxylphenyl)-2-methyl-2,5-dihydro-3*H*-benzo[*c*]pyr ido[3,4-*e*]azepin-3-ketone

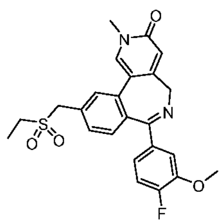

TFA (1 mL) was added into ((5-(5-((ethylsulfonyl)methyl)-2-(4-fluoro-3-methoxylbenzoyl)phenyl)-1-methyl-2-oxo-1,2-dihydr opyridin-4-yl)methyl) tert-butylcarbamate (150 mg, 0.26 mmol) in dichloromethane (4 mL) solution. The reaction mixture was stirred at r.t. for 12 hours then concentrated. The residue was purified by preparative-HPLC, the title compound was obtained (41.5 mg, 35%), which was in form of white solid. LCMS (ESI) $[M+H]^+$ = 455.2; $^1H$ NMR (400 MHz, *D*MSO-$d_6$) $\delta$ 8.07 (s, 1H). 7.708-7.702 (d, *J* = 2.4 Hz, 1H). 7.48-7.45 (m, *J* = 1.6 Hz, 2H). 7.35-7.33 (d, *J* = 8.0 Hz, 1H). 7.21-7.17 (m, *J* = 2.8 Hz, 1H). 6.75-6.71 (m, *J* = 2.0 Hz, 1H). 6.50 (s, 1H). 4.76-4.74 (d, *J* = 10.4 Hz, 1H). 4.66-4.53 (dd, *J* = 13.6 Hz, 2H). 3.89 (s, 1H). 3.87-3.83 (s, *J* = 12.8 Hz, 3H). 3.50 (s, 3H). 3.17-3.11 (q, *J* = 7.6 Hz, 2H).

1.27-1.23 (t, *J* = 7.2 Hz, 3H).

**Embodiment 4090: (*S*)-2-(7-(4-flurophenyl)-2,5-dimethyl-3-oxo-3,5-dihydro-2*H*-benzo[*c*]pyrido[3,4-*e*]azepin-10-yl)-*N*-methylpropane-2-sulfamide**

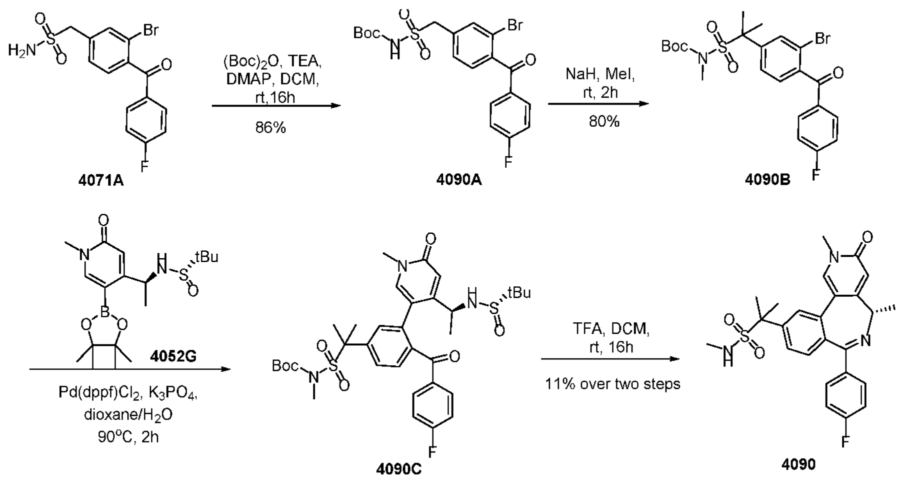


4071A, 4090A, 4090B, 4090C, 4090

Step 1: 3-Bromo-4-(4-fluorobenzoyl)benzylmethylsulfonyl *tert*-butylcarbamate

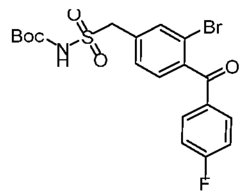

(3-Bromo-4-(4-fluorobenzoyl)phenyl)methylsulfamide (120 mg, 0.269 mmol), di-tert-butyl dicarbonate (106 mg, 0.484 mmol), triethylamine (49 mg, 0.484 mmol) and 4-dimethylaminopyridine (4 mg, 0.03 mmol) was dissolved in dichloromethane (10 mL), stirred at r.t. for 16 hours. Concentration of the reaction mixture, the residue was purified by flash chromatography (PE/EtOAc=1/1). The title compound was obtained (130 mg, 86%), which was in form of white solid. LCMS (ESI) $[M+H+17]^+$ = 488.8.

Step 2: ((2-(3-Bromo-4-(4-fluorobenzoyl)phenyl)propane-2-yl)sulfonyl)(methyl) tert-butylcarbamate

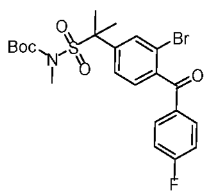

Sodium hydride (34 mg, 0.83 mmol, 60% oily dispersed liquid) was added into 3-bromo-4-(4-fluorobenzoyl)benzylmethylsulfonyl *tert*-butylcarbamate (130 mg, 0.275 mmol) in *N,N*-dimethylformamide (3 mL) solution, the reaction mixture was stirred at r.t. for 0.5 hour. Then added potassium iodide (118 mg, 0.83 mmol) to the reaction solution and stirred at r.t. for 2 hours. EtOAc (80 mL) was added into the reaction solution, and washed with water and saturated

brine, concentrated, then the tiltle compound was obtained (130 mg, 80%), which was in form of yellow solid. LCMS (ESI) $[M+H+17]^+$ = 530.9.

Step 3: ((2-(3-(4-((*S*)-1-(((*S*)-tert-butyl-sulfinyl)amino)ethyl)-1-methyl-6-oxo-1,6-dihydropyridin-3-yl)-4-(4-fluorobenzoyl)phenyl)propane-2-yl)sulfonyl)(methyl)*tert*-butlcarbamate

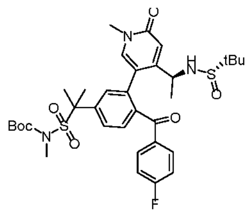

((2-(3-Bromo-4-(4-fluorobenzoyl)phenyl)propane-2-yl)sulfonyl)(methyl) *tert*-butylcarbamate (130 mg, 0.25 mmol), (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide (145 mg, 0.38 mmol), [1,1'-bis(diphenylphosphino)ferrocene]palladium (II) dichloride (18 mg, 0.025 mmol) and potassium phosphate (138 mg, 0.65 mmol) were mixed in 1,4-dioxane (4 mL) and water (0.4 mL), and heated to 90°C then stirred for 2 hours under nitrogen protection. The reaction mixture was cooled down to rt, then filtered and the filtrate was concentrated, the title compound was obtained (crude product). LCMS (ESI) $[M+H]^+$ = 690.0.

Step 4: (*S*)-2-(7-(4-flurophenyl)-2,5-dimethyl-3-oxo-3,5-dihydro-2*H*-benzo[*c*]pyrido[3,4-*e*]azepin-10-yl)-*N*-methylpropane-2-sulfamide

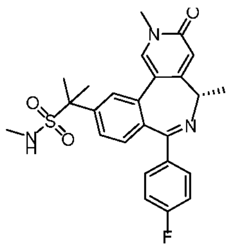

((2-(3-(4-((*S*)-1-(((*S*)-*tert*-butyl-sulfinyl)aminoethyl)-1-methyl-6-oxo-1,6-dihydropyridin-3-yl)-4-(4-fluorobenzoyl)phenyl)propane-2-yl)sulfonyl)(methyl) *tert*-butylcarbamate (crude product) was dissolved in dichloromethane (3 mL) and TFA (2 mL), stirred at r.t. for 16 hours, then concentrated. The residue was purified by preparative-HPLC, the title compound was obtained (13.5 mg, yield of the two steps 11%), which was in form of yellow solid. LCMS (ESI) $[M+H]^+$= 468.0; $^1$H NMR (400 MHz, $CD_3OD$) $\delta$ 8.12 (s, 1H). 7.99 (d, *J* = 2.0 Hz, 1H). 7.83 (d, *J* = 8.8 Hz, 1H). 7.56-7.53 (m, 2H). 7.39 (d, *J* = 8.8 Hz, 1H). 7.22-7.17 (m, 2H). 6.66 (s, 1H). 4.23 (q, 1H). 3.70 (s, 3H). 2.56 (s, 3H). 1.95 (s, 6H). 1.81 (d, 3H).

**Embodiment 4091: 1-((*S*)-7-(4-flurophenyl)-2,5-dimethyl-3-oxo-3,5-dihydro-2*H*-benzo[*c*]pyrido[3,4-*e*]azepin-10-yl)-*N*-methylethane-1-sulfamide**

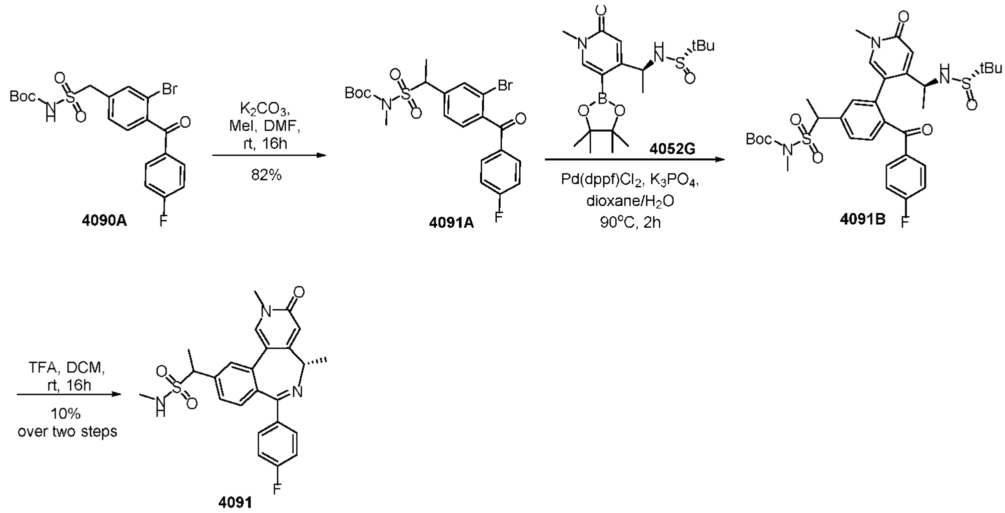


Step 1: ((1-(3-bromo-4-(4-fluorobenzoyl)phenyl)ethyl)sulfonyl)(methyl) *tert*-butylcarbamate

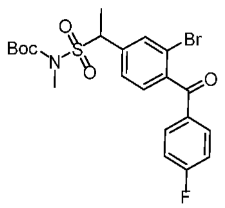

3-Bromo-4-(4-fluorobenzoyl)benzylmethylsulfonyl *tert*-butylcarbamate (330 mg, 0.70 mmol), methyl iodide (149 mg, 1.05 mmol) and potassium carbonate (145 mg, 0.83 mmol) were mixed in *N,N*-dimethylformamide (4 mL), stirred at r.t. for 16 hours. EtOAc (80 mL) was added into the reaction mixture then water and saturated brine was used to wash the reaction solution followed by concentration, the tiltle compound was obtained (280 mg, 82%), which was in form of white solid. LCMS (ESI) $[M+H+17]^+$ = 516.8.

Step 2: ((1-(3-(4-((*S*)-1-(((*S*)-*tert*-butyl-sulfinyl)amino)ethyl)-1-methyl-6-oxo-1,6-dihydropyridin-3-yl)-4-(4-fluorobenzoyl)phenyl)ethyl)sulfonyl)(methyl)*tert*-butylcarbamate

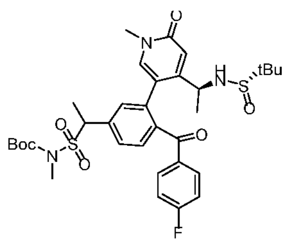

((1-(3-Bromo-4-(4-fluorobenzoyl)phenyl)ethyl)sulfonyl)(methyl)*tert*-butylcarbamate (106 mg, 0.218 mmol), (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide (100 mg, 0.262 mmol), [1,1'-bis(diphenylphosphino)ferrocene]palladium (II) dichloride (15 mg, 0.022 mmol) and potassium phosphate (186 mg, 0.55 mmol) were mixed in 1,4-dioxane/water (4 mL/0.4 mL), and heated to 90°C then stirred for 2 hours under nitrogen protection. The reaction mixture was then cooled down to room temperature, then filtered and the filtrate was concentrated, the title compound was obtained (crude product). LCMS (ESI) $[M+H]^+$ = 676.0.

Step 3: 1-((*S*)-7-(4-flurophenyl)-2,5-dimethyl-3-oxo-3,5-dihydro-2*H*-benzo[c]pyrido[3,4-*e*]azepin-10-yl)-*N*-methyl-ethane-1-sulfamide

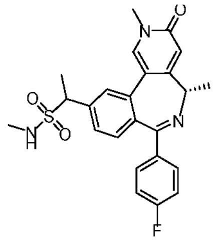

((1-(3-(4-((*S*)-1-(((*S*)-tert-butyl-sulfinyl)amino)ethyl)-1-methyl-6-oxo-1,6-dihydropyridi n-3-yl)-4-(4-fluorobenzoyl)phenyl)ethyl)sulfonyl)(methyl) *tert*-butylcarbamate (crude product) was dissolved in dichloromethane (3 mL) and TFA (2 mL), stirred at r.t. for 16 hours. Concentration of the reaction mixture, the residue was purified by preparative-HPLC to obtain the title compound (9.5 mg, yield of the two steps 10%), which was in form of white solid. LCMS (ESI) $[M+H]^+$= 454.0. $^1H$ NMR (400 MHz, $CD_3OD$) $\delta$ 8.08 (d, *J* = 6.0 Hz, 1H). 7.80 (s, 1H). 7.59 (d, *J* = 8.4 Hz, 1H). 7.52-7.49 (m, 2H). 7.34 (d, *J* = 8.4 Hz, 1H). 7.16-7.11 (m, 2H). 6.63 (s, 1H). 4.61 (q, 1H). 4.13 (q, 1H). 3.69 (s, 3H). 2.70 (s, 3H). 1.85 (d, 3H). 1.77 (d, 3H).

**Embodiment 4092: *N*-(9-fluoro-7-(4-flurophenyl)-2,5-dimethyl-3-oxo-3,5-dihydro-2*H*-benzo[*e*]pyrido[4,3-*c*][1,2]diazepin-10-yl)ethylsulfonamide**

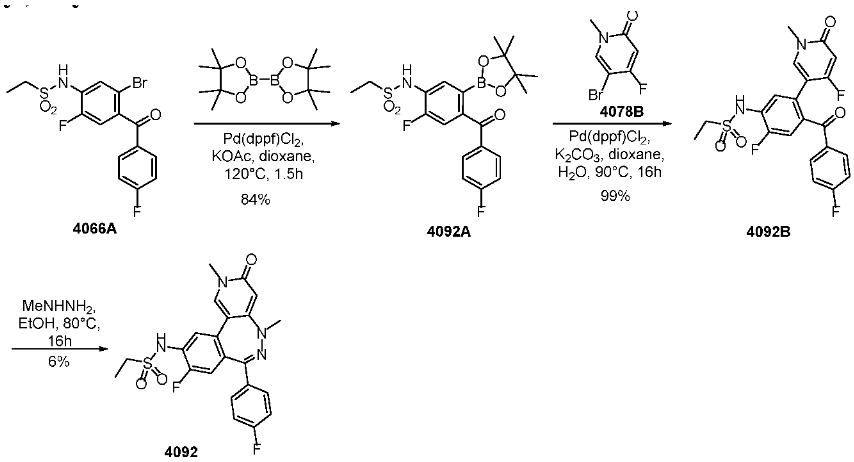


Step 1: *N*-(2-Fluoro-4-(4-fluorobenzoyl)-5-(4,4,5,5-tetramethyl-1,3,2-dioxaborolan-2-yl)phenyl)ethylsulfo namide

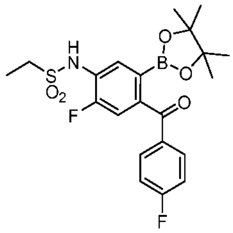

*N*-(5-Bromo-2-fluoro-4-(4-fluorobenzoyl)phenyl)ethylsulfonamide (640 mg, 1.58 mmol), bis(pinacolato)diboron (804 mg, 3.17 mmol), [1,1'-bis(diphenylphosphino)ferrocene]palladium (II) dichloride (232 mg, 0.32 mmol) and potassium

acetate (466 mg, 4.75 mmol) were mixed in 1,4-dioxane (10 mL), and heated to 120°C then stirred for 1.5 hours under nitrogen protection. Concentration of the reaction mixture, the residue was purified by flash chromatography (PE/EtOAc=3/1). The title compound was obtained (600 mg, 84%), in form of red solid. LCMS (ESI) $[M+H]^+$ = 452.2.

Step 2: *N*-(2-Fluoro-5-(4-fluoro-1-methyl-6-oxo-1,6-dihydropyridin-3-yl)-4-(4-fluorobenzoyl)phenyl)ethy lsulfonamide

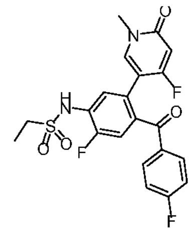

*N*-(2-Fluoro-4-(4-fluorobenzoyl)-5-(4,4,5,5-tetramethyl-1,3,2-dioxaborolan-2-yl)phenyl) ethylsulfonamide (400 mg, 0.89 mmol), 5-bromo-4-fluoro-1-methylpyridin-2(1*H*)-ketone (183 mg, 0.89 mmol), [1,1'-bis(diphenylphosphino)ferrocene]palladium (II) dichloride (65 mg, 0.09 mmol) and potassium carbonate (368 mg, 2.66 mmol) were mixed in 1,4-dioxane (8 mL) and water (0.4 mL), and heated to 90°C then stirred overnight under nitrogen protection. Concentration of the reaction mixture, the residue was purified by flash chromatography (PE/EtOAc=1/1). The title compound was obtained (400 mg, 99%), in form of red solid. LCMS (ESI) $[M+H]^+$ = 451.2.

Step 3: *N*-(9-Fluoro-7-(4-flurophenyl)-2,5-dimethyl-3-oxo-3,5-dihydro-2*H*-benzo[*e*]pyrido[4,3-*c*][1,2]dia zepin-10-yl)ethylsulfonamide

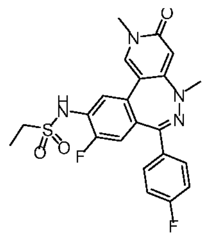

*N*-(2-Fluoro-5-(4-fluoro-1-methyl-6-oxo-1,6-dihydropyridin-3-yl)-4-(4-fluorobenzoyl)p henyl)ethylsulfonamide (200 mg, 0.44 mmol) and methylhydrazine (1 mL) was dissolved in ethanol (4 mL), and heated to 80°C then stirred overnight. Concentration of the reaction mixture, the residue was purified by preparative-HPLC, the title compound was obtained (11.5 mg, 6%), which was in form of white solid. LCMS (ESI) $[M+H]^+$ = 459.2; $^1$H NMR (400 MHz, *D*MSO-$d_6$) $\delta$ 7.82 (s, 1H). 7.52-7.49 (m, 2H).7.43-7.41 (d, *J* = 7.6 Hz, 1H). 7.28-7.22 (m, 2H). 6.89-6.85 (m, 1H). 6.05 (s, 1H). 3.45-3.43 (d, *J* = 7.2 Hz, 3H). 3.17-3.13 (m, 2H). 3.06-3.04 (d, *J* = 8.0 Hz, 3H). 1.28-1.22 (m, 3H).

**Embodiment 4093: 7-(2-chloro-4-flurophenyl)-10-((ethylsulfonyl)methyl)-2,5-dimethyl-2,5-dihydro-3*H*-benzo[*e*] pyrido[4,3-*c*][1,2]diazepin-3-ketone**

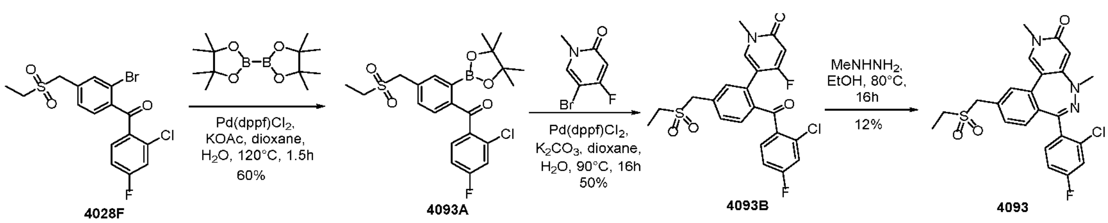

Step 1: (2-Chloro-4-flurophenyl)(4-((ethylsulfonyl)methyl)-2-(4,4,5,5-tetramethyl-1,3,2-dioxaborolan-2-y l)phenyl)methanone

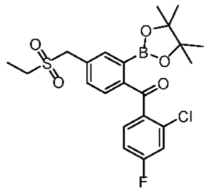

(2-Bromo-4-((ethylsulfonyl)methyl)phenyl)(2-chloro-4-flurophenyl)methanone (303 mg, 0.72 mmol), bis(pinacolato)diboron (367 mg, 1.45 mmol), [1,1'-bis(diphenylphosphino)ferrocene]palladium (II) dichloride (53 mg, 0.07 mmol) and potassium acetate (213 mg, 2.17 mmol) were mixed in 1,4-dioxane (10 mL), and heated to 120°C then stirred for 1.5 hours under nitrogen protection. Concentration of the reaction mixture, the residue was purified by flash chromatography (PE/EtOAc=3/1). The title compound was obtained (200 mg, 60%), in form of red solid. LCMS (ESI) $[M+H]^+$ = 467.1.

Step 2: 5-(2-(2-Chloro-4-fluorobenzoyl)-5-((ethylsulfonyl)methyl)phenyl)-4-fluoro-1-methylpyridin-2(1*H* )-ketone

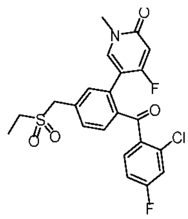

(2-Chloro-4-flurophenyl)(4-((ethylsulfonyl)methyl)-2-(4,4,5,5-tetramethyl-1,3,2-dioxaborolan-2-yl)phenyl)methanone (200 mg, 0.43 mmol), 5-bromo-4-fluoro-1-methylpyridin-2(1*H*)-ketone (90 mg, 0.44 mmol), [1,1'-bis(diphenylphosphino)ferrocene]palladium (II) dichloride (40 mg, 0.05 mmol) and potassium carbonate (180 mg, 1.33 mmol) were mixed in 1,4-dioxane (8 mL) and water (0.8 mL), and heated to 90°C then stirred overnight under nitrogen protection. Concentration of the reaction mixture, the residue was purified by flash chromatography (PE/EtOAc=1/1). The title compound was obtained (100 mg, 50%), in form of red solid. LCMS (ESI) $[M+H]^+$ = 465.9.

Step 3: 7-(2-chloro-4-flurophenyl)-10-((ethylsulfonyl)methyl)-2,5-dimethyl-2,5-dihydro-3*H*-benzo[*e*]pyri do[4,3-*c*][1,2]diazein-3-ketone

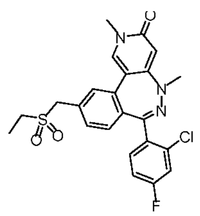

5-(2-(2-Chloro-4-fluorobenzoyl)-5-((ethylsulfonyl)methyl)phenyl)-4-fluoro-1-methylpyr idin-2(1*H*)-ketone (100 mg, 0.21 mmol) and methylhydrazine (1 mL) was dissolved in ethanol (4 mL), and heated to 80°C and stirred overnight. Concentration of the reaction mixture, the residue was purified by preparative-HPLC, the title compound was obtained (11.7 mg, 12%), which was in form of white solid. LCMS (ESI) $[M+H]^+$ = 473.9; $^1$H NMR (400 MHz, *D*MSO-$d_6$) $\delta$ 7.82 (s, 1H). 7.65-7.61 (m, 1H). 7.52-7.49 (m, 2H). 7.39-7.36 (m, 1H). 7.31-7.30 (d, *J* = 1.6 Hz, 1H). 6.66-6.64 (d, *J* = 8 Hz, 1H). 6.05 (s, 1H). 4.75 (s, 2H). 3.37 (s, 3H). 3.09-3.05 (m, 5H). 1.23-1.20 (t, *J* = 4.0 Hz, 3H).

**Embodiment 4094: 10-((Ethylsulfonyl)methyl)-7-(4-flurophenyl)-9-methoxyl-2-methyl-2,5-dihydro-3*H*-benzo[*c*] pyrido[3,4-*e*]azepin-3-ketone**

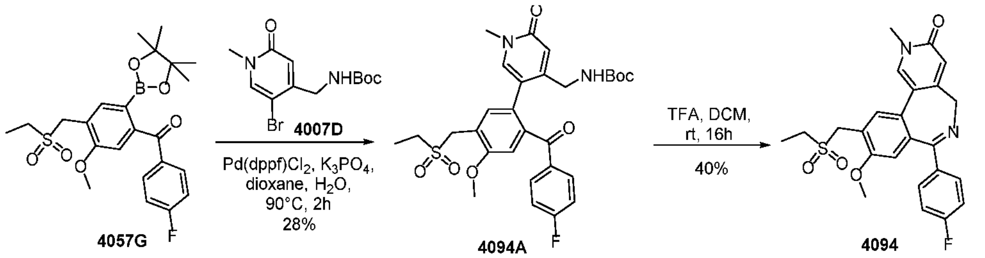


Step 1: ((5-(5-((Ethylsulfonyl)methyl)-2-(4-fluorobenzoyl)-4-methoxylphenyl)-1-methyl-2-oxo-1,2-dihydropyridin-4-yl)methyl)*tert*-butylcarbamate

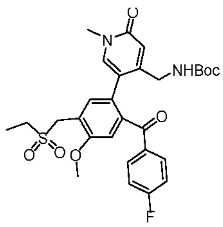

(4-((Ethylsulfonyl)methyl)-5-methoxyl-2-(4,4,5,5-tetramethyl-1,3,2-dioxaborolan-2-yl)phenyl)(4-flurophenyl)methanone (300 mg, 0.65 mmol), ((5-bromo-1-methyl-2-oxo-1,2-dihydropyridin-4-yl)methyl)*tert*-butylcarbamate (82 mg, 0.26 mmol), potassium phosphate (165 mg, 0.78 mmol) and [1,1'-bis(diphenylphosphino)ferrocene]palladium (II) dichloride (28 mg, 0.04 mmol) were mixed in 1,4-dioxane (5 mL) and water (0.5 mL), and heated to 90°C then stirred for 2 hours under nitrogen protection. The reaction mixture was filtered and the filtrate was concentrated, the residue was purified by flash chromatography (EtOAc/methanol= 97/3). The title compound was obtained (160 mg, 28%), which was in form of yellow solid. LCMS (ESI) $[M+H]^+$ = 573.2.

Step 2: 10-((ethylsulfonyl)methyl)-7-(4-flurophenyl)-9-methoxyl-2-methyl-2,5-dihydro-3*H*-benzo[*c*]pyrid o[3,4-*e*]azepin-3-ketone

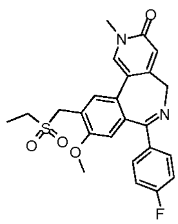

TFA (1 mL) was added into ((5-(5-((ethylsulfonyl)methyl)-2-(4-fluorobenzoyl)-4-methoxylphenyl)-1-methyl-2-oxo-1,2-dihydr opyridin-4-yl)methyl) *tert*-butylcarbamate (160 mg, 0.28 mmol) in dichloromethane (4 mL) solution. The reaction mixture was stirred at r.t. for 16 hours then concentrated. The residue was purified by preparative-HPLC, the title compound was obtained (50.6 mg, 40%), which was in form of white solid. LCMS (ESI) $[M+H]^+$ = 455.0; $^1$H NMR (400 MHz, *D*MSO-$d_6$) $\delta$ 7.99 (s, 1H). 7.69 (s, 1H). 7.57-7.53 (m, 2H). 7.26-7.21 (m, 2H). 6.87 (s, 1H). 6.52 (s, 1H). 4.73 (d, *J* = 6.4 Hz, 1H). 4.61 (d, *J* = 13.6 Hz, 1H). 4.41 (s, *J* = 13.6 Hz, 1H). 3.91 (d, *J* = 10.0 Hz, 1H). 3.72 (s, 3H). 3.49 (s, 3H). 3.11 (q, *J* = 7.6 Hz, 2H). 1.27 (t, *J* = 7.6 Hz, 3H).

**Embodiment 4095: *N*-(9-Fluoro-7-(4-flurophenyl)-2,5-dimethyl-3-oxo-3,5-dihydropyridin-2*H*-benzo[*c*]pyridazino[3,4-*e*]azepin-10-yl)ethylsulfonamide**

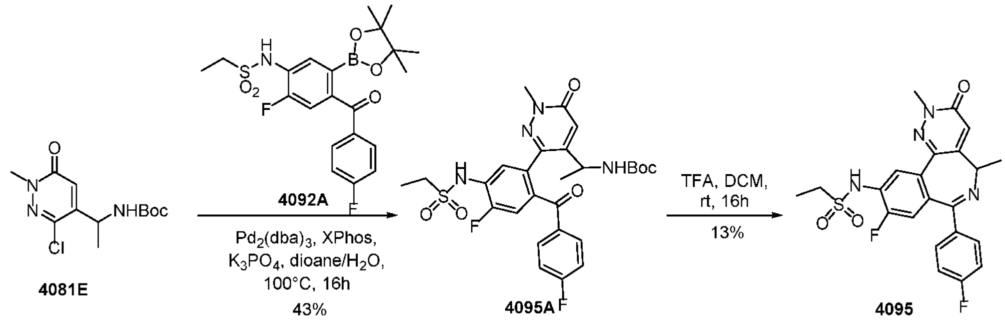


4081E 4095A 4095

Step 1: 1-(3-(5-(Ethylsulfamidyl)-4-fluoro-2-(4-fluorobenzoyl)phenyl)-1-methyl-6-oxo-1,6-dihydropyrida zine-4-yl)ethyl-tert-butylcarbamate

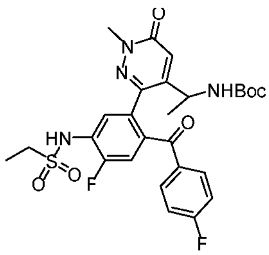

1-(3 -Chloro-1-methyl-6-oxo-1,6-dihydropyridazine-4-yl)ethyltert-butylcarbamate (30 mg, 0.1 mmol), N-(2-fluoro-4-(4-fluorobenzoyl)-5-(4,4,5,5-tetramethyl-1,3,2-dioxaborolan-2-yl)phenyl)ethylsulfo namide (90 mg, 0.2 mmol), tri(dibenzyl acetylacetone) dipalladium (0) (9 mg, 0.01 mmol), 2-dicyclohexylphosphorus-2,4,6-triisopropylbiphenyl (4.5 mg, 0.01 mmol) and potassium phosphate (45 mg, 0.2 mmol) were mixed in 1,4-dioxane (5 mL) and water (1 drop), and heated to 100°C and stirred overnight under nitrogen protection. Concentration of the reaction mixture, the residue was purified by flash chromatography (PE/EtOAc=1/2). The tiltle compound was obtained (25 mg, 43%), in form of yellow oil. LCMS (ESI) $[M+H]^+$ = 577.1.

Step 2: N-(9-Fluoro-7-(4-flurophenyl)-2,5-dimethyl-3-oxo-3,5-dihydro-2*H*-benzo[*c*]pyridazino[3,4-*e*]azep in-10-yl)ethyl-sulfonamide

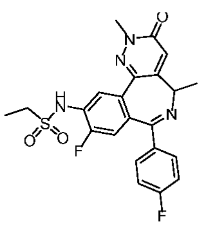

1-(3-(5-(Ethylsulfamidyl)-4-fluoro-2-(4-fluorobenzoyl)phenyl)-1-methyl-6-oxo-1,6-dihydropyridazine-4-yl)ethyltert-butylcarbamate (25 mg, 0.043 mmol) and TFA (1 mL) was dissolved in dichloromethane (4 mL), then stirred at r.t. overnight. Concentration of the reaction mixture, the residue was purified by preparative-HPLC, the tiltle compound was obtained (2.5 mg, 13%), which was in form of white solid. LCMS (ESI) $[M+1]^+$ = 459.0 ; $^1$H NMR (400 MHz, $CD_3OD$) $\delta$

8.18 (d, $J$ = 8.0 Hz, 1H). 7.54 (m, 2H). 7.16 (m, 3H). 6.99 (d, $J$ = 0.8 Hz, 1H). 4.21 (d, $J$ = 6.8 Hz, 1H). 3.89 (s, 3H). 3.30 (m, 2H). 1.78 (d, $J$ = 6.4 Hz, 3H). 1.43 (t, $J$ = 7.6 Hz, 3H).

**Embodiment 4096: 7-(2-chloro-4-fluoro-5-methoxylphenyl)-10-((ethylsulfonyl)methyl)-2-methyl-2,5-dihydro-3*H* -benzo[*c*]rido[34-*e*]azein-3-ketone**

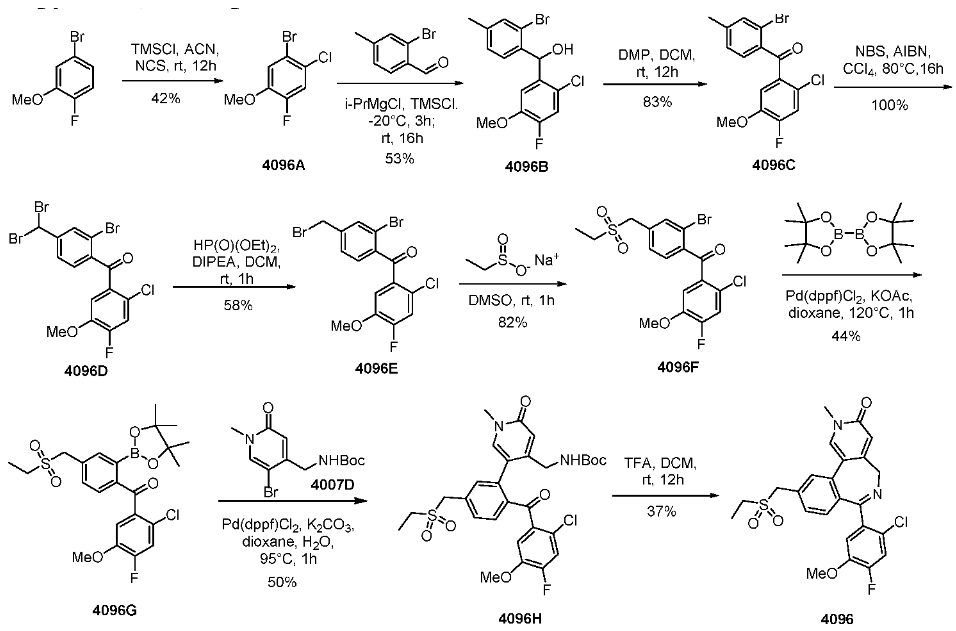


Step 1: 1-Bromo-2-chloro-4-fluoro-5-methoxylphenyl

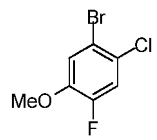

4-Bromo-1-fluoro-2-methoxylphenyl (20.5 g, 10 mmol), *N*-chloro desuccinimide (14.6 g, 11 mmol) and trimethyl chlorosilicane (1.4 mL, 0.1 mmol) was dissolved in acetonitrile (200 mL), and was stirred at r.t. under nitrogen protection for 12 hours. The reaction mixture was filtered and the filtrate was concentrated, the residue was purified by flash chromatography (PE). The title compound was obtained (10 g, 42%), which was in form of white solid. $^1H$ NMR (400 MHz, $CD_3OD$) $\delta$ 7.67 (d, $J$ = 10.8 Hz, 1H). 7.57 (d, $J$ = 8.4 Hz, 1H).

Step 2: (2-Bromo-4-methylphenyl)(2-chloro-4-fluoro-5-methoxylphenyl)methanol

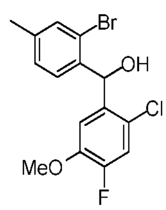

At -20°C and under nitrogen protection, isopropyl magnesium chloride (2 M in THF solution, 21 mL, 42 mmol)

was added into 1-bromo-2-chloro-4-fluoro-5-methoxylphenyl (10.0 g, 42 mmol) in anhydrous THF (50 mL) solution, and stirred at -20°C for 3 hours to obtain solution 1. In another reaction bottle, 2-bromo-4-methylbenzaldehyde (1.3 g, 6.3 mmol) was dissolved in anhydrous THF (10 mL), cooled down to -20°C, added trimethyl chlorosilane (800 mg, 6.3 mmol), then stirred for 30 minutes under nitrogen protection, then the reaction mixture 1 was added to the reaction bottle. The mixutre obtained was then stirred at r.t. overnight. The reaction was quenched by ammonium chloride (10 mL), then extracted by EtOAc (20 mL*3). The organic phase was washed by HCl (1 M, 20 mL) and saturated brine (20 mL), dried over anhydrous sodium sulfate, filtered and concentrated. The residue was purified by flash chromatography (PE/EtOAc=8/1). The title compound was obtained (1.2 g, 53%), in form of yellow oil. LCMS (ESI) $[M-OH]^+$= 341.0, 343.

Step 3: (2-Bromo-4-methylphenyl)(2-chloro-4-fluoro-5-methoxylphenyl)methanone

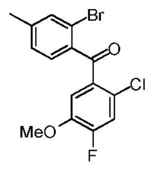

(2-Bromo-4-methylphenyl)(2-chloro-4-fluoro-5-methoxylphenyl)methanol (1.2 g, 3.3 mmol) and Dess-Matin peridinane (2.1 g, 5.0 mmol) were mixed in dichloromethane (50 mL), stirred at r.t. for 12 hours. The reaction mixture was filtered, the filter cake was washed by dichloromethane (10 mL*2). The filtrate was collected, after concentration, the residue was purified by flash chromatography (PE/EtOAc=10/1). The title compound was obtained (1.0 g, 83%), which was in form of white solid. LCMS (ESI) $[M+H]^+$= 359.0, 357.0

Step 4: (2-Bromo-4-(dibromomethyl)phenyl)(2-chloro-4-fluoro-5-methoxylphenyl)methanone

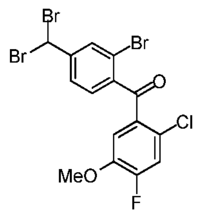

*N*-Bromodesuccinimide (1.5 g, 8.4 mmol), azobisisobutyronitrile (46 mg, 0.28 mmol) and (2-bromo-4-methylphenyl)(2-chloro-4-fluoro-5-methoxylphenyl)methanone (1.0 g, 2.8 mmol) was dissolved in tetrachloromethane (25 mL), and heated to 80°C for 16 hours. Concentration of the reaction mixture, the title compound was obtained (1.4 g, 100%), which was in form of yellow solid, and was used directly in the next step without purification. LCMS (ESI) $[M+H]^+$= 435.0, 437.0.

Step 5: (2-Bromo-4-(bromomethyl)phenyl)(2-chloro-4-fluoro-5-methoxylphenyl)methanone

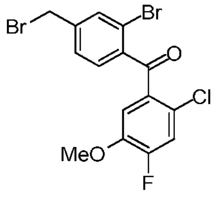

Triethyl phosphate (770 mg, 5.6 mmol), diisopropyl ethylamine (720 mg, 5.6 mmol) and (2-bromo-4-(dibromomethyl)phenyl)(2-chloro-4-fluoro-5-methoxylphenyl)methanone (1.4 g, 2.8 mmol) was dissolved in dichloromethane

(10 mL), then stirred at r.t. for 1 hour. Concentration of the reaction mixture, the residue was purified by flash chromatography (PE/EtOAc=15/1). The title compound was obtained (700 mg, 58%), in form of yellow oil. LCMS (ESI) $[M+H]^+$ = 437.0, 435.0.

Step 6: (2-Bromo-4-((ethylsulfonyl)methyl)phenyl)(2-chloro-4-fluoro-5-methoxylphenyl)methanone

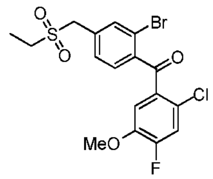

Sodium ethanesulfinate (373 mg, 3.2 mmol) was added into (2-bromo-4-(bromomethyl)phenyl)(2-chloro-4-fluoro-5-methoxylphenyl)methanone (700 mg, 1.6 mmol) in dimethylsulfoxide (5 mL) solution. The reaction mixture was stirred at r.t. for 1 hour, then added EtOAc (20 mL) and water (20 mL), separated the organic phase. The organic phase was washed by saturated brine (10 mL*3), dried over anhydrous sodium sulfate, filtered and concentrated, the residue was purified by flash chromatography (PE/EtOAc=3/1). The title compound was obtained (600 mg, 82%). LCMS (ESI) $[M+H]^+$ = 449.0, 451.0.

Step 7: (2-Chloro-4-fluoro-5-methoxylphenyl)(4-((ethylsulfonyl)methyl)-2-(4,4,5,5-tetramethyl-1,3,2-dio xaborolan-2-yl)phenyl)methanone

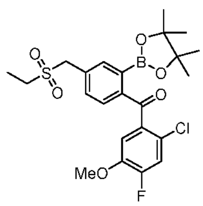

(2-Bromo-4-((ethylsulfonyl)methyl)phenyl)(2-chloro-4-fluoro-5-methoxylphenyl)metha none (100 mg, 0.23 mmol), bis(pinacolato)diboron (117 mg, 0.46 mmol), [1,1'-bis(diphenylphosphino)ferrocene]palladium (II) dichloride (34 mg, 0.05 mmol) and potassium acetate (68 mg, 0.7 mmol) were mixed in 1,4-dioxane (5 mL), heated to 120°C then stirred for 1 hour under nitrogen protection. The reaction mixture was filtered and the filtrate was concentrated, the residue was purified by flash chromatography (PE/EtOAc=1/1). The title compound was obtained (50 mg, 44%), in form of yellow oil. LCMS (ESI) $[M+H]^+$= 497.1.

Step 8: ((5-(2-(2-Chloro-4-fluoro-5-methoxylbenzoyl)-5-((ethylsulfonyl)methyl)phenyl)-1-methyl-2-oxo-1,2-dihydropyridin-4-yl)methyl)*tert*-butylcarbamate

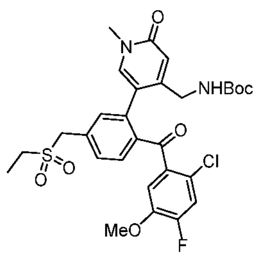

(2-Chloro-4-fluoro-5-methoxylphenyl)(4-((ethylsulfonyl)methyl)-2-(4,4,5,5-tetramethyl -1,3,2-dioxaborolan-2-yl)phenyl)methanone (50 mg, 0.1 mmol), (5-bromo-1-methyl-2-oxo-1,2-dihydropyridin-4-yl)methyl*tert*-butylcarbamate (47 mg, 0.1 mmol), [1,1'-bis(diphenylphosphino)ferrocene]palladium (II) dichloride (7.3 mg, 0.01 mmol) and potassium carbonate (64 mg, 0.3 mmol) were mixed in 1,4-dioxane (5 mL) and water (1 drop), and heated to 95°C then stirred for 1 hour under nitrogen protection. The reaction mixture was filtered and the filtrate was concentrated, the residue was purified by flash chromatography (EtOAc/methanol=30/1). The title compound was obtained (30 mg, 50%), which was in form of yellow solid. LCMS (ESI) $[M+H]^+$ = 607.1.

Step 9: 7-(2-Chloro-4-fluoro-5-methoxylphenyl)-10-((ethylsulfonyl)methyl)-2-methyl-2,5-dihydro-3*H*-be nzo[*c*]pyrido[3,4-*e*]azepin-3-ketone

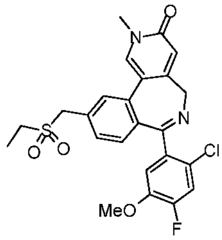

TFA (1 mL) was added into ((5-(2-(2-chloro-4-fluoro-5-methoxylbenzoyl)-5-((ethylsulfonyl)methyl)phenyl)-1-methyl-2-oxo-1 ,2-dihydropyridin-4-yl)methyl)*tert*-butylcarbamate (30 mg, 0.05 mmol) in dichloromethane (4 mL) solution. The reaction mixture was stirred at r.t. for 12 hours then concentrated. The residue was purified by preparative-HPLC, the title compound was obtained (9 mg, 37%), which was in form of white solid. LCMS (ESI) $[M+H]^+$ = 489.1; $^1H$ NMR (400 MHz, $CD_3OD$) $\delta$ 8.15 (s, 1H). 7.77 (d, *J* = 1.6 Hz, 1H). 7.51 (m, 1H). 7.35 (d, *J* = 8.4 Hz, 2H). 7.24 (m, 2H). 6.66 (s, 1H). 4.80 (d, *J* = 10.4 Hz, 1H). 4.57 (s, 2H). 4.17 (d, *J* = 10.4 Hz, 1H). 3.96 (s, 3H). 3.70 (s, 3H). 2.42 (q, *J* = 6.8 Hz, 2H). 1.40 (t, *J* = 7.2 Hz, 3H).

**Embodiment 4097: 7-(2-chloro-4-flurophenyl)-10-((ethylsulfonyl)methyl)-2,5-dimethyl-2,5-dihydro-3*H*-benzo[*c*] pyridazino[3,4-*e*]azepin-3-ketone**

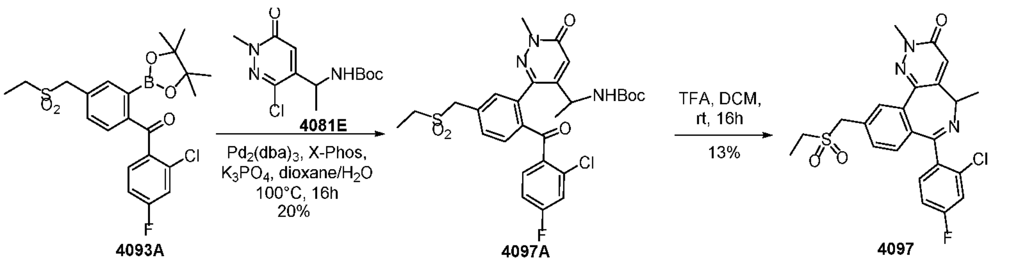

4093A 4097A 4097

Step 1: (1-(3-(2-(2-Chloro-4-fluorobenzoyl)-5-((ethylsulfonyl)methyl)phenyl)-1-methyl-6-oxo-1,6-dihydr opyridazine-4-yl)ethyl)tert-butylcarbamate

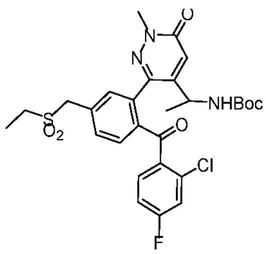

1-(3-Chloro-1-methyl-6-oxo-1,6-dihydropyridazine-4-yl)ethyl)*tert*-butylcarbamate (50 mg, 0.17 mmol), (2-chloro-4-flurophenyl)(4-((ethylsulfonyl)methyl)-2-(4,4,5,5-tetramethyl-1,3,2-dioxaborolan-2-yl )phenyl)methanone (245 mg, 0.52 mmol), tri(dibenzyl acetylacetone) dipalladium (16 mg, 0.02 mmol), 2-bicyclohexylphosphine-2', 4', 6'-triisopropyl biphenyl(16 mg, 0.03 mmol) and potassium phosphate (92 mg, 0.43 mmol) were mixed in 1,4-dioxane (4 mL) and water (2 drops), heated to 100°C and stirred overnight under nitrogen protection. Concentration of the reaction mixture, the residue was purified by flash chromatography (PE/EtOAc=1/2). The title compound was obtained (20 mg, 20%), in form of yellow oil. LCMS (ESI) $[M+H]^+$ = 592.2.

Step 2: 7-(2-Chloro-4-flurophenyl)-10-((ethylsulfonyl)methyl)-2,5-dimethyl-2,5-dihydro-3*H*-benzo[*c*]pyri dazino[3,4-*e*]azepin-3-ketone

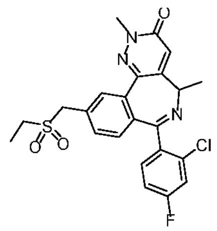

(1-(3-(2-(2-Chloro-4-fluorobenzoyl)-5-((ethylsulfonyl)methyl)phenyl)-1-methyl-6-oxo-1,6-dihydropyridazine-4-yl)ethyl)*tert*-butylcarbamate (20 mg, 0.03 mmol) and TFA (1 mL) was dissolved in dichloromethane (4 mL), then stirred at r.t. overnight. Concentration of the reaction mixture, the residue was purified by preparative-HPLC, the title compound was obtained (2.5 mg, 13%), which was in form of white solid. LCMS (ESI) $[M+H]^+$ = 474.1; $^1$H NMR (400 MHz, $CD_3OD$) $\delta$ 8.10-8.09 (s, 1H). 7.66-7.61 (m, 2H). 7.28-7.20 (m, 3H). 7.03 (s, 1H). 4.60 (s, 1H). 4.32 -4.30 (d, *J* = 6.4 Hz, 1H). 3.93 (s, 3H). 3.15-3.09 (m, 2H). 1.79-1.77 (d, *J* = 6.8 Hz, 3H). 1.40-1.31 (m, 3H).

**Embodiment 4098: 7-(2-chloro-4-flurophenyl)-2-methyl-10-((dimethylfulfone)methyl)-2,5-dihydro-3*H*-benzo[*c*]pyrido[3,4-*e*]azepin-3-ketone**

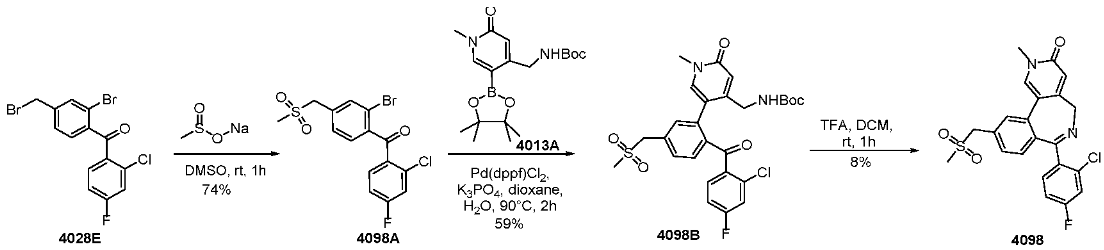


Step 1: (2-bromo-4-((dimethylfulfone)methyl)phenyl)(2-chloro-4-flurophenyl)methanone

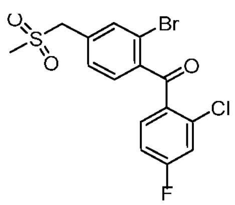

Sodium methanesulphinate (153 mg, 1.5 mmol) was added into (2-bromo-4-(bromomethyl)phenyl)(2-chloro-4-flurophenyl)methanone (400 mg, 1 mmol) in dimethylsulfoxide (6 mL) solution, the reaction mixture was stirred at r.t. for 1 hour. EtOAc (10 mL) and water (15 mL) were added into the miture, separated the organic phase. The organic

phase was washed by saturated brine (15 mL*3), dried over anhydrous sodium sulfate, filtered and concentrated, the title compound was obtained (300 mg, 74%). LCMS (ESI) [M+H]+ = 406.9.

Step 2: ((5-(2-(2-Chloro-4-fluorobenzoyl)-5-((dimethylfulfone)methyl)phenyl)-1-methyl-2-oxo-1,2-dihyd ropyridin-4-yl)methyl)*tert*-butlcarbamate

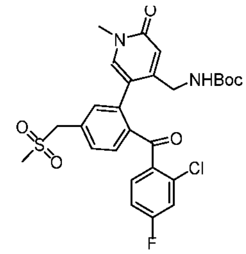

((1-Methyl-2-oxo-5-(4,4,5,5-tetramethyl-1,3,2-dioxaborolan-2-yl)-1,2-dihydropyridin-4-yl)methyl) *tert*-butylcarbamate (121 mg, 0.33 mmol), (2-bromo-4-((dimethylfulfone)methyl)phenyl)(2-chloro-4-flurophenyl)methanone (38 mg, 0.09 mmol), [1,1'-bis(diphenylphosphino)ferrocene]palladium (II) dichloride (10 mg, 0.0135 mmol) and potassium phosphate (57 mg, 0.27 mmol) were mixed in 1,4-dioxane (2 mL) and water (0.2 mL), and heated to 90°C then stirred for 2 hours. Concentration of the reaction mixture under nitrogen protection, the residue was purified by flash chromatography (EtOAc/methanol=10/1). The title compound was obtained (30 mg, 59%), which was in form of yellow solid. LCMS (ESI) [M+H]+ = 563.0.

Step 3: 7-(2-Chloro-4-flurophenyl)-2-methyl-10-((dimethylfulfone)methyl)-2,5-dihydro-3*H*-benzo[*c*]pyri do[3,4-*e*]azepin-3-ketone

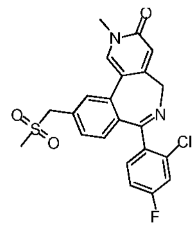

TFA (1 mL) were added into ((5-(2-(2-chloro-4-fluorobenzoyl)-5-((dimethylfulfone)methyl)phenyl)-1-methyl-2-oxo-1,2-dihydr opyridin-4-yl)methyl)*tert*-butylcarbamate (76 mg, 0.13 mmol) in dichloromethane (4 mL) solution. The reaction mixture was stirred at r.t. for 1 hour then concentrated. The residue was purified by preparative-HPLC, the title compound was obtained (4.4 mg, 8%), which was in form of white solid. LCMS (ESI) [M+H]+ = 445.1; $^1$H NMR (400 MHz, *D*MSO-$d_6$) $\delta$ 8.11 (s, 1H). 7.71 (s, 1H). 7.61-7.57 (m, 1H). 7.44-7.33 (m, 3H). 7.07-7.05 (d, *J* = 8.4 Hz, 1H). 6.52 (s, 1H). 4.78-4.75 (d, *J* = 10.8 Hz, 1H). 4.64-4.51 (m, 2H). 3.98-3.95 (d, *J* = 10.8 Hz, 1H). 3.54 (s, 3H). 2.96 (s, 3H).

**Embodiment 4099: 1-(7-(4-flurophenyl)-2,5-dimethyl-3-oxo-3,5-dihydro-2*H*-benzo[*e*]pyrido[4,3-*c*][1,2]diazepin-10-yl)-*N*-methylmethane sulfamide**

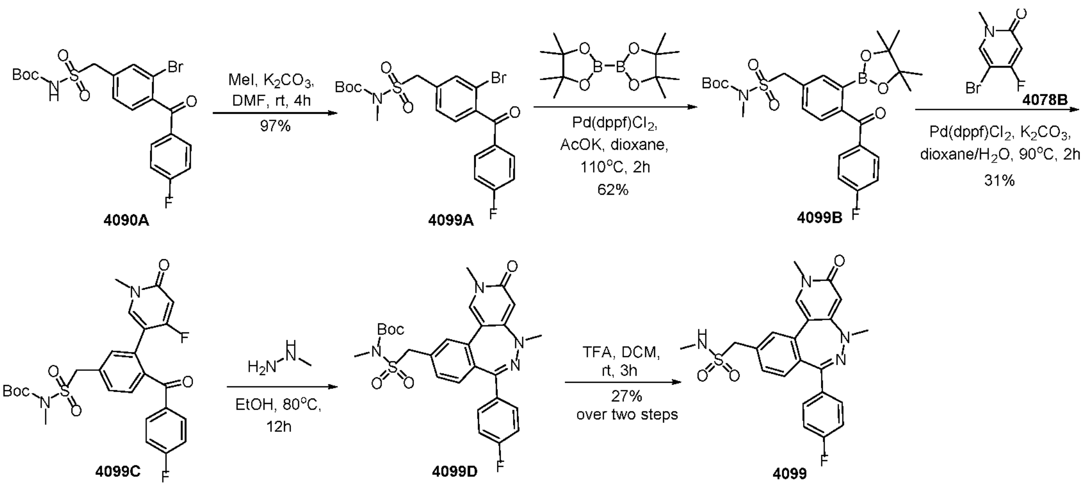


4090A 4099A 4099B 4099C 4099D 4099

Step 1: ((3-Bromo-4-(4-fluorobenzoyl)phenyl)sulfonyl)(methyl)tert-butylcarbamate

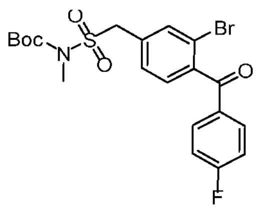

((3-Bromo-4-(4-fluorobenzoyl)benzylmethyl)sulfonyl)*tert*-butylcarbamate (410 mg, 0.87 mmol), methyl iodide (136 mg, 0.96 mmol) and potassium carbonate (180 mg, 1.30 mmol) were mixed in *N,N*-dimethylformamide (5 mL), was stirred at r.t. for 4 hours. The reaction mixture was diluted with EtOAc (60 mL), washed by saturated brine and water, dried over anhydrous sodium sulfate, filtered and concentrated, the title compound was obtained (410 mg, 97%), in form of yellow oil. LCMS (ESI) $[M+H]^+$ = 430.0.

Step 2: ((4-(4-Fluorobenzoyl)-3-(4,4,5,5-tetramethyl-1,3,2-dioxaborolan-2-yl)benzylmethyl)sulfonyl)(met hyl)tert-butylcarbamate

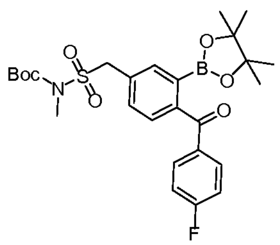

((3-Bromo-4-(4-fluorobenzoyl)benzylmethyl)sulfonyl)(methyl)*tert*-butylcarbamate (410 mg, 0.84 mmol), bis(pinacolato)diboron (428 mg, 1.67 mmol), [1,1'-bis(diphenylphosphino)ferrocene]palladium (II) dichloride (58 mg, 0.08 mmol) and potassium acetate (207 mg, 2.11 mmol) were mixed in 1,4-dioxane (10 mL), heated to 110°C under nitrogen protection then stirred for 2 hours. The reaction mixture was filtered and the filtrate was concentrated, the residue was purified by flash chromatography (PE/EtOAc=3/2). The title compound was obtained (280 mg, 62%), in form of yellow oil. LCMS (ESI) $[M+H]^+$ = 477.9.

Step 3: ((3-(4-Fluoro-1-methyl-6-oxo-1,6-dihydropyridin-3-yl)-4-(4-fluorobenzoyl)benzylmethyl)sulfonyl )(methyl)tert-butylcarbamate

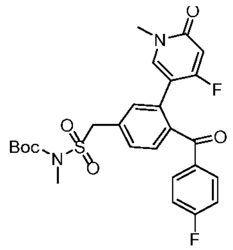

((4-(4-Fluorobenzoyl)-3-(4,4,5,5-tetramethyl-1,3,2-dioxaborolan-2-yl)benzylmethyl)sulfonyl)(methyl)tert-butylcarbamate (230 mg, 0.43 mmol), 5-bromo-4-fluoro-1-methylpyridin-2(1*H*)-ketone (80 mg, 0.39 mmol), [1,1'-bis(diphenylphosphino)ferrocene]palladium (II) dichloride (28 mg, 0.039 mmol) and potassium carbonate (135 mg, 0.98 mmol) were mixed in 1,4-dioxane (5 mL) and water (0.5 mL), and heated to 90°C then stirred for 2 hours under nitrogen protection. Water and EtOAc were added to the reaction mixture, the organic phase was separated. The organic phase was dried over anhydrous sodium sulfate, filtered and concentrated. The residue was purified by flash chromatography (EtOAc/methanol=3/2). The title compound was obtained (70 mg, 31%), which was in form of yellow solid. LCMS (ESI) $[M+H]^+$ = 532.8.

Step 4: (((7-(4-Flurophenyl)-2,5-dimethyl-3-oxo-3,5-dihydro-2*H*-benzo[*e*]pyrido[4,3-*c*][1,2]diazepin-10-y l)methyl)sulfonyl)(methyl)*tert*-butylcarbamate

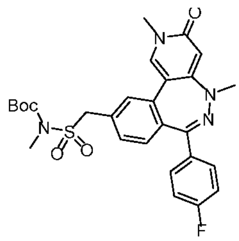

((3-(4-Fluoro-1-methyl-6-oxo-1,6-dihydropyridin-3-yl)-4-(4-fluorobenzoyl)benzylmethyl)sulfonyl)(methanol)*tert*-butylcarbamate (70 mg, 0.13 mmol) and methylhydrazine (61 mg, 1.3 mmol) was dissolved in ethanol (4 mL), and heated to 80°C and stirred for 12 hours. Concentration of the reaction mixture, the crude product of the title compound was obtained, which was in form of yellow oil, and was used directly in the next step without purification. LCMS (ESI) $[M+H]^+$ = 540.9.

Step 5: 1-(7-(4-Flurophenyl)-2,5-dimethyl-3-oxo-3,5-dihydro-2*H*-benzo[*e*]pyrido[4,3-*c*][1,2]diazepin-10-yl)-*N*-methylmethane sulfamide

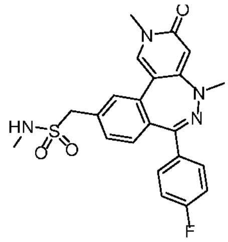

(((7-(4-Flurophenyl)-2,5-dimethyl-3-oxo-3,5-dihydro-2*H*-benzo[*e*]pyrido[4,3-*c*][1,2]diazepin-10-yl)methyl)sulfonyl)(methyl)*tert*-butylcarbamate (crude product) and TFA (2 mL) was dissolved in dichloromethane (3 mL), stirred at r.t. for 3 hours. Concentration of the reaction mixture, the residue was purified by preparative-HPLC, the title compound was obtained (15.5 mg, yield of the two steps 27%), which was in form of yellow solid. LCMS (ESI) $[M+H]^+$ = 440.9; $^1H$ NMR (400 MHz, $CD_3OD$) $\delta$ 7.89 (s, 1H). 7.76 (s, 1H). 7.57-7.53 (m, 3H). 7.45 (d, $J$ = 8.0 Hz, 1H). 7.18-7.13 (m, 3H). 6.22 (s, 1H). 4.46 (s, 2H). 3.63 (s, 3H). 3.16 (s, 2H). 2.76 (s, 3H).

**Embodiment 4100: 10-((ethylsulfonyl)methyl)-7-(4-fluoro-3-methoxylphenyl)-2,5-dimethyl-2,5-dihydro-3*H* -benzo[*c*]pyridazino[3,4-*e*]azepin-3-ketone**

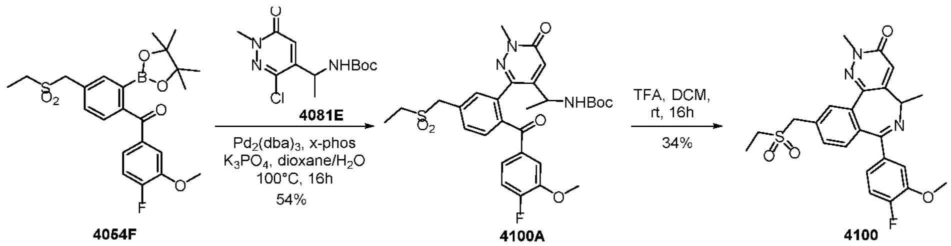


Step 1: (1-(3-(5-((Ethylsulfonyl)methyl)-2-(4-fluoro-3-methoxylbenzoyl)phenyl)-1-methyl-6-oxo-1,6-dih ydropyridazine-4-yl)ethyl)*tert*-butylcarbamate

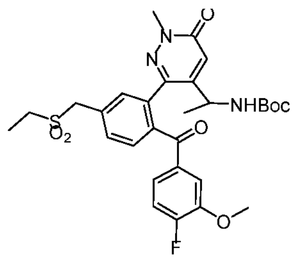

(1-(3-Chloro-1-methyl-6-oxo-1,6-dihydropyridazine-4-yl)ethyl)tert-butylcarbamate (50 mg, 0.17 mmol), (4-((ethylsulfonyl)methyl)-2-(4,4,5,5-tetramethyl-1,3,2-dioxaborolan-2-yl)phenyl)(4-fluoro-3-methoxylphenyl)methanone (241 mg, 0.52 mmol), tri(dibenzyl acetylacetone) dipalladium (16 mg, 0.02 mmol). 2-bicyclohexylphosphine-2',4',6'-triisopropyl biphenyl(16 mg, 0.03 mmol) and potassium phosphate (92 mg, 0.43 mmol) were mixed in 1,4-dioxane (4 mL) and water (2 drops), and heated to 100°C and stirred overnight under nitrogen protection. Concentration of the reaction mixture, the residue was purified by flash chromatography (PE/EtOAc=1/2). The title compound was obtained (55 mg, 54%), in form of yellow oil. LCMS (ESI) $[M+H]^+$ = 588.3.

Step 2: 10-((Ethylsulfonyl)methyl)-7-(4-fluoro-3-methoxylphenyl)-2,5-dimethyl-2,5-dihydro-3*H*-benzo[*c*] pyridazino[3,4-*e*]azepin-3-ketone

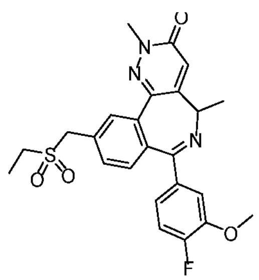

(1-(3-(5-((Ethylsulfonyl)methyl)-2-(4-fluoro-3-methoxylbenzoyl)phenyl)-1-methyl-6-oxo-1,6-dihydropyridazine-4-yl)ethyl)*tert*-butylcarbamate (55 mg, 0.09 mmol) and TFA (1 mL) was dissolved in dichloromethane (4 mL), then stirred at r.t. overnight. Concentration of the reaction mixture, the residue was purified by preparative-HPLC, the title compound was obtained (14.8mg, 34%), which was in form of white solid. LCMS (ESI) $[M+H]^+$ = 470.1; $^1H$ NMR (400 MHz, $CD_3OD$) $\delta$ 8.10-8.09 (s, 1H). 7.70-7.67 (m, 1H). 7.42-7.40 (d, *J* = 8.0 Hz, 1H). 7.38-7.35 (m, 1H). 7.13-7.08 (m, 1H). 7.00 (s, 1H). 6.91-6.87 (m, 1H). 4.63 (s, 2H). 4.21 -4.19 (q, *J* = 6.8 Hz, 1H). 3.90 (s, 1H). 3.88 (s, 3H). 3.20-3.14 (m, 2H). 1.79-1.77 (d, *J* = 6.4 Hz, 3H). 1.43-1.39 (t, *J* = 7.6 Hz, 3H).

**Embodiment 4101: (*S*)-7-(2-chloro-4-flurophenyl)-10-((ethylsulfonyl)methyl)-2,5-dimethyl-2,5-dihydro-3*H*-benz o[*c*]pyrido[3,4-*e*]azepin-3-ketone**

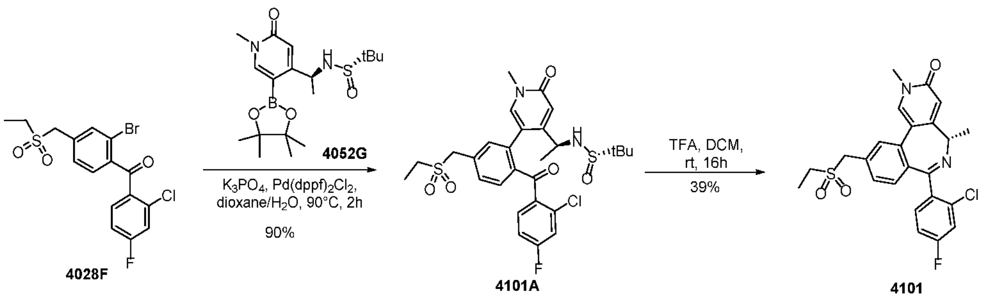


4028F 4101A 4101

Step 1: (*S*)-*N*-(1-(5-(2-(2-chloro-4-fluorobenzoyl)-5-((ethylsulfonyl)methyl)phenyl)-1-methyl-2-oxo-1,2-d ihydropyridin-4-yl)ethyl)-2-methylpropane-2-sulfenamide

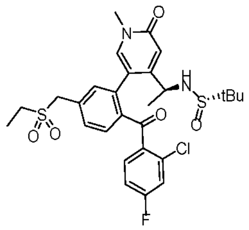

(*S*)-2-Methyl-*N*-((*S*)-1-(1-methyl-2-oxo-5-(4,4,5,5-tetramethyl-1,3,2-dioxaborolan-2-yl)-1,2-dihydropyridin-4-yl)ethyl)propane-2-sulfenamide (76 mg, 0.2 mmol), (2-bromo-4-((ethylsulfonyl)methyl)phenyl)(2-chloro-4-flurophenyl)methanone (41 mg, 0.1 mmol), [1,1'-bis(diphenylphosphino)ferrocene]palladium (II) dichloride (11 mg, 0.015 mmol) and potassium phosphate (64 mg, 0.3 mmol) were mixed in 1,4-dioxane (2 mL) and water (0.2 mL), and heated to 90°C then stirred for 2 hours under nitrogen protection. Concentration of the reaction mixture, the residue was purified by flash chromatography (EtOAc/methanol=10/1). The title compound was obtained (54 mg, 90%), which was in form of yellow solid. LCMS (ESI) $[M+H]^+$ = 595.0.

Step 2: (*S*)-7-(2-Chloro-4-flurophenyl)-10-((ethylsulfonyl)methyl)-2,5-dimethyl-2,5-dihydro-3*H*-benzo[*c*] pyrido[3,4-*e*]azepin-3-ketone

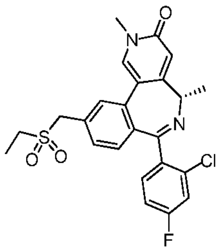

TFA (1 mL) was added into ($S$)-$N$-(1-(5-(2-(2-chloro-4-fluorobenzoyl)-5-((ethylsulfonyl)methyl)phenyl)-1-methyl-2-oxo-1,2-d ihydropyridin-4-yl)ethyl)-2-methylpropane-2-sulfenamide (54 mg, 0.09 mmol) in dichloromethane (4 mL) solution. The reaction mixture was stirred at r.t. for 16 hours then concentrated. The residue was purified by preparative-HPLC, the title compound was obtained (16.1 mg, 39%), which was in form of white solid. LCMS (ESI) $[M+H]^+$ = 473.1; $^1H$ NMR (400 MHz, $D$MSO-$d_6$) $\delta$ 8.05 (s, 1H). 7.70 (s, 1H). 7.60-7.56 (m, 1H). 7.43-7.40 (m, 2H). 7.38-7.31 (m, 1H). 7.07 (d, $J$ = 8.0 Hz, 1H). 6.42 (s, 1H). 4.63-4.50 (m, 2H). 4.02 (q, $J$ = 6.4 Hz, 1H). 3.53 (s, 3H). 3.08 (q, $J$ = 7.6 Hz, 2H). 1.64 (d, $J$ = 5.6 Hz, 3H). 1.22 (s, $J$ = 7.6 Hz, 3H).

**Embodiment 4102: (S)-7-(2-chloro-4-fluoro-5-methoxylphenyl)-10-((ethylsulfonyl)methyl)-2,5-dimethyl-2,5-dihy dro-3*H*-benzo[*c*]pyrido[3,4-*e*]azepin-3-ketone**

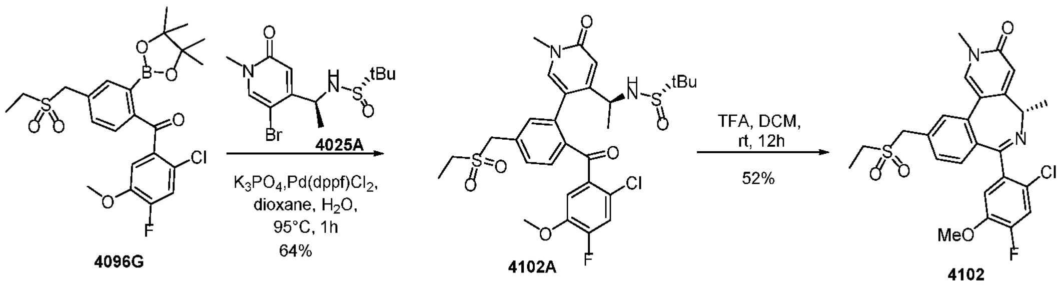


4096G 4102A 4102

Step 1: ($S$)-$N$-(($S$)-1-(5-(2-(2-chloro-4-fluoro-5-methoxylbenzoyl)-5-((ethylsulfonyl)methyl)phenyl)-1-me thyl-2-oxo-1,2-dihydropyridin-4-yl)ethyl)-2-methylpropane-2-sulfenamide

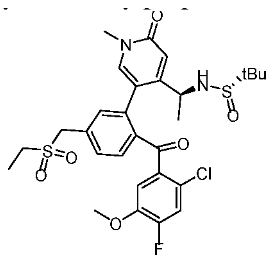

(2-Chloro-4-fluoro-5-methoxylphenyl)(4-((ethylsulfonyl)methyl)-2-(4,4,5,5-tetramethyl -1,3,2-dioxaborolan-2-yl)phenyl)methanone (100 mg, 0.2 mmol), ($S$)-$N$-(($S$)-1-(5-bromo-1-methyl-2-oxo-1,2-dihydropyridin-4-yl)ethyl)-2-methylpropane-2-sulfena mide (90 mg, 0.27 mmol), [1,1'-bis(diphenylphosphino)ferrocene]palladium (II) dichloride (20 mg, 0.03 mmol) and potassium phosphate (130 mg, 0.61 mmol) were mixed in 1,4-dioxane (5 mL) and water (1 drop), and heated to 95°C then stirred for 1 hour under nitrogen protection. The reaction mixture was filtered and the filtrate was concentrated, the residue was purified by flash chromatography (EtOAc/methanol=30/1). The tiltle compound was obtained (80 mg, 64%), which was in form of yellow solid. LCMS (ESI) $[M+H]^+$ = 625.3.

Step 2: (*S*)-7-(2-Chloro-4-fluoro-5-methoxylphenyl)-10-((ethylsulfonyl)methyl)-2,5-dimethyl-2,5-dihydro -3*H*-benzo[*c*]pyrido[3,4-*e*]azepin-3-ketone

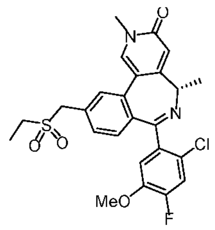

TFA (1 mL) was added into (*S*)-*N*-((*S*)-1-(5-(2-(2-chloro-4-fluoro-5-methoxylbenzoyl)-5-((ethylsulfonyl)methyl)phenyl)-1-met hyl-2-oxo-1,2-dihydropyridin-4-yl)ethyl)-2-methylpropane-2-sulfenamide (80 mg, 0.13 mmol) in dichloromethane (4 mL) solution. The reaction mixture was stirred at r.t. for 12 hours and concentrated, the residue was purified by preparative-HPLC, the tiltle compound was obtained (33.6 mg, 52%), which was in form of white solid. LCMS (ESI) $[M+H]^+$ = 503.3; $^1H$ NMR (400 MHz, DMSO-$d_6$) $\delta$ 8.05 (s, 1H). 7.69 (s, 1H). 7.41-7.38 (t, *J* = 8.0 Hz, 2H). 7.31-7.29 (d, *J* = 9.2 Hz, 1H). 7.14-7.12 (d, *J* = 8 Hz, 1H). 6.4 (s, 1H). 4.63-4.50 (q, *J* = 13.6 Hz, 2H). 4.02-4.00 (q, *J* = 6.8 Hz, 1H). 3.90 (s, 3H). 3.53 (s, 3H). 3.11-3.05 (q, *J* = 7.2 Hz, 2H). 1.68-1.65 (d, *J* = 6.0 Hz, 3H). 1.23-1.20 (t, *J* = 7.6 Hz, 3H).

**Embodiment 4103: (*S*)-1-(7-(4-fluoro-3-methoxylphenyl)-2,5-dimethyl-3-oxo-3,5-dihydro-2*H*-benzo[*c*]pyrido[3,4 -*e*]azepin-10-yl)-*N*-methylmethane sulfamide**

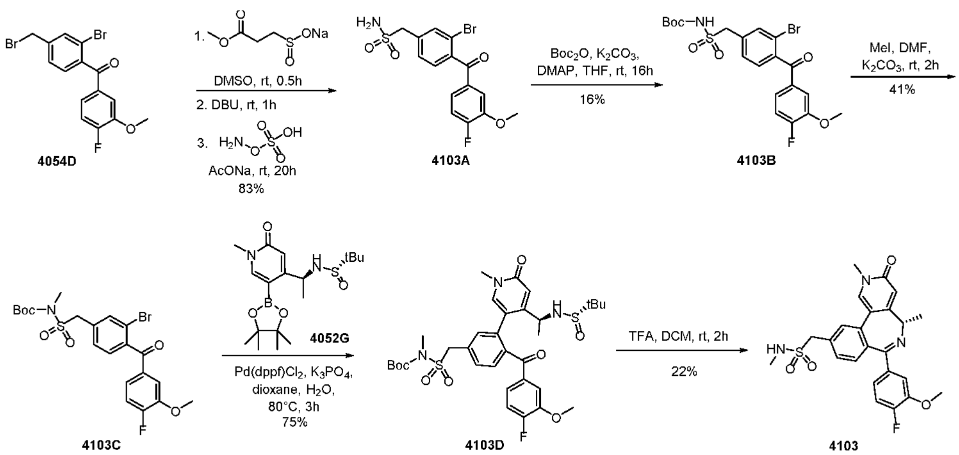


Step 1: (3-Bromo-4-(4-fluoro-3-methoxylbenzoyl)phenyl)methylsulfamide

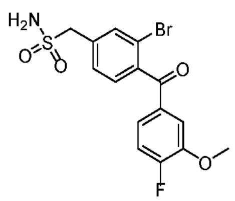

(2-Bromo-4-(bromomethyl)phenyl)(4-fluoro-3-methoxylphenyl)methanone (1.2 g, 3.2 mmol) and 3-methoxyl-3-oxo propane sodium sulfinate (0.62 g, 3.6 mmol) was dissolved in dimethylsulfoxide (20 mL), stirred at r.t. for 0.5 hour. Then added 1,8-diazabicyclo[5.4.0]undec-7-ene (360 mg, 3.6 mmol), and continued stirring at r.t. for 1 hour. Sodium

acetate (830 mg, 9.0 mmol) and hydroxylamine sulfonic acid (1.1 g, 9.0 mmol) in water (5 mL) solution was added into the reaction mixture, the obtained reaction mixture was stirred at r.t. for 20 hours. The reaction mixture was diluted with EtOAc (80 mL), and washed by saturated brine and water then concentrated. The residue was purified by flash chromatography (PE/EtOAc=1/1). The title compound was obtained (1.0 g, 83%), in form of white solid. LCMS (ESI) $[M+H]^+$ = 401.9.

Step 2: ((3-Bromo-4-(4-fluoro-3-methoxylbenzoyl)benzylmethyl)sulfonyl) *tert*-butylcarbamate

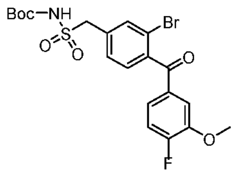

(3-Bromo-4-(4-fluoro-3-methoxylbenzoyl)phenyl)methane sulfamide (400 mg, 1.0 mmol), di-*tert*-butyl dicarbonate (260 mg, 1.2 mmol), potassium carbonate (278 mg, 2.0 mmol) and 4-dimethylaminopyridine (25 mg, 0.2 mmol) was dissolved in THF (10 mL), was stirred at r.t. for 16 hours. Concentration of the reaction mixture, the residue was purified by flash chromatography (PE/EtOAc=1/1). The title compound was obtained (80 mg, 16%), which was in form of yellow solid. LCMS (ESI) $[M+Na]^+$ = 524.0.

Step 3: ((3-Bromo-4-(4-fluoro-3-methoxylbenzoyl)benzylmethyl)sulfonyl)(methyl) *tert*-butylcarbamate

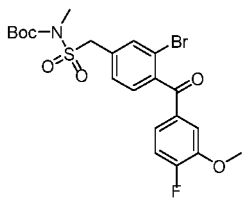

((3-Bromo-4-(4-fluoro-3-methoxylbenzoyl)benzylmethyl)sulfonyl) *tert*-butylcarbamate (500 mg, 1.0 mmol), methyl iodide (295 mg, 2.0 mmol) and potassium carbonate (280 mg, 2.0 mmol) were mixed in *N,N*-dimethylformamide (5 mL), then stirred at r.t. for 2 hours. The reaction mixture was diluted with EtOAc (80 mL), and washed by saturated brine and water, then concentration to deliver the title compound (210 mg, 41%), which was in form of yellow solid. LCMS (ESI) $[M+H-56]^+$ = 460.0.

Step 4: ((3-(4-((*S*)-1-(((*S*)-*tert*-butyl-sulfinyl)amino)ethyl)-1-methyl-6-oxo-1,6-dihydropyridin-3-yl)-4-(4-fluoro-3-methoxylbenzoyl)benzylmethyl)sulfonyl)(methyl) tert-butylcarbamate

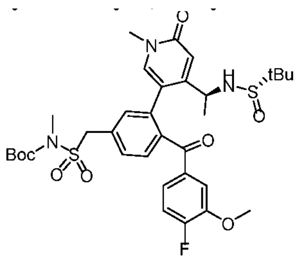

((3-Bromo-4-(4-fluoro-3-methoxylbenzoyl)benzylmethyl)sulfonyl)(methyl) *tert*-butylcarbamate (103 mg, 0.2 mmol), (*S*)-2-methyl-*N*-((*S*)-1-(1-methyl-2-oxo-5-(4,4,5,5-tetramethyl-1,3,2-dioxaborolan-2-yl)-1,2-dihyd ropyridin-4-yl)ethyl)propane-2-sulfenamide (77 mg, 0.2 mmol), [1,1'-bis(diphenylphosphino)ferrocene]palladium (II) dichloride (20 mg, 0.026 mmol) and potassium phosphate (84 mg, 0.396 mmol) were mixed in 1,4-dioxane (4 mL) and water (0.4 mL), and heated to 80°C and stirred for 3 hours. The reaction mixture was filtered and the filtrate was concentrated, the residue was purified by flash chromatography (EtOAc/methanol=30/1). The title compound was obtained (103 mg, 75%), which was in form of yellow solid. LCMS (ESI) $[M+H]^+$ = 692.1.

Step 5: (*S*)-1-(7-(4-Fluoro-3-methoxylbenzoyl)-2,5-dimethyl-3-oxo-3,5-dihydro-2*H*-benzo[*c*]pyrido[3,4-*e* ]azepin-10-yl)-*N*-methylmethane sulfonyl

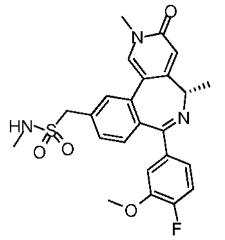

TFA (1 mL) was added into ((3-(4-((*S*)-1-(((*S*)-tert-butyl-sulfinyl)amino)ethyl)-1-methyl-6-oxo-1,6-dihydropyridin-3-yl)-4-(4-fluoro-3-methoxylbenzoyl)benzylmethyl)sulfonyl)(methyl) tert-butylcarbamate (103 mg, 0.15 mmol) in dichloromethane (2 mL) solution. The reaction solution was stirred at r.t. for 2 hours and concentrated, the residue was purified by preparative-HPLC, the title compound was obtained (15.7 mg, 22%) in form of white solid. LCMS (ESI) $[M+H]^+$ = 470.2; $^1$H NMR (400 MHz, $CD_3OD$) $\delta$ 8.09 (s, 1H). 7.75 (s, 1H). 7.54 (d, *J* = 8.0 Hz, 1H). 7.38-7.33 (m, 2H). 7.09 (dd, *J* = 1.6 Hz, 8.0 Hz, 1H). 6.88-6.84 (m, 1H). 6.64 (s, 1H). 4.51 (s, 2H). 4.11 (q, *J* = 6.8 Hz, 1H). 3.88 (s, 3H). 3.68 (s, 3H). 2.77 (s, 3H). 1.78 (d, *J* = 6.4 Hz, 3H).

**Embodiment 4104: (*S*)-10-((ethylsulfonyl)methyl)-7-(3-fluoro-4-methoxylphenyl)-2,5-dimethyl-2,5-dihydro-3*H*-benzo[*c*]pyrido[3,4-*e*]azepin-3-ketone**

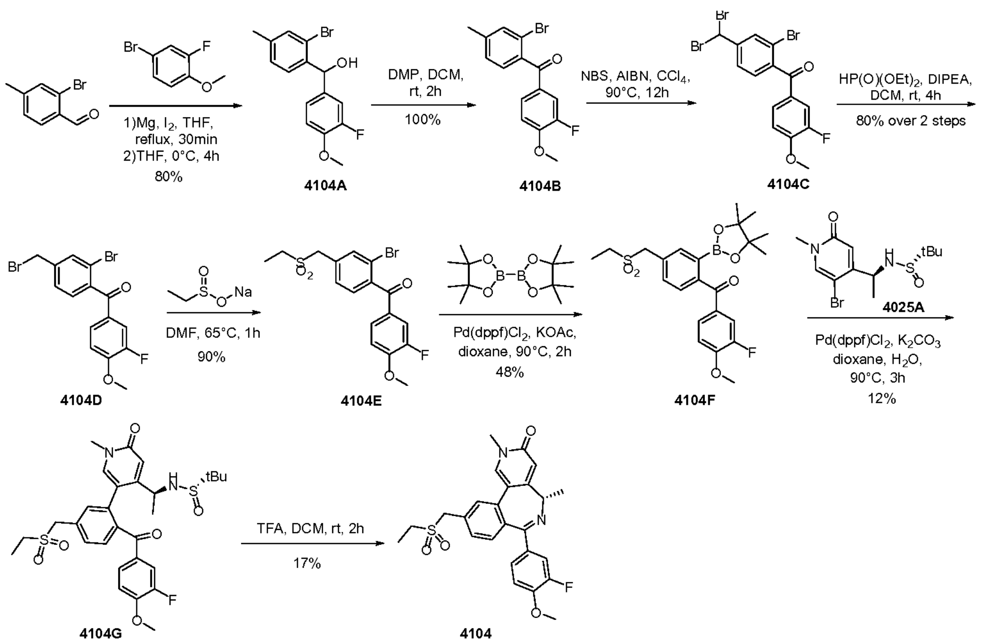


Step 1: (2-Bromo-4-methylphenyl)(3-fluoro-4-methoxylphenyl)methanol

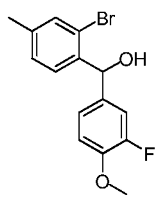

Under nitrogen protection, THF (1 mL) was added into magnesium pells (0.96 g, 40 mmol) and a small piece of $I_2$, then added a small amount of 4-bromo-2-fluoro-1-methoxylbenzyl (4.1 g, 20 mmol) in THF (40 mL). When the reaction was initiated, the reaction mixture was heated to 75°C, then added the rest of the materials in about 15 minutes. The reaction mixture was continued to reflex 30 minutes to obtain the Griganard reagent. 2-Bromo-4-methylbenzaldehyde (4.0 g, 20 mmol) in THF (20 mL) solution was cooled down to 0°C, then slowly added the Griganard reagent, the mixutre obtained was stirred at r.t. for 4 hours. EtOAc and saturated ammonium chloride solution were added into the reaction mixture. The organic phase was washed by saturated brine, dried over anhydrous sodium sulfate then concentrated. The residue was purified by flash chromatography (PE/EtOAc=5/1). The title compound was obtained (5.2 g, 80%), in form of colorless oil. LCMS (ESI) $[M-H_2O+H]^+$ = 307.0.

Step 2: (2-Bromo-4-methylphenyl)(3-fluoro-4-methoxylphenyl)methanone

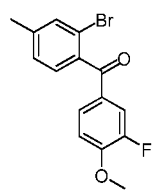

(2-Bromo-4-methylphenyl)(3-fluoro-4-methoxylphenyl)methanol (5.2 g, 16.0 mmol) and Dess-Martin periodinane (7.5 g, 17.6 mmol) were mixed in dichloromethane (40 mL), then stirred at r.t. for 2 hours. Concentration of the reaction mixture, the residue was purified by flash chromatography (PE/EtOAc=5/1). The title compound was obtained (5.2 g, 100%), in form of colorless oil. LCMS (ESI) $[M+H]^+$ = 323.0.

Step 3: (2-Bromo-4-(dibromomethyl)phenyl)(3-fluoro-4-methoxylphenyl)methanone

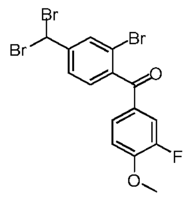

*N*-Bromodesuccinimide (5.7 g, 32 mmol), azobisisobutyl cyanide (50 mg, 0.3 mmol) and (2-bromo-4-methylphenyl)(3-fluoro-4-methoxylphenyl)methanone (5.2 g, 16.0 mmol) were mixed in tetrachloromethane (100 mL), and heated to 90°Cthen stirred for 12 hours. The reaction mixture was concentrated and the crude product of the title compound was obtained, and was used directly in the next step without purification. LCMS (ESI) $[M+H]^+$ = 478.8.

Step 4: (2-Bromo-4-(bromomethyl)phenyl)(3-fluoro-4-methoxylphenyl)methanone

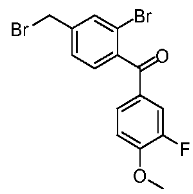

Triethyl phosphate (4.7 g, 32 mmol) and diisopropyl ethylamine (4.1 g, 32 mmol) was added into (2-bromo-4-(dibromomethyl)phenyl)(3-fluoro-4-methoxylphenyl)methanone (7.6 g, 16 mmol) in dichloromethane (100 mL) solution. The reaction mixture was stirred at r.t. for 4 hours and then concentrated. The residue was purified by flash chromatography (PE/EtOAc=3/1). The title compound was obtained (5.14 g, yield of the two steps 80%), in form of colorless oil. LCMS (ESI) $[M+H]^+$ = 400.9.

Step 5: (2-Bromo-4-((ethylsulfonyl)methyl)phenyl)(3-fluoro-4-methoxylphenyl)methanone

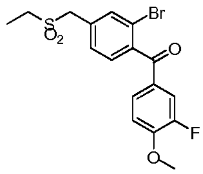

Sodium ethanesulfonate (90 mg, 0.75 mmol) was added into (2-bromo-4-(bromomethyl)phenyl)(3-fluoro-4-methoxylphenyl)methanone (0.3 g, 0.75 mmol) in *N,N*-dimethylformamide (3 mL) solution. The reaction mixture and heated to 65°C then stirred for 1 hour. EtOAc (20 mL) and water (50 mL) were added to the reaction mixture, the organic phase was separated. The organic phase was washed by saturated brine (50 mL*3), dried over anhydrous sodium sulfate, filtered and concentrated. The residue was purified by flash chromatography (PE/EtOAc=1/1). The title compound was obtained (280 mg, 90%). LCMS (ESI) $[M+H]^+$ = 415.0.

Step 6: (4-((Ethylsulfonyl)methyl)-2-(4,4,5,5-tetramethyl-1,3,2-dioxaborolan-2-yl)phenyl)(3-fluoro-4-met hoxylphenyl)methanone

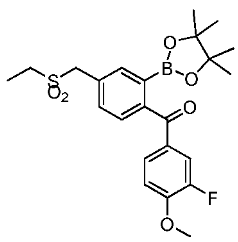

(2-Bromo-4-((ethylsulfonyl)methyl)phenyl)(3-fluoro-4-methoxylphenyl)methanone (210 mg, 0.5 mmol), bis(pinacolato)diboron (270 mg, 1.0 mmol), [1,1'-bis(diphenylphosphino)ferrocene]palladium (II) dichloride (40 mg, 0.0.5 mmol) and potassium acetate (112 mg, 1.1 mmol) were mixed in 1,4-dioxane (5 mL), and heated to 90°C under nitrogen protection then stirred for 2 hours. The reaction mixture was filtered and the filtrate was concentrated, the residue was purified by flash chromatography (PE/EtOAc=1/1). The title compound was obtained (110 mg, 48%), which was in form of yellow solid.CMS (ESI) $[M+H]^+$ = 463.0.

Step 7: (*S*)-*N*-((*S*)-1-(5-(5-((ethylsulfonyl)methyl)-2-(3-fluoro-4-methoxylbenzoyl)phenyl)-1-methyl-2-ox o-1,2-dihydropyridin-4-yl)ethyl)-2-methylpropane-2-sulfenamide

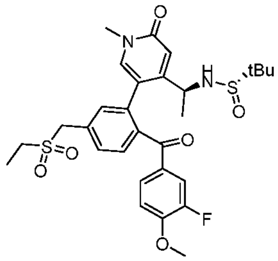

(4-((Ethylsulfonyl)methyl)-2-(4,4,5,5-tetramethyl-1,3,2-dioxaborolan-2-yl)phenyl)(3-flu oro-4-methoxylphenyl)methanone (110 mg, 0.25 mmol), (*S*)-*N*-((*S*)-1-(5-bromo-1-methyl-2-oxo-1,2-dihydropyridin-4-yl)ethyl)-2-methylpropane-2-sulfena mide (82 mg, 0.25 mmol), [1,1'-bis(diphenylphosphino)ferrocene]palladium (II) dichloride (20 mg, 0.025 mmol) and potassium carbonate (70 mg, 0.5 mmol) were mixed in 1,4-dioxane (4 mL) and water (0.4 mL), heated to 90°C and stirred for 3 hours under nitrogen protection. The reaction mixture was filtered and the filtrate was concentrated, the residue was purified by flash chromatography (EtOAc/methanol=2/1). The title compound was obtained (18 mg, 12%), which was in form of yellow solid. LCMS (ESI) $[M+H]^+$ = 591.2.

Step 8: (*S*)-10-((Ethylsulfonyl)methyl)-7-(3-fluoro-4-methoxylphenyl)-2,5-dimethyl-2,5-dihydro-3*H*-benz o[*c*]pyrido[3,4-*e*]azepin-3-ketone

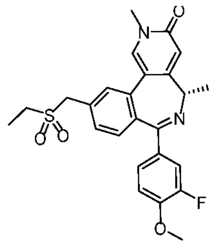

TFA (1 mL) was added into (*S*)-*N*-((*S*)-1-(5-(5-((ethylsulfonyl)methyl)-2-(3-fluoro-4-methoxylbenzoyl)phenyl)-1-methyl-2-oxo-1,2-dihydropyridin-4-yl)ethyl)-2-methylpropane-2-sulfenamide (18 mg, 0.03 mmol) in dichloromethane (1 mL) solution. The reaction solution was stirred at r.t. for 2 hours then concentrated. The residue was purified by preparative-HPLC, the title compound was obtained (2.4 mg, 17%), which was in form of white solid. LCMS (ESI) $[M+H]^+$ = 469.2; $^1$H NMR (400 MHz, $CD_3OD$) $\delta$ 8.09 (s, 1H). 7.76 (s, 1H). 7.55 (d, *J* = 8.0 Hz, 1H). 7.40 (d, *J* = 8.0 Hz, 1H). 7.30 (d, *J* = 8.4 Hz, 1H). 7.17 (d, *J* = 8.4 Hz, 1H). 7.10 (t, *J* = 8.4 Hz, 2H). 6.64 (s, 1H). 4.60 (s, 2H). 4.09 (q, *J* = 6.0 Hz, 1H). 3.92 (s, 3H). 3.68 (s, 3H). 3.19 (q, *J* = 6.0 Hz, 2H). 1.76 (d, *J* = 6.4 Hz, 3H). 1.42 (t, *J* = 6.4 Hz, 3H).

**Embodiment 4105: (*S*)-10-((ethylsulfonyl)methyl)-2,5-dimethyl-7-(pyridin-4-yl)-2,5-dihydro-3*H*-benzo[*c*]pyrido[3,4-*e*]azepin-3-ketone**

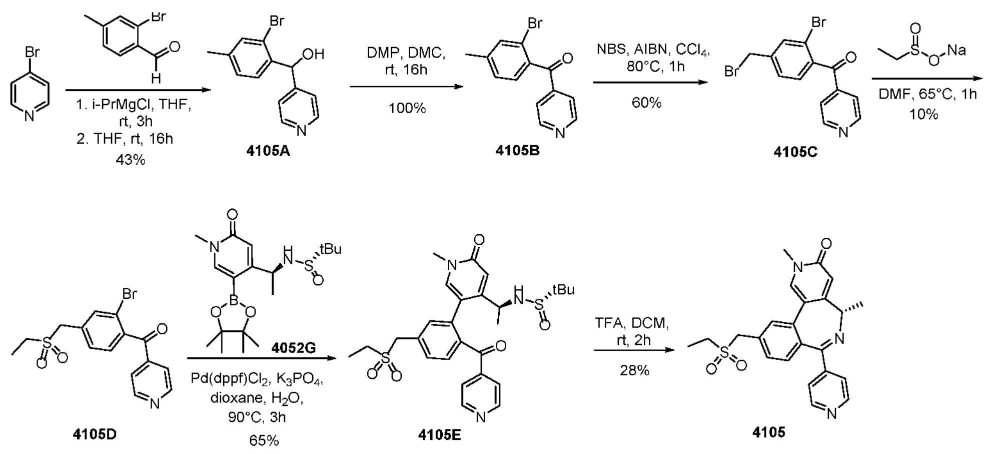


Step 1: (2-Bromo-4-methylphenyl)(pyridin-4-yl)methanol

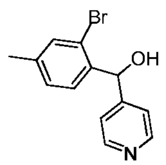

Under nitrogen protection, isopropyl magnesium chloride (2 M in THF solution, 7.5 mL, 15 mmol) was added into 4-bromopyridine (2.3 g, 15 mmol) in anhydrous THF (30 mL) solution, the reaction mixture was stirred at r.t. for 3 hours to obtain the Griganard reagent. Under nitrogen protection, the prepared Griganard reagent was added into 2-bromo-4-methylbenzaldehyde (3.0 g, 15 mmol) in anhydrous THF (10 mL) solution, then the mixture obtained was stirred at r.t. overnight. The reaction was quenched by saturated ammonium chloride (20 mL), and extracted by EtOAc (20 mL*3). The organic phase was washed by saturated brine (50 mL), dried over anhydrous sodium sulfate, filtered and concentrated. The residue was purified by flash chromatography (PE/EtOAc=1/1). The title compound was obtained (1.8 g, 43%), which was in form of white solid. LCMS (ESI) $[M+H]^+$ =278.0.

Step 2: (2-Bromo-4-methylphenyl)(pyridin-4-yl)methanone

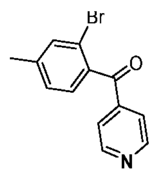

(2-Bromo-4-methylphenyl)(pyridin-4-yl)methanol (1.53 g, 5.5 mmol) and Dess-Martin periodinane (2.6 g, 6.1 mmol) were mixed in dichloromethane (20 mL), was stirred at r.t. for 16 hours. Concentration of the reaction mixture, the residue was purified by flash chromatography (PE/EtOAc=5/1). The title compound was obtained (1.53 g, 100%), in form of colorless oil. LCMS (ESI) $[M+H]^+$ = 276.0.

Step 3: (2-Bromo-4-(bromomethyl)phenyl)(pyridin-4-yl)methanone

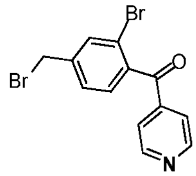

*N*-Bromodesuccinimide (1.06 g, 6.0 mmol), azobisisobutyronitrile (50 mg, 0.3 mmol) and (2-bromo-4-methylphenyl)(pyridin-4-yl)methanone (1.37 g, 5.0 mmol) was dissolved in tetrachloromethane (30 mL), and heated to 80°C then stirred for 1 hour. Concentration of the reaction mixture, the residue was purified by flash chromatography (PE/EtOAc=5/1). The title compound was obtained (1.05 g, 60%), which was in form of white solid. LCMS (ESI) $[M+H]^+$ = 353.9.

Step 4: (2-Bromo-4-((ethylsulfonyl)methyl)phenyl)(pyridin-4-yl)methanone

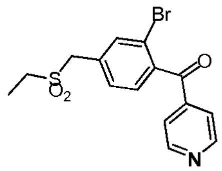

Sodium ethanesulfonate (1.04g, 9.0 mmol) was added into (2-bromo-4-(bromomethyl)phenyl)(pyridin-4-yl)methanone (1.05g, 3.0 mmol) in *N,N*-dimethylformamide (20 mL) solution, the reaction mixture then stirred at 65°C for 1 hour. EtOAc (20 mL) and water (50 mL) was added into the reaction solution, the organic phase was separated. The organic phase was washed by saturated brine (50 mL*3), dried over anhydrous sodium sulfate, filtered and concentrated, the residue was purified by flash chromatography (PE/EtOAc=1/1). The title compound was obtained (110 mg, 10%). LCMS (ESI) $[M+H]^+$ = 368.0.

Step 5: (*S*)-*N*-((*S*)-1-(5-(5-((ethylsulfonyl)methyl)-2-isonicotinylphenyl)-1-methyl-2-oxo-1,2-dihydropyridin-4-yl)ethyl)-2-methylpropane-2-sulfenamide

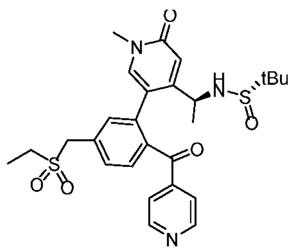

(2-Bromo-4-((ethylsulfonyl)methyl)phenyl)(pyridin-4-yl)methanone (110 mg, 0.3 mmol), (*S*)-2-methyl-*N*-((S)-1-(1-methyl-2-oxo-5-(4,4,5,5-tetramethyl-1,3,2-dioxaborolan-2-yl)-1,2-dihydropyridin-4-yl)ethyl)propane-2-sulfenamide (115 mg, 0.3 mmol), [1,1'-bis(diphenylphosphino)ferrocene]palladium (II) dichloride (20 mg, 0.026 mmol) and potassium phosphate (84 mg, 0.4 mmol) were mixed in 1,4-dioxane (4 mL) and water (0.4 mL), heated to 90°C and stirred for 3 hours under nitrogen protection. The reaction mixture was filtered and the filtrate was concentrated, the residue was purified by flash chromatography (EtOAc/methanol=10/1). The title compound was obtained (105 mg, 65%), which was in form of yellow solid. LCMS (ESI) $[M+H]^+$ = 544.0.

Step 6: (*S*)-10-((ethylsulfonyl)methyl)-2,5-dimethyl-7-(pyridin-4-yl)-2,5-dihydro-3*H*-benzo[*c*]pyrido[3,4-*e*]azepin-3-ketone

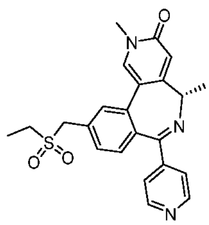

TFA (1 mL) was added into (*S*)-*N*-((*S*)-1-(5-(5-((ethylsulfonyl)methyl)-2-isonicotinylphenyl)-1-methyl-2-oxo-1,2-dihydropyri din-4-yl)ethyl)-2-methylpropane-2-sulfenamide (105 mg, 0.2 mmol) in dichloromethane (2 mL) solution. The reaction solution was stirred at r.t. for 2 hours then concentrated. The residue was purified by preparative-HPLC, the title compound was obtained (23.5 mg, 28%), which was in form of white solid. LCMS (ESI) $[M+H]^+$ = 422.1; $^1H$ NMR (400 MHz, $CD_3OD$) $\delta$ 8.60 (d, *J* = 5.6 Hz, 2H). 8.10 (s, 1H). 7.81 (s, 1H). 7.58 (d, *J* = 8.0 Hz, 1H). 7.51 (d, *J* = 8.0 Hz, 2H). 7.35 (d, *J* = 8.4 Hz, 1H). 6.65 (s, 1H). 4.61 (s, 2H). 4.19 (q, *J* = 6.0 Hz, 1H). 3.68 (s, 3H). 3.20 (q, *J* = 6.0 Hz, 2H). 1.80 (d, *J* = 6.4 Hz, 3H). 1.42 (t, *J* = 6.4 Hz, 3H).

Each of the following effect embodiments 1-3 of the present invention employs (+) **-JQ1** and **ABBV-75** as positive control.

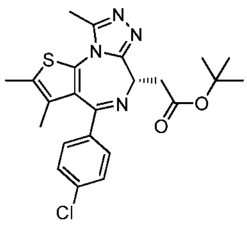

**(+)-JQ1**

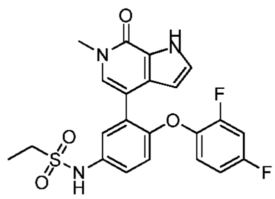

**ABBV-075**

**Effect embodiment 1: Determinations of the enzyme activity levels of the BRD4 (BD1, BD2) of BET protein inhibitor**

Determinations of the enzyme activity levels of the BRD4 (BD1, BD2) of the compounds in the above embodiments, detailed operations are as followed:

Experimental methods:

The binding activity of each compound in the above embodiments to the bromodomain of BRD4 protein was tested by HTRF detection technique, by testing the $IC_{50}$ values of the compound. DMSO was used to serially dilute the compounds. The Diluent Buffer in the reagent kit was used to dilute the BRD4 (BD1, BD2) protein and the Biotin labeled histone H4 peptide fragment, then the reaction solution was prepared. The Detection Buffer in the reagent kit was used to dilute the Anti-GST-$TB^{2+}$Cryptate and SA-XL-665, and the detection solution was prepared. A 384 well plate was used and according to the plate arrangement, the plate was divided into test compound wells, control wells min (intense concentration positive drug), control wells max (DMSO), and control wells containing positive drugs. Then each of the wells were added into corresponding concentration compounds or 20 nL DMSO solution. 10 $\mu$L reaction solution were further added into each of the wells, then followed by 10 $\mu$L detection solution. After 2h incubation at r.t., the Envision detector is used in the TR-Fret mode ($\lambda$ex= 340 nm, $\lambda$em1=615 nm, $\lambda$em2=665 nm) to read the fluorence values and HTRF signal values.

Numerical treatment: inhibition rate = (Max-Signal)/(Max-Min) *100%. Max: the HTRF signal values of Biotin labeled histone H4 peptide fragement binded completely with the protein. Min: the HTRF background value of the Biotin labeled histone H4 peptide that completely non-binding to the protein after adding intensed concentration positive drug. Signal: the HTRF signal value of the corresponding concentration compound. The $IC_{50}$ of the corresponding compound is obtained by fitting the 4-parameters curve with the concentrations of the compounds and its inhibition rates.

Table 1 shows the experimental results of BRD4 biological activity of the compounds.

Table 1

| Compound | BRD4 $IC_{50}$ μM | |
|---|---|---|
| | BD1 | BD2 |
| (+)-JQ1 | 0.013 | 0.006 |
| ABBV-075 | 0.002 | 0.001 |
| 4003 | 0.148 | 0.016 |
| 4001 | 0.124 | 0.025 |
| 4004 | 0.109 | 0.184 |
| 4002 | 0.026 | 0.047 |
| 4005 | 0.163 | 0.550 |
| 4007 | 0.006 | 0.007 |
| 4008 | 0.113 | 0.028 |
| 4009 | 0.006 | 0.005 |
| 4010 | 0.030 | 0.043 |
| 4011 | 0.004 | 0.004 |
| 4012 | 0.004 | 0.002 |
| 4013 | 0.007 | 0.002 |
| 4014 | 0.002 | 0.001 |
| 4015 | 0.002 | 0.001 |
| 4016 | 0.003 | 0.001 |
| 4017 | 0.004 | 0.002 |
| 4018 | 0.003 | 0.002 |
| 4019 | 0.010 | 0.037 |
| 4020 | 0.011 | 0.010 |
| 4022 | 0.012 | 0.007 |
| 4023 | 0.003 | 0.001 |
| 4024 | 0.002 | 0.001 |
| 4025 | 0.007 | 0.003 |
| 4026 | 0.002 | 0.001 |
| 4027 | 0.012 | 0.006 |
| 4028 | 0.022 | 0.002 |
| 4029 | 0.006 | 0.002 |
| 4030 | 0.006 | 0.002 |
| 4031 | 0.002 | 0.001 |
| 4032 | 0.003 | 0.002 |
| 4033 | 0.341 | 0.144 |
| 4034 | 0.004 | 0.001 |
| 4035 | 0.003 | 0.001 |
| 4036 | 0.005 | 0.002 |
| 4037 | 0.002 | 0.001 |

(continued)

| Compound | BRD4 $IC_{50}$ $\mu M$ | |
|---|---|---|
| | BD1 | BD2 |
| **4038** | 0.002 | 0.001 |
| **4039** | 0.163 | 0.204 |
| **4040** | 0.005 | 0.002 |
| **4041** | 0.012 | 0.012 |
| **4042** | 0.001 | 0.001 |
| **4043** | 0.001 | 0.002 |
| **4044** | 0.001 | 0.001 |
| **4045** | 0.001 | 0.001 |
| **4046** | 0.001 | 0.001 |
| **4047** | 0.0004 | 0.001 |
| **4048** | 0.002 | 0.002 |
| **4049** | 0.002 | 0.001 |
| **4050** | 0.017 | 0.009 |
| **4051** | 0.005 | 0.003 |
| **4052** | 0.006 | 0.002 |
| **4053** | 0.002 | 0.001 |
| **4054** | 0.002 | 0.001 |
| **4055** | 0.001 | 0.001 |
| **4056** | 0.001 | 0.001 |
| **4057** | 0.002 | 0.001 |
| **4058** | 0.001 | 0.001 |
| **4059** | 0.002 | 0.001 |
| **4060** | 0.002 | 0.001 |
| **4061** | 0.002 | 0.001 |
| **4062** | 0.001 | 0.001 |
| **4063** | 0.001 | 0.001 |
| **4064** | 0.002 | 0.001 |
| **4065** | 0.002 | 0.001 |
| **4066** | 0.001 | 0.001 |
| **4067** | 0.006 | 0.001 |
| **4068** | 0.001 | 0.0005 |
| **4069** | 0.004 | 0.002 |
| **4070** | 0.003 | 0.002 |
| **4071** | 0.002 | 0.001 |
| **4072** | 0.001 | 0.001 |
| **4073** | 0.001 | 0.001 |
| **4074** | 0.001 | 0.001 |

(continued)

| Compound | BRD4 $IC_{50}$ μM | |
|---|---|---|
| | BD1 | BD2 |
| **4075** | 0.004 | 0.002 |
| **4076** | 0.002 | 0.001 |
| **4077** | 0.001 | 0.0004 |
| **4078** | 0.005 | 0.002 |
| **4079** | 0.003 | 0.001 |
| **4080** | 0.002 | 0.001 |
| **4081** | 0.006 | 0.003 |
| **4082** | 0.002 | 0.001 |
| **4083** | 0.008 | 0.004 |
| **4084** | 0.002 | 0.001 |
| **4085** | 0.002 | 0.001 |
| **4086** | 0.002 | 0.001 |
| **4087** | 0.004 | 0.001 |
| **4088** | 0.003 | 0.001 |
| **4089** | 0.008 | 0.004 |
| **4090** | 0.001 | 0.001 |
| **4091** | 0.001 | 0.0007 |
| **4092** | 0.001 | 0.0006 |
| **4093** | 0.005 | 0.0007 |
| **4094** | 0.003 | 0.002 |
| **4095** | 0.005 | 0.002 |
| **4096** | 0.044 | 0.004 |
| **4097** | 0.020 | 0.003 |
| **4098** | 0.022 | 0.003 |
| **4099** | 0.002 | 0.0009 |
| **4101** | 0.003 | 0.0007 |
| **4102** | 0.005 | 0.0008 |
| **4103** | 0.002 | 0.0006 |
| **4104** | 0.002 | 0.001 |
| **4105** | 0.002 | 0.001 |

Some of the compounds listed in table 1 possess $IC_{50}$ superior than (+)-JQ1, which shows stonger activity, indicating that the compounds of the present invention effectively bind to the protein with bromodomain at biochemical experiment level *in vitro,* thus the compound of the present invention may become an effective therapeutic drug for tumors.

**Effect embodiment 2: the inhibitory effect on MV-4-11 cells**

Determinations of the inhibition of the compounds from the above embodiments against MV-4-11 cells, the detailed operation steps are as followed:

Experimental methods:

**The 1st day: seed the cells on the plate**

1. The cells were verified that they were in good conditions by observation under microscope;
2. The cells were transferred to a 15 mL centrifuge tube, after centrifuged at 1000 rpm for 5 min, the supernatant was discarded;
3. The complete medium (IMDM+10%FBS) was added, then blown and patted into single cell suspension, counted by Vi-cell cytometer, the cell suspension was adjusted to desired cell density by the complete medium.
4. The cells were inoculated into a 96 well plate, the cell number was 15000/well at 100 μL per well, 100 μL complete medium was added into the black control.
5. 37°C, 5% $CO_2$ culture overnight.

**The 2nd day: addition of the drugs**

1. 1000x compound plate preparation

1.1. The test compound was prepared into 10 mM working solution by using DMSO. Then the STS was prepared into 2 mM working solution by using DMSO.
1.2. In the round bottom 96 wells plate, the column 2 of row A to H was added into 60 μL 10 mM test compound (or STS) working solution, columns 3-11 were added into 40 μL DMSO, 20 μL drug solution was pipetted from the column 2 to the column 3, blown and patted for a homogeneous mixture; then 20 μL solution was pipetted from the column 3 to the column 4, blown and patted for a homogeneous mixture, sequently, the drug was diluted by 3 times, 10 concentrations in total. The columns 1 and 12 were supplemented with 40 μL of DMSO.

2. Intermediate plate preparation

A sterile V-bottom 96 deep well plate was used and in each well, 495 μL FBS-free PRMI 1640 medium was added, then 5 μL of the diluted compound (or DMSO) in the 1000x compound plate was pipetted into corresponding position of the V-bottom 96 well plate, blown and patted evenly.

3. Addition of the drugs

3.1. The cell plate was taken out from the incubator and was observed under microscope. The diluted compound or DMSO in the intermediate plate was added into the cell plate, 11 μL each well.
3.2. The cell was continued to be cultured at 37°C, 5% $CO_2$ for 72 hours.

**The 5th day: CellTiter-Glo detection of the cell activity**

1. The CellTiter-Glo buffer and the reaction substrate were taken out from the refrigerator, after balanced to r.t., the buffer was poured into a brown bottle containing the substrate, the bottle was upside down to have the substrate powder sufficientaly dissolved.
2. The cell was observed under microscope; the cell plate was balanced to r.t.
3. The prepared CellTiter-Glo was added into the 96 well plate, at 100 μL/well.
4. The cell plate was placed on a shaker for 10 min, followed by standing at r.t. for 10 min.
5. A white seal film was attached to the bottom of the well plate, and the chemiluminescence signal of each well was detected by the Enspire microplate detector.
6. XLfit was used for data processing. Inhibition rate %=(d-c)/(d-b)×100, while d is the signal value of the DMSO treated group, c is the signal value of the compound treated group, b is signal value of the blank control that only containing medium and DMSO without cells. The data was fitted using the $f(x)205[fit = (A + ((B - A)/(1 + ((C/x)^{\wedge D}))))]$

equation in the XLfit software.

Table 2 shows the experimental results of biological activity of the compounds against MV-4-11 cells.

**Table 2**

| Compound | MV-4-11 (μM) |
|---|---|
| (+)-JQ1 | 0.248 |
| ABBV-075 | 0.007 |
| 4003 | 1.552 |
| 4001 | 1.441 |
| 4002 | 1.125 |
| 4005 | >10 |
| 4007 | 0.118 |
| 4009 | 0.053 |
| 4010 | 0.637 |
| 4011 | 0.065 |
| 4012 | 0.080 |
| 4013 | 0.075 |
| 4014 | 0.292 |
| 4015 | 0.069 |
| 4016 | 0.016 |
| 4017 | 0.010 |
| 4018 | 0.005 |
| 4019 | 0.144 |
| 4020 | 0.134 |
| 4022 | 0.190 |
| 4023 | 0.009 |
| 4024 | 0.009 |
| 4025 | 0.054 |
| 4026 | 0.004 |
| 4027 | 0.128 |
| 4028 | 0.161 |
| 4029 | 0.069 |
| 4030 | 0.017 |
| 4031 | 0.003 |
| 4032 | 0.006 |
| 4033 | 0.985 |
| 4034 | 0.015 |
| 4037 | 0.006 |
| 4038 | 0.004 |
| 4040 | 0.043 |

(continued)

| Compound | MV-4-11 (μM) |
|---|---|
| **4042** | 0.007 |
| **4043** | 0.008 |
| **4044** | 0.007 |
| **4045** | 0.008 |
| **4046** | 0.004 |
| **4047** | 0.002 |
| **4048** | 0.028 |
| **4049** | 0.001 |
| **4051** | 0.042 |
| **4052** | 0.055 |
| **4053** | 0.007 |
| **4054** | 0.009 |
| **4055** | 0.001 |
| **4056** | 0.032 |
| **4057** | 0.004 |
| **4058** | 0.001 |
| **4059** | 0.007 |
| **4060** | 0.019 |
| **4061** | 0.011 |
| **4062** | 0.001 |
| **4063** | 0.003 |
| **4064** | 0.008 |
| **4065** | 0.001 |
| **4066** | 0.0004 |
| **4067** | >2 |
| **4068** | 0.001 |
| **4069** | 0.035 |
| **4070** | 0.016 |
| **4071** | 0.010 |
| **4072** | 0.006 |
| **4073** | 0.002 |
| **4074** | 0.002 |
| **4075** | 0.010 |
| **4076** | 0.114 |
| **4077** | 0.0004 |
| **4078** | 0.014 |
| **4079** | 0.001 |
| **4080** | 0.003 |

(continued)

| Compound | MV-4-11 (μM) |
|---|---|
| **4081** | 0.030 |
| **4082** | 0.011 |
| **4083** | 0.031 |
| **4084** | 0.0005 |
| **4085** | 0.004 |
| **4086** | 0.003 |
| **4087** | 0.026 |
| **4088** | 0.026 |
| **4089** | 0.108 |
| **4090** | 0.003 |
| **4091** | 0.003 |
| **4092** | 0.003 |
| **4093** | 0.017 |
| **4094** | 0.049 |
| **4095** | 0.014 |

**Effect embodiment 3: the inhibitory effect on the SU-DHL-6 cells**

Determinations of the inhibition of the compounds from the above embodiments against **SU-DHL-6** cells, the detailed operation steps are as followed:

Experimental methods:

**The 1st day: seed the cells on the plate**

1. The cells were verified that they were in good conditions by observation under microscope;
2. The cells were transferred to a centrifuge tubes, after centrifuged at 1000rpm for 5 min, the supernatant was discarded;
3. The complete substrate (1640+10%FBS) was added, then blown and patted into single cell suspension, counted by Vi-cell cytometer, the cell suspension was adjusted to desired cell density by the complete medium.
4. The cells were inoculated into a 96 well plate, the cell number was 12000/well at 100 μL per well, 100 μL complete medium was added into the black control;
5. 37°C, 5% $CO_2$ culture overnight.

**The 2nd day: addition of the drugs**

1. 1000x compound plate preparation

1.1. The test compound was prepared into 10 mM working solution by using DMSO. Then the STS was prepared into 2 mM working solution by using DMSO.
1.2. In the round bottom 96 wells plate, the column 2 of row A to H was added into 60 μL 10 mM test compound (or STS) working solution, columns 3-11 were added into 40 μL DMSO, 20 μL drug solution was pipetted from the column 2 to the column 3, blown and patted for a homogeneous mixture; then 20 μL solution was pipetted from the column 3 to the column 4, blown and patted for a homogeneous mixture, sequently, the drug was diluted by 3 times,

10 concentrations in total. The columns 1 and 12 were supplemented with 40 μL of DMSO.

2. Intermediate plate preparation

A sterile V-bottom 96 deep well plate was used and in each well, 495 μL FBS-free PRMI 1640 medium was added, then 5 μL of the diluted compound (or DMSO) in the 1000x compound plate was pipetted into corresponding position of the V-bottom 96 well plate, blown and patted evenly.

3. Addition of the drugs

3.1. The cell plate was taken out from the incubator and was observed under microscope. The diluted compound or DMSO in the intermediate plate was added into the cell plate, 11 μL each well.
3.2. The cell was continued to be cultured at 37°C, 5% $CO_2$ for 72 hours.

**The 5$^{th}$ day: CellTiter- Glo detection of the cell activity**

1. The CellTiter-Glo buffer and the reaction substrate were taken out from the refrigerator, after balanced to r.t., the buffer was poured into a brown bottle containing the substrate, the bottle was upside down to have the substrate powder sufficientaly dissolved.
2. The cell was observed under microscope; the cell plate was balanced to r.t.
3. The prepared CellTiter-Glo was added into the 96 well plate, at 100 μL/well.
4. The cell plate was placed on a shaker for 10 min, followed by standing at r.t. for 10 min.
5. A white seal film was attached to the bottom of the well plate, and the chemiluminescence signal of each well was detected by the Enspire microplate detector.
6. XLfit was used for data processing. Inhibition rate %=(d-c)/(d-b)×100, while d is the signal value of the DMSO treated group, c is the signal value of the compound treated group, b is signal value of the blank control that only containing medium and DMSO without cells. The data was fitted using the $f(x)205[fit = (A + ((B - A)/(1 + ((C/x)^{\wedge D}))))]$ equation in the XLfit software.

Table 3 shows the experimental results of biological activity of the compounds against SU-DHL-6 cells.

Table 3

| Compound | SU-DHL-6 (μM) |
|---|---|
| **(+)-JQ1** | 0.198 |
| **ABBV-075** | 0.021 |
| **4003** | 2.044 |
| **4001** | 1.997 |
| **4002** | 1.931 |
| **4005** | >10 |
| **4007** | 0.253 |
| **4009** | 0.083 |
| **4011** | 0.059 |
| **4026** | 0.010 |

**Effect embodiment 4: Metabolic stability experiment in liver microsomes**

Determinations of the metabolic stability of the compound from the above embodiments, the detailed operation steps are as followed:

Experimental methods:

1. Preparation of buffer C:

Buffer A: 1.0 L of 0.1 M potassium dihydrogen phosphate buffer (containing 1.0 mM EDTA);
Buffer B: 1.0 L of 0.1 M dipotassium hydrogen phosphate buffer (containing 1.0 mM EDTA);
Buffer C: 500 mL Buffer B was titrated with Buffer A to pH 7.4.

2. 10 mM stock solution preparation:

10 mM test compound: A certain amount of the test compound was weighed and dissolved in DMSO.

10 mM control: 5.455 mg of ketanserin dissolved in 1 mL DMSO.

3. Preparation of the drug solution:

500 μM solution: 5 μL of 10 mM stock solution (see step 2) was added into 95 μL ACN;
1.5 μM dosing solution (dissolved in liver microsome solution):
18.75 μL of 20 mg/mL liver microsomes was added into 479.75 μL of Buffer C, then 1.5 μL of 500 μM solution was added and mixed gently by shaking.

4. Preparation of 6 mM NADPH solution:
15mg NADPH was weighted, then added into 3 mL of buffer C to prepare a 6 mM NADPH solution.
5. 30 L of 1.5 μM dosing solution (see step 3) was added into the wells of the 96-well plate set at different time points (0 minutes, 5 minutes, 15 minutes, 30 minutes, 45 minutes), number of dulplicate sample is 2.
6. Preparation of a 0 minutes sample: 135 μL ACN (with internal standard) was added to the 0 minutes well, followed by 15 L of 6 mM NADPH solution.
7. The 96-well plate containing 1.5 μM dosing solution and NADPH solution were preheated in a 37°C water bath for 5 minutes.
8. The 15 L of 6 mM NADPH solution preheated was added into the wells set to 5 minutes, 15 minutes, 30 minutes, 45 minutes, then the reaction was initiated and started counting the time.
9. When the timer showed 5 minutes, 15 minutes, 30 minutes and 45 minutes, add 135 μL ACN (with internal standard) to stop the reaction.
10. The sample was vortexed for 10 min, and then centrifuged for 15 minutes on a centrifuge (Thermo Multifuge × 3R) using 5594xg.
11. 50 μL of the supernatant was taken from the centrifuged sample, transferred to a 96-well sample plate to which 100 μL of ultrapure water had already been added, mixed, and finally sent to LC-MS/MS for analysis.

**Data processing:**

1. Conculation formula: Half time ($T_{1/2}$) =0.693/K (K is the rate constant obtained by linear regression of the logarithm of the concentration as vertical coordinates and the culturing time as the abscissa)

$$\text{Intrinsic clearance rate } (CL_{int}) = (0.693 / T_{1/2}) \times (1 / (\text{liver microsome concentration } (0.5 \text{ mg / mL}))) \times \text{conversion factor (see Table 4)}$$

Table 4 the clearance rate conversion factor of intrinsic clearance rate in mouse, rat, canine, monkey and human liver microsomes *

| Species | Liver microsomal protein per gram of liver | Liver weight per kg of body weight | conversion factor** | Liver blood flow rate (mL/min/kg) |
|---|---|---|---|---|
| mouse | 45 | 87.5 | 3937.5 | 90 |

(continued)

| Species | Liver microsomal protein per gram of liver | Liver weight per kg of body weight | conversion factor** | Liver blood flow rate (mL/min/kg) |
|---|---|---|---|---|
| rat | 44.8 | 40 | 1792 | 55.2 |
| canine | 77.9 | 32 | 2492.8 | 30.9 |
| monkey | 45 | 32.5 | 1462.5 | 44 |
| human | 48.8 | 25.7 | 1254.2 | 20.7 |
| * Handbook of Essential Pharmacokinetics, Pharmacodynamics and Drug Metabolism for Industrial Scientists, Young-gil Kwon (Author)<br>** Conversion factor = (hepatic microsomal protein per gram of liver) × (hepatic weight per kilogram of body weight) | | | | |

2. According to the equation formula bellow, Excel was used to plot the compound content versus reaction time, as well as half life calculation (min)

$$Y = (Y_0 - \text{Plateau}) \times e^{(-K*X)} + \text{Plateau}$$

$Y_0$ represents the compound content at 0 minutes; Y represents the compound content; X represents the time point; K represents degration rate constant of the compound; Plateau represents the lowest possible content of the compound (defined herein as 0).

$$T_{1/2} = 0.693/K.$$

Table 5 shows the experimental results of the metabolic stability of the compounds in the liver microsomes

| Compound | HLM_$T_{1/2}$ (min) | HLM_$CL_{int}$ (mL/min/kg) | MLM_$T_{1/2}$ (min) | MLM_$CL_{int}$ (mL/min/kg) |
|---|---|---|---|---|
| (+)-JQ1 | | | | |
| ABBV-075 | 1465.60 | 1.19 | 81.11 | 67.29 |
| 4002 | 38.19 | 45.52 | 23.42 | 232.98 |
| 4007 | ∞ | 0.00 | 815.54 | 6.69 |
| 4009 | 471.41 | 3.69 | 588.07 | 9.28 |
| 4011 | 1127.52 | 1.54 | 1056.88 | 5.16 |
| 4012 | 1473.23 | 1.18 | 1568.22 | 3.48 |
| 4016 | 34.22 | 50.80 | 21.37 | 255.37 |
| 4017 | 854.27 | 2.03 | 215.28 | 25.35 |
| 4018 | 1043.26 | 1.67 | 352.12 | 15.50 |
| 4023 | 121.24 | 14.34 | 75.05 | 72.71 |
| 4026 | ∞ | 0.00 | ∞ | 0.00 |
| 4031 | ∞ | 0.00 | ∞ | 0.00 |
| 4038 | ∞ | 0.00 | ∞ | 0.00 |
| 4071 | ∞ | 0.00 | ∞ | 0.00 |
| 4072 | ∞ | 0.00 | 372.45 | 14.65 |
| 4073 | 139.87 | 12.43 | 156.12 | 34.96 |
| 4078 | 727.04 | 2.39 | 195.55 | 27.91 |

(continued)

| Compound | HLM_$T_{1/2}$ (min) | HLM_$CL_{int}$ (mL/min/kg) | MLM_$T_{1/2}$ (min) | MLM_$CL_{int}$ (mL/min/kg) |
|---|---|---|---|---|
| (+)-JQ1 | | | | |
| 4081 | 2952.57 | 0.59 | ∞ | 0.00 |

**Effect embodiment 5: pharmacolinetic study**

Determinations of the pharmacokinetic parameters of ABBV-075 (standard control), 4009, 4018, 4026 and 4031 in femal Balb/c nude mice after intravenous or oral administration.

| | |
|---|---|
| **Experimental medication** | ABBV-075, 4009, 4018, 4026 and 4031 |
| **Experimental system** | Female Balb/c nude mice, body weight 18-20g, N=6, purchased from SLAC Laboratory Animal Co. LTD. Certificate of conformity: SCXK (SH) 2013001822494. |
| **Feeding** | Unlimited water and food |
| **Mode of administration** | Intravenous: 0.5 mg/kg (5 mL/kg) injected by caudal vein; Oral: 2 mg/kg (10 mL/kg) injected by intragastric administration (N=3). |
| **Blood sample collection** | The animal was fixed, about 20 μL blood was collected behind the eyeball at the sampling time point and laced in an EDTA-2K tube. The sample was immediately diluted with 40 μL double distilled water and vortexed until well mixed. |
| **Sample storage and management** | The blood sample was stored at -70°C until being analyzed, the spared samples were discarded 1 month after while there was no need of it. |

Detailed experimental steps:

6 female Balb/c nude mice weighing 18-20 g were divided into two groups (3/group), after fixation, the intravenous group: 0.5 mg/kg (5 mL/kg) injected by caudal vein (N=3); the oral group: 2 mg/kg (10 mL/kg) by intragastic administration (N=3). Approximately 20 μL of blood was drawn behind the eyeball at the sampling point and placed in EDTA-2K tube. The sample was immediately diluted with 40 μL of double distilled water and vortexed until well mixed. Blood samples were stored at approximately -70°C and submitted to the BA group for analysis (LC/MS). 30 μL of blood sample was added with 200 μL of $H_2O$ and 30 μL of IS dexamethasone, 200 ng/mL), layered by LLE, and 1 mL of MTBW was added as an organic extraction solvent. The mixture was vortexed for 10 min and centrifuged at 10,000 rpm for 5 min. 850 μL of the supernatant was evaporated under nitrogen until desiccated. The desiccated extract was re-mixed with 150 μL of 50% methanol, vortexed for 2 min, and then analyzed by injecting 5 μL of the supernatant into LC-MS/MS.

**Cassette administration PK parameters of ABBV-075 (standard control), 4009, 4018, 4026 and 4031 in female Balb/c nude mouse by oral or intravenous administration**

| dosage (mg/kg) | Mode of administration | PK parameter (unit) | ABBV-075[a] | 4018[b] | 4009[c] | 4026[d] | 4031[e] |
|---|---|---|---|---|---|---|---|
| 0.5 | IV N=3 | CL (L/hr/kg) | 0.986 | 2.51 | 0.855 | 0.45 | 0.651 |
| | | $V_{ss}$ (L/kg) | 3.06 | 1.51 | 1.38 | 1.43 | 2.34 |
| | | Terminal $t_{1/2}$ (hr) | 2.80 | 0.685 | 1.69 | 2.85 | 2.72 |
| | | $AUC_{last}$ (hr*ng/mL) | 457 | 199 | 575 | 1101 | 729 |
| | | $AUC_{INF}$ (hr*ng/mL) | 510 | 201 | 590 | 1141 | 771 |
| | | $MRT_{INF}$(hr) | 3.10 | 0.590 | 1.61 | 3.21 | 3.5 |

(continued)

| Cassette administration PK parameters of ABBV-075 (standard control), 4009, 4018, 4026 and 4031 in female Balb/c nude mouse by oral or intravenous administration | | | | | | | |
|---|---|---|---|---|---|---|---|
| dosage (mg/kg) | Mode of administration | PK parameter (unit) | ABBV-075[a] | 4018[b] | 4009[c] | 4026[d] | 4031[e] |
| 2 | PO N=3 | $T_{max}$ (hr) | 1.00 | 0.667 | 0.667 | 0.5 | 0.5 |
| | | $C_{max}$ (hr*ng/mL) | 223 | 41.9 | 742 | 1082 | 1009 |
| | | Terminal $t_{1/2}$ (hr) | 4.56 | 2.18 | 4.21 | 4.35 | 3.84 |
| | | $AUC_{last}$ (hr*ng/mL) | 1144 | 89.7 | 2253 | 3744 | 3299 |
| | | $AUC_{INF}$ (hr*ng/mL) | 1685 | 98.1 | 2352 | 4051 | 3329 |
| | | F (%) | 62.5 | 12.2 | 99.7 | 89.5 | 108 |
| a The 4 cassette experimental results of the standard control was stable, with smaller error; b, c, d, e are 4 individual cassete-PK experiments | | | | | | | |

1. No abnormal clinical manifestation was observed in the whole animal experiment,
2. Intravenous/oral administration formula: 5% DMSO, 95% (10% HP-β-CD in water)
3. PK calculation parameter used non-compartment model, WinNonlin 6.4 software calculation

**Effect embodiment 6: drug metabolism *in vivo* and pharmacodynamics of compound 4009 against the human leukemia cell MV-4-11 subcutaneous xenograft tumor model**

**6.1. Experimental design**

**PKPD experimental animal grouping and administration protocol**

| group | Number of mouse | treatment | Dosage (mg/kg) | Mode of administration | Time point |
|---|---|---|---|---|---|
| 1 | 3 | blank | -- | Intragastric | - |
| 2 | 18 | 4009 | 15, single dose | intragastric | 1, 2, 4, 6, 8, 24h |
| Note: 1) dosing volume: calculation of administration volume for each animal according to the weight of the mouse, by 10 μL/g.<br>2) Tumor and plasma were collected at the indicated time point after administration for pharmacokinetics and pharmacodynamics study. | | | | | |

**6.2. Experiment material**

**6.2.1. Experiment animal**

Species: mouse; strain: BALB/c nude mice
Weekly age and weight at the start of dosing: 6-8 weeks old, weight 18-20 g
Source: Shanghai Lingchang Biotechnology Co., Ltd.
Animal Production License No.: SCXK (Shanghai) 2013-0018

### 6.2.2 Experimental animal feeding conditions

The experiment was started 7 days after the animals arrived in the experimental environment. Animals were housed in an IVC (independent air supply system) cage in an SPF animal room. Each cage animal information card indicates the number of animals in the cage, gender, strain, date of receipt, dosing schedule, experiment number, group, and start date of the experiment. All cages, litter and drinking water are sterilized prior to use. Cages, feeds and drinking water were changed twice a week. Feeding environment and lighting conditions are as follows

√ Temperature: 20-26°C
√ Humidity: 40-70%
√ photo period: 12 hours of light, 12 hours of no light (lights at 8 am -8 pm)

### 6.2.3. Test sample information

**Ingredient:** HP-β-CD

Manufacturer: Shanghai Xibao Biotechnology Co., Ltd.
Lot: OP606A
Packing: 100g/bottle
Storage conditions: RT, sealed.

**Ingredient:** dimethyl sulfoxide (DMSO) (DIMETHYL SULPHOXIDE)

Manufacturer: Sigma
Lot: RNBD6799
Shelf life: 03/2017
Packing: 100mL / bottle
Storage conditions: RT, protected from light, sealed.

### Experimental methods and steps

### Cell culture

MV-4-11 (purchased from ATCC, CRL-9591) cells were single-layer-cultured *in vitro*, culture condition is IMDM medium supplemented with 10% heat-inactivated fetal bovine serum and 1% penicillin-streptomycin double antibody, at 37°C, 5% CO2. According to the growth of the cells, the treatment was passaged twice a week. When the cells are in the exponential growth phase, the cells are collected, counted, and inoculated.

### Tumor cell inoculation and group administration

Experimental protocol: 0.2 mL of cell suspension containing about 5 x 10^6 MV-4-11 cells (cell suspended in IMDM medium and added with 50% Matrigel) was inoculated subcutaneously into the right back of each mouse. Group administration was initiated when the average tumor volume reached approximately 257.89 $mm^3$ ($day^{15}$ post inoculation). Grouping method: animals were weighed before dosing and tumor volume was measured. Grouping designed based on tumor volume.

### 6.3.3. Preparation of test substance

**Preparation methods of drugs**

| compound | package | preparation | Concentration (mg/mL) | storage |
|---|---|---|---|---|
| comparative 5%DMSO, 95% of 10% β cyclodextrin (HP-β-CD) | HP-β-CD (100 g/ tube) | 1. 25 g of β-cyclodextrin was weighed in a 250 mL tube, 150 mL of deionized water was added, shaked and sonicated for 1-2 minutes to obtain a clear solution. | -- | 4°C |
| | | 2. deionized water was added until 250 mL | | |
| 4009(molecular mass=424.49) | 327mg/ tube | 1. 8.1 mg of the compound was weighed into a 5 mL tube. | 1.5 | |
| | | 2. 270 μL of dimethyl sulfoxide solution was added to obtain a mother liquor of 30 mg/mL. | | |
| | | 3. 5.13 mL of 10% β cyclodextrin was added then vortexed to obtain a clear solution. | | |
| Note: (1) dosing volume: the dosing volume is calculated according to the body weight of the mouse, by 10 μL/g. | | | | |

### 6.3.4. Daily observation of experimental animals

The preparation and any revision of this experimental protocol are carried out in accordance with the evaluation criteria of the "Institute for Animal Care and Use (IACUC)" of Shanghai Chempartner Chemical Research Co., Ltd. The use and welfare of laboratory animals are carried out in accordance with the provisions of the Association for Assessment and Accreditation of Laboratory Animal Care (AAALAC). Animals are monitored daily for health and death. Routine examinations include observations of the effects of the test substance and the drug on the daily behavior of the animal such as behavioral activities, intake of water and feeds, weight change (three weights measured weekly), appearance signs or other anormal circumstance. The number of animal deaths and side effects in the group were recorded based on the number of animals in each group.

### 6.3.5. Sample collection

21 mice were divided into 2 groups (6 time points), 3 were blank control group, and 18 were drug-administered groups. After administration, mice were euthanized at a given time point, and plasma and tumor were collected. The tumor was divided into two parts, one for pharmacokinetic analysis and one for pharmacodynamic analysis. Plasma and tumors were immediately frozen on dry ice and then transferred to a -80°C cryostat (additional 5 blank tumors and 5 blank plasmas as blank controls).

### 6.3.6. Sample collection time point.

| **Time point (hrs)** | | 1 | 2 | 4 | 6 | 8 | 24 |
|---|---|---|---|---|---|---|---|
| **Animal number (#4~#21)** | **4009, 15 mg/kg** | #4-#6 | #7-#9 | #10-#12 | #13-#15 | #16-#18 | #19-#21 |

Plasma collection: according to the set time point, the collected blood samples were anticoagulated with EDTA-K2 and stored on wet ice before centrifugation. The sample was centrifuged within 15 minutes after sampling (about 6,000 rpm at 4°C for 5 minutes) to obtain plasma, the plasma was pipetted into a 1.5 mL polypropylene tube, and the tube was placed in a labeled sample box, and was stored at 80°C.

Tumor collection: according to the above time points, after the mice were euthanized, the tumors were taken out, divided into two parts, one for pharmacological analysis and the other for pharmacodynamic analysis. The relevant samples were stored at -80°C refrigerator before testing.

**6.3.7. PD experimental method** (detection of MYC mRNA expression level in MV-4-11 xenografts tumor by RT-qPCR method)

RNA extraction: total RNA from tumor samples was extracted using the NucleoSpin® RNA Plus kit. There were 3 samples in each group, 21 samples in total, including the control group and the drug-administered group at different time points.

Preparation of cDNA: The concentration of RNA (NanaDrop2000) was determined, 2 μg RNA was used as a template for reverse transcription, and cDNA preparation (RT-PCR) was performed using a High Capacity cDNA Reverse Transcription Kit.

Quantitative amplification of the target gene: Real-time qPCR amplification was performed using TaqMan fluorescent probe and cDNA as a template. GAPDH was used as an internal reference gene. The reaction system was separately added to a 384-well plate, and each sample of the target gene and the comparative gene was measured for three replicate wells, and the reaction was run on a prepared 384-well plate instrument (ABI ViiA7).

**6.4. Experimental result**

21 tubes of plasma and 21 tubes of tumor (1 hour, 2 hours, 4 hours, 6 hours, 8 hours and 24 hours after single administration, collected at 6 time points) were sent for pharamcokinetic analysis, 21 tubes of tumor (1 hour, 2 hours, 4 hours, 6 hours, 8 hours and 24 hours after single administration, collected at 6 time points) were sent for pharmacodynamics analysis.

**6.4.1. Pharmacokinetic results**

The results of the detection of the drug 4009 in tumor are shown in Fig. 1. The detection result of the drug 4009 in the plasma is shown in Fig. 2, and Fig. 3 shows the relationship between the expression level of the MYC mRNA in the tumor and the sampling time. The comparison between Fig. 1 and Fig. 3 shows that the inhibition of the expression level of MYC mRNA in the tumor has a good correlation with the concentration of the drug in the tumor.

**Effect embodiment 7:** anti-tumor efficacy *in vivo* of compound 4009 against MV-4-11 subcutaneous xenograft model compared to positive controls ABBV-075 and GSK762

**7.1. Experimental design**

**Table 7-0 *In vivo* pharmacodynamics experiment animal grouping and administration protocol**

| group | Number of mouse | treatment | Dosage (mg/kg) | Mode of administration | Time point |
|---|---|---|---|---|---|
| 1 | 12 | blank | -- | Intragastric | QD*14 |
| 2 | 6 | ABBV-075 | 3 | Intragastric | QD*14 |
| 3 | 6 | GSK762 | 15 | Intragastric | QD*14 |
| 4 | 6 | 4009 | 10 | Intragastric | QD*14 |
| Note: dosing volume: calculation of administration volume for each animal according to the weight of the mouse, by 10 μL/g. | | | | | |

**7.2. Experimental material**

**7.2.1. Experiment animals, feeding conditions and parts of the test sample information see embodiment 6.2**

**7.2.2. Test sample information**

**Ingredient:** Solutol HS 15

Manufacturer: BASF
Lot: 25540356PO
Received date: 2015-8-18
Packing: 500g / bottle
Storage conditions: RT, sealed.

**Ingredient:** VE (D-a-Tocopherol polyethylene glycol 1000 succinate)

Manufacturer: Sigma
Lot: BCBR0150V
Packing: 25g / bottle
Storage conditions: 2-8°C, sealed.

### 7.3. Experiment methods and procedure

#### 7.3.1. Cell culture

MV-4-11 (purchased from ATCC, CRL-9591) cells were single-layer-cultured *in vitro,* culture condition is IMDM medium supplemented with 10% heat-inactivated fetal bovine serum and 1% penicillin-streptomycin double antibody, at 37°C, 5% $CO_2$. According to the growth of the cells, the treatment was passaged twice a week. When the cells are in the exponential growth phase, the cells are collected, counted, and inoculated.

#### 7.3.2. Tumor cell inoculation and group administration

Experimental protocol: 0.2 mL of cell suspension containing about 5 x 10^6 MV-4-11 cells (cell suspended in IMDM medium and added with 50% Matrigel) was inoculated subcutaneously into the right back of each mouse. Group administration was initiated when the average tumor volume reached approximately 136.94 $mm^3$ (day 15 post inoculation). Grouping method: Animals were weighed before dosing and tumor volume was measured. Grouping designed based on tumor volume.

#### 7.3.3. Preparation of test substance

**Table 7-1 Preparation methods of drugs**

| compound | package | Preparation | Concentration (mg/m L) | storage |
|---|---|---|---|---|
| Blank control group 5%DMSO, 5%Solutol HS 15, 90%(10%VE-TPGS) | VE-TPGS (25 g/tube) | 1. VE-TPGS was heated in a 60°C water bath and dissolved, 25 mL VE-TPGS was added to 225 mL deionized water. Stirred for 20-30 minutes to obtain a 10% VE-TPGS clear solution. | -- | |
| | | 2. 2 mL of DMSO, 2 mL of Solutol HS 15 and 36 mL of 10% VE-TPGS were added to obtain 5% DMSO, 5% Solutol HS 15, 90% (10% VE-TPGS) as a control solution. | | |

(continued)

| compound | package | Preparation | Concentration (mg/m L) | storage |
|---|---|---|---|---|
| ABBV-075 (molecular weight =459.47) | 10.7 mg/ tube | 1. Weigh 10.7 mg of the compound into a 5 mL tube. | 0.3 | 4°C |
| | | 2. 6.783 mL of DMSO was added to obtain 6 mg/mL mother liquor, and dispensed in an average of 14 tubes, 90 μL/tube stored in a -20°C refrigerator. | | |
| | | 3. A tube of mother liquor was taken and added 90 μL of Solutol HS 15, vortexed for 1-2 minutes to obtain a clear solution, then added 1.62 mL of 10% VE-TPGS, and vortexed to obtain the final solution. | | |
| GSK-762 (molecular mass=423.9) | 37.8 mg/ tube | 1. 37.8 mg of the compound was weighted in a 5 mL tube. | 1.5 | |
| | | 2.1.26 mL of DMSO was added to obtain a 30 mg/mL mother liquor, and dispensed in average of 14 tubes, 90 μL/tube, and stored in a -20°C refrigerator. | | |
| | | 3. a tube of mother liquor was taken then added 90 μL of Solutol HS 15, vortexed for 1-2 minutes to obtain a clear solution, then added 1.62 mL of 10% VE-TPGS, and vortexed to obtain the final solution. | | |
| 4009(MW=42 4.49) | | 1. 12.6 mg of the compound was weighted in a 5 mL tube. | 1 | |
| | | 2. 0.63 mL of DMSO was added to obtain a 20 mg/mL mother liquor with an average of 7 tubes, 90 μL/tube, and stored in a -20°C refrigerator. | | |
| | | 3. a tube of mother liquor was taken and added 90 μL of Solutol HS 15, vortexed for 1-2 minutes then obtained a clear solution and added 1.62 mL of 10% VE-TPGS then vortexed to obtain the final solution. | | |
| Note: dosing volume: the dosing volume is calculated according to the body weight of the mouse, by 10 μL/g. | | | | |

### 7.3.4. Daily observation of experimental animals

The preparation and any revision of this experimental protocol are carried out in accordance with the evaluation criteria of the "Institute for Animal Care and Use (IACUC)" of Shanghai Chempartner Chemical Research Co., Ltd. The use and welfare of laboratory animals are carried out in accordance with the provisions of the Association for Assessment and Accreditation of Laboratory Animal Care (AAALAC). Animals are monitored daily for health and death. Routine examinations include observations of the effects of the test substance and the drug on the daily behavior of the animal such as behavioral activities, intake of water and feeds, weight change (three weights measured weekly), appearance signs or other anormal circumstance. The number of animal deaths and side effects in the group were recorded based on the number of animals in each group.

### 7.3.5. Experimental indicator

The experimental indicator is to investigate whether tumor growth can be inhibited, delayed or cured. Tumor

diameters were measured with vernier calipers three times a week. The formula for calculating tumor volume is: V = 0.5 × a × b2, a and b represent the long and short diameters of the tumor, respectively. The antitumor effect of the compound was evaluated by T/C (%). T/C%=$T_{RTV}$/$C_{RTV}$ ×100% ($T_{RTV}$: treatment group RTV; $C_{RTV}$: negative comparative group RTV). The relative tumor volume RTV is calculated as RTV = $V_t$ / $V_1$. Where $V_1$ is the measured tumor volume at the time of group administration (i.e., Day 1), and $V_t$ is the tumor volume at each measurement. The percentage value of T/C (%) reflects the tumor growth inhibition rate, which the drug is considered to be effective according to the guidelines of the anti-tumor drugs of the drug testing center, if T/C% ≤ 40%. Tumor volume was evaluated by TGI%, tumor weight inhibition rate (TGI) (1-($TV_{Treatment/Dx}$-$TV_{Treatment/D1}$) / ($TV_{ControL/Dx}$-$TV_{Contro/D1}$)) x 100%, $TV_{Control}$: control tumor volume, $TV_{Treatment}$: tumor volume in the treatment group. According to the guidelines of the drug trial center for anti-tumor drugs, TGI ≥ 58%, the drug is considered effective.

### 7.3.6. Sample collection

15 days after administration, 4009 10mg/kg group number 4-6 mouse was euthanized 3 hours after admnistraion, collect tumor and plasma samples of each mouse from the administrated group; plasma and tumors were placed on dry ice, for detection of the drug content in tumors and plasma. The other experiment mice were euthanized on the 15th day 24 hours after administration.

### 7.3.7. Statistical analysis

The tumor volume and tumor size were analysed by statistical software. GraphPad Prism software was used for analyzation, $p < 0.05$ is considered to have significant difference, p<0.01 is considered to have extremely significant difference.

Two-way Anova Bonferroni post-test is used to compare the tumor volume difference between the control group and the treatment group.

### 7.4. Experimental result

### 7.4.1. Body weight

The body weight changes of the tumor-bearing mice in each group are shown in Fig. 4, and the body weight of the mice are summarized in Table 7-2.

**Table 7-2 body weight of each group of tumor-bearing mice ( $\bar{x}$ ± SEM)**

| group | Number of animal | medicine | Dosage (mg/kg) | Average body weight (**g**) | | Average RCBW* (%) |
|---|---|---|---|---|---|---|
| | | | | Treatment start Day 1 | Treatment end Day 15 | Treatment end Day 15 |
| 1 | 12 | blank | -- | 21.56±0.35 | 22.73±0.33 | 5.51 |
| 2 | 6 | ABBV-075 | 3 | 21.36±0.21 | 21.95±0.36 | 2.72 |
| 3 | 6 | GSK762 | 15 | 20.82±0.40 | 21.86±0.38 | 5.07 |
| 4 | 6 | 4009 | 10 | 21.26±0.38 | 21.88±0.73 | 2.82 |

Note: * Relative body weight change (RCBW) reflects the effect of drug on the body weight, the time of the first dose is defined as Day 1, RCBW (%) is calculated based on the body weight of the group administered on the first day. Calculation formula: RCBW (%) = (weight of the day of administration-body weight of the first day of administration) / body weight of the first day of administration x 100%.

### 7.4.2. Tumor volume

The effect of each treatment group on the tumor volume of MV-4-11 tumor-bearing mice is shown in Fig 5 and Fig 6.

### 7.4.3. Inhibition on tumor growth

The inhibition of tumor growth on MV-4-11 tumor-bearing mice are shown in Table 7-3, Table 7-4 and Table 7-5.

**Table 7-1 inhibition on the tumor growth in each group of tumor-bearing mice**

| group | Number of animal | Dosage (mg/kg) | Tumor volume **TGI (%)** * | | | | | |
|---|---|---|---|---|---|---|---|---|
| | | | $4^{th}$ day | $6^{th}$ day | $8^{th}$ day | $11^{th}$ day | $13^{th}$ day | **$15^{th}$ day** |
| blank | 1 | - | - | - | - | - | - | - |
| ABBV-075 | 2 | 3 | 97.12 | 69.45 | 73.8 | 72.41 | 75.81 | 75.56 |
| GSK76 2 | 3 | 15 | 88.15 | 65.11 | 60.5 | 51.86 | 51.9 | 46.58 |
| 4009 | 4 | 10 | 81.27 | 75.63 | 79.64 | 83.17 | 81.77 | 81.16 |

Note: *inhibition rate on the tumor volume (TGI)%=(1-($TV_{treatement\ volume/Dx}$ -$TV_{treatement\ volume/D1}$)/($TV_{control\ group/Dx}$-$TV_{comparative\ group/D1}$)) × 100 %, $TV_{control\ group}$: volume of tumor in the comparative group, $TV_{Treatment}$: volume of tumor in the treatement group. TGI≥58%, the compound is effective.

**Table 7-2 effect of the compound on the tumor volume in MV-4-11 tumor**

| Group | Number of animals | Medicine | Dosage (mg/kg) | **Average tumor volume ($mm^3$)** | | **T/C% |
|---|---|---|---|---|---|---|
| | | | | Treatement starts | Treatement ends | |
| | | | | $1^{st}$ day | $15^{th}$ day | |
| 1 | 12 | blank | - | 139.70±12.27 | 1036.78±122.54 | - |
| 2 | 6 | ABBV-075 | 3 | 136.45±14.05 | 346.72±68.96 | 34.24 |
| 3 | 6 | GSK762 | 15 | 137.82±18.64 | 617.02±131.69 | 60.33 |
| 4 | 6 | 4009 | 10 | 136.78±14.97 | 305.80±81.48 | 30.13 |

Note: ** T/C%=$T_{RTV}/C_{RTV}$ ×100% ($T_{RTV}$: treatment group RTV; $C_{RTV}$: negative comparative group RTV). Where $V_1$ is the measured tumor volume at the time of group administration (i.e., Day 1), and $V_t$ is the tumor volume at each measurement.

**Table 7-3 the tumor volume difference between the control group and each of the administrated group analysed by statistical software**

| The tumor volume difference between the comparative group and each of the administrated group analysed by statistical software | | | | |
|---|---|---|---|---|
| Days after administration | Blank group | ABBV-075 3 mg/kg | GSK762 15 mg/kg | 4009 10 mg/kg |
| 1 | - | P > 0.05 | P > 0.05 | P > 0.05 |
| 4 | - | P > 0.05 | P > 0.05 | P > 0.05 |
| 6 | - | P > 0.05 | P > 0.05 | P > 0.05 |
| 8 | - | P > 0.05 | P > 0.05 | P > 0.05 |
| 11 | - | P<0.001 (***) | P<0.05 (*) | P<0.001 (***) |
| 13 | - | P<0.001 (***) | P<0.01 (**) | P<0.001 (***) |
| 15 | - | P<0.001 (***) | P<0.001 (***) | P<0.001 (***) |

Note: $p < 0.05$ is considered to have significant difference, $p<0.01$ is considered to have extremely significant difference.

#### 7.4.4 Tumor weight

The inhibitiory effect of compounds on the tumor growth in MV-4-11 tumor-bearing mice are shown in Table 7-6 and Fig 7.

**Table 7-4 The inhibitiory effect of compounds on the tumor growth in MV-4-11 tumor-hearing mice**

| Average tumor weight (g) | |
|---|---|
| Treatement group | $15^{th}$ day of treatement |
| Blank group | 0.901±0.11 |
| ABBV-075 ,3mg/kg | 0.314±0.05 |
| GSK762, 15 mg/kg | 0.522±0.11 |
| 4009, 10 mg/kg | 0.276±0.08 |

### 7.5. Conclusion and discussion of the experimental results

In the present experiment, we evaluated the *in vivo* anti-tumor efficacy of test compound 4009 in a MV-4-11 acute myeloid leukemia cell xenograft tumor model.

The changes in body weight of mice in each group after administration are shown in Fig. 4. During the entire administration period, the mice in the 4009 dose groups did not decrease in body weight and were in good condition, and the mice were tolerant to the dose of the test compound.

The tumor volume changes of the animals in each group after administration are shown in Fig. 5 and Fig. 6. Tumor volume TGI (%) values, T/C (%) and statistical evaluation analysis, see Table 7-3 and Table 7-4 and Table 7-5.

On the $15^{th}$ day after administration, the tumor volumes of the blank control group, ABBV-075 3mg/kg, GSK762 15mg/kg, and 4009 10mg/kg were respectively 1036.78 $mm^3$, 346.72 $mm^3$, 617.02 $mm^3$, and 305.80 $mm^3$.

During the experimental administration, the tumor volume TGI (%) analysis, ABBV-075 3 mg/kg and 4009 10 mg/kg group from the $4^{th}$ day of administration to the $15^{th}$ day of administration, TGI (%) were greater than 58%, GSK762 15 mg/kg from $4^{th}$ of administration to $8^{th}$ after administration TGI (%) was greater than 58%, and TGI (%) was less than 58% from $11^{th}$ to $15^{th}$ of administration.

In summary, ABBV-075 3 mg/kg and 4009 10 mg/kg group under the experimental conditions, has significant anti-tumor effect on the MV-4-11 cell xenograft tumor mouse model. GSK762 15 mg/kg under this experimental condition, has no significant anti-tumor effect on the mouse model of MV-4-11 cell xenograft tumor.

Althought the above have described the detailed embodiments of the present invention, the man skilled in the art should appreciate that, these are only for examples and descriptions, modifications and changes could be made based on the embodiments in the premise of not departing from the principles and substance of the present invention. Accordingly, the scope of the invention is defined by the appended claims.

## Claims

1. A nitrogenous macrocyclic compound represented by formula **III-0**, a tautomer thereof, an optical isomer thereof, a hydrate thereof, a solvate thereof, a pharmaceutically acceptable salt thereof or a prodrug thereof;

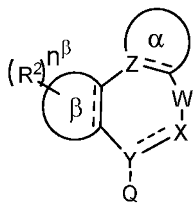

**III-0** ;

wherein, W is $-C(R^1)(R^3)-$ or $-N(R^4)-$;

each of $R^1$, $R^3$, $R^4$ is independently -H, $C_1$-$C_5$ alkyl, $C_3$-$C_7$ cycloalkyl, $C_1$-$C_5$ alkoxyl, $C_1$-$C_5$ haloalkyl, $C_1$-$C_5$ alkyl substituted by one or more of $R^{R15}$ , halogen, $-(CH_2)n_0C(=O)N(R^{R1})(R^{R2})$, $-NH(CH_2)n_1C(=O)N(R^{R3})(R^{R4})$,

$-(CH_2)n_2N(R^{R5})(R^{R6})$, $-(CH_2)n_3OC(=O)N(R^{R7})(R^{R8})$, $-(CH_2)n_4NC(=O)R^{R9}$, $-(CH_2)n_5NHC(=O)OR^{R10}$, $-(CH_2)n_6NHS(=O)_2R^{R11}$ or $-(CH_2)n_7S(=O)_2N(R^{R12})(R^{R13})$;
each $R^{15}$ is independently $C_1$-$C_5$ alkoxyl, -COOH or -OH;
all of the $R^{R1}$, $R^{R2}$, $R^{R3}$, $R^{R4}$, $R^{R5}$, $R^{R6}$, $R^{R7}$, $R^{R8}$, $R^{R9}$, $RR^{10}$, $R^{R11}$, $R^{R12}$ and $R^{R13}$ are independently -H, $C_1$-$C_5$ alkyl, $C_1$-$C_5$ haloalkyl, $-(CH_2)m_1CN$, $-C(CH_3)_2CN$, 3-7 membered cycloalkyl group, or , $C_6$-$C_{10}$ aryl substituted by one or more of $R^{R14}$; each $R^{R14}$ is independently -H, $C_1$-$C_5$ alkyl, $C_1$-$C_5$ alkoxyl, $C_1$-$C_5$ haloalkyl, halogen, -OH, -CN or $-NH_2$;
each of $n_0$, $n_1$, $n_2$, $n_3$, $n_4$, $n_5$, $n_6$, $n_7$ and $m_1$ is independently 0, 1 2 or 3;
or, $R^1$, $R^3$ and the carbon atom they are attached to together form a $C_3$-$C_7$ cycloalkyl;

"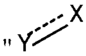"

represents the bond between X and Y is a single or double bond;
X is $-CH_2-$, =N-, -NH-, -O-, $-S(=O)_2-$ or -C(=O)-;
Y is

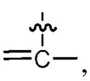, 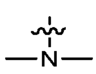 or 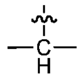;

and at least one of X and Y is a nitrogen atom;
Q is a phenyl substituted by one or more of $R^{Q1}$, 3-6 membered cyclohydrocarbyl-$(CH_2)_{nQ}$-substituted by one or more of $R^{Q2}$, 3-6 membered heterocyclohydrocarbyl-$(CH_2)_{mQ}$- substituted by one or more of $R^{Q3}$, or, 5-6 membered heteroaryl substituted by one or more of $R^{Q4}$;
each of $n_Q$ and $m_Q$ is independently 0, 1 or 2;
each of the all $R^{Q1}$, $R^{Q2}$, $R^{Q3}$ and $R^{Q4}$ is independently -H, -OH, -CN, halogen, halogenated or unsubstituted $C_1$-$C_5$ alkyl, $C_1$-$C_5$ alkoxyl, or $C_3$-$C_7$ cycloalkyl;

the "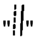" in

"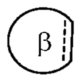"

refers to the bond β ring and 7-mebered ring fused by is a single or double bond;
β ring is 5-6 membered aromatic ring, or, "5-6 membered heteroaromatic ring containing 1-3 heteroatom selected from the group consisting of N, O and S";
$n^\beta$ is 0, 1, 2, 3 or 4;
each of the all of $R^2$ is independently halogenated or unsubstituted $C_1$-$C_5$ alkyl, $C_3$-$C_7$ cycloalkyl, hydroxyl, halogen, $C_1$-$C_5$ alkoxyl, $-N(R^{2a})(R^{2b})$, $-NHC(R^{2C})H_2$, $-(CH_2)n^{\beta2}N(R^{2k})S(=O)_2(R^{2d})$, $-(CH_2)n^{\beta2}N(R^{2k})S(=O)_2NH(R^{2e})$, $-(CH_2)n^{\beta2}-S(=O)_2N(R^{2f})(R^{2g})$, $-(CR^{2x}R^{2y})-S(=O)_2N(R^{2f})(R^{2g})$, $-(CH_2)n^{\beta2}-S(=O)(R^{2i})$, $-(CH_2)n^{\beta2}NHC(=O)NH(R^{2e})$, $-(CH_2)n^{\beta2}NHC(=O)(R^{2e})$, $-(CH_2)n^{\beta2}OC(=O)NH(R^{2e})$, $-(CH_2)n^{\beta2}NHC(=O)O(R^{2e})$, $-(CH_2)n^{\beta2}-C(=O)NH(R^{2e})$, $-S(=O)_2N(R^{2h})$, $-C_1$-$C_6$ alkyl-$S(=O)_2N(R^2)$, or, "a 5-6 membered heteroaryl substituted by one or more of $R^{2j}$";
each of the all $n^{\beta2}$ is independently 0, 1, 2, 3 or 4;
wherein, each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently -H, $C_1$-$C_6$ alkyl, $C_1$-$C_3$ haloalkyl, 3-7 membered cycloalkyl, $-N(CH_3)_2$, or, 5-6 membered nitrogen-containing heteroaryl; each $R^{2j}$ is independently -H, $C_1$-$C_3$ alkyl, or halogen; each of $R^{2x}$ and $R^{2y}$ is independently $C_1$-$C_6$ alkyl;

"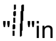"in

"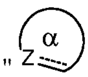"

refers to the bond α ring and 7-membered ring fused by is a single or double bond;
Z is

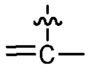 or 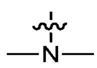;

α ring is a 5-10 membered heteroaromatic ring substituted by one or more of $R^{\alpha}$;
each of the all $R^{\alpha}$ is independently -H, -CN, $C_1$-$C_5$ alkyl, $C_3$-$C_7$ cycloalkyl, $C_1$-$C_5$ alkoxyl, $C_1$-$C_5$ haloalkyl, halogen, -C(=O)N($R^{\alpha1}$)($R^{\alpha2}$), or, -N($R^{\alpha1}$)($R^{\alpha2}$);
wherein, each of the all $R^{\alpha1}$, $R^{\alpha2}$, $R^{\alpha4}$ and $R^{\alpha4}$ is independently -H, $C_1$-$C_5$ alkyl, or $C_1$-$C_5$ haloalkyl.

**2.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 1, wherein, when each of $R^1$, $R^3$ and $R^4$ is independently $C_1$-$C_5$ alkyl, the $C_1$-$C_5$ alkyl is $C_1$-$C_3$ alkyl;
and/or, when each of $R^1$, $R^3$ and $R^4$ is independently $C_3$-$C_7$ cycloalkyl, the $C_3$-$C_7$ cycloalkyl is cyclopropyl;
and/or, when each of $R^1$, $R^3$ and $R^4$ is independently $C_1$-$C_5$ alkoxyl, the $C_1$-$C_5$ alkoxyl is $C_1$-$C_3$ alkoxyl;
and/or, when each of $R^1$, $R^3$ and $R^4$ is independently $C_1$-$C_5$ haloalkyl, the number of the halogen is one or more, when more than one halogen exists, the halogens are the same or different;
and/or, when each of $R^1$, $R^3$ and $R^4$ is independtly $C_1$-$C_5$ haloalkyl, the halogen is fluorine, chlorine, bromine or iodine;
and/or, when each of $R^1$, $R^3$ and $R^4$ is independently $C_1$-$C_5$ haloalkyl, the $C_1$-$C_5$ haloalkyl is $C_1$-$C_3$ haloalkyl;
and/or, when each of $R^1$, $R^3$ and $R^4$ is independently $C_1$-$C_5$ alkyl substituted by one or more of $R^{R15}$, the more refers to 2, 3 or 4;
and/or, when each of $R^1$, $R^3$ and $R^4$ is independently $C_1$-$C_5$ alkyl substituted by one or more of $R^{R15}$, the "$C_1$-$C_5$ alkyl" is methyl or ethyl;
and/or, when each of $R^1$, $R^3$ and $R^4$ is independently halogen, the halogen is fluorine, chlorine, bromine or iodine;
and/or, when each of $R^{15}$ is independently $C_1$-$C_5$ alkoxyl, the $C_1$-$C_5$ alkoxyl is each independently methoxyl;
and/or, when each of $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is indenpendently $C_1$-$C_5$ alkyl, the $C_1$-$C_5$ alkyl is $C_1$-$C_3$ alkyl;
and/or, when each of $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is indenpendently 3-7 membered cycloalkyl, the 3-7 membered cycloalkyl is cycloproyl;
and/or, when each of $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is indenpendently $C_6$-$C_{10}$ aryl substituted by one or more of $R^{R14}$, the more refers to 2, 3 or 4;
and/or, when each of $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is indenpendently $C_6$-$C_{10}$ aryl substituted by one or more of $R^{R14}$, the "$C_6$-$C_{10}$ aryl" is phenyl;
and/or, when each $R^{R14}$ is independently $C_1$-$C_5$ alkyl, the $C_1$-$C_5$ alkyl is $C_1$-$C_3$ alkyl;
and/or, when $R^1$, $R^3$ and the carbon atom they are attached to together form a $C_3$-$C_7$ cylcloalkyl, the $C_3$-$C_7$ cylcloalkyl is cyclopropyl;
and/or, when Q is phenyl substituted by one or more of $R^{Q1}$, the more is 2, 3 or 4;
and/or, when Q is phenyl substituted by one or more of $R^{Q1}$, each $R^{Q1}$ is independently attached to the meta, para or ortho position of phenyl;
and/or, when Q is 3-6 membered cyclohydrocarbyl-$(CH_2)_{nQ}$- substituted by one or more of $R^{Q2}$, the more is 2, 3 or 4;
and/or, when Q is 3-6 membered cyclohydrocarbyl-$(CH_2)_{nQ}$- substituted by one or more of $R^{Q2}$, the 3-6 membered cyclohydrocarbyl is 3-6 membered cycloalkyl;
and/or, when Q is 3-6 membered heterocyclohydrocarbyl-$(CH_2)_{mQ}$- substituted by one or more of $R^{Q3}$, the more is 2, 3 or 4;
and/or, when Q is 3-6 membered heterocyclohydrocarbyl-$(CH_2)_{mQ}$- substituted by one or more of $R^{Q3}$, the heteroatom in the heterocyclohydrocarbyl is N and/or O;
and/or, when Q is 3-6 membered heterocyclohydrocarbyl-$(CH_2)_{mQ}$- substituted by one or more of $R^{Q3}$, the number of the heteroatom in the heterocyclohydrocarbyl is 1-3;
and/or, when Q is 3-6 membered heterocyclohydrocarbyl-$(CH_2)_{mQ}$- substituted by one or more of $R^{Q3}$, the 3-6 membered heterocyclohydrocarbyl is 5-6 membered heteroaryl or 3-6 membered heterocycloalkyl;
and/or, when $R^{Q1}$, $R^{Q2}$, $R^{Q3}$ and $R^{Q4}$ are each independently halogen, the halogen is fluorine or chlorine;
and/or, when $R^{Q1}$, $R^{Q2}$, $R^{Q3}$ and $R^{Q4}$ are each independently halogenated or unsubstituted $C_1$-$C_5$ alkyl, the number of the halogen is one or more, when more than one halogen exists, the halogens may be the same or different;
and/or, when $R^{Q1}$, $R^{Q2}$, $R^{Q3}$ and $R^{Q4}$ are each independently halogenated or unsubstituted $C_1$-$C_5$ alkyl, the "halogen" is independently fluorine, chlorine or bromine;
and/or, when $R^{Q1}$, $R^{Q2}$, $R^{Q3}$ and $R^{Q4}$ are each independently halogenated or unsubstituted $C_1$-$C_5$ alkyl, the "$C_1$-$C_5$ alkyl" is independently $C_1$-$C_3$ alkyl;
and/or, when $R^{Q1}$, $R^{Q2}$, $R^{Q3}$ and $R^{Q4}$ are each independently $C_1$-$C_5$ alkoxyl, the $C_1$-$C_5$ alkoxyl is $C_1$-$C_3$ alkoxyl;
and/or, when $R^{Q1}$, $R^{Q2}$, $R^{Q3}$ and $R^{Q4}$ are each independently $C_3$-$C_7$ cylcloalkyl, the $C_3$-$C_7$ cylcloalkyl is cyclopropyl;

and/or, when β ring is 5-6 membered aromatic ring, the 5-6 membered aromatic ring is benzene ring; and/or, when β ring is "5-6 heteroaromatic ring substituted by 1-3 heteroatoms selected from the group consisting of N, O and S", the "5-6 heteroaromatic ring substituted by 1-3 heteroatoms selected from the group consisting of N, O and S" is pyridine ring, pyridazine ring, pyrrole ring, pyrazole ring, furan ring or thiophene ring; and/or, when each $R^2$ is independently halogenated or unsubstituted $C_1$-$C_5$ alkyl, the number of the halogen is one or more, when more than one halogen exists, the halogens may be the same or different; and/or, when each $R^2$ is independently halogenated or unsubstituted $C_1$-$C_5$ alkyl, the "halogen" is independently fluorine, chlorine or bromine; and/or, when each $R^2$ is independently halogenated or unsubstituted $C_1$-$C_5$ alkyl, the "$C_1$-$C_5$ alkyl" is independently $C_1$-$C_3$ alkyl; and/or, when each $R^2$ is independently $C_3$-$C_7$ cylcloalkyl, the $C_3$-$C_7$ cycloalkyl is cyclopropyl; and/or, when each $R^2$ is independently halogen, the halogen is fluorine or chlorine; and/or, when each $R^2$ is independently $C_1$-$C_5$ alkoxyl, the $C_1$-$C_5$ alkoxyl is $C_1$-$C_3$ alkoxyl; and/or, any of $R^2$ is independently located at the ortho, para or meta position of Y; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_6$ alkyl, the $C_1$-$C_6$ alkyl is $C_1$-$C_3$ alkyl; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_3$ haloalkyl, the number of the "halogen" is one or more, when more than one halogen exists, the halogens may be the same or different; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_3$ haloalkyl, the "halogen" is independently fluorine, chlorine or bromine; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_3$ haloalkyl, the "$C_1$-$C_3$ alkyl" is independently methyl, ethyl, n-propyl or isopropyl; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 3-7 membered cylcloalkyl, the 3-7 membered cylcloalkyl is cyclopropyl, cyclobutyl, cyclopentyl or cyclohexyl; and/or, when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 5-6 membered nitrogenous heteroaryl, the 5-6 membered nitrogenous heteroaryl is pyridyl, pyrazinyl, pyridazinyl, pyrimidinyl, pyrrolyl, pyrazolyl or imidazolyl; and/or, when $R^{2x}$ and $R^{2y}$ are each independently $C_1$-$C_6$ alkyl, the $C_1$-$C_6$ alkyl is $C_1$-$C_3$ alkyl; and/or, when α ring is 5-10 membered heteroaromatic ring substituted by one or more of $R^\alpha$, the more refers to 2, 3 or 4; and/or, when α ring is a 5-10 membered heteroaromatic ring substituted by one or more of $R^\alpha$, the "5-10 membered heteroaromatic ring" is "5-10 membered heteroaromatic ring containing 1-6 heteroatoms selected from the group consisting of N, O or S"; and/or, when α ring is a 5-10 membered heteroaromatic ring substituted by one or more of $R^\alpha$, the "5-10 membered heteroaromatic ring" is 5-6 membered heteroaromatic ring, or, fused ring formed by two 5-6 membered heteroaromatic ring; and/or, when each $R^\alpha$ is independently $C_1$-$C_5$ alkyl, the $C_1$-$C_5$ alkyl is $C_1$-$C_3$ alkyl; and/or, when each $R^\alpha$ is independently $C_3$-$C_7$ cylcloalkyl, the $C_3$-$C_7$ cylcloalkyl is cyclopropyl; and/or, when each $R^\alpha$ is independently $C_1$-$C_5$ alkoxyl, the $C_1$-$C_5$ alkoxyl is $C_1$-$C_3$ alkoxyl; and/or, when each $R^\alpha$ is independently halogen, the halogen is fluorine or chlorine; and/or, each $R^\alpha$ is independently located at the ortho, para or meta position of Z; and/or, when α ring contains -NH-, the $R^\alpha$ is attached to the -NH-; and/or, when $R^{\alpha1}$, $R^{\alpha2}$, $R^{\alpha3}$ and $R^{\alpha4}$ are each independently $C_1$-$C_5$ alkyl, the $C_1$-$C_5$ alkyl is $C_1$-$C_3$ alkyl; and/or, when $R^{\alpha1}$, $R^{\alpha2}$, $R^{\alpha3}$ and $R^{\alpha4}$ are each independently $C_1$-$C_5$ haloalkyl, the halogen number is one or more, when more than one halogen exists, the halogens may be the same or different; and/or, when $R^{\alpha1}$, $R^{\alpha2}$, $R^{\alpha3}$ and $R^{\alpha4}$ are each independently $C_1$-$C_5$ haloalkyl, the halogen is independently fluorine, chlorine, bromine or iodine; and/or, when $R^{\alpha1}$, $R^{\alpha2}$, $R^{\alpha3}$ and $R^{\alpha4}$ are each independently $C_1$-$C_5$ haloalkyl, the $C_1$-$C_5$ haloalkyl is $C_1$-$C_3$ haloalkyl.

**3.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 2, wherein, when $R^1$, $R^3$ and $R^4$ are each independently $C_1$-$C_5$ alkyl, the $C_1$-$C_5$ alkyl is methyl, ethyl, n-propyl or isospropyl; and/or, when $R^1$, $R^3$ and $R^4$ are each independently $C_1$-$C_5$ alkoxyl, the $C_1$-$C_5$ alkoxyl is methoxyl, ethoxyl, n-propoxyl or isopropoxyl; and/or, when $R^1$, $R^3$ and $R^4$ are each independently $C_1$-$C_5$ haloalkyl, the $C_1$-$C_5$ haloalkyl is trifluoromethyl, difluoromethyl or 1, 2-difluoroethyl; and/or, when $R^1$, $R^3$ and $R^4$ are each independently $C_1$-$C_5$ alkyl substituted by one or more of $R^{R15}$, the $C_1$-$C_5$ alkyl

substituted by one or more of $R^{R15}$ is 2-hydroxyethyl, carboxymethyl, hydroxymethyl or methoxymethyl;

and/or, when $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ independently are $C_1$-$C_5$ alkyl, the $C_1$-$C_5$ alkyl is methyl, ethyl, n-propyl or isopropyl;

and/or, when $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently $C_6$-$C_{10}$ aryl substituted by one or more of $R^{R14}$, the " $C_6$-$C_{10}$ aryl" is phenyl, the $R^{R14}$ is independently located at the ortho, para or meta position of the phenyl;

and/or, when $R^{R14}$ are each independently $C_1$-$C_5$ alkyl, the $C_1$-$C_5$ alkyl is methyl, ethyl, n-propyl or isopropyl group;

and/or, when Q is phenyl substituted by one or more of $R^{Q1}$, the "phenyl substituted by one or more of $R^{Q1}$" is 4-fluorophenyl, 4-hydroxyphenyl, 3-hydroxy-4-fluorophenyl, 4-methoxyphenyl, 4-chlorophenyl, 4-cyanophenyl, 3-chlorophenyl, 3 -fluorophenyl, 4-cyanophenyl, 2, 4-difluorophenyl, 2, 4-dichlorophenyl, 2-fluoro-4-chlorophenyl, 2-chloro-4-fluoro-5-methoxyphenyl, 3-methoxy-4-fluorophenyl,4-methoxy-3-fluorophenyl, 2-methoxy-4-fluorophenyl, 2-chloro-4-fluorophenyl, or, 2-fluoro-4-cyanophenyl;

and/or, when Q is 3-6 membered cyclohydrocarbyl-$(CH_2)n_Q$- substituted by one or more of $R^{Q2}$, the 3-6 membered cyclohydrocarbyl is cyclohexyl;

and/or, when Q is 3-6 membered heterocyclohydrocarbyl-$(CH_2)m_Q$- substituted by one or more of $R^{Q3}$, the 3-6 membered heterocyclohydrocarbyl is 5-6 membered heteroaryl, the "5-6 membered heteroaryl" is pyridyl or pyrimidinyl;

and/or, when Q is 3-6 membered heterocyclohydrocarbyl-$(CH_2)m_Q$- substituted by one or more of $R^{Q3}$, the 3-6 membered heterocyclohydrocarbyl is 3-6 membered heterocycloalkyl, the 3-6 membered heterocycloalkyl is 2-pyranyl, 3-pyranyl or 2-tetrahydrofuranyl;

and/or, when $R^{Q1}$, $R^{Q2}$, $R^{Q3}$ 和 $R^{Q4}$ are each independently halogenated or unsubstituted $C_1$-$C_5$ alkyl, the "$C_1$-$C_5$ alkyl" is independently methyl, ethyl, n-propyl or isopropyl;

and/or, when $R^{Q1}$, $R^{Q2}$, $R^{Q3}$ 和 $R^{Q4}$ are each independently $C_1$-$C_5$ alkoxyl, the $C_1$-$C_5$ alkoxyl is methoxyl, ethoxyl, n-propoxyl or isopropoxyl;

and/or, when β ring is a pyridine ring, the N atom in the pyridine ring is located at the ortho, meta or para position of Y;

and/or, when $R^2$ is independently halogenated or unsubstituted $C_1$-$C_5$ alkyl, the "$C_1$-$C_5$ alkyl" is independently methyl, ethyl, n-propyl or isopropyl;

and/or, when $R^2$ is independently $C_1$-$C_5$ alkoxyl, the $C_1$-$C_5$ alkoxyl is methoxyl;

and/or, when $n^\beta$ is1, and β ring is 6 membered ring, the $R^2$ is located at the para or meta position of Y;

and/or, when $n^\beta$ is 2, and β ring is 6 membered ring, the two $R^2$ are located at the meta and para, ortho and para or ortho and meta position of Y;

and/or, when $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently $C_1$-$C_6$ alkyl, the $C_1$-$C_6$ alkyl is methyl, ethyl, n-propyl or isopropyl;

and/or, when $R^{2a}$ , $R^{2b}$, $R^{2c}$, $R^{2d}$, $R^{2e}$, $R^{2f}$, $R^{2g}$, $R^{2h}$, $R^{2i}$ and $R^{2k}$ are each independently $C_1$-$C_3$ haloalkyl, the $C_1$-$C_3$ haloalkyl is trifluoromethyl;

and/or, when $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are independently 3-7 membered cycloalkyl, the 3-7 membered cycloalkyl is cyclopropyl;

and/or, when $R^{2x}$ and $R^{2y}$ are each independently $C_1$-$C_6$ alkyl, the $C_1$-$C_6$ alkyl is methyl, ethyl, n-propyl or isopropyl;

and/or, when α ring is 5-10 membered heteroaromatic ring substituted by one or more of $R^\alpha$, the "5-10 membered heteroaromatic ring" is 5-6 membered heteroaromatic ring, the "5-6 membered heteroaromatic ring" is " 5-6 membered heteroaromatic ring substituted by 1-4 heteroatoms selected from the group consisting of N, O or S";

and/or, when α ring is 5-10 membered heteroaromatic ring substituted by one or more of $R^\alpha$, the "5-10 membered heteroaromatic ring" is fused ring formed by two 5-6 membered heteroaromatic rings, the "fused ring formed by two 5-6 membered heteroaromatic rings" is "fused ring formed by two 5-6 membered heteroaromatic rings conctaining 1-6 heteroatoms selected from the group consisting of N, O or S";

and/or, when $R^\alpha$ is independently $C_1$-$C_5$ alkyl, the $C_1$-$C_5$ alkyl is methyl, ethyl, n-propyl or isopropyl;

and/or, when the $R^\alpha$ is independently $C_1$-$C_5$ alkoxyl, the $C_1$-$C_5$ alkoxyl is methoxyl, ethoxyl, n-propoxyl or isopropoxyl;

and/or, when $R^{\alpha 1}$, $R^{\alpha 2}$, $R^{\alpha 3}$ and $R^{\alpha 4}$ are each independently $C_1$-$C_5$ alkyl, the $C_1$-$C_5$ alkyl is methyl, ethyl, n-propyl or isopropyl;

and/or, when the $R^{\alpha 1}$, $R^{\alpha 2}$, $R^{\alpha 3}$ and $R^{\alpha 4}$ are each independently $C_1$-$C_5$ haloalkyl, the $C_1$-$C_5$ haloalkyl is trifluoromethyl.

**4.** The nitrogenous macrocyclic compound represented by formula **III-0**, the tautomer thereof, the optical isomer thereof,

the hydrate thereof, the solvate thereof, the pharmaceutically acceptable salt thereof or the prodrug thereof as defined in claim 3, wherein, when $R^1$, $R^3$ and $R^4$ are each independently $C_1$-$C_5$ alkyl, the $C_1$-$C_5$ alkyl is methyl, ethyl, or isospropyl;
and/or, when $R^1$, $R^3$ and $R^4$ are each independently $C_1$-$C_5$ haloalkyl, the $C_1$-$C_5$ haloalkyl is trifluoromethyl;
and/or, when $R^1$, $R^3$ and $R^4$ are each independtly $C_1$-$C_5$ alkyl substituted by one or more of $R^{R15}$, the $C_1$-$C_5$ alkyl substituted by one or more of $R^{R15}$ is 2-hydroxyethyl, carboxymethyl, or hydroxymethyl;
and/or, when $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently $C_1$-$C_5$ alkyl, the $C_1$-$C_5$ alkyl is methyl, ethyl, or isopropyl; and/or, when $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently $C_6$-$C_{10}$ aryl substituted by one or more of $R^{R14}$, the "$C_6$-$C_{10}$ aryl substituted by one or more of $R^{R14}$" is 4-hydroxylphenyl;
and/or, when Q is 3-6 membered heterocyclohydrocarbyl-$(CH_2)m_Q$- substituted by one or more of $R^{Q3}$, the 3-6 membered heterocyclohydrocarbyl is 5-6 membered heteroaryl, the "5-6 membered heteroaryl" is pyridyl-2-yl or pyridyl-4-yl;
and/or, when $R^{Q1}$, $R^{Q2}$, $R^{Q3}$ and $R^{Q4}$ are each independently $C_1$-$C_5$ alkoxyl, the $C_1$-$C_5$ alkoxyl is methoxyl;
and/or, when $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently $C_1$-$C_6$ alkyl, the $C_1$-$C_6$ alkyl is methyl, ethyl or isopropyl;
and/or, when $R^{2x}$ and $R2^y$ are each independently $C_1$-$C_6$ alkyl, the $C_1$-$C_6$ alkyl is methyl;
and/or, when $\alpha$ ring is a 5-10 membered heteroaromatic ring substituted by one or more of $R^\alpha$, the "5-10 membered heteroaromatic ring" is 5-6 membered heteroaromatic ring, the "5-6 membered heteroaromatic ring" is pyridine, pyridazine, pyrimidine, pyrazine, furan, thiophene, pyrrole, pyrazole, imidazole, oxazole, thiazole, pyrimidinone, oxadiazole, pyridone or triazole;
and/or, when $\alpha$ ring is a 5-10 membered heteroaromatic ring substituted by one or more of $R^\alpha$, the "5-10 membered heteroaromatic ring" is fused ring formed by two 5-6 membered heteroaromatic rings, the "fused ring formed by two 5-6 membered heteroaromatic rings" is fused ring formed by any two heteroaromatic rings selected from the group consisting of pyridine, pyridazine, pyrimidine, pyrazine, furan, thiophene, pyrrole, pyrazole, imidazole, oxazole, thiazole, pyrimidinone, oxadiazole, pyridone or triazole;
and/or, when $R^\alpha$ is indenpendently $C_1$-$C_5$ alkyl, the $C_1$-$C_5$ alkyl is methyl, ethyl n-propyl or isopropyl;
and/or, when $R^\alpha$ is indenpendently $C_1$-$C_5$ alkoxyl, the $C_1$-$C_5$ alkoxyl is methoxyl, ethoxyl, n-propoxyl or isopropoxyl.

5. The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 4, wherein, when $R^2$ is indenpendently $-N(R^{2a})(R^{2b})$, the $-N(R^{2a})(R^{2b})$ is $-NH_2$;
and/or, when $R^2$ is independently $-(CH_2)n^{\beta2}N(R^{2k})S(=O)_2(R^{2d})$, the $-(CH_2)_n{}^{\beta2}N(R^{2k})S(=O)_2(R^{2d})$ is $-N(R^{2k})S(=O)_2(R^{2d})$;
and/or, when $R^2$ is independently $-(CH_2)n^{\beta2}N(R^{2k})S(=O)_2NH(R^{2e})$, the $-(CH_2)n^{\beta2}N(R^{2k})S(=O)_2NH(R^{2e})$ is $-N(R^{2k})S(=O)_2NH(R^{2e})$; and/or, when $R^2$ is independently $-(CH_2)n^{\beta2}-S(=O)_2N(R^{2f})(R^{2g})$, the $-(CH_2)n^{\beta2}-S(=O)_2N(R^{2f})(R^{2g})$ is $-S(=O)_2N(R^{2f})(R^{2g})$ or $-CH_2-S(=O)_2N(R^{2f})(R^{2g})$;
and/or, when $R^2$ is independently $-(CR^{2x}R^{2y})-S(=O)_2N(R^{2f})(R^{2g})$, the $-(CR^{2x}R^{2y})-S(=O)_2N(R^{2f})(R^{2g})$ is

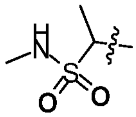 or 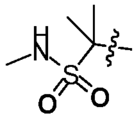 ;

and/or, when $R^2$ is independently $-(CH_2)n^{\beta2}-S(=O)(R^{2i})$, the $-(CH_2)n^{\beta2}-S(=O)(R^{2i})$ is

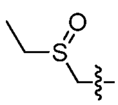

or $-S(=O)(R^{2i})$;
and/or, when $R^2$ is independently $-(CH_2)n^{\beta2}NHC(=O)NH(R^{2e})$, the $-(CH_2)n^{\beta2}NHC(=O)NH(R^{2e})$ is $-NHC(=O)NH(R^{2e})$;
and/or, when $R^2$ is independently $-(CH_2)n^{\beta2}NHC(=O)(R^{2e})$, the $-(CH_2)n^{\beta2}NHC(=O)(R^{2e})$ is $-NHC(=O)(R^{2e})$;
and/or, when $R^2$ is independently $-C_1-C_6$ alkyl-$S(=O)_2(R^{2i})$, the $-C_1-C_6$ alkyl-$S(=O)_2(R^{2i})$ is $-(CH_2)n^{\beta2}-S(=O)_2(R^{2i})$;
and/or, when $R^2$ is independently "5-6 heteroaryl substituted by one or more of $R^{2j}$", the more refers to 2, 3 or 4;
and/or, when $R^2$ is independently "5-6 heteroaryl substituted by one or more of $R^{2j}$", the "5-6 heteroaryl" is 5-6

heteroaryl substituted by 1-3 heteroatoms selected from the group consisting of N, O and S"; and/or, when $\alpha$ ring is 5-10 membered heteroaromatic ring substituted by one or more of $R^{\alpha}$, the "5-10 membered heteroaromatic ring" is 5-6 membered heteroaromatic ring, the "5-6 membered heteroaromatic ring" is

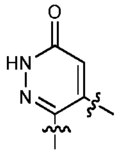, 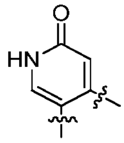, 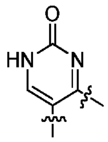, 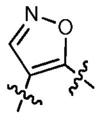, 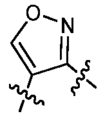, 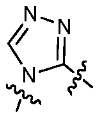,

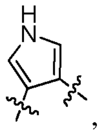, 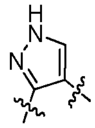 or 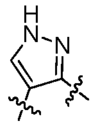;

and/or, when $\alpha$ ring is 5-10 membered heteroaromatic ring substituted by one or more of $R^{\alpha}$, the "5-10 membered heteroaromatic ring" is fused ring formed by two 5-6 membered heteroaromatic rings, the "fused ring formed by two 5-6 membered heteroaromatic rings" is

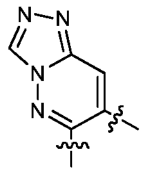,

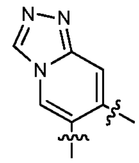, 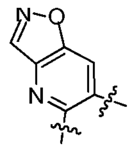 or 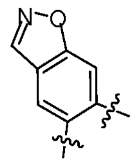;

and/or, when $R^{\alpha}$ is independently $C_1$-$C_5$ alkyl, the $C_1$-$C_5$ alkyl is methyl.

**6.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 5, wherein, when $R^2$ is indenpendently $-(CH_2)n^{\beta 2}N(R^{2k})S(=O)_2(R^{2d})$, the $-(CH_2)n^{\beta 2}N(R^{2k})S(=O)_2(R^{2d})$ is

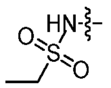, 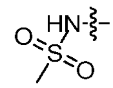

or

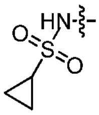;

and/or, when $R^2$ is independently $-(CH_2)n^{\beta 2}N(R^{2k})S(=O)_2NH(R^{2e})$, the $-(CH_2)n^{\beta 2}N(R^{2k})S(=O)_2NH(R^{2e})$ is $-NHS(=O)_2NH(R^{2e})$;
and/or, when $R^2$ is independently $-(CH_2)n^{\beta 2}-S(=O)_2N(R^{2f})(R^{2g})$, the $-(CH_2)n^{\beta 2}-S(=O)_2N(R^{2f})(R^{2g})$ is $-S(=O)_2N(R^{2f})(R^{2g})$, the $-S(=O)_2N(R^{2f})(R^{2g})$ is $-S(=O)_2N(R^{2g})H$;

and/or, when $R^2$ is independently $-(CH_2)n^{\beta2}-S(=O)_2N(R^{2f})(R^{2g})$, the $-(CH_2)n^{\beta2}-S(=O)_2N(R^{2f})(R^{2g})$ is $-CH_2-S(=O)_2N(R^{2f})(R^{2g})$, the $-CH_2-S(=O)_2N(R^{2f})(R^{2g})$ is

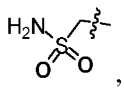 , 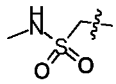 , or 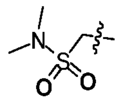 ;

and/or, when $R^2$ is independently $-(CH_2)n^{\beta2}NHC(=O)NH(R^{2e})$, the $-(CH_2)n^{\beta2}NHC(=O)NH(R^{2e})$ is

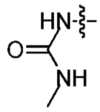 ;

and/or, when $R^2$ is independently $-(CH_2)n^{\beta2}NHC(=O)(R^{2e})$, the $-(CH_2)n^{\beta2}NHC(=O)(R^{2e})$ is acetamino;
and/or, when $R^2$ is independently $-C_1-C_6$ alkyl-$S(=O)_2(R^{2i})$, the $-C_1-C_6$ alkyl-$S(=O)_2(R^{2i})$ is $-CH_2-S(=O)_2(R^{2i})$;
and/or, when $\alpha$ ring is 5-10 membered heteroaromatic ring substituted by one or more of $R^{\alpha}$, the "5-10 membered heteroaromatic ring" is 5-6 membered heteroaromatic ring, the "5-10 membered heteroaromatic ring substituted by one or more of $R^{\alpha}$" is

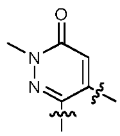 , 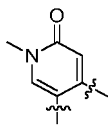 , 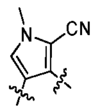

or

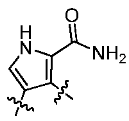 .

7. The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 6, wherein, when $R^2$ is indenpendently $-(CH_2)n^{\beta2}N(R^{2k})S(=O)_2NH(R^{2e})$, the $-(CH_2)n^{\beta2}N(R^{2k})S(=O)_2NH(R^{2e})$ is

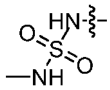 ;

and/or, when $R^2$ is independently $-(CH_2)n^{\beta2}-S(=O)_2N(R^{2f})(R^{2g})$, the $-(CH_2)n^{\beta2}-S(=O)_2N(R^{2f})(R^{2g})$ is $-S(=O)_2N(R^{2f})(R^{2g})$, the $-S(=O)_2N(R^{2f})(R^{2g})$ is

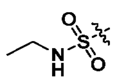 ;

and/or, when $R^2$ is independently $-C_1-C_6$ alkyl-$S(=O)_2(R^{2i})$, the $-C_1-C_6$ alkyl-$S(=O)_2(R^{2i})$ is

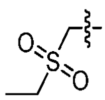 , 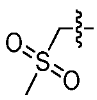 , 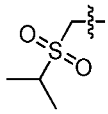 , 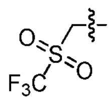 or 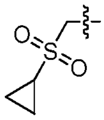 .

**8.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 1, wherein, the compound **III-0** is

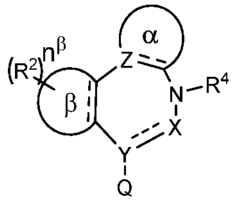

**III-1** or

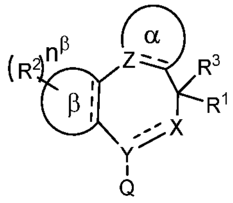

**III-2** ;

and/or, $R^1$, $R^4$ are each independently -H, $C_1$-$C_5$ alkyl, $C_3$-$C_7$ cycloalkyl, $C_1$-$C_5$ haloalkyl, $C_1$-$C_5$ alkyl substituted by one or more of $R^{R15}$, or, $-(CH_2)n_0C(=O)N(R^{R1})(R^{R2})$; $R^3$ is -H;
and/or, all of the $R^{R1}$ and $R^{R2}$ are each independently -H, 4-hydroxylphenyl or $C_1$-$C_5$ alkyl; and/or, $n_0$, $n_1$, $n_2$, $n_3$, $n_4$, $n_5$, $n_6$, $n_7$ and $m_1$ are each independently 0, or 1;
and/or, when the bond between X and Y

" 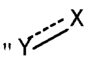 "

is a single bond, the X is $-CH_2-$, -C(=O)- or -NH-;
and/or, when the bond between X and Y

" 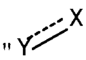 "

is a double bond, the X is =N-;
and/or, Q is phenyl substituted by one or more of $R^{Q1}$, 3-6 membered cyclohydrocarbyl substituted by one or more of $R^{Q2}$, or 3-6 membered heterocyclohydrocarbyl substituted by one or more of $R^{Q3}$;
and/or, all of $R^{Q1}$, $R^{Q2}$, $R^{Q3}$ and $R^{Q4}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;
and/or, the "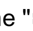" in

" 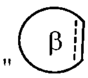 "

β ring and 7-membered ring fused by is a double bond;
and/or, the β ring is benzene ring;
and/or, $n^\beta$ is 0, 1 or 2;
and/or, all of $R^2$ are each independently hydroxyl, halogen, halogenated or unsubstituted $C_1$-$C_5$ alkyl, $C_3$-$C_7$ cycloalkyl, $C_1$-$C_5$ alkoxyl, $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CR^{2x}R^{2y})-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl-$S(=O)_2(R^{2i})$;
and/or, all $n^{\beta 2}$ are each independently 0, 1 or 2;
and/or, $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_6$ alkyl, $C_1$-$C_3$ haloalkyl or 3-7 membered cycloalkyl;
and/or, "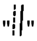" in

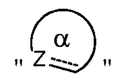

the α ring and 7-membered ring fused by is a double bond;
and/or, Z is

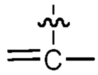

;

and/or, α ring is 5-10 membered heteroaromatic ring substituted by one or more of $R^{\alpha}$;
and/or, all $R^{\alpha}$ are each independently -H, -CN, $C_1$-$C_5$ alkyl, $C_1$-$C_5$ alkoxyl, $C_1$-$C_5$ haloalkyl, halogen or $C_3$-$C_7$ cycloalkyl-C(=O)N($R^{\alpha1}$)($R^{\alpha2}$).

**9.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 8, wherein, when the compound **III-0** is

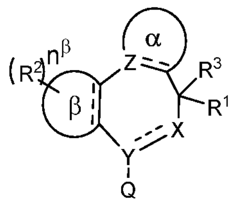

III-2

,

and $R^3$ is H, the

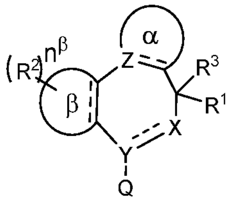

III-2 is

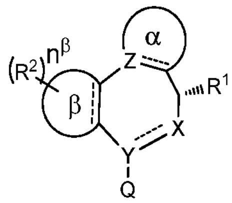

III-2-1 ;

and/or, all of the $R^{R1}$ and $R^{R2}$ are each independently -H, 4-hydroxylphenyl or $C_1$-$C_5$ alkyl; and/or, $n_0$ is 0 or 1; and/or,

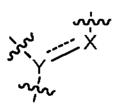

is

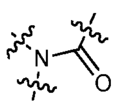

,

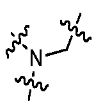

,

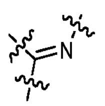

or

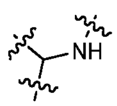

;

and/or, Q is phenyl substituted by one or more of $R^{Q1}$;
and/or, all $R^{Q1}$, $R^{Q2}$, $R^{Q3}$ and $R^{Q4}$ are each independently -H, -OH, -CN, halogen or $C_1$-$C_5$ alkoxyl; and/or, $n^{\beta}$ is 0, 1 or 2;
and/or, all of $R^2$ are each independently hydroxyl, halogen, $C_1$-$C_5$ alkoxyl, -N($R^{2a}$)($R^{2b}$), -NHC($R^{2c}$)$H_2$, -($CH_2$)$n^{\beta2}$N($R^{2k}$)S(=O)$_2$($R^{2d}$), -($CH_2$)$n^{\beta2}$N($R^{2k}$)S(=O)$_2$NH($R^{2e}$), -($CH_2$)$n^{\beta2}$-S(=O)$_2$N($R^{2f}$)($R^{2g}$), -($CR^{2x}R^{2y}$)-S(=O)$_2$N($R^{2f}$)($R^{2g}$), -($CH_2$)$n^{\beta2}$-S(=O)($R^{2i}$), -($CH_2$)$n^{\beta2}$NHC(=O)NH($R^{2e}$), -($CH_2$)$n^{\beta2}$NHC(=O)($R^{2e}$), or, -$C_1$-$C_6$ alkyl -S(=O)$_2$($R^{2i}$);
and/or, all of the $n^{\beta2}$ are each independently 0, or 1;
and/or, α ring is 5-10 membered heteroaromatic ring substituted by one or more of $R^{\alpha}$;
and/or, all of the $R^{\alpha}$ are each independently -H, -CN, $C_1$-$C_5$ alkyl or -C(=O)N($R^{\alpha1}$)($R^{\alpha2}$).

**10.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 9, wherein, all of the $R^2$ are each independetly hydroxyl, halogen, $C_1$-$C_5$ alkoxyl, $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or , $-C_1$-$C_6$ alkyl-$S(=O)_2(R^{2i})$;
and/or, all of the $R^{\alpha}$ are each independently -H or $C_1$-$C_5$ alkyl.

**11.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 8, wherein, when $n^{\beta}$ is 1, $R^2$ is located at the para or meta position of Y;
and/or, when $n^{\beta}$ is 1, $R^2$ is $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CR^{2x}R^{2y})-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl-$S(=O)_2(R^{2i})$;
and/or, when $n^{\beta}$ is 2, one of the $R^2$ is located at the para position of Y, which is $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CR^{2x}R^{2y})-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl-$S(=O)_2(R^{2i})$; the other $R^2$ is located at the meta position of Y, which is hydroxyl, halogenated or unsubstituted $C_1$-$C_5$ alkyl, $C_3$-$C_7$ cycloalkyl, halogen or $C_1$-$C_5$ alkoxyl.

**12.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 11, wherein, when $n^{\beta}$ is 1, $R^2$ is located at the para position of Y;
and/or, when $n^{\beta}$ is 1, $R^2$ is $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl $-S(=O)_2(R^{2i})$,
and/or, when $n^{\beta}$ is 2 , one of $R^2$ is located at the para position of Y, which is $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl-$S(=O)_2(R^{2i})$; the other $R^2$ is located at the meta position of Y, which is hydroxyl, halogen or $C_1$-$C_5$ alkoxyl.

**13.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 1, wherein, the compound **III-0** is

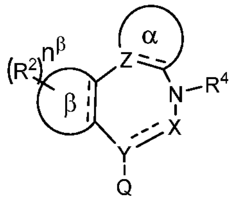

III-1 or

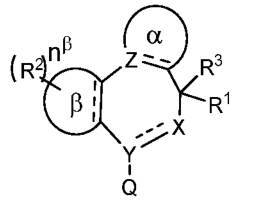

III-2 ;

$R^1$ and $R^4$ are independently -H, $C_1$-$C_5$ alkyl, $C_3$-$C_7$ cycloalkyl, $C_1$-$C_5$ haloalkyl, $C_1$-$C_5$ alkyl substituted by one or more of $R^{R15}$, or, $-(CH_2)n_0C(=O)N(R^{R1})(R^{R2})$; $R^3$ is -H;
$R^{R15}$ are each independently $C_1$-$C_5$ alkoxyl, -COOH or -OH;
all of the $R^{R1}$ and $R^{R2}$ are each independently -H, 4-hydroxylphenyl or $C_1$-$C_5$ alkyl; $n_0$ is 0 or 1;
or, $R_1$, $R_3$ and the carbon atom they are attached to together form $C_3$-$C_7$ cycloalkyl;

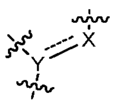 is 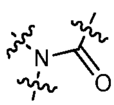, 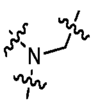, 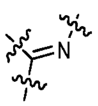 or 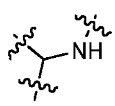 ;

Q is phenyl substituted by one or more of $R^{Q1}$, 3-6 membered cyclohydrocarbyl substituted by one or more of $R^{Q2}$, or, 3-6 membered heterocyclohydrocarbyl substituted by one or more of $R^{Q3}$;

all $R^{Q1}$, $R^{Q2}$, $R^{Q3}$ and $R^{Q4}$ are each independently -H, -OH, -CN, halogen, halogenated or unsubstituted $C_1$-$C_5$ alkyl, $C_3$-$C_7$ cycloalkyl, or $C_1$-$C_5$ alkoxyl;

the "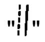" in

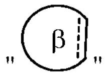

β ring and 7-mebered ring fused by is a double bond;
β ring is benzene ring;
$n^{\beta}$ is 0, 1 or 2; when $n^{\beta}$ is 1 or 2, at least one of the $R^2$ located at the para or meta position of Y;
all of the $R^2$ are each independently hydroxyl, halogen, halogenated or unsubstituted $C_1$-$C_5$ alkyl, $C_3$-$C_7$ cycloalkyl, $C_1$-$C_5$ alkoxyl, $-N(R^{2a})(R^{2b})$, $-NHC(R^{2c})H_2$, $-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CR^{2x}R^{2y})-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1$-$C_6$ alkyl-$S(=O)_2(R^{2i})$;
all of the $n^{\beta 2}$ are each independently 0, 1 or 2;
$R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ are each independently -H, $C_1$-$C_6$ alkyl, $C_1$-$C_3$ haloalkyl or 3-7 membered cycloalkyl;

"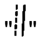" in

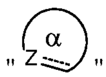

α ring and 7-membered ring fused by is a double bond;
Z is

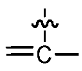;

α ring is 5-10 membered heteroaromatic ring substituted by one or more of $R^{\alpha}$;
all of the $R^{\alpha}$ are each independently -H, -CN, $C_1$-$C_5$ alkyl, $C_1$-$C_5$ alkoxyl, $C_1$-$C_5$ haloalkyl, $C_3$-$C_7$ cycloalkyl or $-C(=O)N(R^{\alpha 1})(R^{\alpha 2})$, all of the $R^{\alpha 1}$ and $R^{\alpha 2}$ are each independently -H or $C_1$-$C_5$ haloalkyl.

**14.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 13, wherein, the compound **III-0** is

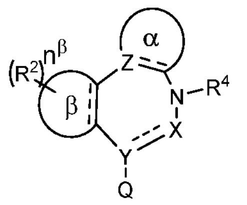

**III-1** or

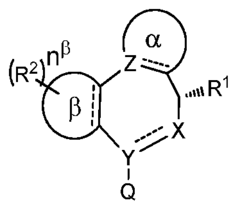

**III-2-1** ;

all of the $R^{R1}$ and $R^{R2}$ are each independently -H or $C_1$-$C_5$ alkyl;
Q is phenyl substituted by one or more of $R^{Q1}$;
all $R^{Q1}$ are each independently -H, -OH, -CN, halogen or $C_1$-$C_5$ alkoxyl;
$n^{\beta}$ is 0, 1 or 2; when $n^{\beta}$ is 1 or 2, at least one of the $R^2$ is located at the para position of Y;
all of the $R^2$ are each independently hydroxyl, halogen, $C_1$-$C_5$ alkoxyl, $-N(R^{2a})(R^{2b})$, $-NHC(R^{2c})H_2$,

$-(CH_2)n^{\beta 2}N(R^{2k})S(=O)_2(R^{2d})$, $-(CH_2)n^{\beta 2}N(R^{2k})S(=O)_2NH(R^{2e})$, $-(CH_2)n^{\beta 2}-S(=O)_2N(R^{2f})(R^{2g})$, $-(CH_2)n^{\beta 2}-S(=O)(R^{2i})$, $-(CH_2)n^{\beta 2}NHC(=O)NH(R^{2e})$, $-(CH_2)n^{\beta 2}NHC(=O)(R^{2e})$, or, $-C_1-C_6$ alkyl-$S(=O)_2(R^{2i})$;
all of the $n^{\beta 2}$ are each independently 0 or 1;
all of the $R^{\alpha}$ are each independently -H, -CN, $C_1-C_5$ alkyl or $-C(=O)N(R^{\alpha 1})(R^{\alpha 2})$.

**15.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 1, wherein, the compound **III-0** is

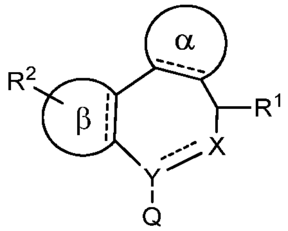

III ;

$R^1$ is selected from -H, $C_1-C_5$ linear or branched alkyl, $C_1-C_5$ linear or branched alkoxyl, $C_1-C_5$ linear or branched haloalkyl, halogen, $-(CH_2)n_0C(=O)N(R^{R1})(R^{R2})$, $-NH(CH_2)n_1C(=O)N(R^{R3})(R^{R4})$, $-(CH_2)n_2N(R^{R5})(R^{R6})$, $-(CH_2)n_3OC(=O)N(R^{R7})(R^{R8})$, $-(CH_2)n_4NHC(=O)R^{R9}$, $-(CH_2)n_5NHC(=O)OR^{R10}$, $-(CH_2)n_6NHS(=O)_2R^{R11}$ or $-(CH_2)n_7S(=)_2N(R^{12}R^{13})$;
wherein, $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ are each independently selected from -H, $C_1-C_5$ linear or branched alkyl, $C_1-C_5$ linear or branched haloalkyl, $-(CH_2)m_1CN$, $-C(CH_3)_2CN$, 3-7 membered cycloalkyl, or $C_6-C_{10}$ aryl substituted by $R^{R14}$; $n_0$, $n_1$, $n_2$, $n_3$, $n_4$, $n_5$, $n_6$, $n_7$ and $m_1$ are each independently 0, 1, 2 or 3; $R^{R14}$ is selected from -H, $C_1-C_5$ linear or branched alkyl, $C_1-C_5$ linear or branched alkoxyl, $C_1-C_5$ linear or branched haloalkyl, halogen, -OH, -CN or $-NH_2$; the substitution is mono- or poly- substitution;
X is selected from $-CH_2-$, =N-, -NH-, -O-, $-S(=O)_2-$ or -C(=O)-;
Y is selected from

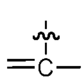, 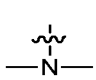 or 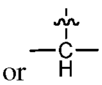 ;

at least one of the X and Y is a nitrogen atom;
Q is selected from phenyl substituted by $R^{Q1}$, 3-6 membered cyclohydrocarbyl-$(CH_2)n_Q$-substituted by $R^{Q2}$, 3-6 membered heterocyclohydrocarbyl-$(CH_2)n_Q$- substituted by $R^{Q3}$, or 5-6 membered heteroaryl substituted by $R^{Q4}$; each of $n_Q$, $m_Q$ is independently selected from 0, 1 or 2; each of $R^{Q1}$, $R^{Q2}$, $R^{Q3}$ or $R^{Q4}$ is independently selected from -H, -CN or halogen; the substitution is mono- or poly- substitution; each of the heteroatom in the heterocyclohydrocarbyl and the heteroaryl is independently selected from N or O, each of the number of the heteroatom is independently 1-3;
β ring is 5-6 membered aromatic ring or heteroaromatic ring, the heteroaromatic ring contains 1-3 heteroatoms, which is selected from N, O or S;
$R^2$ is selected from $-N(R^{2a})(R^{2b})$, $-NHC(R^{2c})H_2$, $-N(R^{2k})S(=O)_2(R^{2d})$, $-NHS(=O)_2NH(R^{2e})$, $-NHC(=O)(R^{2f})$, $-S(=O)_2N(R^{2g})H$, $-S(=O)_2(R^{2h})$, $-CH_2S(=O)_2(R^{2i})$, or 5-6 membered heteroaryl substituted by $R^{2j}$; the substitution is mono- or poly- substitution;
wherein, $R^{2a}$ , $R^{2b}$, $R^{2c}$, $R^{2d}$, $R^{2e}$, $R^{2f}$, $R^{2g}$, $R^{2h}$, $R^{2i}$ and $R^{2k}$ are each independently selected from -H, $C_1-C_3$ linear or branched alkyl, 3-7 membered cycloalkyl, $-N(CH_3)_2$, or 5-6 membered nitrogenous heteroaryl; $R^{2j}$ is selected from -H, $C_1-C_3$ linear or branched alkyl or halogen;
α ring is 5-6 membered heteroaromatic ring or fused ring formed by two 5-6 membered heteroaromatic ring, each of the 5-6 membered heteroaromatic ring and the fused ring formed by two 5-6 membered heteroaromatic ring is independently substituted by $R^{\alpha}$; the substitution is mono- or poly- substitution;
wherein, $R^{\alpha}$ is selected from -H, -CN, $C_1-C_5$ linear or branched alkyl, $C_1-C_5$ linear or branched alkoxyl, $C_1-C_5$ linear or branched haloalkyl, halogen, $-C(=O)N(R^{\alpha 1})(R^{\alpha 2})$, or $-N(R^{\alpha 3})(R^{\alpha 4})$; wherein, each of the $R^{\alpha 1}$, $R^{\alpha 2}$, $R^{\alpha 3}$ and $R^{\alpha 4}$ is independently selected from -H, $C_1-C_5$ linear or branched alkyl, or $C_1-C_5$ linear or branched haloalkyl;
wherein, when α ring is 5-6 membered heteroaromatic ring, the 5-6 membered heteroaromatic ring is not isoxazole

ring.

**16.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 15, wherein

when $R^1$ is not -H, the cyclocarbon atom the $R^1$ directly is attached to is preferably in the configuration of S;
when $R^1$ is $C_1$-$C_5$ linear or branched alkyl, the $C_1$-$C_5$ linear or branched alkyl is preferably $C_1$-$C_3$ linear or branched alkyl, more preferably methyl, ethyl, propyl or isopropyl;
when $R^1$ is $C_1$-$C_5$ linear or branched alkoxyl, the $C_1$-$C_5$ linear or branched alkoxyl is preferably $C_1$-$C_3$ linear or branched alkoxyl, more preferably methoxyl, ethoxyl, n-propoxyl or isopropoxyl; when $R^1$ is $C_1$-$C_5$ linear or branched haloalkyl, the $C_1$-$C_5$ linear or branched haloalkyl is $C_1$-$C_5$ linear or branched alkyl that substituted by one or more same or different halogen atom, the halogenation may be on the same of different carbon atom; the $C_1$-$C_5$ linear or branched haloalkyl is preferably $C_1$-$C_3$ linear or branched haloalkyl, more preferably trifluoromethyl, difluoromethyl or 1, 2-difluoethyl etc.;
when $R^1$ is halogen, the halogen is preferably fluorine or chlorine;
each of the $n_0$, $n_1$, $n_2$, $n_3$, $n_4$, $n_5$, $n_6$, $n_7$ and $m_1$ is independently preferably 0 or 1;
when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently $C_1$-$C_5$ linear or branched alkyl, the $C_1$-$C_5$ linear or branched alkyl is referably $C_1$-$C_3$ linear or branched alkyl, more preferably methyl, ethyl, propyl or isopropyl;
when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently $C_1$-$C_5$ linear or branched haloalkyl, the $C_1$-$C_5$ linear or branched haloalkyl is $C_1$-$C_5$ linear or branched alkyl that substituted by one or more same or different halogen atom, the halogenation may be on the same of different carbon atom; the $C_1$-$C_5$ linear or branched haloalkyl is preferably $C_1$-$C_3$ linear or branched haloalkyl, more preferably trifluoromethyl, difluoromethyl or 1, 2-difluoethyl etc.;
when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently 3-7 membered cycloalkyl, the 3-7 membered cycloalkyl is preferably cyclopropyl, cyclobutyl, cyclopentyl or cyclohexyl;
when each of the $R^{R1}$, $R^{R2}$, $R^{R3}$, $R^{R4}$, $R^{R5}$, $R^{R6}$, $R^{R7}$, $R^{R8}$, $R^{R9}$, $R^{R10}$, $R^{R11}$, $R^{R12}$ and $R^{R13}$ is independently $C_6$-$C_{10}$ aryl substituted by $R^{R14}$, the $C_6$-$C_{10}$ aryl is preferably phenyl; the $R^{R14}$ is selected from -H, $C_1$-$C_5$ linear or branched alkyl, $C_1$-$C_5$ linear or branched alkoxyl, $C_1$-$C_5$ linear or branched haloalkyl, halogen, -OH, -CN, or -$NH_2$; wherein, the $C_1$-$C_5$ linear or branched alkyl is preferably $C_1$-$C_3$ linear or branched alkyl, more preferably methyl, ethyl, propyl or isopropyl; the $C_1$-$C_5$ linear or branched alkoxyl is preferably $C_1$-$C_3$ linear or branched alkoxyl, more preferably methoxyl, ethoxyl, n-propoxyl or isopropoxyl; the $C_1$-$C_5$ linear or branched haloalkyl is preferably $C_1$-$C_3$ linear or branched haloalkyl, more preferably trifluoromethyl, difluoromethyl or 1,2-difluoethyl etc.; the substitution is preferably mono- or di- substitution;
X is preferably -$CH_2$-, =N-, -NH-, or -C(=O)-;
Y is preferably

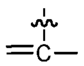 or 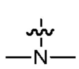;

when Q is phenyl substituted by $R^{Q1}$, the substitution is preferably mono- or di- substitution, more preferably para-substitution or 2,4-disubstitution; $R^{Q1}$ is preferably halogen, more preferably fluorine or chlorine;
when Q is 3-6 membered cyclohydrocarbyl-$(CH_2)_{nQ}$-bsubstituted by $R^{Q2}$, the 3-6 membered cyclohydrocarbyl is preferably 3-6 membered cycloalkyl; $n_Q$ is preferably 0 or 1; the substitution is preferably mono- or di- substitution, more preferably on the same carbon atom; $R^{Q2}$ is preferably -H or halogen (e.g, fluorine or chlorine);
when Q is 3-6 membered heterocyclohydrocarbyl-$(CH_2)_{mQ}$- substituted by $R^{Q3}$, the 3-6 membered heterocyclohydrocarbyl is preferably 3-6 membered cycloalkyl; $m_Q$ is preferably 0; the heteroatom is preferably O; the number of heteroatom is preferably 1, $R^{Q3}$ is preferably -H; the 3-6 membered heterocycloalkyl preferably is attached to the rest moiety of the general formula **III** by the carbon on the ring; the 3-6 membered heterocycloalkyl is preferably 2-pyranyl, 3-pyranyl, 2-tetrahydrofuranyl etc.;
when Q is 5-6 membered heteroaryl substituted by $R^{Q4}$, the 5-6 membered heteroaryl is preferably pyridyl or pyrimidyl; $R^{Q4}$ is preferably halogen (e.g., fluorine or chlorine);
β ring is preferably benzene ring, pyridine ring, pyridazine ring, pyrrole ring, furan ring or thiophene ring; further preferably benzene ring or pyridine ring;
when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently $C_1$-$C_3$ linear or branched alkyl, the $C_1$-$C_3$ linear or branched alkyl is preferably methyl, ethyl, propyl or isopropyl; when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 3-7 membered cycloalkyl, the 3-7 membered cycloalkyl is preferably

cyclopropyl, cyclobutyl, cyclopentyl or cyclohexyl;

when each of $R^{2a}$, $R^{2b}$, $R^{2c}$, $R^{2d}$, $R^{2e}$, $R^{2f}$, $R^{2g}$, $R^{2h}$, $R^{2i}$ and $R^{2k}$ is independently 5-6 membered nitrogenous heteroaryl, the 5-6 membered nitrogenous heteroaryl is preferably pyridyl, pyrazinyl, pyridazinyl, pyrimidinyl, pyrrolyl, pyrazolyl or imidazolyl; more preferably is attached to the rest moiety of the general formula **III** by the carbon atom on the ring;

when $R^2$ is 5-6 membered heteroaryl substituted by $R^{2j}$, $R^{2j}$ is preferably methyl, ethyl, propyl or isopropyl; the heteroatom of the 5-6 membered heteroaryl is preferably a nitrogen atom, the 5-6 membered heteroaryl is more preferably pyridyl, pyrazinyl, pyridazinyl, pyrimidinyl, pyrrolyl, pyrazolyl or imidazolyl;

when $\alpha$ ring is 5-6 membered heteroaromatic ring substituted by $R^{\alpha}$, the 5-6 membered heteroaromatic ring is preferably pyridine, pyridazine, pyrimidine, pyrazine, furan, thiophene, pyrrole, pyrazole, imidazole, oxazole, thiazole, pyrimidinone, oxadiazole, pyridone, triazole; the substitution is preferably mono- or di- substitution;

when $\alpha$ ring is $R^{\alpha}$ substituted fused ring formed by two 5-6 membered heteroaromatic rings, the fused ring formed by two 5-6 membered heteroaromatic rings is preferably formed by any two selected from the group consisting of pyridine, pyridazine, pyrimidine, pyrazine, furan, thiophene, pyrrole, pyrazole, imidazole, oxazole, thiazole, pyrimidinone, oxadiazole, pyridone, triazole; the substitution is preferably mono- or di- substitution;

when $R^{\alpha}$ is $C_1$-$C_5$ linear or branched alkyl, the $C_1$-$C_5$ linear or branched alkyl is preferably $C_1$-$C_3$ linear or branched alkyl, more preferably methyl, ethyl, propyl or isopropyl;

when $R^{\alpha}$ is $C_1$-$C_5$ linear or branched alkoxyl, the $C_1$-$C_5$ linear or branched alkoxyl is preferably $C_1$-$C_3$ linear or branched alkoxyl, more preferably methoxyl, ethoxyl, n-propoxyl or isopropoxyl; when $R^{\alpha}$ is $C_1$-$C_5$ linear or branched haloalkyl, the $C_1$-$C_5$ linear or branched haloalkyl is $C_1$-$C_5$ linear or branched alkyl substituted by one or more same or different halogen atom, the halogenation may be on the same or different carbon atom; the $C_1$-$C_5$ linear or branched haloalkyl is preferably $C_1$-$C_3$ linear or branched haloalkyl, more preferably trifluoromethyl, difluoromethyl or 1, 2-difluoethyl etc.;

when $R^{\alpha}$ is halogen, the halogen is preferably fluorine or chlorine;

when $R^{\alpha}$ is $C(=O)N(R^{\alpha1})(R^{\alpha2})$, each of the $R^{\alpha1}$ and $R^{\alpha2}$ is preferably -H, $C_1$-$C_5$ linear or branched alkyl, more preferably methyl, ethyl, propyl or isopropyl;

when $R^{\alpha}$ is $-N(R^{\alpha3})(R^{\alpha4})$, each of the $R^{\alpha3}$ and $R^{\alpha4}$ is preferably -H, $C_1$-$C_5$ linear or branched alkyl, more preferably methyl, ethyl, propyl or isopropyl.

**17.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 15, wherein, $\alpha$ ring is $R^{\alpha}$ substituted

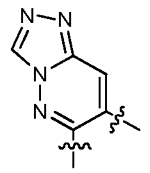 , 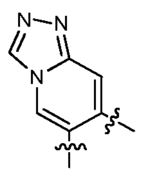 , 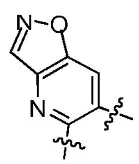 , 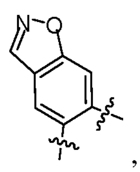 , 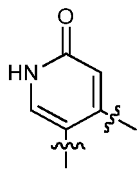 , 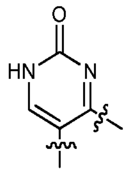 , 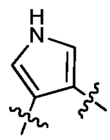 ,

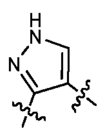 or 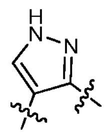 ;

the substitution is preferably mono- or di- substitution; wherein, $R^{\alpha}$ is as defined in claim 1; preferably methyl, methoxyl, ethylamino, carbamoyl or cyano.

**18.** The nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in claim 1, wherein the compound **III-0** is any compound as followed:

6-(4-fluorophenyl)-2-methyl-9-(methylsulfonemethyl)-5-oxo-2,4,5,6-tetrahydrobenzo[*b*]pyrrolo[3,4-d]azepin-3-nitrile

6-(4-fluorophenyl)-2-methyl-9-(methylsulfonemethyl)-5-oxo-2,4,5,6-tetrahydrobenzo[*b*]pyrrolo[3,4-d]azepin-3-carboxamide

6-(4-fluorophenyl)-2-methyl-9-(methylsulfonemethyl)-2,4,5,6-tetrahydrobenzo[b]pyrrolo[3,4-*d*]azepin-3-nitrile

6-(4-fluorophenyl)-2-methyl-9-(methylsulfonemethyl)-2,4,5,6-tetrahydrobenzo[*b*]pyrrolo[3,4-d]azepin-3-carboxamide
9-fluoro-6-(4-fluorophenyl)-2-methyl-2,4,5,6-tetrahydropyrido[2,3-*b*]pyrrolo[3,4-*d*]azepin-3-carboxamide
7-(4-fluorophenyl)-2-methyl-10-(methylsulfonemethyl)-2*H*-benzo[*c*]pyrido[3,4-*e*]azepin-3(*5H*)-ketone
7-(4-fluorophenyl)-2-methyl-10-(methylsulfonemethyl)-6,7-dihydro-2*H*-benzo[*c*]pyrido[3,4-*e*]azepin-3(5*H*)-ketoformate;
7-(4-Fluorophenyl)-2-methyl-10-(methylsulfonemethyl)-6,7-dihydro-2*H*-benzo[*c*]pyrido[3,4-*e*]azepin-3(5*H*)-ketone;
7-(4-Fluorophenyl)-2-methyl-10-(ethylsulfonemethyl)-2*H*-benzo[*c*]pyrido[3,4-e]azepin-3(5*H*)-ketone;
7-(4-Chlorophenyl)-2-methyl-2,5-dihydro-3*H*-benzo[*c*]pyrido[3,4-*e*]azepin-3-one;
7-(4-Chlorophenyl)-10-((ethylsulfone)methyl)-2-methyl-2,5-dihydro-3*H*-benzo[*c*]pyrido[3,4-*e*]azepin-3-one;
7-(2,4-Difluorophenyl)-2-methyl-10-(ethylsulfonemethyl)-2*H*-benzo[*c*]pyrido[3,4-*e*]azepin-3(5*H*)-ketone;
7-(4-Chloro-2-fluorophenyl)-10-((ethylsulfone)methyl)-2-methyl-2,5-dihydro-3*H*-benzo[*c*]pyrido[3,4-*e*]azepin-3-one;
(*S*)-2-(7-(4-fluorophenyl)-2-methyl-10-(methylsulfonylmethyl)-3-oxo-3,5-dihydro-2*H*-benzo[*c*]Pyrido[3,4-*e*]azepin-5-yl)acetamide;
(*S*)-*N*-ethyl-2-(7-(4-fluorophenyl)-2-methyl-10-(methylsulfonemethyl)-3-oxo-3,5-dihydro-2*H*-benzo[*c*]pyrido[3,4-*e*]azepin-5-yl)acetamide;
(*S*)-2-(7-(4-chlorophenyl)-2-methyl-3-oxo-3,5-dihydro-2*H*-benzo[*c*]pyrido[3,4-*e*]azepin-5-yl)-*N*-ethylacetamide;
(*S*)-2-(7-(4-chlorophenyl)-2-methyl-3-oxo-3,5-dihydro-2*H*-benzo[*c*]pyrido[3,4-*e*]azepin-5-yl)-acetamide;
(*S*)-2-(7-(4-chlorophenyl)-2-methyl-3-oxo-3,5-dihydro-2*H*-benzo[*c*]pyrido[3,4-*e*]azepin-5-yl)-*N*-is opropylacetamide;
*N*-(7-(4-fluorophenyl)-2-methyl-3-oxo-3,5-dihydro-2*H*-benzo[*c*]pyrido[3,4-*e*]azepin-10-yl)ethanesulfonamide;
7-(4-Fluorophenyl)-10-methoxy-2-methyl-2,5-dihydro-3*H*-benzo[*c*]pyrido[3,4-*e*]azepin-3-ketone;
7-(4-Fluorophenyl)-9-methoxy-2-methyl-2,5-dihydro-3*H*-benzo[*c*]pyrido[3,4-*e*]azepin-3-ketone;
(*S*)-*N*-ethyl-2-(7-(4-fluorophenyl)-10-methoxy-2-methyl-3-oxo-3,5-dihydro-2*H*-benzo[*c*]pyrido[3, 4-*e*]azepin-5-yl)acetamide;
10-((Ethylsulfonyl)methyl)-7-(4-fluorophenyl)-2-methyl-2,5-dihydro-3*H*-dipyrido[2,3-*c*:3',4'-*e*]azepin-3-one;
(*S*)-*N*-ethyl-2-(7-(4-fluorophenyl)-9-methoxy-2-methyl-3-oxo-3,5-dihydro-2*H*-benzo[*c*]pyrido[3,4-*e*]azepin-5-yl)acetamide;
*N*-(7-(4-chlorophenyl)-2-methyl-3-oxo-3,5-dihydro-2*H*-benzo[*c*]pyrido[3,4-*e*]azepin-10-yl)ethanesulfonamide;
(*S*)-7-(4-chlorophenyl)-2,5-dimethyl-2,5-dihydro-3H-benzo[*c*]pyrido[3,4-*e*]azepin-3-ketone;
(*S*)-10-((ethylsulfone)methyl)-7-(4-fluorophenyl)-2,5-dimethyl-2,5-dihydro-3*H*-benzo[*c*]pyrido[3,4-*e*]azepin-3-one;
(*R*)-10-((ethylsulfone)methyl)-7-(4-fluorophenyl)-2,5-dimethyl-2,5-dihydro-3*H*-benzo[*c*]pyrido[3,4-e]azepin-3-one;
7-(4-Chlorophenyl)-10-((ethylsulfone)methyl)-2-methyl-2,5-dihydro-3*H*-dipyrido[3,4-*c*:3',4'-*e*]azepin-3-one;
7-(2-Chloro-4-fluorophenyl)-10-((ethylsulfone)methyl)-2-methyl-2,5-dihydro-3*H*-benzo[*c*]pyrido[3,4-*e*]azepin-3-one;
(*S*)-2-(7-(4-chlorophenyl)-2-methyl-3-oxo-3,5-dihydro-2*H*-dipyrido[2,3-*c*:3',4'-*e*]azepin-5-yl)acetamide;
(*S*)-2-(7-(4-chlorophenyl)-2-methyl-3-oxo-3,5-dihydro-2*H*-dipyrido[2,3-*c*:3',4'-*e*]azepin-5-yl)-*N*-ethylacetamide;
*N*-(9-chloro-7-(4-fluorophenyl)-2-methyl-3-oxo-3,5-dihydro-2*H*-benzo[*c*]pyrido[3,4-*e*]azepin-10-yl)ethanesulfonamide;
(*S*)-2-(7-(4-chlorophenyl)-9-fluoro-2-methyl-3-oxo-3,5-dihydro-2*H*-benzo[*c*]pyrido[3,4-*e*]azepin-5-yl)-*N*-ethylacetamide;
(*S*)-*N*-ethyl-2-(8-fluoro-7-(4-fluorophenyl)-9-methoxy-2-methyl-3-oxo-3,5-dihydro-2*H*-benzo[*c*]pyrido[3,4-*e*]azepin-5-yl)acetamide;
(*S*)-*N*-ethyl-2-(10-fluoro-7-(4-fluorophenyl)-9-methoxy-2-methyl-3-oxo-3,5-dihydro-2*H*-benzo[*c*]pyrido[3,4-*e*]azepin-5-yl)acetamide;
(*S*)-10-((ethylsulfone)methyl)-7-(4-chlorophenyl)-2,5-dimethyl-2,5-dihydro-3*H*-benzo[*c*]pyrido[3,4-*e*]azepin-3-one;
(*S*)-5-ethyl-10-((ethylsulfone)methyl)-7-(4-fluorophenyl)-2-methyl-2,5-dihydro-3*H*-benzo[*c*]pyrido[3,4-*e*]azepin-3-one;
9-((Ethylsulfonyl)methyl)-7-(4-fluorophenyl)-2-methyl-2,5-dihydro-3*H*-benzo[*c*]pyrido[3,4-*e*]azepin-3-one;
(*R*)-10-((ethylsulfone)methyl)-7-(4-fluorophenyl)-2-methyl-5-(trifluoromethyl)-2,5-dihydro-3*H*-benzo[*c*]pyrido[3,4-*e*]azepin-3-one;
10-((Ethylsulfonyl)methyl)-7-(4-fluorophenyl)-2-methylspiro[benzo[*c*]pyrido[3,4-*e*]azepin-5,1'-cyclopropane]-3(2*H*)-one;

(*S*)-5-cyclopropyl-10-((ethylsulfone)methyl)-7-(4-fluorophenyl)-2-methyl-2,5-dihydro-3*H*-benzo[c]pyrido[3,4-*e*]azepin-3-one;
(*S*)-7-(4-fluorophenyl)-2,5-dimethyl-10-((trifluoromethylsulfone)methyl)-2,5-dihydro-3*H*-benzo[*c*]pyrido[3,4-*e*]azepin-3-one;
(*S*)-10-((isopropylsulfone)methyl)-7-(4-fluorophenyl)-2,5-dimethyl-2,5-dihydro-3*H*-benzo[*c*]pyrido[3,4-*e*]azepin-3-one;
(*S*)-7-(4-fluorophenyl)-2,5-dimethyl-10-((cyclopropylsulfone)methyl)-2,5-dihydro-3*H*-benzo[*c*]pyrido[3,4-*e*]azepin-3-one;
(*S*)-7-(2,4-difluorophenyl)-10-((ethylsulfone)methyl)-2,5-dimethyl-2,5-dihydro-3*H*-benzo[*c*]pyrido[3,4-*e*]azepin-3-one;
(*S*)-5-ethyl-10-((ethylsulfone)methyl)-7-(4-chlorophenyl)-2-methyl-2,5-dihydro-3*H*-benzo[*c*]pyrido[3,4-*e*]azepin-3-one;
(5*S*)-10-((ethylsulfoxide)methyl)-7-(4-fluorophenyl)-2,5-dimethyl-2,5-dihydro-3*H*-benzo[*c*]pyrido[3,4-*e*]azepin-3-one;
(*S*)-*N*-(7-(4-fluorophenyl)-2,5-dimethyl-3-oxo-3,5-dihydro-2*H*-benzo[*c*]pyrido[3,4-*e*]azepin-10-yl)ethanesulfonamide;
(*S*)-*N*-(7-(4-fluorophenyl)-2,5-dimethyl-3-oxo-3,5-dihydro-2*H*-benzo[*c*]pyrido[3,4-*e*]azepin-10-yl)methylaminosulfonamide;
(*S*)-1-ethyl-3-(7-(4-fluorophenyl)-2,5-dimethyl-3-oxo-3,5-dihydro-2*H*-benzo[*c*]pyrido[3,4-*e*]azepin-10-yl)urea;
(*S*)-*N*-ethyl-7-(4-fluorophenyl)-2,5-dimethyl-3-oxo-3,5-dihydro-2*H*-benzo[*c*]pyrido[3,4-*e*]azepin-10-sulfonamide;
(*S*)-10-((ethylsulfone)methyl)-7-(4-methoxyphenyl)-2,5-dimethyl-2,5-dihydro-3*H*-benzo[*c*]pyrido[3,4-*e*]azepin-3-one;
(*S*)-10-((ethylsulfone)methyl)-7-(4-hydroxyphenyl)-2,5-dimethyl-2,5-dihydro-3*H*-benzo[*c*]pyrido[3,4-*e*]azepin-3-one;
(*S*)-10-((ethylsulfone)methyl)-7-(4-fluoro-3-methoxyphenyl)-2,5-dimethyl-2,5-dihydro-3*H*-benzo[*c*]pyrido[3,4-*e*]azepin-3-one;
(*S*)-10-((ethylsulfone)methyl)-7-(4-fluoro-3-hydroxyphenyl)-2,5-dimethyl-2,5-dihydro-3*H*-benzo[*c*]pyrido[3,4-*e*]azepin-3-one;
(*S*)-*N*-(9-fluoro-7-(4-fluorophenyl)-2,5-dimethyl-3-oxo-3,5-dihydro-2*H*-benzo[c]pyrido[3,4-*e*]azepin-10-yl)ethanesulfonamide;
(*S*)-*N*-ethyl-2-(10-((ethylsulfonyl)methyl)-7-(4-fluorophenyl)-2-methyl-3-oxo-3,5-dihydro-2*H*-benzo[*c*]pyrido[3,4-*e*]azepin-5-yl)acetamide;
(*S*)-2-(10-((ethylsulfonyl)methyl)-7-(4-fluorophenyl)-2-methyl-3-oxo-3,5-dihydro-2*H*-benzo[*c*]pyrido[3,4-*e*]azepin-5-yl)aceticacid;
(*S*)-2-(10-((ethylsulfonyl)methyl)-7-(4-fluorophenyl)-2-methyl-3-oxo-3,5-dihydro-2*H*-benzo[*c*]pyrido[3,4-*e*]azepin-5-yl)acetamide;
(*S*)-10-((ethylsulfone)methyl)-7-(4-fluorophenyl)-9-methoxy-2,5-dimethyl-2,5-dihydro-3*H*-benzo[*c*]pyrido[3,4-*e*] azepin-3-one;
(*S*)-10-((ethylsulfone)methyl)-7-(4-fluorophenyl)-9-hydroxy-2,5-dimethyl-2,5-dihydro-3*H*-benzo[*c*]pyrido[3,4-*e*]azepin-3-one;
(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)ethylsulfonamide;
(*S*)-7-(4-fluorophenyl)-2,5-dimethyl-10-((methylsulfone)methyl)-2,5-dihydro-3*H*-benzo[*c*]pyrido[3,4-*e*]azepin-3-one;
(*S*)-10-((ethylsulfone)methyl)-7-(4-fluorophenyl)-5-isopropyl-2-methyl-2,5-dihydro-3*H*-benzo[*c*]pyrido[3,4-*e*]azepin-3-one;
(*S*)-*N*-(7-(4-chlorophenyl)-9-fluoro-2,5-dimethyl-3-oxo-3,5-dihydro-2*H*-benzo[*c*]pyrido[3,4-*e*]azepin-10-yl)ethanesulfonamide;
(*S*)-*N*-(9-chloro-5-ethyl-7-(4-fluorophenyl)-2-methyl-3-oxo-3,5-dihydro-2*H*-benzo[*c*]pyrido[3,4-*e*]azepin-10-yl)ethanesulfonamide;
(*S*)-*N*-(5-ethyl-9-fluoro-7-(4-fluorophenyl)-2-methyl-3-oxo-3,5-dihydro-2*H*-benzo[*c*]pyrido[3,4-*e*]azepin-10-yl)ethanesulfonamide;
(*S*)-*N*-(9-chloro-7-(4-chlorophenyl)-2,5-dimethyl-3-oxo-3,5-dihydro-2*H*-benzo[*c*]pyrido[3,4-*e*]azepin-10-yl)ethanesulfonamide;
(*S*)-10-amino-9-chloro-7-(4-fluorophenyl)-2,5-dimethyl-2,5-dihydro-3*H*-benzo[*c*]pyrido[3,4-*e*]azepin-3-one;
(*S*)-10-amino-9-fluoro-7-(4-fluorophenyl)-2,5-dimethyl-2,5-dihydro-3*H*-benzo[*c*]pyrido[3,4-*e*]azepin-3-one;
(*S*)-(7-(4-fluorophenyl)-2,5-dimethyl-3-oxo-3,5-dihydro-2*H*-benzo[*c*]pyrido[3,4-*e*]azepin-10-yl)methanesulfonamide;
(*S*)-1-(7-(4-fluorophenyl)-2,5-dimethyl-3-oxo-3,5-dihydro-2*H*-benzo[*c*]pyrido[3,4-*e*]azepin-10-yl)-*N*-methylmethanesulfonamide;
(*S*)-1-(7-(4-fluorophenyl)-2,5-dimethyl-3-oxo-3,5-dihydro-2*H*-benzo[*c*]pyrido[3,4-*e*]azepin-10-)-*N*,*N*-dimethylmethanesulfonamide;
(*S*)-*N*-(9-fluoro-7-(4-fluorophenyl)-2,5-dimethyl-3-oxo-3,5-dihydro-2*H*-benzo[c]pyrido[3,4-*e*]azepin-10-yl)methanesulfonamide;
(*S*)-4-(10-((ethylsulfonyl)methyl)-2,5-dimethyl-3-oxo-3,5-dihydro-2*H*-benzo[*c*]pyrazolo[3,4-*e*]azepin-7-yl)benzonitrile;
(*S*)-*N*-(9-chloro-5-cyclopropyl-7-(4-fluorophenyl)-2-methyl-3-oxo-3,5-dihydro-2*H*-benzo[*c*]pyrido[3,4-*e*] azepin-10-yl)ethanesulfonamide;
10-((Ethylsulfonyl)methyl)-7-(4-fluorophenyl)-2,5-dimethyl-2,5-dihydro-3*H*-benzo[*e*]pyrido[4,3-*c*][1,2]diazepin-3-one;
(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)methanesulfonamide;
(*S*)-*N*-(9-chloro-7-(4-fluorophenyl)-2,5-dimethyl-3-oxo-3,5-dihydro-2*H*-benzo[*c*]pyrido[3,4-*e*]azepin-10-yl)cyclopropylsulfonamide;
10-((Ethylsulfonyl)methyl)-7-(4-fluorophenyl)-2,5-dimethyl-2,5-dihydro-3*H*-benzo[*c*]pyridazino[3,4-*e*]azepin-3-one;
10-((Ethylsulfonyl)methyl)-7-(4-fluorophenyl)-5-(methoxymethyl)-2-methyl-2,5-dihydro-3*H*-benzo[*c*]pyrido[3,4-*e*]azepin-3-one;
(*S*)-*N*-(9-fluoro-7-(4-fluorophenyl)-2,5-dimethyl-3-oxo-3,5-dihydro-2*H*-benzo[c]pyrido[3,4-*e*]azepin-10-yl)propanamide;
(*S*)-*N*-(5-cyclopropyl-9-fluoro-7-(4-fluorophenyl)-2-methyl-3-oxo-3,5-dihydro-2*H*-benzo[*c*]pyrido[3,4-*e*]azepin-10-yl)ethanesulfonamide;
(*S*)-10-((ethylsulfone)methyl)-9-fluoro-7-(4-fluorophenyl)-2,5-dimethyl-2,5-dihydro-3*H*-benzo[*c*]pyrido[3,4-*e*]azepin-3-one;
(*S*)-9-chloro-10-((ethylsulfone)methyl)-7-(4-fluorophenyl)-2,5-dimethyl-2,5-dihydro-3*H*-benzo[*c*]pyrido[3,4-*e*]azepin-3-one;
(*S*)-10-((ethylsulfone)methyl)-7-(4-fluoro-2-methoxyphenyl)-2,5-dimethyl-2,5-dihydro-3*H*-benzo[ *c*]pyrido[3,4-*e*]azepin-3-one;
(*S*)-10-((ethylsulfone)methyl)-7-(4-fluorophenyl)-5-(2-hydroxyethyl)-2-methyl-2,5-dihydro-3H-benzo[*c*]pyrido[3,4-*e*]azepin-3-one;
10-((Ethylsulfonyl)methyl)-7-(4-fluoro-3-methoxyphenyl)-2-methyl-2,5-dihydro-3*H*-benzo[*c*]pyrido[3,4-*e*]azepin-3-one;
(*S*)-2-(7-(4-fluorophenyl)-2,5-dimethyl-3-oxo-3,5-dihydro-2*H*-benzo[*c*]pyrido[3,4-*e*]azepin-10-yl)-*N*-methylpropane-2-sulfonamide;
1-((*S*)-7-(4-fluorophenyl)-2,5-dimethyl-3-oxo-3,5-dihydro-2*H*-benzo[*c*]pyrido[3,4-*e*]azepin-10-yl)-*N*-methylethane-1-sulfonamide;
*N*-(9-fluoro-7-(4-fluorophenyl)-2,5-dimethyl-3-oxo-3,5-dihydro-2*H*-benzo[*e*]pyrido[4,3-*c*][1,2]diazepin-10-yl)ethanesulfonamide;
7-(2-Chloro-4-fluorophenyl)-10-((ethylsulfone)methyl)-2,5-dimethyl-2,5-dihydro-3*H*-benzo[*e*]pyrido[4,3-*c*][1,2]diazepin-3-one;
10-((ethylsulfone)methyl)-7-(4-fluorophenyl)-9-methoxy-2-methyl-2,5-dihydro-3*H*-benzo[*c*]pyrido[3,4-*e*]azepin-3-one;
*N*-(9-fluoro-7-(4-fluorophenyl)-2,5-dimethyl-3-oxo-3,5-dihydropyrido-2*H*-benzo[*c*]pyridazino[3,4-*e*]azepin-10-yl)ethanesulfonamide;
7-(2-Chloro-4-fluoro-5-methoxyphenyl)-10-((ethylsulfone)methyl)-2-methyl-2,5-dihydro-3*H*-benzo[*c*]pyrido[3,4-*e*]azepin-3-one;
7-(2-Chloro-4-fluorophenyl)-10-((ethylsulfone)methyl)-2,5-dimethyl-2,5-dihydro-3*H*-benzo[*c*]pyridazino[3,4-*e*]azepin-3-one;
7-(2-Chloro-4-fluorophenyl)-2-methyl-10-((methylsulfone)methyl)-2,5-dihydro-3*H*-benzo[*c*]pyrido[3,4-*e*]azepin-3-one;
1-(7-(4-fluorophenyl)-2,5-dimethyl-3-oxo-3,5-dihydro-2*H*-benzo[*e*]pyrido[4,3-*c*][1,2]diazepin-10-yl)-*N*-methylmethanesulfonamide;
10-((Ethylsulfonyl)methyl)-7-(4-fluoro-3-methoxyphenyl)-2,5-dimethyl-2,5-dihydro-3*H*-benzo[*c*]pyridazino[3,4-*e*]azepin-3-one;
(*S*)-7-(2-chloro-4-fluorophenyl)-10-((ethylsulfone)methyl)-2,5-dimethyl-2,5-dihydro-3*H*-benzo[*c*]pyrido[3,4-

*e*]azepin-3-one;
(*S*)-7-(2-chloro-4-fluoro-5-methoxyphenyl)-10-((ethylsulfone)methyl)-2,5-dimethyl-2,5-dihydro-3 *H*-benzo[*c*]pyrido[3,4-*e*]azepin-3-one;
(*S*)-1-(7-(4-fluoro-3-methoxyphenyl)-2,5-dimethyl-3-oxo-3,5-dihydro-2*H*-benzo[*c*]pyrido[3,4-*e*]azepin-10-yl)-*N*-methylmethanesulfonamide;
(*S*)-10-((ethylsulfone)methyl)-7-(3-fluoro-4-methoxyphenyl)-2,5-dimethyl-2,5-dihydro-3*H*-benzo[*c*]pyrido[3,4-*e*]azepin-3-one;
(*S*)-10-((ethylsulfone)methyl)-2,5-dimethyl-7-(pyrido-4-yl)-2,5-dihydro-3*H*-benzo[*c*]pyrido[3,4-*e*]azepin-3-one.

**19.** A preparation method for the nitrogenous macrocycle compound represented by the formula (**III-0-A**), comprising: carrying out an amide reduction on the intermediate compound represented by formula (**III-0-B**):

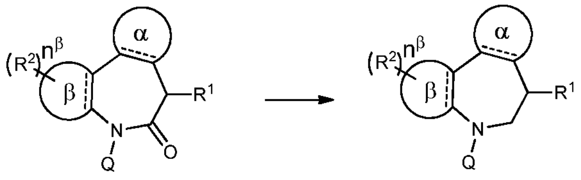

III-0-B III-0-A ;

wherein, the definitions of the substituents are as defined in at least one of claims 1-18.

**20.** A **III-0-C, III-0-E, III-0-F, III-G, III-0-H, III-0-J, III-0-L, III-I** or **III-K**:

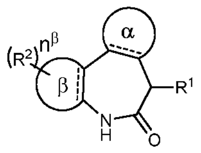

III-0-C

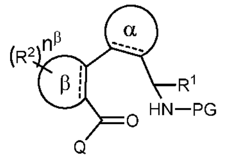

III-0-E

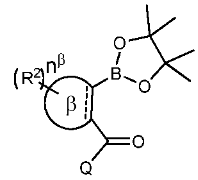

III-0-F

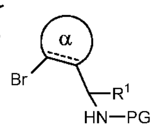

III-G

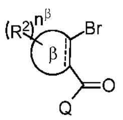

III-0-H

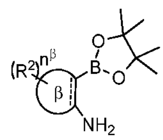

III-0-J

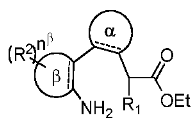

III-0-L

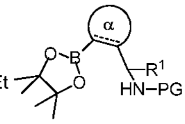

III-I

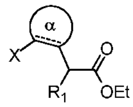

III-K

wherein, the definitions of the substituents are as defined in at least one of claims 1-18.

**21.** Use of the nitrogenous macrocyclic compound represented by formula **III-0**, the tautomer thereof, the optical isomer thereof, the hydrate thereof, the solvate thereof, the pharmaceutically acceptable salt thereof or the prodrug thereof as defined in at least one of claims 1-18 in manufacturing a bromodomain inhibitor, or in manufacturing a medicament for treating and/or preventing a disease requiring modulation of the binding ability of bromodomain and acetylated protein for treating and/or preventing;
the "diseases require modulation of the binding ability of bromodomain and acetylated protein for treating and/or preventing" are, for example, tumor, pulmonary disease, inflammatory disease or autoimmune disease, for another example acoustic neuroma, acute leukemia, acute lymphocytic leukemia, acute myeloid leukemia, acute t-cell leukemia, basal cell carcinoma, cholangiocarcinoma, bladder cancer, brain cancer, breast cancer, bronchial cancer, cervical cancer, chondrosarcoma, chordoma, choriocarcinoma, chronic leukemia, chronic lymphocytic leukemia, chronic myeloid leukemia, chronic myelogenous leukemia, colon cancer, colorectal cancer, craniopharyngioma, cystadenocarcinoma, diffuse large B-cell lymphoma, dysproliferativechanges, embryonic cancer, endometrial cancer, endothelial sarcoma, ependymoma, epithelial cancer, erythroleukemia, esophageal cancer, estrogen receptor-positive breast cancer, essential thrombocytosis, Ewing's sarcoma, fibrosarcoma, follicular lymphoma, germ cell

testicular cancer, glioma, glioblastoma, glioma, heavy chain disease, hemangioblasts, liver cancer, hepatocellular carcinoma, hormone-insensitive prostate cancer, leiomyosarcoma, leukemia, liposarcoma, lung carcinoma, lymphangioendotheliosarcoma, lymphatic sarcoma, lymphoblastic leukemia, lymphoma, bladder, breast, colon, lung, ovary, pancreas, prostate, skin and uterus malignant tumors and hyperproliferative disorders, T-Cell or B-cell derived lymphoid malignancy, leukemia, lymphoma, medullary carcinoma, medulloblastoma, melanoma, meningioma, mesothelioma, multiple myeloma, myeloid leukemia, myeloma, myxosarcoma, neuroblastoma, NUT midline cancer, non-small cell lung cancer, oligodendroglioma, oral cancer, osteogenic sarcoma, ovarian cancer, pancreatic cancer, papillary adenocarcinoma, papillary carcinoma, pineal gland tumor, polycythemia vera, prostate cancer, rectal cancer, renal cell carcinoma, retinoblastoma, rhabdomyosarcoma, sarcoma, sebaceous gland cancer, seminoma, skin cancer, small cell lung cancer, solid tumor, small cell lung cancer, gastric cancer, squamous cell carcinoma, synovial tumor, sweat adenoma, thyroid cancer, primary macroglobulinemia, testicular tumor, uterine cancer, and nephroblastoma etc.

**22.** A pharmaceutical composition, comprising the nitrogenous macrocylic compound represented by the formula **III-0**, a tautomer thereof, an optical somer thereof, a hydrate thereof, a solvate thereof, a pharmaceutically acceptable salt thereof or a prodrug thereof as defined in at least one of claims 1-18, and at least one pharmaceutically acceptable excipient.

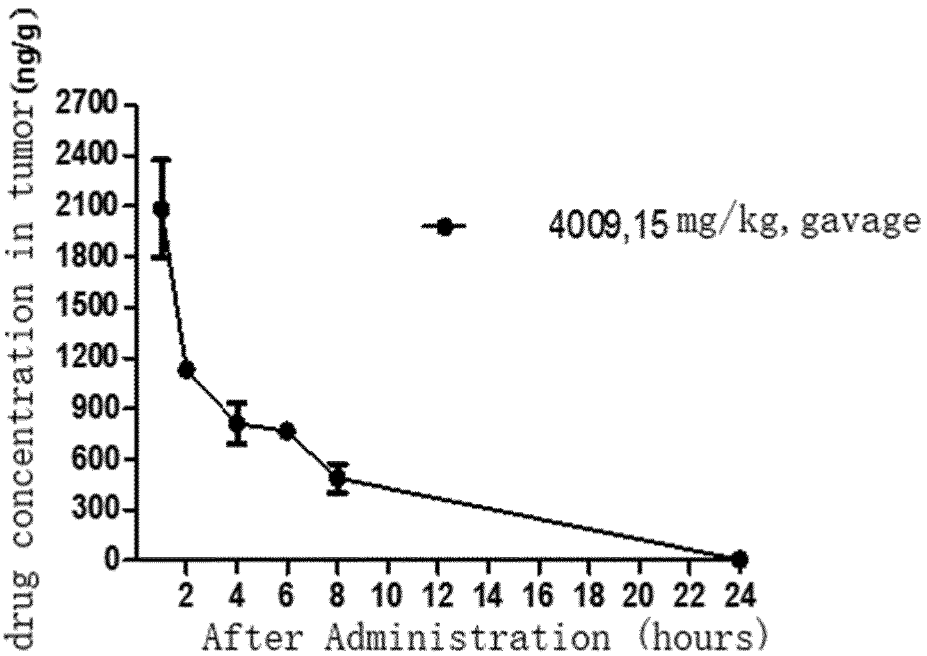


Figure 1

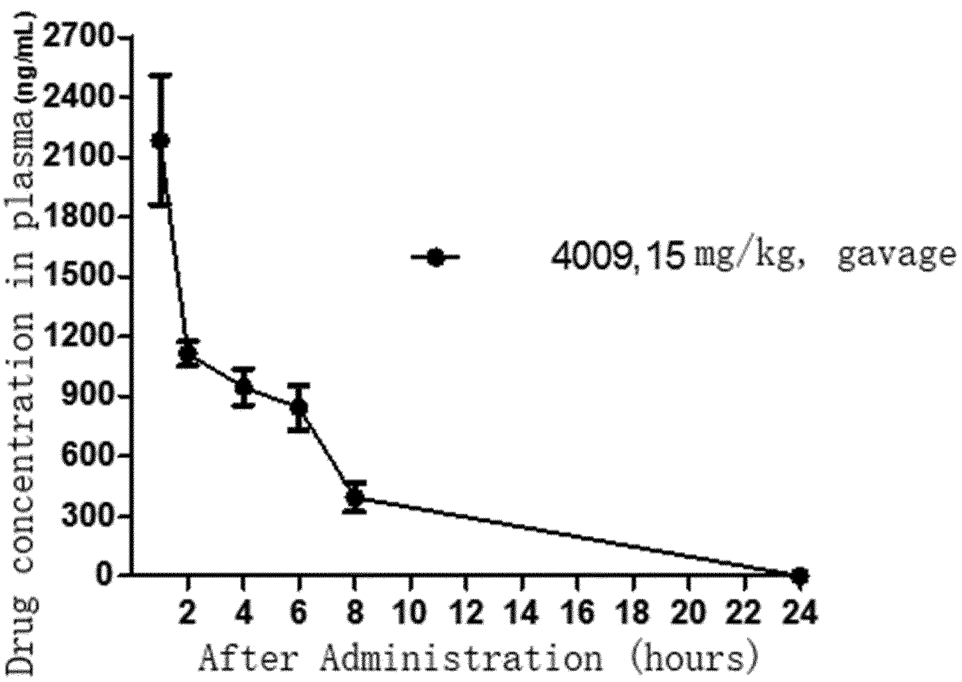


Figure 2

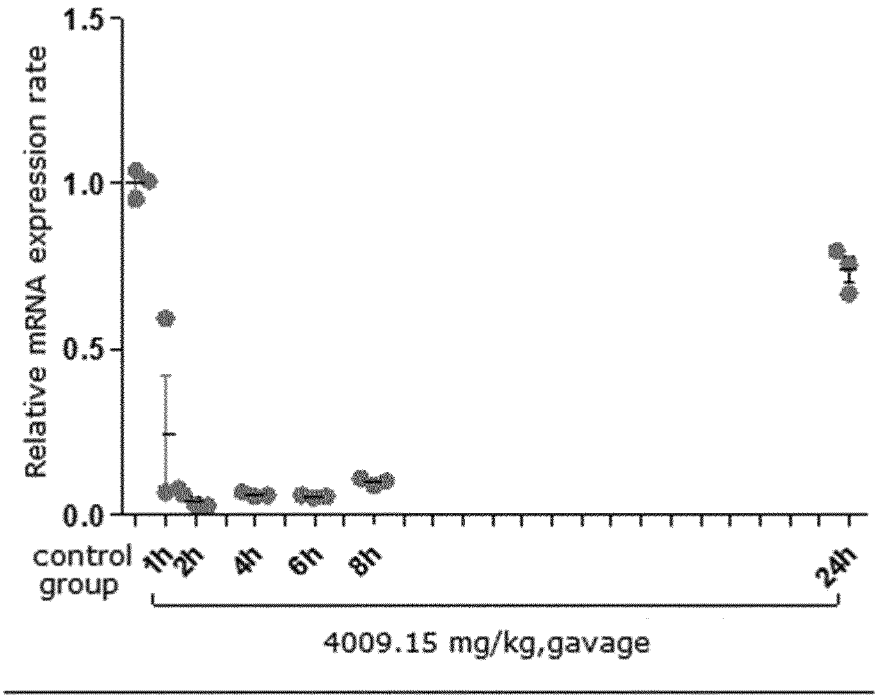


Figure 3

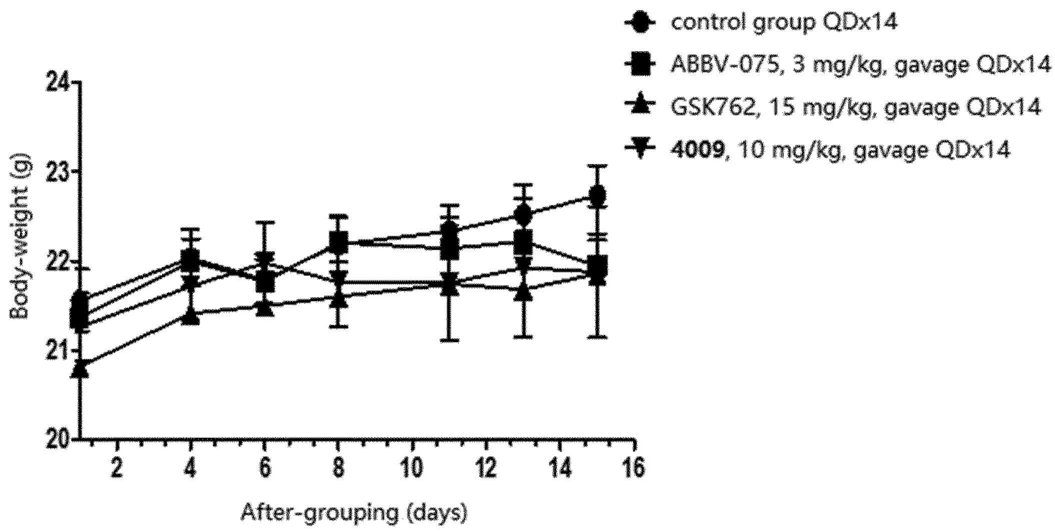


Figure 4

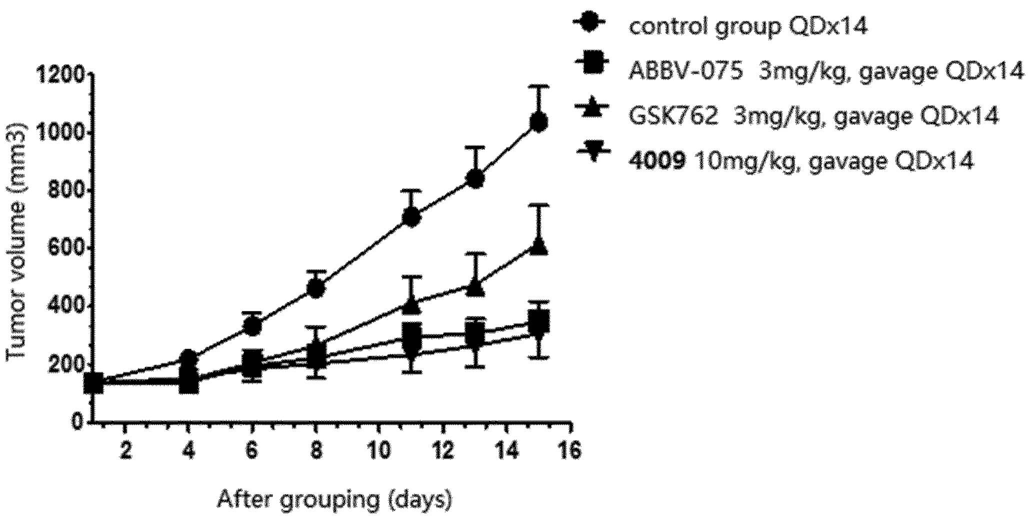


Figure 5

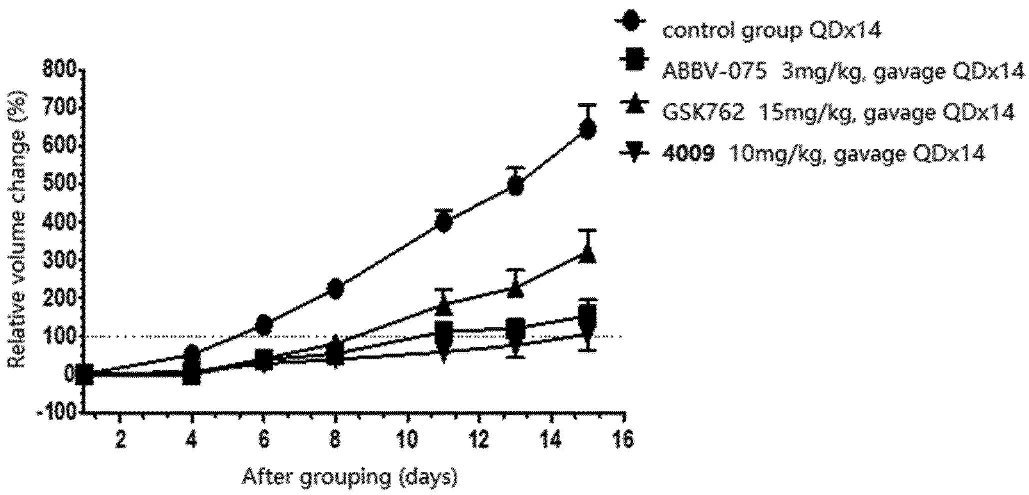


Figure 6

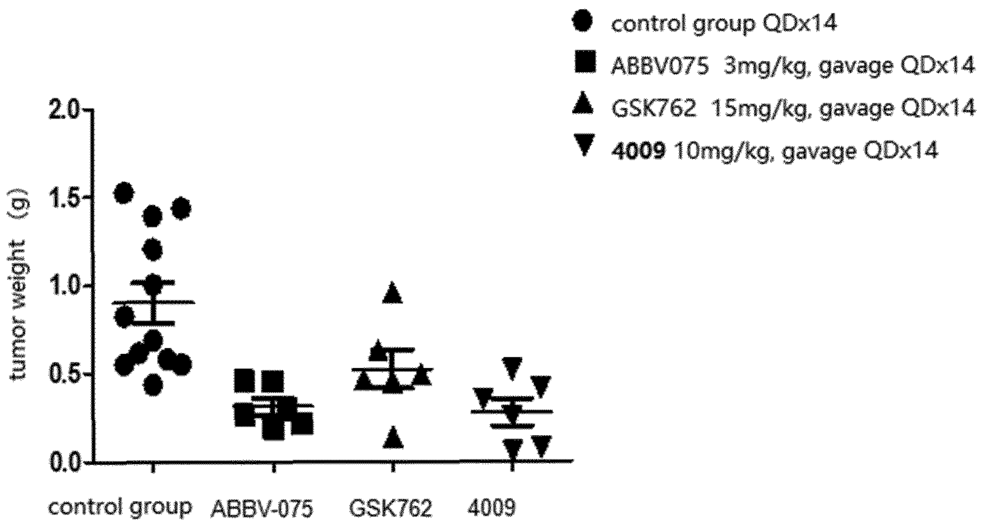
control group QDx14
ABBV075 3mg/kg, gavage QDx14
GSK762 15mg/kg, gavage QDx14
4009 10mg/kg, gavage QDx14
2.0
1.5
1.0
0.5
0.0
tumor weight (g)
control group
ABBV-075
GSK762
4009

Figure 7

**INTERNATIONAL SEARCH REPORT**

International application No.
**PCT/CN2017/110565**

**A. CLASSIFICATION OF SUBJECT MATTER**

C07D 471/04(2006.01)i; C07D 487/14(2006.01)i; C07D 487/04(2006.01)i; A61K 31/55(2006.01)i; A61P 35/00(2006.01)i; A61P 29/00(2006.01)i

According to International Patent Classification (IPC) or to both national classification and IPC

**B. FIELDS SEARCHED**

Minimum documentation searched (classification system followed by classification symbols)

C07D 471; C07D 487; A61K 31; A61P 35; A61P 29

Documentation searched other than minimum documentation to the extent that such documents are included in the fields searched

Electronic data base consulted during the international search (name of data base and, where practicable, search terms used)

VEN, CNABS, CPRSABS, CNKI, REGISTRY, CAPLUS (STN), 罗欣, 溴结构域, 抑制, 肿瘤, 癌, 苯基, 吡啶, +苯并+, +吡咯并+, +吡啶并+, +哒嗪并+, +吖庚因+, +氮杂卓+, BET, BRD?, bromodomain?, inhibit+, +tumor+, +tumour+, +cancer+, phenyl, +pyrid+, +benz+, +pyrrol+, +pyridazin+, +azepin+, 根据对权利要求1-17要求保护的化合物所合理预期的结构检索

**C. DOCUMENTS CONSIDERED TO BE RELEVANT**

| Category* | Citation of document, with indication, where appropriate, of the relevant passages | Relevant to claim No. |
|---|---|---|
| X | WO 2016157221 A1 (JUBILANT BIOSYS LTD.) 06 October 2016 (2016-10-06) claims, descriptioin, embodiments 25, 25A, 25B, 28, 29, 33, 35-40, 40A, 40B, 41, 41A, 41B and 42-46, and abstract | 1-18、21和22 |
| X | DEMOPOULOS, B.J. et al. "Pyrrole Chemistry. XXVI. A Synthesis of Porphobilinogen from Pyrrole" *Canadian Journal of Chemistry,* Vol. 61, No. 10; pages 2415-2422, compounds 20 and 21, 31 December 1983 (1983-12-31), 第2415-2422页，化合物20和21 | 20 |
| A | WO 2016157221 A1 (JUBILANT BIOSYS LTD.) 06 October 2016 (2016-10-06) entire document | 19和20 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 January 2018** | **12 February 2018** |

| Name and mailing address of the ISA/CN | Authorized officer |
|---|---|
| **State Intellectual Property Office of the P. R. China**<br>**No. 6, Xitucheng Road, Jimenqiao**<br>**Haidian District, Beijing 100088**<br>**China** | |
| Facsimile No. **(86-10) 62019451** | Telephone No. |

Form PCT/ISA/210 (second sheet) (July 2009)

## INTERNATIONAL SEARCH REPORT

International application No.
**PCT/CN2017/110565**

**Box No. III Observations where unity of invention is lacking (Continuation of item 3 of first sheet)**

This International Searching Authority found multiple inventions in this international application, as follows:

[1] I: In claims 1-19, 21 and 22, claims 1-18 relate to a nitrogen-containing macrocyclic compound represented by formula III-0, a tautomer thereof, an optical isomer thereof, a hydrate thereof, a solvate thereof and a pharmaceutically acceptable salt or prodrug thereof; claim 19 relates to a preparation method for some of the compounds; and claim 21 relates to the use of the compound for preparing a medicament and claim 22 relates to a pharmaceutical composition comprising the compound.

[2] II: The compounds of formulas III-0-C, III-0-J, III-K and III-0-L in claim 20 relate to an intermediate for preparing the compound where Y is -N-, X is $-CH_2-$ or -C(=O)- and W is $-C(R^1)(R^3)$.

[3] III: The compounds of formulas III-0-E, III-0-F, III-G, III-0-H and III-I in claim 20 relate to an intermediate for preparing the compound where Y is =C- or -CH-, X is =N- or -NH- and W is $-C(R^1)(R^3)-$.

[4] The compound (i.e., a final product) in claims 1-19 is not manufactured directly from the intermediate of claim 20, and is also not separated directly from the few intermediates comprising a same basic structural unit. In addition, the compounds of formulas III-0-C, III-0-J, III-K and III-0-L and the compounds of formulas III-0-E, III-0-F, III-G, III-0-H and III-I in claim 20 are intermediates for preparing compounds with different main structures. Therefore, the above-mentioned three claims do not share a same or corresponding special technical feature, and the present application does not meet the requirement of unity under PCT Rule 13.1.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2009)

**INTERNATIONAL SEARCH REPORT**
**Information on patent family members**

International application No.
**PCT/CN2017/110565**

| Patent document cited in search report | Publication date (day/month/year) | Patent family member(s) | Publication date (day/month/year) |
|---|---|---|---|
| WO 2016157221 A1 | 06 October 2016 | AU 2016242473 A1 | 26 October 2017 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201610990554X
- CN 201710296368
- WO 2011054553 A
- WO 2011054845 A
- WO 2013097052 A
- WO 2013185284 A
- WO 2014139324 A
- WO 2014164771 A
- WO 2015100282 A
- WO 2015075665 A
- WO 2015080707 A
- WO 2015164480 A
- WO 2015195862 A
- WO 2016050821 A


### Non-patent literature cited in the description


- **BERGE et al.** Pharmaceutical Salts. *Journal of Pharmaceutical Science,* 1977, vol. 66, 1-19
- Remington: The Science and Practice of Pharmacy. Lippincott, Williams & Wilkins, 2005
- **MAEHR.** *J. Chem. Ed.,* 1985, vol. 62, 114-120
- **T. GREENE ; P. WUTS.** Protecting Groups in Organic Synthesis. John Wiley & Sons, 1999